(19) Europäisches Patentamt / European Patent Office / Office européen des brevets

(11) EP 2 100 876 A2

(12) EUROPEAN PATENT APPLICATION

(43) Date of publication: **16.09.2009 Bulletin 2009/38**

(21) Application number: **09007837.9**

(22) Date of filing: **18.09.2003**

(51) Int Cl.: ***C07C 275/34*** *(2006.01)* ***A61K 31/155*** *(2006.01)* ***A61K 31/135*** *(2006.01)* ***A61K 31/165*** *(2006.01)* ***A61K 31/17*** *(2006.01)* ***A61K 31/18*** *(2006.01)*

(84) Designated Contracting States: **AT BE BG CH CY CZ DE DK EE ES FI FR GB GR HU IE IT LI LU MC NL PT RO SE SI SK TR**

(30) Priority: **18.09.2002 EP 02020987**

(62) Document number(s) of the earlier application(s) in accordance with Art. 76 EPC: **03753427.8 / 1 539 683**

(71) Applicant: **Jerini AG**
**10115 Berlin (DE)**

(72) Inventor: **The designation of the inventor has not yet been filed**

(74) Representative: **Bohmann, Armin K.**
**Bohmann & Loosen**
**Anwaltssozietät**
**Nymphenburger Strasse 1**
**80335 München (DE)**

Remarks:
This application was filed on 15-06-2009 as a divisional application to the application mentioned under INID code 62.

# (54) New compounds for the inhibition of rotamases and use thereof

(57) The present invention is related to a compound of the formula (I):

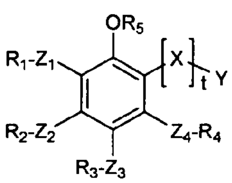

I

wherein $Z_1$, $Z_2$, $Z_3$ and $Z_4$ are each and independently selected from the group comprising - C(O)-, -C(S)-, -C (O)-$NR_{10}$-, -C(S)-$NR_{11}$-, -C(N-CN)-$NR_{12}$-, -S(O)-, -S ($O_2$)-, -S(O)-$NR_{13}$-, and -S($O_2$)-$NR_{14}$-, -O-, -S- or are each and individually absent; X is a spacer and is independently selected from the group comprising -M1-L1-K-L2-M2-, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl, wherein Y is different from a peptide or is absent.

EP 2 100 876 A2

## Description

The present invention is related to new compounds and the use of said compounds as an inhibitor to rotamases and for the manufacture of medicaments.

Rotamases, also referred to as peptidyl-prolyl *cis*-trans isomerases (PPIases) are a family of enzymes important in protein folding, assembly and transport. They act as catalysts to promote isomerization about the peptidyl-prolyl bond, which can have profound effects on protein function.

PPIases are divided into three classes, cyclophilins, FK-506 binding proteins (FKBPs) and the Pinl/parvulin class. While cyclophilins and FKBPs are distinguished by their ability to bind immunosuppressant molecules cyclosporin and FK-506, respectively, the Pinl/parvulin class binds neither of these immunosuppressants and is structurally unrelated to the other two classes. Known members of the Pinl/parvulin class include Pins 1 - 3 (Lu et al., Nature 380:544-547, 1996), Pin-L (Campbell et al., Genomics 44:157-162, 1997), parvulin (Rahfeld et al., FEBS Letts 352:180-184, 1994), dodo (Maleszka et al., Proc Natl Acad Sci USA 93:447-451, 1996) and Ess1/Pft1 (Hanes et al., Yeast 5:55-72, 1989; and Hani et al., FEBS Letts 365:198-202, 1995).

Recent research suggests that members of the Pinl/parvulin class are essential modulators of the cell cycle, and mitosis in particular. Lu et al., Nature 380:544-547, 1996 reports that depletion of Pin1/Ess1 in yeast or human cells induces mitotic arrest followed by apoptosis, indicating that enzymes in this class serve an essential function in cell division and proliferation.

Accordingly, compounds inhibiting rotamases can serve as agents for the treatment of a variety of disorders which are characterized by an inappropriate cell proliferation including cancer and infectious diseases.

In the prior art a huge number of compounds are described which are active as inhibitors to rotamase. The respective compounds are, among others, peptide derivatives such as amino methylene-peptides which are described in European patent EP 0 610 743, or non-peptidic or non-peptidomimetic molecules.

Given the importance of rotamase there is an ongoing need in the art to provide further compounds which are suitable as inhibitors to rotamases and thus suitable to be used as a medicament for those diseases wherein a rotamase is involved in the pathological mechanism.

Accordingly, the problem underlying the present invention is to provide compounds which inhibit a rotamase. A further problem underlying the present invention is to provide new compounds for the treatment of diseases the pathophysiology of which involves an imbalanced or undesired activity of a rotamase.

In a first aspect the problem underlying the present invention is solved by a compound having the structure

$$A\text{-}[X]_t\text{-}Y \qquad \textbf{(0)}$$

wherein A is selected from the group comprising cycloalkyl, substituted cycloalkyl, heterocyclyl, substituted heterocyclyl, aryl, substituted aryl, heteroaryl and substituted heteroaryl;

X is a spacer and is independently selected from the group comprising
-M1-L1-K-L2-M2-,

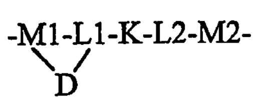 , 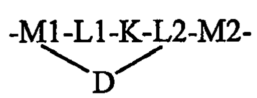 , 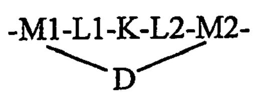 , 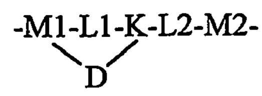 ,

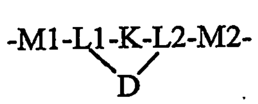 , 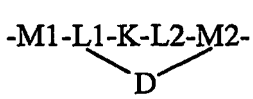 , 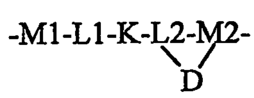 or 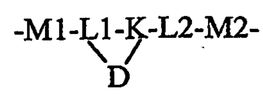

wherein

K is selected from the group comprising

C=T,
O, S, S(O) and $S(O_2)$,

or is absent,
with =T being selected from the group comprising
=O, =S, =N-$R^e$, =N-CN, =N-$NO_2$ and =CH-$NO_2$,

L1 and L2 are each and independently selected from the group comprising O, S and primary amines, more particularly $NR^c$, $NR^d$; or being individually and independent from each other absent

M1 and M2 are each and independently selected from the group comprising
-$(CR^aR^b)n$-,
-$(CR^fR^g)m$-,
cycloalkyl, substituted cycloakyl, heterocyclyl, substituted heterocyclyl, aryl, substituted aryl, heteroaryl and substituted heteroaryl heteroaryl, or being individually and independent from each other absent,

wherein t is independently selected from n and/or m and is any integer from 0 to 10, whereby if t is 2 or more any of the spacer -M1-L1-K-L2-M2- can be the same or different from any of the spacer(s) X repeated,

wherein $R^c$, $R^d$ and $R^e$ are independently from each other selected from the group H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl;

wherein $R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, mono-substituted poly-unsaturated heterocyclyl, aryl, substituted aryl, heteroaryl and substituted heteroaryl,

or wherein Y is absent.

In a preferred embodiment Y is different from a peptide.

In a further embodiment A is

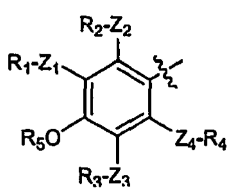

or

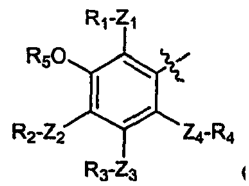

or

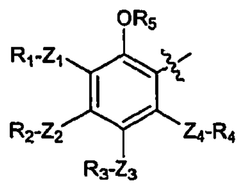

In a second aspect which is actually an embodiment of the first aspect of the invention, the problem is solved by a compound which has any of the structures according to formulae (I), (II), (III), (IV) or (V):

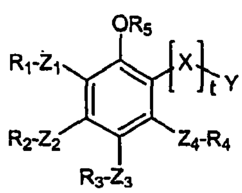 I ,

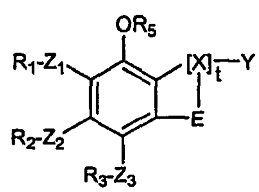 II ,

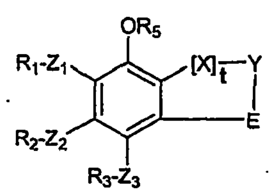 III ,

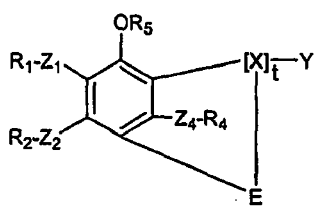 IV or

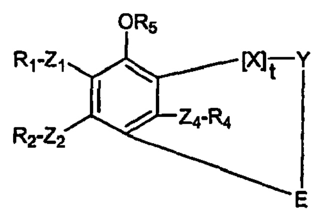 V

wherein $R_1$, $R_2$, $R_3$ and $R_4$ are each independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl;

wherein $R_1$ and $R_2$, $R_2$ and $R_3$, $R_3$ and $R_4$, $R_1$ and $R_3$, $R_1$ and $R_4$, and $R_2$ and $R_4$ may be linked so as to form a ring comprising 4 to 12 members, preferably 5 to 10 members, more preferably 5 or 6 or 7 members,

wherein $Z_1$, $Z_2$, $Z_3$ and $Z_4$ are each and independently selected from the group comprising - C(O)-, -C(S)-, -C (O)-$NR_{10}$-, -C(S)-$NR_{11}$-, -C(N-CN)-$NR_{12}$-, -S(O)-, -S($O_2$)-, -S(O)-$NR_{13}$-, -S($O_2$)-$NR_{14}$-, -O-, and -S-, or are each and individually absent;

$R_5$ is selected from the group comprising H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, substituted alkylheteroaryl and -C(O)-Q;

wherein Q is selected from the group comprising H, $NHR_{15}$, alkyl, substituted alkyl, cycloalkyl, substituted

cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, and substituted alkylheteroaryl; and

$R_6$, $R_7$, $R_8$, $R_9$, $R_{10}$, $R_{11}$, $R_{12}$, $R_{13}$, $R_{14}$ and $R_{15}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

X is a spacer and is independently selected from the group comprising
-M1-L1-K-L2-M2-,

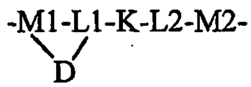 , 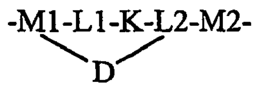 , 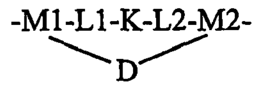 , 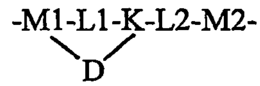 ,

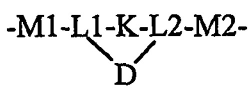 , 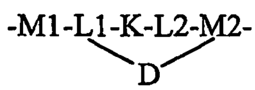 , 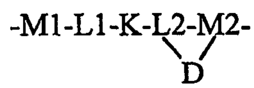 or 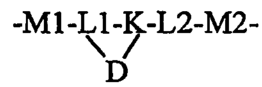

wherein

K is selected from the group comprising

C=T,
O, S, S(O) and $S(O_2)$,
or is absent,
with =T being selected from the group comprising
=O, =S, $=N-R^e$, =N-CN, $=N-NO_2$ and $=CH-NO_2$,

L1 and L2 are each and independently selected from the group comprising O, S and primary amines, more particularly $NR^c$, $NR^d$; or being individually and independent from each other absent

M1 and M2 are each and independently selected from the group comprising
$-(CR^aR^b)n-$,
$-(CR^fR^g)m-$,
cycloalkyl, substituted cycloakyl, heterocyclyl, substituted heterocyclyl, aryl, substituted aryl, heteroaryl and substituted heteroaryl heteroaryl], or being individually and independent from each other absent,

wherein D is $C_1$-$C_6$ alkyl, preferably straight $C_1$-$C_6$ alkyl, $C_1$-$C_6$ alkenyl, preferably straight $C_1$-$C_6$ alkenyl, $C_1$-$C_6$ alkynyl , preferably straight $C_1$-$C_6$ alkynyl, whereby preferably any of the alkyl, alkenyl and alkynyl may individually and independently comprise from 0 to 3 heteroatoms, and/or whereby preferably any of the alkyl, alkenyl and alkynyl can be individually and independently substituted, more preferably by 1 or 2 substituent(s) preferably each independently selected from H, halo, $OR_{16}$, alkyl, and substituted alkyl,

wherein n and m are each and independently selected from each other and are each any integer from 0 to 10,

whereby if n is 2 or more, the group(s) $-(CR^aR^b)-$ which is/are repeated, can be the same or different from any of the group(s) $-(CR^aR^b)-$,

whereby any individual group can be linked to any other group or any moiety of the compound through a bond selected from the group comprising single bonds, double bonds and triple bonds,

whereby if m is 2 or more, the group(s) -($CR^fR^g$)- which is/are repeated, can be the same or different from any of the group(s) -($CR^fR^g$)-,

whereby any individual group can be linked to any other group or any moiety of the compound through a bond selected from the group comprising single bonds, double bonds and triple bonds,

wherein t is independently selected from n and/or m and is any integer from 0 to10, whereby if t is 2 or more any of the spacer -M1-L1-K-L2-M2- can be the same or different from any of the spacer(s) X repeated,

wherein

$R^c$, $R^d$ and $R^e$ are independently from each other selected from the group H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl; and

$R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

wherein E is $C_1$-$C_6$ alkyl, preferably straight $C_1$-$C_6$ alkyl, $C_1$-$C_6$ alkenyl, preferably straight $C_1$-$C_6$ alkenyl, $C_1$-$C_6$ alkynyl, preferably straight $C_1$-$C_6$ alkynyl, whereby preferably any of the alkyl, alkenyl and alkynyl may comprise individually and independently from 0 to 3 heteroatoms, and/or whereby preferably any of the alkyl, alkenyl and alkynyl can be individually and independently substituted by preferably 1 or 2 substituent(s) each preferably independently selected from the group comprising H, halo, $OR_{21}$, alkyl, and substituted alkyl.

$R_{16}$, $R_{17}$, $R_{18}$, $R_{19}$, $R_{20}$ and $R_{21}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl,

or wherein Y is absent.

In an embodiment Y is different from a peptide.

In an embodiment of the first and the second aspect of the present invention the moiety A and phenol moiety, respectively forms a cyclic structure with the spacer X and/or Y.

In an embodiment of the first and the second aspect of the present invention the compound is

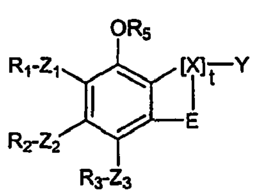 II ,

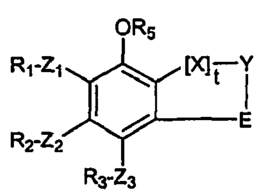 III ,

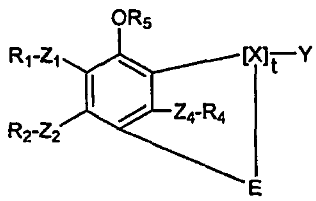

IV

or

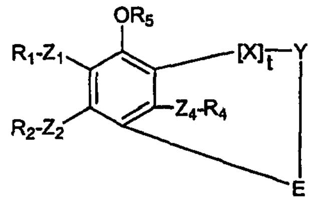

V

In a further embodiment of the first and the second aspect of the present invention the compound is selected from the group comprising

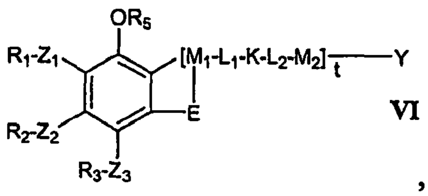

VI ,

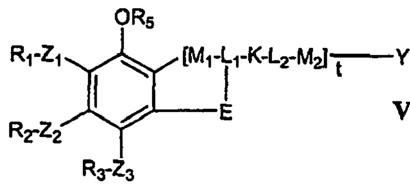

VII ,

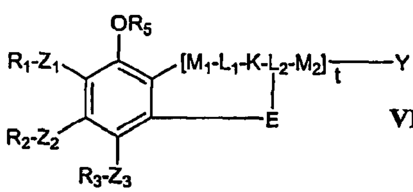

VIII ,

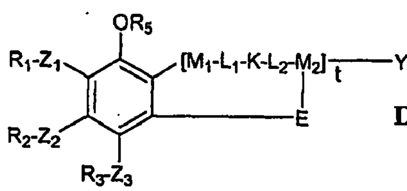

IX ,

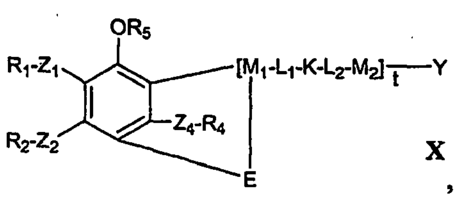

X ,

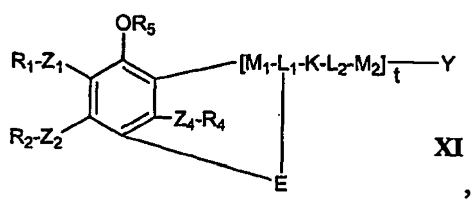

XI ,

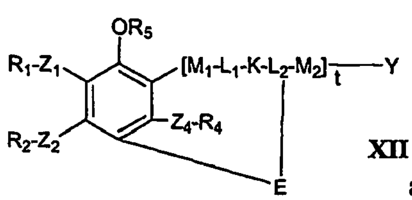

XII and

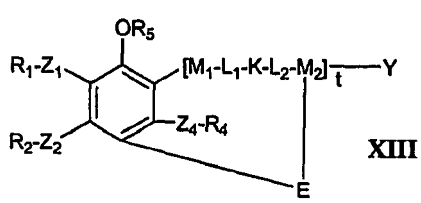

XIII

In an embodiment of the first and the second aspect of the present invention K is C=T.

In a further embodiment of the first and the second aspect of the present invention T is selected from the group comprising O and S.

In a preferred embodiment of the first and the second aspect of the present invention T is O.

In an alternative preferred embodiment of the first and the second aspect of the present invention T is S.

In a further alternative embodiment of the first and the second aspect of the present invention T is selected from the group comprising N-CN, N-$NO_2$, CH-$NO_2$ and N-$R^e$.

In an embodiment of the first and the second aspect of the present invention, more particularly the embodiment where T is either O or S, L1 and L2 are each and independently a primary amine, preferably $NR^c$ and/or $NR^d$.

In an embodiment of the first and the second aspect of the present invention n = 0 and m is any integer from 0 to 10.

In an embodiment of the first and the second aspect of the present invention $R_1$ and/or $R_3$ are selected from the group comprising halo, alkyl, substituted alkyl, heterocyclyl, substituted heterocyclyl, heteroaryl and substituted heteroaryl. In an even more preferred embodiment $R_1$ is halo.

In an embodiment of the first and the second aspect of the present invention $R_5$ is selected from the group comprising H and -C(O)-Q. In a preferred embodiment Q is selected from alkylheterocyclyl and substituted alkylheterocyclyl . In an even more preferred embodiment Q is selected from the group comprising N-acylated morpholino-, N-acylated piperazino- and N-acyl-derivatives.

In an embodiment of the first and the second aspect of the present invention $R_6$ is alkyl or substituted alkyl.

In an embodiment of the first and the second aspect of the present invention $R_8$ and $R_9$ are individually and separately selected from the group comprising H, alkyl and substituted alkyl.

In an embodiment of the first and the second aspect of the present invention n and m are individually and independently any integer from 1 to 3.

In an alternative embodiment of the first and the second aspect of the present invention n is any integer from 0 to 3 and is preferably 0 or 1.

In a further alternative embodiment of the first and the second aspect of the present invention n and m are both 0.

In an embodiment of the first and the second aspect of the present invention t is 1 or 2.

In another embodiment of the first and the second aspect of the present invention $R^c$ and/or $R^d$ are each and independently from each other selected from the group comprising alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

In still another embodiment of the first and the second aspect of the present invention $R^a$, $R^b$, $R^f$ and $R^g$ are each individually and independently from, each other selected from the group comprising H, $OR_{17}$, $SR_{18}$, $NR_{19}R_{20}$, halo, alkyl and substituted alkyl.

In a preferred embodiment of the first and the second aspect of the present invention Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkylnyl and substituted branched alkynyl.

In an alternate preferred embodiment of the first and the second aspect of the present invention Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted mono-unsaturated heterocyclyl, poly-substituted mono-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

In a particularly preferred embodiment of the first and the second aspect of the present invention which is also referred to as the NR-CZ-NR embodiment X is

$-(CR^aR^b)_n-NR^c-CZ-NR^d-(CR^fR^g)_m-$

and Z is preferably selected from the group comprising O, S, N-CN, $N-NO_2$ and $CH-NO_2$.

In an embodiment of the NR-CZ-NR embodiment m is any integer from 1 to 10.

In an embodiment of the NR-CZ-NR embodiment $R_5$ is selected from the group comprising H and -C(O)-Q, preferably m is any integer from 1 to 10.

In an embodiment of the NR-CZ-NR embodiment wherein $R_5$ is H.

In an embodiment of the NR-CZ-NR embodiment n is 0, preferably $R_5$ being selected from the group comprising H and -C(O)-Q, more preferably m being any integer from 1 to 10. In an alternative embodiment n is any integer from 1 to 10.

In an embodiment of the NR-CZ-NR embodiment t is 1.

In an embodiment of the first and/or second aspect of the present invention and particularly in an embodiment of the NR-CZ-NR embodiment Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkynyl and substituted branched alkynyl.

In an embodiment of the first and/or second aspect of the present invention and particularly in an embodiment of the NR-CZ-NR embodiment Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide. Alternatively, Y can be absent.

In an embodiment of the NR-CZ-NR embodiment $R^c$ and/or $R^d$ are independently from each other selected from the group alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

In a particularly preferred embodiment of the first and the second aspect of the present invention which is also referred to as the NR embodiment is X is

$-(CR^aR^b)_n-NR^c-(CR^fR_g)_m-$

In an embodiment $R^a$, $R^b$, $R^c$, $R^d$, $R^e$, $R^f$ and $R^g$ are independently from each other selected from the group H, alkyl, substituted alkyl, cycloalkyl, substituted cycloalkyl, aryl, substituted aryl, alkylaryl, substituted alkylaryl, heterocyclyl, substituted heterocyclyl, heteroaryl, substituted heteroaryl.

In an embodiment $R_5$ is selected from the group comprising H and -C(O)-Q. Preferably $R_5$ is H.

In a further embodiment m is any integer between 1 and 10. Preferably n is 0.

In a still further embodiment $R_5$ is selected from the group comprising H and -C(O)-Q, whereby m is any integer between 1 and 10. Preferably n is 0. In an even more preferred embodiment $R_5$ is H.

In a preferred embodiment of the NR embodiment X is

$-(CR^aR^b)_n-NR^c-(CR^fR^g)_m-$, and

wherein t is 1. Preferably, Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, and substituted branched alkynyl. Preferably $R_5$ is selected from the group comprising H and -C(O)-Q and even more preferably $R_5$ is H. In a still further preferred embodiment n is 0.

In a preferred embodiment of the NR embodiment, wherein X is

$-(CR^aR^b)_n-NR^c-(CR^fR_g)_m-$,

wherein t is 1m is any integer between 1 and 10, preferably m is any integer between 2 and 10. Preferably, $R_5$ is selected from the group comprising H and -C(O)-Q, and more preferably $R_5$ is H. Also preferably Y is selected from the group comprising cycloalkyl; substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide or is absent.

In a preferred embodiment $R_5$ is selected from the group comprising H and -C(O)-Q, preferably $R_5$ is H and even more preferably n is 0.

In a particularly preferred embodiment of the first and the second aspect of the present invention which is also referred to as the NR-Z embodiment X is

$-(CR^aR^b)_n-NR^c-Z-(CR^fR^g)_m-$ and can be inserted in any orientation into any of the preceding formulae,

and wherein Z is selected from the group comprising C(O), C(S), $S(O_2)$, C(O)-O, and C(O)-S.

In an embodiment $R_5$ is selected from the group comprising H and -C(O)-Q, preferably $R_5$ is H.

In an embodiment n is 0.

In a further embodiment of the NR-Z embodiment X is

$-(CR^aR^b)_n-NR^c-Z-(CR^fR_g)_m-$ and can be inserted in any orientation into any of the preceding formulae,

and Z is selected from the group comprising C(O), C(S), $S(O_2)$, C(O)-O, and C(O(-S, and

wherein preferably t is 1. Preferably Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, and substituted branched alkynyl. More preferably $R_5$ is selected from the group comprising H and -C(O)-Q and even more preferably $R_5$ is H. In any of the latter embodiments n is 0.

In a further embodiment of the NR-Z embodiment wherein X is

$-(CR^aR^b)_n-NR^c-Z-(CR^fR^g)_m-$ and can be inserted in any orientation into any of the preceding formulae,

and Z is selected from the group comprising C(O), C(S), $S(O_2)$, C(O)-O, and C(O)-S, and

wherein preferably t is 1, m is any integer between 1 and 10. Preferably, $R_5$ is selected from the group comprising H and -C(O)-Q, and more preferably $R_5$ is H. In an embodiment of the latter embodiments Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide or wherein Y is absent. Preferably, $R_5$ is selected from the group comprising H and -C(O)-Q, more preferably $R_5$ is H. In a particularly preferred embodiment n is 0.

In the embodiment where Y is as defined in the preceding paragraph, m is preferably any integer between 2

and 10. Preferably, $R_5$ is selected from the group comprising H and -C(O)-Q and more preferably $R_5$ is H. Even more preferably, in any of these embodiments n is 0.

In a third aspect which is actually an embodiment of the first aspect of the invention, the problem is solved by a compound which has any of the structures according to formulae (XIV), (XV), (XVI), (XVIII) or (XVIII):

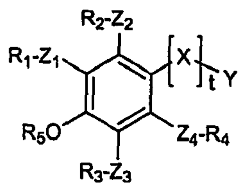
XIV ,
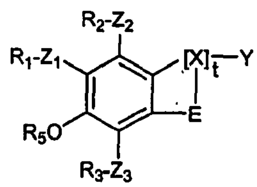
XV or
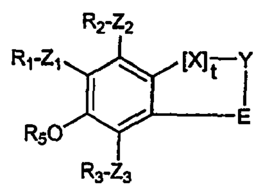
XVI

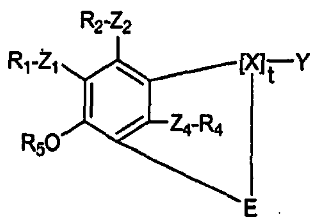
XVII or
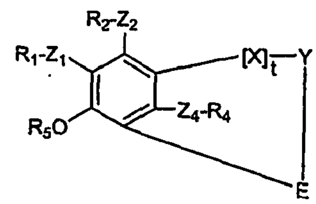
XVIII

wherein $R_1$, $R_2$, $R_3$ and $R_4$ are each independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl;

wherein $R_1$ and $R_2$, $R_2$ and $R_3$, $R_3$ and $R_4$, $R_1$ and $R_3$, $R_1$ and $R_4$, and $R_2$ and $R_4$ may be linked so as to form a ring comprising 4 to 12 members, preferably 5 to 10 members, more preferably 5 or 6 or 7 members,

wherein $Z_1$, $Z_2$, $Z_3$ and $Z_4$ are each and independently selected from the group comprising - C(O)-, -C(S)-, -C (O)-$NR_{10}$-, -C(S)-$NR_{11}$-, -C(N-CN)-$NR_{12}$-, -S(O)-, -S($O_2$)-, -S(O)-$NR_{13}$-, -S($O_2$)-$NR_{14}$-, -O-, and -S-, or are each and individually absent;

$R_5$ is selected from the group comprising H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, substituted alkylheteroaryl and -C(O)-Q;

wherein Q is selected from the group comprising H, $NHR_{15}$, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, and substituted alkylheteroaryl; and

$R_6$, $R_7$, $R_8$, $R_9$, $R_{10}$, $R_{11}$, $R_{12}$, $R_{13}$, $R_{14}$ and $R_{15}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

X is a spacer and is independently selected from the group comprising
-M1-L1-K-L2-M2-,

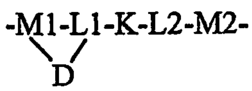 , 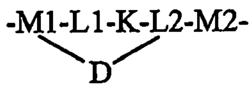 , 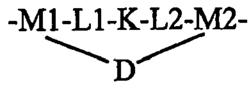 , 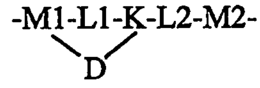 ,

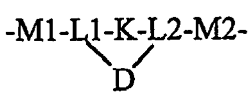 , 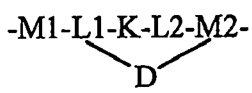 , 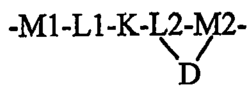 or 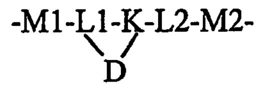

wherein

K is selected from the group comprising

C=T,
O, S, S(O) and $S(O_2)$,
or is absent,
with =T being selected from the group comprising
=O, =S, $=N-R^e$, =N-CN, $=N-NO_2$ and $=CH-NO_2$.

L1 and L2 are each and independently selected from the group comprising O, S and primary amines, more particularly $NR^c$, $NR^d$; or being individually and independent from each other absent

M1 and M2 are each and independently selected from the group comprising
$-(CR^aR^b)n-$,
$-(CR^fR^g)m-$,
cycloalkyl, substituted cycloakyl, heterocyclyl, substituted heterocyclyl, aryl, substituted aryl, heteroaryl and substituted heteroaryl heteroaryl], or being individually and independent from each other absent,

wherein D is $C_1$-$C_6$ alkyl, preferably straight $C_1$-$C_6$ alkyl, $C_1$-$C_6$ alkenyl, preferably straight $C_1$-$C_6$ alkenyl, $C_1$-$C_6$ alkynyl, preferably straight $C_1$-$C_6$alkynyl, whereby preferably any of the alkyl, alkenyl and alkynyl may individually and independently comprise from 0 to 3 heteroatoms, and/or whereby preferably any of the alkyl, alkenyl and alkynyl can be individually and independently substituted, more preferably by 1 or 2 substituent(s) preferably each independently selected from H, halo, $OR_{16}$, alkyl, and substituted alkyl,

wherein n and m are each and independently selected from each other and are each any integer from 0 to 10,

whereby if n is 2 or more, the group(s) $-(CR^aR^b)-$ which is/are repeated, can be the same or different from any of the group(s) $-(CR^aR^b)-$,

whereby any individual group can be linked to any other group or any moiety of the compound through a bond selected from the group comprising single bonds, double bonds and triple bonds,

whereby if m is 2 or more, the group(s) $-(CR^fR^g)-$ which is/are repeated, can be the same or different from any of the group(s) $-(CR^fR^g)-$,

whereby any individual group can be linked to any other group or any moiety of the compound through a bond selected from the group comprising single bonds, double bonds and triple bonds,

wherein t is independently selected from n and/or m and is any integer from 0 to 10, whereby if t is 2 or more any of the spacer -M1-L1-K-L2-M2- can be the same or different from any of the spacer(s) X repeated,
wherein

$R^c$, $R^d$ and $R^e$ are independently from each other selected from the group H, alkyl, substituted alkyl, cycloalkyl,

substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl; alkylheteroaryl and substituted alkylheteroaryl; and

$R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

wherein E is $C_1$-$C_6$ alkyl, preferably straight $C_1$-$C_6$ alkyl, $C_1$-$C_6$ alkenyl, preferably straight $C_1$-$C_6$ alkenyl, $C_1$-$C_6$ alkynyl, preferably straight $C_1$-$C_6$ alkynyl, whereby preferably any of the alkyl, alkenyl and alkynyl may comprise individually and independently from 0 to 3 heteroatoms, and/or whereby preferably any of the alkyl, alkenyl and alkynyl can be individually and independently substituted by preferably 1 or 2 substituent(s) each preferably independently selected from the group comprising H, halo, $OR_{21}$, alkyl, and substituted alkyl.

$R_{16}$, $R_{17}$, $R_{18}$, $R_{19}$, $R_{20}$ and $R_{21}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

wherein Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl,

or wherein Y is absent.

In an embodiment Y is different from a peptide.

In an embodiment of the first and the third aspect of the present invention the moiety A and phenol moiety, respectively forms a cyclic structure with the spacer X and/or Y.

In an embodiment of the first and the third aspect of the present invention the compound is

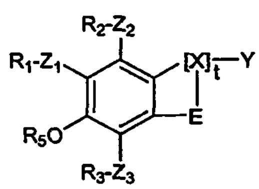 XV ,

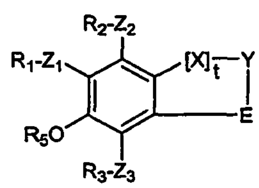 XVI ,

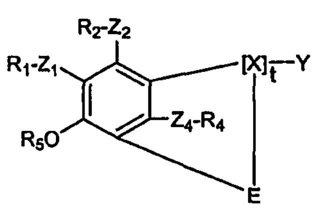 XVII

or

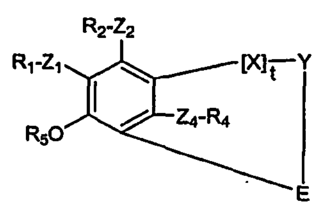 XVIII

In a further embodiment of the first and the third aspect of the present invention the compound is selected from the group comprising

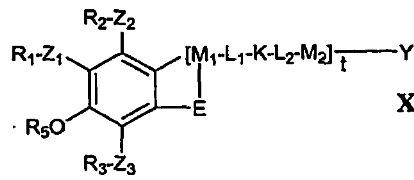
XIX ,

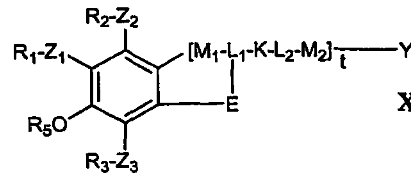
XX ,

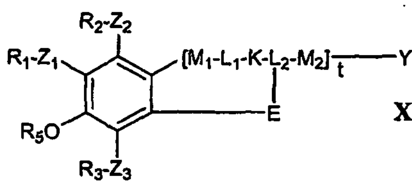
XXI ,

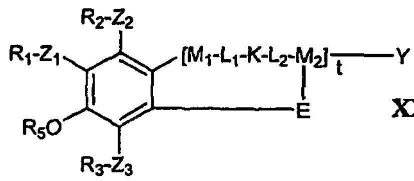
XXII ,

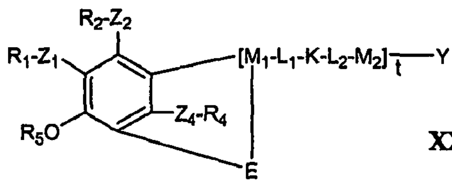
XXIII ,

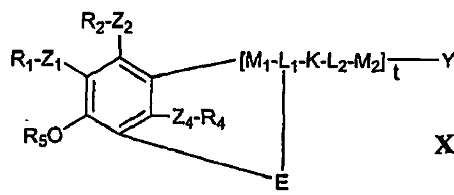
XXIV ,

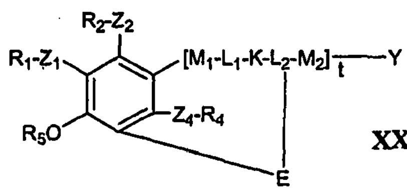
XXV or

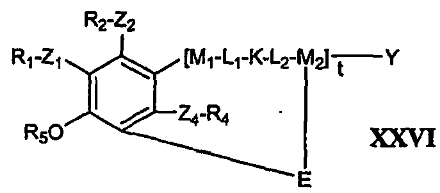
XXVI

In an embodiment of the first and the third aspect of the present invention K is C=T.

In a further embodiment of the first and the third aspect of the present invention T is selected from the group comprising O and S.

In a preferred embodiment of the first and the third aspect of the present invention T is O.

In an alternative preferred embodiment of the first and the third aspect of the present invention T is S.

In a further alternative embodiment of the first and the third aspect of the present invention T is selected from the group comprising N-CN, N-$NO_2$, CH-$NO_2$ and N-$R^e$.

In an embodiment of the first and the third aspect of the present invention, more particularly the embodiment where T is either O or S, L1 and L2 are each and independently a primary amine, preferably $NR^c$ and/or $NR^d$.

In an embodiment of the first and the third aspect of the present invention n = 0 and m is any integer from 0 to 10.

In an embodiment of the first and the third aspect of the present invention $R_1$ and/or $R_3$ are selected from the group comprising halo, alkyl, substituted alkyl, heterocyclyl, substituted heterocyclyl, heteroaryl and substituted heteroaryl. In an even more preferred embodiment $R_1$ is halo.

In an embodiment of the first and the third aspect of the present invention $R_5$ is selected from the group comprising H and -C(O)-Q. In a preferred embodiment Q is selected from alkylheterocyclyl and substituted alkylheterocyclyl . In an even more preferred embodiment Q is selected from the group comprising N-acylated morpholino-, N-acylated piperazino- and N-acyl-derivatives.

In an embodiment of the first and the third aspect of the present invention $R_6$ is alkyl or substituted alkyl.

In an embodiment of the first and the third aspect of the present invention $R_8$ and $R_9$ are individually and separately selected from the group comprising H, alkyl and substituted alkyl.

In an embodiment of the first and the third aspect of the present invention n and m are individually and independently any integer from 1 to 3.
In an alternative embodiment of the first and the third aspect of the present invention n is any integer from 0 to 3 and is preferably 0 or 1.
In a further alternative embodiment of the first and the third aspect of the present invention n and m are both 0.
In an embodiment of the first and the third aspect of the present invention t is 1 or 2.
In another embodiment of the first and the third aspect of the present invention $R^c$ and/or $R^d$ are each and independently from each other selected from the group comprising alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.
In still another embodiment of the first and the third aspect of the present invention $R^a$, $R^b$, $R^f$ and $R^g$ are each individually and independently from each other selected from the group comprising H, $OR_{17}$, $SR_{18}$, $NR_{19}R_{20}$, halo, alkyl and substituted alkyl.
In a preferred embodiment of the first and the third aspect of the present invention Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkylnyl and substituted branched alkynyl.
In an alternate preferred embodiment of the first and the third aspect of the present invention Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted mono-unsaturated heterocyclyl, poly-substituted mono-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.
In a particularly preferred embodiment of the first and the third aspect of the present invention which is also referred to as the NR-CZ-NR embodiment X is
$-(CR^aR^b)_n-NR^c-CZ-NR^d-(CR^fR^g)_m-$
and Z is preferably selected from the group comprising O, S, N-CN, $N-NO_2$ and $CH-NO_2$.
In an embodiment of the NR-CZ-NR embodiment m is any integer from 1 to10.
In an embodiment of the NR-CZ-NR embodiment $R_5$ is selected from the group comprising H and -C(O)-Q, preferably m is any integer from 1 to 10.
In an embodiment of the NR-CZ-NR embodiment wherein $R_5$ is H.
In an embodiment of the NR-CZ-NR embodiment n is 0, preferably $R_5$ being selected from the group comprising H and -C(O)-Q, more preferably m being any integer from 1 to10. In an alternative embodiment n is any integer from 1 to 10.
In an embodiment of the NR-CZ-NR embodiment t is 1.
In an embodiment of the first and/or third aspect of the present invention and particularly in an embodiment of the NR-CZ-NR embodiment Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkylnyl and substituted branched alkynyl.
In an embodiment of the first and/or third aspect of the present invention and particularly in an embodiment of the NR-CZ-NR embodiment Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide. Alternatively, Y can be absent.
In an embodiment of the NR-CZ-NR embodiment $R^c$ and/or $R^d$ are independently from each other selected from the group alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.
In a particularly preferred embodiment of the first and the third aspect of the present invention which is also referred to as the NR embodiment is X is
$-(CR^aR^b)_n-NR^c-(CR^fR^g)_m-$
In an embodiment $R^a$, $R^b$, $R^c$, $R^d$, $R^e$, $R^f$ and $R^g$ are independently from each other selected from the group H, alkyl, substituted alkyl, cycloalkyl, substituted cycloalkyl, aryl, substituted aryl, alkylaryl, substituted alkylaryl, heterocyclyl, substituted heterocyclyl, heteroaryl, substituted heteroaryl.
In an embodiment $R_5$ is selected from the group comprising H and -C(O)-Q. Preferably $R_5$ is H.
In a further embodiment m is any integer between 1 and 10. Preferably n is 0.

In a still further embodiment $R_5$ is selected from the group comprising H and -C(O)-Q, whereby m is any integer between 1 and 10. Preferably n is 0. In an even more preferred embodiment $R_5$ is H.

In a preferred embodiment of the NR embodiment X is

$-(CR^aR^b)_n-NR^c-(CR^fR^g)_m-$, and

wherein t is 1. Preferably, Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, and substituted branched alkynyl. Preferably $R_5$ is selected from the group comprising H and -C(O)-Q and even more preferably $R_5$ is H. In a still further preferred embodiment n is 0.

In a preferred embodiment of the NR embodiment, wherein X is

$-(CR^aR^b)_n-NR^c-(CR^fR_g)_m-$, and

wherein t is 1m is any integer between 1 and 10, preferably m is any integer between 2 and 10. Preferably, $R_5$ is selected from the group comprising H and -C(O)-Q, and more preferably $R_5$ is H. Also preferably Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide or is absent.

In a preferred embodiment $R_5$ is selected from the group comprising H and -C(O)-Q, preferably $R_5$ is H and even more preferably n is 0.

In a particularly preferred embodiment of the first and the third aspect of the present invention which is also referred to as the NR-Z embodiment X is

$-(CR^aR^b)_n-NR^c-Z-(CR^fR^g)_m-$ and can be inserted in any orientation into any of the preceding formulae,

and wherein Z is selected from the group comprising C(O), C(S), $S(O_2)$, C(O)-O, and C(O)-S.

In an embodiment $R_5$ is selected from the group comprising H and -C(O)-Q, preferably $R_5$ is H.

In an embodiment n is 0.

In a further embodiment of the NR-Z embodiment X is

$-(CR^aR^b)_n-NR^c-Z-(CR^fR^g)_m-$ and can be inserted in any orientation into any of the preceding formulae,

and Z is selected from the group comprising C(O), C(S), $S(O_2)$, C(O)-O, and C(O)-S, and wherein preferably t is 1. Preferably Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, and substituted branched alkynyl. More preferably $R_5$ is selected from the group comprising H and -C(O)-Q and even more preferably $R_5$ is H. In any of the latter embodiments n is 0.

In a further embodiment of the NR-Z embodiment wherein X is

$-(CR^aR^b)_n-NR^c-Z-(CR^fR^g)_m-$ and can be inserted in any orientation into any of the preceding formulae,

and Z is selected from the group comprising C(O), C(S), $S(O_2)$, C(O)-O, and C(O)-S, and

wherein preferably t is 1, m is any integer between 1 and 10. Preferably, $R_5$ is selected from the group comprising H and -C(O)-Q, and more preferably $R_5$ is H. In an embodiment of the latter embodiments Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, mono-substituted mono-unsaturated heterocyclyl, poly-substituted mono-unsaturated heterocyclyl, poly-substituted poly-unsaturated heterocyclyl, aryl, substituted aryl, heteroaryl and substituted heteroaryl, wherein Y is different from a peptide or wherein Y is absent. Preferably, $R_5$ is selected from the group comprising H and -C(O)-Q, more preferably $R_5$ is H. In a particularly preferred embodiment n is 0.

In the embodiment where Y is as defined in the preceding paragraph, m is preferably any integer between 2 and 10. Preferably, $R_5$ is selected from the group comprising H and -C(O)-Q and more preferably $R_5$ is H. Even more preferably, in any of these embodiments n is 0.

As used herein, any integer between or any integer from e.g., 0 and 10 or 0 to 10 means 1, 2, 3,4, 5, 6, 7, 8, 9 and 10.

In an embodiment of any aspect of the present invention Y is different from a peptide. As used herein peptide means a polymer of at least two amino acids which are linked by an amide bond. Any of the amino acids may be a natural or a non natural acid.

In a preferred embodiment of any aspect of the present invention m, n and t are independently selected from each other and are preferably any integer between 0 and 5; more preferably if n is 0, m is different from 0 and if m is 0, n is different from 0.

Also as used herein the term compound(s) according to the present invention means any compound(s) according to any aspect of the present invention. If not indicated to the contrary, any embodiment of the present invention

is an embodiment of any aspect of the present invention.

$R^e$ is selected from the group comprising H, alkyl, aryl, alkoxy, aryloxy, alkylamino and arylamino.

In an even more preferred embodiment of the inventive compound $R_1$, $R_2$, $R_3$ $R_4$ and/or $R_5$ have independently from each other one or more groups of the formula $R^f$; whereby $R^f$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, alkanoylamino, aroylamino, alkylthio, arylthio, ureido and amine.

In a further preferred embodiment

the alkylthio group is derivatized, preferably the sulfur atom of the alkylthio group is oxidized to a sulfoxide or sulfone;

the arylthio group is derivatized, preferably the sulfur atom of the arylthio group is oxidized to a sulfoxide or sulfone,

the ureido group is derivatized, preferably the nitrogen atom of the ureido group is independently mono- or di-substituted, more preferably the substitution is selected from the group comprising alkyl, aryl, heterocyclyl, heteroaryl, alkoxycarbonylamino, aryloxycarbonylamino, alkylcarbamoyloxy, arylcarbamoyloxy, alkylsulfonylamino, arylsulfonylamino, alkylaminosulfonyl, and arylaminosulfonyl; and/or

the amino group is derivatized, preferably the nitrogen atom is independently mono- or di-substituted by alkly, aryl, heterocyclyl, heteroalyl, halogen, hydroxy, oxo, carboxy, cyano, nitro, amidino and guanidino.

In a still more preferred embodiment $R^f$ is further substituted by one ore more groups $R^g$, whereby $R^g$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

Particularly preferred compounds according to the present invention are the compounds specified in the following table 1:

| | Structure | Chemical name | Formula | Synthesis methods | MolWeight | MS data |
|---|---|---|---|---|---|---|
| 1 | 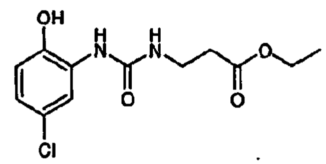 | 3-[3-(5-Chloro-2-hydroxy-phenyl)-ureido]-propionic acid ethyl ester | C12H15ClN2O4 | A | 286.71 | 286.9 |
| 2 | 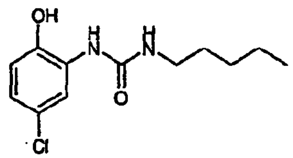 | 1-(5-Chloro-2-hydroxy-phenyl)-3-pentyl-urea | C12H17ClN2O2 | A | 256.73 | 256.9 |
| 3 | 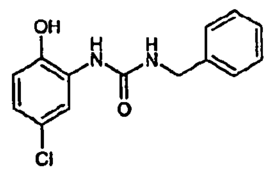 | 1-Benzyl-3-(5-chloro-2-hydroxy-phenyl)-urea | C14H13ClN2O2 | A | 276.72 | 276.9 |
| 4 | 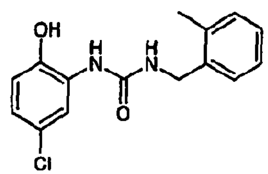 | 1-(5-Chloro-2-hydroxy-phenyl)-3-(2-methyl-benzyl)-urea | C15H15ClN2O2 | A | 290.74 | 290.9 |
| 5 | 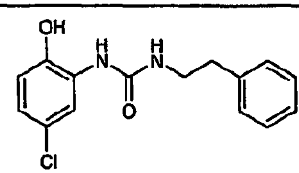 | 1-(5-Chloro-2-hydroxy-phenyl)-3-phenethyl-urea | C15H15ClN2O2 | A | 290.75 | 291.2 |

| | | | | | | |
|---|---|---|---|---|---|---|
| 6 | 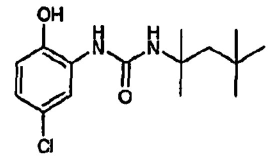 | 1-(5-Chloro-2-hydroxy-phenyl)-3-(1,1,3,3-tetramethyl-butyl)-urea | C15H23ClN2O2 | A | 298.81 | 299.4 |
| 7 | 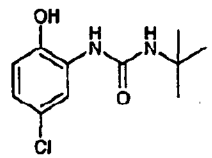 | 1-tert-Butyl-3-(5-chloro-2-hydroxy-phenyl)-urea | C11H15ClN2O2 | A | 242.70 | 243.0 |
| 8 | 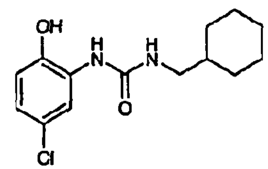 | 1-(5-Chloro-2-hydroxy-phenyl)-3-cyclohexylmethyl-urea | C14H19ClN2O2 | A | 282.77 | 283.1 |
| 9 | 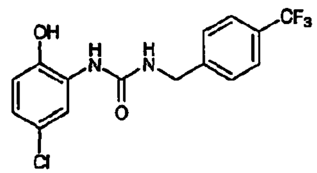 | 1-(5-Chloro-2-hydroxy-phenyl)-3-(4-trifluoromethyl-benzyl)-urea | C15H12ClF3N2O2 | A | 344.72 | 345.0 |
| 10 | 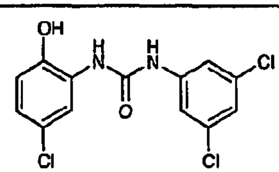 | 1-(5-Chloro-2-hydroxy-phenyl)-3-(3,5-dichloro-phenyl)-urea | C13H9Cl3N2O2 | A | 331.59 | 331.9 |

| | | | | | | |
|---|---|---|---|---|---|---|
| 11 | 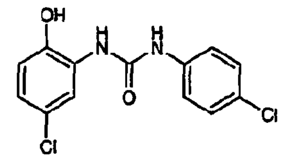 | 1-(5-Chloro-2-hydroxy-phenyl)-3-(4-chloro-phenyl)-urea | C13H10Cl2N2O2 | A | 297.14 | 297.7 |
| 12 | 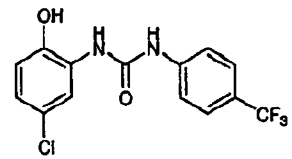 | 1-(5-Chloro-2-hydroxy-phenyl)-3-(4-trifluoromethyl-phenyl)-urea | C14H10ClF3N2O2 | A | 330.69 | 331.0 |
| 13 | 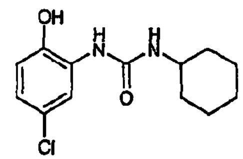 | 1-(5-Chloro-2-hydroxy-phenyl)-3-cyclohexyl-urea | C13H17ClN2O2 | A | 268.74 | 269.1 |
| 14 | 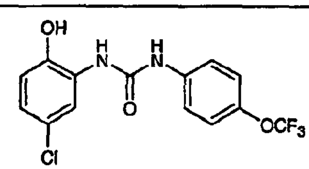 | 1-(5-Chloro-2-hydroxy-phenyl)-3-(4-trifluoromethoxy-phenyl)-urea | C14H10ClF3N2O3 | A | 346.69 | 346.9 |
| 15 | 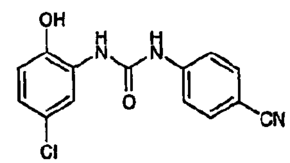 | 1-(5-Chloro-2-hydroxy-phenyl)-3-(4-cyano-phenyl)-urea | C14H10ClN3O2 | A | 287.70 | 287.9 |

| | | | | | | |
|---|---|---|---|---|---|---|
| 16 | 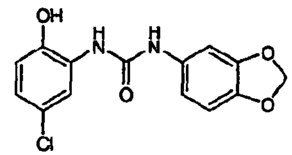 | 1-Benzo[1,3]dioxol-5-yl-3-(5-chloro-2-hydroxy-phenyl)-urea | C14H11ClN2O4 | A | 306.70 | 306.9 |
| 17 | 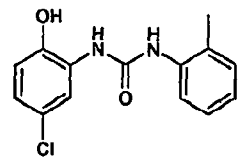 | 1-(5-Chloro-2-hydroxy-phenyl)-3-o-tolyl-urea | C14H13ClN2O2 | A | 276.72 | 276.9 |
| 18 | 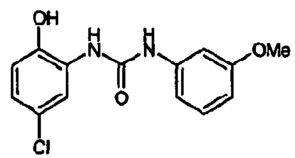 | 1-(5-Chloro-2-hydroxy-phenyl)-3-(3-methoxy-phenyl)-urea | C14H13ClN2O3 | A | 292.72 | 292.9 |
| 19 | 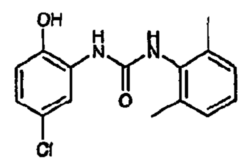 | 1-(5-Chloro-2-hydroxy-phenyl)-3-(2,6-dimethyl-phenyl)-urea | C15H15ClN2O2 | A | 290.75 | 290.9 |
| 20 | 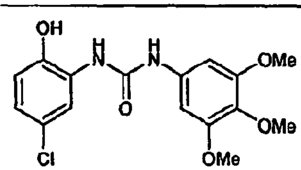 | 1-(5-Chloro-2-hydroxy-phenyl)-3-(3,4,5-trimethoxy-phenyl)-urea | C16H17ClN2O5 | A | 352.77 | 353.2 |

| | | | | | | |
|---|---|---|---|---|---|---|
| 21 | 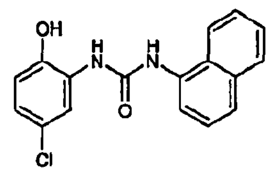 | 1-(5-Chloro-2-hydroxy-phenyl)-3-naphthalen-1-yl-urea | C17H13ClN2O2 | A | 312.75 | 313.1 |
| 22 | 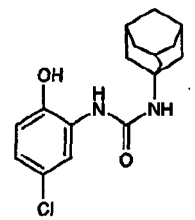 | 1-Adamantan-1-yl-3-(5-chloro-2-hydroxy-phenyl)-urea | C17H21ClN2O2 | A | 320.82 | 321.4 |
| 23 | 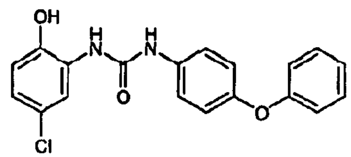 | 1-(5-Chloro-2-hydroxy-phenyl)-3-(4-phenoxy-phenyl)-urea | C19H15ClN2O3 | A | 354.79 | 354.9 |
| 24 | 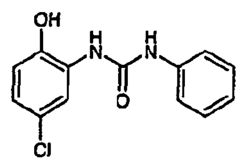 | 1-(5-Chloro-2-hydroxy-phenyl)-3-phenyl-urea | C13H11ClN2O2 | A | 262.70 | 263.2 |
| 25 | 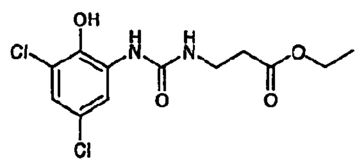 | 3-[3-(3,5-Dichloro-2-hydroxy-phenyl)-ureido]-propionic acid ethyl ester | C12H14Cl2N2O4 | A | 321.16 | 321.9 |

| | | | | | | |
|---|---|---|---|---|---|---|
| 26 | 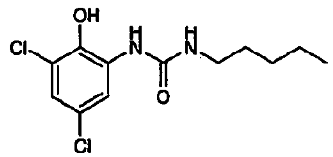 | 1-(3,5-Dichloro-2-hydroxy-phenyl)-3-pentyl-urea | C12H16Cl2N2O2 | A | 291.17 | 292.0 |
| 27 | 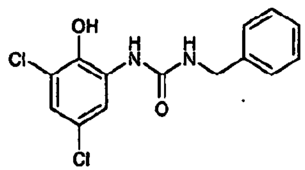 | 1-Benzyl-3-(3,5-dichloro-2-hydroxy-phenyl)-urea | C14H12Cl2N2O2 | A | 311.16 | 312.0 |
| 28 | 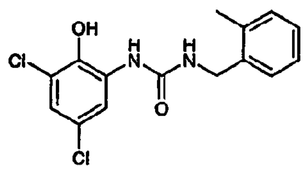 | 1-(3,5-Dichloro-2-hydroxy-phenyl)-3-(2-methyl-benzyl)-urea | C15H14Cl2N2O2 | A | 325.19 | 325.9 |
| 29 | 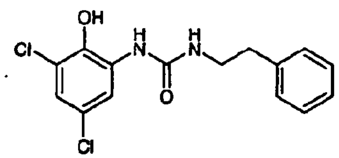 | 1-(3,5-Dichloro-2-hydroxy-phenyl)-3-phenethyl-urea | C15H14Cl2N2O2 | A | 325.19 | 325.9 |
| 30 | 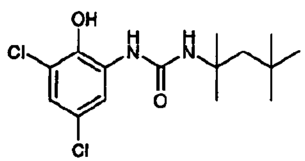 | 1-(3,5-Dichloro-2-hydroxy-phenyl)-3-(1,1,3,3-tetramethyl-butyl)-urea | C15H22Cl2N2O2 | A | 333.25 | 334.0 |

| | | | | | | |
|---|---|---|---|---|---|---|
| 31 | 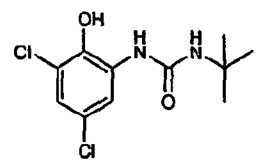 | 1-tert-Butyl-3-(3,5-dichloro-2-hydroxy-phenyl)-urea | C11H14Cl2N2O2 | A | 277.15 | 277.8 |
| 32 | 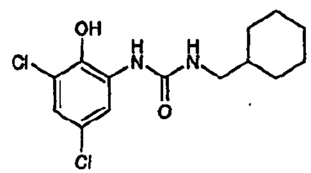 | 1-(3,5-Dichloro-2-hydroxy-phenyl)-3-cyclohexylmethyl-urea | C14H18Cl2N2O2 | A | 317.21 | 318.1 |
| 33 | 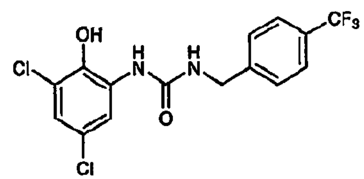 | 1-(3,5-Dichloro-2-hydroxy-phenyl)-3-(4-trifluoromethyl-benzyl)-urea | C15H11Cl2F3N2O2 | A | 379.16 | 379.8 |
| 34 | 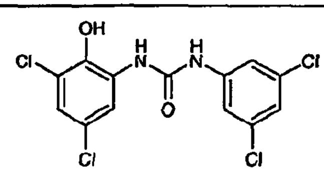 | 1-(3,5-Dichloro-2-hydroxy-phenyl)-3-(3,5-dichloro-phenyl)-urea | C13H8Cl4N2O2 | A | 366.03 | 366.9 |
| 35 | 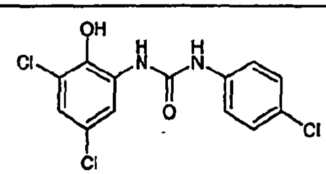 | 1-(3,5-Dichloro-2-hydroxy-phenyl)-3-(4-chloro-phenyl)-urea | C13H9Cl3N2O2 | A | 331.58 | 331.9 |

| | | | | | | |
|---|---|---|---|---|---|---|
| 36 | 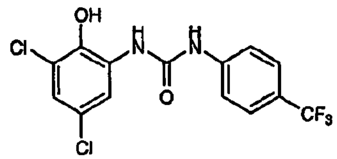 | 1-(3,5-Dichloro-2-hydroxy-phenyl)-3-(4-trifluoromethyl-phenyl)-urea | C14H9Cl2F3N2O2 | A | 365.13 | 365.9 |
| 37 | 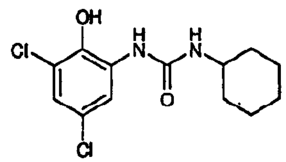 | 1-(3,5-Dichloro-2-hydroxy-phenyl)-3-cyclohexyl-urea | C13H16Cl2N2O2 | A | 303.18 | 304.0 |
| 38 | 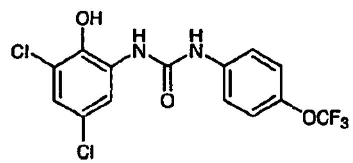 | 1-(3,5-Dichloro-2-hydroxy-phenyl)-3-(4-trifluoromethoxy-phenyl)-urea | C14H9Cl2F3N2O3 | A | 381.13 | 381.8 |
| 39 | 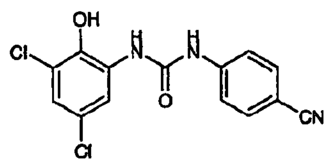 | 1-(3,5-Dichloro-2-hydroxy-phenyl)-3-(4-cyano-phenyl)-urea | C14H9Cl2N3O2 | A | 322.15 | 322.9 |
| 40 | 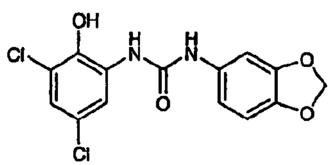 | 1-Benzo[1,3]dioxol-5-yl-3-(3,5-dichloro-2-hydroxy-phenyl)-urea | C14H10Cl2N2O4 | A | 341.15 | 341.7 |

| | | | | | | |
|---|---|---|---|---|---|---|
| 41 | 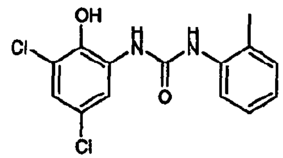 | 1-(3,5-Dichloro-2-hydroxy-phenyl)-3-o-tolyl-urea | C14H12Cl2N2O2 | A | 311.16 | 311.8 |
| 42 | 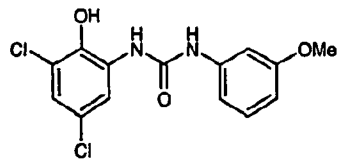 | 1-(3,5-Dichloro-2-hydroxy-phenyl)-3-(3-methoxy-phenyl)-urea | C14H12Cl2N2O3 | A | 327.16 | 327.5 |
| 43 | 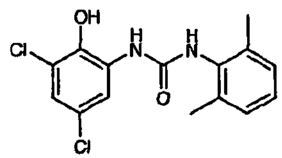 | 1-(3,5-Dichloro-2-hydroxy-phenyl)-3-(2,6-dimethyl-phenyl)-urea | C15H14Cl2N2O2 | A | 325.19 | 325.6 |
| 44 | 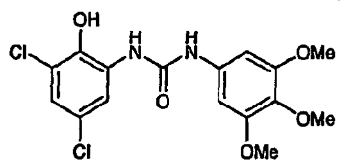 | 1-(3,5-Dichloro-2-hydroxy-phenyl)-3-(3,4,5-trimethoxy-phenyl)-urea | C16H17Cl2N2O5 | A | 387.21 | 387.6 |
| 45 | 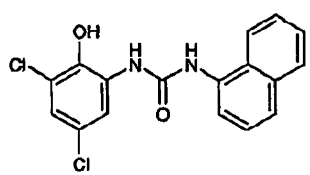 | 1-(3,5-Dichloro-2-hydroxy-phenyl)-3-naphthalen-1-yl-urea | C17H12Cl2N2O2 | A | 347.20 | 347.6 |

| | | | | | | |
|---|---|---|---|---|---|---|
| 46 | 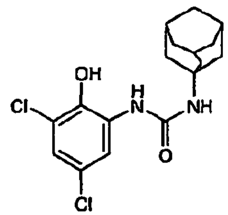 | 1-Adamantan-1-yl-3-(3,5-dichloro-2-hydroxy-phenyl)-urea | C17H20Cl2N2O2 | A | 355.26 | 355.7 |
| 47 | 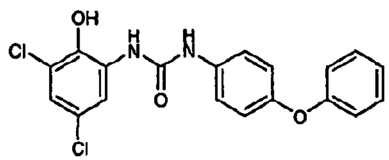 | 1-(3,5-Dichloro-2-hydroxy-phenyl)-3-(4-phenoxy-phenyl)-urea | C19H14Cl2N2O3 | A | 389.23 | 389.6 |
| 48 | 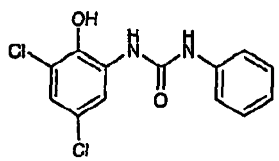 | 1-(3,5-Dichloro-2-hydroxy-phenyl)-3-phenyl-urea | C13H10Cl2N2O2 | A | 297.14 | 297.7 |
| 49 | 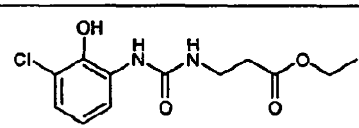 | 3-[3-(3-Chloro-2-hydroxy-phenyl)-ureido]-propionic acid ethyl ester | C12H15ClN2O4 | A | 286.71 | 286.9 |
| 50 | 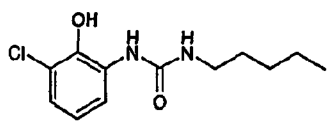 | 1-(3-Chloro-2-hydroxy-phenyl)-3-pentyl-urea | C12H17ClN2O2 | A | 256.73 | 256.9 |
| 51 | 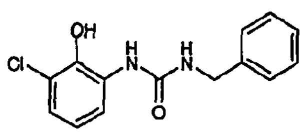 | 1-Benzyl-3-(3-chloro-2-hydroxy-phenyl)-urea | C14H13ClN2O2 | A | 276.72 | 276.9 |

| | | | | | | |
|---|---|---|---|---|---|---|
| 52 | 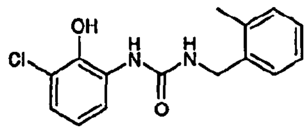 | 1-(3-Chloro-2-hydroxy-phenyl)-3-(2-methyl-benzyl)-urea | C15H15ClN2O2 | A | 290.74 | 290.9 |
| 53 | 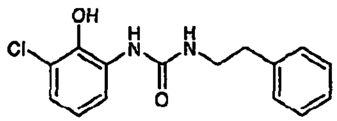 | 1-(3-Chloro-2-hydroxy-phenyl)-3-phenethyl-urea | C15H15ClN2O2 | A | 290.75 | 291.2 |
| 54 | 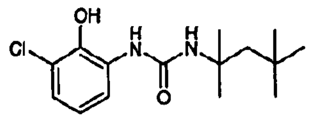 | 1-(3-Chloro-2-hydroxy-phenyl)-3-(1,1,3,3-tetramethyl-butyl)-urea | C15H23ClN2O2 | A | 298.81 | 299.4 |
| 55 | 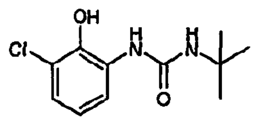 | 1-tert-Butyl-3-(3-chloro-2-hydroxy-phenyl)-urea | C11H15ClN2O2 | A | 242.70 | 243.0 |
| 56 | 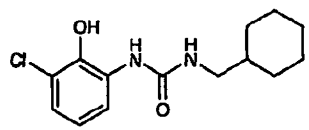 | 1-(3-Chloro-2-hydroxy-phenyl)-3-cyclohexylmethyl-urea | C14H19ClN2O2 | A | 282.77 | 283.1 |
| 57 | 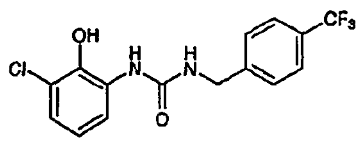 | 1-(3-Chloro-2-hydroxy-phenyl)-3-(4-trifluoromethyl-benzyl)-urea | C15H12ClF3N2O2 | A | 344.72 | 345.0 |
| 58 | 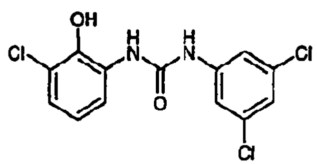 | 1-(3-Chloro-2-hydroxy-phenyl)-3-(3,5-dichloro-phenyl)-urea | C13H9Cl3N2O2 | A | 331.59 | 331.9 |

| | | | | | | |
|---|---|---|---|---|---|---|
| 59 | 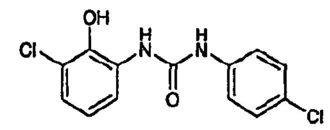 | 1-(3-Chloro-2-hydroxy-phenyl)-3-(4-chloro-phenyl)-urea | C13H10Cl2N2O2 | A | 297.14 | 297.7 |
| 60 | 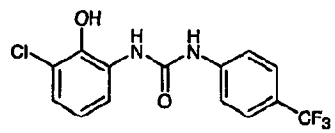 | 1-(3-Chloro-2-hydroxy-phenyl)-3-(4-trifluoromethyl-phenyl)-urea | C14H10ClF3N2O2 | A | 330.69 | 331.0 |
| 61 | 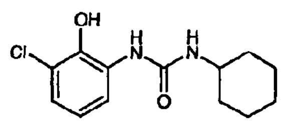 | 1-(3-Chloro-2-hydroxy-phenyl)-3-cyclohexyl-urea | C13H17ClN2O2 | A | 268.74 | 269.1 |
| 62 | 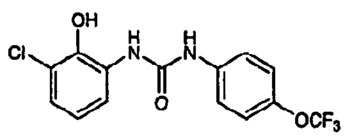 | 1-(3-Chloro-2-hydroxy-phenyl)-3-(4-trifluoromethoxy-phenyl)-urea | C14H10ClF3N2O3 | A | 346.69 | 346.9 |
| 63 | 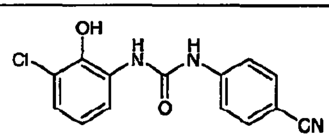 | 1-(3-Chloro-2-hydroxy-phenyl)-3-(4-cyano-phenyl)-urea | C14H10ClN3O2 | A | 287.70 | 287.9 |
| 64 | 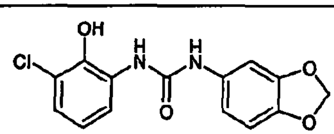 | 1-Benzo[1,3]dioxol-5-yl-3-(3-chloro-2-hydroxy-phenyl)-urea | C14H11ClN2O4 | A | 306.70 | 306.9 |
| 65 | 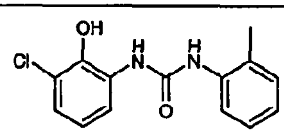 | 1-(3-Chloro-2-hydroxy-phenyl)-3-o-tolyl-urea | C14H13ClN2O2 | A | 276.72 | 276.9 |

| | | | | | | |
|---|---|---|---|---|---|---|
| 66 | 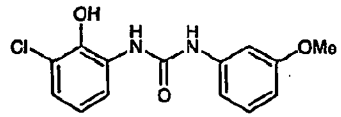 | 1-(3-Chloro-2-hydroxy-phenyl)-3-(3-methoxy-phenyl)-urea | C14H13ClN2O3 | A | 292.72 | 292.9 |
| 67 | 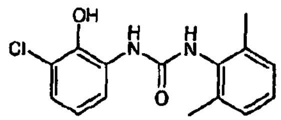 | 1-(3-Chloro-2-hydroxy-phenyl)-3-(2,6-dimethyl-phenyl)-urea | C15H15ClN2O2 | A | 290.75 | 290.9 |
| 68 | 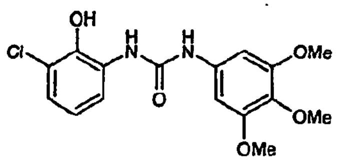 | 1-(3-Chloro-2-hydroxy-phenyl)-3-(3,4,5-trimethoxy-phenyl)-urea | C16H17ClN2O5 | A | 352.77 | 353.2 |
| 69 | 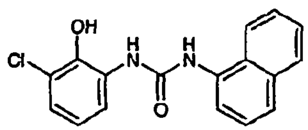 | 1-(3-Chloro-2-hydroxy-phenyl)-3-naphthalen-1-yl-urea | C17H13ClN2O2 | A | 312.75 | 313.1 |
| 70 | 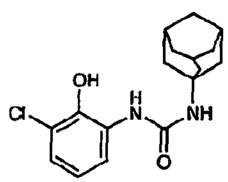 | 1-Adamantan-1-yl-3-(3-chloro-2-hydroxy-phenyl)-urea | C17H21ClN2O2 | A | 320.82 | 321.4 |
| 71 | 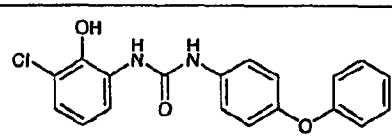 | 1-(3-Chloro-2-hydroxy-phenyl)-3-(4-phenoxy-phenyl)-urea | C19H15ClN2O3 | A | 354.79 | 354.9 |

| | | | | | | |
|---|---|---|---|---|---|---|
| 72 | 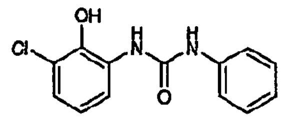 | 1-(3-Chloro-2-hydroxy-phenyl)-3-phenyl-urea | C13H11ClN2O2 | A | 262.70 | 263.2 |
| 73 | 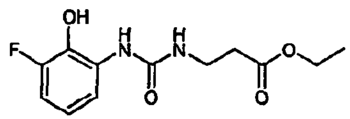 | 3-[3-(3-Fluoro-2-hydroxy-phenyl)-ureido]-propionic acid ethyl ester | C12H15FN2O4 | A | 270.26 | 270.6 |
| 74 | 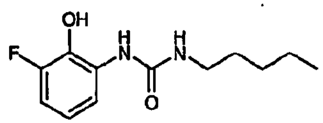 | 1-(3-Fluoro-2-hydroxy-phenyl)-3-pentyl-urea | C12H17FN2O2 | A | 240.27 | 240.5 |
| 75 | 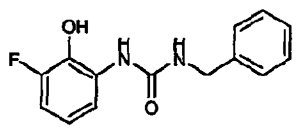 | 1-Benzyl-3-(3-fluoro-2-hydroxy-phenyl)-urea | C14H13FN2O2 | A | 260.26 | 260.8 |
| 76 | 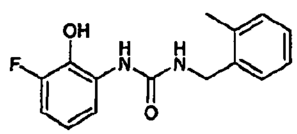 | 1-(3-Fluoro-2-hydroxy-phenyl)-3-(2-methyl-benzyl)-urea | C15H15FN2O2 | A | 274.29 | 274.9 |
| 77 | 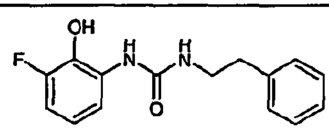 | 1-(3-Fluoro-2-hydroxy-phenyl)-3-phenethyl-urea | C15H15FN2O2 | A | 274.29 | 274.6 |
| 78 | 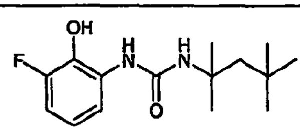 | 1-(3-Fluoro-2-hydroxy-phenyl)-3-(1,1,3,3-tetramethyl-butyl)-urea | C15H23FN2O2 | A | 282.35 | 282.9 |

| | | | | | | |
|---|---|---|---|---|---|---|
| 79 | 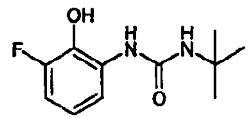 | 1-tert-Butyl-3-(3-fluoro-2-hydroxy-phenyl)-urea | C11H15FN2O2 | A | 226.25 | 226.5 |
| 80 | 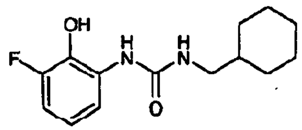 | 1-(3-Fluoro-2-hydroxy-phenyl)-3-cyclohexylmethyl-urea | C14H19FN2O2 | A | 266.31 | 266.7 |
| 81 | 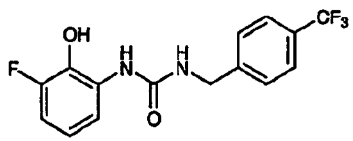 | 1-(3-Fluoro-2-hydroxy-phenyl)-3-(4-trifluoromethyl-benzyl)-urea | C15H12F4N2O2 | A | 328.26 | 328.4 |
| 82 | 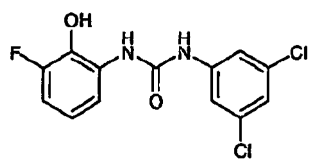 | 1-(3-Fluoro-2-hydroxy-phenyl)-3-(3,5-dichloro-phenyl)-urea | C13H9Cl2FN2O2 | A | 315.13 | 315.5 |
| 83 | 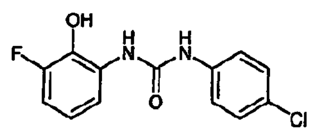 | 1-(3-Fluoro-2-hydroxy-phenyl)-3-(4-chloro-phenyl)-urea | C13H10ClFN2O2 | A | 280.68 | 281.1 |
| 84 | 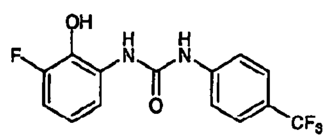 | 1-(3-Fluoro-2-hydroxy-phenyl)-3-(4-trifluoromethyl-phenyl)-urea | C14H10F4N2O2 | A | 314.24 | 314.6 |

| | | | | | | |
|---|---|---|---|---|---|---|
| 85 | 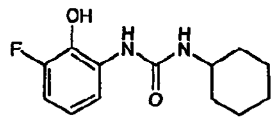 | 1-(3-Fluoro-2-hydroxy-phenyl)-3-cyclohexyl-urea | C13H17FN2O2 | A | 252.28 | 252.6 |
| 86 | 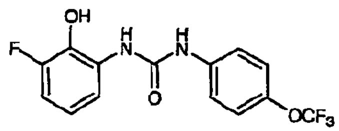 | 1-(3-Fluoro-2-hydroxy-phenyl)-3-(4-trifluoromethoxy-phenyl)-urea | C14H10FF3N2O3 | A | 330.23 | 330.5 |
| 87 | 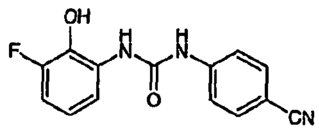 | 1-(3-Fluoro-2-hydroxy-phenyl)-3-(4-cyano-phenyl)-urea | C14H10FFN3O3 | A | 271.25 | 271.4 |
| 88 | 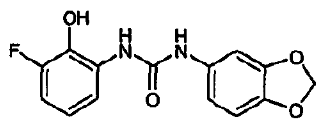 | 1-Benzo[1,3]dioxol-5-yl-3-(3-fluoro-2-hydroxy-phenyl)-urea | C14H11FN2O4 | A | 290.25 | 290.7 |
| 89 | 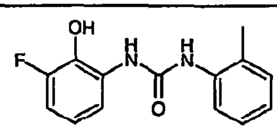 | 1-(3-Fluoro-2-hydroxy-phenyl)-3-o-tolyl-urea | C14H13FN2O2 | A | 260.26 | 260.5 |
| 90 | 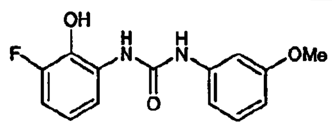 | 1-(3-Fluoro-2-hydroxy-phenyl)-3-(3-methoxy-phenyl)-urea | C14H13FN2O3 | A | 276.26 | 276.5 |
| 91 | 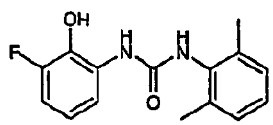 | 1-(3-Fluoro-2-hydroxy-phenyl)-3-(2,6-dimethyl-phenyl)-urea | C15H15FN2O2 | A | 274.29 | 274.7 |

| | | | | | | |
|---|---|---|---|---|---|---|
| 92 | 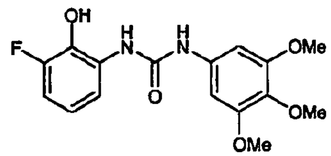 | 1-(3-Fluoro-2-hydroxy-phenyl)-3-(3,4,5-trimethoxy-phenyl)-urea | C16H17FN2O5 | A | 336.31 | 336.6 |
| 93 | 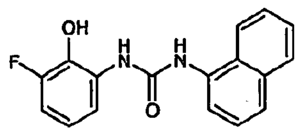 | 1-(3-Fluoro-2-hydroxy-phenyl)-3-naphthalen-1-yl-urea | C17H13FN2O2 | A | 296.3 | 296.6 |
| 94 | 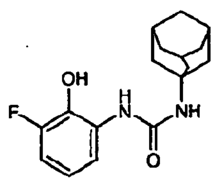 | 1-Adamantan-1-yl-3-(3-fluoro-2-hydroxy-phenyl)-urea | C17H21FN2O2 | A | 304.36 | 304.6 |
| 95 | 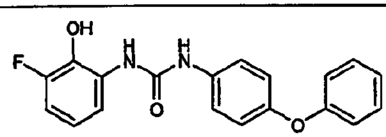 | 1-(3-Fluoro-2-hydroxy-phenyl)-3-(4-phenoxy-phenyl)-urea | C19H15FN2O3 | A | 338.33 | 338.6 |
| 96 | 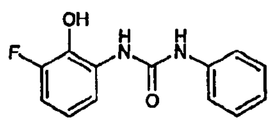 | 1-(3-Fluoro-2-hydroxy-phenyl)-3-phenyl-urea | C13H11FN2O2 | A | 246.24 | 246.5 |
| 97 | 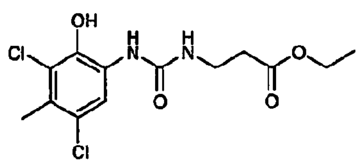 | 3-[3-(3,5-Dichloro-2-hydroxy-4-methyl-phenyl)-ureido]-propionic acid ethyl ester | C13H16Cl2N2O4 | A | 335.19 | 335.9 |

| | | | | | | |
|---|---|---|---|---|---|---|
| 98 | 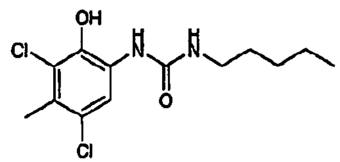 | 1-(3,5-Dichloro-2-hydroxy-4-methyl-phenyl)-3-pentyl-urea | C13H18Cl2N2O2 | A | 305.20 | 305.9 |
| 99 | 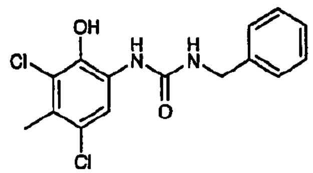 | 1-Benzyl-3-(3,5-dichloro-2-hydroxy-4-methyl-phenyl)-urea | C15H14Cl2N2O2 | A | 325.19 | 325.5 |
| 100 | 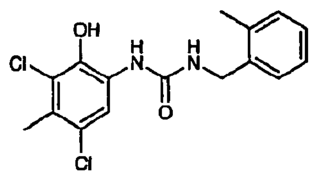 | 1-(3,5-Dichloro-2-hydroxy-4-methyl-phenyl)-3-(2-methyl-benzyl)-urea | C16H16Cl2N2O2 | A | 339.22 | 339.9 |
| 101 | 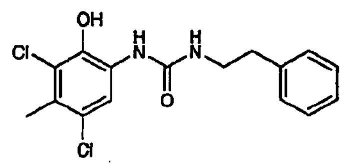 | 1-(3,5-Dichloro-2-hydroxy-4-methyl-phenyl)-3-phenethyl-urea | C16H16Cl2N2O2 | A | 339.22 | 339.4 |
| 102 | 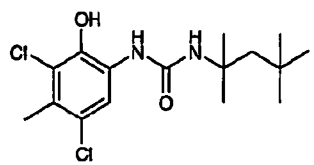 | 1-(3,5-Dichloro-2-hydroxy-4-methyl-phenyl)-3-(1,1,3,3-tetramethyl-butyl)-urea | C16H24Cl2N2O2 | A | 347.28 | 347.4 |

| | | | | | | |
|---|---|---|---|---|---|---|
| 103 | 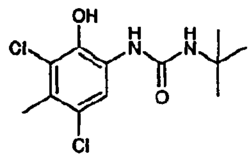 | 1-tert-Butyl-3-(3,5-dichloro-2-hydroxy-4-methyl-phenyl)-urea | C12H16Cl2N2O2 | A | 291.18 | 291.2 |
| 104 | 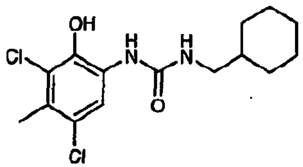 | 1-Cyclohexylmethyl-3-(3,5-dichloro-2-hydroxy-4-methyl-phenyl)-urea | C15H20Cl2N2O2 | A | 331.24 | 332.1 |
| 105 | 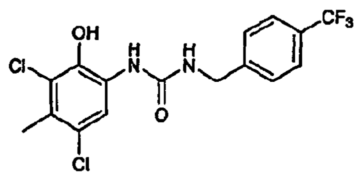 | 1-(3,5-Dichloro-2-hydroxy-4-methyl-phenyl)-3-(4-trifluoromethyl-benzyl)-urea | C16H13Cl2F3N2O2 | A | 393.19 | 393.8 |
| 106 | 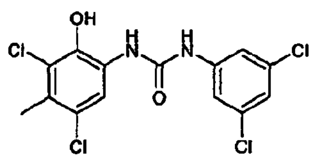 | 1-(3,5-Dichloro-2-hydroxy-4-methyl-phenyl)-3-(3,5-dichloro-phenyl)-urea | C14H10Cl4N2O2 | A | 380.06 | 380.2 |
| 107 | 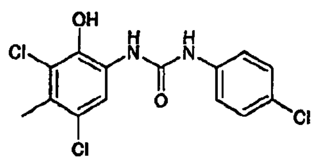 | 1-(4-Chloro-phenyl)-3-(3,5-dichloro-2-hydroxy-4-methyl-phenyl)-urea | C14H11Cl3N2O2 | A | 345.61 | 345.9 |

| | | | | | | |
|---|---|---|---|---|---|---|
| 108 | 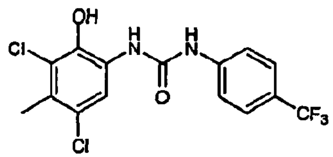 | 1-(3,5-Dichloro-2-hydroxy-4-methyl-phenyl)-3-(4-trifluoromethyl-phenyl)-urea | C15H11Cl2F3N2O2 | A | 379.16 | 379.9 |
| 109 | 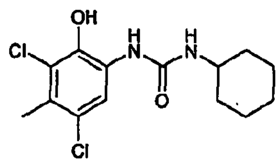 | 1-Cyclohexyl-3-(3,5-dichloro-2-hydroxy-4-methyl-phenyl)-urea | C14H18Cl2N2O2 | A | 317.21 | 317.8 |
| 110 | 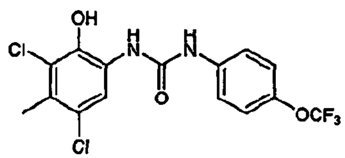 | 1-(3,5-Dichloro-2-hydroxy-4-methyl-phenyl)-3-(4-trifluoromethoxy-phenyl)-urea | C15H11Cl2F3N2O3 | A | 395.16 | 395.4 |
| 111 | 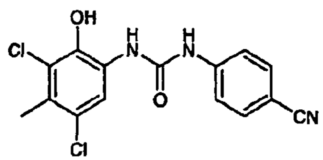 | 1-(4-Cyano-phenyl)-3-(3,5-dichloro-2-hydroxy-4-methyl-phenyl)-urea | C15H11Cl2N3O2 | A | 336.18 | 336.4 |
| 112 | 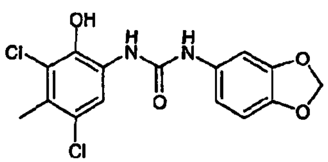 | 1-Benzo[1,3]dioxol-5-yl-3-(3,5-dichloro-2-hydroxy-4-methyl-phenyl)-urea | C15H12Cl2N2O4 | A | 355.18 | 355.2 |

| | | | | | | |
|---|---|---|---|---|---|---|
| 113 | 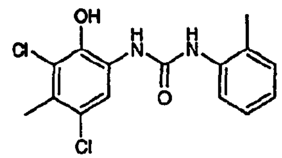 | 1-(3,5-Dichloro-2-hydroxy-4-methyl-phenyl)-3-o-tolyl-urea | C15H14Cl2N2O2 | A | 325.19 | 325.5 |
| 114 | 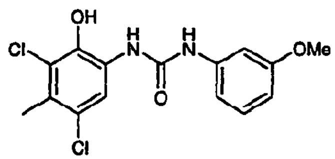 | 1-(3,5-Dichloro-2-hydroxy-4-methyl-phenyl)-3-(3-methoxy-phenyl)-urea | C15H14Cl2N2O3 | A | 341.19 | 341.4 |
| 115 | 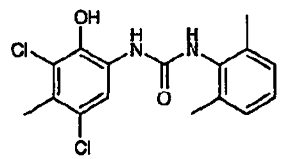 | 1-(3,5-Dichloro-2-hydroxy-4-methyl-phenyl)-3-(2,6-dimethyl-phenyl)-urea | C16H16Cl2N2O2 | A | 339.22 | 339.4 |
| 116 | 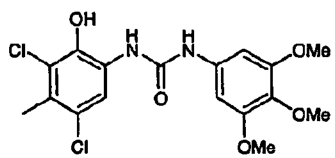 | 1-(3,5-Dichloro-2-hydroxy-4-methyl-phenyl)-3-(3,4,5-trimethoxy-phenyl)-urea | C17H18Cl2N2O5 | A | 401.24 | 401.3 |
| 117 | 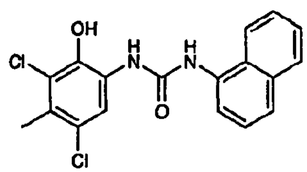 | 1-(3,5-Dichloro-2-hydroxy-4-methyl-phenyl)-3-naphthalen-1-yl-urea | C18H14Cl2N2O2 | A | 361.23 | 361.3 |

| | | | | | | |
|---|---|---|---|---|---|---|
| 118 | 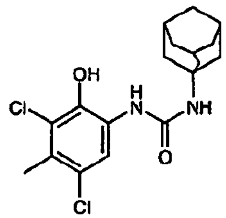 | 1-Adamantan-1-yl-3-(3,5-dichloro-2-hydroxy-4-methyl-phenyl)-urea | C18H22Cl2N2O2 | A | 369.29 | 369.3 |
| 119 | 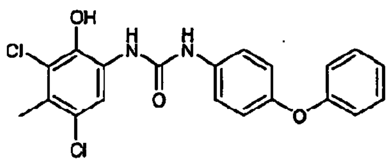 | 1-(3,5-Dichloro-2-hydroxy-4-methyl-phenyl)-3-(4-phenoxy-phenyl)-urea | C20H16Cl2N2O3 | A | 403.26 | 403.3 |
| 120 | 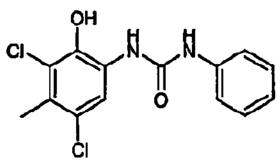 | 1-(3,5-Dichloro-2-hydroxy-4-methyl-phenyl)-3-phenyl-urea | C14H12Cl2N2O2 | A | 311.17 | 311.3 |
| 121 | 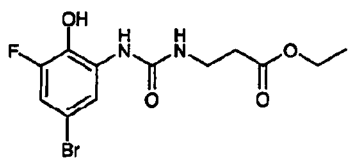 | 3-[3-(5-Bromo-3-fluoro-2-hydroxy-phenyl)-ureido]-propionic acid ethyl ester | C12H14BrFN2O4 | A | 349.15 | 349.9 |
| 122 | 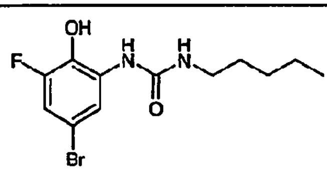 | 1-(5-Bromo-3-fluoro-2-hydroxy-phenyl)-3-pentyl-urea | C12H16BrFN2O2 | A | 319.17 | 320.0 |

| | | | | | | |
|---|---|---|---|---|---|---|
| 123 | 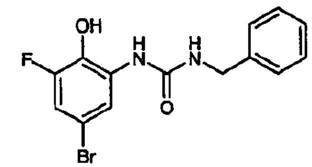 | 1-Benzyl-3-(5-bromo-3-fluoro-2-hydroxy-phenyl)-urea | C14H12BrFN2O2 | A | 339.16 | 340.0 |
| 124 | 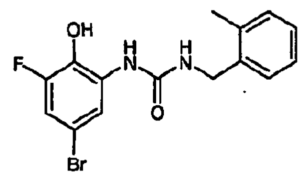 | 1-(5-Bromo-3-fluoro-2-hydroxy-phenyl)-3-(2-methyl-benzyl)-urea | C15H14BrFN2O2 | A | 353.19 | 353.9 |
| 125 | 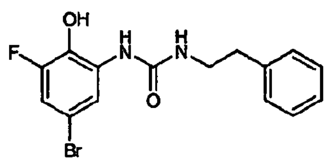 | 1-(5-Bromo-3-fluoro-2-hydroxy-phenyl)-3-phenethyl-urea | C15H14BrFN2O2 | A | 353.19 | 353.9 |
| 126 | 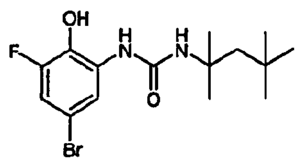 | 1-(5-Bromo-3-fluoro-2-hydroxy-phenyl)-3-(1,1,3,3-tetramethyl-butyl)-urea | C15H22BrFN2O2 | A | 361.25 | 362.0 |
| 127 | 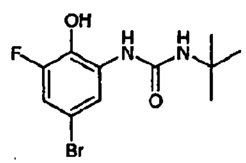 | 1-tert-Butyl-3-(5-bromo-3-fluoro-2-hydroxy-phenyl)-urea | C11H14BrFN2O2 | A | 305.14 | 305.8 |

| | | | | | | |
|---|---|---|---|---|---|---|
| 128 | 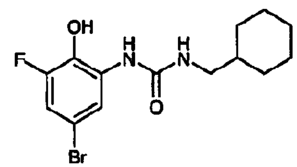 | 1-(5-Bromo-3-fluoro-2-hydroxy-phenyl)-3-cyclohexylmethyl-urea | C14H18BrFN2O2 | A | 345.21 | 346.1 |
| 129 | 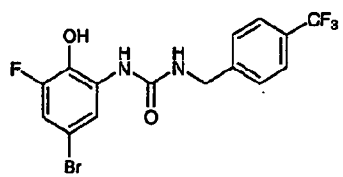 | 1-(5-Bromo-3-fluoro-2-hydroxy-phenyl)-3-(4-trifluoromethyl-benzyl)-urea | C15H11BrF4N2O2 | A | 407.16 | 407.8 |
| 130 | 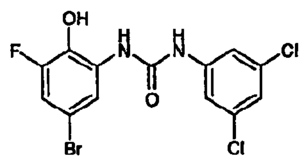 | 1-(5-Bromo-3-fluoro-2-hydroxy-phenyl)-3-(3,5-dichloro-phenyl)-urea | C13H8BrCl2FN2O2 | A | 394.02 | 394.9 |
| 131 | 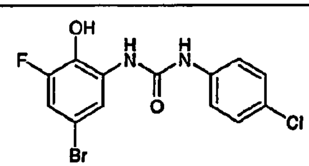 | 1-(5-Bromo-3-fluoro-2-hydroxy-phenyl)-3-(4-chloro-phenyl)-urea | C13H9BrClFN2O2 | A | 359.58 | 359.9 |
| 132 | 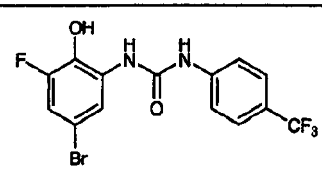 | 1-(5-Bromo-3-fluoro-2-hydroxy-phenyl)-3-(4-trifluoromethyl-phenyl)-urea | C14H9BrF4N2O2 | A | 393.13 | 393.9 |

| | | | | | | |
|---|---|---|---|---|---|---|
| 133 | 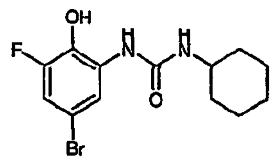 | 1-(5-Bromo-3-fluoro-2-hydroxy-phenyl)-3-cyclohexyl-urea | $C_{13}H_{16}BrFN_2O_2$ | A | 331.18 | 332.0 |
| 134 | 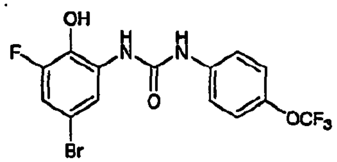 | 1-(5-Bromo-3-fluoro-2-hydroxy-phenyl)-3-(4-trifluoromethoxy-phenyl)-urea | $C_{14}H_9BrF_4N_2O_3$ | A | 409.13 | 409.8 |
| 135 | 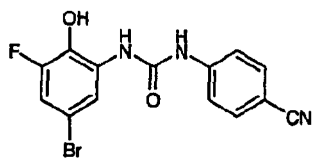 | 1-(5-Bromo-3-fluoro-2-hydroxy-phenyl)-3-(4-cyano-phenyl)-urea | $C_{14}H_9BrFN_3O_2$ | A | 350.14 | 350.9 |
| 136 | 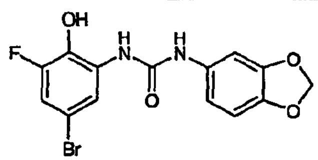 | 1-Benzo[1,3]dioxol-5-yl-3-(5-bromo-3-fluoro-2-hydroxy-phenyl)-urea | $C_{14}H_{10}BrFN_2O_4$ | A | 369.14 | 369.7 |
| 137 | 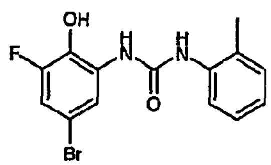 | 1-(5-Bromo-3-fluoro-2-hydroxy-phenyl)-3-o-tolyl-urea | $C_{14}H_{12}BrFN_2O_2$ | A | 339.16 | 339.8 |

| | | | | | | |
|---|---|---|---|---|---|---|
| 138 | 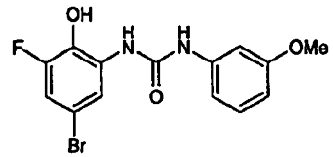 | 1-(5-Bromo-3-fluoro-2-hydroxy-phenyl)-3-(3-methoxy-phenyl)-urea | C14H12BrFN2O3 | A | 355.16 | 355.5 |
| 139 | 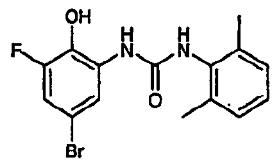 | 1-(5-Bromo-3-fluoro-2-hydroxy-phenyl)-3-(2,6-dimethyl-phenyl)-urea | C15H14BrFN2O2 | A | 353.19 | 353.6 |
| 140 | 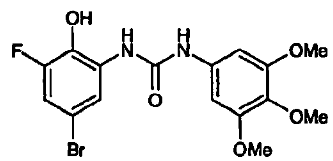 | 1-(5-Bromo-3-fluoro-2-hydroxy-phenyl)-3-(3,4,5-trimethoxy-phenyl)-urea | C16H17BrFN2O5 | A | 415.21 | 415.6 |
| 141 | 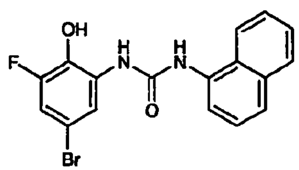 | 1-(5-Bromo-3-fluoro-2-hydroxy-phenyl)-3-naphthalen-1-yl-urea | C17H12BrFN2O2 | A | 375.19 | 375.6 |
| 142 | 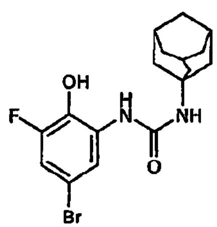 | 1-Adamantan-1-yl-3-(5-Bromo-3-fluoro-2-hydroxy-phenyl)-urea | C17H20BrFN2O2 | A | 383.26 | 383.7 |

| | | | | | | |
|---|---|---|---|---|---|---|
| 143 | 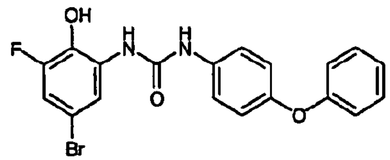 | 1-(5-Bromo-3-fluoro-2-hydroxy-phenyl)-3-(4-phenoxy-phenyl)-urea | C19H14BrFN2O3 | A | 417.23 | 417.6 |
| 144 | 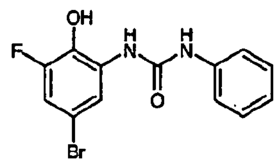 | 1-(5-Bromo-3-fluoro-2-hydroxy-phenyl)-3-phenyl-urea | C13H10BrFN2O2 | A | 325.13 | 325.7 |
| 145 | 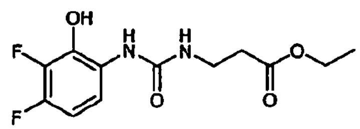 | 3-[3-(3,5-Difluoro-2-hydroxy-phenyl)-ureido]-propionic acid ethyl ester | C12H14F2N2O4 | A | 288.25 | 288.9 |
| 146 | 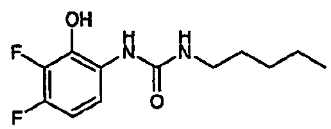 | 1-(3,5-Difluoro-2-hydroxy-phenyl)-3-pentyl-urea | C12H16F2N2O2 | A | 258.26 | 258.9 |
| 147 | 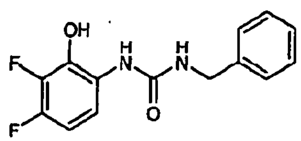 | 1-Benzyl-3-(3,5-difluoro-2-hydroxy-phenyl)-urea | C14H12F2N2O2 | A | 278.25 | 278.8 |
| 148 | 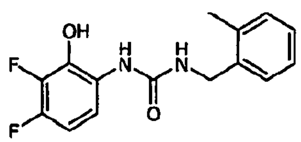 | 1-(3,5-Difluoro-2-hydroxy-phenyl)-3-(2-methyl-benzyl)-urea | C15H14F2N2O2 | A | 292.28 | 292.9 |

| | | | | | | |
|---|---|---|---|---|---|---|
| 149 | 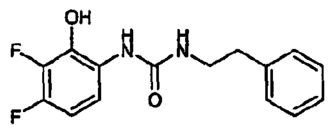 | 1-(3,5-Difluoro-2-hydroxy-phenyl)-3-phenethyl-urea | C15H14F2N2O2 | A | 292.28 | 292.9 |
| 150 | 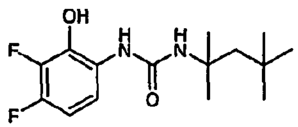 | 1-(3,5-Difluoro-2-hydroxy-phenyl)-3-(1,1,3,3-tetramethyl-butyl)-urea | C15H22F2N2O2 | A | 300.34 | 300.5 |
| 151 | 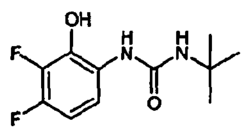 | 1-tert-Butyl-3-(3,5-difluoro-2-hydroxy-phenyl)-urea | C11H14F2N2O2 | A | 244.24 | 244.3 |
| 152 | 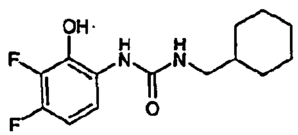 | 1-(3,5-Difluoro-2-hydroxy-phenyl)-3-cyclohexylmethyl-urea | C14H18F2N2O2 | A | 284.30 | 284.6 |
| 153 | 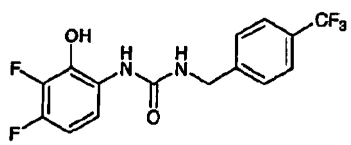 | 1-(3,5-Difluoro-2-hydroxy-phenyl)-3-(4-trifluoromethyl-benzyl)-urea | C15H11F5N2O2 | A | 346.25 | 346.5 |
| 154 | 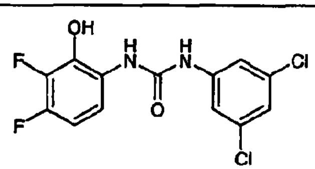 | 1-(3,5-Difluoro-2-hydroxy-phenyl)-3-(3,5-dichloro-phenyl)-urea | C13H8Cl2F2N2O2 | A | 333.12 | 333.3 |

| | | | | | | |
|---|---|---|---|---|---|---|
| 155 | 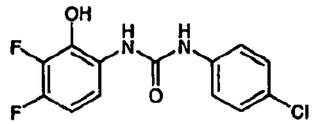 | 1-(3,5-Difluoro-2-hydroxy-phenyl)-3-(4-chloro-phenyl)-urea | C13H9ClF2N2O2 | A | 298.67 | 298.9 |
| 156 | 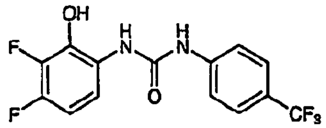 | 1-(3,5-Difluoro-2-hydroxy-phenyl)-3-(4-trifluoromethyl-phenyl)-urea | C14H9F5N2O2 | A | 332.23 | 332.7 |
| 157 | 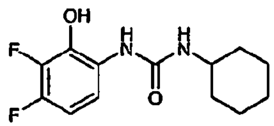 | 1-(3,5-Difluoro-2-hydroxy-phenyl)-3-cyclohexyl-urea | C13H16F2N2O2 | A | 270.28 | 270.7 |
| 158 | 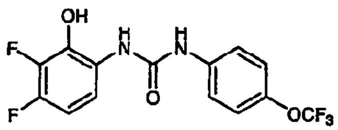 | 1-(3,5-Difluoro-2-hydroxy-phenyl)-3-(4-trifluoromethoxy-phenyl)-urea | C14H9F2F3N2O3 | A | 348.22 | 348.6 |
| 159 | 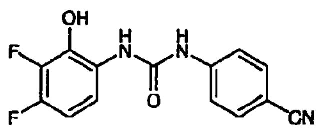 | 1-(3,5-Difluoro-2-hydroxy-phenyl)-3-(4-cyano-phenyl)-urea | C14H9F2N3O2 | A | 289.24 | 289.5 |
| 160 | 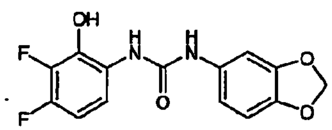 | 1-Benzo[1,3]dioxol-5-yl-3-(3,5-difluoro-2-hydroxy-phenyl)-urea | C14H10F2N2O4 | A | 308.24 | 308.5 |
| 161 | 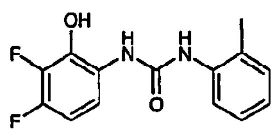 | 1-(3,5-Difluoro-2-hydroxy-phenyl)-3-o-tolyl-urea | C14H12F2N2O2 | A | 278.25 | 278.6 |

| | | | | | | |
|---|---|---|---|---|---|---|
| 162 | 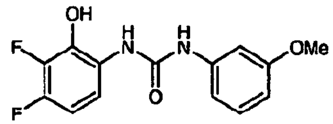 | 1-(3,5-Difluoro-2-hydroxy-phenyl)-3-(3-methoxy-phenyl)-urea | C14H12F2N2O3 | A | 294.25 | 294.5 |
| 163 | 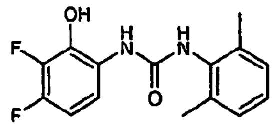 | 1-(3,5-Difluoro-2-hydroxy-phenyl)-3-(2,6-dimethyl-phenyl)-urea | C15H14F2N2O2 | A | 292.28 | 292.6 |
| 164 | 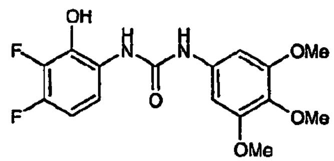 | 1-(3,5-Difluoro-2-hydroxy-phenyl)-3-(3,4,5-trimethoxy-phenyl)-urea | C16H17F2N2O5 | A | 354.31 | 354.6 |
| 165 | 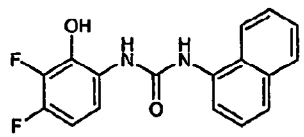 | 1-(3,5-Difluoro-2-hydroxy-phenyl)-3-naphthalen-1-yl-urea | C17H12F2N2O2 | A | 314.29 | 314.6 |
| 166 | 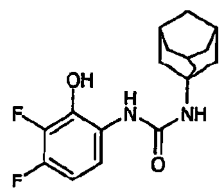 | 1-Adamantan-1-yl-3-(3,5-difluoro-2-hydroxy-phenyl)-urea | C17H20F2N2O2 | A | 322.35 | 322.7 |
| 167 | 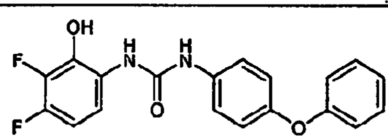 | 1-(3,5-Difluoro-2-hydroxy-phenyl)-3-(4-phenoxy-phenyl)-urea | C19H14F2N2O3 | A | 356.32 | 356.6 |

| | | | | | | |
|---|---|---|---|---|---|---|
| 168 | 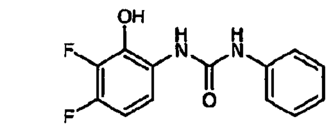 | 1-(3,5-Difluoro-2-hydroxy-phenyl)-3-phenyl-urea | C13H10F2N2O2 | A | 264.23 | 264.7 |
| 169 | 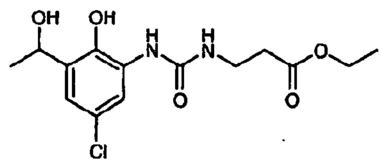 | 3-{3-[5-Chloro-2-hydroxy-3-(1-hydroxy-ethyl)-phenyl]-ureido}-propionic acid ethyl ester | C14H19ClN2O5 | A | 330.76 | 331.1 |
| 170 | 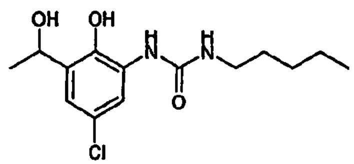 | 1-[5-Chloro-2-hydroxy-3-(1-hydroxy-ethyl)-phenyl]-3-pentyl-urea | C14H21ClN2O3 | A | 300.78 | 301.2 |
| 171 | 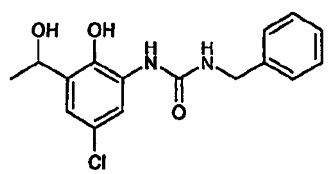 | 1-[5-Chloro-2-hydroxy-3-(1-hydroxy-ethyl)-phenyl]-3-pentyl-urea | C16H17ClN2O3 | A | 320.77 | 321.0 |
| 172 | 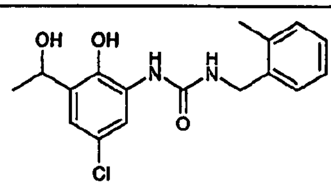 | 1-[5-Chloro-2-hydroxy-3-(1-hydroxy-ethyl)-phenyl]-3-(2-methyl-benzyl)-urea | C17H19ClN2O3 | A | 334.80 | 335.1 |

| | | | | | | |
|---|---|---|---|---|---|---|
| 173 | 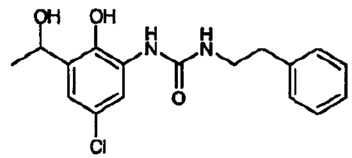 | 1-[5-Chloro-2-hydroxy-3-(1-hydroxy-ethyl)-phenyl]-3-phenethyl-urea | C17H19ClN2O3 | A | 334.80 | 335.3 |
| 174 | 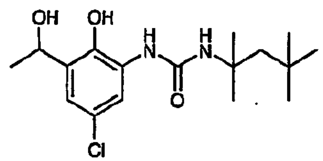 | 1-[5-Chloro-2-hydroxy-3-(1-hydroxy-ethyl)-phenyl]-3-(1,1,3,3-tetramethyl-butyl)-urea | C17H27Cl2N2O2 | A | 342.86 | 343.7 |
| 175 | 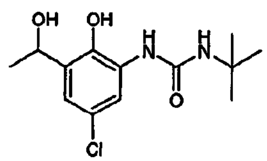 | 1-tert-Butyl-3-[5-chloro-2-hydroxy-3-(1-hydroxy-ethyl)-phenyl]-urea | C13H19ClN2O3 | A | 286.75 | 287.6 |
| 176 | 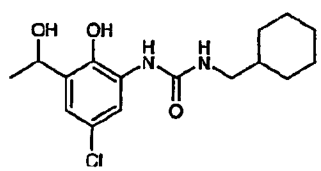 | 1-[5-Chloro-2-hydroxy-3-(1-hydroxy-ethyl)-phenyl]-3-cyclohexylmethyl-urea | C16H23ClN2O3 | A | 326.82 | 327.5 |
| 177 | 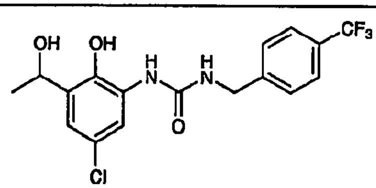 | 1-[5-Chloro-2-hydroxy-3-(1-hydroxy-ethyl)-phenyl]-3-(4-trifluoromethyl-benzyl)-urea | C17H16ClF3N2O3 | A | 388.77 | 389.4 |

| | | | | | | |
|---|---|---|---|---|---|---|
| 178 | 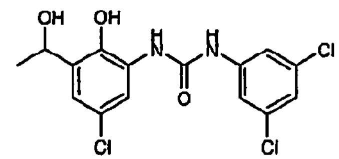 | 1-[5-Chloro-2-hydroxy-3-(1-hydroxy-ethyl)-phenyl]-3-(4-chloro-phenyl)-urea | C15H13Cl3N2O3 | A | 375.63 | 376.2 |
| 179 | 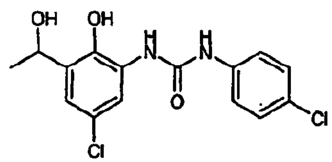 | 1-[5-Chloro-2-hydroxy-3-(1-hydroxy-ethyl)-phenyl]-3-(4-chloro-phenyl)-urea | C15H14Cl2N2O3 | A | 341.19 | 341.6 |
| 180 | 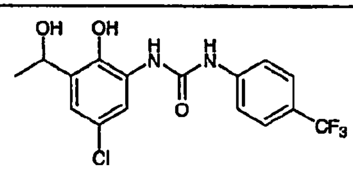 | 1-[5-Chloro-2-hydroxy-3-(1-hydroxy-ethyl)-phenyl]-3-(4-trifluoromethyl-phenyl)-urea | C16H14ClF3N2O3 | A | 374.74 | 375.0 |
| 181 | 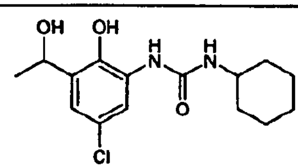 | 1-[5-Chloro-2-hydroxy-3-(1-hydroxy-ethyl)-phenyl]-3-cyclohexyl-urea | C15H21ClN2O3 | A | 312.79 | 313.0 |
| 182 | 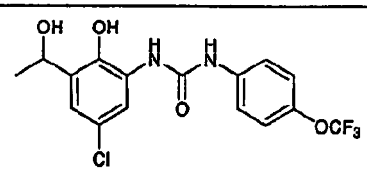 | 1-[5-Chloro-2-hydroxy-3-(1-hydroxy-ethyl)-phenyl]-3-(4-trifluoromethoxy-phenyl)-urea | C16H14ClF3N2O4 | A | 390.74 | 391.2 |

| | | | | | | |
|---|---|---|---|---|---|---|
| 183 | 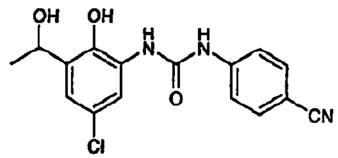 | 1-[5-Chloro-2-hydroxy-3-(1-hydroxy-ethyl)-phenyl]-3-(4-cyano-phenyl)-urea | C16H14ClN3O3 | A | 331.75 | 332.0 |
| 184 | 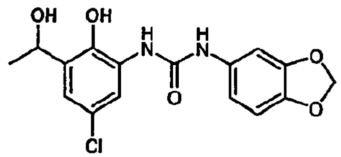 | 1-Benzo[1,3]dioxol-5-yl-3-[5-chloro-2-hydroxy-3-(1-hydroxy-ethyl)-phenyl]-urea | C16H15ClN2O5 | A | 350.75 | 351.1 |
| 185 | 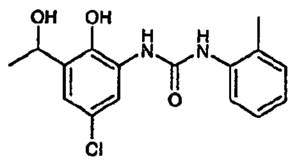 | 1-[5-Chloro-2-hydroxy-3-(1-hydroxy-ethyl)-phenyl]-3-o-tolyl-urea | C16H17ClN2O3 | A | 320.77 | 321.0 |
| 186 | 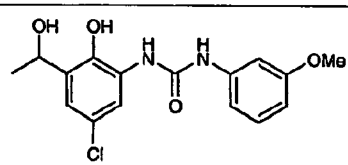 | 1-[5-Chloro-2-hydroxy-3-(1-hydroxy-ethyl)-phenyl]-3-(3-methoxy-phenyl)-urea | C16H17ClN2O4 | A | 336.77 | 337.1 |
| 187 | 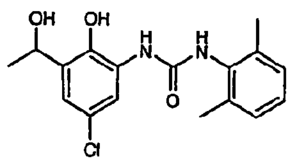 | 1-[5-Chloro-2-hydroxy-3-(1-hydroxy-ethyl)-phenyl]-3-(2,6-dimethyl-phenyl)-urea | C17H19ClN2O3 | A | 334.80 | 335.2 |

| | | | | | | |
|---|---|---|---|---|---|---|
| 188 | 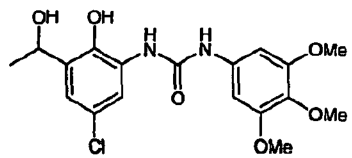 | 1-[5-Chloro-2-hydroxy-3-(1-hydroxy-ethyl)-phenyl]-3-(3,4,5-trimethoxy-phenyl)-urea | C18H21ClN2O6 | A | 396.82 | 397.2 |
| 189 | 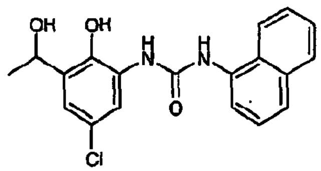 | 1-[5-Chloro-2-hydroxy-3-(1-hydroxy-ethyl)-phenyl]-3-naphthalen-1-yl-urea | C19H17ClN2O3 | A | 356.80 | 357.1 |
| 190 | 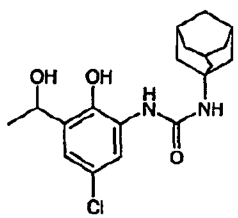 | 1-Adamantan-1-yl-3-[5-chloro-2-hydroxy-3-(1-hydroxy-ethyl)-phenyl]-urea | C19H25ClN2O3 | A | 364.87 | 365.1 |
| 191 | 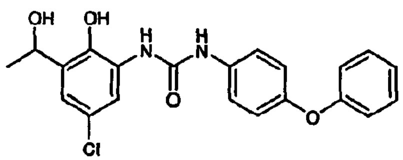 | 1-[5-Chloro-2-hydroxy-3-(1-hydroxy-ethyl)-phenyl]-3-(4-phenoxy-phenyl)-urea | C21H19ClN2O4 | A | 398.84 | 399.0 |
| 192 | 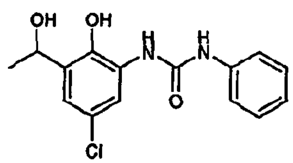 | 1-[5-Chloro-2-hydroxy-3-(1-hydroxy-ethyl)-phenyl]-3-phenyl-urea | C15H15ClN2O3 | A | 306.74 | 307.0 |

| | | | | | | |
|---|---|---|---|---|---|---|
| 193 | 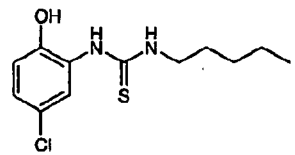 | 1-(5-Chloro-2-hydroxy-phenyl)-3-pentyl-thiourea | C12H17ClN2OS | I | 272.79 | 272.9 |
| 194 | 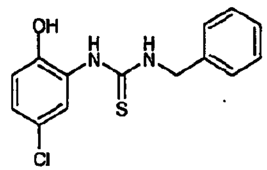 | 1-Benzyl-3-(5-chloro-2-hydroxy-phenyl)-thiourea | C14H13ClN2OS | I | 292.78 | 293.1 |
| 195 | 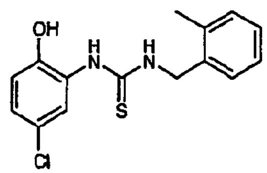 | 1-(5-Chloro-2-hydroxy-phenyl)-3-(2-methyl-benzyl)-thiourea | C15H15ClN2OS | I | 306.81 | 306.9 |
| 196 | 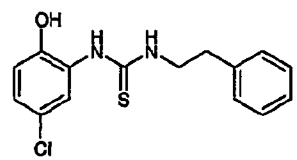 | 1-(5-Chloro-2-hydroxy-phenyl)-3-phenethyl-thiourea | C15H15ClN2OS | I | 306.81 | 307.1 |
| 197 | 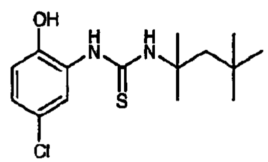 | 1-(5-Chloro-2-hydroxy-phenyl)-3-(1,1,3,3-tetramethyl-butyl)-thiourea | C15H23ClN2OS | I | 314.87 | 315.0 |

| | | | | | | |
|---|---|---|---|---|---|---|
| 198 | 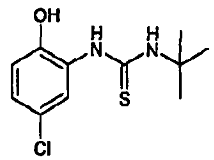 | 1-tert-Butyl-3-(5-chloro-2-hydroxy-phenyl)-thiourea | C11H15ClN2OS | I | 258.77 | 258.9 |
| 199 | 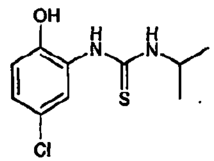 | 1-(5-Chloro-2-hydroxy-phenyl)-3-isopropyl-thiourea | C10H13ClN2OS | I | 244.74 | 245.0 |
| 200 | 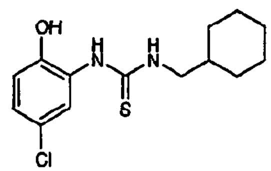 | 1-(5-Chloro-2-hydroxy-phenyl)-3-cyclohexylmethyl-thiourea | C14H19ClN2OS | I | 298.83 | 299.1 |
| 201 | 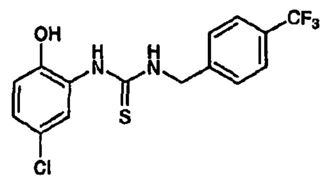 | 1-(5-Chloro-2-hydroxy-phenyl)-3-(4-trifluoromethyl-benzyl)-thiourea | C15H12ClF3N2OS | I | 360.78 | 361.1 |
| 202 | 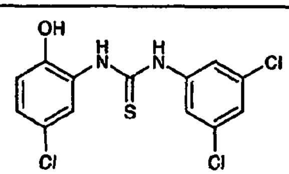 | 1-(5-Chloro-2-hydroxy-phenyl)-3-(3,5-dichloro-phenyl)-thiourea | C13H9Cl3N2OS | I | 347.65 | 347.9 |

| | | | | | | |
|---|---|---|---|---|---|---|
| 203 | 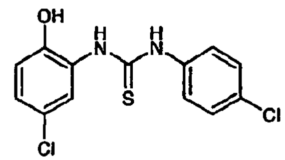 | 1-(5-Chloro-2-hydroxy-phenyl)-3-(4-chloro-phenyl)-thiourea | C13H10Cl2N2OS | I | 313.20 | 313.5 |
| 204 | 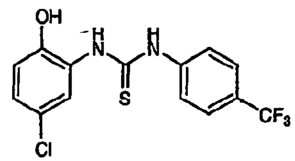 | 1-(5-Chloro-2-hydroxy-phenyl)-3-(4-trifluoromethyl-phenyl)-thiourea | C14H10ClF3N2OS | I | 346.76 | 347.0 |
| 205 | 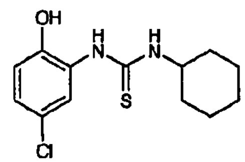 | 1-(5-Chloro-2-hydroxy-phenyl)-3-cyclohexyl-thiourea | C13H17ClN2OS | I | 284.80 | 285.0 |
| 206 | 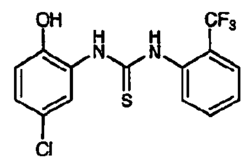 | 1-(5-Chloro-2-hydroxy-phenyl)-3-(2-trifluoromethyl-phenyl)-thiourea | C14H10ClF3N2OS | I | 346.76 | 347.0 |
| 207 | 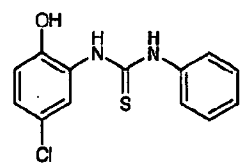 | 1-(5-Chloro-2-hydroxy-phenyl)-3-phenyl-thiourea | C13H11ClN2OS | I | 278.76 | 279.0 |

| | | | | | | |
|---|---|---|---|---|---|---|
| 208 | 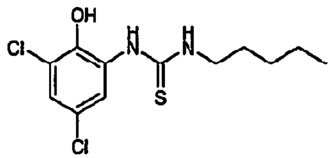 | 1-(3,5-Dichloro-2-hydroxy-phenyl)-3-pentyl-thiourea | C12H16Cl2N2OS | I | 307.24 | 307.5 |
| 209 | 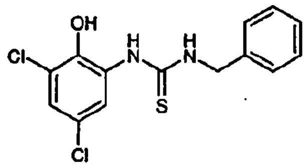 | 1-Benzyl-3-(3,5-dichloro-2-hydroxy-phenyl)-thiourea | C14H12Cl2N2OS | I | 327.23 | 327.4 |
| 210 | 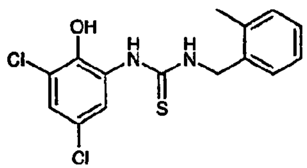 | 1-(3,5-Dichloro-2-hydroxy-phenyl)-3-(2-methyl-benzyl)-thiourea | C15H14Cl2N2OS | I | 341.26 | 341.4 |
| 211 | 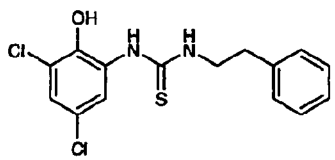 | 1-(3,5-Dichloro-2-hydroxy-phenyl)-3-phenethyl-thiourea | C15H14Cl2N2OS | I | 341.26 | 341.5 |
| 212 | 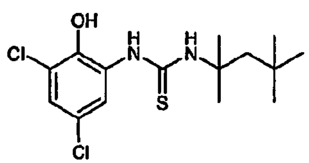 | 1-(3,5-Dichloro-2-hydroxy-phenyl)-3-(1,1,3,3-tetramethyl-butyl)-thiourea | C15H22Cl2N2OS | I | 349.32 | 349.6 |

| | | | | | | |
|---|---|---|---|---|---|---|
| 213 | 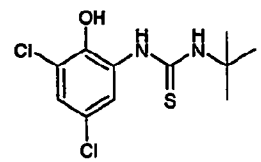 | 1-tert-Butyl-3-(3,5-dichloro-2-hydroxy-phenyl)-thiourea | C11H14Cl2N2OS | I | 293.21 | 293.6 |
| 214 | 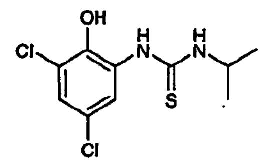 | 1-(5-Chloro-2-hydroxy-phenyl)-3-isopropyl-thiourea | C10H12Cl2N2OS | I | 279.19 | 279.5 |
| 215 | 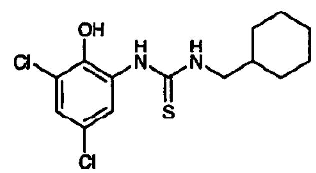 | 1-(3,5-Dichloro-2-hydroxy-phenyl)-3-cyclohexylmethyl-thiourea | C14H18Cl2N2OS | I | 333.28 | 333.5 |
| 216 | 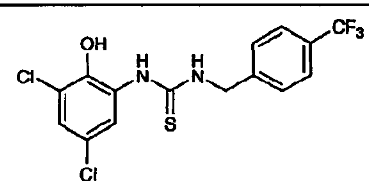 | 1-(3,5-Dichloro-2-hydroxy-phenyl)-3-(4-trifluoromethyl-benzyl)-thiourea | C15H11Cl2F3N2OS | I | 395.23 | 395.5 |
| 217 | 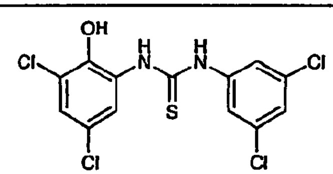 | 1-(3,5-Dichloro-2-hydroxy-phenyl)-3-(3,5-dichloro-phenyl)-thiourea | C13H8Cl4N2OS | I | 382.09 | 382.3 |

| | | | | | | |
|---|---|---|---|---|---|---|
| 218 | 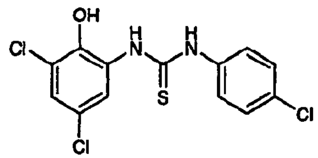 | 1-(3,5-Dichloro-2-hydroxy-phenyl)-3-(4-chloro-phenyl)-thiourea | C13H9Cl3N2OS | I | 345.65 | 345.9 |
| 219 | 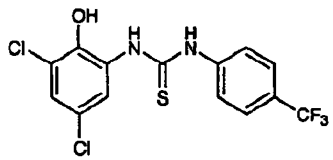 | 1-(3,5-Dichloro-2-hydroxy-phenyl)-3-(4-trifluoromethyl-phenyl)-thiourea | C14H9Cl2F3N2OS | I | 381.2 | 381.6 |
| 220 | 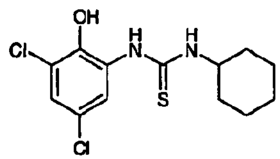 | 1-(3,5-Dichloro-2-hydroxy-phenyl)-3-cyclohexyl-thiourea | C13H16Cl2N2OS | I | 319.25 | 319.6 |
| 221 | 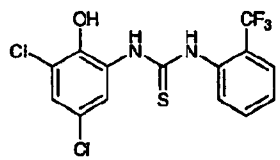 | 1-(3,5-Dichloro-2-hydroxy-phenyl)-3-(2-trifluoromethyl-phenyl)-thiourea | C14H9Cl2F3N2OS | I | 381.20 | 381.4 |
| 222 | 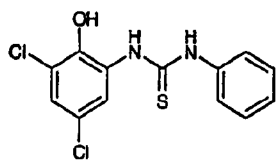 | 1-(3,5-Dichloro-2-hydroxy-phenyl)-3-phenyl-thiourea | C13H10Cl2N2OS | I | 313.20 | 313.5 |
| 223 | 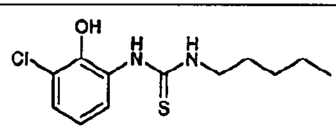 | 1-(3-Chloro-2-hydroxy-phenyl)-3-pentyl-thiourea | C12H17ClN2OS | I | 272.79 | 273.2 |

| | | | | | | |
|---|---|---|---|---|---|---|
| 224 | 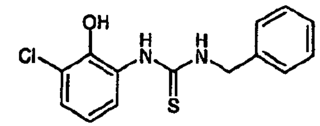 | 1-Benzyl-3-(3-chloro-2-hydroxy-phenyl)-thiourea | C14H13ClN2OS | I | 292.78 | 293.1 |
| 225 | 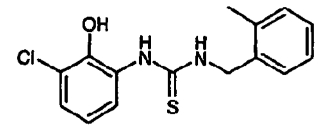 | 1-(3-Chloro-2-hydroxy-phenyl)-3-(2-methyl-benzyl)-thiourea | C15H15ClN2OS | I | 306.81 | 307.0 |
| 226 | 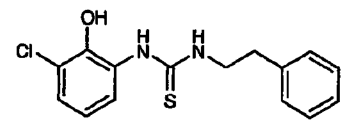 | 1-(3-Chloro-2-hydroxy-phenyl)-3-phenethyl-thiourea | C15H15ClN2OS | I | 306.81 | 307.1 |
| 227 | 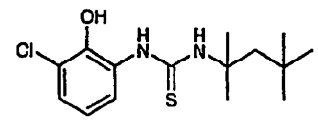 | 1-(3-Chloro-2-hydroxy-phenyl)-3-(1,1,3,3-tetramethyl-butyl)-thiourea | C15H23ClN2OS | I | 314.87 | 315.2 |
| 228 | 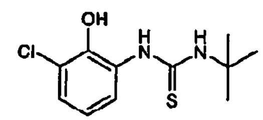 | 1-tert-Butyl-3-(3-Chloro-2-hydroxy-phenyl)-thiourea | C11H15ClN2OS | I | 258.77 | 259.0 |
| 229 | 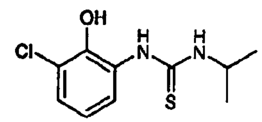 | 1-(3-Chloro-2-hydroxy-phenyl)-3-isopropyl-thiourea | C10H13ClN2OS | I | 244.74 | 244.9 |
| 230 | 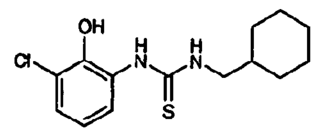 | 1-(3-Chloro-2-hydroxy-phenyl)-3-cyclohexylmethyl-thiourea | C14H19ClN2OS | I | 298.83 | 299.3 |

| | | | | | | |
|---|---|---|---|---|---|---|
| 231 | 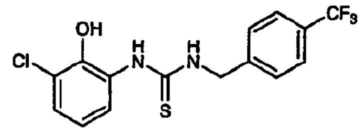 | 1-(3-Chloro-2-hydroxy-phenyl)-3-(4-trifluoromethyl-benzyl)-thiourea | C15H12ClF3N2OS | I | 360.78 | 361.1 |
| 232 | 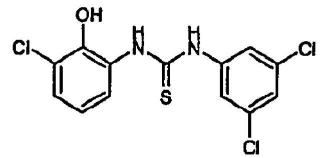 | 1-(3-Chloro-2-hydroxy-phenyl)-3-(3,5-dichloro-phenyl)-thiourea | C13H9Cl3N2OS | I | 347.65 | 348.0 |
| 233 | 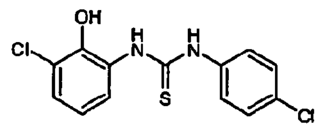 | 1-(3-Chloro-2-hydroxy-phenyl)-3-(4-chloro-phenyl)-thiourea | C13H10Cl2N2OS | I | 313.20 | 313.5 |
| 234 | 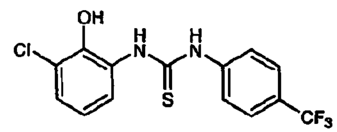 | 1-(3-Chloro-2-hydroxy-phenyl)-3-(4-trifluoromethyl-phenyl)-thiourea | C14H10ClF3N2OS | I | 346.76 | 347.2 |
| 235 | 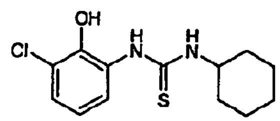 | 1-(3-Chloro-2-hydroxy-phenyl)-3-cyclohexyl-thiourea | C13H17ClN2OS | I | 284.8 | 285.1 |
| 236 | 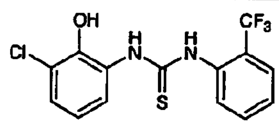 | 1-(3-Chloro-2-hydroxy-phenyl)-3-(2-trifluoromethyl-phenyl)-thiourea | C14H10ClF3N2OS | I | 346.76 | 347.2 |
| 237 | 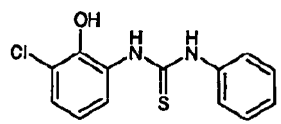 | 1-(3-Chloro-2-hydroxy-phenyl)-3-phenyl-thiourea | C13H11ClN2OS | I | 278.76 | 279.0 |

| | | | | | | |
|---|---|---|---|---|---|---|
| 238 | 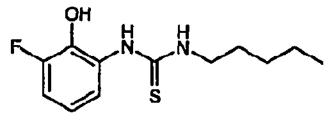 | 1-(3-Fluoro-2-hydroxy-phenyl)-3-pentyl-thiourea | C12H17FN2OS | I | 256.34 | 256.8 |
| 239 | 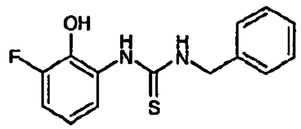 | 1-Benzyl-3-(3-fluoro-2-hydroxy-phenyl)-thiourea | C14H13FN2OS | I | 276.33 | 276.7 |
| 240 | 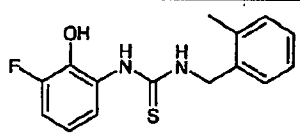 | 1-(3-Fluoro-2-hydroxy-phenyl)-3-(2-methyl-benzyl)-thiourea | C15H15FN2OS | I | 290.36 | 290.5 |
| 241 | 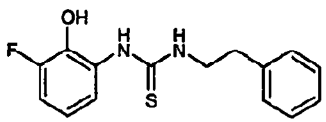 | 1-(3-Fluoro-2-hydroxy-phenyl)-3-phenethyl-thiourea | C15H15FN2OS | I | 290.36 | 290.5 |
| 242 | 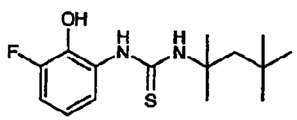 | 1-(3-Fluoro-2-hydroxy-phenyl)-3-(1,1,3,3-tetramethyl-butyl)-thiourea | C15H23FN2OS | I | 298.42 | 298.7 |
| 243 | 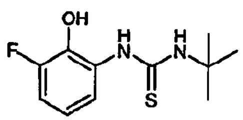 | 1-tert-Butyl-3-(3-fluoro-2-hydroxy-phenyl)-thiourea | C11H15FN2OS | I | 242.31 | 242.8 |
| 244 | 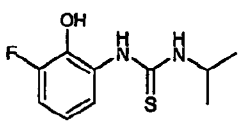 | 1-(3-Fluoro-2-hydroxy-phenyl)-3-isopropyl-thiourea | C10H13FN2OS | I | 228.29 | 228.5 |

| | | | | | | |
|---|---|---|---|---|---|---|
| 245 | 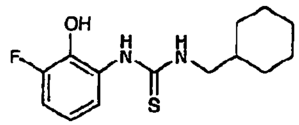 | 1-(3-Fluoro-2-hydroxy-phenyl)-3-cyclohexylmethyl-thiourea | C14H19FN2OS | I | 282.38 | 282.7 |
| 246 | 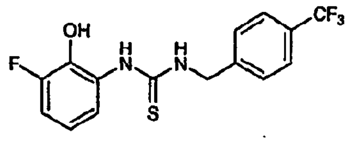 | 1-(3-Fluoro-2-hydroxy-phenyl)-3-(4-trifluoromethyl-benzyl)-thiourea | C15H12F4N2OS | I | 344.33 | 344.7 |
| 247 | 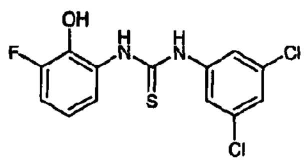 | 1-(3-Fluoro-2-hydroxy-phenyl)-3-(3,5-dichloro-phenyl)-thiourea | C13H9Cl2FN2OS | I | 331.19 | 331.5 |
| 248 | 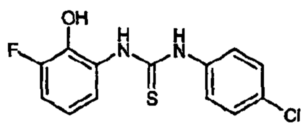 | 1-(3-Fluoro-2-hydroxy-phenyl)-3-(4-chloro-phenyl)-thiourea | C13H10ClFN2OS | I | 296.75 | 297.1 |
| 249 | 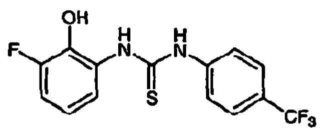 | 1-(3-Fluoro-2-hydroxy-phenyl)-3-(4-trifluoromethyl-phenyl)-thiourea | C14H10F4N2OS | I | 330.30 | 330.6 |
| 250 | 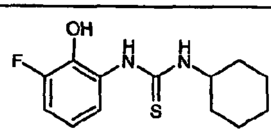 | 1-(3-Fluoro-2-hydroxy-phenyl)-3-cyclohexyl-thiourea | C13H17FN2OS | I | 268.35 | 268.6 |

| | | | | | | |
|---|---|---|---|---|---|---|
| 251 | 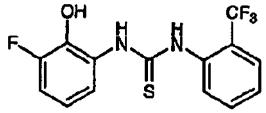 | 1-(3-Fluoro-2-hydroxy-phenyl)-3-(2-trifluoromethyl-phenyl)-thiourea | C14H10F4N2OS | I | 330.30 | 330.5 |
| 252 | 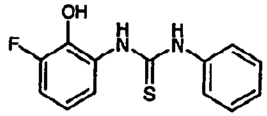 | 1-(3-Fluoro-2-hydroxy-phenyl)-3-phenyl-thiourea | C13H11FN2OS | I | 262.30 | 262.5 |
| 253 | 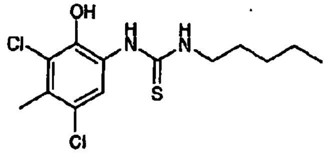 | 1-(3,5-Dichloro-2-hydroxy-4-methyl-phenyl)-3-pentyl-thiourea | C13H18Cl2N2OS | I | 321.27 | 321.8 |
| 254 | 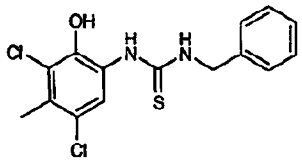 | 1-Benzyl-3-(3,5-dichloro-2-hydroxy-4-methyl-phenyl)-thiourea | C15H14Cl2N2OS | I | 341.26 | 341.5 |
| 255 | 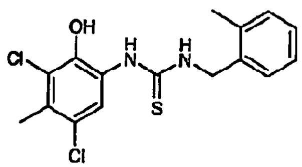 | 1-(3,5-Dichloro-2-hydroxy-4-methyl-phenyl)-3-(2-methyl-benzyl)-thiourea | C16H16Cl2N2OS | I | 355.28 | 355.6 |
| 256 | 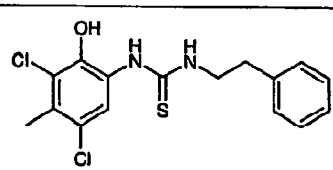 | 1-(3,5-Dichloro-2-hydroxy-4-methyl-phenyl)-3-phenethyl-thiourea | C16H16Cl2N2OS | I | 355.28 | 355.6 |

| | | | | | | |
|---|---|---|---|---|---|---|
| 257 | 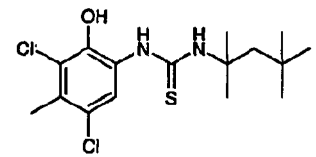 | 1-(3,5-Dichloro-2-hydroxy-4-methyl-phenyl)-3-(1,1,3,3-tetramethyl-butyl)-thiourea | C16H24Cl2N2OS | I | 363.35 | 363.6 |
| 258 | 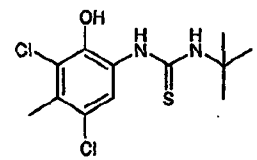 | 1-tert-Butyl-3-(3,5-dichloro-2-hydroxy-4-methyl-phenyl)-thiourea | C12H16Cl2N2OS | I | 307.24 | 307.5 |
| 259 | 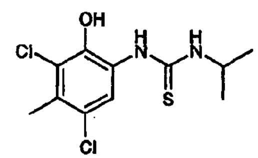 | 1-(5-Chloro-2-hydroxy-4-methyl-phenyl)-3-isopropyl-thiourea | C11H14Cl2N2OS | I | 293.21 | 293.6 |
| 260 | 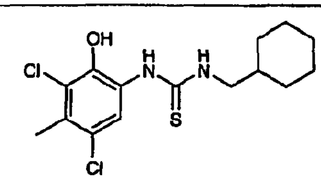 | 1-(3,5-Dichloro-2-hydroxy-4-methyl-phenyl)-3-cyclohexylmethyl-thiourea | C15H20Cl2N2OS | I | 347.30 | 347.5 |
| 261 | 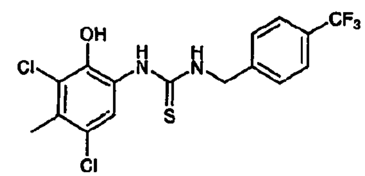 | 1-(3,5-Dichloro-2-hydroxy-4-methyl-phenyl)-3-(4-trifluoromethyl-benzyl)-thiourea | C16H13Cl2F3N2OS | I | 409.25 | 409.5 |

| | | | | | | |
|---|---|---|---|---|---|---|
| 262 | 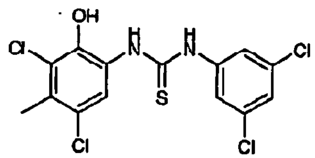 | 1-(3,5-Dichloro-2-hydroxy-4-methyl-phenyl)-3-(3,5-dichloro-phenyl)-thiourea | C14H10Cl4N2OS | I | 396.12 | 396.3 |
| 263 | 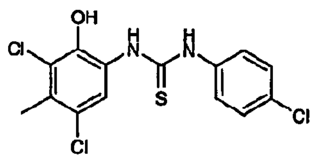 | 1-(3,5-Dichloro-2-hydroxy-4-methyl-phenyl)-3-(4-chloro-phenyl)-thiourea | C14H11Cl3N2OS | I | 361.67 | 361.9 |
| 264 | 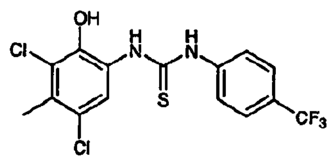 | 1-(3,5-Dichloro-2-hydroxy-4-methyl-phenyl)-3-(4-trifluoromethyl-phenyl)-thiourea | C15H11Cl2F3N2OS | I | 395.23 | 395.7 |
| 265 | 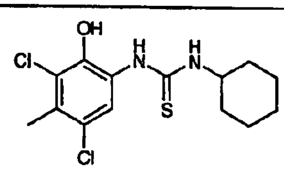 | 1-(3,5-Dichloro-2-hydroxy-4-methyl-phenyl)-3-cyclohexyl-thiourea | C14H18Cl2N2OS | I | 333.28 | 333.8 |
| 266 | 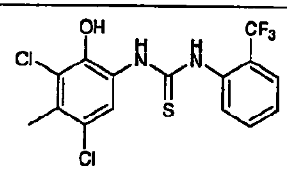 | 1-(3,5-Dichloro-2-hydroxy-4-methyl-phenyl)-3-(2-trifluoromethyl-phenyl)-thiourea | C15H11Cl2F3N2OS | I | 395.23 | 395.8 |

| | | | | | | |
|---|---|---|---|---|---|---|
| 267 | 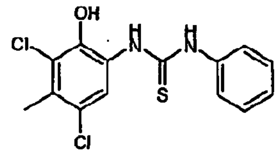 | 1-(3,5-Dichloro-2-hydroxy-4-methyl-phenyl)-3-phenyl-thiourea | C14H12Cl2N2OS | I | 327.23 | 327.7 |
| 268 | 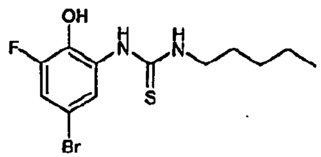 | 1-(5-Bromo-3-fluoro-2-hydroxy-phenyl)-3-pentyl-thiourea | C12H16BrFN2OS | I | 335.24 | 335.8 |
| 269 | 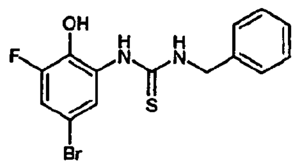 | 1-Benzyl-3-(5-bromo-3-fluoro-2-hydroxy-phenyl)-thiourea | C14H12BrFN2OS | I | 355.23 | 355.4 |
| 270 | 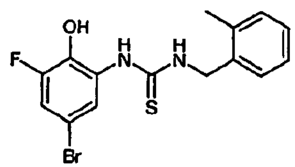 | 1-(5-Bromo-3-fluoro-2-hydroxy-phenyl)-3-(2-methyl-benzyl)-thiourea | C15H14BrFN2OS | I | 369.25 | 369.5 |
| 271 | 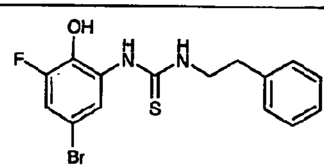 | 1-(5-Bromo-3-fluoro-2-hydroxy-phenyl)-3-phenethyl-thiourea | C15H14BrFN2OS | I | 369.25 | 369.5 |

| | | | | | | |
|---|---|---|---|---|---|---|
| 272 | 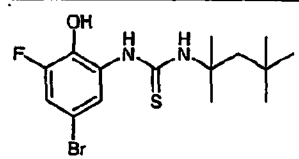 | 1-(5-Bromo-3-fluoro-2-hydroxy-phenyl)-3-(1,1,3,3-tetramethyl-butyl)-thiourea | C15H22BrFN2OS | I | 377.32 | 377.5 |
| 273 | 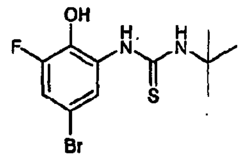 | 1-tert-Butyl-3-(5-bromo-3-fluoro-2-hydroxy-phenyl)-thiourea | C11H14BrFN2OS | I | 321.21 | 321.5 |
| 274 | 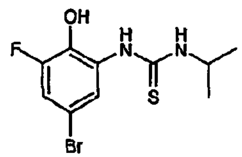 | 1-(5-Bromo-3-fluoro-2-hydroxy-phenyl)-3-isopropyl-thiourea | C10H12BrFN2OS | I | 307.19 | 307.4 |
| 275 | 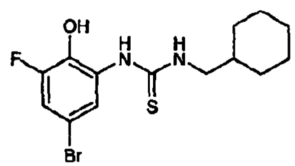 | 1-(5-Bromo-3-fluoro-2-hydroxy-phenyl)-3-cyclohexylmethyl-thiourea | C14H18BrFN2OS | I | 361.28 | 361.5 |
| 276 | 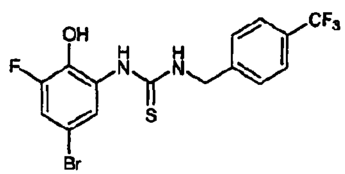 | 1-(5-Bromo-3-fluoro-2-hydroxy-phenyl)-3-(4-trifluoromethyl-benzyl)-thiourea | C15H11BrF4N2OS | I | 423.23 | 423.5 |

| | | | | | | |
|---|---|---|---|---|---|---|
| 277 | 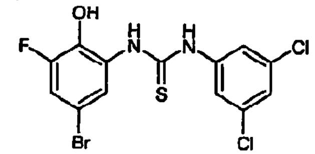 | 1-(5-Bromo-3-fluoro-2-hydroxy-phenyl)-3-(3,5-dichloro-phenyl)-thiourea | C13H8BrCl2FN2OS | I | 410.09 | 410.5 |
| 278 | 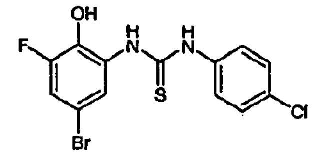 | 1-(5-Bromo-3-fluoro-2-hydroxy-phenyl)-3-(4-chloro-phenyl)-thiourea | C13H9BrClFN2OS | I | 375.65 | 375.9 |
| 279 | 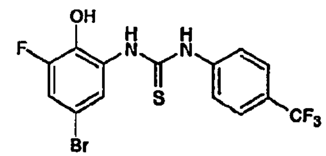 | 1-(5-Bromo-3-fluoro-2-hydroxy-phenyl)-3-(4-trifluoromethyl-phenyl)-thiourea | C14H9BrF4N2OS | I | 409.20 | 409.5 |
| 280 | 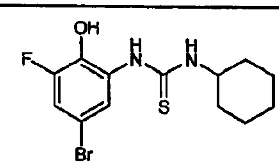 | 1-(5-Bromo-3-fluoro-2-hydroxy-phenyl)-3-cyclohexyl-thiourea | C13H16BrFN2OS | I | 347.25 | 347.6 |
| 281 | 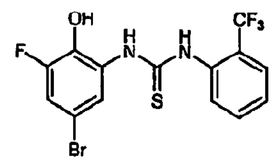 | 1-(5-Bromo-3-fluoro-2-hydroxy-phenyl)-3-(2-trifluoromethyl-phenyl)-thiourea | C14H9BrF4N2OS | I | 409.20 | 409.5 |

| | | | | | | |
|---|---|---|---|---|---|---|
| 282 | 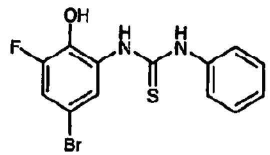 | 1-(5-Bromo-3-fluoro-2-hydroxy-phenyl)-3-phenyl-thiourea | C13H10BrFN2OS | I | 341.20 | 341.5 |
| 283 | 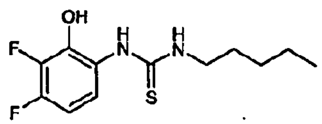 | 1-(3,4-Difluoro-2-hydroxy-phenyl)-3-pentyl-thiourea | C12H16F2N2OS | I | 274.33 | 274.8 |
| 284 | 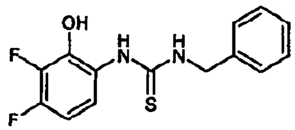 | 1-Benzyl-3-(3,4-difluoro-2-hydroxy-phenyl)-thiourea | C14H12F2N2OS | I | 294.32 | 294.7 |
| 285 | 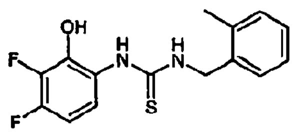 | 1-(3,4-Difluoro-2-hydroxy-phenyl)-3-(2-methyl-benzyl)-thiourea | C15H14F2N2OS | I | 308.35 | 308.6 |
| 286 | 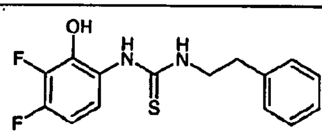 | 1-(3,4-Difluoro-2-hydroxy-phenyl)-3-phenethyl-thiourea | C15H14F2N2OS | I | 308.35 | 308.6 |
| 287 | 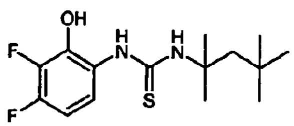 | 1-(3,4-Difluoro-2-hydroxy-phenyl)-3-(1,1,3,3-tetramethyl-butyl)-thiourea | C15H22F2N2OS | I | 316.41 | 316.8 |

| | | | | | | |
|---|---|---|---|---|---|---|
| 288 | 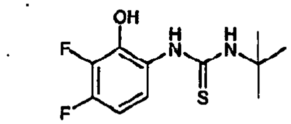 | 1-tert-Butyl-3-(3,4-Difluoro-2-hydroxy-phenyl)-thiourea | C11H14F2N2OS | I | 260.30 | 260.5 |
| 289 | 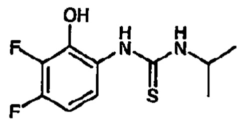 | 1-(3,4-Difluoro-2-hydroxy-phenyl)-3-isopropyl-thiourea | C10H12F2N2OS | I | 246.28 | 246.5 |
| 290 | 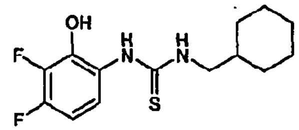 | 1-(3,4-Difluoro-2-hydroxy-phenyl)-3-cyclohexylmethyl-thiourea | C14H18F2N2OS | I | 300.37 | 300.6 |
| 291 | 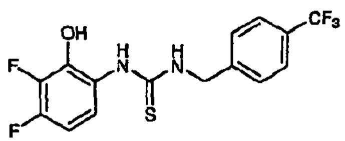 | 1-(3,4-Difluoro-2-hydroxy-phenyl)-3-(4-trifluoromethyl-benzyl)-thiourea | C15H11F5N2OS | I | 362.32 | 362.8 |
| 292 | 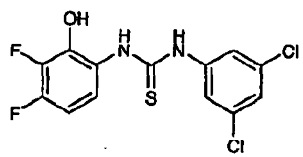 | 1-(3,4-Difluoro-2-hydroxy-phenyl)-3-(3,5-dichloro-phenyl)-thiourea | C13H8Cl2F2N2OS | I | 349.18 | 349.7 |
| 293 | 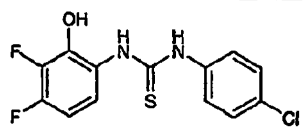 | 1-(3,4-Difluoro-2-hydroxy-phenyl)-3-(4-chloro-phenyl)-thiourea | C13H9ClF2N2OS | I | 314.74 | 315.2 |

| | | | | | | |
|---|---|---|---|---|---|---|
| 294 | 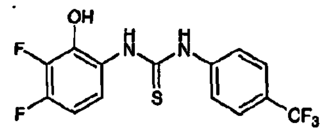 | 1-(3,4-Difluoro-2-hydroxy-phenyl)-3-(4-trifluoromethyl-phenyl)-thiourea | C14H9F5N2OS | I | 348.29 | 348.8 |
| 295 | 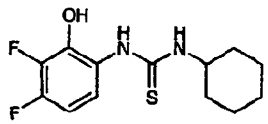 | 1-(3,4-Difluoro-2-hydroxy-phenyl)-3-cyclohexyl-thiourea | C13H16F2N2OS | I | 286.34 | 286.6 |
| 296 | 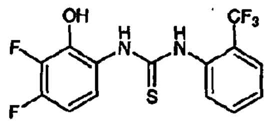 | 1-(3,4-Difluoro-2-hydroxy-phenyl)-3-(2-trifluoromethyl-phenyl)-thiourea | C14H9F5N2OS | I | 348.29 | 348.8 |
| 297 | 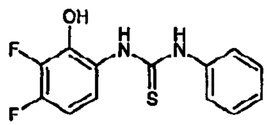 | 1-(3,4-Difluoro-2-hydroxy-phenyl)-3-phenyl-thiourea | C13H10F2N2OS | I | 280.29 | 280.5 |
| 298 | 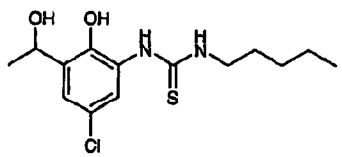 | 1-[5-Chloro-2-hydroxy-3-(1-hydroxy-ethyl)-phenyl]-3-pentyl-thiourea | C14H21ClN2O2S | I | 316.85 | 316.9 |
| 299 | 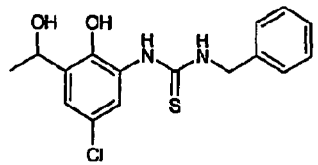 | 1-Benzyl-3-[5-chloro-2-hydroxy-3-(1-hydroxy-ethyl)-phenyl]-thiourea | C16H17ClN2O2S | I | 336.84 | 337.2 |

| | Structure | Chemical name | Formula | Synthesis methods | MolWeight | MS data |
|---|---|---|---|---|---|---|
| 300 | 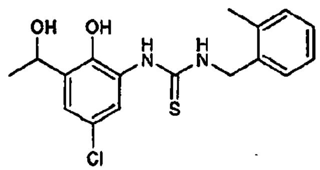 | 1-[5-Chloro-2-hydroxy-3-(1-hydroxy-ethyl)-phenyl]-3-(2-methyl-benzyl)-thiourea | C17H19ClN2O2S | I | 350.86 | 351.1 |
| 301 | 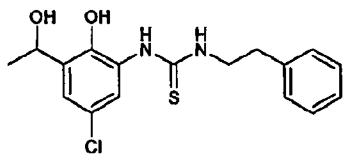 | 1-[5-Chloro-2-hydroxy-3-(1-hydroxy-ethyl)-phenyl]-3-phenethyl-thiourea | C17H19ClN2O2S | I | 350.86 | 351.0 |
| 302 | 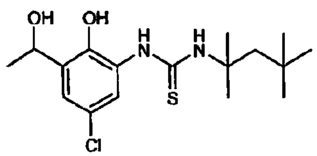 | 1-[5-Chloro-2-hydroxy-3-(1-hydroxy-ethyl)-phenyl]-3-(1,1,3,3-tetramethyl-butyl)-thiourea | C17H27ClN2O2S | I | 358.93 | 359.2 |
| 303 | 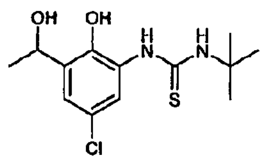 | 1-tert-Butyl-3-[5-chloro-2-hydroxy-3-(1-hydroxy-ethyl)-phenyl]-thiourea | C13H19ClN2O2S | I | 302.82 | 303.0 |
| 304 | 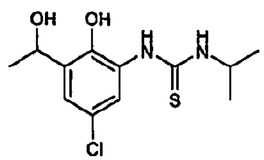 | 1-[5-Chloro-2-hydroxy-3-(1-hydroxy-ethyl)-phenyl]-3-isopropyl-thiourea | C12H17ClN2O2S | I | 288.79 | 289.0 |

| | | | | | | |
|---|---|---|---|---|---|---|
| 305 | 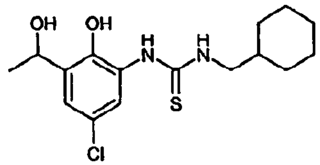 | 1-[5-Chloro-2-hydroxy-3-(1-hydroxy-ethyl)-phenyl]-3-cyclohexylmethyl-thiourea | C16H23ClN2O2S | I | 342.88 | 343.1 |
| 306 | 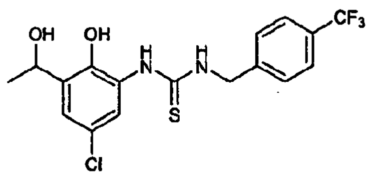 | 1-[5-Chloro-2-hydroxy-3-(1-hydroxy-ethyl)-phenyl]-3-(4-trifluoromethyl-benzyl)-thiourea | C17H16ClF3N2O2S | I | 404.83 | 405.2 |
| 307 | 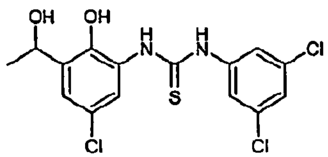 | 1-[5-Chloro-2-hydroxy-3-(1-hydroxy-ethyl)-phenyl]-3-(3,5-dichloro-phenyl)-thiourea | C15H13Cl3N2O2S | I | 391.70 | 392.0 |
| 308 | 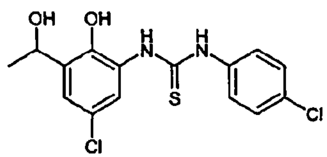 | 1-[5-Chloro-2-hydroxy-3-(1-hydroxy-ethyl)-phenyl]-3-(4-chloro-phenyl)-thiourea | C15H14Cl2N2O2S | I | 357.25 | 357.6 |
| 309 | 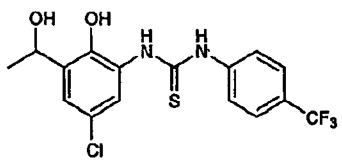 | 1-[5-Chloro-2-hydroxy-3-(1-hydroxy-ethyl)-phenyl]-3-(4-trifluoromethyl-phenyl)-thiourea | C16H14ClF3N2O2S | I | 390.81 | 391.0 |

| | | | | | | |
|---|---|---|---|---|---|---|
| 310 | 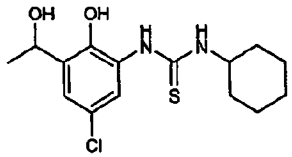 | 1-[5-Chloro-2-hydroxy-3-(1-hydroxy-ethyl)-phenyl]-3-cyclohexyl-thiourea | C15H21ClN2O2S | I | 328.86 | 329.4 |
| 311 | 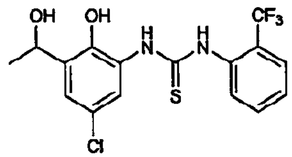 | 1-[5-Chloro-2-hydroxy-3-(1-hydroxy-ethyl)-phenyl]-3-(2-trifluoromethyl-phenyl)-thiourea | C16H14ClF3N2O2S | I | 390.81 | 391.0 |
| 312 | 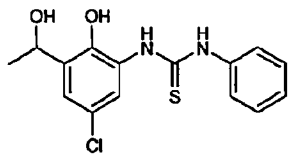 | 1-[5-Chloro-2-hydroxy-3-(1-hydroxy-ethyl)-phenyl]-3-phenyl-thiourea | C15H15ClN2O2S | I | 322.81 | 323.1 |
| 313 | 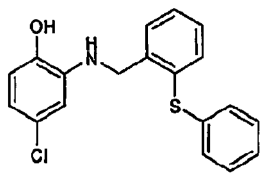 | 4-Chloro-2-(2-phenylsulfanyl-benzylamino)-phenol | C19H16ClNOS | D | 341.85 | 342.2 |
| 314 | 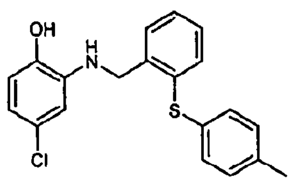 | 4-Chloro-2-(2-p-tolylsulfanyl-benzylamino)-phenol | C20H18ClNOS | D | 355.88 | 356.3 |

| | | | | | | |
|---|---|---|---|---|---|---|
| 315 | 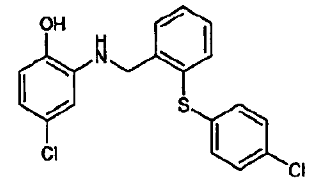 | 4-Chloro-2-[2-(4-chloro-phenylsulfanyl)-benzylamino]-phenol | C19H15Cl2NOS | D | 376.30 | 376.8 |
| 316 | 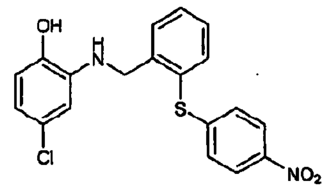 | 4-Chloro-2-[2-(4-nitro-phenylsulfanyl)-benzylamino]-phenol | C19H15ClN2O3S | D | 386.85 | 387.2 |
| 317 | 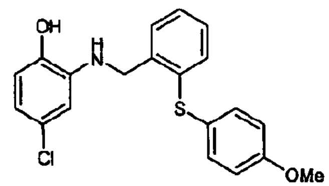 | 4-Chloro-2-[2-(4-methoxy-phenylsulfanyl)-benzylamino]-phenol | C20H18ClNO2S | D | 371.88 | 372.4 |
| 318 | 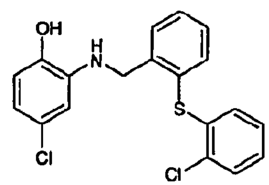 | 4-Chloro-2-[2-(2-chloro-phenylsulfanyl)-benzylamino]-phenol | C19H15Cl2NOS | D | 376.30 | 376.8 |
| 319 | 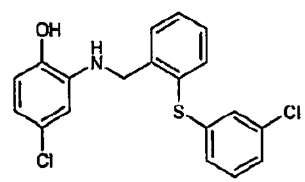 | 4-Chloro-2-[2-(3-chloro-phenylsulfanyl)-benzylamino]-phenol | C19H15Cl2NOS | D | 376.30 | 376.8 |

| | | | | | | |
|---|---|---|---|---|---|---|
| 320 | 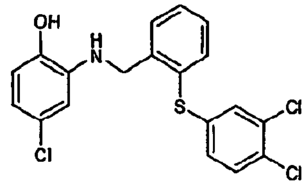 | 4-Chloro-2-[2-(3,4-dichloro-phenylsulfanyl)-benzylamino]-phenol | C19H14Cl3NOS | D | 410.74 | 411.2 |
| 321 | 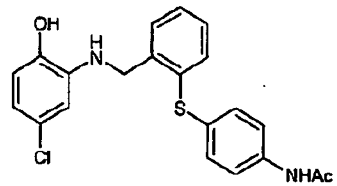 | N-(4-{2-[(5-Chloro-2-hydroxy-phenylamino)-methyl]-phenylsulfanyl}-phenyl)-acetamide | C21H19ClN2O2S | D | 398.91 | 399.3 |
| 322 | 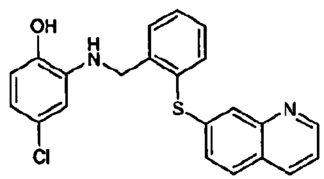 | 4-Chloro-2-[2-(quinolin-7-ylsulfanyl)-benzylamino]-phenol | C22H17ClN2OS | D | 392.90 | 393.4 |
| 323 | 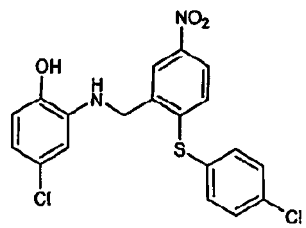 | 4-Chloro-2-[2-(4-chloro-phenylsulfanyl)-5-nitro-benzylamino]-phenol | C19H14Cl2N2O3S | D | 421.30 | 421.7 |

| | | | | | | |
|---|---|---|---|---|---|---|
| 324 | 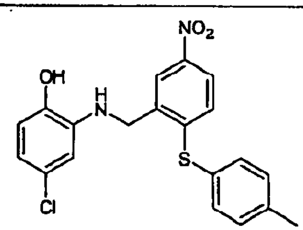 | 4-Chloro-2-(5-nitro-2-p-tolylsulfanyl-benzylamino)-phenol | C20H17ClN2O3S | D | 400.88 | 401.2 |
| 325 | 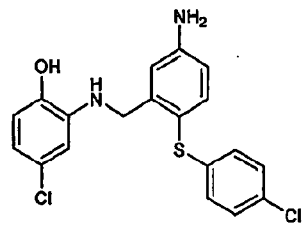 | 2-[5-Amino-2-(4-chloro-phenylsulfanyl)-benzylamino]-4-chloro-phenol | C19H16Cl2N2OS | D | 391.31 | 391.9 |
| 326 | 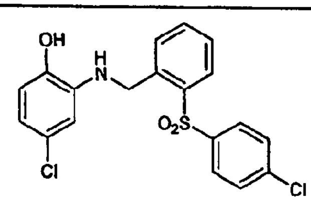 | 4-Chloro-2-[2-(4-chloro-benzenesulfonyl)-benzylamino]-phenol | C19H15Cl2NO3S | D | 408.30 | 408.7 |
| 327 | 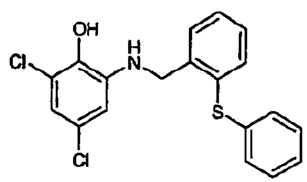 | 2,4-Dichloro-6-(2-phenylsulfanyl-benzylamino)-phenol | C19H15Cl2NOS | D | 376.30 | 376.7 |

| | | | | | | |
|---|---|---|---|---|---|---|
| 328 | 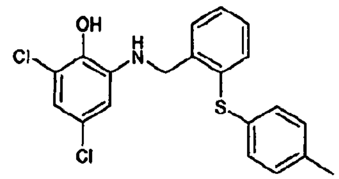 | 2,4-Dichloro-6-(2-p-tolylsulfanyl-benzylamino)-phenol | C20H17Cl2NOS | D | 390.33 | 390.7 |
| 329 | 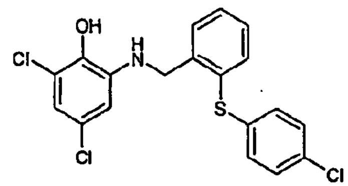 | 2,4-Dichloro-6-[2-(4-chloro-phenylsulfanyl)-benzylamino]-phenol | C19H14Cl3NOS | D | 410.75 | 411.1 |
| 330 | 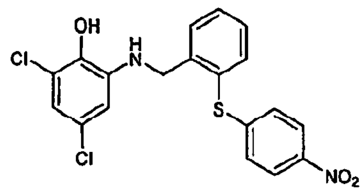 | 2,4-Dichloro-6-[2-(4-nitro-phenylsulfanyl)-benzylamino]-phenol | C19H14Cl2N2O3S | D | 421.30 | 421.8 |
| 331 | 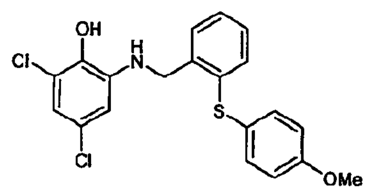 | 2,4-Dichloro-6-[2-(4-methoxy-phenylsulfanyl)-benzylamino]-phenol | C20H17Cl2NO2S | D | 406.33 | 406.7 |
| 332 | 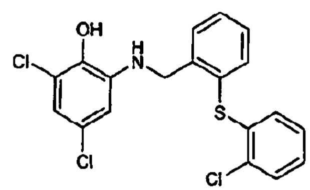 | 2,4-Dichloro-6-[2-(2-chloro-phenylsulfanyl)-benzylamino]-phenol | C19H14Cl3NOS | D | 410.75 | 411.2 |

| | | | | | | |
|---|---|---|---|---|---|---|
| 333 | 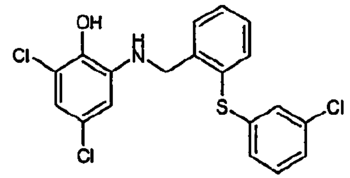 | 2,4-Dichloro-6-[2-(3-chloro-phenylsulfanyl)-benzylamino]-phenol | C19H14Cl3NOS | D | 410.75 | 411.2 |
| 334 | 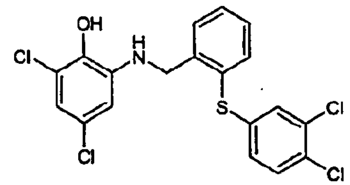 | 2,4-Dichloro-6-[2-(3,4-dichloro-phenylsulfanyl)-benzylamino]-phenol | C19H13Cl4NOS | D | 445.19 | 445.6 |
| 335 | 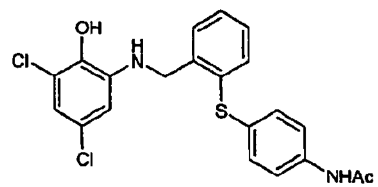 | N-(4-{2-[(3,5-Dichloro-2-hydroxy-phenylamino)-methyl]-phenylsulfanyl}-phenyl)-acetamide | C21H18Cl2N2O2S | D | 433.36 | 433.8 |
| 336 | 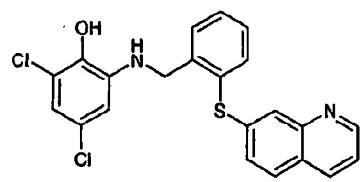 | 2,4-Dichloro-6-[2-(quinolin-7-ylsulfanyl)-benzylamino]-phenol | C22H16Cl2N2OS | D | 427.35 | 427.8 |

| | | | | | | |
|---|---|---|---|---|---|---|
| 337 | 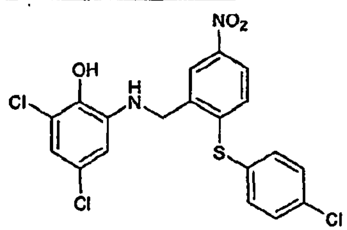 | 2,4-Dichloro-6-[2-(4-chloro-phenylsulfanyl)-5-nitro-benzylamino]-phenol | C19H13Cl3N2O3S | D | 455.74 | 456.0 |
| 338 | 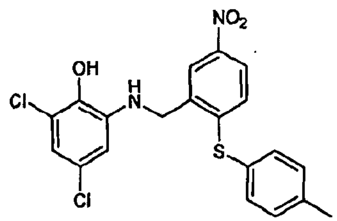 | 2,4-Dichloro-6-(5-nitro-2-p-tolylsulfanyl-benzylamino)-phenol | C20H16Cl2N2O3S | D | 435.33 | 435.7 |
| 339 | 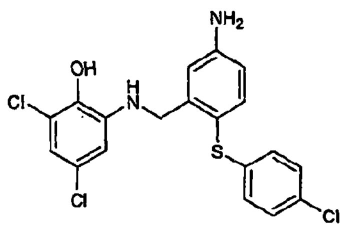 | 2-[5-Amino-2-(4-chloro-phenylsulfanyl)-benzylamino]-4,6-dichloro-phenol | C19H15Cl3N2OS | D | 425.76 | 426.0 |
| 340 | 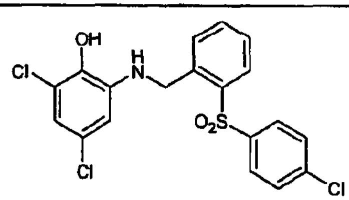 | 2,4-Dichloro-6-[2-(4-chloro-benzenesulfonyl)-benzylamino]-phenol | C19H14Cl3NO3S | D | 442.75 | 443.1 |

| | | | | | | |
|---|---|---|---|---|---|---|
| 341 | 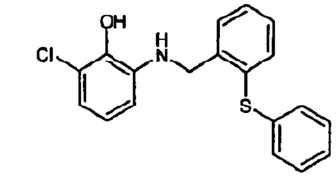 | 2-Chloro-6-(2-phenylsulfanyl-benzylamino)-phenol | C19H16ClNOS | D | 341.85 | 342.3 |
| 342 | 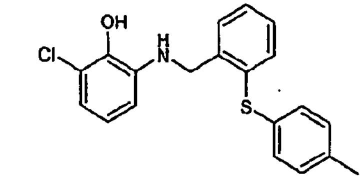 | 2-Chloro-6-(2-p-tolylsulfanyl-benzylamino)-phenol | C20H18ClNOS | D | 355.88 | 356.3 |
| 343 | 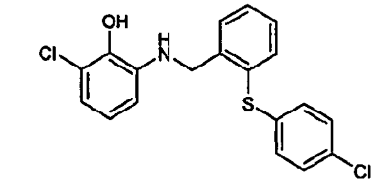 | 2-Chloro-6-[2-(4-chloro-phenylsulfanyl)-benzylamino]-phenol | C19H15Cl2NOS | D | 376.30 | 376.7 |
| 344 | 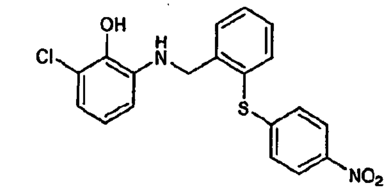 | 2-Chloro-6-[2-(4-nitro-phenylsulfanyl)-benzylamino]-phenol | C19H15ClN2O3S | D | 386.85 | 387.2 |
| 345 | 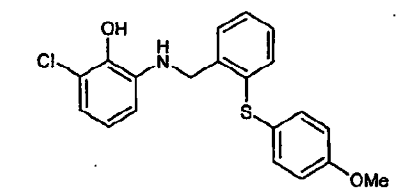 | 2-Chloro-6-[2-(4-methoxy-phenylsulfanyl)-benzylamino]-phenol | C20H18ClNO2S | D | 371.88 | 372.2 |

| | | | | | | |
|---|---|---|---|---|---|---|
| 346 | 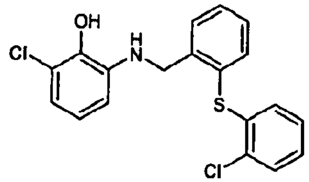 | 2-Chloro-6-[2-(2-chloro-phenylsulfanyl)-benzylamino]-phenol | C19H15Cl2NOS | D | 376.30 | 376.8 |
| 347 | 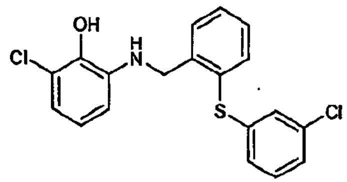 | 2-Chloro-6-[2-(3-chloro-phenylsulfanyl)-benzylamino]-phenol | C19H15Cl2NOS | D | 376.30 | 376.8 |
| 348 | 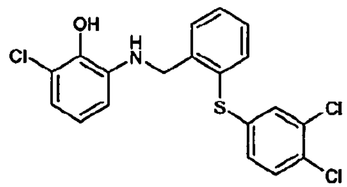 | 2-Chloro-6-[2-(3,4-dichloro-phenylsulfanyl)-benzylamino]-phenol | C19H14Cl3NOS | D | 410.74 | 411.0 |
| 349 | 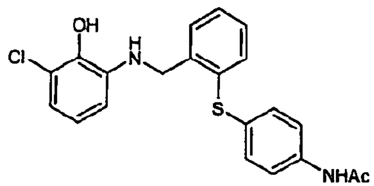 | N-(4-{2-[(3-Chloro-2-hydroxy-phenylamino)-methyl]-phenylsulfanyl}-phenyl)-acetamide | C21H19ClN2O2S | D | 398.91 | 399.3 |
| 350 | 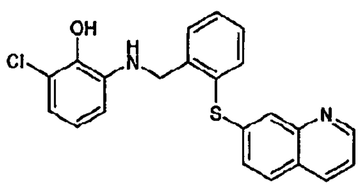 | 2-Chloro-6-[2-(quinolin-7-ylsulfanyl)-benzylamino]-phenol | C22H17ClN2OS | D | 392.90 | 393.4 |

| | | | | | | |
|---|---|---|---|---|---|---|
| 351 | 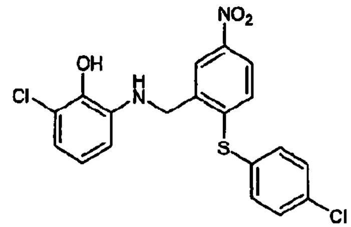 | 2-Chloro-6-[2-(4-chloro-phenylsulfanyl)-5-nitro-benzylamino]-phenol | C19H14Cl2N2O3S | D | 421.30 | 421.5 |
| 352 | 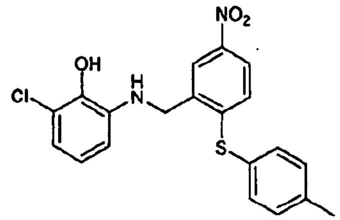 | 2-Chloro-6-(5-nitro-2-p-tolylsulfanyl-benzylamino)-phenol | C20H17ClN2O3S | D | 400.88 | 401.3 |
| 353 | 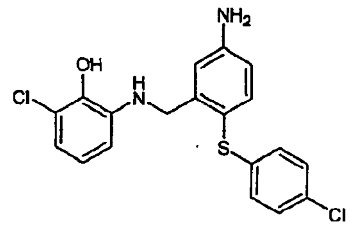 | 2-[5-Amino-2-(4-chloro-phenylsulfanyl)-benzylamino]-6-chloro-phenol | C19H16Cl2N2OS | D | 391.31 | 392.4 |
| 354 | 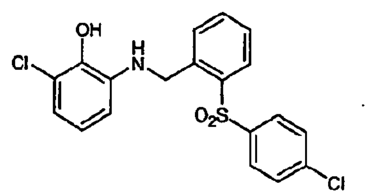 | 2-Chloro-6-[2-(4-chloro-benzenesulfonyl)-benzylamino]-phenol | C19H15Cl2NO3S | D | 408.30 | 408.6 |

| | | | | | | |
|---|---|---|---|---|---|---|
| 355 | 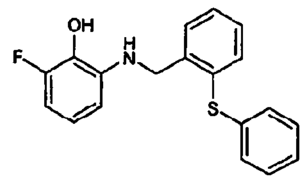 | 2-Fluoro-6-(2-phenylsulfanyl-benzylamino)-phenol | C19H16FNOS | D | 325.40 | 324.6 |
| 356 | 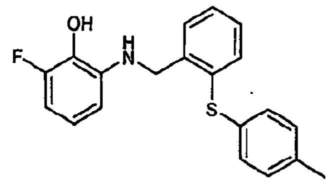 | 2-Fluoro-6-(2-p-tolylsulfanyl-benzylamino)-phenol | C20H18FNOS | D | 339.43 | 340.8 |
| 357 | 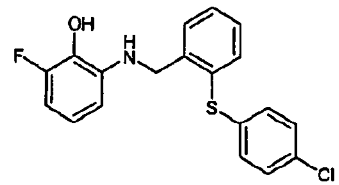 | 2-Fluoro-6-[2-(4-chloro-phenylsulfanyl)-benzylamino]-phenol | C19H15ClFNOS | D | 359.84 | 360.1 |
| 358 | 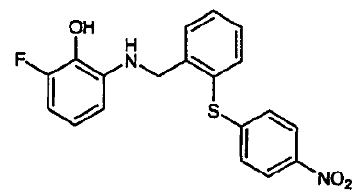 | 2-Fluoro-6-[2-(4-nitro-phenylsulfanyl)-benzylamino]-phenol | C19H15FN2O3S | D | 370.40 | 370.8 |
| 359 | 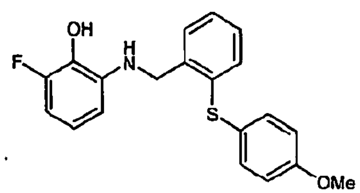 | 2-Fluoro-6-[2-(4-methoxy-phenylsulfanyl)-benzylamino]-phenol | C20H18FNO2S | D | 355.43 | 355.8 |

| | | | | | | |
|---|---|---|---|---|---|---|
| 360 | 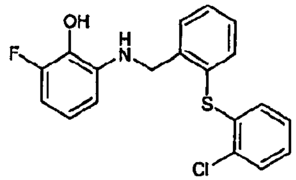 | 2-Fluoro-6-[2-(2-chloro-phenylsulfanyl)-benzylamino]-phenol | C19H15ClFNOS | D | 359.84 | 360.2 |
| 361 | 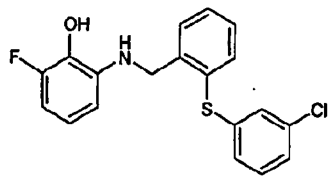 | 2-Fluoro-6-[2-(3-chloro-phenylsulfanyl)-benzylamino]-phenol | C19H15ClFNOS | D | 359.84 | 360.3 |
| 362 | 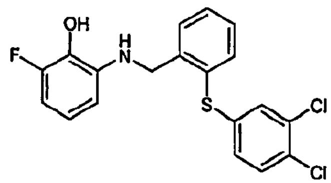 | 2-Fluoro-6-[2-(3,4-dichloro-phenylsulfanyl)-benzylamino]-phenol | C19H14Cl2FNOS | D | 394.29 | 394.6 |
| 363 | 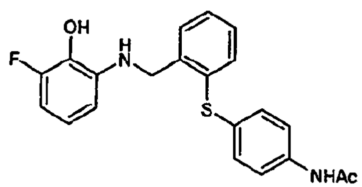 | N-(4-{2-[(3-Fluoro-2-hydroxy-phenylamino)-methyl]-phenylsulfanyl}-phenyl)-acetamide | C21H19FN2O2S | D | 382.45 | 382.9 |
| 364 | 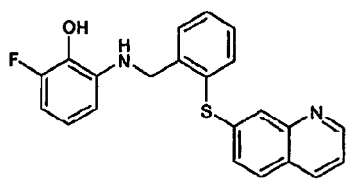 | 2-Fluoro-6-[2-(quinolin-7-ylsulfanyl)-benzylamino]-phenol | C22H17FN2OS | D | 376.45 | 377.0 |

| | | | | | | |
|---|---|---|---|---|---|---|
| 365 | 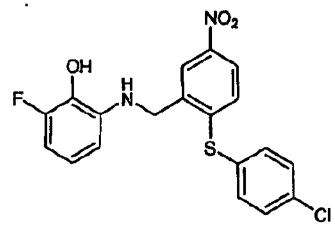 | 2-Fluoro-6-[2-(4-chloro-phenylsulfanyl)-5-nitro-benzylamino]-phenol | C19H14ClFN2O3S | D | 404.84 | 405.3 |
| 366 | 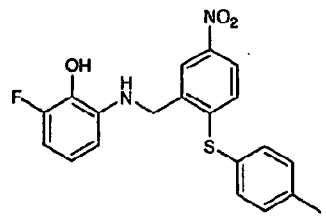 | 2-Fluoro-6-(5-nitro-2-p-tolylsulfanyl-benzylamino)-phenol | C20H17FN2O3S | D | 384.42 | 384.9 |
| 367 | 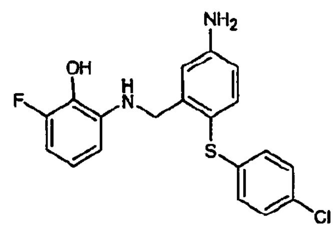 | 2-[5-Amino-2-(4-chloro-phenylsulfanyl)-benzylamino]-6-fluoro-phenol | C19H16ClFN2OS | D | 374.86 | 375.1 |
| 368 | 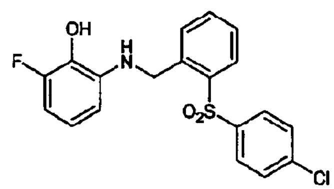 | 2-Fluoro-6-[2-(4-chloro-benzenesulfonyl)-benzylamino]-phenol | C19H15ClFNO3S | D | 391.84 | 392.0 |

| | | | | | | |
|---|---|---|---|---|---|---|
| 369 | 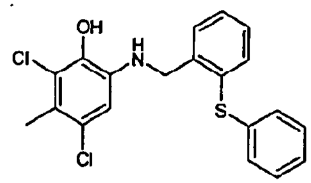 | 2,4-Dichloro-3-methyl-6-(2-phenylsulfanyl-benzylamino)-phenol | C20H17Cl2NOS | D | 390.33 | 390.6 |
| 370 | 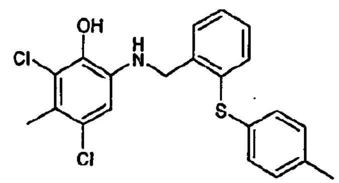 | 2,4-Dichloro-3-methyl-6-(2-p-tolylsulfanyl-benzylamino)-phenol | C21H19Cl2NOS | D | 404.35 | 404.6 |
| 371 | 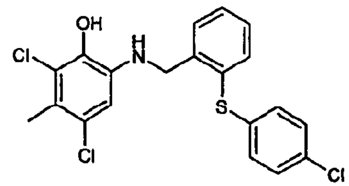 | 2,4-Dichloro-3-methyl-6-[2-(4-chloro-phenylsulfanyl)-benzylamino]-phenol | C20H16Cl3NOS | D | 424.77 | 425.2 |
| 372 | 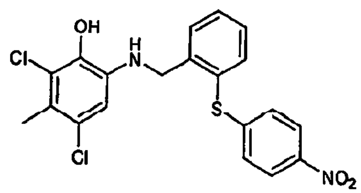 | 2,4-Dichloro-3-methyl-6-[2-(4-nitro-phenylsulfanyl)-benzylamino]-phenol | C20H16Cl2N2O3S | D | 435.32 | 435.8 |
| 373 | 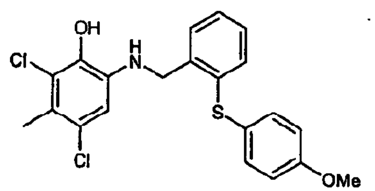 | 2,4-Dichloro-3-methyl-6-[2-(4-methoxy-phenylsulfanyl)-benzylamino]-phenol | C21H19Cl2NO2S | D | 420.35 | 420.6 |

| | | | | | | |
|---|---|---|---|---|---|---|
| 374 | 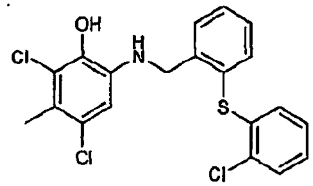 | 2,4-Dichloro-3-methyl-6-[2-(2-chloro-phenylsulfanyl)-benzylamino]-phenol | C20H16Cl3NOS | D | 424.77 | 425.1 |
| 375 | 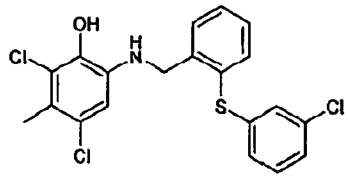 | 2,4-Dichloro-3-methyl-6-[2-(3-chloro-phenylsulfanyl)-benzylamino]-phenol | C20H16Cl3NOS | D | 424.77 | 425.1 |
| 376 | 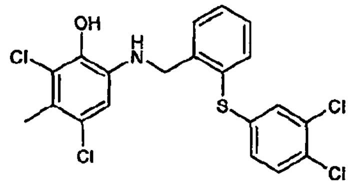 | 2,4-Dichloro-3-methyl-6-[2-(3,4-dichloro-phenylsulfanyl)-benzylamino]-phenol | C20H15Cl4NOS | D | 459.22 | 459.5 |
| 377 | 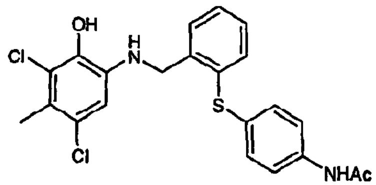 | N-(4-{2-[(3,5-Dichloro-2-hydroxy-4-methyl-phenylamino)-methyl]-phenylsulfanyl}-phenyl)-acetamide | C22H20Cl2N2O2S | D | 447.38 | 447.8 |
| 378 | 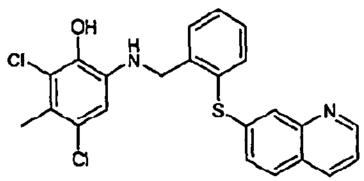 | 2,4-Dichloro-3-methyl-6-[2-(quinolin-7-ylsulfanyl)-benzylamino]-phenol | C23H18Cl2N2OS | D | 441.37 | 441.6 |

| | | | | | | |
|---|---|---|---|---|---|---|
| 379 | 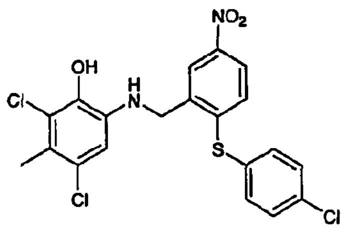 | 2,4-Dichloro-3-methyl-6-[2-(4-chloro-phenylsulfanyl)-5-nitro-benzylamino]-phenol | C20H15Cl3N2O3S | D | 469.77 | 470.0 |
| 380 | 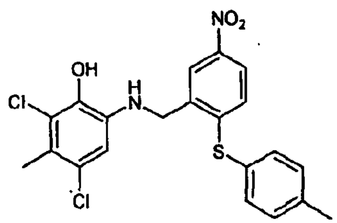 | 2,4-Dichloro-3-methyl-6-(5-nitro-2-p-tolylsulfanyl-benzylamino)-phenol | C21H18Cl2N2O3S | D | 449.35 | 449.6 |
| 381 | 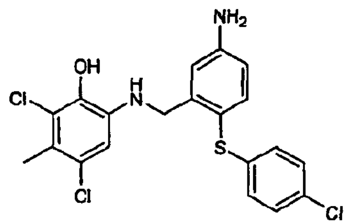 | 2-[5-Amino-2-(4-chloro-phenylsulfanyl)-benzylamino]-4,6-dichloro-5-methyl-phenol | C20H17Cl3N2OS | D | 439.79 | 439.9 |
| 382 | 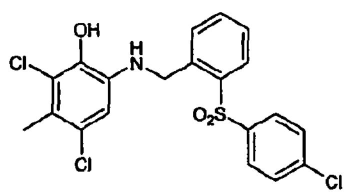 | 2,4-Dichloro-3-methyl-6-[2-(4-chloro-benzenesulfonyl)-benzylamino]-phenol | C20H16Cl3NO3S | D | 456.77 | 457.0 |

| | | | | | | |
|---|---|---|---|---|---|---|
| 383 | 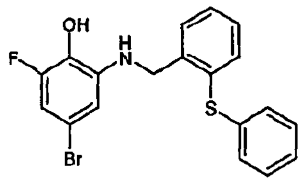 | 4-Bromo-2-fluoro-6-(2-phenylsulfanyl-benzylamino)-phenol | C19H15BrFNOS | D | 404.30 | 404.7 |
| 384 | 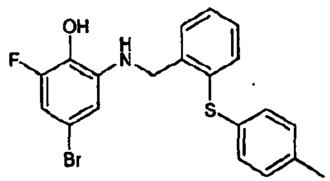 | 4-Bromo-2-fluoro-6-(2-p-tolylsulfanyl-benzylamino)-phenol | C20H17BrFNOS | D | 418.33 | 418.6 |
| 385 | 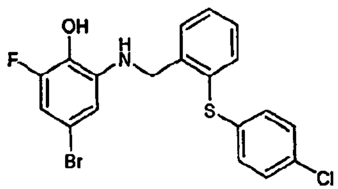 | 4-Bromo-2-[2-(4-chloro-phenylsulfanyl)-benzylamino]-6-fluoro-phenol | C19H14BrClFNOS | D | 438.74 | 439.0 |
| 386 | 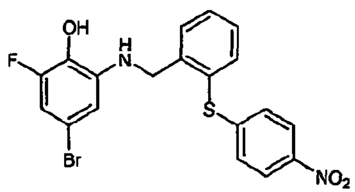 | 4-Bromo-2-fluoro-6-[2-(4-nitro-phenylsulfanyl)-benzylamino]-phenol | C19H14BrFN2O3S | D | 449.30 | 449.8 |
| 387 | 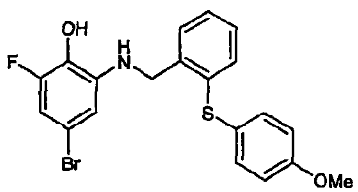 | 4-Bromo-2-fluoro-6-[2-(4-methoxy-phenylsulfanyl)-benzylamino]-phenol | C20H17BrFNO2S | D | 434.33 | 434.7 |

| | | | | | | |
|---|---|---|---|---|---|---|
| 388 | 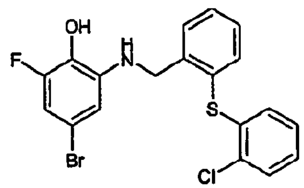 | 4-Bromo-2-[2-(2-chloro-phenylsulfanyl)-benzylamino]-6-fluoro-phenol | C19H14BrClFNOS | D | 438.74 | 439.2 |
| 389 | 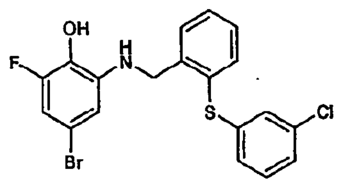 | 4-Bromo-2-[2-(3-chloro-phenylsulfanyl)-benzylamino]-6-fluoro-phenol | C19H14BrClFNOS | D | 438.74 | 439.1 |
| 390 | 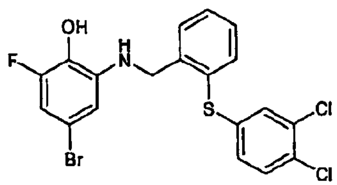 | 4-Bromo-2-[2-(3,4-dichloro-phenylsulfanyl)-benzylamino]-6-fluoro-phenol | C19H13BrCl2FNOS | D | 473.19 | 473.6 |
| 391 | 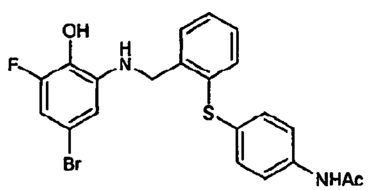 | N-(4-{2-[(5-Bromo-3-fluoro-2-hydroxy-phenylamino)-methyl]-phenylsulfanyl}-phenyl)-acetamide | C21H18BrFN2O2S | D | 461.35 | 461.8 |
| 392 | 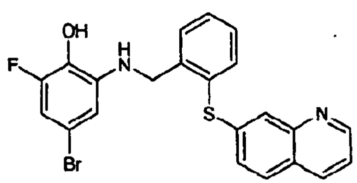 | 4-Bromo-2-fluoro-6-[2-(quinolin-7-ylsulfanyl)-benzylamino]-phenol | C22H16BrFN2OS | D | 455.35 | 455.7 |

| | | | | | | |
|---|---|---|---|---|---|---|
| 393 | 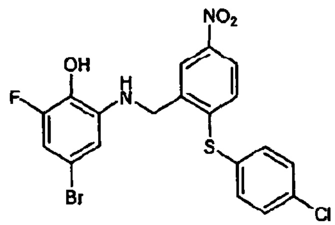 | 4-Bromo-2-[2-(4-chloro-phenylsulfanyl)-5-nitro-benzylamino]-6-fluoro-phenol | C19H13BrClFN2O3S | D | 483.74 | 484.1 |
| 394 | 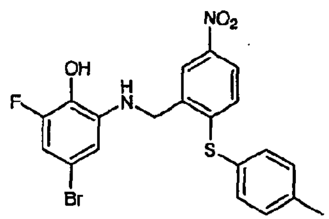 | 4-Bromo-2-fluoro-6-(5-nitro-2-p-tolylsulfanyl-benzylamino)-phenol | C20H16BrFN2O3S | D | 463.32 | 463.7 |
| 395 | 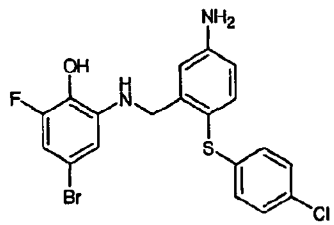 | 2-[5-Amino-2-(4-chloro-phenylsulfanyl)-benzylamino]-4-bromo-6-fluoro-phenol | C19H15BrClFN2OS | D | 453.76 | 454.1 |
| 396 | 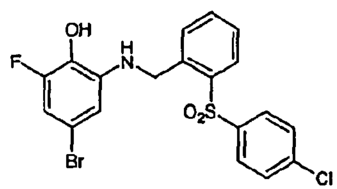 | 4-Bromo-2-[2-(4-chloro-benzenesulfonyl)-benzylamino]-6-fluoro-phenol | C19H14BrClFNO3S | D | 470.74 | 471.2 |

| | | | | | | |
|---|---|---|---|---|---|---|
| 397 | 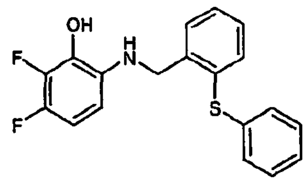 | 2,3-Difluoro-6-(2-phenylsulfanyl-benzylamino)-phenol | C19H15F2NOS | D | 343.39 | 343.6 |
| 398 | 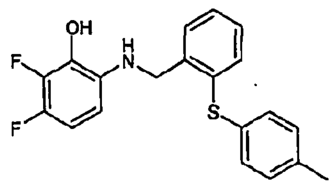 | 2,3-Difluoro-6-(2-p-tolylsulfanyl-benzylamino)-phenol | C20H17F2NOS | D | 357.42 | 357.8 |
| 399 | 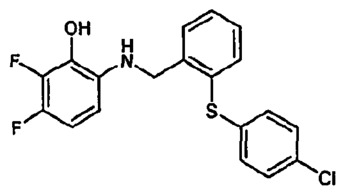 | 6-[2-(4-Chloro-phenylsulfanyl)-benzylamino]-2,3-difluoro-phenol | C19H14ClF2NOS | D | 377.83 | 378.1 |
| 400 | 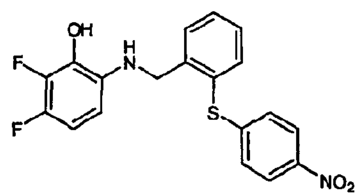 | 2,3-Difluoro-6-[2-(4-nitro-phenylsulfanyl)-benzylamino]-phenol | C19H14F2N2O3S | D | 388.39 | 388.8 |
| 401 | 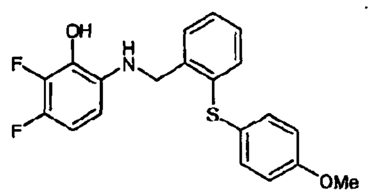 | 2,3-Difluoro-6-[2-(4-methoxy-phenylsulfanyl)-benzylamino]-phenol | C20H17F2NO2S | D | 373.42 | 373.7 |

| | | | | | | |
|---|---|---|---|---|---|---|
| 402 | 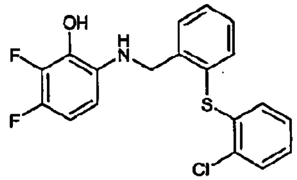 | 6-[2-(2-Chloro-phenylsulfanyl)-benzylamino]-2,3-difluoro-phenol | C19H14ClF2NOS | D | 377.83 | 378.1 |
| 403 | 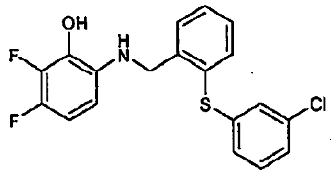 | 6-[2-(3-Chloro-phenylsulfanyl)-benzylamino]-2,3-difluoro-phenol | C19H14ClF2NOS | D | 377.83 | 378.2 |
| 404 | 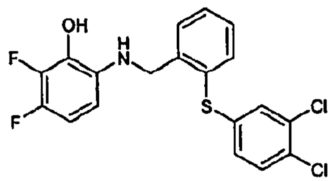 | 6-[2-(3,4-Dichloro-phenylsulfanyl)-benzylamino]-2,3-difluoro-phenol | C19H13Cl2F2NOS | D | 412.28 | 412.6 |
| 405 | 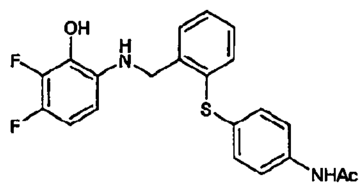 | N-(4-{2-[(3,4-Difluoro-2-hydroxy-phenylamino)-methyl]-phenylsulfanyl}-phenyl)-acetamide | C21H18F2N2O2S | D | 400.44 | 400.8 |
| 406 | 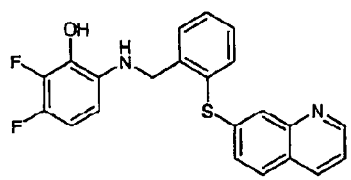 | 2,3-Difluoro-6-[2-(quinolin-7-ylsulfanyl)-benzylamino]-phenol | C22H16F2N2OS | D | 394.44 | 394.9 |

| | | | | | | |
|---|---|---|---|---|---|---|
| 407 | 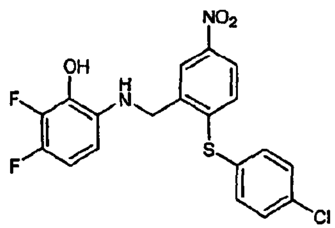 | 6-[2-(4-Chloro-phenylsulfanyl)-5-nitro-benzylamino]-2,3-difluoro-phenol | C19H13ClF2N2O3S | D | 422.83 | 423.3 |
| 408 | 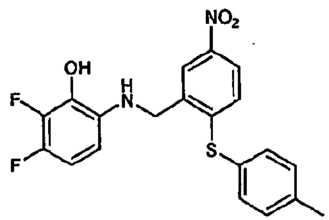 | 2,3-Difluoro-6-(5-nitro-2-p-tolylsulfanyl-benzylamino)-phenol | C20H16F2N2O3S | D | 402.41 | 402.8 |
| 409 | 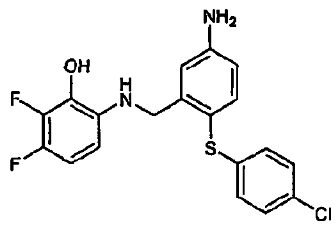 | 6-[5-Amino-2-(4-chloro-phenylsulfanyl)-benzylamino]-2,3-difluoro-phenol | C19H15ClF2N2OS | D | 392.85 | 393.0 |
| 410 | 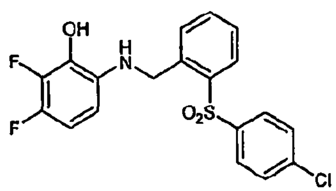 | 6-[2-(4-Chloro-benzenesulfonyl)-benzylamino]-2,3-difluoro-phenol | C19H14ClF2NO3S | D | 391.84 | 392.3 |

| | | | | | | |
|---|---|---|---|---|---|---|
| 411 | 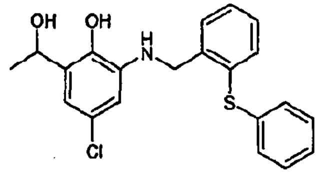 | 4-Chloro-2-(1-hydroxy-ethyl)-6-(2-phenylsulfanyl-benzylamino)-phenol | C21H20ClNO2S | D | 385.91 | 386.4 |
| 412 | 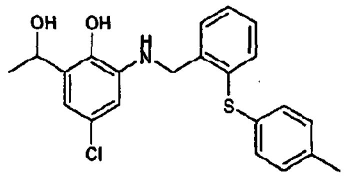 | 4-Chloro-2-(1-hydroxy-ethyl)-6-(2-p-tolylsulfanyl-benzylamino)-phenol | C22H22ClNO2S | D | 399.93 | 400.2 |
| 413 | 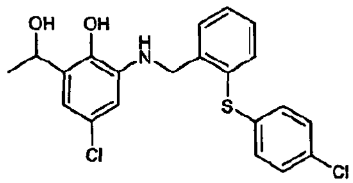 | 4-Chloro-2-[2-(4-chloro-phenylsulfanyl)-benzylamino]-6-(1-hydroxy-ethyl)-phenol | C21H19Cl2NO2S | D | 420.35 | 420.9 |
| 414 | 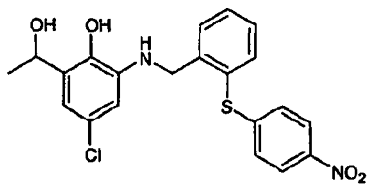 | 4-Chloro-2-(1-hydroxy-ethyl)-6-[2-(4-nitro-phenylsulfanyl)-benzylamino]-phenol | C21H19ClN2O4S | D | 430.90 | 431.3 |
| 415 | 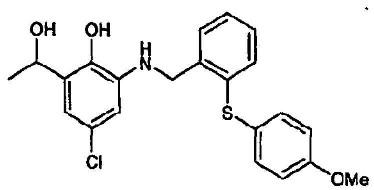 | 4-Chloro-2-(1-hydroxy-ethyl)-6-[2-(4-methoxy-phenylsulfanyl)-benzylamino]-phenol | C22H22ClNO3S | D | 415.93 | 416.3 |

| | | | | | | |
|---|---|---|---|---|---|---|
| 416 | 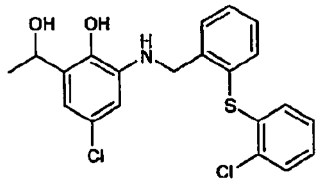 | 4-Chloro-2-[2-(2-chloro-phenylsulfanyl)-benzylamino]-6-(1-hydroxy-ethyl)-phenol | C21H19Cl2NO2S | D | 420.35 | 420.9 |
| 417 | 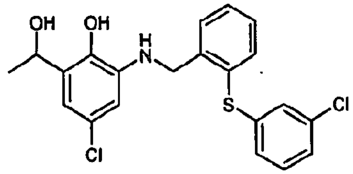 | 4-Chloro-2-[2-(3-chloro-phenylsulfanyl)-benzylamino]-6-(1-hydroxy-ethyl)-phenol | C21H19Cl2NO2S | D | 420.35 | 420.8 |
| 418 | 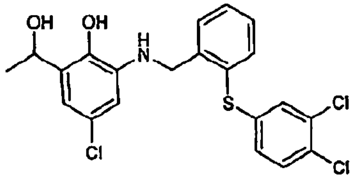 | 4-Chloro-2-[2-(3,4-dichloro-phenylsulfanyl)-benzylamino]-6-(1-hydroxy-ethyl)-phenol | C21H18Cl3NO2S | D | 454.80 | 455.2 |
| 419 | 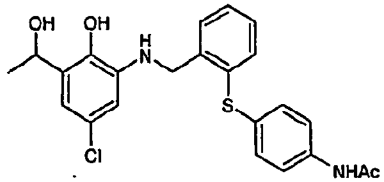 | N-[4-(2-{[5-Chloro-2-hydroxy-3-(1-hydroxy-ethyl)-phenylamino]-methyl}-phenylsulfanyl)-phenyl]-acetamide | C23H23ClN2O3S | D | 442.96 | 443.4 |
| 420 | 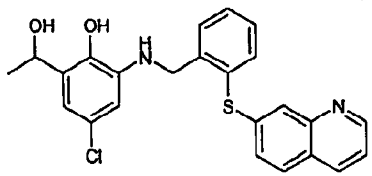 | 4-Chloro-2-(1-hydroxy-ethyl)-6-[2-(quinolin-7-ylsulfanyl)-benzylamino]-phenol | C24H21ClN2O2S | D | 436.95 | 437.3 |

| | | | | | | |
|---|---|---|---|---|---|---|
| 421 | 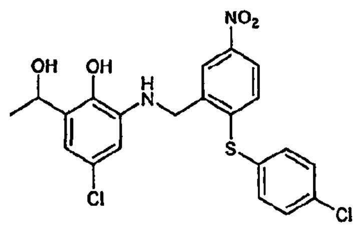 | 4-Chloro-2-[2-(4-chloro-phenylsulfanyl)-5-nitro-benzylamino]-6-(1-hydroxy-ethyl)-phenol | C21H18Cl2N2O4S | D | 465.35 | 465.9 |
| 422 | 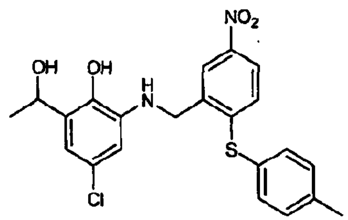 | 4-Chloro-2-(1-hydroxy-ethyl)-6-(5-nitro-2-p-tolylsulfanyl-benzylamino)-phenol | C22H21ClN2O4S | D | 444.93 | 445.3 |
| 423 | 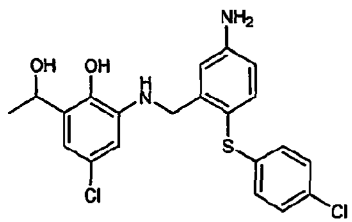 | 2-[5-Amino-2-(4-chloro-phenylsulfanyl)-benzylamino]-4-chloro-6-(1-hydroxy-ethyl)-phenol | C21H20Cl2N2O2S | D | 435.37 | 435.6 |
| 424 | 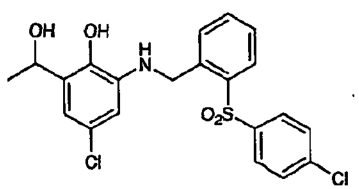 | 4-Chloro-2-[2-(4-chloro-benzenesulfonyl)-benzylamino]-6-(1-hydroxy-ethyl)-phenol | C21H19Cl2NO4S | D | 452.35 | 452.5 |

| | | | | | | |
|---|---|---|---|---|---|---|
| 425 | 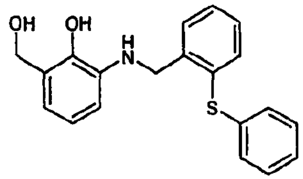 | 2-Hydroxymethyl-6-(2-phenylsulfanyl-benzylamino)-phenol | C20H19NO2S | D | 337.44 | 337.8 |
| 426 | 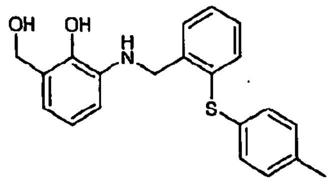 | 2-Hydroxymethyl-6-(2-p-tolylsulfanyl-benzylamino)-phenol | C21H21NO2S | D | 351.46 | 351.8 |
| 427 | 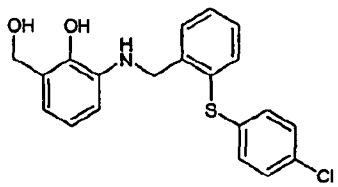 | 2-[2-(4-Chloro-phenylsulfanyl)-benzylamino]-6-hydroxymethyl-phenol | C20H18ClNO2S | D | 371.88 | 371.9 |
| 428 | 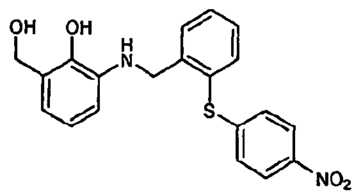 | 2-Hydroxymethyl-6-[2-(4-nitro-phenylsulfanyl)-benzylamino]-phenol | C20H18N2O4S | D | 382.43 | 382.7 |
| 429 | 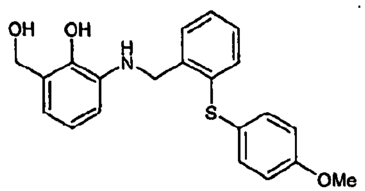 | 2-Hydroxymethyl-6-[2-(4-methoxy-phenylsulfanyl)-benzylamino]-phenol | C21H21NO3S | D | 367.46 | 367.9 |

| | | | | | | |
|---|---|---|---|---|---|---|
| 430 | 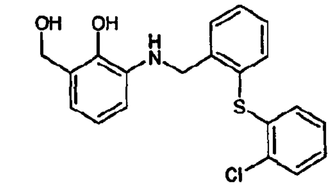 | 2-[2-(2-Chloro-phenylsulfanyl)-benzylamino]-6-hydroxymethyl-phenol | C20H18ClNO2S | D | 371.88 | 371.9 |
| 431 | 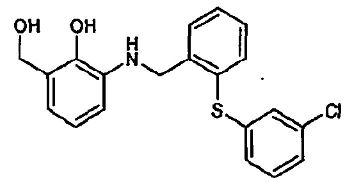 | 2-[2-(3-Chloro-phenylsulfanyl)-benzylamino]-6-hydroxymethyl-phenol | C20H18ClNO2S | D | 371.88 | 371.9 |
| 432 | 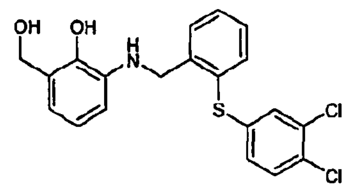 | 2-[2-(3,4-Dichloro-phenylsulfanyl)-benzylamino]-6-hydroxymethyl-phenol | C20H17Cl2NO2S | D | 406.33 | 406.5 |
| 433 | 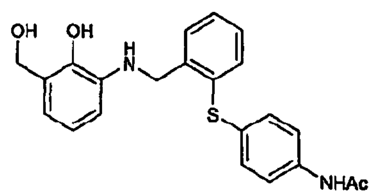 | N-(4-{2-[(2-Hydroxy-3-hydroxymethyl-phenylamino)-methyl]-phenylsulfanyl}-phenyl)-acetamide | C22H22N2O3S | D | 394.49 | 349.9 |
| 434 | 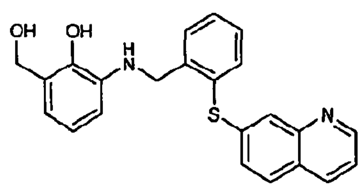 | 2-Hydroxymethyl-6-[2-(quinolin-7-ylsulfanyl)-benzylamino]-phenol | C23H20N2O2S | D | 388.48 | 388.8 |

| | | | | | | |
|---|---|---|---|---|---|---|
| 435 | 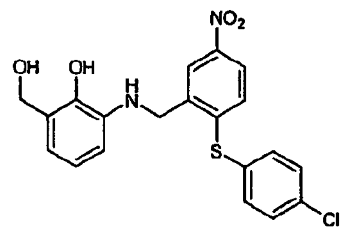 | 2-[2-(4-Chloro-phenylsulfanyl)-5-nitro-benzylamino]-6-hydroxymethyl-phenol | C20H17ClN2O4S | D | 416.88 | 417.2 |
| 436 | 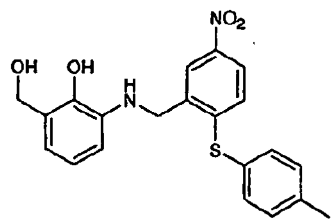 | 2-Hydroxymethyl-6-(5-nitro-2-p-tolylsulfanyl-benzylamino)-phenol | C21H20N2O4S | D | 396.46 | 396.8 |
| 437 | 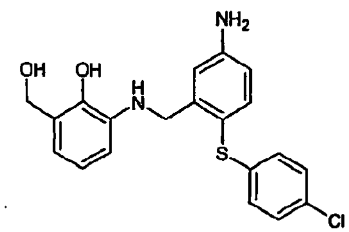 | 2-[5-Amino-2-(4-chloro-phenylsulfanyl)-benzylamino]-6-hydroxymethyl-phenol | C20H19ClN2O2S | D | 386.90 | 387.1 |
| 438 | 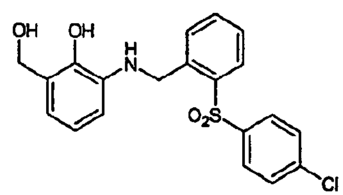 | 2-[2-(4-Chloro-benzenesulfonyl)-benzylamino]-6-hydroxymethyl-phenol | C20H18ClNO4S | D | 403.88 | 44.0 |

| | | | | | | |
|---|---|---|---|---|---|---|
| 439 | 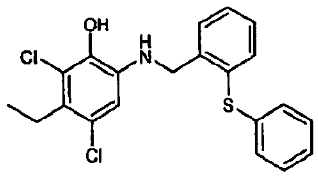 | 2,4-Dichloro-3-ethyl-6-(2-phenylsulfanyl-benzylamino)-phenol | C21H19Cl2NOS | D | 390.33 | 390.5 |
| 440 | 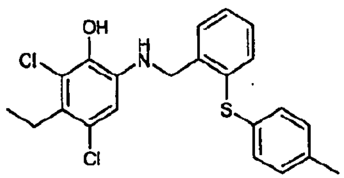 | 2,4-Dichloro-3-ethyl-6-(2-p-tolylsulfanyl-benzylamino)-phenol | C22H21Cl2NOS | D | 418.37 | 418.7 |
| 441 | 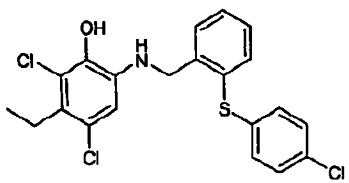 | 2,4-Dichloro-3-ethyl-6-[2-(4-chloro-phenylsulfanyl)-benzylamino]-phenol | C21H18Cl3NOS | D | 438.79 | 439.0 |
| 442 | 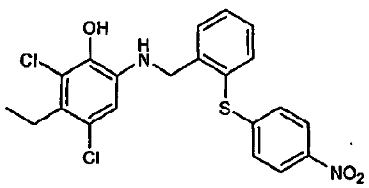 | 2,4-Dichloro-3-ethyl-6-[2-(4-nitro-phenylsulfanyl)-benzylamino]-phenol | C21H18Cl2N2O3S | D | 449.34 | 449.7 |
| 443 | 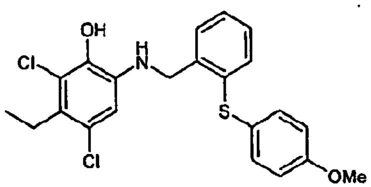 | 2,4-Dichloro-3-ethyl-6-[2-(4-methoxy-phenylsulfanyl)-benzylamino]-phenol | C22H21Cl2NO2S | D | 434.37 | 434.6 |

| | | | | | | |
|---|---|---|---|---|---|---|
| 444 | 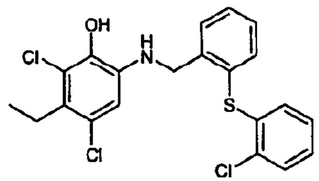 | 2,4-Dichloro-3-ethyl-6-[2-(2-chloro-phenylsulfanyl)-benzylamino]-phenol | C21H18Cl3NOS | D | 438.79 | 438.9 |
| 445 | 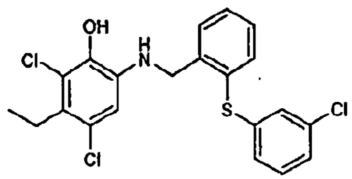 | 2,4-Dichloro-3-ethyl-6-[2-(3-chloro-phenylsulfanyl)-benzylamino]-phenol | C21H18Cl3NOS | D | 438.79 | 438.9 |
| 446 | 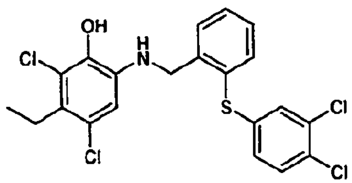 | 2,4-Dichloro-3-ethyl-6-[2-(3,4-dichloro-phenylsulfanyl)-benzylamino]-phenol | C21H17Cl4NOS | D | 473.24 | 473.5 |
| 447 | 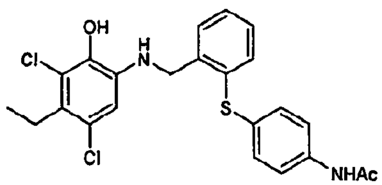 | N-(4-{2-[(3,5-Dichloro-4-ethyl-2-hydroxy-phenylamino)-methyl]-phenylsulfanyl}-phenyl)-acetamide | C23H22Cl2N2O2S | D | 461.40 | 461.6 |
| 448 | 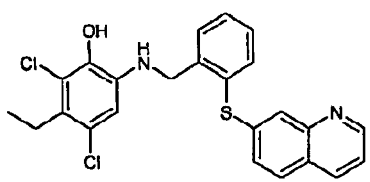 | 2,4-Dichloro-3-ethyl-6-[2-(quinolin-7-ylsulfanyl)-benzylamino]-phenol | C24H20Cl2N2OS | D | 455.39 | 455.7 |

| | | | | | | |
|---|---|---|---|---|---|---|
| 449 | 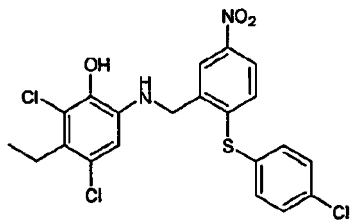 | 2,4-Dichloro-3-ethyl-6-[2-(4-chloro-phenylsulfanyl)-5-nitro-benzylamino]-phenol | C21H17Cl3N2O3S | D | 483.79 | 484.0 |
| 450 | 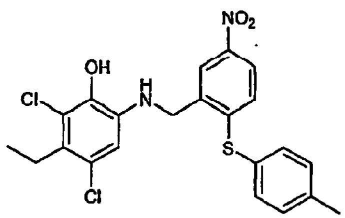 | 2,4-Dichloro-3-ethyl-6-(5-nitro-2-p-tolylsulfanyl-benzylamino)-phenol | C22H20Cl2N2O3S | D | 463.37 | 463.5 |
| 451 | 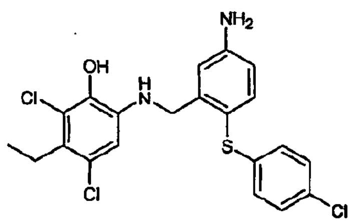 | 2-[5-Amino-2-(4-chloro-phenylsulfanyl)-benzylamino]-4,6-dichloro-5-ethyl-phenol | C21H19Cl3N2OS | D | 453.81 | 454.1 |
| 452 | 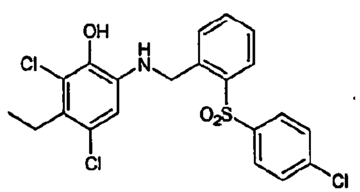 | 2,4-Dichloro-3-ethyl-6-[2-(4-chloro-benzenesulfonyl)-benzylamino]-phenol | C21H18Cl3NO3S | D | 470.79 | 471.0 |

| | | | | | | |
|---|---|---|---|---|---|---|
| 453 | 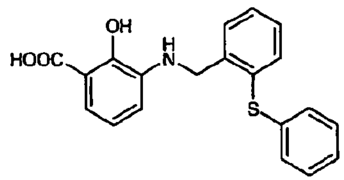 | 2-Hydroxy-3-(2-phenylsulfanyl-benzylamino)-benzoic acid | C20H17NO3S | D | 351.42 | 351.6 |
| 454 | 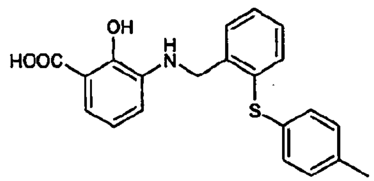 | 2-Hydroxy-3-(2-p-tolylsulfanyl-benzylamino)-benzoic acid | C21H19NO3S | D | 365.44 | 365.8 |
| 455 | 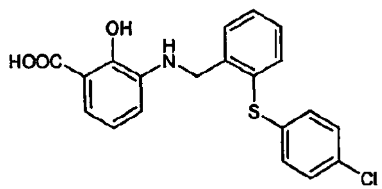 | 3-[2-(4-Chloro-phenylsulfanyl)-benzylamino]-2-hydroxy-benzoic acid | C20H16ClNO3S | D | 385.86 | 385.1 |
| 456 | 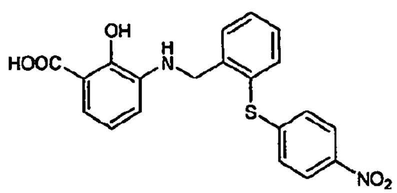 | 2-Hydroxy-3-[2-(4-nitro-phenylsulfanyl)-benzylamino]-benzoic acid | C20H16N2O5S | D | 396.11 | 396.4 |
| 457 | 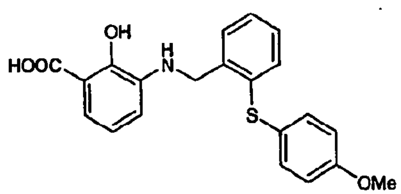 | 2-Hydroxy-3-[2-(4-methoxy-phenylsulfanyl)-benzylamino]-benzoic acid | C21H19NO4S | D | 381.44 | 381.6 |

| | | | | | | |
|---|---|---|---|---|---|---|
| 458 | 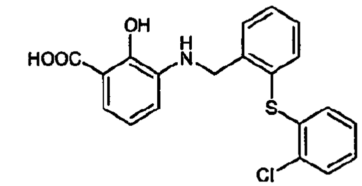 | 3-[2-(2-Chloro-phenylsulfanyl)-benzylamino]-2-hydroxy-benzoic acid | C20H16ClNO3S | D | 385.86 | 386.0 |
| 459 | 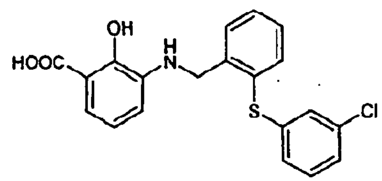 | 3-[2-(3-Chloro-phenylsulfanyl)-benzylamino]-2-hydroxy-benzoic acid | C20H16ClNO3S | D | 385.86 | 386.0 |
| 460 | 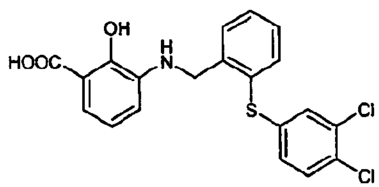 | 3-[2-(3,4-Dichloro-phenylsulfanyl)-benzylamino]-2-hydroxy-benzoic acid | C20H15Cl2NO3S | D | 420.31 | 420.7 |
| 461 | 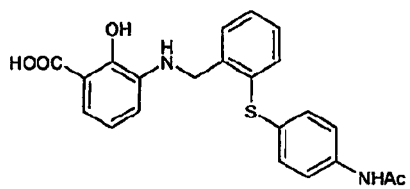 | 3-[2-(4-Acetylamino-phenylsulfanyl)-benzylamino]-2-hydroxy-benzoic acid | C22H20N2O4S | D | 408.47 | 408.7 |
| 462 | 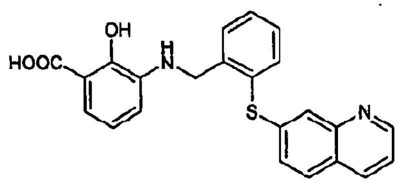 | 2-Hydroxy-3-[2-(quinolin-7-ylsulfanyl)-benzylamino]-benzoic acid | C23H18N2O3S | D | 402.46 | 402.8 |

| | | | | | | |
|---|---|---|---|---|---|---|
| 463 | 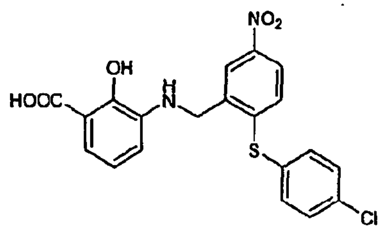 | 3-[2-(4-Chloro-phenylsulfanyl)-5-nitro-benzylamino]-2-hydroxy-benzoic acid | C20H15ClN2O5S | D | 430.86 | 430.9 |
| 464 | 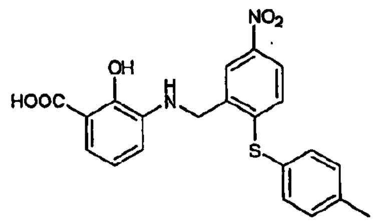 | 2-Hydroxy-3-(5-nitro-2-p-tolylsulfanyl-benzylamino)-benzoic acid | C21H18N2O5S | D | 410.44 | 410.7 |
| 465 | 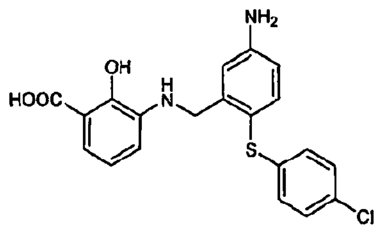 | 2-[5-Amino-2-(4-chloro-phenylsulfanyl)-benzylamino]-6-hydroxymethyl-phenol | C20H19ClN2O2S | D | 386.90 | 387.2 |
| 466 | 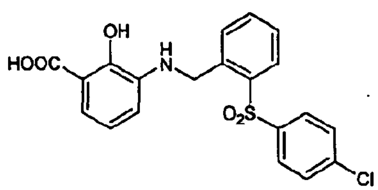 | 3-[2-(4-Chloro-benzenesulfonyl)-benzylamino]-2-hydroxy-benzoic acid | C20H16ClNO5S | D | 403.88 | 404.1 |

| | | | | | | |
|---|---|---|---|---|---|---|
| 467 | 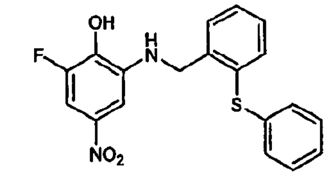 | 2-Fluoro-4-nitro-6-(2-phenylsulfanyl-benzylamino)-phenol | C19H15FN2O3S | D | 370.40 | 370.8 |
| 468 | 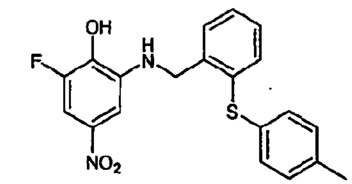 | 2-Fluoro-4-nitro-6-(2-p-tolylsulfanyl-benzylamino)-phenol | C20H17FN2O3S | D | 384.43 | 384.9 |
| 469 | 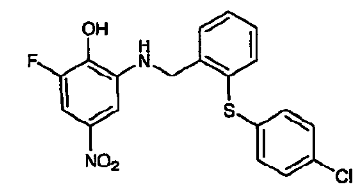 | 2-[2-(4-Chloro-phenylsulfanyl)-benzylamino]-6-fluoro-4-nitro-phenol | C19H14ClFN2O3S | D | 404.84 | 405.0 |
| 470 | 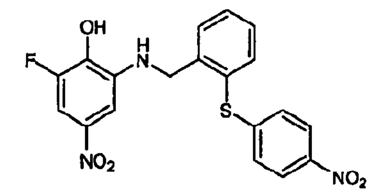 | 2-Fluoro-4-nitro-6-[2-(4-nitro-phenylsulfanyl)-benzylamino]-phenol | C19H14FN3O5S | D | 415.40 | 415.8 |
| 471 | 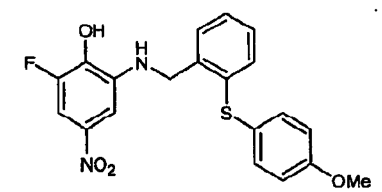 | 2-Fluoro-6-[2-(4-methoxy-phenylsulfanyl)-benzylamino]-4-nitro-phenol | C20H17FN2O4S | D | 400.43 | 400.9 |

| | | | | | | |
|---|---|---|---|---|---|---|
| 472 | 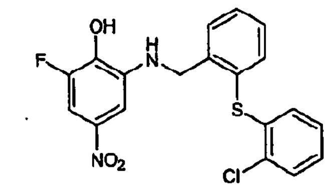 | 2-[2-(2-Chloro-phenylsulfanyl)-benzylamino]-6-fluoro-4-nitro-phenol | C19H14ClFN2O3S | D | 404.84 | 405.0 |
| 473 | 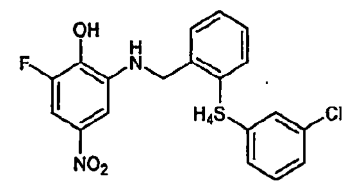 | 2-[2-(3-Chloro-phenylsulfanyl)-benzylamino]-6-fluoro-4-nitro-phenol | C19H14ClFN2O3S | D | 404.84 | 405.0 |
| 474 | 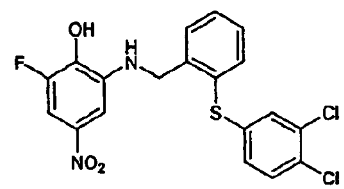 | 2-[2-(3,4-Dichloro-phenylsulfanyl)-benzylamino]-6-fluoro-4-nitro-phenol | C19H13Cl2FN2O3S | D | 439.29 | 439.9 |
| 475 | 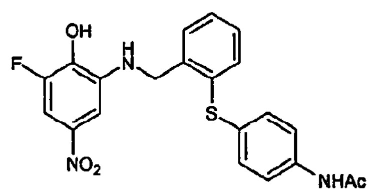 | N-(4-{2-[(3-Fluoro-2-hydroxy-5-nitro-phenylamino)-methyl]-phenylsulfanyl}-phenyl)-acetamide | C21H18FN3O4S | D | 427.45 | 427.9 |
| 476 | 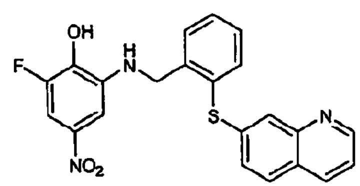 | 2-Fluoro-4-nitro-6-[2-(quinolin-7-ylsulfanyl)-benzylamino]-phenol | C22H16FN3O3S | D | 421.45 | 421.6 |

| | | | | | | |
|---|---|---|---|---|---|---|
| 477 | 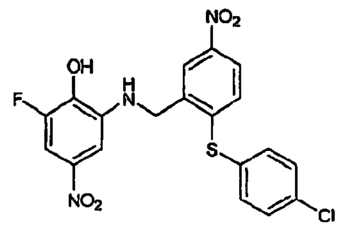 | 2-[2-(4-Chloro-phenylsulfanyl)-5-nitro-benzylamino]-6-fluoro-4-nitro-phenol | C19H13ClFN3O5S | D | 449.84 | 450.1 |
| 478 | 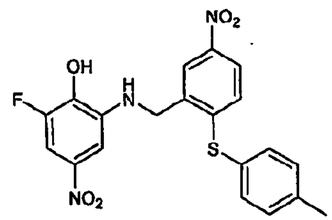 | 2-Fluoro-4-nitro-6-(5-nitro-2-p-tolylsulfanyl-benzylamino)-phenol | C20H16FN3O4S | D | 429.42 | 429.8 |
| 479 | 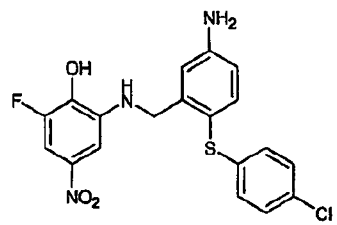 | 2-[5-Amino-2-(4-chloro-phenylsulfanyl)-benzylamino]-6-fluoro-4-nitro-phenol | C19H15ClFN3O2S | D | 419.86 | 420.2 |
| 480 | 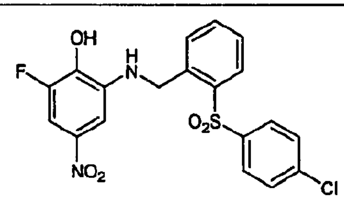 | 2-[2-(4-Chloro-benzenesulfonyl)-benzylamino]-6-fluoro-4-nitro-phenol | C19H15ClFN2O5S | D | 436.84 | 437.1 |

| | | | | | | |
|---|---|---|---|---|---|---|
| 481 | 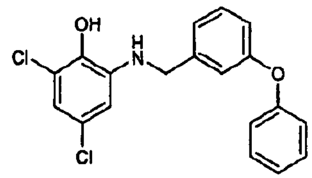 | 2,4-Dichloro-6-(3-phenoxy-benzylamino)-phenol | C19H15Cl2NO2 | D | 360.23 | 360.5 |
| 482 | 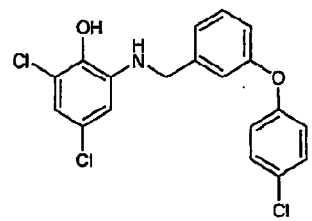 | 2,4-Dichloro-6-[3-(4-chloro-phenoxy)-benzylamino]-phenol | C19H15Cl3NO2 | D | 394.68 | 395.0 |
| 483 | 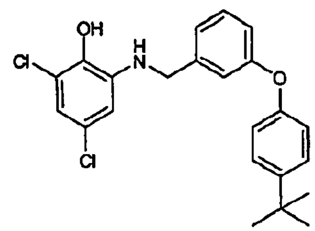 | 2-[3-(4-tert-Butyl-phenoxy)-benzylamino]-4,6-dichloro-phenol | C23H23Cl2NO2 | D | 416.34 | 416.8 |
| 484 | 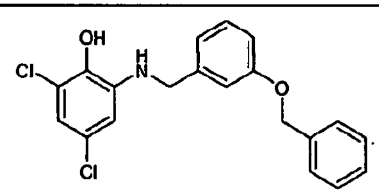 | 2-(3-Benzyloxy-benzylamino)-4,6-dichloro-phenol | C20H17Cl2NO2 | D | 374.26 | 374.6 |

| | | | | | | |
|---|---|---|---|---|---|---|
| 485 | 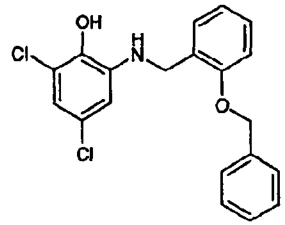 | 2-(2-Benzyloxy-benzylamino)-4,6-dichloro-phenol | C20H17Cl2NO2 | D | 374.26 | 374.6 |
| 486 | 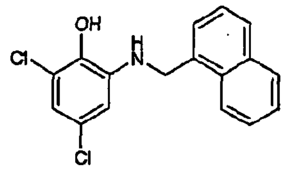 | 2,4-Dichloro-6-[(naphthalen-1-ylmethyl)-amino]-phenol | C17H13Cl2NO | D | 318.20 | 318.6 |
| 487 | 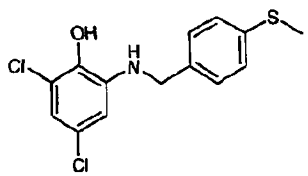 | 2,4-Dichloro-6-(4-methylsulfanyl-benzylamino)-phenol | C14H13Cl2NOS | D | 314.23 | 314.7 |
| 488 | 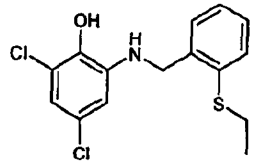 | 2,4-Dichloro-6-(2-ethylsulfanyl-benzylamino)-phenol | C15H15Cl2NOS | D | 328.26 | 328.6 |
| 489 | 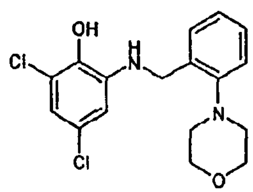 | 2,4-Dichloro-6-(2-morpholin-4-yl-benzylamino)-phenol | C17H18Cl2N2O2 | D | 353.24 | 353.6 |

| | | | | | | |
|---|---|---|---|---|---|---|
| 490 | 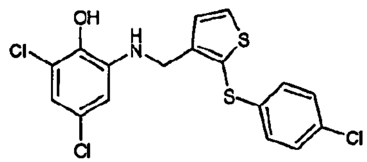 | 2,4-Dichloro-6-{[2-(4-chloro-phenylsulfanyl)-thiophen-3-ylmethyl]-amino}-phenol | C17H12Cl3NOS2 | D | 416.77 | 416.9 |
| 491 | 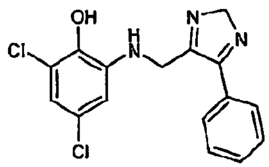 | 2,4-Dichloro-6-[(5-phenyl-2H-imidazol-4-ylmethyl)-amino]-phenol | C16H13Cl2N3O | D | 334.20 | 334.6 |
| 492 | 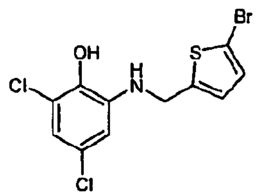 | 2-[(5-Bromo-thiophen-2-ylmethyl)-amino]-4,6-dichloro-phenol | C11H8BrCl2NOS | D | 353.06 | 353.4 |
| 493 | 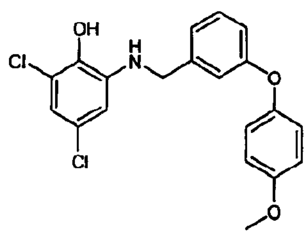 | 2,4-Dichloro-6-[3-(4-methoxy-phenoxy)-benzylamino]-phenol | C20H17Cl2NO3 | D | 390.26 | 390.6 |
| 494 | 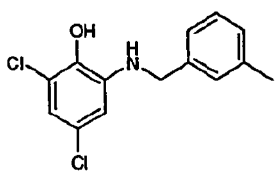 | 2,4-Dichloro-6-(3-methyl-benzylamino)-phenol | C14H13Cl2NO | D | 282.17 | 282.4 |

| | | | | | | |
|---|---|---|---|---|---|---|
| 495 | 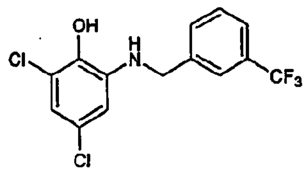 | 2,4-Dichloro-6-(3-trifluoromethyl-benzylamino)-phenol | C14H10Cl2F3NO | D | 336.14 | 336.5 |
| 496 | 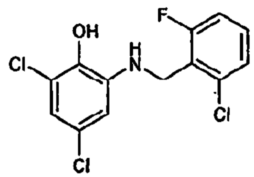 | 2,4-Dichloro-6-(2-chloro-6-fluoro-benzylamino)-phenol | C13H9Cl3FNO | D | 320.57 | 320.8 |
| 497 | 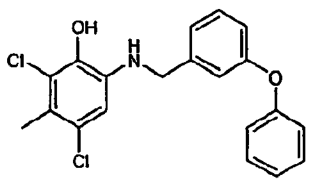 | 2,4-Dichloro-3-methyl-6-(3-phenoxy-benzylamino)-phenol | C20H17Cl2NO2 | D | 374.26 | 374.6 |
| 498 | 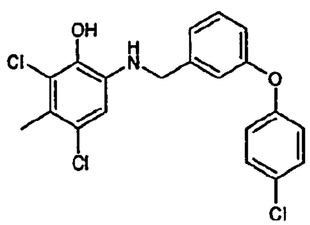 | 2,4-Dichloro-3-methyl-6-[3-(4-chloro-phenoxy)-benzylamino]-phenol | C20H17Cl3NO2 | D | 408.71 | 409.2 |

| | | | | | | |
|---|---|---|---|---|---|---|
| 499 | 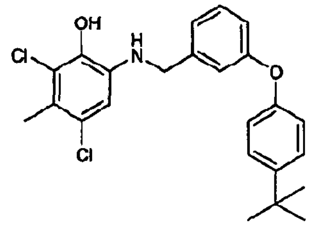 | 2-[3-(4-tert-Butyl-phenoxy)-benzylamino]-4,6-dichloro-3-methyl-phenol | C24H25Cl2NO2 | D | 430.37 | 430.5 |
| 500 | 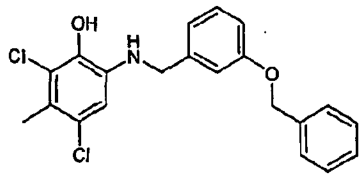 | 2-(3-Benzyloxy-benzylamino)-4,6-dichloro-3-methyl-phenol | C21H19Cl2NO2 | D | 388.29 | 388.6 |
| 501 | 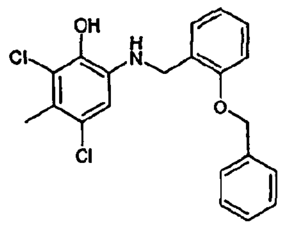 | 2-(2-Benzyloxy-benzylamino)-4,6-dichloro-3-methyl-phenol | C21H19Cl2NO2 | D | 388.29 | 388.6 |
| 502 | 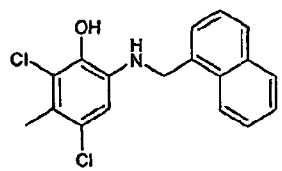 | 2,4-Dichloro-3-methyl-6-[(naphthalen-1-ylmethyl)-amino]-phenol | C18H15Cl2NO | D | 332.23 | 332.6 |

| | | | | | | |
|---|---|---|---|---|---|---|
| 503 | 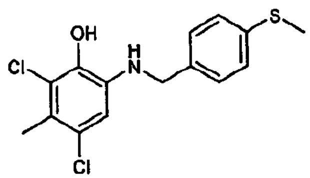 | 2,4-Dichloro-3-methyl-6-(4-methylsulfanyl-benzylamino)-phenol | C15H15Cl2NOS | D | 328.26 | 328.6 |
| 504 | 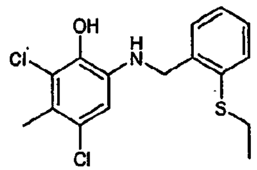 | 2,4-Dichloro-3-methyl-6-(2-ethylsulfanyl-benzylamino)-phenol | C16H17Cl2NOS | D | 342.29 | 342.8 |
| 505 | 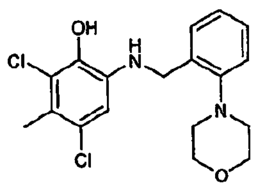 | 2,4-Dichloro-3-methyl-6-(2-morpholin-4-yl-benzylamino)-phenol | C18H20Cl2N2O2 | D | 367.27 | 367.6 |
| 506 | 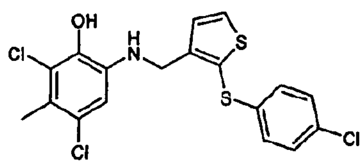 | 2,4-Dichloro-3-methyl-6-{[2-(4-chloro-phenylsulfanyl)-thiophen-3-ylmethyl]-amino}-phenol | C18H14Cl3NOS2 | D | 430.80 | 431.2 |
| 507 | 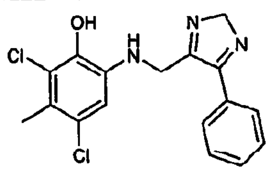 | 2,4-Dichloro-3-methyl-6-[(5-phenyl-2H-imidazol-4-ylmethyl)-amino]-phenol | C17H15Cl2N3O | D | 348.23 | 348.8 |

| | | | | | | |
|---|---|---|---|---|---|---|
| 508 | 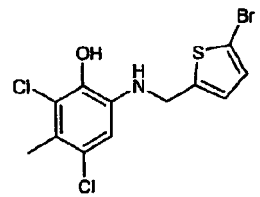 | 2-[(5-Bromo-thiophen-2-ylmethyl)-amino]-4,6-dichloro-3-methyl-phenol | C12H10BrCl2NOS | D | 367.09 | 367.4 |
| 509 | 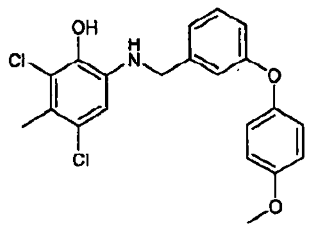 | 2,4-Dichloro-6-[3-(4-methoxy-phenoxy)-benzylamino]-3-methyl-phenol | C21H19Cl2NO3 | D | 404.29 | 404.5 |
| 510 | 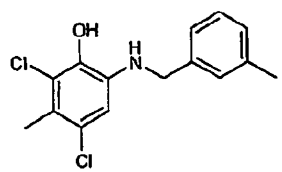 | 2,4-Dichloro-6-(3-methyl-benzylamino)-3-methyl-phenol | C15H15Cl2NO | D | 296.20 | 296.8 |
| 511 | 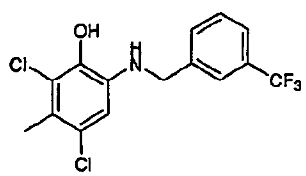 | 2,4-Dichloro-3-methyl-6-(3-trifluoromethyl-benzylamino)-phenol | C15H12Cl2F3NO | D | 350.17 | 350.7 |
| 512 | 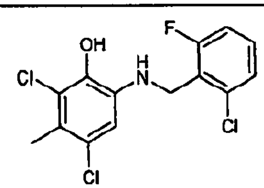 | 2,4-Dichloro-3-methyl-6-(2-chloro-6-fluoro-benzylamino)-phenol | C14H11Cl3FNO | D | 334.60 | 335.0 |

| | | | | | | |
|---|---|---|---|---|---|---|
| 513 | 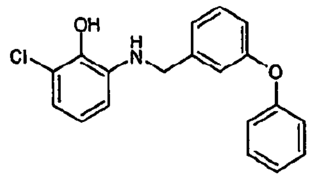 | 2-Chloro-6-(3-phenoxy-benzylamino)-phenol | C19H16ClNO2 | D | 325.79 | 326.1 |
| 514 | 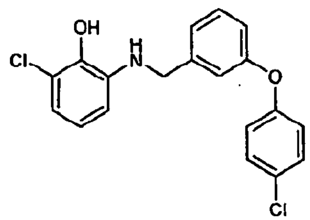 | 2-Chloro-6-[3-(4-chloro-phenoxy)-benzylamino]-phenol | C19H15Cl2NO2 | D | 360.23 | 360.6 |
| 515 | 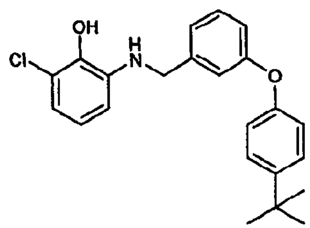 | 2-[3-(4-tert-Butyl-phenoxy)-benzylamino]-6-chloro-phenol | C23H24ClNO2 | D | 381.90 | 382.2 |
| 516 | 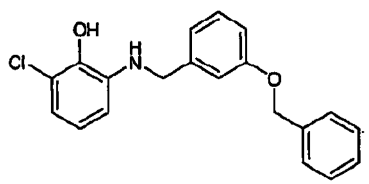 | 2-(3-Benzyloxy-benzylamino)-6-chloro-phenol | C20H18ClNO2 | D | 339.82 | 340.1 |

| | | | | | | |
|---|---|---|---|---|---|---|
| 517 | 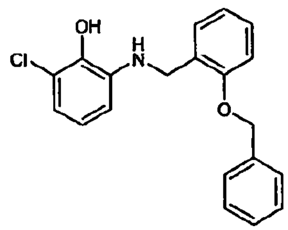 | 2-(2-Benzyloxy-benzylamino)-6-chloro-phenol | C20H18ClNO2 | D | 339.82 | 340.1 |
| 518 | 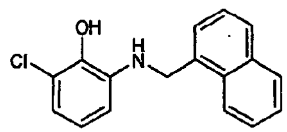 | 2-Chloro-6-[(naphthalen-1-ylmethyl)-amino]-phenol | C17H14ClNO | D | 283.75 | 284.0 |
| 519 | 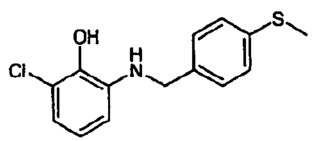 | 2-Chloro-6-(4-methylsulfanyl-benzylamino)-phenol | C14H14ClNOS | D | 279.79 | 280.2 |
| 520 | 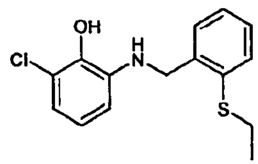 | 2-Chloro-6-(2-ethylsulfanyl-benzylamino)-phenol | C15H16ClNOS | D | 293.81 | 294.3 |
| 521 | 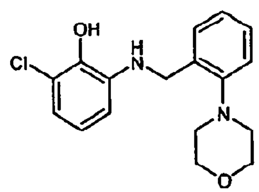 | 2-Chloro-6-(2-morpholin-4-yl-benzylamino)-phenol | C17H19ClN2O2 | D | 318.80 | 319.1 |

| | | | | | | |
|---|---|---|---|---|---|---|
| 522 | 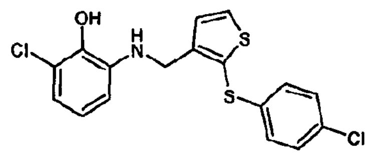 | 2-Chloro-6-{[2-(4-chloro-phenylsulfanyl)-thiophen-3-ylmethyl]-amino}-phenol | C17H13Cl2NOS2 | D | 382.33 | 382.6 |
| 523 | 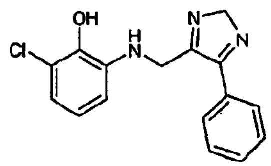 | 2-Chloro-6-[(5-phenyl-2H-imidazol-4-ylmethyl)-amino]-phenol | C16H14ClN3O | D | 299.75 | 300.2 |
| 524 | 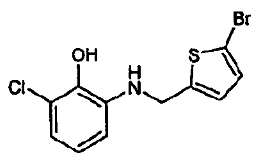 | 2-[(5-Bromo-thiophen-2-ylmethyl)-amino]-6-chloro-phenol | C11H9BrClNOS | D | 318.62 | 319.5 |
| 525 | 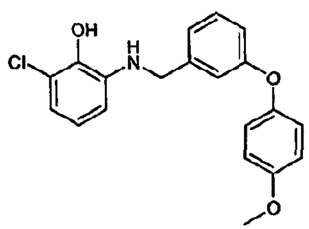 | 2-Chloro-6-[3-(4-methoxy-phenoxy)-benzylamino]-phenol | C20H18ClNO3 | D | 355.81 | 356.5 |
| 526 | 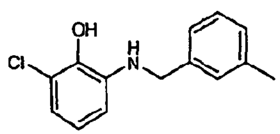 | 2-Chloro-6-(3-methyl-benzylamino)-phenol | C14H14ClNO | D | 247.72 | 248.5 |

| | | | | | | |
|---|---|---|---|---|---|---|
| 527 | 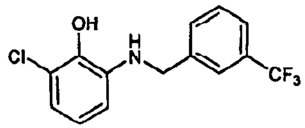 | 2-Chloro-6-(3-trifluoromethyl-benzylamino)-phenol | C14H11ClF3NO | D | 301.69 | 302.2 |
| 528 | 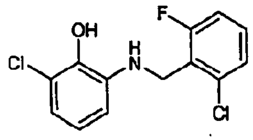 | 2-Chloro-6-(2-chloro-6-fluoro-benzylamino)-phenol | C13H10Cl2FNO | D | 286.13 | 286.6 |
| 529 | 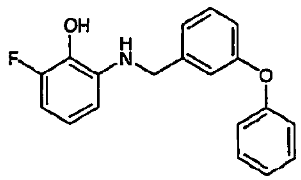 | 2-Fluoro-6-(3-phenoxy-benzylamino)-phenol | C19H16FNO2 | D | 309.33 | 309.8 |
| 530 | 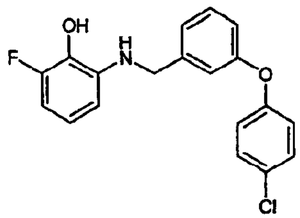 | 2-Fluoro-6-[3-(4-chloro-phenoxy)-benzylamino]-phenol | C19H15ClFNO2 | D | 343.77 | 344.0 |
| 531 | 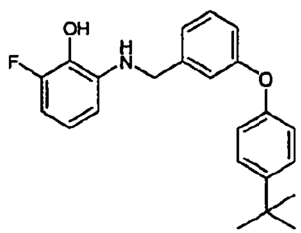 | 2-[3-(4-tert-Butyl-phenoxy)-benzylamino]-6-fluoro-phenol | C23H24FNO2 | D | 365.44 | 366.0 |

| | | | | | | |
|---|---|---|---|---|---|---|
| 532 | 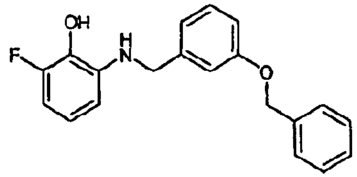 | 2-(3-Benzyloxy-benzylamino)-6-fluoro-phenol | C20H18FNO2 | D | 323.36 | 323.8 |
| 533 | 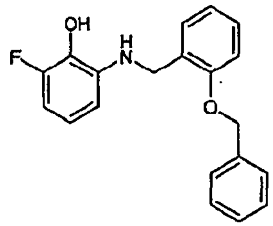 | 2-(2-Benzyloxy-benzylamino)-6-fluoro-phenol | C20H18FNO2 | D | 323.36 | 323.8 |
| 534 | 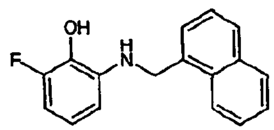 | 2-Fluoro-6-[(naphthalen-1-ylmethyl)-amino]-phenol | C17H14FNO | D | 267.30 | 267.7 |
| 535 | 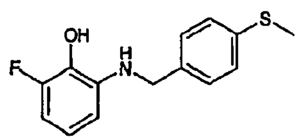 | 2-Fluoro-6-(4-methylsulfanyl-benzylamino)-phenol | C14H14FNOS | D | 263.33 | 263.5 |
| 536 | 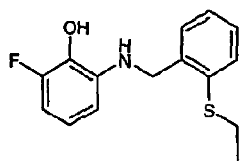 | 2-Fluoro-6-(2-ethylsulfanyl-benzylamino)-phenol | C15H16FNOS | D | 277.36 | 277.8 |

| | | | | | | |
|---|---|---|---|---|---|---|
| 537 | 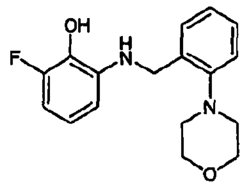 | 2-Fluoro-6-(2-morpholin-4-yl-benzylamino)-phenol | C17H19FN2O2 | D | 302.34 | 302.8 |
| 538 | 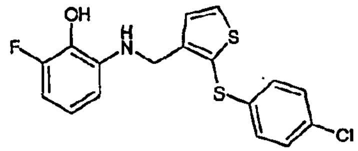 | 2-Fluoro-6-{[2-(4-chloro-phenylsulfanyl)-thiophen-3-ylmethyl]-amino}-phenol | C17H13ClFNOS2 | D | 365.87 | 366.0 |
| 539 | 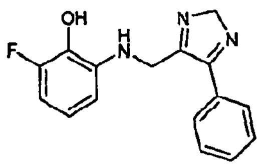 | 2-Fluoro-6-[(5-phenyl-2H-imidazol-4-ylmethyl)-amino]-phenol | C16H14FN3O | D | 283.30 | 283.8 |
| 540 | 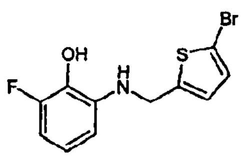 | 2-[(5-Bromo-thiophen-2-ylmethyl)-amino]-6-fluoro-phenol | C11H9BrFNOS | D | 302.16 | 302.5 |
| 541 | 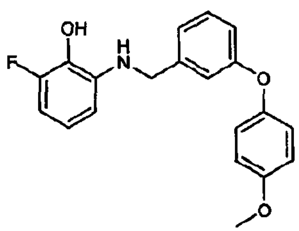 | 2-Fluoro-6-[3-(4-methoxy-phenoxy)-benzylamino]-phenol | C20H18FNO3 | D | 339.36 | 339.8 |

| | | | | | | |
|---|---|---|---|---|---|---|
| 542 | 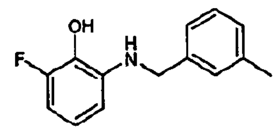 | 2-Fluoro-6-(3-methyl-benzylamino)-phenol | C14H14FNO | D | 231.27 | 231.4 |
| 543 | 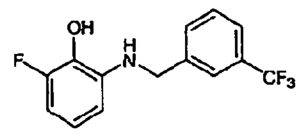 | 2-Fluoro-6-(3-trifluoromethyl-benzylamino)-phenol | C14H11F4NO | D | 285.24 | 285.5 |
| 544 | 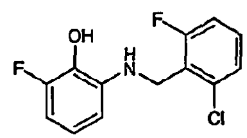 | 2-Fluoro-6-(2-chloro-6-fluoro-benzylamino)-phenol | C13H10ClF2NO | D | 269.67 | 270.1 |
| 545 | 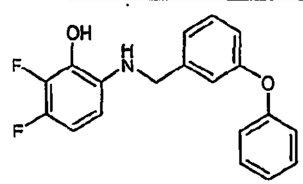 | 2,3-Difluoro-6-(3-phenoxy-benzylamino)-phenol | C19H15F2NO2 | D | 327.32 | 327.6 |
| 546 | 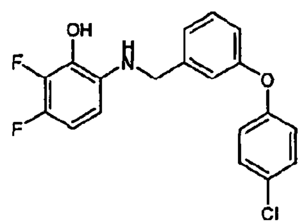 | 2,3-Difluoro-6-[3-(4-chloro-phenoxy)-benzylamino]-phenol | C19H14ClF2NO2 | D | 361.76 | 362.0 |

| | | | | | | |
|---|---|---|---|---|---|---|
| 547 | 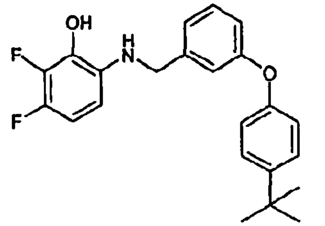 | 2-[3-(4-tert-Butyl-phenoxy)-benzylamino]-5,6-difluoro-phenol | C23H23F2NO2 | D | 383.43 | 383.8 |
| 548 | 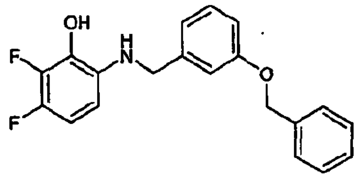 | 2-(3-Benzyloxy-benzylamino)-5,6-difluoro-phenol | C20H17F2NO2 | D | 341.35 | 341.7 |
| 549 | 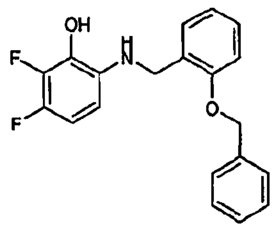 | 2-(2-Benzyloxy-benzylamino)-5,6-difluoro-phenol | C20H17F2NO2 | D | 341.35 | 341.7 |
| 550 | 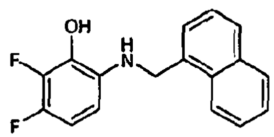 | 2,3-Difluoro-6-[(naphthalen-1-ylmethyl)-amino]-phenol | C17H13F2NO | D | 285.29 | 285.6 |
| 551 | 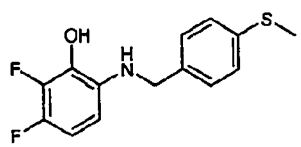 | 2,3-Difluoro-6-(4-methylsulfanyl-benzylamino)-phenol | C14H13F2NOS | D | 281.32 | 281.6 |

| | | | | | | |
|---|---|---|---|---|---|---|
| 552 | 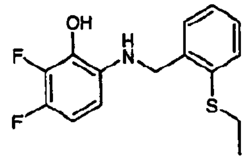 | 2,3-Difluoro-6-(2-ethylsulfanyl-benzylamino)-phenol | C15H15F2NOS | D | 295.35 | 295.7 |
| 553 | 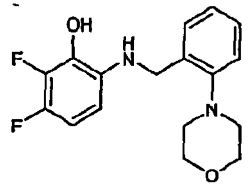 | 2,3-Difluoro-6-(2-morpholin-4-yl-benzylamino)-phenol | C17H18F2N2O2 | D | 320.33 | 320.8 |
| 554 | 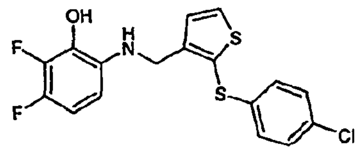 | 2,3-Difluoro-6-{[2-(4-chloro-phenylsulfanyl)-thiophen-3-ylmethyl]-amino}-phenol | C17H12ClF2NOS2 | D | 383.86 | 384.2 |
| 555 | 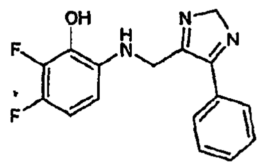 | 2,3-Difluoro-6-[(5-phenyl-2H-imidazol-4-ylmethyl)-amino]-phenol | C16H13F2N3O | D | 301.29 | 301.6 |
| 556 | 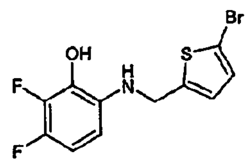 | 2-[(5-Bromo-thiophen-2-ylmethyl)-amino]-5,6-difluoro-phenol | C11H8BrF2NOS | D | 320.15 | 320.4 |

| | | | | | | |
|---|---|---|---|---|---|---|
| 557 | 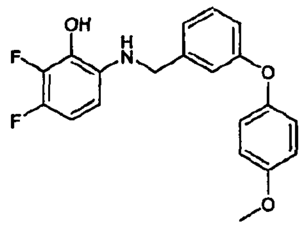 | 2,3-Difluoro-6-[3-(4-methoxy-phenoxy)-benzylamino]-phenol | C20H17F2NO3 | D | 357.35 | 357.6 |
| 558 | 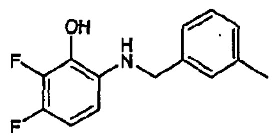 | 2,3-Difluoro-6-(3-methyl-benzylamino)-phenol | C14H13F2NO | D | 249.26 | 249.6 |
| 559 | 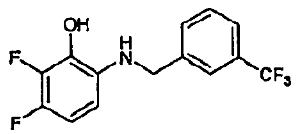 | 2,3-Difluoro-6-(3-trifluoromethyl-benzylamino)-phenol | C14H10F5NO | D | 303.23 | 303.7 |
| 560 | 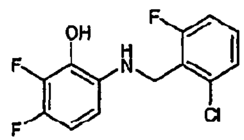 | 2,3-Difluoro-6-(2-chloro-6-fluoro-benzylamino)-phenol | C13H9ClF3NO | D | 287.66 | 288.1 |
| 561 | 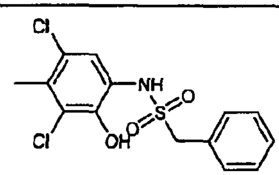 | N-(3,5-Dichloro-2-hydroxy-4-methyl-phenyl)-C-phenyl-methanesulfonamide | C14H13Cl2NO3S | B | 346.23 | 347.0 |

| | | | | | | |
|---|---|---|---|---|---|---|
| 562 | 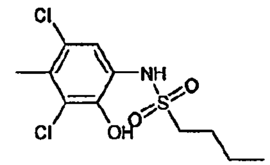 | Butane-1-sulfonic acid (3,5-dichloro-2-hydroxy-4-methyl-phenyl)-amide | C11H15Cl2NO3S | B | 312.21 | 312.1 |
| 563 | 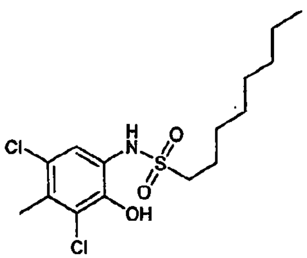 | Octane-1-sulfonic acid (3,5-dichloro-2-hydroxy-4-methyl-phenyl)-amide | C15H23Cl2NO3S | B | 368.32 | 368.3 |
| 564 | 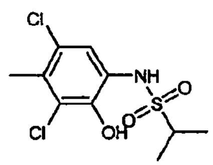 | Propane-2-sulfonic acid (3,5-dichloro-2-hydroxy-4-methyl-phenyl)-amide | C10H13Cl2NO3S | B | 298.18 | 298.1 |
| 565 | 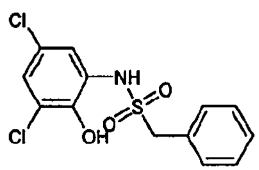 | N-(3,5-Dichloro-2-hydroxy-phenyl)-C-phenyl-methanesulfonamide | C13H11Cl2NO3S | B | 332.20 | 332.4 |
| 566 | 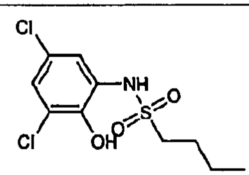 | Butane-1-sulfonic acid (3,5-dichloro-2-hydroxy-phenyl)-amide | C10H13Cl2NO3S | B | 298.19 | 298.5 |

| | | | | | | |
|---|---|---|---|---|---|---|
| 567 | 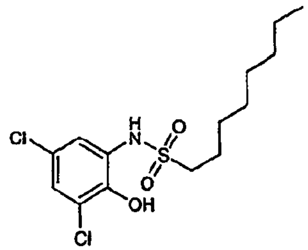 | Octane-1-sulfonic acid (3,5-dichloro-2-hydroxy-phenyl)-amide | C14H21Cl2NO3S | B | 354.29 | 354.6 |
| 568 | 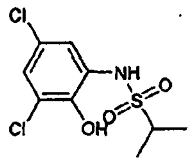 | Propane-2-sulfonic acid (3,5-dichloro-2-hydroxy-phenyl)-amide | C9H11Cl2NO3S | B | 284.16 | 284.5 |
| 569 | 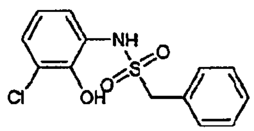 | N-(3-Chloro-2-hydroxy-phenyl)-C-phenyl-methanesulfonamide | C13H12ClNO3S | B | 297.76 | 297.9 |
| 570 | 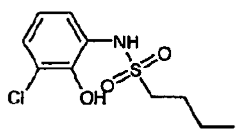 | Butane-1-sulfonic acid (3-chloro-2-hydroxy-phenyl)-amide | C10H14ClNO3S | B | 263.74 | 264.0 |
| 571 | 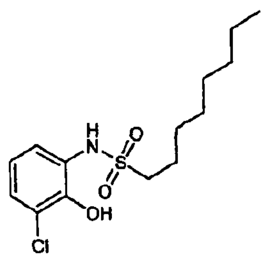 | Octane-1-sulfonic acid (3-chloro-2-hydroxy-phenyl)-amide | C14H22ClNO3S | B | 319.85 | 320.3 |

| | | | | | | |
|---|---|---|---|---|---|---|
| 572 | 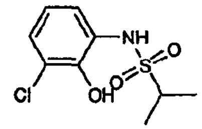 | Propane-2-sulfonic acid (3-chloro-2-hydroxy-phenyl)-amide | C9H12ClNO3S | B | 249.71 | 250.4 |
| 573 | 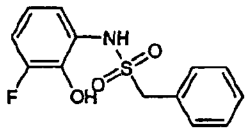 | N-(3-Fluoro-2-hydroxy-phenyl)-C-phenyl-methanesulfonamide | C13H12FNO3S | B | 281.30 | 281.8 |
| 574 | 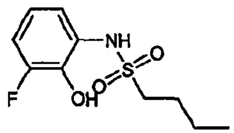 | Butane-1-sulfonic acid (3-fluoro-2-hydroxy-phenyl)-amide | C10H14FNO3S | B | 247.29 | 247.8 |
| 575 | 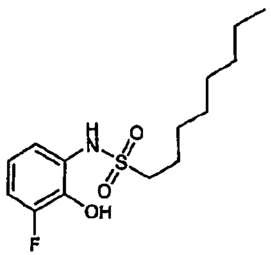 | Octane-1-sulfonic acid (3-fluoro-2-hydroxy-phenyl)-amide | C14H22FNO3S | B | 303.39 | 303.8 |
| 576 | 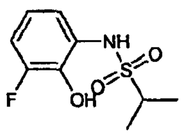 | Propane-2-sulfonic acid (3-fluoro-2-hydroxy-phenyl)-amide | C9H12FNO3S | B | 233.26 | 233.7 |
| 577 | 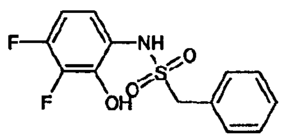 | N-(3,4-Difluoro-2-hydroxy-phenyl)-C-phenyl-methanesulfonamide | C13H11F2NO3S | B | 299.29 | 300.8 |

| | | | | | | |
|---|---|---|---|---|---|---|
| 578 | 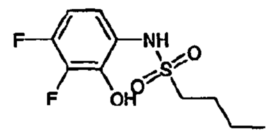 | Butane-1-sulfonic acid (3,4-difluoro-2-hydroxy-phenyl)-amide | C10H13F2NO3S | B | 265.28 | 265.7 |
| 579 | 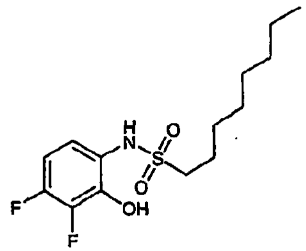 | Octane-1-sulfonic acid (3,4-difluoro-2-hydroxy-phenyl)-amide | C14H21F2NO3S | B | 321.38 | 321.8 |
| 580 | 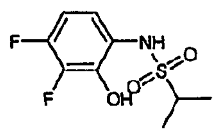 | Propane-2-sulfonic acid (3,4-difluoro-2-hydroxy-phenyl)-amide | C9H11F2NO3S | B | 251.25 | 251.6 |
| 581 | 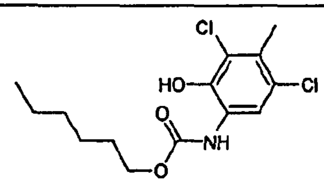 | (3,5-Dichloro-2-hydroxy-4-methyl-phenyl)-carbamic acid hexyl ester | C14H19Cl2NO3 | F, G, H | 320.22 | 320.2 |
| 582 | 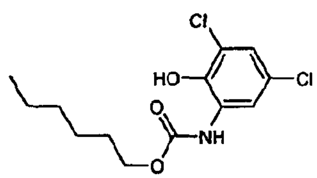 | (3,5-Dichloro-2-hydroxy-phenyl)-carbamic acid hexyl ester | C13H17Cl2NO3 | F, G, H | 306.18 | 306.4 |

| | | | | | | |
|---|---|---|---|---|---|---|
| 583 | 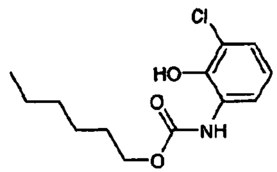 | (3-Chloro-2-hydroxy-phenyl)-carbamic acid hexyl ester | C13H18ClNO3 | F, G, H | 271.74 | 272.1 |
| 584 | 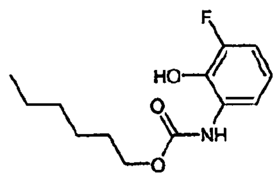 | (3-Fluoro-2-hydroxy-phenyl)-carbamic acid hexyl ester | C13H18FNO3 | F, G, H | 255.29 | 255.6 |
| 585 | 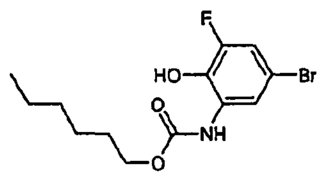 | (5-Bromo-3-fluoro-2-hydroxy-phenyl)-carbamic acid hexyl ester | C13H17BrFNO3 | F, G, H | 334.18 | 334.5 |
| 586 | 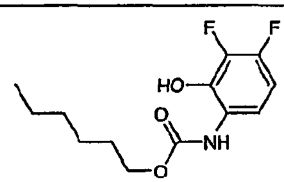 | (3,4-Difluoro-2-hydroxy-phenyl)-carbamic acid hexyl ester | C13H17F2NO3 | F, G, H | 273.28 | 273.6 |
| 587 | 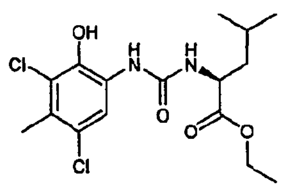 | 2-[3-(3,5-Dichloro-2-hydroxy-4-methyl-phenyl)-ureido]-4-methyl-pentanoic acid ethyl ester | C16H22Cl2N2O4 | A | 377.26 | 377.1 |

| | | | | | | |
|---|---|---|---|---|---|---|
| 588 | 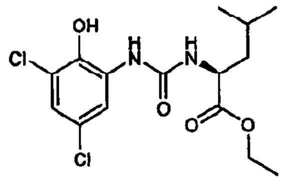 | 2-[3-(3,5-Dichloro-2-hydroxy-phenyl)-ureido]-4-methyl-pentanoic acid ethyl ester | C15H20Cl2N2O4 | A | 363.24 | 363.6 |
| 589 | 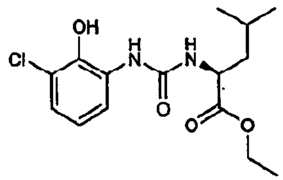 | 2-[3-(3-Chloro-2-hydroxy-phenyl)-ureido]-4-methyl-pentanoic acid ethyl ester | C15H21ClN2O4 | A | 328.79 | 329.1 |
| 590 | 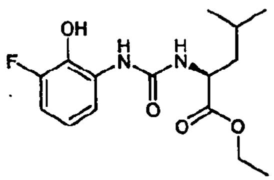 | 2-[3-(3-Fluoro-2-hydroxy-phenyl)-ureido]-4-methyl-pentanoic acid ethyl ester | C15H21FN2O4 | A | 312.34 | 312.8 |
| 591 | 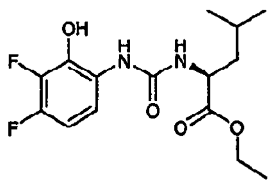 | 2-[3-(3,4-Difluoro-2-hydroxy-4-phenyl)-ureido]-4-methyl-pentanoic acid ethyl ester | C15H20F2N2O4 | A | 330.33 | 330.8 |
| 592 | 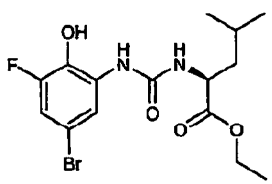 | 2-[3-(5-Bromo-3-fluoro-2-hydroxy-4-methyl-phenyl)-ureido]-4-methyl-pentanoic acid ethyl ester | C15H20BrFN2O4 | A | 391.23 | 391.6 |

| | | | | | | |
|---|---|---|---|---|---|---|
| 593 | 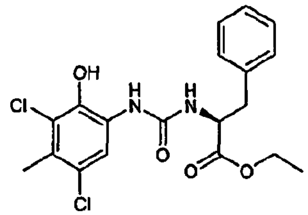 | 2-[3-(3,5-Dichloro-2-hydroxy-4-methyl-phenyl)-ureido]-3-phenyl-propionic acid ethyl ester | C19H20Cl2N2O4 | A | 411.28 | 411.1 |
| 594 | 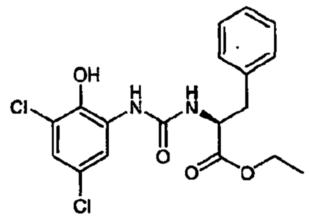 | 2-[3-(3,5-Dichloro-2-hydroxy-phenyl)-ureido]-3-phenyl-propionic acid ethyl ester | C18H18Cl2N2O4 | A | 397.25 | 397.6 |
| 595 | 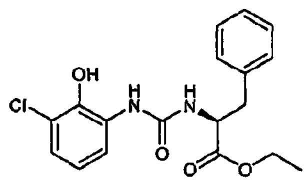 | 2-[3-(3-Chloro-2-hydroxy-phenyl)-ureido]-3-phenyl-propionic acid ethyl ester | C18H19ClN2O4 | A | 362.81 | 363.0 |
| 596 | 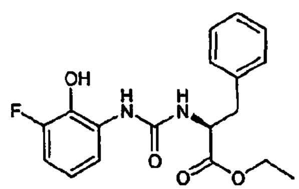 | 2-[3-(3-Fluoro-2-hydroxy-phenyl)-ureido]-3-phenyl-propionic acid ethyl ester | C18H19FN2O4 | A | 346.35 | 346.7 |

| | | | | | | |
|---|---|---|---|---|---|---|
| 597 | 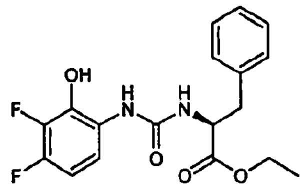 | 2-[3-(3,4-Difluoro-2-hydroxy-phenyl)-ureido]-3-phenyl-propionic acid ethyl ester | $C_{18}H_{18}F_2N_2O_4$ | A | 364.34 | 364.8 |
| 598 | 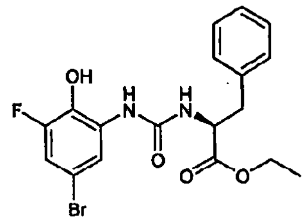 | 2-[3-(5-Bromo-3-fluoro-2-hydroxy-phenyl)-ureido]-3-phenyl-propionic acid ethyl ester | $C_{18}H_{18}BrFN_2O_4$ | A | 425.25 | 425.6 |
| 599 | 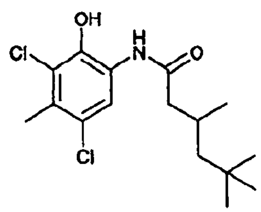 | 3,5,5-Trimethyl-hexanoic acid (3,5-dichloro-2-hydroxy-4-methyl-phenyl)-amide | $C_{16}H_{23}Cl_2NO_2$ | C, E | 332.27 | 332.5 |

| | Structure | Chemical name | Formula | Synthesis methods | MolWeight | MS data |
|---|---|---|---|---|---|---|
| 600 | 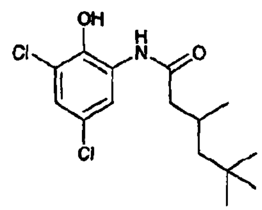 | 3,5,5-Trimethyl-hexanoic acid (3,5-dichloro-2-hydroxy-phenyl)-amide | C15H21Cl2NO2 | C, E | 318.24 | 318.5 |
| 601 | 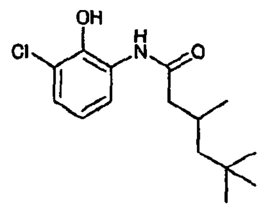 | 3,5,5-Trimethyl-hexanoic acid (3-chloro-2-hydroxy-phenyl)-amide | C15H22ClNO2 | C, E | 283.79 | 284.0 |
| 602 | 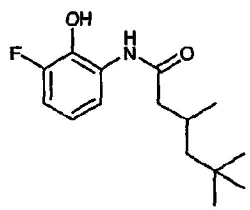 | 3,5,5-Trimethyl-hexanoic acid (3-fluoro-2-hydroxy-phenyl)-amide | C15H21FNO2 | C, E | 267.34 | 267.7 |
| 603 | 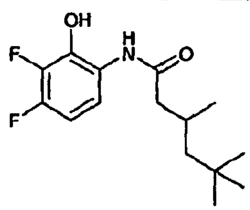 | 3,5,5-Trimethyl-hexanoic acid (3,4-difluoro-2-hydroxy-phenyl)-amide | C15H21F2NO2 | C, E | 285.33 | 285.7 |

| | | | | | | |
|---|---|---|---|---|---|---|
| 604 | 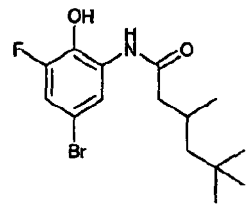 | 3,5,5-Trimethyl-hexanoic acid (5-bromo-3-fluoro-2-hydroxy-phenyl)-amide | C15H21BrFNO2 | C, E | 346.24 | 346.5 |
| 605 | 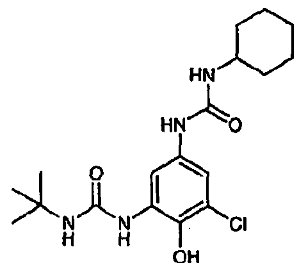 | 1-tert-Butyl-3-[3-chloro-5-(3-cyclohexyl-ureido)-2-hydroxy-phenyl]-urea | C18H27ClN4O3 | J followed by A | 382.88 | 383.4 |
| 606 | 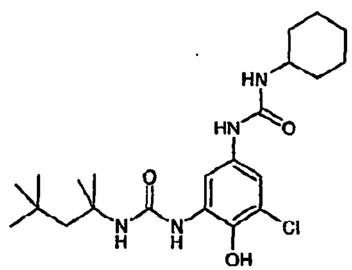 | 1-[3-Chloro-5-(3-cyclohexyl-ureido)-2-hydroxy-phenyl]-3-(1,1,3,3-tetramethyl-butyl)-urea | C22H35ClN4O3 | J followed by A | 438.99 | 439.8 |

| | | | | | | |
|---|---|---|---|---|---|---|
| 607 | 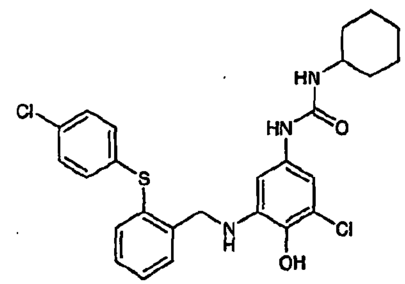 | 1-{3-Chloro-5-[2-(4-chloro-phenylsulfanyl)-benzylamino]-4-hydroxy-phenyl}-3-cyclohexyl-urea | C26H27Cl2N3O2S | J followed by D | 516.48 | 517.4 |
| 608 | 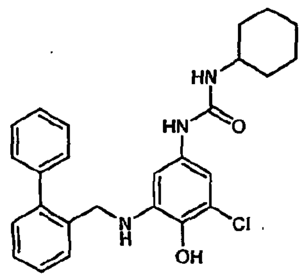 | 1-{3-[(Biphenyl-2-ylmethyl)-amino]-5-chloro-4-hydroxy-phenyl}-3-cyclohexyl-urea | C26H28ClN3O2 | J followed by D | 449.97 | 450.9 |
| 609 | 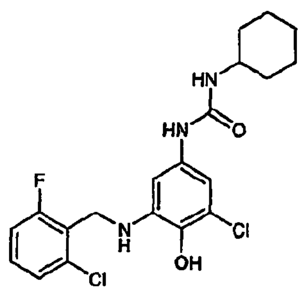 | 1-[3-Chloro-5-(2-chloro-6-fluoro-benzylamino)-4-hydroxy-phenyl]-3-cyclohexyl-urea | C20H22Cl2FN3O2 | J followed by D | 426.31 | 426.4 |

| | | | | | | |
|---|---|---|---|---|---|---|
| 610 | 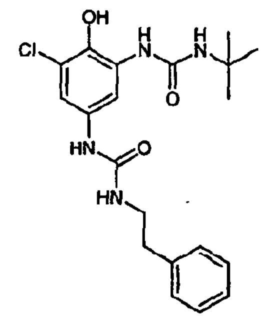 | 1-tert-Butyl-3-[3-chloro-2-hydroxy-5-(3-phenethyl-ureido)-phenyl]-urea | C20H25ClN4O3 | J followed by A | 404.89 | 404.9 |
| 611 | 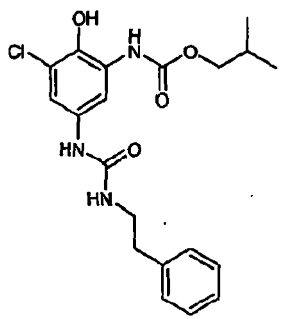 | [3-Chloro-2-hydroxy-5-(3-phenethyl-ureido)-phenyl]-carbamic acid isobutyl ester | C20H24ClN3O4 | J followed by G | 405.88 | 406.8 |
| 612 | 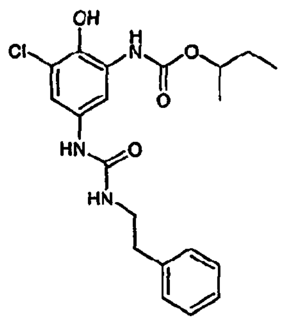 | [3-Chloro-2-hydroxy-5-(3-phenethyl-ureido)-phenyl]-carbamic acid sec-butyl ester | C20H24ClN3O4 | J followed by G | 405.88 | 406.8 |

| | | | | | | |
|---|---|---|---|---|---|---|
| 613 | 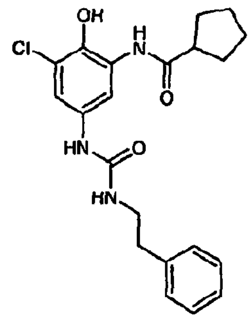 | Cyclopentanecarboxylic acid [3-chloro-2-hydroxy-5-(3-phenethyl-ureido)-phenyl]-amide | C21H24ClN3O3 | J followed by C | 401.89 | 401.9 |
| 614 | 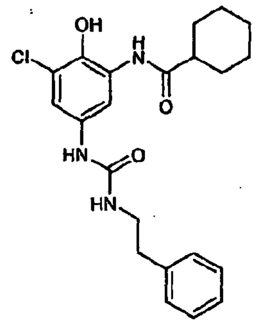 | Cyclohexanecarboxylic acid [3-chloro-2-hydroxy-5-(3-phenethyl-ureido)-phenyl]-amide | C22H26ClN3O3 | J followed by C | 415.92 | 415.9 |
| 615 | 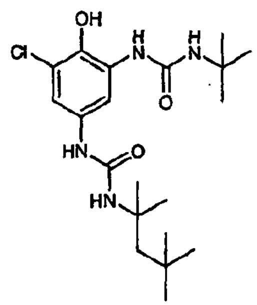 | 1-tert-Butyl-3-{3-chloro-2-hydroxy-5-[3-(1,1,3,3-tetramethyl-butyl)-ureido]-phenyl}-urea | C20H33ClN4O3 | J followed by A | 412.95 | 413.8 |

| | | | | | | |
|---|---|---|---|---|---|---|
| 616 | 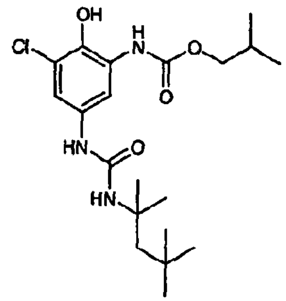 | {3-Chloro-2-hydroxy-5-[3-(1,1,3,3-tetramethyl-butyl)-ureido]-phenyl}-carbamic acid isobutyl ester | C20H32ClN3O4 | J followed by G | 413.94 | 413.8 |
| 617 | 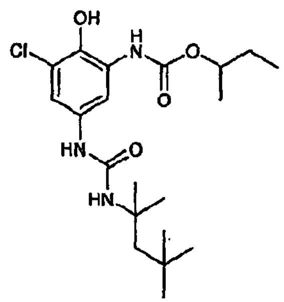 | {3-Chloro-2-hydroxy-5-[3-(1,1,3,3-tetramethyl-butyl)-ureido]-phenyl}-carbamic acid sec-butyl ester | C20H32ClN3O4 | J followed by G | 413.94 | 414.1 |
| 618 | 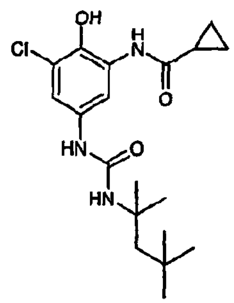 | Cyclopropanecarboxylic acid {3-chloro-2-hydroxy-5-[3-(1,1,3,3-tetramethyl-butyl)-ureido]-phenyl}-amide | C19H28ClN3O3 | J followed by C | 381.90 | 381.9 |

| | | | | | | |
|---|---|---|---|---|---|---|
| 619 | 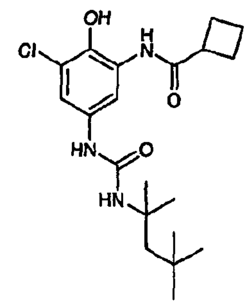 | Cyclobutanecarboxylic acid {3-chloro-2-hydroxy-5-[3-(1,1,3,3-tetramethyl-butyl)-ureido]-phenyl}-amide | C20H30ClN3O3 | J followed by C | 395.92 | 395.9 |
| 620 | 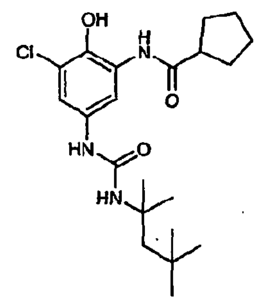 | Cyclopentanecarboxylic acid {3-chloro-2-hydroxy-5-[3-(1,1,3,3-tetramethyl-butyl)-ureido]-phenyl}-amide | C21H32ClN3O3 | J followed by C | 409.95 | 410.1 |
| 621 | 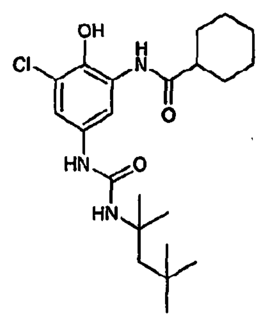 | Cyclohexanecarboxylic acid {3-chloro-2-hydroxy-5-[3-(1,1,3,3-tetramethyl-butyl)-ureido]-phenyl}-amide | C22H34ClN3O3 | J followed by C | 423.98 | 424.0 |

| | | | | | | |
|---|---|---|---|---|---|---|
| 622 | 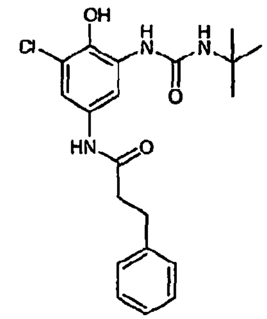 | N-[3-(3-tert-Butyl-ureido)-5-chloro-4-hydroxy-phenyl]-3-phenyl-propionamide | C20H24ClN3O3 | L followed by A | 389.88 | 389.9 |
| 623 | 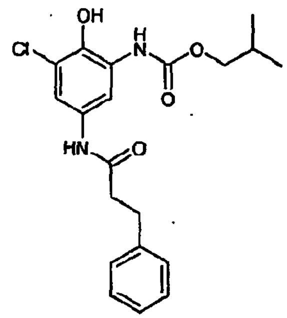 | [3-Chloro-2-hydroxy-5-(3-phenyl-propionylamino)-phenyl]-carbamic acid isobutyl ester | C20H23ClN2O4 | L followed by G | 390.86 | 390.8 |
| 624 | 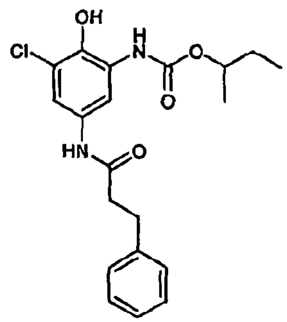 | [3-Chloro-2-hydroxy-5-(3-phenyl-propionylamino)-phenyl]-carbamic acid sec-butyl ester | C20H23ClN2O4 | L followed by G | 390.86 | 390.8 |

| | | | | | | |
|---|---|---|---|---|---|---|
| 625 | 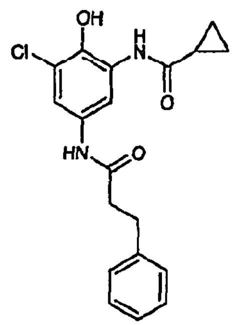 | Cyclopropanecarboxylic acid [3-chloro-2-hydroxy-5-(3-phenyl-propionylamino)-phenyl]-amide | C19H19ClN2O3 | L followed by C | 358.82 | 358.9 |
| 626 | 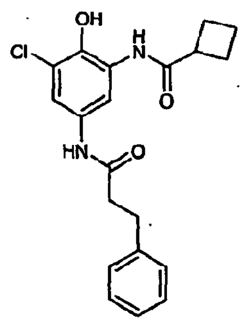 | Cyclobutanecarboxylic acid [3-chloro-2-hydroxy-5-(3-phenyl-propionylamino)-phenyl]-amide | C20H21ClN2O3 | L followed by C | 372.85 | 373.8 |
| 627 | 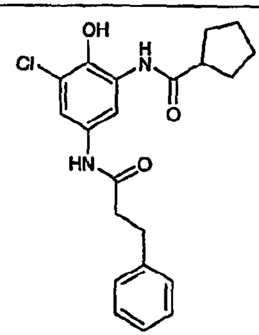 | Cyclopentanecarboxylic acid [3-chloro-2-hydroxy-5-(3-phenyl-propionylamino)-phenyl]-amide | C21H23ClN2O3 | L followed by C | 386.87 | 386.8 |

| | | | | | | |
|---|---|---|---|---|---|---|
| 628 | 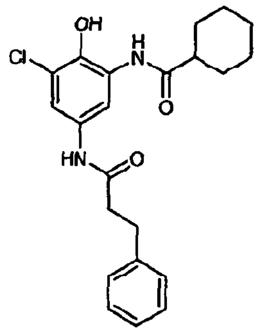 | Cyclohexanecarboxylic acid [3-chloro-2-hydroxy-5-(3-phenyl-propionylamino)-phenyl]-amide | C22H24ClN2O3 | L followed by C | 400.90 | 401.8 |
| 629 | 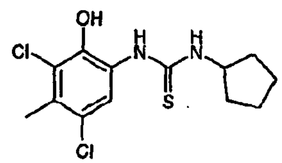 | 1-Cyclopentyl-3-(3,5-dichloro-2-hydroxy-4-methyl-phenyl)-thiourea | C13H16Cl2N2OS | I | 319.25 | 319.7 |
| 630 | 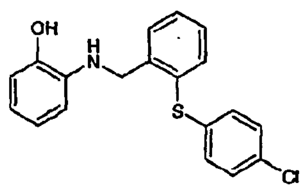 | 2-[2-(4-Chloro-phenylsulfanyl)-benzylamino]-phenol | C19H16ClNOS | D | 341.85 | 342.3 |

| | | | | | | |
|---|---|---|---|---|---|---|
| 631 | 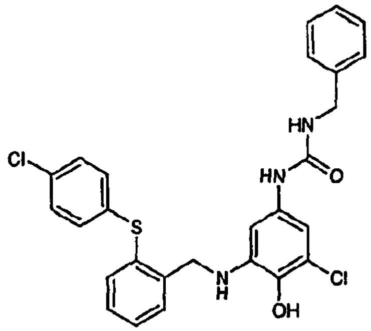 | 1-Benzyl-3-{3-chloro-5-[2-(4-chloro-phenylsulfanyl)-benzylamino]-4-hydroxy-phenyl}-urea | C27H23Cl2N3O2S | J followed by D | 524.46 | 525.1 |
| 632 | 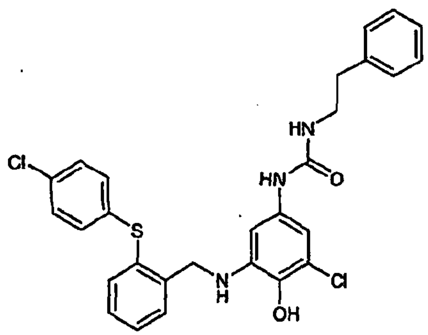 | 1-{3-Chloro-5-[2-(4-chloro-phenylsulfanyl)-benzylamino]-4-hydroxy-phenyl}-3-phenethyl-urea | C28H25Cl2N3O2S | J followed by D | 538.49 | 539.1 |
| 633 | 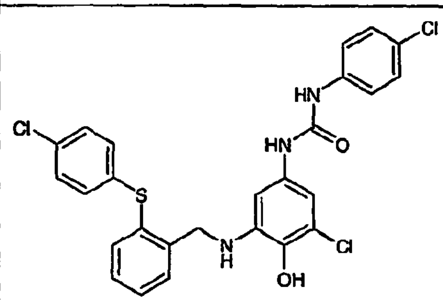 | 1-{3-Chloro-5-[2-(4-chloro-phenylsulfanyl)-benzylamino]-4-hydroxy-phenyl}-3-(4-chloro-phenyl)-urea | C26H20Cl3N3O2S | J followed by D | 544.88 | 545.5 |

| | | | | | | |
|---|---|---|---|---|---|---|
| 634 | 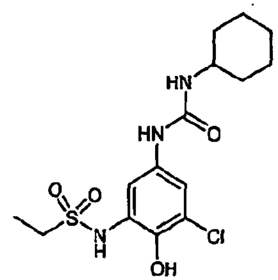 | Ethanesulfonic acid [3-chloro-5-(3-cyclohexyl-ureido)-2-hydroxy-phenyl]-amide | $C_{15}H_{22}ClN_3O_4S$ | J followed by B | 375.9 | 376.1 |
| 635 | 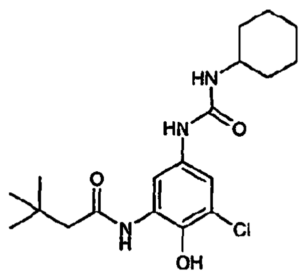 | N-[3-Chloro-5-(3-cyclohexyl-ureido)-2-hydroxy-phenyl]-3,3-dimethyl-butyramide | $C_{19}H_{28}ClN_3O_3$ | J followed by C | 381.90 | 382.2 |
| 636 | 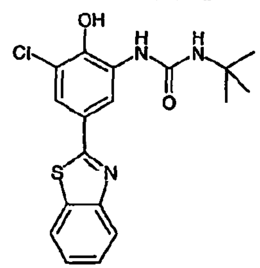 | 1-(5-Benzothiazol-2-yl-3-chloro-2-hydroxy-phenyl)-3-tert-butyl-urea | $C_{18}H_{18}ClN_3O_2S$ | K followed by A | 375.9 | 376.1 |

| | | | | | | |
|---|---|---|---|---|---|---|
| 637 | 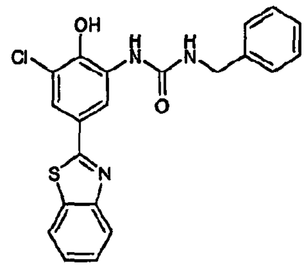 | 1-(5-Benzothiazol-2-yl-3-chloro-2-hydroxy-phenyl)-3-benzyl-urea | C21H16ClN3O2S | K followed by A | 409.9 | 410.1 |
| 638 | 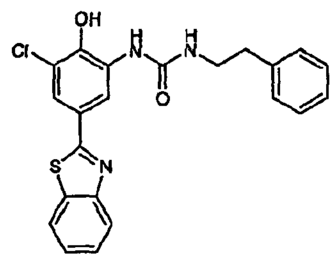 | 1-(5-Benzothiazol-2-yl-3-chloro-2-hydroxy-phenyl)-3-phenethyl-urea | C22H18ClN3O2S | K followed by A | 423.9 | 424.1 |
| 639 | 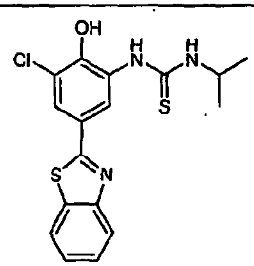 | 1-(5-Benzothiazol-2-yl-3-chloro-2-hydroxy-phenyl)-3-isopropyl-thiourea | C17H16ClN3OS2 | K followed by I | 377.9 | 378.1 |

| | | | | | | |
|---|---|---|---|---|---|---|
| 640 | 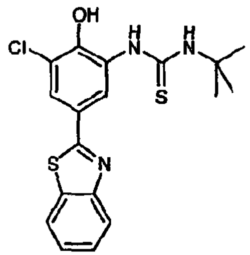 | 1-(5-Benzothiazol-2-yl-3-chloro-2-hydroxy-phenyl)-3-tert-butyl-thiourea | C18H18ClN3OS2 | K followed by I | 391.9 | 392.1 |
| 641 | 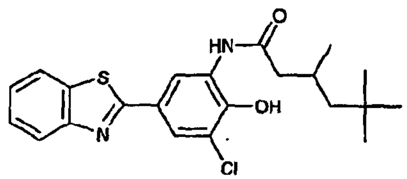 | 3,5,5-Trimethyl-hexanoic acid (5-benzothiazol-2-yl-3-chloro-2-hydroxy-phenyl)-amide | C22H25ClN2O2S | K followed by C | 417.0 | 417.2 |
| 642 | 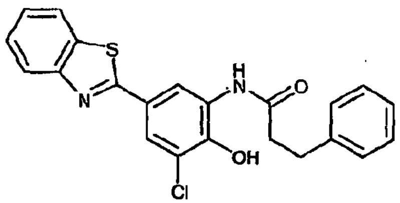 | N-(5-Benzothiazol-2-yl-3-chloro-2-hydroxy-phenyl)-3-phenyl-propionamide | C22H17ClN2O2S | K followed by C | 408.9 | 409.2 |
| 643 | 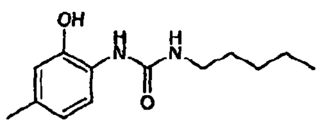 | 1-(2-Hydroxy-4-methyl-phenyl)-3-pentyl-urea | C13H20N2O2 | A | 263.31 | 256.9 |

| | | | | | | |
|---|---|---|---|---|---|---|
| 644 | 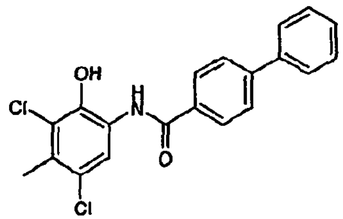 | Biphenyl-4-carboxylic acid (3,5-dichloro-2-hydroxy-4-methyl-phenyl)-amide | C20H15Cl2NO2 | C or E | 372.24 | 372.6 |
| 645 | 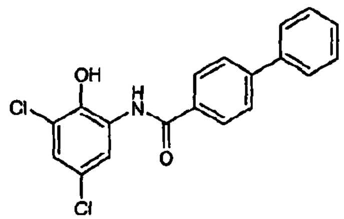 | Biphenyl-4-carboxylic acid (3,5-dichloro-2-hydroxy-phenyl)-amide | C20H15Cl2NO2 | C or E | 358.22 | 358.5 |
| 646 | 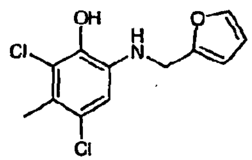 | 2,4-Dichloro-6-[(furan-2-ylmethyl)-amino]-3-methyl-phenol | C12H11Cl2NO2 | D | 272.13 | 272.6 |
| 647 | 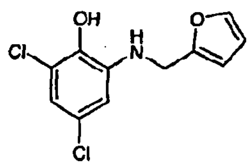 | 2,4-Dichloro-6-[(furan-2-ylmethyl)-amino]-phenol | C11H9Cl2NO2 | D | 258.10 | 258.4 |
| 648 | 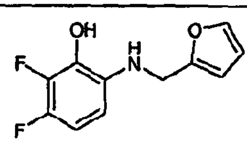 | 2,3-Difluoro-6-[(furan-2-ylmethyl)-amino]-phenol | C11H9F2NO2 | D | 225.19 | 225.7 |

| | | | | | | |
|---|---|---|---|---|---|---|
| 649 | 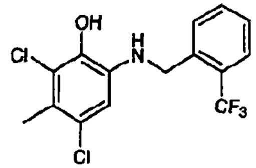 | 2,4-Dichloro-3-methyl-6-(2-trifluoromethyl-benzylamino)-phenol | C15H12Cl2F3NO | D | 350.16 | 350.5 |
| 650 | 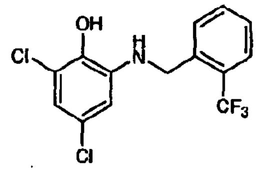 | 2,4-Dichloro-6-(2-trifluoromethyl-benzylamino)-phenol | C14H10Cl2F3NO | D | 336.14 | 336.5 |
| 651 | 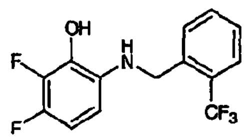 | 2,3-Difluoro-6-(2-trifluoromethyl-benzylamino)-phenol | C14H10F5NO | D | 303.23 | 303.6 |
| 652 | 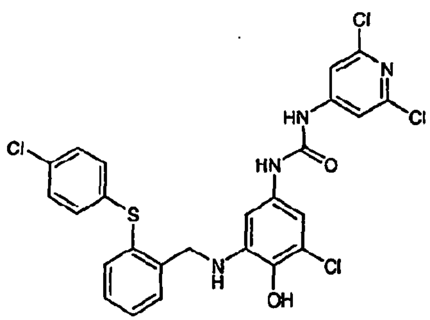 | 1-{3-Chloro-5-[2-(4-chloro-phenylsulfanyl)-benzylamino]-4-hydroxy-phenyl}-3-(2,6-dichloro-pyridin-4-yl)-urea | C25H18Cl4N4O2S | J followed by D | 580.31 | 580.9 |

| | | | | | | |
|---|---|---|---|---|---|---|
| 653 | 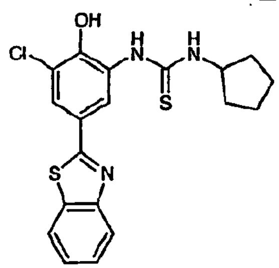 | 1-(5-Benzothiazol-2-yl-3-chloro-2-hydroxy-phenyl)-3-cyclopentyl-thiourea | C19H18ClN3OS2 | K followed by I | 403.95 | 404.6 |
| 654 | 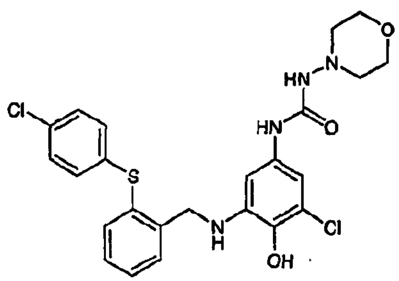 | 1-{3-Chloro-5-[2-(4-chloro-phenylsulfanyl)-benzylamino]-4-hydroxy-phenyl}-3-morpholin-4-yl-urea | C24H24Cl2N4O3S | J followed by D | 519.44 | 520.1 |
| 655 | 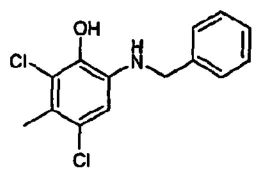 | 6-Benzylamino-2,4-dichloro-3-methyl-phenol | C14H13Cl2NO | D | 282.17 | 282.7 |
| 656 | 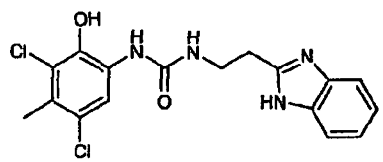 | 1-[2-(1H-Benzoimidazol-2-yl)-ethyl]-3-(3,5-dichloro-2-hydroxy-4-methyl-phenyl)-urea | C17H16Cl2N4O2 | A | 379.24 | 379.9 |

| | | | | | | |
|---|---|---|---|---|---|---|
| 657 | 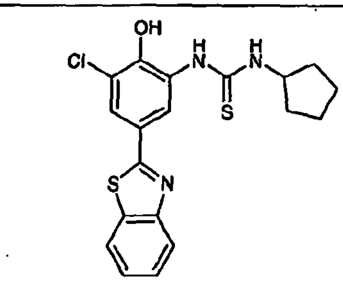 | 1-(5-Benzothiazol-2-yl-3-chloro-2-hydroxy-phenyl)-3-cyclopentyl-thiourea | C19H18ClN3OS2 | K followed by I | 403.95 | 404.6 |
| 658 | 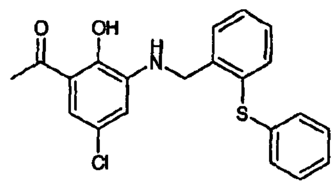 | 1-[5-Chloro-2-hydroxy-3-(2-phenylsulfanyl-benzylamino)-phenyl]-ethanone | C21H18ClNO2S | D | 383.89 | 384.4 |
| 659 | 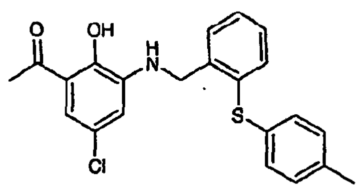 | 1-[5-Chloro-2-hydroxy-3-(2-p-tolylsulfanyl-benzylamino)-phenyl]-ethanone | C22H20ClNO2S | D | 397.92 | 398.2 |
| 660 | 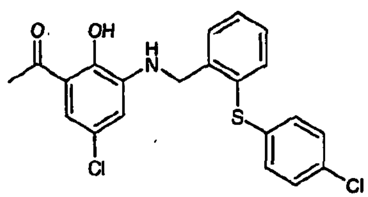 | 1-{5-Chloro-3-[2-(4-chloro-phenylsulfanyl)-benzylamino]-2-hydroxy-phenyl}-ethanone | C21H17Cl2NO2S | D | 418.34 | 418.9 |

| | | | | | | |
|---|---|---|---|---|---|---|
| 661 | 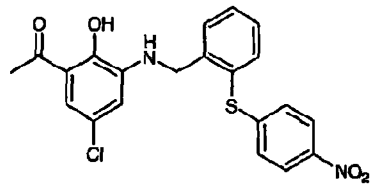 | 1-{5-Chloro-2-hydroxy-3-[2-(4-nitro-phenylsulfanyl)-benzylamino]-phenyl}-ethanone | C21H17ClN2O4S | D | 428.89 | 429.3 |
| 662 | 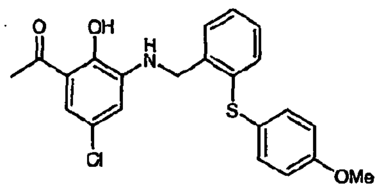 | 1-{5-Chloro-2-hydroxy-3-[2-(4-methoxy-phenylsulfanyl)-benzylamino]-phenyl}-ethanone | C22H20ClNO3S | D | 413.92 | 414.3 |
| 663 | 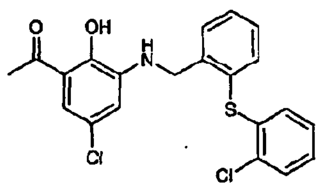 | 1-{5-Chloro-3-[2-(2-chloro-phenylsulfanyl)-benzylamino]-2-hydroxy-phenyl}-ethanone | C21H17Cl2NO2S | D | 418.34 | 418.9 |
| 664 | 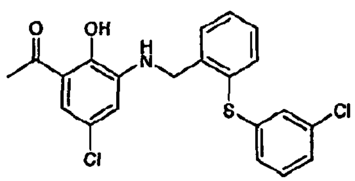 | 1-{5-Chloro-3-[2-(3-chloro-phenylsulfanyl)-benzylamino]-2-hydroxy-phenyl}-ethanone | C21H17Cl2NO2S | D | 418.34 | 418.8 |
| 665 | 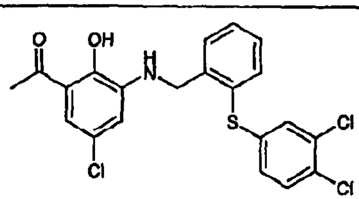 | 1-{5-Chloro-3-[2-(3,4-dichloro-phenylsulfanyl)-benzylamino]-2-hydroxy-phenyl}-ethanone | C21H16Cl3NO2S | D | 452.78 | 453.2 |

| | | | | | | |
|---|---|---|---|---|---|---|
| 666 | 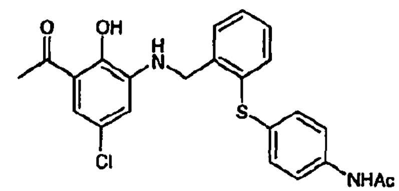 | N-(4-{2-[(3-Acetyl-5-chloro-2-hydroxy-phenylamino)-methyl]-phenylsulfanyl}-phenyl)-acetamide | C23H21ClN2O3S | D | 440.94 | 441.4 |
| 667 | 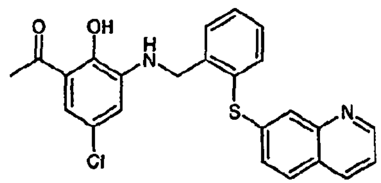 | 1-{5-Chloro-2-hydroxy-3-[2-(quinolin-7-ylsulfanyl)-benzylamino]-phenyl}-ethanone | C24H19ClN2O2S | D | 434.94 | 435.3 |
| 668 | 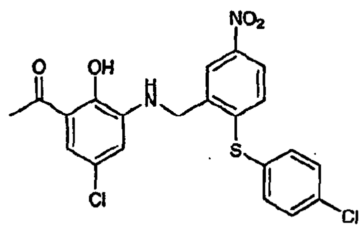 | 1-{5-Chloro-3-[2-(4-chloro-phenylsulfanyl)-5-nitro-benzylamino]-2-hydroxy-phenyl}-ethanone | C21H16Cl2N2O4S | D | 463.33 | 463.9 |
| 669 | 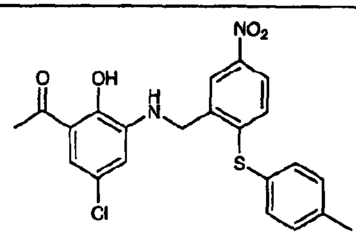 | 1-[5-Chloro-2-hydroxy-3-(5-nitro-2-p-tolylsulfanyl-benzylamino)-phenyl]-ethanone | C22H19ClN2O4S | D | 442.92 | 443.3 |

| | | | | | | |
|---|---|---|---|---|---|---|
| 670 | 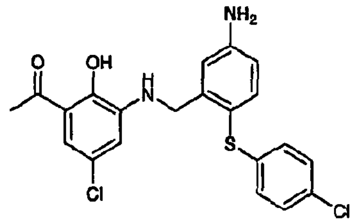 | 1-{3-[5-Amino-2-(4-chloro-phenylsulfanyl)-benzylamino]-5-chloro-2-hydroxy-phenyl}-ethanone | C21H18Cl2N2O2S | D | 433.35 | 433.6 |
| 671 | 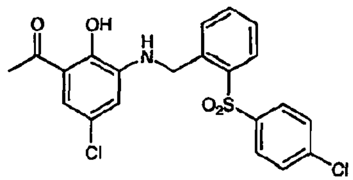 | 1-{5-Chloro-3-[2-(4-chloro-benzenesulfonyl)-benzylamino]-2-hydroxy-phenyl}-ethanone | C21H17Cl2NO4S | D | 450.33 | 448.5 |
| 672 | 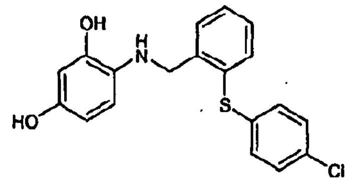 | 4-[2-(4-Chloro-phenylsulfanyl)-benzylamino]-benzene-1,3-diol | C19H16ClNO2S | D | 357.85 | 358.4 |
| 673 | 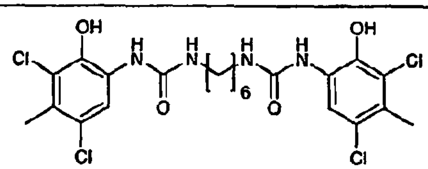 | 1,6-Di-(3,5-Dichloro-2-hydroxy-4-methyl-phenyl)-3-hexyl-urea | C22H26Cl4N4O4 | A followed by A | 552.28 | 552.5 |
| 674 | 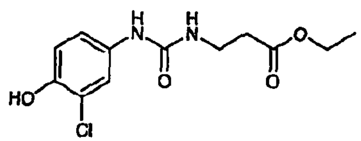 | 3-[3-(3-Chloro-4-hydroxy-phenyl)-ureido]-propionic acid ethyl ester | C12H15ClN2O4 | A | 286.71 | 286.9 |

| | | | | | | |
|---|---|---|---|---|---|---|
| 675 | 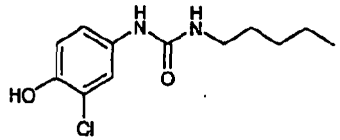 | 1-(3-Chloro-4-hydroxy-phenyl)-3-pentyl-urea | C12H17ClN2O2 | A | 256.73 | 256.9 |
| 676 | 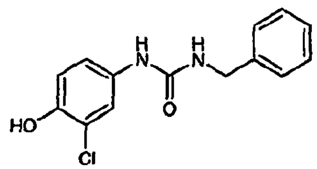 | 1-Benzyl-3-(3-chloro-4-hydroxy-phenyl)-urea | C14H13ClN2O2 | A | 276.72 | 276.9 |
| 677 | 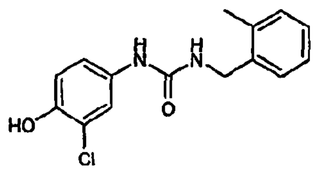 | 1-(3-Chloro-4-hydroxy-phenyl)-3-(2-methyl-benzyl)-urea | C15H15ClN2O2 | A | 290.74 | 290.9 |
| 678 | 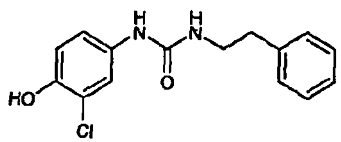 | 1-(3-Chloro-4-hydroxy-phenyl)-3-phenethyl-urea | C15H15ClN2O2 | A | 290.75 | 291.2 |
| 679 | 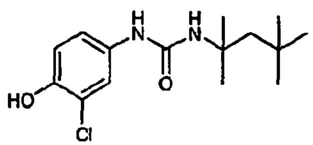 | 1-(3-Chloro-4-hydroxy-phenyl)-3-(1,1,3,3-tetramethyl-butyl)-urea | C15H23ClN2O2 | A | 298.81 | 299.4 |
| 680 | 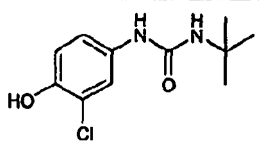 | 1-tert-Butyl-3-(3-chloro-4-hydroxy-phenyl)-urea | C11H15ClN2O2 | A | 242.70 | 243.0 |

| | | | | | | |
|---|---|---|---|---|---|---|
| 681 | 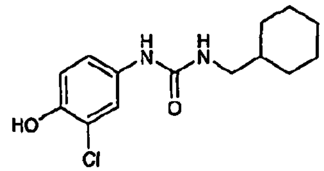 | 1-(3-Chloro-4-hydroxy-phenyl)-3-cyclohexylmethyl-urea | C14H19ClN2O2 | A | 282.77 | 283.1 |
| 682 | 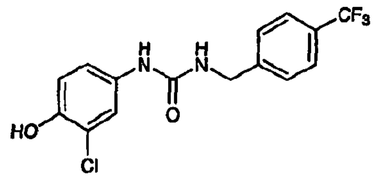 | 1-(3-Chloro-4-hydroxy-phenyl)-3-(4-trifluoromethyl-benzyl)-urea | C15H12ClF3N2O2 | A | 344.72 | 345.0 |
| 683 | 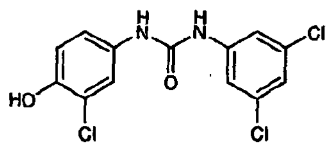 | 1-(3-Chloro-3-hydroxy-phenyl)-3-(3,5-dichloro-phenyl)-urea | C13H9Cl3N2O2 | A | 331.59 | 331.9 |
| 684 | 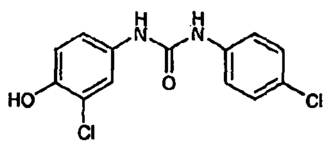 | 1-(3-Chloro-4-hydroxy-phenyl)-3-(4-chloro-phenyl)-urea | C13H10Cl2N2O2 | A | 297.14 | 297.7 |
| 685 | 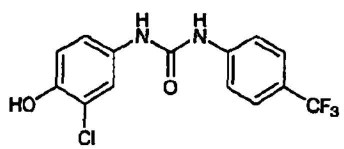 | 1-(3-Chloro-4-hydroxy-phenyl)-3-(4-trifluoromethyl-phenyl)-urea | C14H10ClF3N2O2 | A | 330.69 | 331.0 |
| 686 | 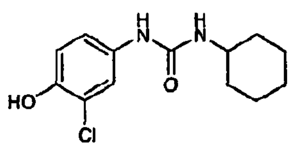 | 1-(3-Chloro-4-hydroxy-phenyl)-3-cyclohexyl-urea | C13H17ClN2O2 | A | 268.74 | 269.1 |

| | | | | | | |
|---|---|---|---|---|---|---|
| 687 | 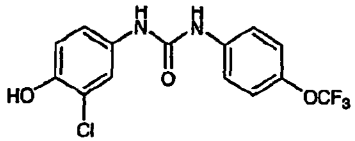 | 1-(3-Chloro-4-hydroxy-phenyl)-3-(4-trifluoromethoxy-phenyl)-urea | C14H10ClF3N2O3 | A | 346.69 | 346.9 |
| 688 | 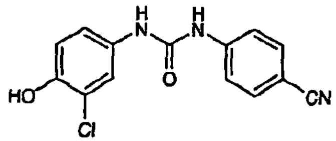 | 1-(3-Chloro-4-hydroxy-phenyl)-3-(4-cyano-phenyl)-urea | C14H10ClN3O2 | A | 287.70 | 287.9 |
| 689 | 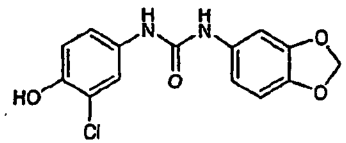 | 1-Benzo[1,3]dioxol-5-yl-3-(3-chloro-4-hydroxy-phenyl)-urea | C14H11ClN2O4 | A | 306.70 | 306.9 |
| 690 | 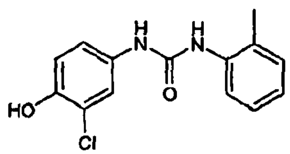 | 1-(3-Chloro-4-hydroxy-phenyl)-3-o-tolyl-urea | C14H13ClN2O2 | A | 276.72 | 276.9 |
| 691 | 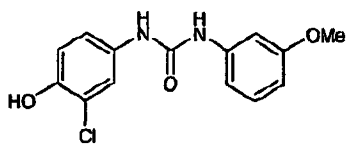 | 1-(3-Chloro-4-hydroxy-phenyl)-3-(3-methoxy-phenyl)-urea | C14H13ClN2O3 | A | 292.72 | 292.9 |
| 692 | 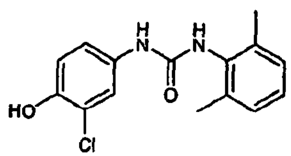 | 1-(3-Chloro-4-hydroxy-phenyl)-3-(2,6-dimethyl-phenyl)-urea | C15H15ClN2O2 | A | 290.75 | 290.9 |

| | | | | | | |
|---|---|---|---|---|---|---|
| 693 | 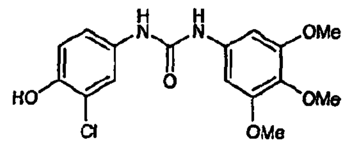 | 1-(3-Chloro-4-hydroxy-phenyl)-3-(3,4,5-trimethoxy-phenyl)-urea | C16H17ClN2O5 | A | 352.77 | 353.2 |
| 694 | 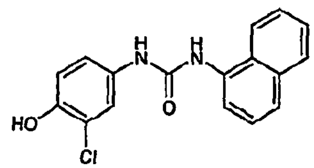 | 1-(3-Chloro-4-hydroxy-phenyl)-3-naphthalen-1-yl-urea | C17H13ClN2O2 | A | 312.75 | 313.1 |
| 695 | 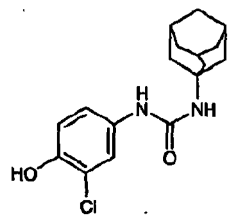 | 1-Adamantan-1-yl-3-(3-chloro-4-hydroxy-phenyl)-urea | C17H21ClN2O2 | A | 320.82 | 321.4 |
| 696 | 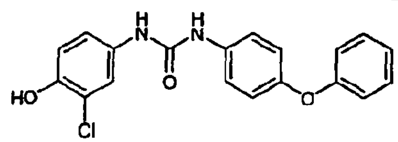 | 1-(3-Chloro-4-hydroxy-phenyl)-3-(4-phenoxy-phenyl)-urea | C19H15ClN2O3 | A | 354.79 | 354.9 |
| 697 | 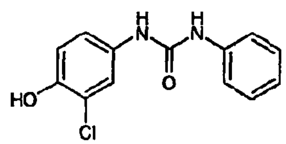 | 1-(3-Chloro-4-hydroxy-phenyl)-3-phenyl-urea | C13H11ClN2O2 | A | 262.70 | 263.2 |

| | | | | | | |
|---|---|---|---|---|---|---|
| 698 | 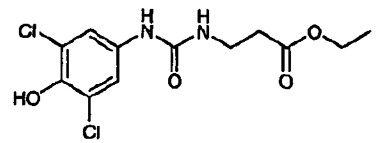 | 3-[3-(3,5-Dichloro-4-hydroxy-phenyl)-ureido]-propionic acid ethyl ester | C12H14Cl2N2O4 | A | 321.16 | 321.9 |
| 699 | 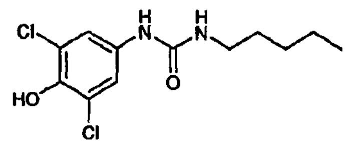 | 1-(3,5-Dichloro-4-hydroxy-phenyl)-3-pentyl-urea | C12H16Cl2N2O2 | A | 291.17 | 292.0 |
| 700 | 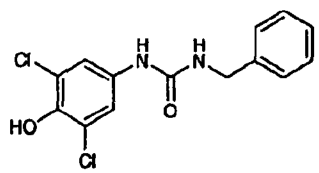 | 1-Benzyl-3-(3,5-dichloro-4-hydroxy-phenyl)-urea | C14H12Cl2N2O2 | A | 311.16 | 312.0 |
| 701 | 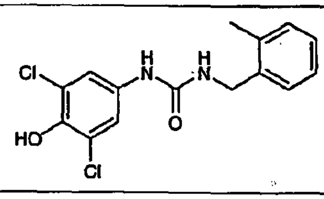 | 1-(3,5-Dichloro-4-hydroxy-phenyl)-3-(2-methyl-benzyl)-urea | C15H14Cl2N2O2 | A | 325.19 | 325.9 |
| 702 | 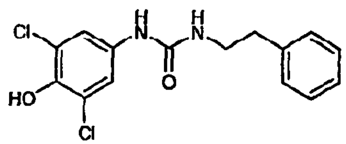 | 1-(3,5-Dichloro-4-hydroxy-phenyl)-3-phenethyl-urea | C15H14Cl2N2O2 | A | 325.19 | 325.9 |
| 703 | 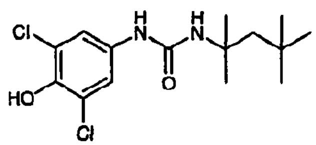 | 1-(3,5-Dichloro-4-hydroxy-phenyl)-3-(1,1,3,3-tetramethyl-butyl)-urea | C15H22Cl2N2O2 | A | 333.25 | 334.0 |

| | | | | | | |
|---|---|---|---|---|---|---|
| 704 | 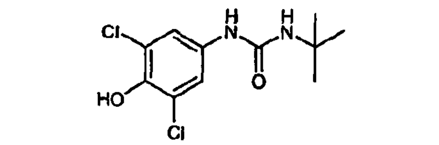 | 1-tert-Butyl-3-(3,5-dichloro-4-hydroxy-phenyl)-urea | C11H14Cl2N2O2 | A | 277.15 | 277.8 |
| 705 | 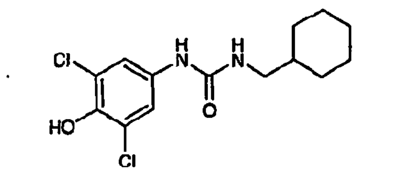 | 1-(3,5-Dichloro-4-hydroxy-phenyl)-3-cyclohexylmethyl-urea | C14H18Cl2N2O2 | A | 317.21 | 318.1 |
| 706 | 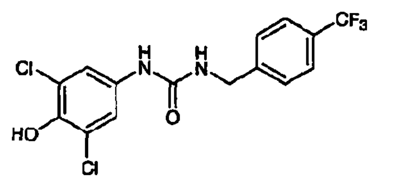 | 1-(3,5-Dichloro-4-hydroxy-phenyl)-3-(4-trifluoromethyl-benzyl)-urea | C15H11Cl2F3N2O2 | A | 379.16 | 379.8 |
| 707 | 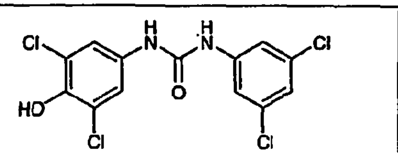 | 1-(3,5-Dichloro-4-hydroxy-phenyl)-3-(3,5-dichloro-phenyl)-urea | C13H8Cl4N2O2 | A | 366.03 | 366.9 |
| 708 | 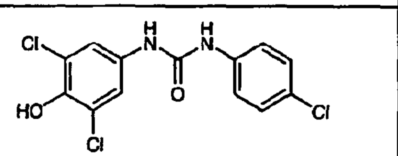 | 1-(3,5-Dichloro-4-hydroxy-phenyl)-3-(4-chloro-phenyl)-urea | C13H9Cl3N2O2 | A | 331.58 | 331.9 |
| 709 | 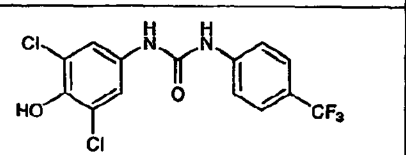 | 1-(3,5-Dichloro-4-hydroxy-phenyl)-3-(4-trifluoromethyl-phenyl)-urea | C14H9Cl2F3N2O2 | A | 365.13 | 365.9 |

| | | | | | | |
|---|---|---|---|---|---|---|
| 710 | 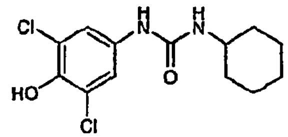 | 1-(3,5-Dichloro-4-hydroxy-phenyl)-3-cyclohexyl-urea | C13H16Cl2N2O2 | A | 303.18 | 304.0 |
| 711 | 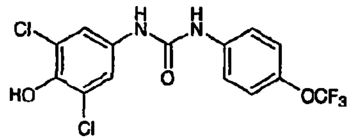 | 1-(3,5-Dichloro-4-hydroxy-phenyl)-3-(4-trifluoromethoxy-phenyl)-urea | C14H9Cl2F3N2O3 | A | 381.13 | 381.8 |
| 712 | 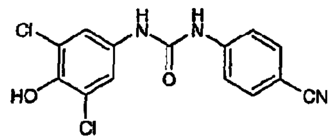 | 1-(3,5-Dichloro-4-hydroxy-phenyl)-3-(4-cyano-phenyl)-urea | C14H9Cl2N3O2 | A | 322.15 | 322.9 |
| 713 | 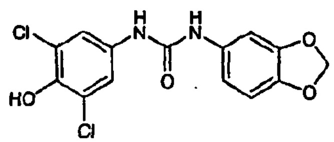 | 1-Benzo[1,3]dioxol-5-yl-3-(3,5-dichloro-4-hydroxy-phenyl)-urea | C14H10Cl2N2O4 | A | 341.15 | 341.7 |
| 714 | 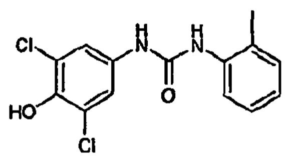 | 1-(3,5-Dichloro-4-hydroxy-phenyl)-3-o-tolyl-urea | C14H12Cl2N2O2 | A | 311.16 | 311.8 |
| 715 | 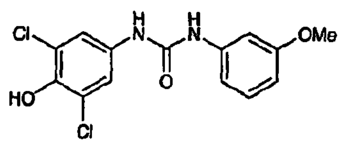 | 1-(3,5-Dichloro-4-hydroxy-phenyl)-3-(3-methoxy-phenyl)-urea | C14H12Cl2N2O3 | A | 327.16 | 327.5 |

| | | | | | | |
|---|---|---|---|---|---|---|
| 716 | 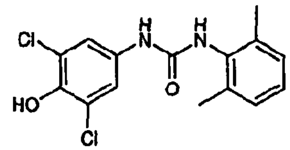 | 1-(3,5-Dichloro-4-hydroxy-phenyl)-3-(2,6-dimethyl-phenyl)-urea | C15H14Cl2N2O2 | A | 325.19 | 325.6 |
| 717 | 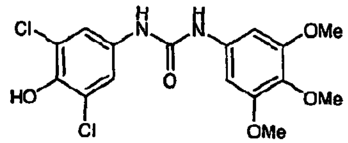 | 1-(3,5-Dichloro-4-hydroxy-phenyl)-3-(3,4,5-trimethoxy-phenyl)-urea | C16H17Cl2N2O5 | A | 387.21 | 387.6 |
| 718 | 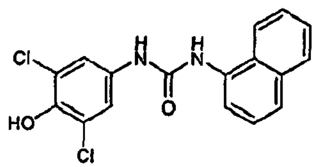 | 1-(3,5-Dichloro-4-hydroxy-phenyl)-3-naphthalen-1-yl-urea | C17H12Cl2N2O2 | A | 347.20 | 347.6 |
| 719 | 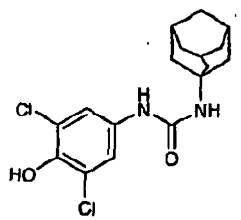 | 1-Adamantan-1-yl-3-(3,5-dichloro-4-hydroxy-phenyl)-urea | C17H20Cl2N2O2 | A | 355.26 | 355.7 |
| 720 | 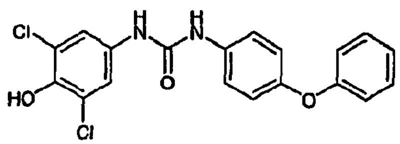 | 1-(3,5-Dichloro-4-hydroxy-phenyl)-3-(4-phenoxy-phenyl)-urea | C19H14Cl2N2O3 | A | 389.23 | 389.6 |

| | | | | | | |
|---|---|---|---|---|---|---|
| 721 | 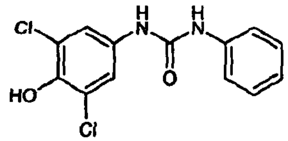 | 1-(3,5-Dichloro-4-hydroxy-phenyl)-3-phenyl-urea | C13H10Cl2N2O2 | A | 297.14 | 297.7 |
| 722 | 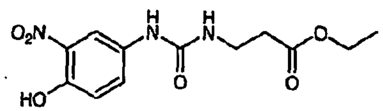 | 3-[3-(4-Hydroxy-3-nitro-phenyl)-ureido]-propionic acid ethyl ester | C12H15N3O6 | A | 297.26 | 297.8 |
| 723 | 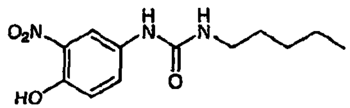 | 1-(4-Hydroxy-3-nitro-phenyl)-3-pentyl-urea | C12H17N3O4 | A | 267.28 | 267.9 |
| 724 | 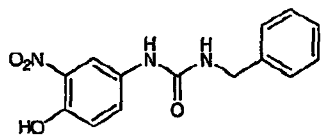 | 1-Benzyl-3-(4-hydroxy-3-nitro-phenyl)-urea | C14H13N3O4 | A | 287.27 | 287.9 |
| 725 | 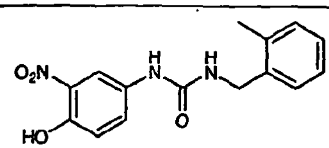 | 1-(4-Hydroxy-3-nitro-phenyl)-3-(2-methyl-benzyl)-urea | C15H15N3O4 | A | 301.30 | 301.8 |
| 726 | 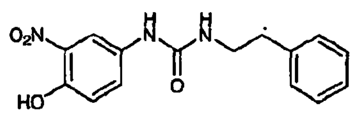 | 1-(4-Hydroxy-3-nitro-phenyl)-3-phenethyl-urea | C15H15N3O4 | A | 301.30 | 301.8 |
| 727 | 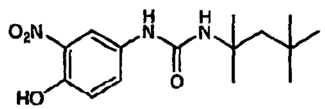 | 1-(4-Hydroxy-3-nitro-phenyl)-3-(1,1,3,3-tetramethyl-butyl)-urea | C15H23N3O4 | A | 309.36 | 309.8 |

| | | | | | | |
|---|---|---|---|---|---|---|
| 728 | 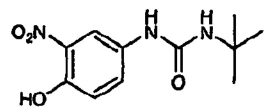 | 1-tert-Butyl-3-(4-hydroxy-3-nitro-phenyl)-urea | C11H15N3O4 | A | 253.25 | 253.8 |
| 729 | 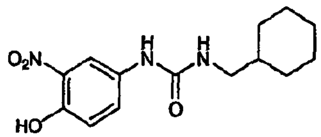 | 1-Cyclohexylmethyl-3-(4-hydroxy-3-nitro-phenyl)-urea | C14H19N3O4 | A | 293.32 | 293.9 |
| 730 | 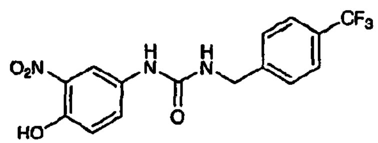 | 1-(4-Hydroxy-3-nitro-phenyl)-3-(4-trifluoromethyl-benzyl)-urea | C15H12F3N3O4 | A | 355.27 | 355.8 |
| 731 | 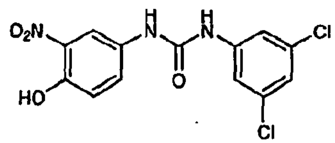 | 1-(3,5-Dichloro-phenyl)-3-(4-hydroxy-3-nitro-phenyl)-urea | C13H9Cl2N3O4 | A | 342.13 | 342.7 |
| 732 | 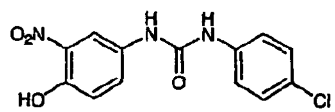 | 1-(4-Chloro-phenyl)-3-(4-hydroxy-3-nitro-phenyl)-urea | C13H10ClN3O4 | A | 307.69 | 308.4 |
| 733 | 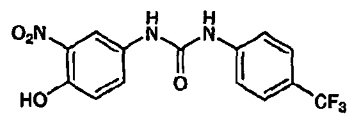 | 1-(4-Hydroxy-3-nitro-phenyl)-3-(4-trifluoromethyl-phenyl)-urea | C14H10F3N3O4 | A | 341.24 | 341.8 |
| 734 | 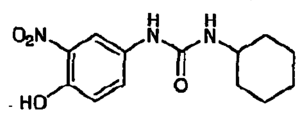 | 1-Cyclohexyl-3-(4-hydroxy-3-nitro-phenyl)-urea | C13H17N3O4 | A | 279.29 | 279.8 |

| | | | | | | |
|---|---|---|---|---|---|---|
| 735 | 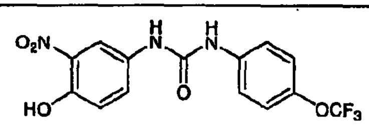 | 1-(4-Hydroxy-3-nitro-phenyl)-3-(4-trifluoromethoxy-phenyl)-urea | C14H10F3N3O5 | A | 357.24 | 357.9 |
| 736 | 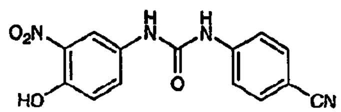 | 1-(4-Cyano-phenyl)-3-(4-hydroxy-3-nitro-phenyl)-urea | C14H10N4O4 | A | 298.25 | 298.9 |
| 737 | 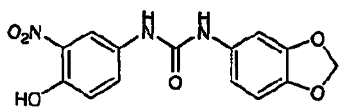 | 1-Benzo[1,3]dioxol-5-yl-3-(4-hydroxy-3-nitro-phenyl)-urea | C14H11N3O6 | A | 317.25 | 317.9 |
| 738 | 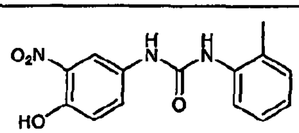 | 1-(4-Hydroxy-3-nitro-phenyl)-3-o-tolyl-urea | C14H13N3O4 | A | 287.27 | 287.9 |
| 739 | 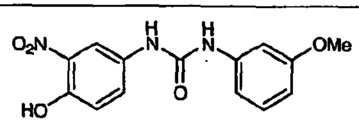 | 1-(4-Hydroxy-3-nitro-phenyl)-3-(3-methoxy-phenyl)-urea | C14H13N3O5 | A | 303.27 | 303.8 |
| 740 | 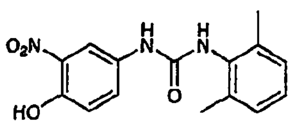 | 1-(2,6-Dimethyl-phenyl)-3-(4-hydroxy-3-nitro-phenyl)-urea | C15H15N3O4 | A | 301.30 | 301.9 |
| 741 | 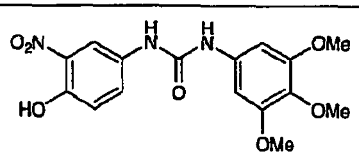 | 1-(4-Hydroxy-3-nitro-phenyl)-3-(3,4,5-trimethoxy-phenyl)-urea | C16H17N3O7 | A | 363.32 | 364.0 |

| | | | | | | |
|---|---|---|---|---|---|---|
| 742 | 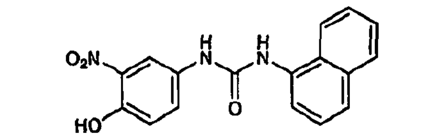 | 1-(4-Hydroxy-3-nitro-phenyl)-3-naphthalen-1-yl-urea | C17H13N3O4 | A | 323.30 | 323.9 |
| 743 | 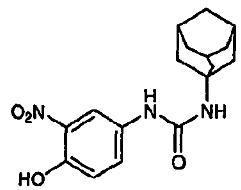 | 1-Adamantan-1-yl-3-(4-hydroxy-3-nitro-phenyl)-urea | C17H21N3O4 | A | 331.37 | 331.9 |
| 744 | 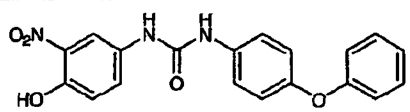 | 1-(4-Hydroxy-3-nitro-phenyl)-3-(4-phenoxy-phenyl)-urea | C19H15N3O5 | A | 365.34 | 366.0 |
| 745 | 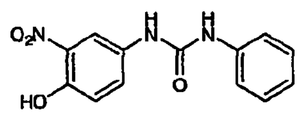 | 1-(4-Hydroxy-3-nitro-phenyl)-3-phenyl-urea | C13H11N3O4 | A | 273.24 | 273.9 |
| 746 | 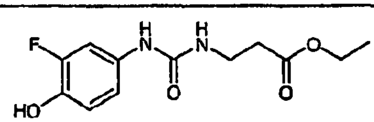 | 3-[3-(3-Fluoro-4-hydroxy-phenyl)-ureido]-propionic acid ethyl ester | C12H15FN2O4 | A | 270.26 | 270.6 |
| 747 | 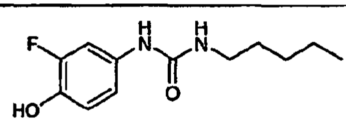 | 1-(3-Fluoro-4-hydroxy-phenyl)-3-pentyl-urea | C12H17FN2O2 | A | 240.27 | 240.5 |
| 748 | 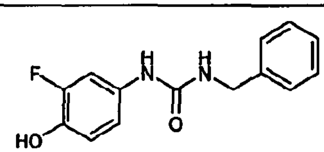 | 1-Benzyl-3-(3-fluoro-4-hydroxy-phenyl)-urea | C14H13FN2O2 | A | 260.26 | 260.8 |

| | | | | | | |
|---|---|---|---|---|---|---|
| 749 | 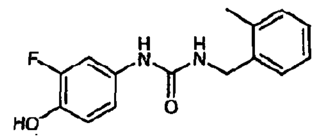 | 1-(3-Fluoro-4-hydroxy-phenyl)-3-(2-methyl-benzyl)-urea | C15H15FN2O2 | A | 274.29 | 274.9 |
| 750 | 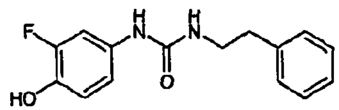 | 1-(3-Fluoro-4-hydroxy-phenyl)-3-phenethyl-urea | C15H15FN2O2 | A | 274.29 | 274.6 |
| 751 | 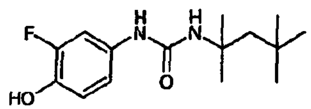 | 1-(3-Fluoro-4-hydroxy-phenyl)-3-(1,1,3,3-tetramethyl-butyl)-urea | C15H23FN2O2 | A | 282.35 | 282.9 |
| 752 | 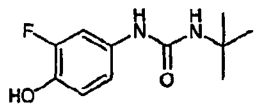 | 1-tert-Butyl-3-(3-fluoro-4-hydroxy-phenyl)-urea | C11H15FN2O2 | A | 226.25 | 226.5 |
| 753 | 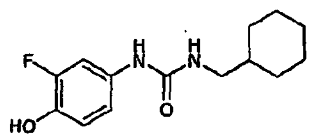 | 1-(3-Fluoro-4-hydroxy-phenyl)-3-cyclohexylmethyl-urea | C14H19FN2O2 | A | 266.31 | 266.7 |
| 754 | 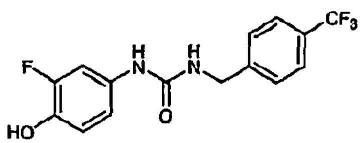 | 1-(3-Fluoro-4-hydroxy-phenyl)-3-(4-trifluoromethyl-benzyl)-urea | C15H12F4N2O2 | A | 328.26 | 328.4 |
| 755 | 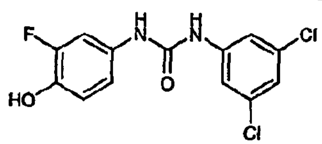 | 1-(3-Fluoro-4-hydroxy-phenyl)-3-(3,5-dichloro-phenyl)-urea | C13H9Cl2FN2O2 | A | 315.13 | 315.5 |

| | | | | | | |
|---|---|---|---|---|---|---|
| 756 | 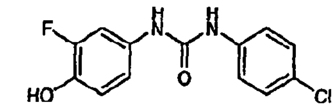 | 1-(3-Fluoro-4-hydroxy-phenyl)-3-(4-chloro-phenyl)-urea | C13H10ClFN2O2 | A | 280.68 | 281.1 |
| 757 | 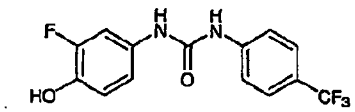 | 1-(3-Fluoro-2-hydroxy-phenyl)-3-(4-trifluoromethyl-phenyl)-urea | C14H10F4N2O2 | A | 314.24 | 314.6 |
| 758 | 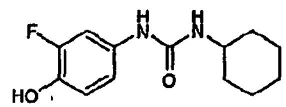 | 1-(3-Fluoro-4-hydroxy-phenyl)-3-cyclohexyl-urea | C13H17FN2O2 | A | 252.28 | 252.6 |
| 759 | 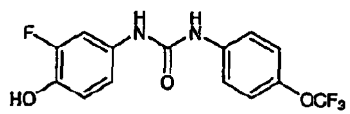 | 1-(3-Fluoro-4-hydroxy-phenyl)-3-(4-trifluoromethoxy-phenyl)-urea | C14H10FF3N2O3 | A | 330.23 | 330.5 |
| 760 | 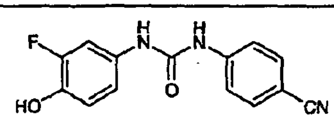 | 1-(3-Fluoro-4-hydroxy-phenyl)-3-(4-cyano-phenyl)-urea | C14H10FN3O2 | A | 271.25 | 271.5 |
| 761 | 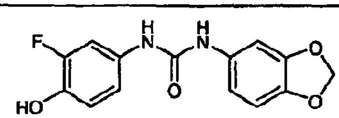 | 1-Benzo[1,3]dioxol-5-yl-3-(3-fluoro-4-hydroxy-phenyl)-urea | C14H11FN2O4 | A | 290.25 | 290.7 |
| 762 | 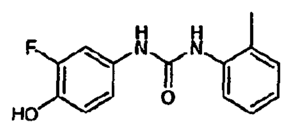 | 1-(3-Fluoro-4-hydroxy-phenyl)-3-o-tolyl-urea | C14H13FN2O2 | A | 260.26 | 260.5 |
| 763 | 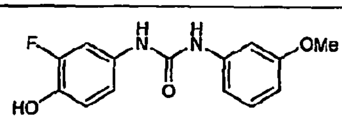 | 1-(3-Fluoro-4-hydroxy-phenyl)-3-(3-methoxy-phenyl)-urea | C14H13FN2O3 | A | 276.26 | 276.5 |

| | | | | | | |
|---|---|---|---|---|---|---|
| 764 | 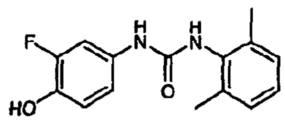 | 1-(3-Fluoro-4-hydroxy-phenyl)-3-(2,6-dimethyl-phenyl)-urea | C15H15FN2O2 | A | 274.29 | 274.7 |
| 765 | 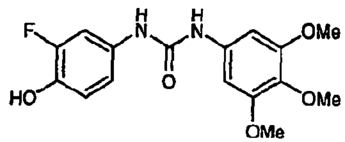 | 1-(3-Fluoro-4-hydroxy-phenyl)-3-(3,4,5-trimethoxy-phenyl)-urea | C16H17FN2O5 | A | 336.31 | 336.6 |
| 766 | 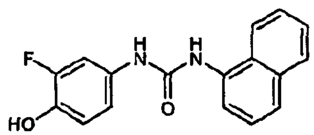 | 1-(3-Fluoro-4-hydroxy-phenyl)-3-naphthalen-1-yl-urea | C17H13FN2O2 | A | 296.3 | 296.6 |
| 767 | 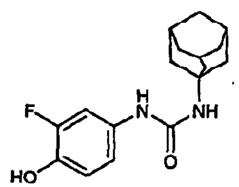 | 1-Adamantan-1-yl-3-(3-fluoro-4-hydroxy-phenyl)-urea | C17H21FN2O2 | A | 304.36 | 304.6 |
| 768 | 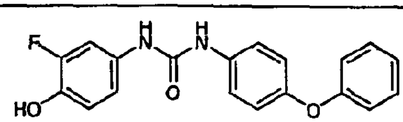 | 1-(3-Fluoro-4-hydroxy-phenyl)-3-(4-phenoxy-phenyl)-urea | C19H15FN2O3 | A | 338.33 | 338.6 |
| 769 | 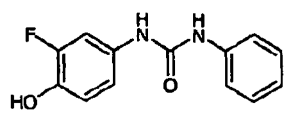 | 1-(3-Fluoro-4-hydroxy-phenyl)-3-phenyl-urea | C13H11FN2O2 | A | 246.24 | 246.5 |

| | | | | | | |
|---|---|---|---|---|---|---|
| 770 | 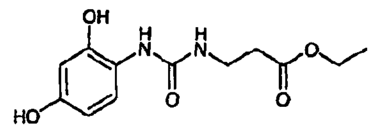 | 3-[3-(2,4-Dihydroxy-phenyl)-ureido]-propionic acid ethyl ester | C12H16N2O5 | A | 268.27 | 268.7 |
| 771 | 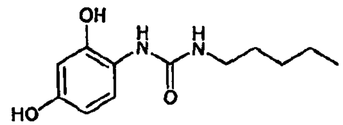 | 1-(2,4-Dihydroxy-phenyl)-3-pentyl-urea | C12H18N2O3 | A | 238.28 | 238.6 |
| 772 | 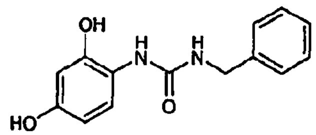 | 1-Benzyl-3-(2,4-dihydroxy-phenyl)-urea | C14H14N2O3 | A | 258.27 | 258.6 |
| 773 | 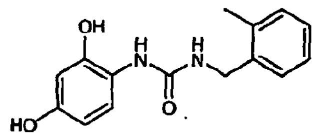 | 1-(2,4-Dihydroxy-phenyl)-3-(2-methyl-benzyl)-urea | C15H16N2O3 | A | 272.30 | 272.5 |
| 774 | 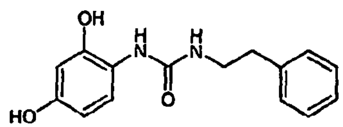 | 1-(2,4-Dihydroxy-phenyl)-3-phenethyl-urea | C15H16N2O3 | A | 272.30 | 272.5 |
| 775 | 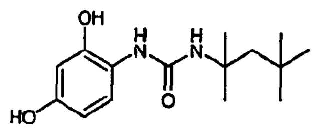 | 1-(2,4-Dihydroxy-phenyl)-3-(1,1,3,3-tetramethyl-butyl)-urea | C15H24N2O3 | A | 280.36 | 280.7 |
| 776 | 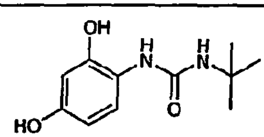 | 1-tert-Butyl-3-(2,4-dihydroxy-phenyl)-urea | C11H16N2O3 | A | 224.26 | 224.8 |

| | | | | | | |
|---|---|---|---|---|---|---|
| 777 | 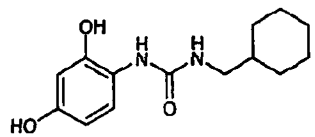 | 1-Cyclohexylmethyl-3-(2,4-dihydroxy-phenyl)-urea | C14H20N2O3 | A | 264.32 | 264.7 |
| 778 | 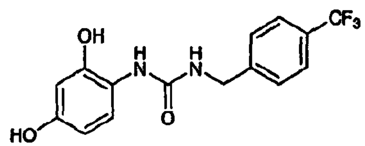 | 1-(2,4-Dihydroxy-phenyl)-3-(4-trifluoromethyl-benzyl)-urea | C15H13F3N2O3 | A | 326.27 | 326.8 |
| 779 | 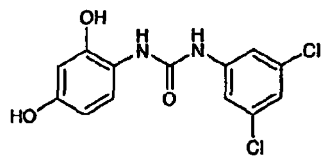 | 1-(3,5-Dichloro-phenyl)-3-(2,4-dihydroxy-phenyl)-urea | C13H10Cl2N2O3 | A | 313.14 | 313.7 |
| 780 | 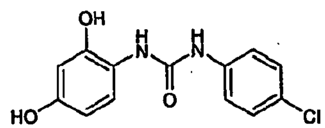 | 1-(4-Chloro-phenyl)-3-(2,4-dihydroxy-phenyl)-urea | C13H11ClN2O3 | A | 278.69 | 279.2 |
| 781 | 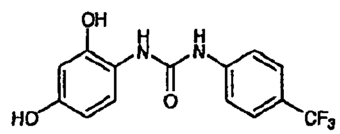 | 1-(2,4-Dihydroxy-phenyl)-3-(4-trifluoromethyl-phenyl)-urea | C14H11F3N2O3 | A | 312.24 | 312.8 |
| 782 | 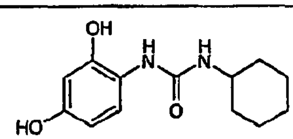 | 1-Cyclohexyl-3-(2,4-dihydroxy-phenyl)-urea | C13H18N2O3 | A | 250.29 | 250.8 |

| | | | | | | |
|---|---|---|---|---|---|---|
| 783 | 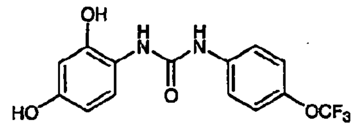 | 1-(2,4-Dihydroxy-phenyl)-3-(4-trifluoromethoxy-phenyl)-urea | C14H11F3N2O4 | A | 328.24 | 328.7 |
| 784 | 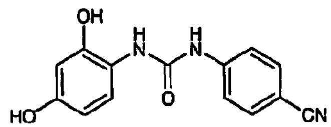 | 1-(4-Cyano-phenyl)-3-(2,4-dihydroxy-phenyl)-urea | C14H11N3O3 | A | 269.26 | 269.8 |
| 785 | 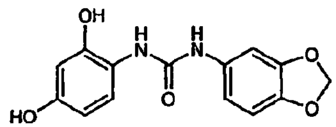 | 1-Benzo[1,3]dioxol-5-yl-3-(2,4-dihydroxy-phenyl)-urea | C14H12N2O5 | A | 288.26 | 288.9 |
| 786 | 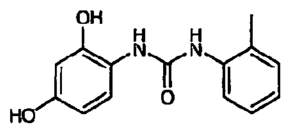 | 1-(2,4-Dihydroxy-phenyl)-3-o-tolyl-urea | C14H14N2O3 | A | 258.27 | 258.7 |
| 787 | 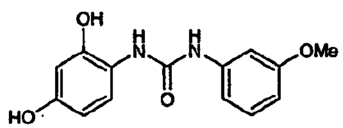 | 1-(2,4-Dihydroxy-phenyl)-3-(3-methoxy-phenyl)-urea | C14H14N2O4 | A | 274.27 | 274.8 |
| 788 | 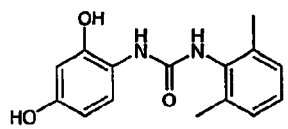 | 1-(2,4-Dihydroxy-phenyl)-3-(2,6-dimethyl-phenyl)-urea | C15H16N2O3 | A | 272.30 | 272.9 |
| 789 | 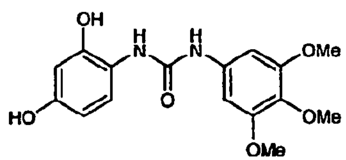 | 1-(2,4-Dihydroxy-phenyl)-3-(3,4,5-trimethoxy-phenyl)-urea | C16H18N2O6 | A | 334.32 | 334.8 |

| | | | | | | |
|---|---|---|---|---|---|---|
| 790 | 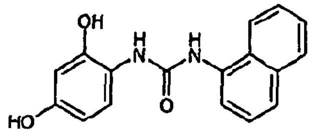 | 1-(2,4-Dihydroxy-phenyl)-3-naphthalen-1-yl-urea | C17H14N2O3 | A | 294.30 | 294.9 |
| 791 | 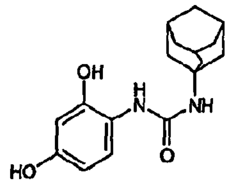 | 1-Adamantan-1-yl-3-(2,4-dihydroxy-phenyl)-urea | C17H22N2O3 | A | 302.37 | 302.8 |
| 792 | 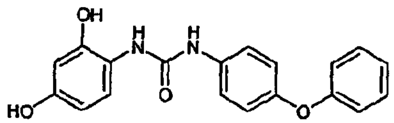 | 1-(2,4-Dihydroxy-phenyl)-3-(4-phenoxy-phenyl)-urea | C19H16N2O4 | A | 336.34 | 336.9 |
| 793 | 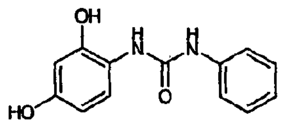 | 1-(2,4-Dihydroxy-phenyl)-3-phenyl-urea | C13H12N2O3 | A | 244.25 | 244.7 |
| 794 | 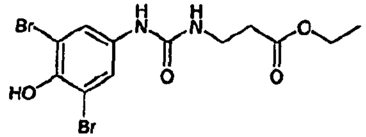 | 3-[3-(3,5-Dibromo-4-hydroxy-phenyl)-ureido]-propionic acid ethyl ester | C12H14Br2N2O4 | A | 410.06 | 410.4 |
| 795 | 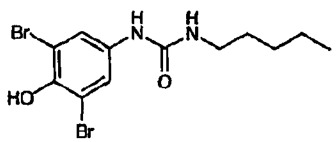 | 1-(3,5-Dibromo-4-hydroxy-phenyl)-3-pentyl-urea | C12H16Br2N2O2 | A | 380.08 | 380.5 |

| | | | | | | |
|---|---|---|---|---|---|---|
| 796 | 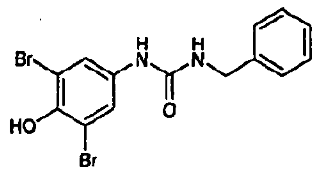 | 1-Benzyl-3-(3,5-Dibromo-4-hydroxy-phenyl)-urea | $C_{14}H_{12}Br_2N_2O_2$ | A | 400.07 | 400.4 |
| 797 | 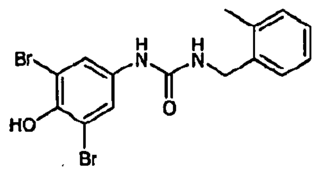 | 1-(5-Bromo-3-fluoro-2-hydroxy-phenyl)-3-(2-methyl-benzyl)-urea | $C_{15}H_{14}Br_2N_2O_2$ | A | 414.09 | 414.5 |
| 798 | 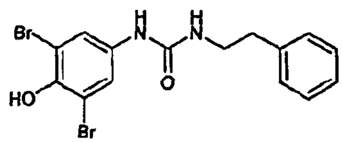 | 1-(3,5-Dibromo-4-hydroxy-phenyl)-3-phenethyl-urea | $C_{15}H_{14}Br_2N_2O_2$ | A | 414.09 | 414.4 |
| 799 | 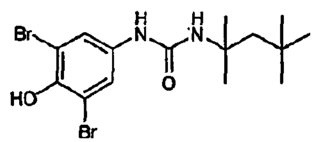 | 1-(3,5-Dibromo-4-hydroxy-phenyl)-3-(1,1,3,3-tetramethyl-butyl)-urea | $C_{15}H_{22}Br_2N_2O_2$ | A | 422.16 | 422.5 |
| 800 | 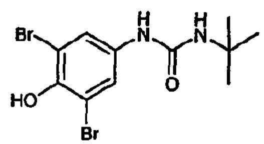 | 1-tert-Butyl-3-(3,5-dibromo-4-hydroxy-phenyl)-urea | $C_{11}H_{14}Br_2N_2O_2$ | A | 366.05 | 366.4 |
| 801 | 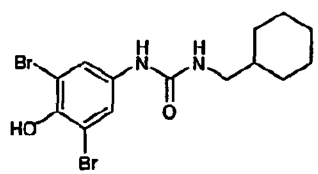 | 1-(3,5-Dibromo-4-hydroxy-phenyl)-3-cyclohexylmethyl-urea | $C_{14}H_{18}Br_2N_2O_2$ | A | 406.11 | 406.5 |

| | | | | | | |
|---|---|---|---|---|---|---|
| 802 | 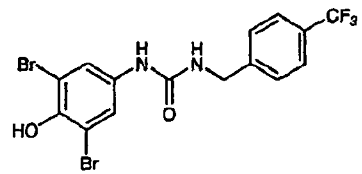 | 1-(3,5-Dibromo-4-hydroxy-phenyl)-3-(4-trifluoromethyl-benzyl)-urea | C15H11Br2F3N2O2 | A | 468.06 | 468.4 |
| 803 | 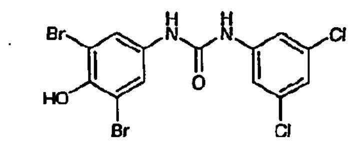 | 1-(3,5-Dibromo-4-hydroxy-phenyl)-3-(3,5-dichloro-phenyl)-urea | C13H8Br2Cl2N2O2 | A | 454.93 | 455.4 |
| 804 | 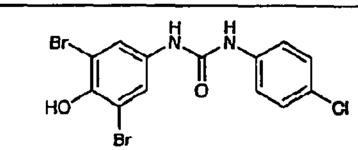 | 1-(3,5-Dibromo-4-hydroxy-phenyl)-3-(4-chloro-phenyl)-urea | C13H9Br2ClN2O2 | A | 420.48 | 420.7 |
| 805 | 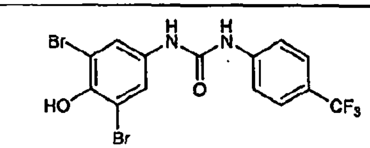 | 1-(3,5-Dibromo-4-hydroxy-phenyl)-3-(4-trifluoromethyl-phenyl)-urea | C14H9Br2F3N2O2 | A | 454.04 | 454.3 |
| 806 | 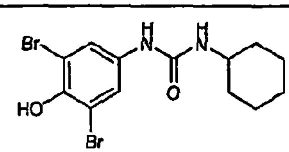 | 1-(3,5-Dibromo-4-hydroxy-phenyl)-3-cyclohexyl-urea | C13H16Br2N2O2 | A | 392.09 | 392.5 |
| 807 | 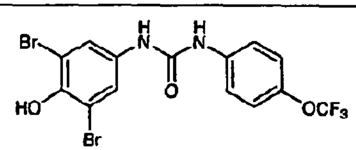 | 1-(3,5-Dibromo-4-hydroxy-phenyl)-3-(4-trifluoromethoxy-phenyl)-urea | C14H9Br2F3N2O3 | A | 470.04 | 470.3 |

| | | | | | | |
|---|---|---|---|---|---|---|
| 808 | 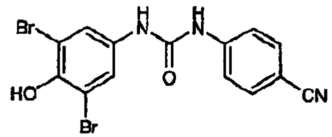 | 1-(3,5-Dibromo-4-hydroxy-phenyl)-3-(4-cyano-phenyl)-urea | C14H9Br2N3O2 | A | 411.05 | 411.5 |
| 809 | 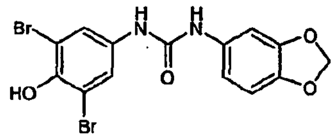 | 1-Benzo[1,3]dioxol-5-yl-3-(3,5-dibromo-4-hydroxy-phenyl)-urea | C14H10Br2N2O4 | A | 430.05 | 430.3 |
| 810 | 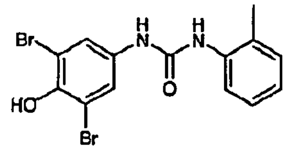 | 1-(3,5-Dibromo-4-hydroxy-phenyl)-3-o-tolyl-urea | C14H12Br2N2O2 | A | 400.07 | 400.4 |
| 811 | 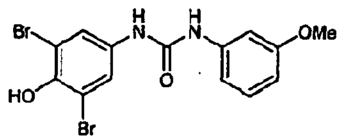 | 1-(3,5-Dibromo-4-hydroxy-phenyl)-3-(3-methoxy-phenyl)-urea | C14H12Br2N2O3 | A | 416.06 | 416.3 |
| 812 | 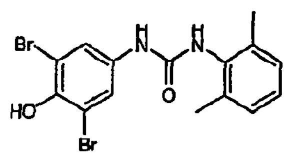 | 1-(3,5-Dibromo-4-hydroxy-phenyl)-3-(2,6-dimethyl-phenyl)-urea | C15H14Br2N2O2 | A | 414.09 | 414.5 |
| 813 | 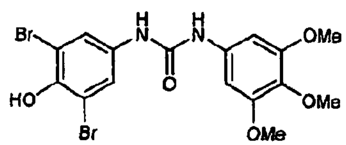 | 1-(3,5-Dibromo-4-hydroxy-phenyl)-3-(3,4,5-trimethoxy-phenyl)-urea | C16H17Br2N2O5 | A | 476.12 | 476.4 |

| | | | | | | |
|---|---|---|---|---|---|---|
| 814 | 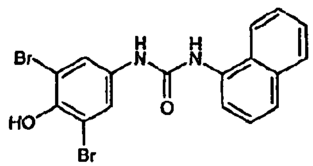 | 1-(3,5-Dibromo-4-hydroxy-phenyl)-3-naphthalen-1-yl-urea | $C_{17}H_{12}Br_2N_2O_2$ | A | 436.10 | 436.4 |
| 815 | 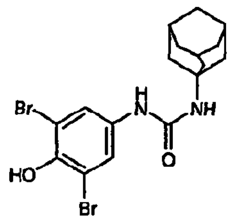 | 1-Adamantan-1-yl-3-(3,5-dibromo-4-hydroxy-phenyl)-urea | $C_{17}H_{20}Br_2N_2O_2$ | A | 444.16 | 444.2 |
| 816 | 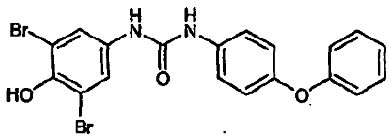 | 1-(3,5-Dibromo-4-hydroxy-phenyl)-3-(4-phenoxy-phenyl)-urea | $C_{19}H_{14}Br_2N_2O_3$ | A | 478.13 | 478.3 |
| 817 | 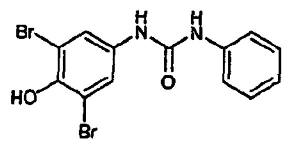 | 1-(3,5-Dibromo-4-hydroxy-phenyl)-3-phenyl-urea | $C_{13}H_{10}Br_2N_2O_2$ | A | 386.04 | 386.2 |
| 818 | 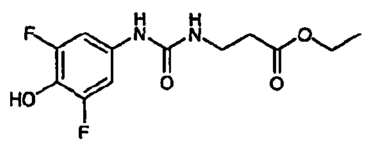 | 3-[3-(3,5-Difluoro-4-hydroxy-phenyl)-ureido]-propionic acid ethyl ester | $C_{12}H_{14}F_2N_2O_4$ | A | 288.25 | 288.9 |

| | | | | | | |
|---|---|---|---|---|---|---|
| 819 | 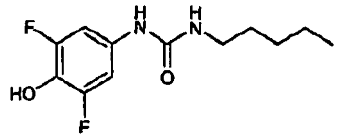 | 1-(3,5-Difluoro-4-hydroxy-phenyl)-3-pentyl-urea | C12H16F2N2O2 | A | 258.26 | 258.9 |
| 820 | 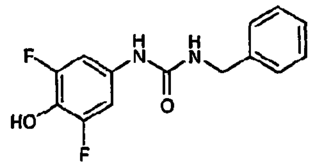 | 1-Benzyl-3-(3,5-difluoro-4-hydroxy-phenyl)-urea | C14H12F2N2O2 | A | 278.25 | 278.8 |
| 821 | 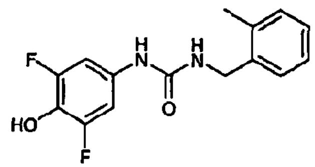 | 1-(3,5-Difluoro-4-hydroxy-phenyl)-3-(2-methyl-benzyl)-urea | C15H14F2N2O2 | A | 292.28 | 292.9 |
| 822 | 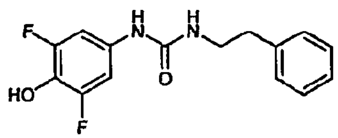 | 1-(3,5-Difluoro-4-hydroxy-phenyl)-3-phenethyl-urea | C15H14F2N2O2 | A | 292.28 | 292.9 |
| 823 | 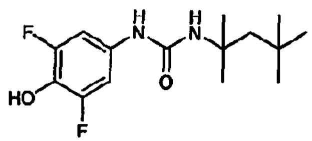 | 1-(3,5-Difluoro-4-hydroxy-phenyl)-3-(1,1,3,3-tetramethyl-butyl)-urea | C15H22F2N2O2 | A | 300.34 | 300.5 |
| 824 | 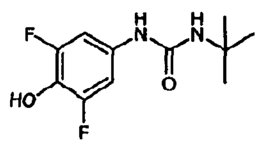 | 1-tert-Butyl-3-(3,5-difluoro-4-hydroxy-phenyl)-urea | C11H14F2N2O2 | A | 244.24 | 244.3 |

| | | | | | | |
|---|---|---|---|---|---|---|
| 825 | 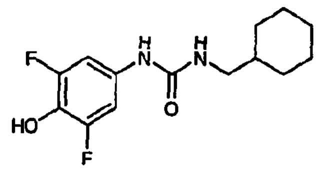 | 1-(3,5-Difluoro-4-hydroxy-phenyl)-3-cyclohexylmethyl-urea | C14H18F2N2O2 | A | 284.30 | 284.6 |
| 826 | 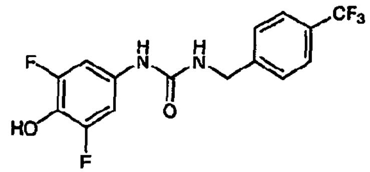 | 1-(3,5-Difluoro-4-hydroxy-phenyl)-3-(4-trifluoromethyl-benzyl)-urea | C15H11F5N2O2 | A | 346.25 | 346.5 |
| 827 | 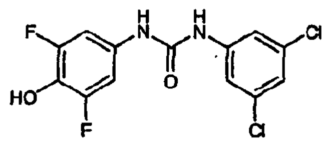 | 1-(3,5-Difluoro-4-hydroxy-phenyl)-3-(3,5-dichloro-phenyl)-urea | C13H8Cl2F2N2O2 | A | 333.12 | 333.3 |
| 828 | 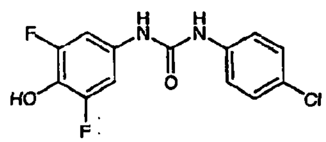 | 1-(3,5-Difluoro-4-hydroxy-phenyl)-3-(4-chloro-phenyl)-urea | C13H9ClF2N2O2 | A | 298.67 | 298.9 |
| 829 | 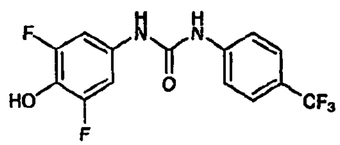 | 1-(3,5-Difluoro-4-hydroxy-phenyl)-3-(4-trifluoromethyl-phenyl)-urea | C14H9F5N2O2 | A | 332.23 | 332.7 |
| 830 | 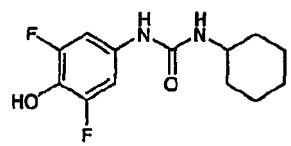 | 1-(3,5-Difluoro-4-hydroxy-phenyl)-3-cyclohexyl-urea | C13H16F2N2O2 | A | 270.28 | 270.7 |

| | | | | | | |
|---|---|---|---|---|---|---|
| 831 | 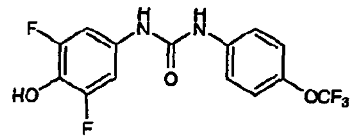 | 1-(3,5-Difluoro-4-hydroxy-phenyl)-3-(4-trifluoromethoxy-phenyl)-urea | C14H9F2F3N2O3 | A | 348.22 | 348.6 |
| 832 | 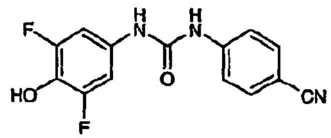 | 1-(3,5-Difluoro-4-hydroxy-phenyl)-3-(4-cyano-phenyl)-urea | C14H9F2N3O2 | A | 289.24 | 289.5 |
| 833 | 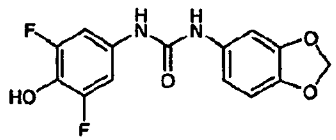 | 1-Benzo[1,3]dioxol-5-yl-3-(3,5-difluoro-4-hydroxy-phenyl)-urea | C14H10F2N2O4 | A | 308.24 | 308.5 |
| 834 | 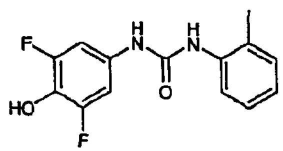 | 1-(3,5-Difluoro-4-hydroxy-phenyl)-3-o-tolyl-urea | C14H12F2N2O2 | A | 278.25 | 278.6 |
| 835 | 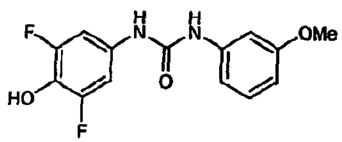 | 1-(3,5-Difluoro-4-hydroxy-phenyl)-3-(3-methoxy-phenyl)-urea | C14H12F2N2O3 | A | 294.25 | 294.5 |
| 836 | 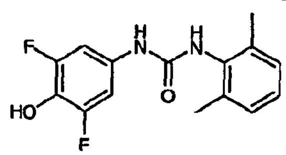 | 1-(3,5-Difluoro-4-hydroxy-phenyl)-3-(2,6-dimethyl-phenyl)-urea | C15H14F2N2O2 | A | 292.28 | 292.6 |

| | | | | | | |
|---|---|---|---|---|---|---|
| 837 | 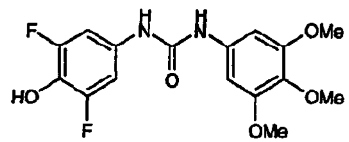 | 1-(3,5-Difluoro-4-hydroxy-phenyl)-3-(3,4,5-trimethoxy-phenyl)-urea | C16H17F2N2O5 | A | 354.31 | 354.6 |
| 838 | 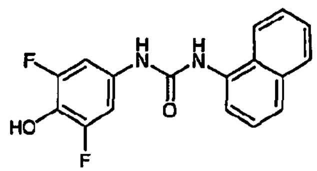 | 1-(3,5-Difluoro-4-hydroxy-phenyl)-3-naphthalen-1-yl-urea | C17H12F2N2O2 | A | 314.29 | 314.6 |
| 839 | 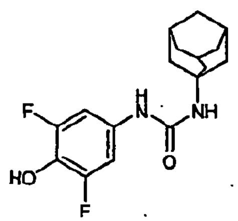 | 1-Adamantan-1-yl-3-(3,5-difluoro-4-hydroxy-phenyl)-urea | C17H20F2N2O2 | A | 322.35 | 322.7 |
| 840 | 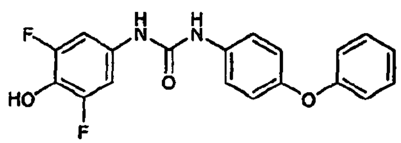 | 1-(3,5-Difluoro-4-hydroxy-phenyl)-3-(4-phenoxy-phenyl)-urea | C19H14F2N2O3 | A | 356.32 | 356.6 |
| 841 | 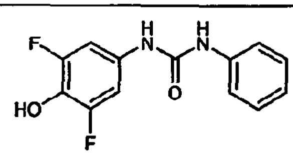 | 1-(3,5-Difluoro-4-hydroxy-phenyl)-3-phenyl-urea | C13H10F2N2O2 | A | 264.23 | 264.7 |

| | | | | | | |
|---|---|---|---|---|---|---|
| 842 | 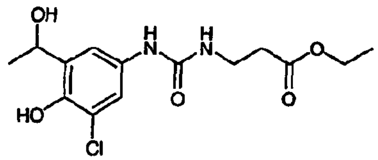 | 3-{3-[3-Chloro-4-hydroxy-5-(1-hydroxy-ethyl)-phenyl]-ureido}-propionic acid ethyl ester | C14H19ClN2O5 | A | 330.76 | 331.1 |
| 843 | 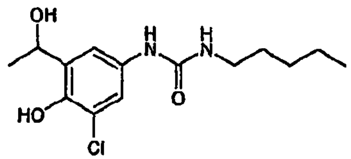 | 1-[3-Chloro-4-hydroxy-5-(1-hydroxy-ethyl)-phenyl]-3-pentyl-urea | C14H21ClN2O3 | A | 300.78 | 301.2 |
| 844 | 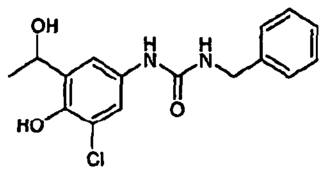 | 1-[3-Chloro-4-hydroxy-5-(1-hydroxy-ethyl)-phenyl]-3-pentyl-urea | C16H17ClN2O3 | A | 320.77 | 321.0 |
| 845 | 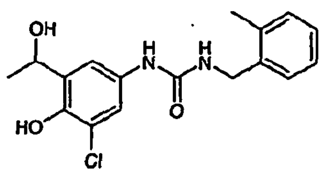 | 1-[3-Chloro-4-hydroxy-5-(1-hydroxy-ethyl)-phenyl]-3-(2-methyl-benzyl)-urea | C17H19ClN2O3 | A | 334.80 | 335.1 |
| 846 | 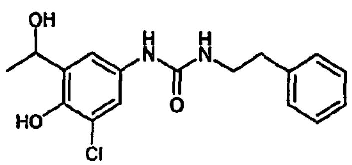 | 1-[3-Chloro-4-hydroxy-5-(1-hydroxy-ethyl)-phenyl]-3-phenethyl-urea | C17H19ClN2O3 | A | 334.80 | 335.3 |

| | | | | | | |
|---|---|---|---|---|---|---|
| 847 | 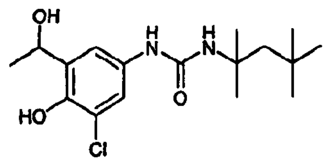 | 1-[3-Chloro-4-hydroxy-5-(1-hydroxy-ethyl)-phenyl]-3-(1,1,3,3-tetramethyl-butyl)-urea | C17H27Cl2N2O2 | A | 342.86 | 343.7 |
| 848 | 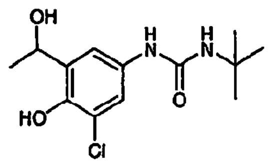 | 1-tert-Butyl-3-[3-chloro-4-hydroxy-5-(1-hydroxy-ethyl)-phenyl]-urea | C13H19ClN2O3 | A | 286.75 | 287.6 |
| 859 | 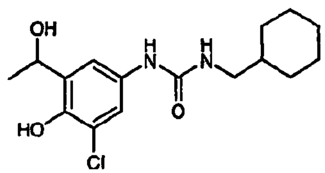 | 1-[3-Chloro-4-hydroxy-5-(1-hydroxy-ethyl)-phenyl]-3-cyclohexylmethyl-urea | C16H23ClN2O3 | A | 326.82 | 327.5 |
| 850 | 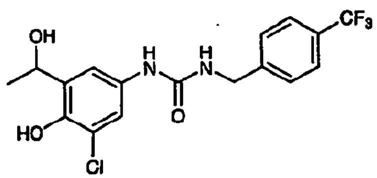 | 1-[3-Chloro-4-hydroxy-5-(1-hydroxy-ethyl)-phenyl]-3-(4-trifluoromethyl-benzyl)-urea | C17H16ClF3N2O3 | A | 388.77 | 389.4 |
| 851 | 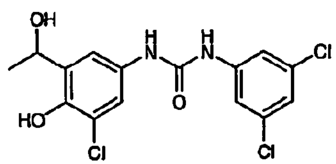 | 1-[3-Chloro-4-hydroxy-5-(1-hydroxy-ethyl)-phenyl]-3-(4-chloro-phenyl)-urea | C15H13Cl3N2O3 | A | 375.63 | 376.2 |

| | | | | | | |
|---|---|---|---|---|---|---|
| 852 | 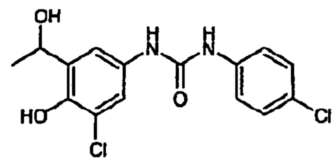 | 1-[3-Chloro-4-hydroxy-5-(1-hydroxy-ethyl)-phenyl]-3-(4-chloro-phenyl)-urea | C15H14Cl2N2O3 | A | 341.19 | 341.6 |
| 853 | 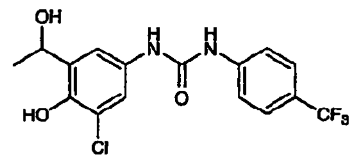 | 1-[3-Chloro-4-hydroxy-5-(1-hydroxy-ethyl)-phenyl]-3-(4-trifluoromethyl-phenyl)-urea | C16H14ClF3N2O3 | A | 374.74 | 375.0 |
| 854 | 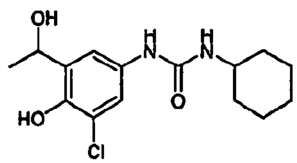 | 1-[3-Chloro-4-hydroxy-5-(1-hydroxy-ethyl)-phenyl]-3-cyclohexyl-urea | C15H21ClN2O3 | A | 312.79 | 313.0 |
| 855 | 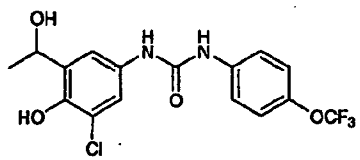 | 1-[3-Chloro-4-hydroxy-5-(1-hydroxy-ethyl)-phenyl]-3-(4-trifluoromethoxy-phenyl)-urea | C16H14ClF3N2O4 | A | 390.74 | 391.2 |
| 856 | 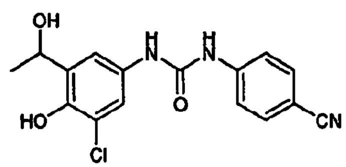 | 1-[3-Chloro-4-hydroxy-5-(1-hydroxy-ethyl)-phenyl]-3-(4-cyano-phenyl)-urea | C16H14ClN3O3 | A | 331.75 | 332.0 |

| | | | | | | |
|---|---|---|---|---|---|---|
| 857 | 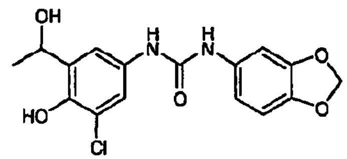 | 1-Benzo[1,3]dioxol-5-yl-3-[3-chloro-4-hydroxy-5-(1-hydroxy-ethyl)-phenyl]-urea | $C_{16}H_{15}ClN_2O_5$ | A | 350.75 | 351.1 |
| 858 | 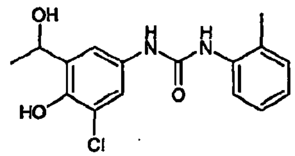 | 1-[3-Chloro-4-hydroxy-5-(1-hydroxy-ethyl)-phenyl]-3-o-tolyl-urea | $C_{16}H_{17}ClN_2O_3$ | A | 320.77 | 321.0 |
| 859 | 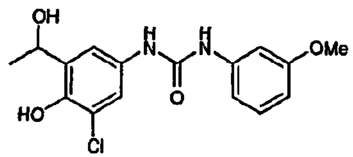 | 1-[3-Chloro-4-hydroxy-5-(1-hydroxy-ethyl)-phenyl]-3-(3-methoxy-phenyl)-urea | $C_{16}H_{17}ClN_2O_4$ | A | 336.77 | 337.1 |
| 860 | 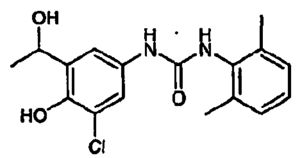 | 1-[3-Chloro-4-hydroxy-5-(1-hydroxy-ethyl)-phenyl]-3-(2,6-dimethyl-phenyl)-urea | $C_{17}H_{19}ClN_2O_3$ | A | 334.80 | 335.2 |
| 861 | 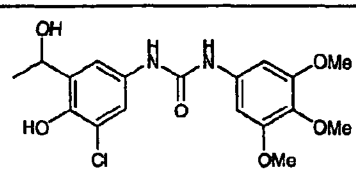 | 1-[3-Chloro-4-hydroxy-5-(1-hydroxy-ethyl)-phenyl]-3-(3,4,5-trimethoxy-phenyl)-urea | $C_{18}H_{21}ClN_2O_6$ | A | 396.82 | 397.2 |

| | | | | | | |
|---|---|---|---|---|---|---|
| 862 | 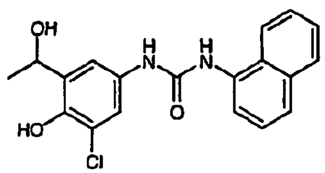 | 1-[3-Chloro-4-hydroxy-5-(1-hydroxy-ethyl)-phenyl]-3-naphthalen-1-yl-urea | C19H17ClN2O3 | A | 356.80 | 357.1 |
| 863 | 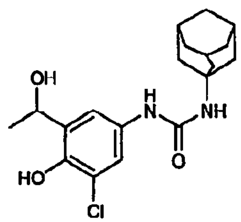 | 1-Adamantan-1-yl-3-[3-chloro-4-hydroxy-5-(1-hydroxy-ethyl)-phenyl]-urea | C19H25ClN2O3 | A | 364.87 | 365.1 |
| 864 | 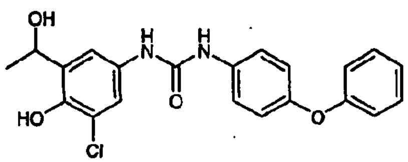 | 1-[3-Chloro-4-hydroxy-5-(1-hydroxy-ethyl)-phenyl]-3-(4-phenoxy-phenyl)-urea | C21H19ClN2O4 | A | 398.84 | 399.0 |
| 865 | 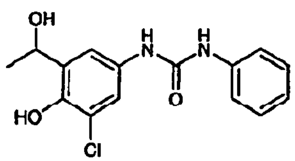 | 1-[3-Chloro-4-hydroxy-5-(1-hydroxy-ethyl)-phenyl]-3-phenyl-urea | C15H15ClN2O3 | A | 306.74 | 307.0 |
| 866 | 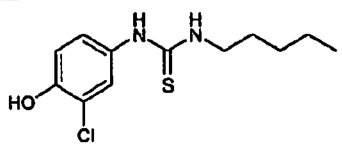 | 1-(3-Chloro-4-hydroxy-phenyl)-3-pentyl-thiourea | C12H17ClN2OS | I | 272.79 | 272.9 |

| | | | | | | |
|---|---|---|---|---|---|---|
| 867 | 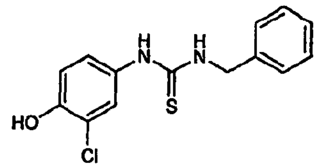 | 1-Benzyl-3-(3-chloro-4-hydroxy-phenyl)-thiourea | C14H13ClN2OS | I | 292.78 | 293.1 |
| 868 | 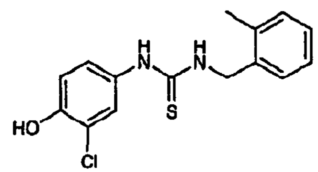 | 1-(3-Chloro-4-hydroxy-phenyl)-3-(2-methyl-benzyl)-thiourea | C15H15ClN2OS | I | 306.81 | 306.9 |
| 869 | 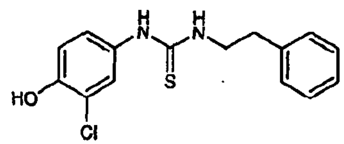 | 1-(3-Chloro-4-hydroxy-phenyl)-3-phenethyl-thiourea | C15H15ClN2OS | I | 306.81 | 307.1 |
| 870 | 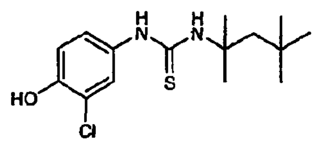 | 1-(3-Chloro-4-hydroxy-phenyl)-3-(1,1,3,3-tetramethyl-butyl)-thiourea | C15H23ClN2OS | I | 314.87 | 315.0 |
| 871 | 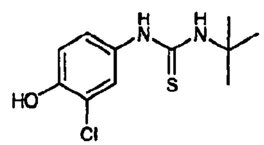 | 1-tert-Butyl-3-(3-chloro-4-hydroxy-phenyl)-thiourea | C11H15ClN2OS | I | 258.77 | 258.9 |
| 872 | 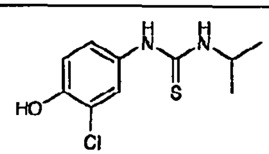 | 1-(5-Chloro-2-hydroxy-phenyl)-3-isopropyl-thiourea | C10H13ClN2OS | I | 244.74 | 245.0 |

| | | | | | | |
|---|---|---|---|---|---|---|
| 873 | 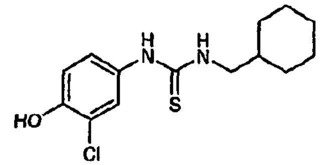 | 1-(3-Chloro-4-hydroxy-phenyl)-3-cyclohexylmethyl-thiourea | C14H19ClN2OS | I | 298.83 | 299.1 |
| 874 | 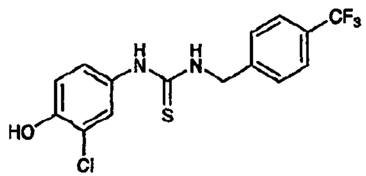 | 1-(3-Chloro-4-hydroxy-phenyl)-3-(4-trifluoromethyl-benzyl)-thiourea | C15H12ClF3N2OS | I | 360.78 | 361.1 |
| 875 | 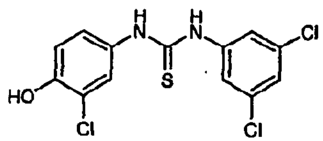 | 1-(3-Chloro-4-hydroxy-phenyl)-3-(3,5-dichloro-phenyl)-thiourea | C13H9Cl3N2OS | I | 347.65 | 347.9 |
| 876 | 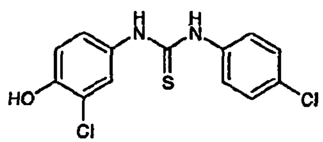 | 1-(3-Chloro-4-hydroxy-phenyl)-3-(4-chloro-phenyl)-thiourea | C13H10Cl2N2OS | I | 313.20 | 313.5 |
| 877 | 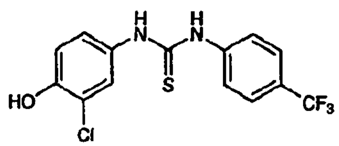 | 1-(3-Chloro-4-hydroxy-phenyl)-3-(4-trifluoromethyl-phenyl)-thiourea | C14H10ClF3N2OS | I | 346.76 | 347.0 |
| 878 | 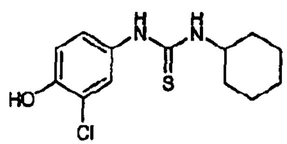 | 1-(3-Chloro-4-hydroxy-phenyl)-3-cyclohexyl-thiourea | C13H17ClN2OS | Ii | 284.80 | 285.0 |

| | | | | | | |
|---|---|---|---|---|---|---|
| 879 | 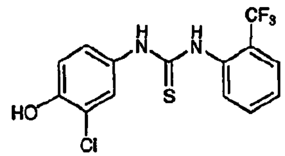 | 1-(3-Chloro-4-hydroxy-phenyl)-3-(2-trifluoromethyl-phenyl)-thiourea | C14H10ClF3N2OS | I | 346.76 | 347.0 |
| 880 | 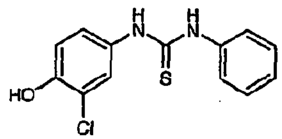 | 1-(3-Chloro-4-hydroxy-phenyl)-3-phenyl-thiourea | C13H11ClN2OS | I | 278.76 | 279.0 |
| 881 | 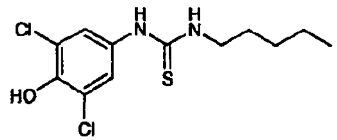 | 1-(3,5-Dichloro-4-hydroxy-phenyl)-3-pentyl-thiourea | C12H16Cl2N2OS | I | 307.24 | 307.5 |
| 882 | 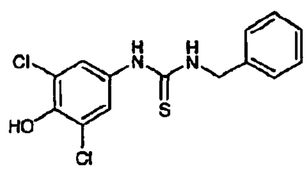 | 1-Benzyl-3-(3,5-dichloro-4-hydroxy-phenyl)-thiourea | C14H12Cl2N2OS | I | 327.23 | 327.4 |
| 883 | 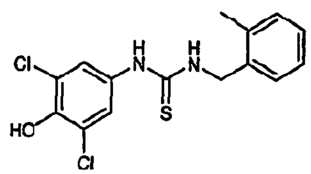 | 1-(3,5-Dichloro-4-hydroxy-phenyl)-3-(2-methyl-benzyl)-thiourea | C15H14Cl2N2OS | I | 341.26 | 341.4 |
| 884 | 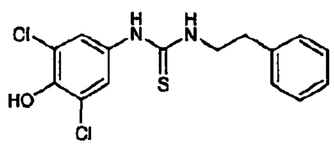 | 1-(3,5-Dichloro-4-hydroxy-phenyl)-3-phenethyl-thiourea | C15H14Cl2N2OS | I | 341.26 | 341.5 |

| | | | | | | |
|---|---|---|---|---|---|---|
| 885 | 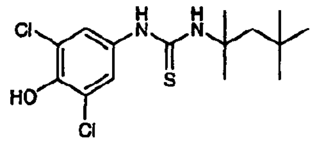 | 1-(3,5-Dichloro-4-hydroxy-phenyl)-3-(1,1,3,3-tetramethyl-butyl)-thiourea | C15H22Cl2N2OS | I | 349.32 | 349.6 |
| 886 | 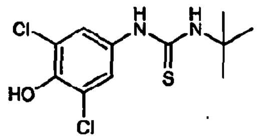 | 1-tert-Butyl-3-(3,5-dichloro-4-hydroxy-phenyl)-thiourea | C11H14Cl2N2OS | I | 293.21 | 293.6 |
| 887 | 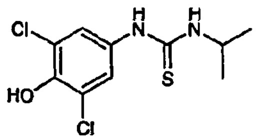 | 1-(5-Chloro-4-hydroxy-phenyl)-3-isopropyl-thiourea | C10H12Cl2N2OS | I | 279.19 | 279.5 |
| 888 | 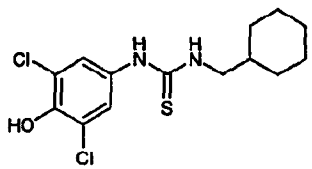 | 1-(3,5-Dichloro-4-hydroxy-phenyl)-3-cyclohexylmethyl-thiourea | C14H18Cl2N2OS | I | 333.28 | 333.5 |
| 889 | 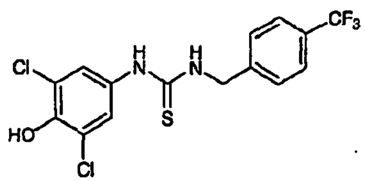 | 1-(3,5-Dichloro-4-hydroxy-phenyl)-3-(4-trifluoromethyl-benzyl)-thiourea | C15H11Cl2F3N2OS | I | 395.23 | 395.5 |
| 890 | 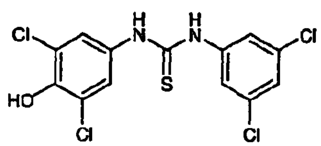 | 1-(3,5-Dichloro-4-hydroxy-phenyl)-3-(3,5-dichloro-phenyl)-thiourea | C13H8Cl4N2OS | I | 382.09 | 382.3 |

| | | | | | | |
|---|---|---|---|---|---|---|
| 891 | 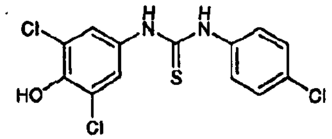 | 1-(3,5-Dichloro-4-hydroxy-phenyl)-3-(4-chloro-phenyl)-thiourea | C13H9Cl3N2OS | I | 345.65 | 345.9 |
| 892 | 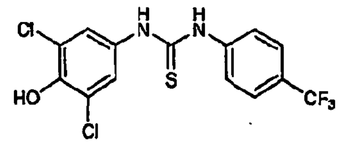 | 1-(3,5-Dichloro-4-hydroxy-phenyl)-3-(4-trifluoromethyl-phenyl)-thiourea | C14H9Cl2F3N2OS | I | 381.2 | 381.6 |
| 893 | 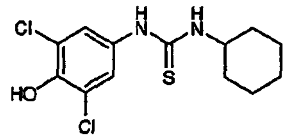 | 1-(3,5-Dichloro-4-hydroxy-phenyl)-3-cyclohexyl-thiourea | C13H16Cl2N2OS | I | 319.25 | 319.6 |
| 894 | 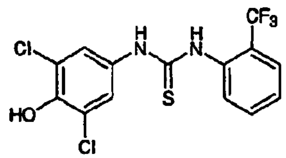 | 1-(3,5-Dichloro-4-hydroxy-phenyl)-3-(2-trifluoromethyl-phenyl)-thiourea | C14H9Cl2F3N2OS | I | 381.20 | 381.4 |
| 895 | 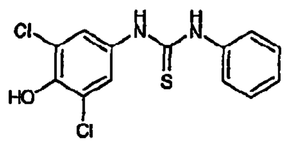 | 1-(3,5-Dichloro-4-hydroxy-phenyl)-3-phenyl-thiourea | C13H10Cl2N2OS | I | 313.20 | 313.5 |
| 896 | 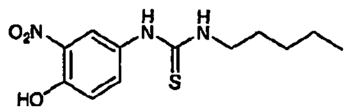 | 1-(4-Hydroxy-3-nitro-phenyl)-3-pentyl-thiourea | C12H17N3O3S | I | 283.35 | 283.9 |

| | | | | | | |
|---|---|---|---|---|---|---|
| 897 | 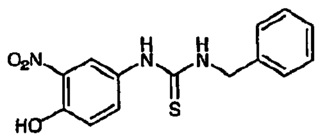 | 1-Benzyl-3-(4-hydroxy-3-nitro-phenyl)-thiourea | C14H13N3O3S | I | 303.34 | 304.0 |
| 898 | 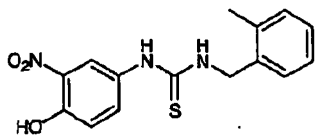 | 1-(4-Hydroxy-3-nitro-phenyl)-3-(2-methyl-benzyl)-thiourea | C15H15N3O3S | I | 317.36 | 317.9 |
| 899 | 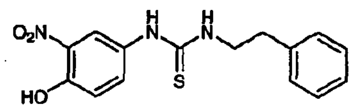 | 1-(4-Hydroxy-3-nitro-phenyl)-3-phenethyl-thiourea | C15H15N3O3S | I | 317.36 | 318.0 |

| | Structure | Chemical name | Formula | Synthesis methods | MolWeight | MS data |
|---|---|---|---|---|---|---|
| 900 | 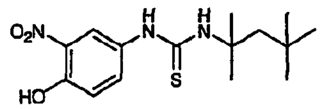 | 1-(4-Hydroxy-3-nitro-phenyl)-3-(1,1,3,3-tetramethyl-butyl)-thiourea | C15H23N3O3S | I | 325.43 | 326.1 |
| 901 | 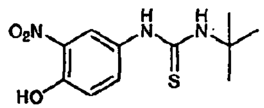 | 1-tert-Butyl-3-(4-hydroxy-3-nitro-phenyl)-thiourea | C11H15N3O3S | I | 269.32 | 239.9 |
| 902 | 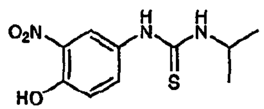 | 1-(4-Hydroxy-3-nitro-phenyl)-3-isopropyl-thiourea | C10H13N3O3S | I | 255.29 | 255.9 |
| 903 | 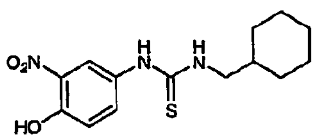 | 1-Cyclohexylmethyl-3-(4-hydroxy-3-nitro-phenyl)-thiourea | C14H19N3O3S | I | 309.38 | 309.9 |
| 904 | 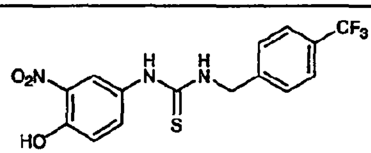 | 1-(4-Hydroxy-3-nitro-phenyl)-3-(4-trifluoromethyl-benzyl)-thiourea | C15H12F3N3O3S | I | 371.33 | 372.0 |
| 905 | 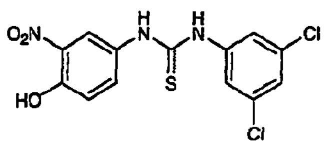 | 1-(3,5-Dichloro-phenyl)-3-(4-hydroxy-3-nitro-phenyl)-thiourea | C13H9Cl2N3O3S | I | 358.20 | 358.6 |

| | | | | | | |
|---|---|---|---|---|---|---|
| 906 | 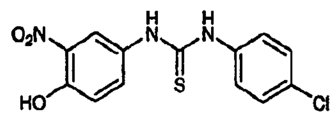 | 1-(4-Chloro-phenyl)-3-(4-hydroxy-3-nitro-phenyl)-thiourea | C13H10ClN3O3S | I | 323.75 | 324.2 |
| 907 | 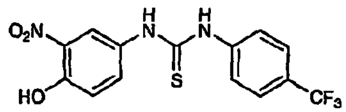 | 1-(4-Hydroxy-3-nitro-phenyl)-3-(4-trifluoromethyl-phenyl)-thiourea | C14H10F3N3O3S | I | 357.31 | 357.9 |
| 908 | 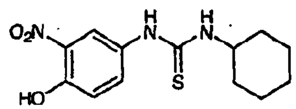 | 1-Cyclohexyl-3-(4-hydroxy-3-nitro-phenyl)-thiourea | C13H17N3O3S | I | 295.36 | 296.0 |
| 909 | 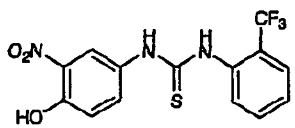 | 1-(4-Hydroxy-3-nitro-phenyl)-3-(2-trifluoromethyl-phenyl)-thiourea | C14H10F3N3O3S | I | 357.31 | 357.6 |
| 910 | 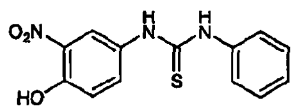 | 1-(4-Hydroxy-3-nitro-phenyl)-3-phenyl-thiourea | C13H11N3O3S | I | 289.31 | 289.5 |
| 911 | 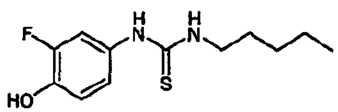 | 1-(3-Fluoro-4-hydroxy-phenyl)-3-pentyl-thiourea | C12H17FN2OS | I | 256.34 | 256.8 |
| 912 | 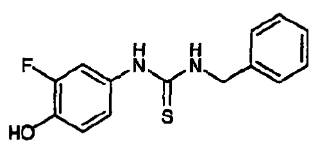 | 1-Benzyl-3-(3-fluoro-4-hydroxy-phenyl)-thiourea | C14H13FN2OS | I | 276.33 | 276.7 |

| | | | | | | |
|---|---|---|---|---|---|---|
| 913 | 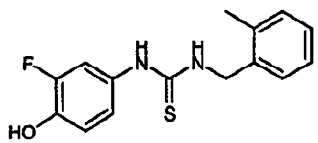 | 1-(3-Fluoro-4-hydroxy-phenyl)-3-(2-methyl-benzyl)-thiourea | C15H15FN2OS | I | 290.36 | 290.5 |
| 914 | 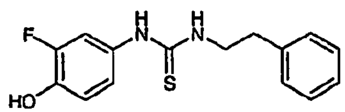 | 1-(3-Fluoro-4-hydroxy-phenyl)-3-phenethyl-thiourea | C15H15FN2OS | I | 290.36 | 290.5 |
| 915 | 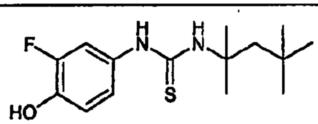 | 1-(3-Fluoro-4-hydroxy-phenyl)-3-(1,1,3,3-tetramethyl-butyl)-thiourea | C15H23FN2OS | I | 298.42 | 298.7 |
| 916 | 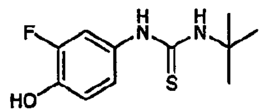 | 1-tert-Butyl-3-(3-fluoro-4-hydroxy-phenyl)-thiourea | C11H15FN2OS | I | 242.31 | 242.8 |
| 917 | 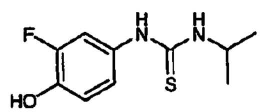 | 1-(3-Fluoro-4-hydroxy-phenyl)-3-isopropyl-thiourea | C10H13FN2OS | I | 228.29 | 228.5 |
| 918 | 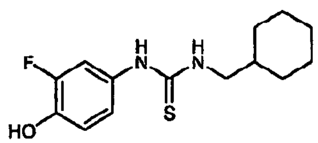 | 1-(3-Fluoro-4-hydroxy-phenyl)-3-cyclohexylmethyl-thiourea | C14H19FN2OS | I | 282.38 | 282.7 |
| 919 | 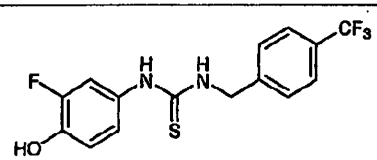 | 1-(3-Fluoro-4-hydroxy-phenyl)-3-(4-trifluoromethyl-benzyl)-thiourea | C15H12F4N2OS | I | 344.33 | 344.7 |

| | | | | | | |
|---|---|---|---|---|---|---|
| 920 | 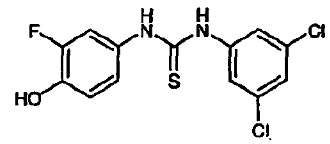 | 1-(3-Fluoro-4-hydroxy-phenyl)-3-(3,5-dichloro-phenyl)-thiourea | C13H9Cl2FN2OS | I | 331.19 | 331.5 |
| 921 | 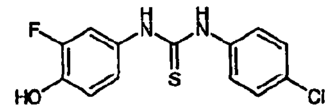 | 1-(3-Fluoro-4-hydroxy-phenyl)-3-(4-chloro-phenyl)-thiourea | C13H10ClFN2OS | I | 296.75 | 297.1 |
| 922 | 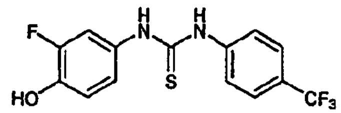 | 1-(3-Fluoro-4-hydroxy-phenyl)-3-(4-trifluoromethyl-phenyl)-thiourea | C14H10F4N2OS | I | 330.30 | 330.6 |
| 923 | 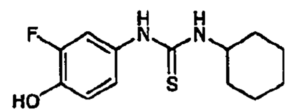 | 1-(3-Fluoro-4-hydroxy-phenyl)-3-cyclohexyl-thiourea | C13H17FN2OS | I | 268.35 | 268.6 |
| 924 | 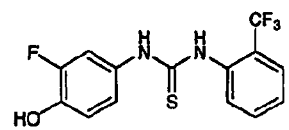 | 1-(3-Fluoro-4-hydroxy-phenyl)-3-(2-trifluoromethyl-phenyl)-thiourea | C14H10F4N2OS | I | 330.30 | 330.5 |
| 925 | 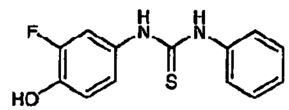 | 1-(3-Fluoro-4-hydroxy-phenyl)-3-phenyl-thiourea | C13H11FN2OS | I | 262.30 | 262.5 |
| 926 | 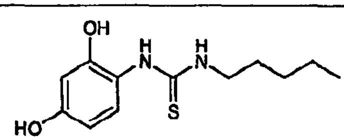 | 1-(2,4-Dihydroxy-phenyl)-3-pentyl-thiourea | C12H18N2O2S | I | 254.35 | 254.6 |

| | | | | | | |
|---|---|---|---|---|---|---|
| 927 | 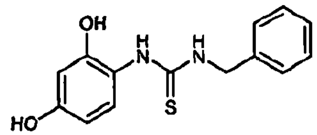 | 1-Benzyl-3-(2,4-dihydroxy-phenyl)-thiourea | C14H14N2O2S | I | 274.34 | 274.7 |
| 928 | 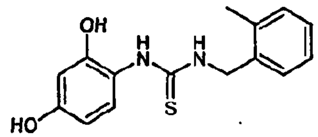 | 1-(2,4-Dihydroxy-phenyl)-3-(2-methyl-benzyl)-thiourea | C15H16N2O2S | I | 288.36 | 288.6 |
| 929 | 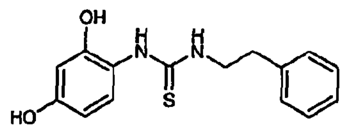 | 1-(2,4-Dihydroxy-phenyl)-3-phenethyl-thiourea | C15H16N2O2S | I | 288.36 | 288.6 |
| 930 | 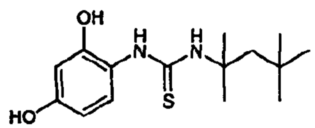 | 1-(2,4-Dihydroxy-phenyl)-3-(1,1,3,3-tetramethyl-butyl)-thiourea | C15H24N2O2S | I | 296.43 | 296.7 |
| 931 | 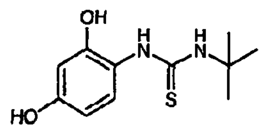 | 1-tert-Butyl-3-(2,4-dihydroxy-phenyl)-thiourea | C11H16N2O2S | I | 240.32 | 240.6 |
| 932 | 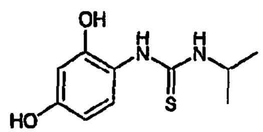 | 1-(2,4-Dihydroxy-phenyl)-3-isopropyl-thiourea | C10H14N2O2S | I | 226.30 | 226.7 |
| 933 | 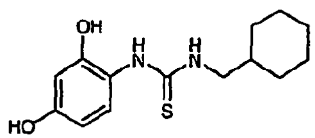 | 1-Cyclohexylmethyl-3-(2,4-dihydroxy-phenyl)-thiourea | C14H20N2O2S | I | 280.39 | 280.7 |

| | | | | | | |
|---|---|---|---|---|---|---|
| 934 | 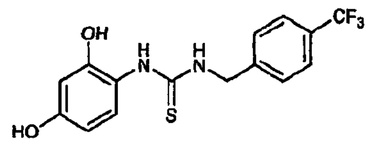 | 1-(2,4-Dihydroxy-phenyl)-3-(4-trifluoromethyl-benzyl)-thiourea | C15H13F3N2O2S | I | 342.34 | 342.6 |
| 935 | 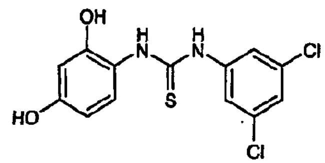 | 1-(3,5-Dichloro-phenyl)-3-(2,4-dihydroxy-phenyl)-thiourea | C13H10Cl2N2O2S | I | 329.20 | 329.5 |
| 936 | 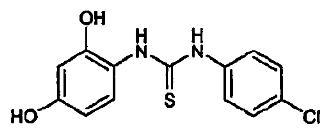 | 1-(4-Chloro-phenyl)-3-(2,4-dihydroxy-phenyl)-thiourea | C13H11ClN2O2S | I | 294.76 | 295.0 |
| 937 | 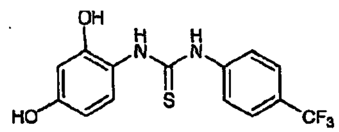 | 1-(2,4-Dihydroxy-phenyl)-3-(4-trifluoromethyl-phenyl)-thiourea | C14H11F3N2O2S | I | 328.31 | 328.7 |
| 938 | 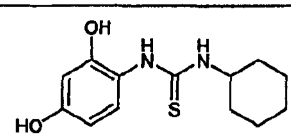 | 1-Cyclohexyl-3-(2,4-dihydroxy-phenyl)-thiourea | C13H18N2O2S | I | 266.36 | 266.7 |
| 939 | 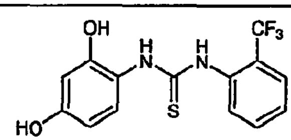 | 1-(2,4-Dihydroxy-phenyl)-3-(2-trifluoromethyl-phenyl)-thiourea | C14H11F3N2O2S | I | 328.31 | 328.6 |

| | | | | | | |
|---|---|---|---|---|---|---|
| 940 | 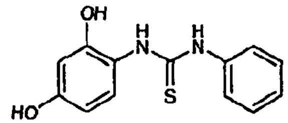 | 1-(2,4-Dihydroxy-phenyl)-3-phenyl-thiourea | C13H12N2O2S | I | 260.31 | 260.6 |
| 941 | 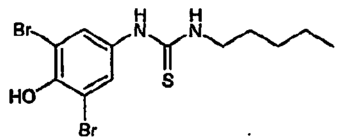 | 1-(3,5-Dibromo-4-hydroxy-phenyl)-3-pentyl-thiourea | C12H16Br2N2OS | I | 396.14 | 396.5 |
| 942 | 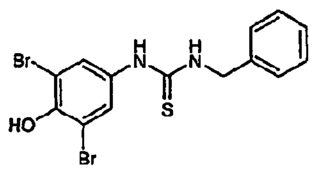 | 1-Benzyl-3-(3,5-dibromo-4-hydroxy-phenyl)-thiourea | C14H12Br2N2OS | I | 416.13 | 416.4 |
| 943 | 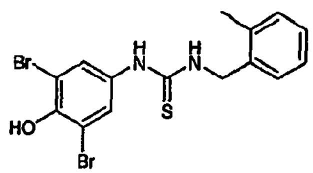 | 1-(3,5-Dibromo-4-hydroxy-phenyl)-3-(2-methyl-benzyl)-thiourea | C15H14Br2N2OS | I | 430.16 | 430.5 |
| 944 | 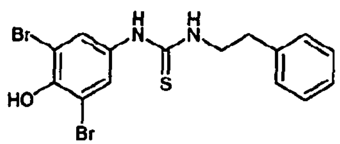 | 1-(3,5-Dibromo-4-hydroxy-phenyl)-3-phenethyl-thiourea | C15H14Br2N2OS | I | 430.16 | 430.5 |
| 945 | 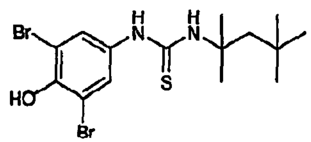 | 1-(3,5-Dibromo-4-hydroxy-phenyl)-3-(1,1,3,3-tetramethyl-butyl)-thiourea | C15H22Br2N2OS | I | 438.22 | 438.6 |

| | | | | | | |
|---|---|---|---|---|---|---|
| 946 | 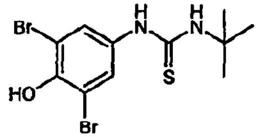 | 1-tert-Butyl-3-(3,5-dibromo-4-hydroxy-phenyl)-thiourea | C11H14Br2N2OS | I | 382.11 | 382.4 |
| 947 | 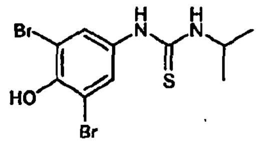 | 1-(3,5-Dibromo-4-hydroxy-phenyl)-3-isopropyl-thiourea | C10H12Br2N2OS | I | 368.09 | 368.4 |
| 948 | 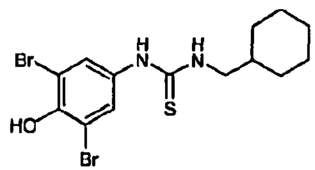 | 1-(3,5-Dibromo-4-hydroxy-phenyl)-3-cyclohexylmethyl-thiourea | C14H18Br2N2OS | I | 422.18 | 422.5 |
| 949 | 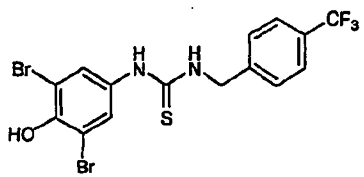 | 1-(3,5-Dibromo-4-hydroxy-phenyl)-3-(4-trifluoromethyl-benzyl)-thiourea | C15H11Br2F3N2OS | I | 484.13 | 484.5 |
| 950 | 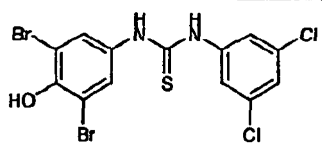 | 1-(3,5-Dibromo-4-hydroxy-phenyl)-3-(3,5-dichloro-phenyl)-thiourea | C13H8Br2Cl2N2OS | I | 470.99 | 471.4 |
| 951 | 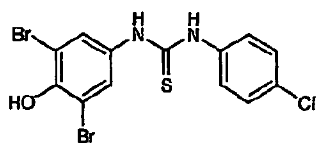 | 1-(3,5-Dibromo-4-hydroxy-phenyl)-3-(4-chloro-phenyl)-thiourea | C13H9Br2ClN2OS | I | 436.55 | 437.0 |

| | | | | | | |
|---|---|---|---|---|---|---|
| 952 | 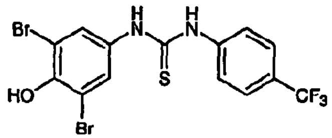 | 1-(3,5-Dibromo-4-hydroxy-phenyl)-3-(4-trifluoromethyl-phenyl)-thiourea | C14H9Br2F3N2OS | I | 470.10 | 470.6 |
| 953 | 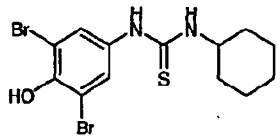 | 1-(3,5-Dibromo-4-hydroxy-phenyl)-3-cyclohexyl-thiourea | C13H16Br2N2OS | I | 408.15 | 408.5 |
| 954 | 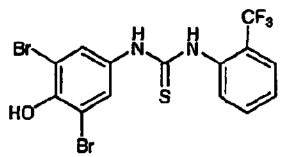 | 1-(3,5-Dibromo-4-hydroxy-phenyl)-3-(2-trifluoromethyl-phenyl)-thiourea | C14H9Br2F3N2OS | I | 470.10 | 470.5 |
| 955 | 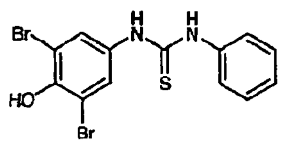 | 1-(3,5-Dibromo-4-hydroxy-phenyl)-3-phenyl-thiourea | C13H10Br2N2OS | I | 402.10 | 402.6 |
| 956 | 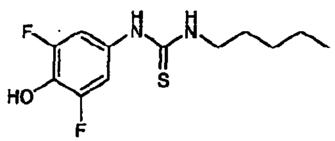 | 1-(3,5-Difluoro-4-hydroxy-phenyl)-3-pentyl-thiourea | C12H16F2N2OS | I | 274.33 | 274.8 |
| 957 | 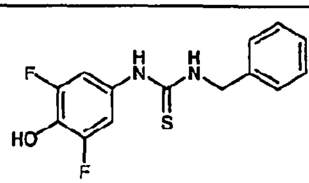 | 1-Benzyl-3-(3,5-difluoro-4-hydroxy-phenyl)-thiourea | C14H12F2N2OS | I | 294.32 | 294.7 |

| | | | | | | |
|---|---|---|---|---|---|---|
| 958 | 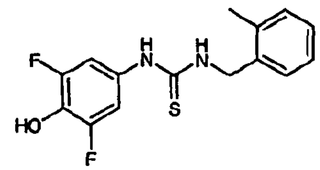 | 1-(3,5-Difluoro-4-hydroxy-phenyl)-3-(2-methyl-benzyl)-thiourea | C15H14F2N2OS | I | 308.35 | 308.6 |
| 959 | 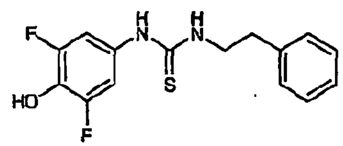 | 1-(3,5-Difluoro-4-hydroxy-phenyl)-3-phenethyl-thiourea | C15H14F2N2OS | I | 308.35 | 308.6 |
| 960 | 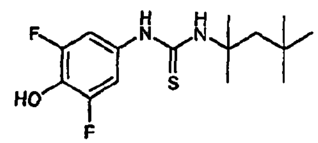 | 1-(3,5-Difluoro-4-hydroxy-phenyl)-3-(1,1,3,3-tetramethyl-butyl)-thiourea | C15H22F2N2OS | I | 316.41 | 316.8 |
| 961 | 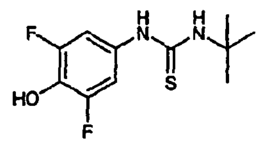 | 1-tert-Butyl-3-(3,5-Difluoro-4-hydroxy-phenyl)-thiourea | C11H14F2N2OS | I | 260.30 | 260.5 |
| 962 | 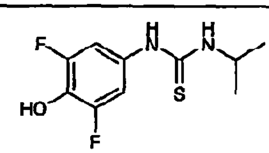 | 1-(3,5-Difluoro-4-hydroxy-phenyl)-3-isopropyl-thiourea | C10H12F2N2OS | I | 246.28 | 246.5 |
| 963 | 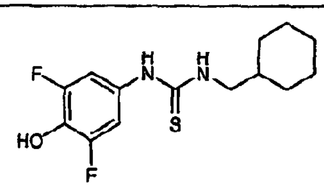 | 1-(3,5-Difluoro-4-hydroxy-phenyl)-3-cyclohexylmethyl-thiourea | C14H18F2N2OS | I | 300.37 | 300.6 |

| | | | | | | |
|---|---|---|---|---|---|---|
| 964 | 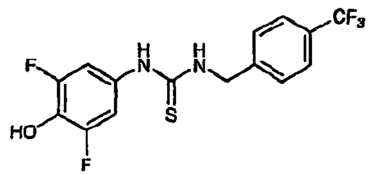 | 1-(3,5-Difluoro-4-hydroxy-phenyl)-3-(4-trifluoromethyl-benzyl)-thiourea | C15H11F5N2OS | I | 362.32 | 362.8 |
| 965 | 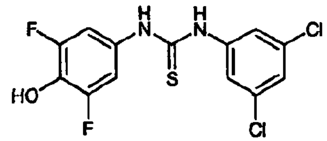 | 1-(3,5-Difluoro-4-hydroxy-phenyl)-3-(3,5-dichloro-phenyl)-thiourea | C13H8Cl2F2N2OS | I | 349.18 | 349.7 |
| 966 | 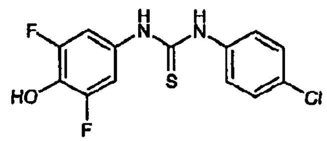 | 1-(3,5-Difluoro-4-hydroxy-phenyl)-3-(4-chloro-phenyl)-thiourea | C13H9ClF2N2OS | I | 314.74 | 315.2 |
| 967 | 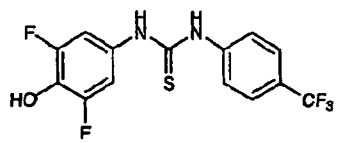 | 1-(3,5-Difluoro-4-hydroxy-phenyl)-3-(4-trifluoromethyl-phenyl)-thiourea | C14H9F5N2OS | I | 348.29 | 348.8 |
| 968 | 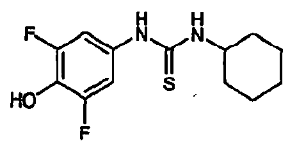 | 1-(3,5-Difluoro-4-hydroxy-phenyl)-3-cyclohexyl-thiourea | C13H16F2N2OS | I | 286.34 | 286.6 |
| 969 | 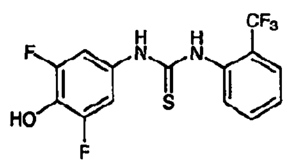 | 1-(3,5-Difluoro-4-hydroxy-phenyl)-3-(2-trifluoromethyl-phenyl)-thiourea | C14H9F5N2OS | I | 348.29 | 348.8 |

| | | | | | | |
|---|---|---|---|---|---|---|
| 970 | 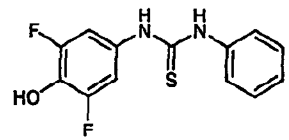 | 1-(3,5-Difluoro-4-hydroxy-phenyl)-3-phenyl-thiourea | C13H10F2N2OS | I | 280.29 | 280.5 |
| 971 | 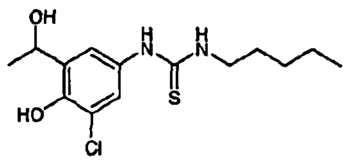 | 1-[3-Chloro-4-hydroxy-5-(1-hydroxy-ethyl)-phenyl]-3-pentyl-thiourea | C14H21ClN2O2S | I | 316.85 | 316.9 |
| 972 | 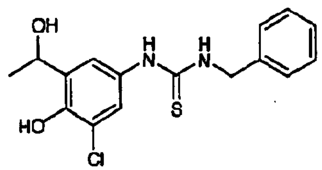 | 1-Benzyl-3-[3-chloro-4-hydroxy-5-(1-hydroxy-ethyl)-phenyl]-thiourea | C16H17ClN2O2S | I | 336.84 | 337.2 |
| 973 | 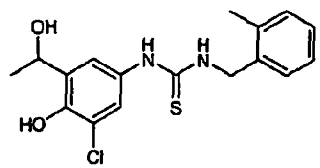 | 1-[3-Chloro-4-hydroxy-5-(1-hydroxy-ethyl)-phenyl]-3-(2-methyl-benzyl)-thiourea | C17H19ClN2O2S | I | 350.86 | 351.1 |
| 974 | 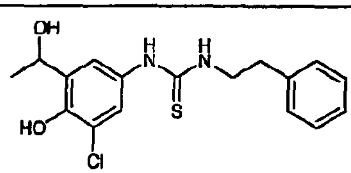 | 1-[3-Chloro-4-hydroxy-5-(1-hydroxy-ethyl)-phenyl]-3-phenethyl-thiourea | C17H19ClN2O2S | I | 350.86 | 351.0 |

| | | | | | | |
|---|---|---|---|---|---|---|
| 975 | 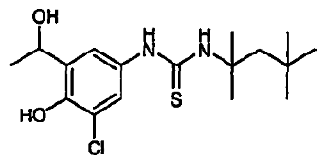 | 1-[3-Chloro-4-hydroxy-5-(1-hydroxy-ethyl)-phenyl]-3-(1,1,3,3-tetramethyl-butyl)-thiourea | C17H27ClN2O2S | I | 358.93 | 359.2 |
| 976 | 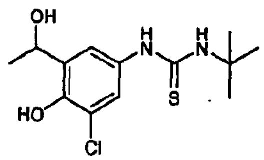 | 1-tert-Butyl-3-[3-chloro-4-hydroxy-5-(1-hydroxy-ethyl)-phenyl]-thiourea | C13H19ClN2O2S | I | 302.82 | 303.0 |
| 977 | 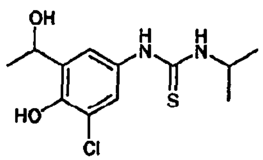 | 1-[3-Chloro-4-hydroxy-5-(1-hydroxy-ethyl)-phenyl]-3-isopropyl-thiourea | C12H17ClN2O2S | I | 288.79 | 289.0 |
| 978 | 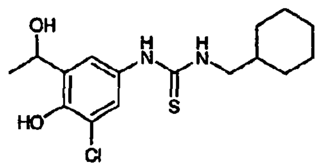 | 1-[3-Chloro-4-hydroxy-5-(1-hydroxy-ethyl)-phenyl]-3-cyclohexylmethyl-thiourea | C16H23ClN2O2S | I | 342.88 | 343.1 |
| 979 | 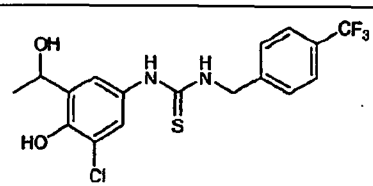 | 1-[3-Chloro-4-hydroxy-5-(1-hydroxy-ethyl)-phenyl]-3-(4-trifluoromethyl-benzyl)-thiourea | C17H16ClF3N2O2S | I | 404.83 | 405.2 |

| | | | | | | |
|---|---|---|---|---|---|---|
| 980 | 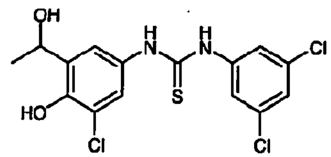 | 1-[3-Chloro-4-hydroxy-5-(1-hydroxy-ethyl)-phenyl]-3-(3,5-dichloro-phenyl)-thiourea | C15H13Cl3N2O2S | I | 391.70 | 392.0 |
| 981 | 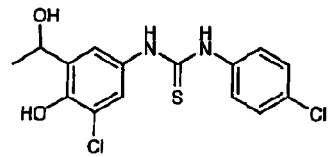 | 1-[3-Chloro-4-hydroxy-5-(1-hydroxy-ethyl)-phenyl]-3-(4-chloro-phenyl)-thiourea | C15H14Cl2N2O2S | I | 357.25 | 357.6 |
| 982 | 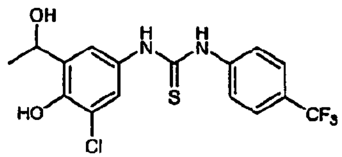 | 1-[3-Chloro-4-hydroxy-5-(1-hydroxy-ethyl)-phenyl]-3-(4-trifluoromethyl-phenyl)-thiourea | C16H14ClF3N2O2S | I | 390.81 | 391.0 |
| 983 | 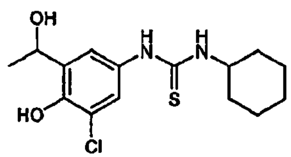 | 1-[3-Chloro-4-hydroxy-5-(1-hydroxy-ethyl)-phenyl]-3-cyclohexyl-thiourea | C15H21ClN2O2S | I | 328.86 | 329.4 |
| 984 | 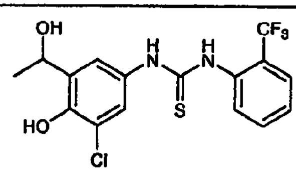 | 1-[3-Chloro-4-hydroxy-5-(1-hydroxy-ethyl)-phenyl]-3-(2-trifluoromethyl-phenyl)-thiourea | C16H14ClF3N2O2S | I | 390.81 | 391.0 |

| | | | | | | |
|---|---|---|---|---|---|---|
| 985 | 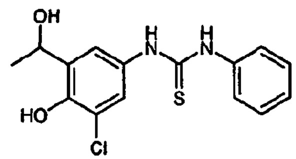 | 1-[3-Chloro-4-hydroxy-5-(1-hydroxy-ethyl)-phenyl]-3-phenyl-thiourea | C15H15ClN2O2S | I | 322.81 | 323.1 |
| 986 | 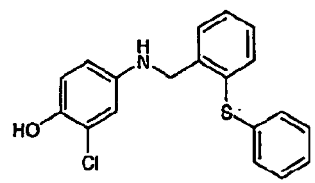 | 2-Chloro-4-(2-phenylsulfanyl-benzylamino)-phenol | C19H16ClNOS | D | 341.85 | 342.2 |
| 987 | 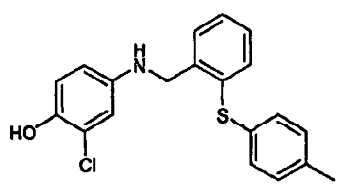 | 2-Chloro-4-(2-p-tolylsulfanyl-benzylamino)-phenol | C20H18ClNOS | D | 355.88 | 356.3 |
| 988 | 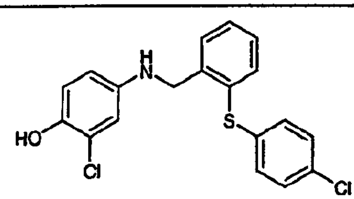 | 2-Chloro-4-[2-(4-chloro-phenylsulfanyl)-benzylamino]-phenol | C19H15Cl2NOS | D | 376.30 | 376.8 |
| 989 | 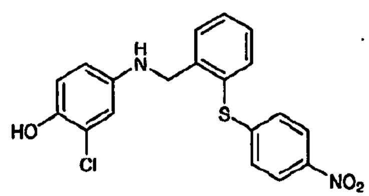 | 2-Chloro-4-[2-(4-nitro-phenylsulfanyl)-benzylamino]-phenol | C19H15ClN2O3S | D | 386.85 | 387.2 |

| | | | | | | |
|---|---|---|---|---|---|---|
| 990 | 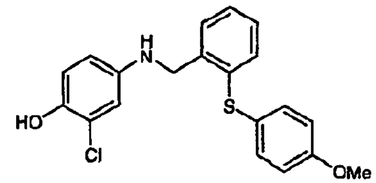 | 2-Chloro-4-[2-(4-methoxy-phenylsulfanyl)-benzylamino]-phenol | C20H18ClNO2S | D | 371.88 | 372.4 |
| 991 | 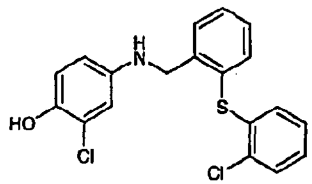 | 2-Chloro-4-[2-(2-chloro-phenylsulfanyl)-benzylamino]-phenol | C19H15Cl2NOS | D | 376.30 | 376.8 |
| 992 | 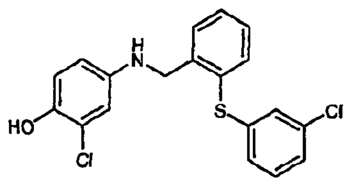 | 2-Chloro-4-[2-(3-chloro-phenylsulfanyl)-benzylamino]-phenol | C19H15Cl2NOS | D | 376.30 | 376.8 |
| 993 | 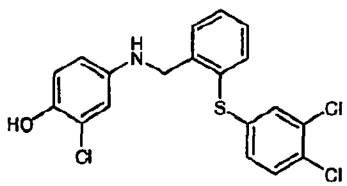 | 2-Chloro-4-[2-(3,4-dichloro-phenylsulfanyl)-benzylamino]-phenol | C19H14Cl3NOS | D | 410.74 | 411.2 |
| 994 | 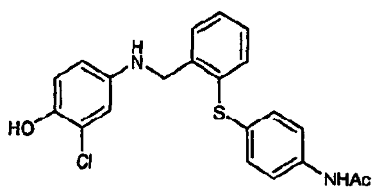 | N-(4-{2-[(3-Chloro-4-hydroxy-phenylamino)-methyl]-phenylsulfanyl}-phenyl)-acetamide | C21H19ClN2O2S | D | 398.91 | 399.3 |

| | | | | | | |
|---|---|---|---|---|---|---|
| 995 | 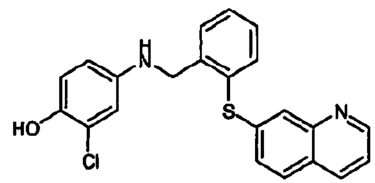 | 2-Chloro-4-[2-(quinolin-7-ylsulfanyl)-benzylamino]-phenol | C22H17ClN2OS | D | 392.90 | 393.4 |
| 996 | 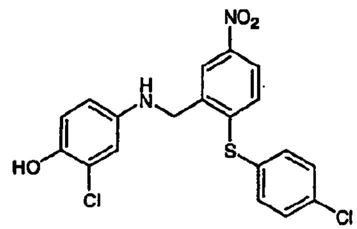 | 2-Chloro-4-[2-(4-chloro-phenylsulfanyl)-5-nitro-benzylamino]-phenol | C19H14Cl2N2O3S | D | 421.30 | 421.7 |
| 997 | 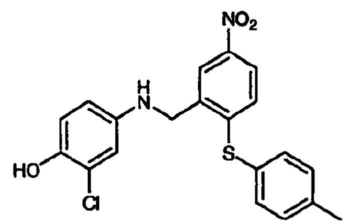 | 2-Chloro-4-(5-nitro-2-p-tolylsulfanyl-benzylamino)-phenol | C20H17ClN2O3S | D | 400.88 | 401.2 |
| 998 | 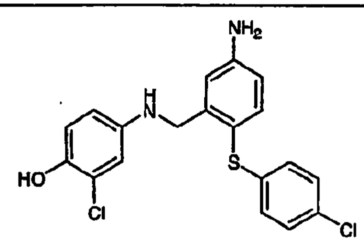 | 4-[5-Amino-2-(4-chloro-phenylsulfanyl)-benzylamino]-2-chloro-phenol | C19H16Cl2N2OS | D | 391.31 | 391.9 |

| | | | | | | |
|---|---|---|---|---|---|---|
| 999 | 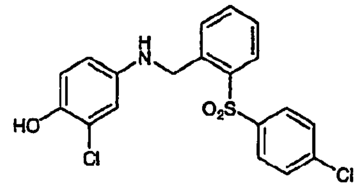 | 2-Chloro-4-[2-(4-chloro-benzenesulfonyl)-benzylamino]-phenol | C19H15Cl2NO3S | D | 408.30 | 408.7 |
| 1000 | 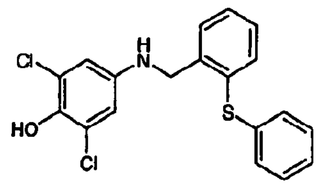 | 2,6-Dichloro-4-(2-phenylsulfanyl-benzylamino)-phenol | C19H15Cl2NOS | D | 376.30 | 376.7 |
| 1001 | 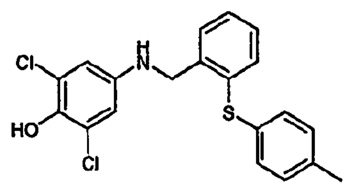 | 2,6-Dichloro-4-(2-p-tolylsulfanyl-benzylamino)-phenol | C20H17Cl2NOS | D | 390.33 | 390.7 |
| 1002 | 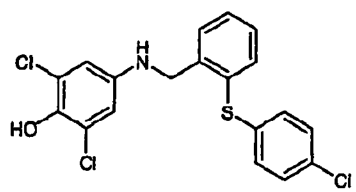 | 2,6-Dichloro-4-[2-(4-chloro-phenylsulfanyl)-benzylamino]-phenol | C19H14Cl3NOS | D | 410.75 | 411.1 |
| 1003 | 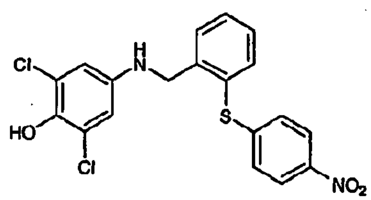 | 2,6-Dichloro-4-[2-(4-nitro-phenylsulfanyl)-benzylamino]-phenol | C19H14Cl2N2O3S | D | 421.30 | 421.8 |

| | | | | | | |
|---|---|---|---|---|---|---|
| 1004 | 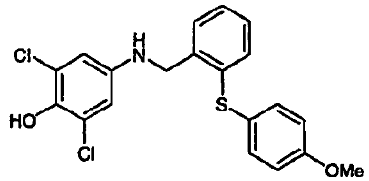 | 2,6-Dichloro-4-[2-(4-methoxy-phenylsulfanyl)-benzylamino]-phenol | C20H17Cl2NO2S | D | 406.33 | 406.7 |
| 1005 | 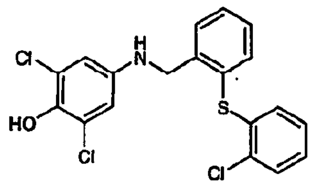 | 2,6-Dichloro-4-[2-(2-chloro-phenylsulfanyl)-benzylamino]-phenol | C19H14Cl3NOS | D | 410.75 | 411.2 |
| 1006 | 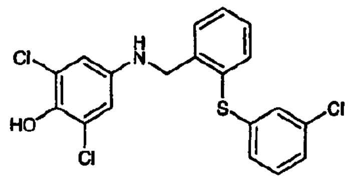 | 2,6-Dichloro-4-[2-(3-chloro-phenylsulfanyl)-benzylamino]-phenol | C19H14Cl3NOS | D | 410.75 | 411.2 |
| 1007 | 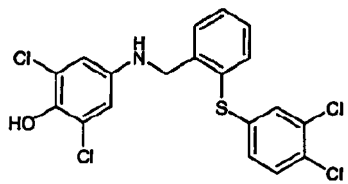 | 2,6-Dichloro-4-[2-(3,4-dichloro-phenylsulfanyl)-benzylamino]-phenol | C19H13Cl4NOS | D | 445.19 | 445.6 |
| 1008 | 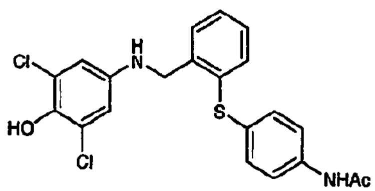 | N-(4-{2-[(3,5-Dichloro-4-hydroxy-phenylamino)-methyl]-phenylsulfanyl}-phenyl)-acetamide | C21H18Cl2N2O2S | D | 433.36 | 433.8 |

| | | | | | | |
|---|---|---|---|---|---|---|
| 1009 | 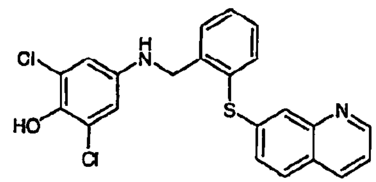 | 2,6-Dichloro-4-[2-(quinolin-7-ylsulfanyl)-benzylamino]-phenol | C22H16Cl2N2OS | D | 427.35 | 427.8 |
| 1010 | 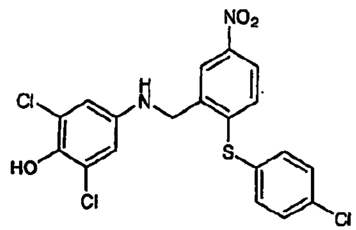 | 2,6-Dichloro-4-[2-(4-chloro-phenylsulfanyl)-5-nitro-benzylamino]-phenol | C19H13Cl3N2O3S | D | 455.74 | 456.0 |
| 1011 | 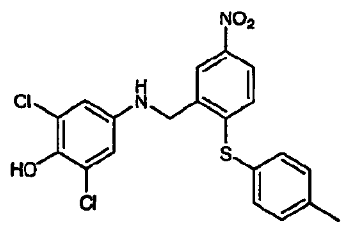 | 2,6-Dichloro-4-(5-nitro-2-p-tolylsulfanyl-benzylamino)-phenol | C20H16Cl2N2O3S | D | 435.33 | 435.7 |
| 1012 | 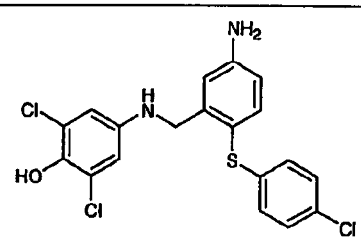 | 4-[5-Amino-2-(4-chloro-phenylsulfanyl)-benzylamino]-2,6-dichloro-phenol | C19H15Cl3N2OS | D | 425.76 | 426.0 |

| | | | | | | |
|---|---|---|---|---|---|---|
| 1013 | 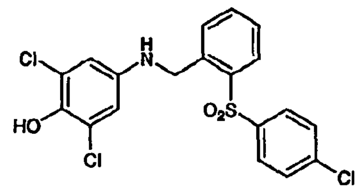 | 2,6-Dichloro-4-[2-(4-chloro-benzenesulfonyl)-benzylamino]-phenol | C19H14Cl3NO3S | D | 442.75 | 443.1 |
| 1014 | 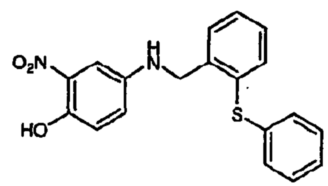 | 2-Nitro-4-(2-phenylsulfanyl-benzylamino)-phenol | C19H16N2O3S | D | 352.41 | 352.8 |
| 1015 | 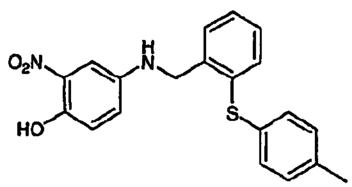 | 2-Nitro-4-(2-p-tolylsulfanyl-benzylamino)-phenol | C20H18N2O3S | D | 366.43 | 366.8 |
| 1016 | 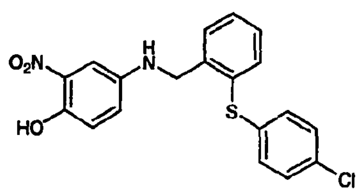 | 4-[2-(4-Chloro-phenylsulfanyl)-benzylamino]-2-nitro-phenol | C19H15ClN2O3S | D | 386.85 | 387.2 |
| 1017 | 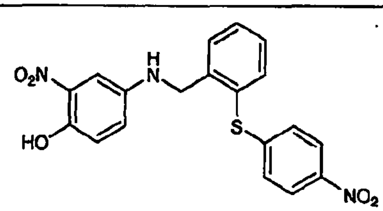 | 2-Nitro-4-[2-(4-nitro-phenylsulfanyl)-benzylamino]-phenol | C19H15N3O5S | D | 397.40 | 397.7 |

| | | | | | | |
|---|---|---|---|---|---|---|
| 1018 | 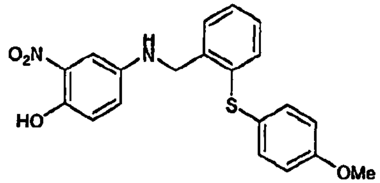 | 4-[2-(4-Methoxy-phenylsulfanyl)-benzylamino]-2-nitro-phenol | C20H18N2O4S | D | 382.43 | 382.8 |
| 1019 | 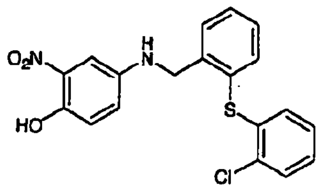 | 4-[2-(2-Chloro-phenylsulfanyl)-benzylamino]-2-nitro-phenol | C19H15ClN2O3S | D | 386.85 | 387.2 |
| 1020 | 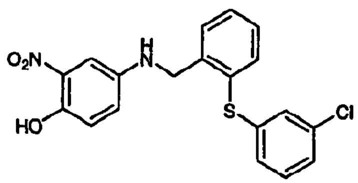 | 4-[2-(3-Chloro-phenylsulfanyl)-benzylamino]-2-nitro-phenol | C19H15ClN2O3S | D | 386.85 | 387.1 |
| 1021 | 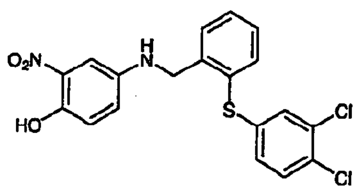 | 4-[2-(3,4-Dichloro-phenylsulfanyl)-benzylamino]-2-nitro-phenol | C19H14Cl2N2O3S | D | 421.30 | 421.7 |
| 1022 | 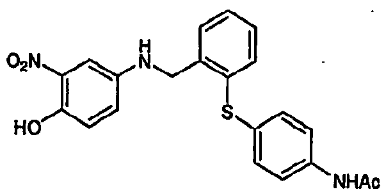 | N-(4-{2-[(4-Hydroxy-3-nitro-phenylamino)-methyl]-phenylsulfanyl}-phenyl)-acetamide | C21H19N3O4S | D | 409.46 | 409.9 |

| | | | | | | |
|---|---|---|---|---|---|---|
| 1023 | 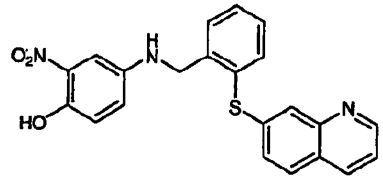 | 2-Nitro-4-[2-(quinolin-7-ylsulfanyl)-benzylamino]-phenol | C22H17N3O3S | D | 403.45 | 403.8 |
| 1024 | 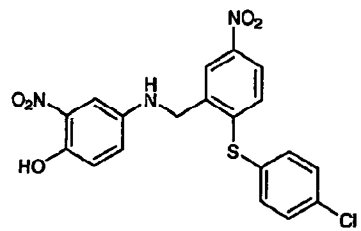 | 4-[2-(4-Chloro-phenylsulfanyl)-5-nitro-benzylamino]-2-nitro-phenol | C19H14ClN3O5S | D | 431.85 | 432.2 |
| 1025 | 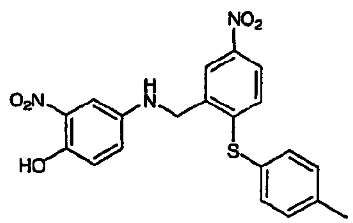 | 2-Nitro-4-(5-nitro-2-p-tolylsulfanyl-benzylamino)-phenol | C20H17N3O5S | D | 411.43 | 411.9 |
| 1026 | 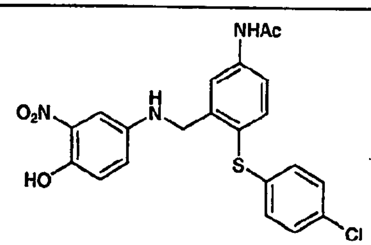 | N-{4-(4-Chloro-phenylsulfanyl)-3-[(4-hydroxy-3-nitro-phenylamino)-methyl]-phenyl}-acetamide | C21H18ClN3O4S | D | 443.90 | 444.3 |

| | | | | | | |
|---|---|---|---|---|---|---|
| 1027 | 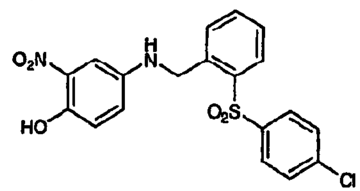 | 4-[2-(4-Chloro-benzenesulfonyl)-benzylamino]-2-nitro-phenol | C19H15ClN2O5S | D | 418.85 | 419.2 |
| 1028 | 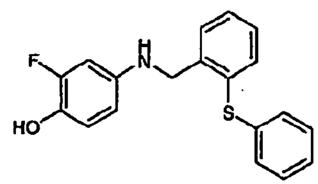 | 2-Fluoro-4-(2-phenylsulfanyl-benzylamino)-phenol | C19H16FNOS | D | 325.40 | 324.6 |
| 1029 | 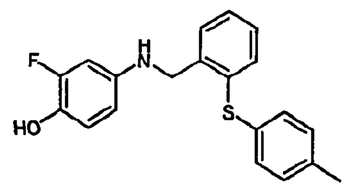 | 2-Fluoro-4-(2-p-tolylsulfanyl-benzylamino)-phenol | C20H18FNOS | D | 339.43 | 340.8 |
| 1030 | 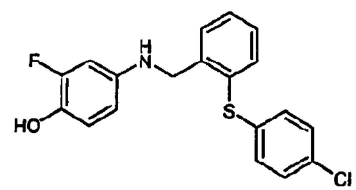 | 2-Fluoro-4-[2-(4-chloro-phenylsulfanyl)-benzylamino]-phenol | C19H15ClFNOS | D | 359.84 | 360.1 |
| 1031 | 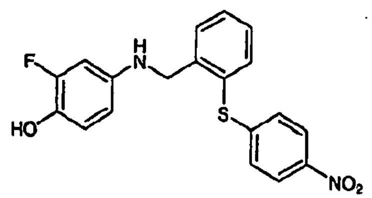 | 2-Fluoro-4-[2-(4-nitro-phenylsulfanyl)-benzylamino]-phenol | C19H15FN2O3S | D | 370.40 | 370.8 |

| | | | | | | |
|---|---|---|---|---|---|---|
| 1032 | 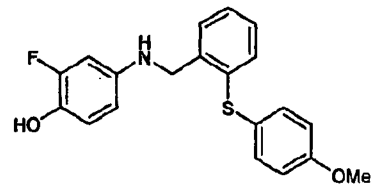 | 2-Fluoro-4-[2-(4-methoxy-phenylsulfanyl)-benzylamino]-phenol | C20H18FNO2S | D | 355.43 | 355.8 |
| 1033 | 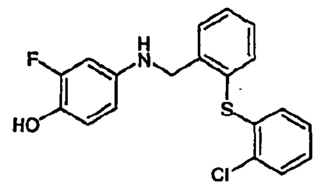 | 2-Fluoro-4-[2-(2-chloro-phenylsulfanyl)-benzylamino]-phenol | C19H15ClFNOS | D | 359.84 | 360.2 |
| 1034 | 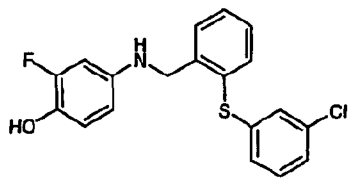 | 2-Fluoro-4-[2-(3-chloro-phenylsulfanyl)-benzylamino]-phenol | C19H15ClFNOS | D | 359.84 | 360.3 |
| 1035 | 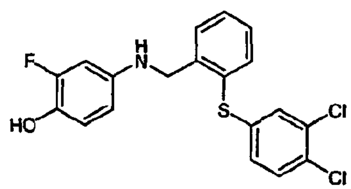 | 2-Fluoro-4-[2-(3,4-dichloro-phenylsulfanyl)-benzylamino]-phenol | C19H14Cl2FNOS | D | 394.29 | 394.6 |
| 1036 | 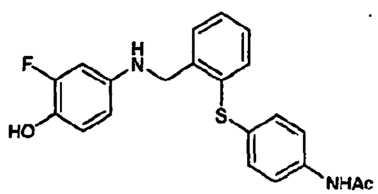 | N-(4-{2-[(3-Fluoro-4-hydroxy-phenylamino)-methyl]-phenylsulfanyl}-phenyl)-acetamide | C21H19FN2O2S | D | 382.45 | 382.9 |

| | | | | | | |
|---|---|---|---|---|---|---|
| 1037 | 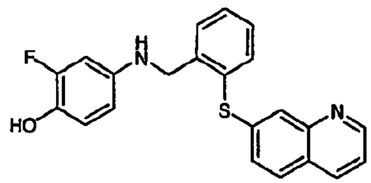 | 2-Fluoro-4-[2-(quinolin-7-ylsulfanyl)-benzylamino]-phenol | C22H17FN2OS | D | 376.45 | 377.0 |
| 1038 | 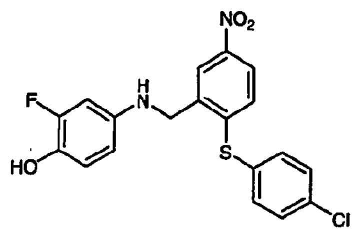 | 2-Fluoro-4-[2-(4-chloro-phenylsulfanyl)-5-nitro-benzylamino]-phenol | C19H14ClFN2O3S | D | 404.84 | 405.3 |
| 1039 | 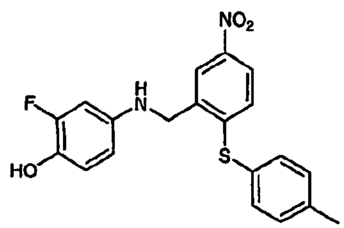 | 2-Fluoro-4-(5-nitro-2-p-tolylsulfanyl-benzylamino)-phenol | C20H17FN2O3S | D | 384.42 | 384.9 |
| 1040 | 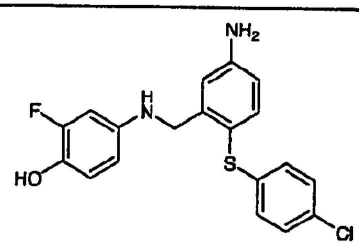 | 4-[5-Amino-2-(4-chloro-phenylsulfanyl)-benzylamino]-2-fluoro-phenol | C19H16ClFN2OS | D | 374.86 | 375.1 |

| | | | | | | |
|---|---|---|---|---|---|---|
| 1041 | 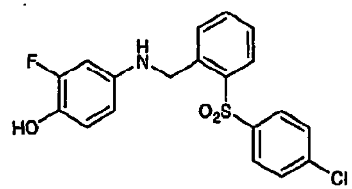 | 2-Fluoro-4-[2-(4-chloro-benzenesulfonyl)-benzylamino]-phenol | C19H15ClFNO3S | D | 391.84 | 392.0 |
| 1042 | 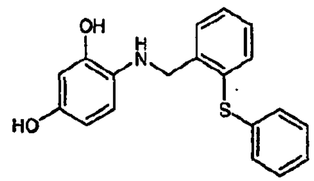 | 4-(2-Phenylsulfanyl-benzylamino)-benzene-1,3-diol | C19H17NO2S | D | 323.41 | 323.8 |
| 1043 | 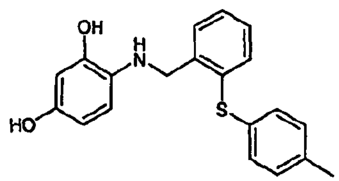 | 4-(2-p-Tolylsulfanyl-benzylamino)-benzene-1,3-diol | C20H19NO2S | D | 337.44 | 337.8 |
| 1044 | 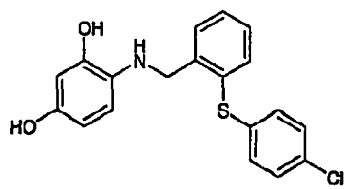 | 4-[2-(4-Chloro-phenylsulfanyl)-benzylamino]-benzene-1,3-diol | C19H16ClNO2S | D | 357.85 | 358.2 |
| 1045 | 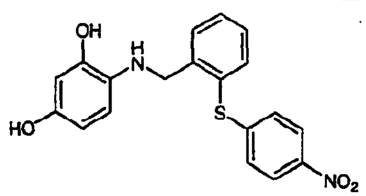 | 4-[2-(4-Nitro-phenylsulfanyl)-benzylamino]-benzene-1,3-diol | C19H16N2O4S | D | 368.41 | 368.7 |

| | | | | | | |
|---|---|---|---|---|---|---|
| 1046 | 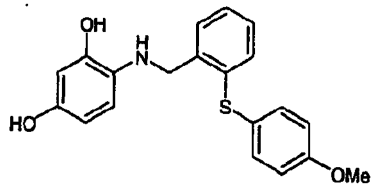 | 4-[2-(4-Methoxy-phenylsulfanyl)-benzylamino]-benzene-1,3-diol | C20H19NO3S | D | 353.43 | 353.7 |
| 1047 | 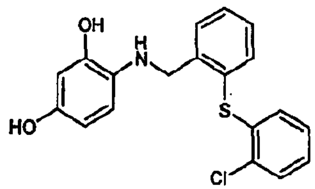 | 4-[2-(2-Chloro-phenylsulfanyl)-benzylamino]-benzene-1,3-diol | C19H16ClNO2S | D | 357.85 | 358.3 |
| 1048 | 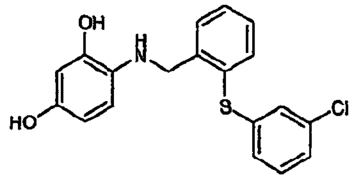 | 4-[2-(3-Chloro-phenylsulfanyl)-benzylamino]-benzene-1,3-diol | C19H16ClNO2S | D | 357.86 | 358.1 |
| 1049 | 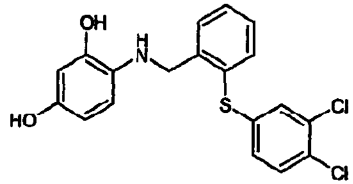 | 4-[2-(3,4-Dichloro-phenylsulfanyl)-benzylamino]-benzene-1,3-diol | C19H15Cl2NO2S | D | 392.30 | 392.7 |
| 1050 | 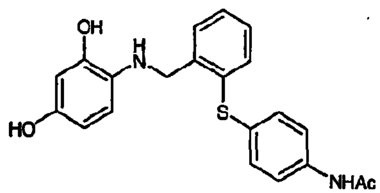 | N-(4-{2-[(2,4-Dihydroxy-phenylamino)-methyl]-phenylsulfanyl}-phenyl)-acetamide | C21H20N2O3S | D | 380.46 | 380.8 |

| | | | | | | |
|---|---|---|---|---|---|---|
| 1051 | 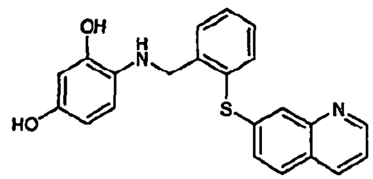 | 4-[2-(Quinolin-7-ylsulfanyl)-benzylamino]-benzene-1,3-diol | C22H18N2O2S | D | 374.46 | 374.9 |
| 1052 | 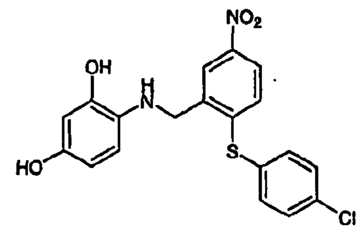 | 4-[2-(4-Chloro-phenylsulfanyl)-5-nitro-benzylamino]-benzene-1,3-diol | C19H15ClN2O4S | D | 402.85 | 403.2 |
| 1053 | 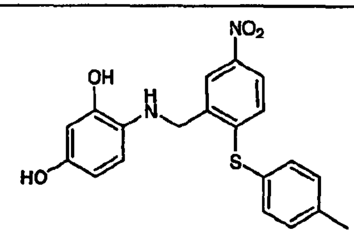 | 4-(5-Nitro-2-p-tolylsulfanyl-benzylamino)-benzene-1,3-diol | C20H18N2O4S | D | 382.43 | 382.7 |
| 1054 | 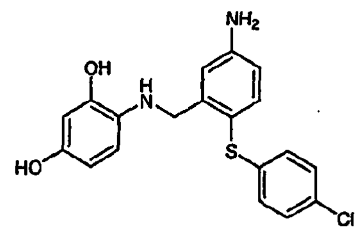 | 4-[5-Amino-2-(4-chloro-phenylsulfanyl)-benzylamino]-benzene-1,3-diol | C19H17ClN2O2S | D | 372.87 | 373.0 |

| | | | | | | |
|---|---|---|---|---|---|---|
| 1055 | 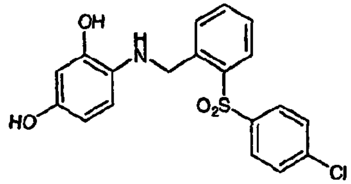 | 4-[2-(4-Chloro-benzenesulfonyl)-benzylamino]-benzene-1,3-diol | C19H16ClNO4S | D | 389.85 | 390.2 |
| 1056 | 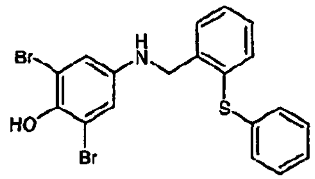 | 2,6-Dibromo-4-(2-phenylsulfanyl-benzylamino)-phenol | C19H15Br2NOS | D | 465.20 | 465.7 |
| 1057 | 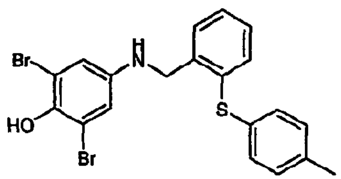 | 2,6-Dibromo-4-(2-p-tolylsulfanyl-benzylamino)-phenol | C20H17Br2NOS | D | 479.23 | 479.3 |
| 1058 | 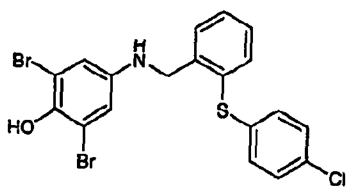 | 2,6-Dibromo-4-[2-(4-chloro-phenylsulfanyl)-benzylamino]-phenol | C19H14Br2ClNOS | D | 499.65 | 500.1 |
| 1059 | 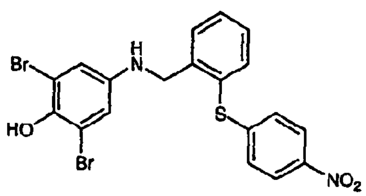 | 2,6-Dibromo-4-[2-(4-nitro-phenylsulfanyl)-benzylamino]-phenol | C19H14Br2N2O3S | D | 510.2 | 510.1 |

| | | | | | | |
|---|---|---|---|---|---|---|
| 1360 | 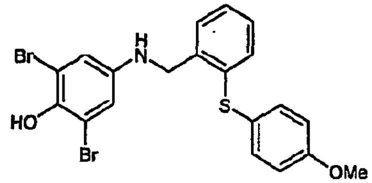 | 2,6-Dibromo-4-[2-(4-methoxy-phenylsulfanyl)-benzylamino]-phenol | C20H17Br2NO2S | D | 495.23 | 495.3 |
| 1061 | 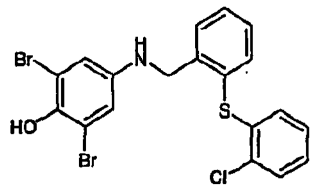 | 2,6-Dibromo-4-[2-(2-chloro-phenylsulfanyl)-benzylamino]-phenol | C19H14Br2ClNOS | D | 499.65 | 499.8 |
| 1062 | 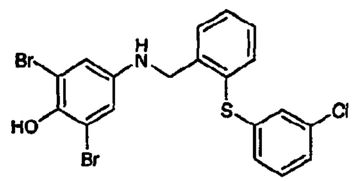 | 2,6-Dibromo-4-[2-(3-chloro-phenylsulfanyl)-benzylamino]-phenol | C19H14Br2ClNOS | D | 499.65 | 499.8 |
| 1063 | 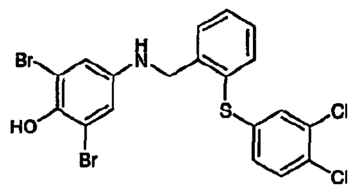 | 2,6-Dibromo-4-[2-(3,4-dichloro-phenylsulfanyl)-benzylamino]-phenol | C19H13Br2Cl2NOS | D | 534.09 | 534.2 |
| 1064 | 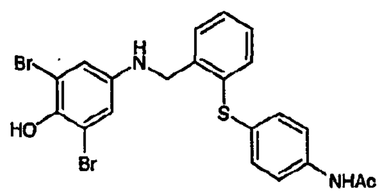 | N-(4-{2-[(3,5-Dibromo-4-hydroxy-phenylamino)-methyl]-phenylsulfanyl}-phenyl)-acetamide | C21H18Br2N2O2S | D | 522.25 | 522.4 |

| | | | | | | |
|---|---|---|---|---|---|---|
| 1065 | 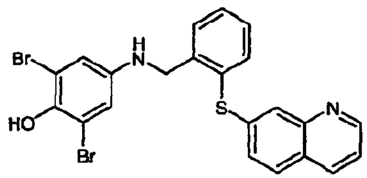 | 2,6-Dibromo-4-[2-(quinolin-7-ylsulfanyl)-benzylamino]-phenol | C22H16Br2N2OS | D | 516.25 | 516.4 |
| 1066 | 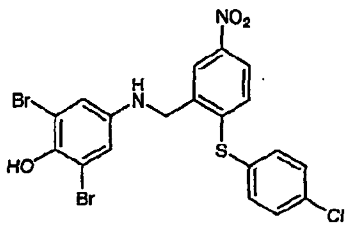 | 2,6-Dibromo-4-[2-(4-chloro-phenylsulfanyl)-5-nitro-benzylamino]-phenol | C19H13Br2ClN2O3 S | D | 544.64 | 544.8 |
| 1067 | 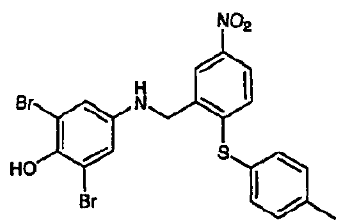 | 2,6-Dibromo-4-(5-nitro-2-p-tolylsulfanyl-benzylamino)-phenol | C20H16Br2N2O3S | D | 524.23 | 524.6 |
| 1068 | 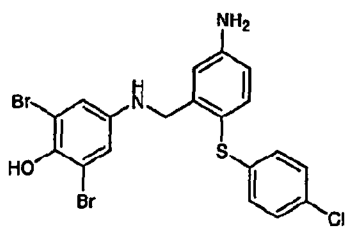 | 4-[5-Amino-2-(4-chloro-phenylsulfanyl)-benzylamino]-2,6-dibromo-phenol | C19H15Br2ClN2OS | D | 514.66 | 514.9 |

| | | | | | | |
|---|---|---|---|---|---|---|
| 1069 | 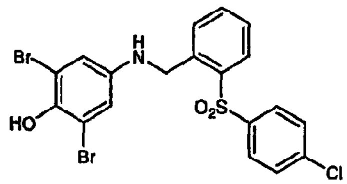 | 2,6-Dibromo-4-[2-(4-chloro-benzenesulfonyl)-benzylamino]-phenol | C19H14Br2ClNO3S | D | 531.65 | 531.7 |
| 1070 | 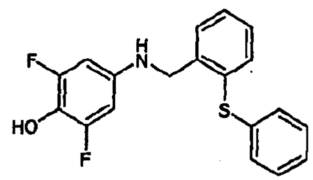 | 2,6-Difluoro-4-(2-phenylsulfanyl-benzylamino)-phenol | C19H15F2NOS | D | 343.39 | 343.6 |
| 1071 | 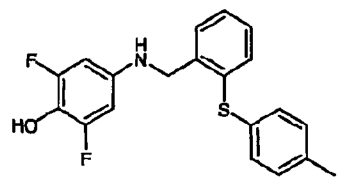 | 2,6-Difluoro-4-(2-p-tolylsulfanyl-benzylamino)-phenol | C20H17F2NOS | D | 357.42 | 357.8 |
| 1072 | 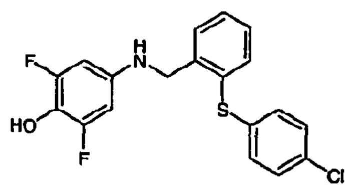 | 4-[2-(4-Chloro-phenylsulfanyl)-benzylamino]-2,6-difluoro-phenol | C19H14ClF2NOS | D | 377.83 | 378.1 |
| 1073 | 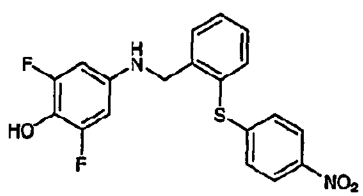 | 2,6-Difluoro-4-[2-(4-nitro-phenylsulfanyl)-benzylamino]-phenol | C19H14F2N2O3S | D | 388.39 | 388.8 |

| | | | | | | |
|---|---|---|---|---|---|---|
| 1074 | 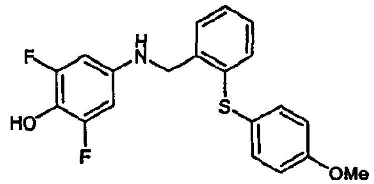 | 2,6-Difluoro-4-[2-(4-methoxy-phenylsulfanyl)-benzylamino]-phenol | C20H17F2NO2S | D | 373.42 | 373.7 |
| 1075 | 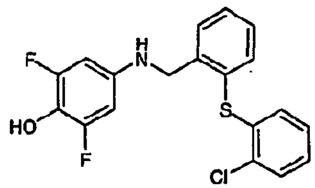 | 4-[2-(2-Chloro-phenylsulfanyl)-benzylamino]-2,6-difluoro-phenol | C19H14ClF2NOS | D | 377.83 | 378.1 |
| 1076 | 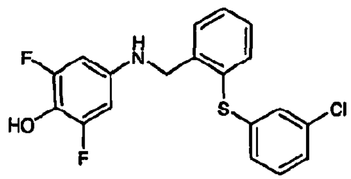 | 4-[2-(3-Chloro-phenylsulfanyl)-benzylamino]-2,6-difluoro-phenol | C19H14ClF2NOS | D | 377.83 | 378.2 |
| 1077 | 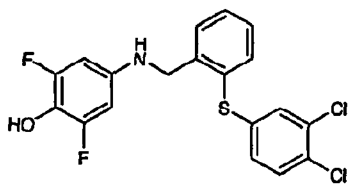 | 4-[2-(3,4-Dichloro-phenylsulfanyl)-benzylamino]-2,6-difluoro-phenol | C19H13Cl2F2NOS | D | 412.28 | 412.6 |
| 1078 | 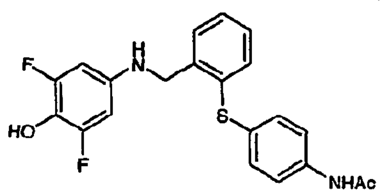 | N-(4-{2-[(3,5-Difluoro-4-hydroxy-phenylamino)-methyl]-phenylsulfanyl}-phenyl)-acetamide | C21H18F2N2O2S | D | 400.44 | 400.8 |

| | | | | | | |
|---|---|---|---|---|---|---|
| 1079 | 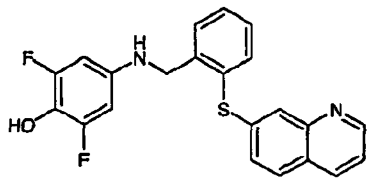 | 2,6-Difluoro-4-[2-(quinolin-7-ylsulfanyl)-benzylamino]-phenol | C22H16F2N2OS | D | 394.44 | 394.9 |
| 1080 | 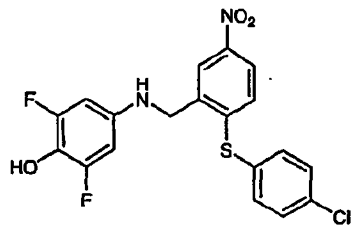 | 4-[2-(4-Chloro-phenylsulfanyl)-5-nitro-benzylamino]-2,6-difluoro-phenol | C19H13ClF2N2O3S | D | 422.83 | 423.3 |
| 1081 | 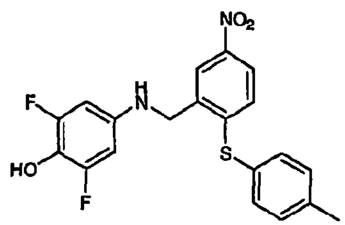 | 2,6-Difluoro-4-(5-nitro-2-p-tolylsulfanyl-benzylamino)-phenol | C20H16F2N2O3S | D | 402.41 | 402.8 |
| 1082 | 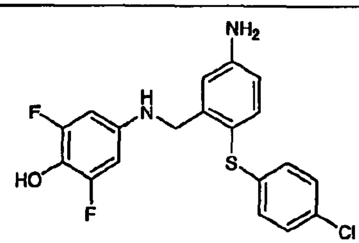 | 4-[5-Amino-2-(4-chloro-phenylsulfanyl)-benzylamino]-2,6-difluoro-phenol | C19H15ClF2N2OS | D | 392.85 | 393.0 |

| | | | | | | |
|---|---|---|---|---|---|---|
| 1083 | 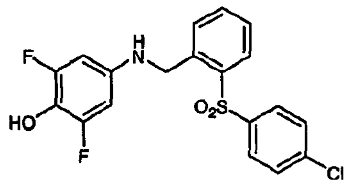 | 4-[2-(4-Chloro-benzenesulfonyl)-benzylamino]-2,6-difluoro-phenol | C19H14ClF2NO3S | D | 391.84 | 392.3 |
| 1084 | 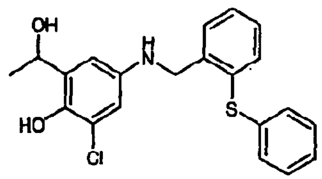 | 2-Chloro-6-(1-hydroxy-ethyl)-4-(2-phenylsulfanyl-benzylamino)-phenol | C21H20ClNO2S | D | 341.85 | 342.3 |
| 1085 | 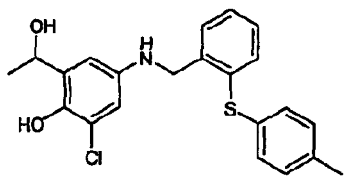 | 2-Chloro-6-(1-hydroxy-ethyl)-4-(2-p-tolylsulfanyl-benzylamino)-phenol | C22H22ClNO2S | D | 399.93 | 400.2 |
| 1086 | 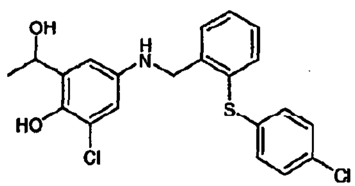 | 2-Chloro-4-[2-(4-chloro-phenylsulfanyl)-benzylamino]-6-(1-hydroxy-ethyl)-phenol | C21H19Cl2NO2S | D | 420.35 | 420.9 |
| 1087 | 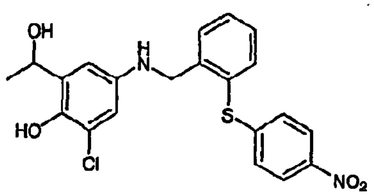 | 2-Chloro-6-(1-hydroxy-ethyl)-4-[2-(4-nitro-phenylsulfanyl)-benzylamino]-phenol | C21H19ClN2O4S | D | 430.90 | 431.3 |

| | | | | | | |
|---|---|---|---|---|---|---|
| 1088 | 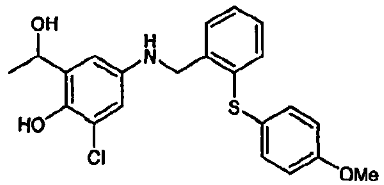 | 2-Chloro-6-(1-hydroxy-ethyl)-4-[2-(4-methoxy-phenylsulfanyl)-benzylamino]-phenol | C22H22ClNO3S | D | 415.93 | 416.3 |
| 1089 | 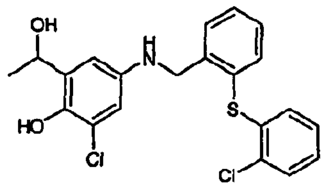 | 2-Chloro-4-[2-(2-chloro-phenylsulfanyl)-benzylamino]-6-(1-hydroxy-ethyl)-phenol | C21H19Cl2NO2S | D | 420.35 | 420.9 |
| 1090 | 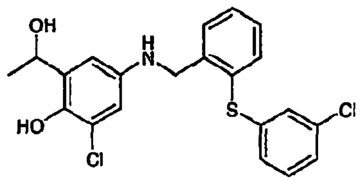 | 2-Chloro-4-[2-(3-chloro-phenylsulfanyl)-benzylamino]-6-(1-hydroxy-ethyl)-phenol | C21H19Cl2NO2S | D | 420.35 | 420.8 |
| 1091 | 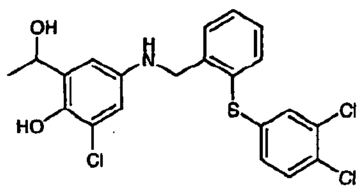 | 2-Chloro-4-[2-(3,4-dichloro-phenylsulfanyl)-benzylamino]-6-(1-hydroxy-ethyl)-phenol | C21H18Cl3NO2S | D | 454.80 | 455.2 |
| 1092 | 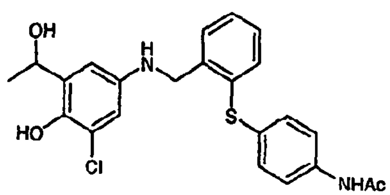 | N-[4-(2-{[3-Chloro-4-hydroxy-5-(1-hydroxy-ethyl)-phenylamino]-methyl}-phenylsulfanyl)-phenyl]-acetamide | C23H23ClN2O3S | D | 442.96 | 443.4 |

| | | | | | | |
|---|---|---|---|---|---|---|
| 1093 | 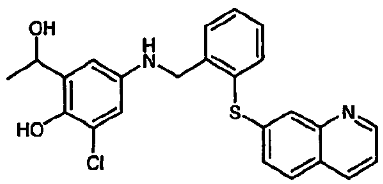 | 2-Chloro-6-(1-hydroxy-ethyl)-4-[2-(quinolin-7-ylsulfanyl)-benzylamino]-phenol | C24H21ClN2O2S | D | 436.95 | 437.3 |
| 1094 | 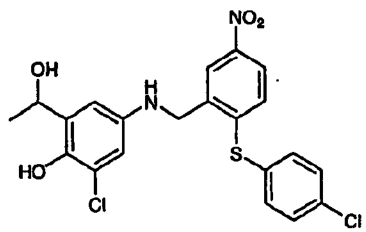 | 2-Chloro-4-[2-(4-chloro-phenylsulfanyl)-5-nitro-benzylamino]-6-(1-hydroxy-ethyl)-phenol | C21H18Cl2N2O4S | D | 465.35 | 465.9 |
| 1095 | 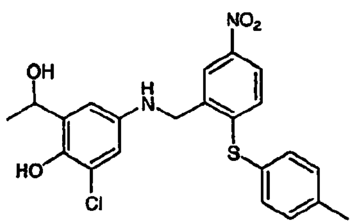 | 2-Chloro-4-(1-hydroxy-ethyl)-6-(5-nitro-2-p-tolylsulfanyl-benzylamino)-phenol | C22H21ClN2O4S | D | 444.93 | 445.3 |
| 1096 | 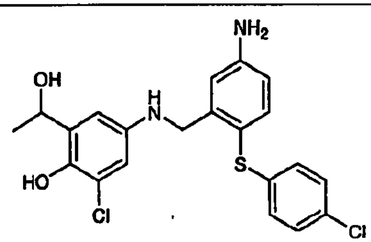 | 4-[5-Amino-2-(4-chloro-phenylsulfanyl)-benzylamino]-2-chloro-6-(1-hydroxy-ethyl)-phenol | C21H20Cl2N2O2S | D | 435.37 | 435.6 |

| | | | | | | |
|---|---|---|---|---|---|---|
| 1097 | 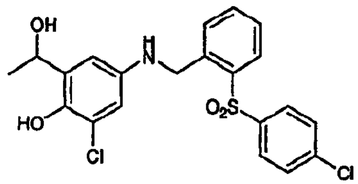 | 2-Chloro-4-[2-(4-chloro-benzenesulfonyl)-benzylamino]-6-(1-hydroxy-ethyl)-phenol | C21H19Cl2NO4S | D | 452.35 | 452.5 |
| 1098 | 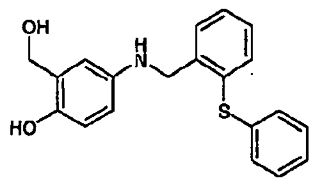 | 2-Hydroxymethyl-4-(2-phenylsulfanyl-benzylamino)-phenol | C20H19NO2S | D | 337.44 | 337.8 |
| 1099 | 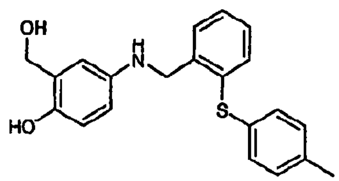 | 2-Hydroxymethyl-4-(2-p-tolylsulfanyl-benzylamino)-phenol | C21H21NO2S | D | 351.46 | 351.8 |
| 1100 | 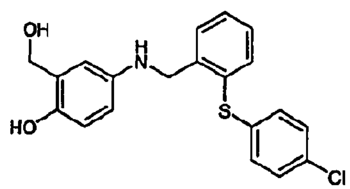 | 4-[2-(4-Chloro-phenylsulfanyl)-benzylamino]-2-hydroxymethyl-phenol | C20H18ClNO2S | D | 371.88 | 371.9 |
| 1101 | 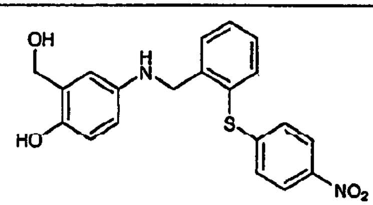 | 2-Hydroxymethyl-4-[2-(4-nitro-phenylsulfanyl)-benzylamino]-phenol | C20H18N2O4S | D | 382.43 | 382.7 |

| | | | | | | |
|---|---|---|---|---|---|---|
| 1102 | 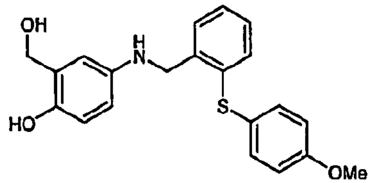 | 2-Hydroxymethyl-4-[2-(4-methoxy-phenylsulfanyl)-benzylamino]-phenol | C21H21NO3S | D | 367.46 | 367.9 |
| 1103 | 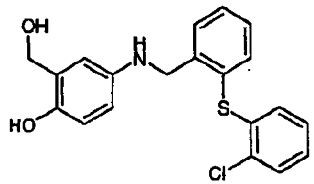 | 4-[2-(2-Chloro-phenylsulfanyl)-benzylamino]-2-hydroxymethyl-phenol | C20H18ClNO2S | D | 371.88 | 371.9 |
| 1104 | 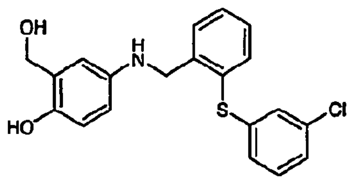 | 4-[2-(3-Chloro-phenylsulfanyl)-benzylamino]-2-hydroxymethyl-phenol | C20H18ClNO2S | D | 371.88 | 371.9 |
| 1105 | 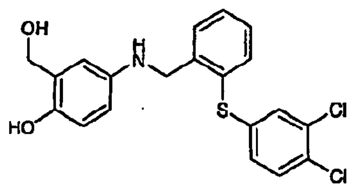 | 4-[2-(3,4-Dichloro-phenylsulfanyl)-benzylamino]-2-hydroxymethyl-phenol | C20H17Cl2NO2S | D | 406.33 | 406.5 |
| 1106 | 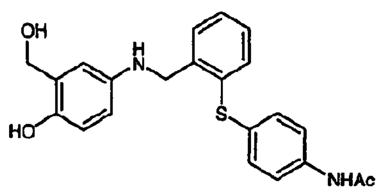 | N-(4-{2-[(4-Hydroxy-3-hydroxymethyl-phenylamino)-methyl]-phenylsulfanyl}-phenyl)-acetamide | C22H22N2O3S | D | 394.49 | 349.9 |

| | | | | | | |
|---|---|---|---|---|---|---|
| 1107 | 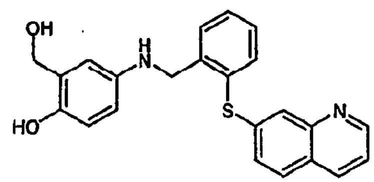 | 2-Hydroxymethyl-4-[2-(quinolin-7-ylsulfanyl)-benzylamino]-phenol | C23H20N2O2S | D | 388.48 | 388.8 |
| 1108 | 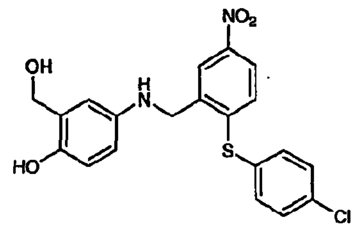 | 4-[2-(4-Chloro-phenylsulfanyl)-5-nitro-benzylamino]-2-hydroxymethyl-phenol | C20H17ClN2O4S | D | 416.88 | 417.2 |
| 1109 | 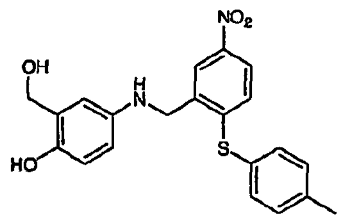 | 2-Hydroxymethyl-4-(5-nitro-2-p-tolylsulfanyl-benzylamino)-phenol | C21H20N2O4S | D | 396.46 | 396.8 |
| 1110 | 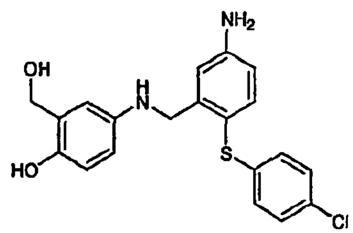 | 4-[5-Amino-2-(4-chloro-phenylsulfanyl)-benzylamino]-2-hydroxymethyl-phenol | C20H19ClN2O2S | D | 386.90 | 387.1 |

| | | | | | | |
|---|---|---|---|---|---|---|
| 1111 | 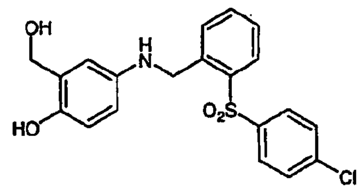 | 4-[2-(4-Chloro-benzenesulfonyl)-benzylamino]-2-hydroxymethyl-phenol | C20H18ClNO4S | D | 403.88 | 44.0 |
| 1112 | 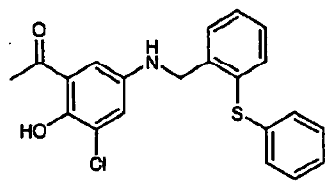 | 1-[3-Chloro-2-hydroxy-5-(2-phenylsulfanyl-benzylamino)-phenyl]-ethanone | C21H18ClNO2S | D | 383.89 | 384.2 |
| 1113 | 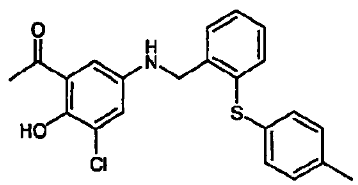 | 1-[3-Chloro-2-hydroxy-5-(2-p-tolylsulfanyl-benzylamino)-phenyl]-ethanone | C22H20ClNO2S | D | 397.92 | 398.2 |
| 1114 | 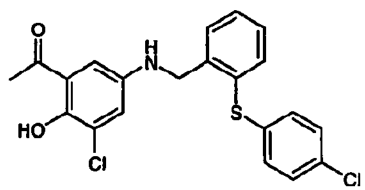 | 1-{3-Chloro-5-[2-(4-chloro-phenylsulfanyl)-benzylamino]-2-hydroxy-phenyl}-ethanone | C21H17Cl2NO2S | D | 418.34 | 418.8 |
| 1115 | 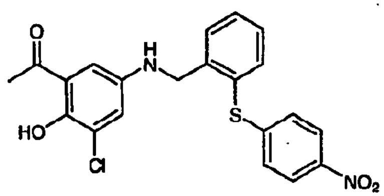 | 1-{3-Chloro-2-hydroxy-5-[2-(4-nitro-phenylsulfanyl)-benzylamino]-phenyl}-ethanone | C21H17ClN2O4S | D | 428.89 | 429.3 |

| | | | | | | |
|---|---|---|---|---|---|---|
| 1116 | 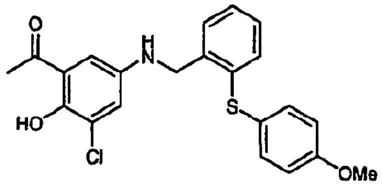 | 1-{3-Chloro-2-hydroxy-5-[2-(4-methoxy-phenylsulfanyl)-benzylamino]-phenyl}-ethanone | C22H20ClNO3S | D | 413.92 | 414.5 |
| 1117 | 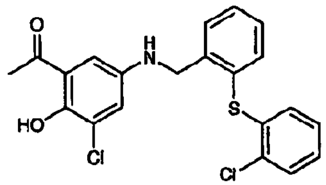 | 1-{3-Chloro-5-[2-(2-chloro-phenylsulfanyl)-benzylamino]-2-hydroxy-phenyl}-ethanone | C21H17Cl2NO2S | D | 418.34 | 418.6 |
| 1118 | 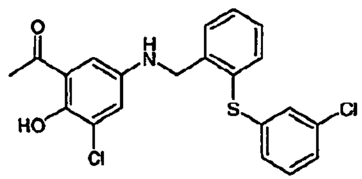 | 1-{3-Chloro-5-[2-(3-chloro-phenylsulfanyl)-benzylamino]-2-hydroxy-phenyl}-ethanone | C21H17Cl2NO2S | D | 418.34 | 418.5 |
| 1119 | 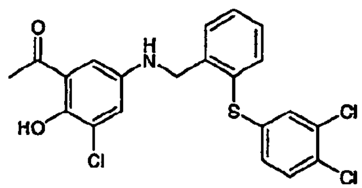 | 1-{3-Chloro-5-[2-(3,4-dichloro-phenylsulfanyl)-benzylamino]-2-hydroxy-phenyl}-ethanone | C21H16Cl3NO2S | D | 452.78 | 453.0 |
| 1120 | 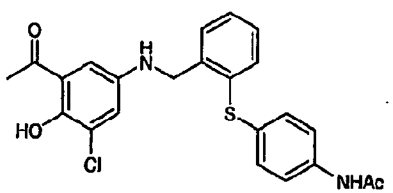 | N-(4-{2-[(3-Acetyl-5-chloro-4-hydroxy-phenylamino)-methyl]-phenylsulfanyl}-phenyl)-acetamide | C23H21ClN2O3S | D | 440.94 | 441.2 |

| | | | | | | |
|---|---|---|---|---|---|---|
| 1121 | 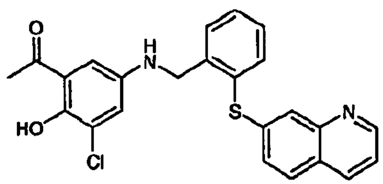 | 1-{3-Chloro-2-hydroxy-5-[2-(quinolin-7-ylsulfanyl)-benzylamino]-phenyl}-ethanone | C24H19ClN2O2S | D | 434.94 | 435.2 |
| 1122 | 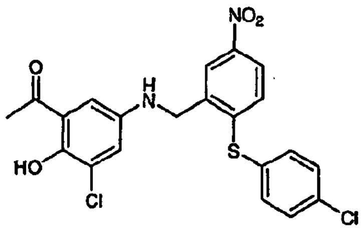 | 1-{3-Chloro-5-[2-(4-chloro-phenylsulfanyl)-5-nitro-benzylamino]-2-hydroxy-phenyl}-ethanone | C21H16Cl2N2O4S | D | 463.33 | 463.7 |
| 1123 | 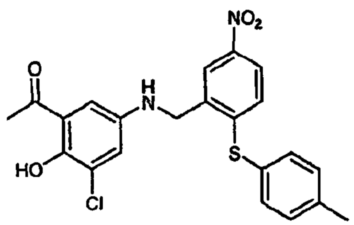 | 1-[3-Chloro-2-hydroxy-5-(5-nitro-2-p-tolylsulfanyl-benzylamino)-phenyl]-ethanone | C22H19ClN2O4S | D | 442.92 | 443.2 |
| 1124 | 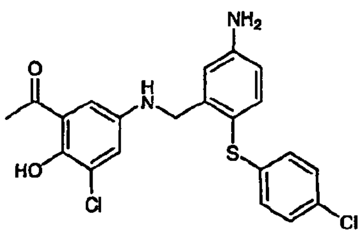 | 1-{5-[5-Amino-2-(4-chloro-phenylsulfanyl)-benzylamino]-3-chloro-2-hydroxy-phenyl}-ethanone | C21H18Cl2N2O2S | D | 433.35 | 433.6 |

| | | | | | | |
|---|---|---|---|---|---|---|
| 1125 | 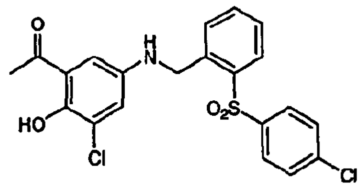 | 1-{3-Chloro-5-[2-(4-chloro-benzenesulfonyl)-benzylamino]-2-hydroxy-phenyl}-ethanone | C21H17Cl2NO4S | D | 450.33 | 450.5 |
| 1126 | 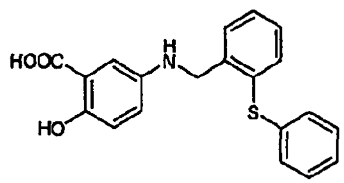 | 2-Hydroxy-5-(2-phenylsulfanyl-benzylamino)-benzoic acid | C20H17NO3S | D | 351.42 | 351.6 |
| 1127 | 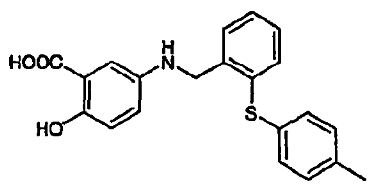 | 2-Hydroxy-5-(2-p-tolylsulfanyl-benzylamino)-benzoic acid | C21H19NO3S | D | 365.44 | 365.8 |
| 1128 | 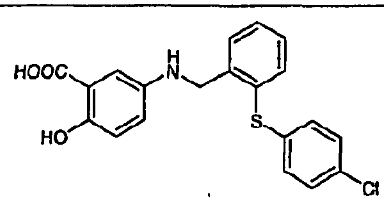 | 5-[2-(4-Chloro-phenylsulfanyl)-benzylamino]-2-hydroxy-benzoic acid | C20H16ClNO3S | D | 385.86 | 385.1 |
| 1129 | 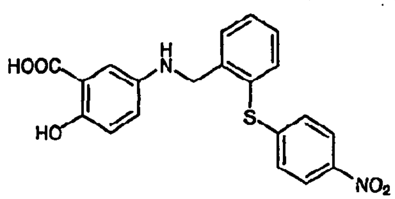 | 2-Hydroxy-5-[2-(4-nitro-phenylsulfanyl)-benzylamino]-benzoic acid | C20H16N2O5S | D | 396.11 | 396.4 |

| | | | | | | |
|---|---|---|---|---|---|---|
| 1130 | 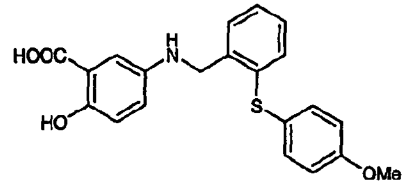 | 2-Hydroxy-5-[2-(4-methoxy-phenylsulfanyl)-benzylamino]-benzoic acid | $C_{21}H_{19}NO_4S$ | D | 381.44 | 381.6 |
| 1131 | 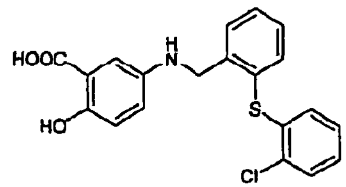 | 5-[2-(2-Chloro-phenylsulfanyl)-benzylamino]-2-hydroxy-benzoic acid | $C_{20}H_{16}ClNO_3S$ | D | 385.86 | 386.0 |
| 1132 | 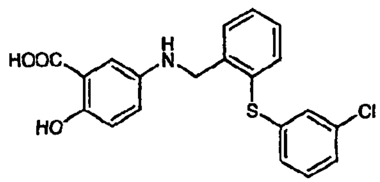 | 5-[2-(3-Chloro-phenylsulfanyl)-benzylamino]-2-hydroxy-benzoic acid | $C_{20}H_{16}ClNO_3S$ | D | 385.86 | 386.0 |
| 1133 | 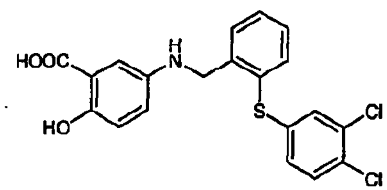 | 5-[2-(3,4-Dichloro-phenylsulfanyl)-benzylamino]-2-hydroxy-benzoic acid | $C_{20}H_{15}Cl_2NO_3S$ | D | 420.31 | 420.7 |
| 1134 | 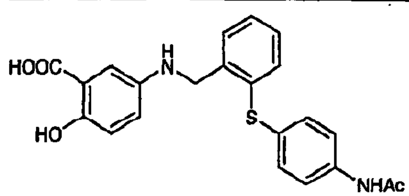 | 5-[2-(4-Acetylamino-phenylsulfanyl)-benzylamino]-2-hydroxy-benzoic acid | $C_{22}H_{20}N_2O_4S$ | D | 408.47 | 408.7 |

| | | | | | | |
|---|---|---|---|---|---|---|
| 1135 | 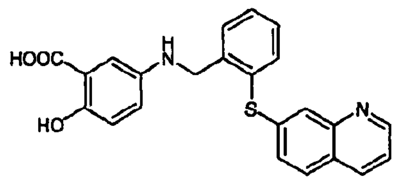 | 2-Hydroxy-5-[2-(quinolin-7-ylsulfanyl)-benzylamino]-benzoic acid | C23H18N2O3S | D | 402.46 | 402.8 |
| 1136 | 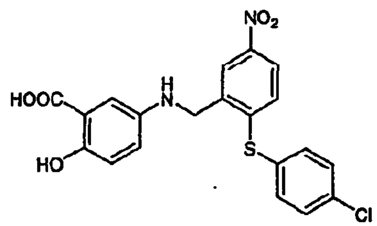 | 5-[2-(4-Chloro-phenylsulfanyl)-5-nitro-benzylamino]-2-hydroxy-benzoic acid | C20H15ClN2O5S | D | 430.86 | 430.9 |
| 1137 | 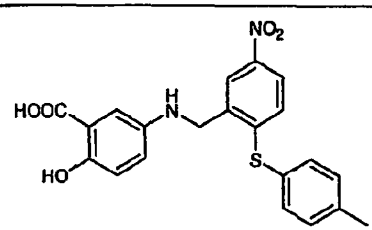 | 2-Hydroxy-5-(5-nitro-2-p-tolylsulfanyl-benzylamino)-benzoic acid | C21H18N2O5S | D | 410.44 | 410.7 |
| 1138 | 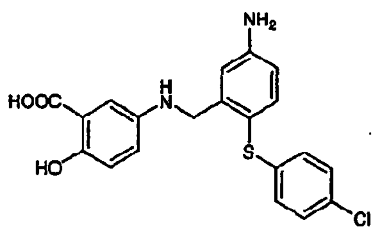 | 5-[5-Amino-2-(4-chloro-phenylsulfanyl)-benzylamino]-2-hydroxymethyl-phenol | C20H19ClN2O2S | D | 386.90 | 387.2 |

| | | | | | | |
|---|---|---|---|---|---|---|
| 1139 | 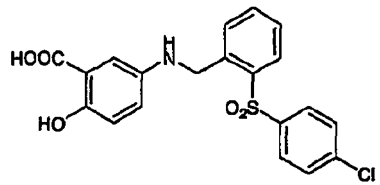 | 5-[2-(4-Chloro-benzenesulfonyl)-benzylamino]-2-hydroxy-benzoic acid | C20H16ClNO5S | D | 403.88 | 404.1 |
| 1140 | 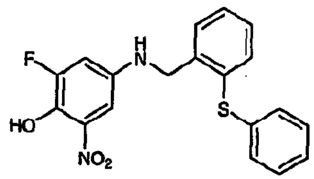 | 2-Fluoro-6-nitro-4-(2-phenylsulfanyl-benzylamino)-phenol | C19H15FN2O3S | D | 370.40 | 370.8 |
| 1141 | 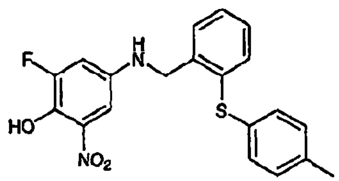 | 2-Fluoro-6-nitro-4-(2-p-tolylsulfanyl-benzylamino)-phenol | C20H17FN2O3S | D | 384.43 | 384.9 |
| 1142 | 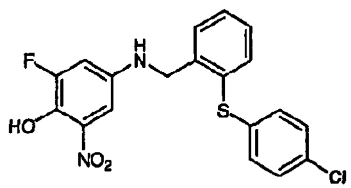 | 4-[2-(4-Chloro-phenylsulfanyl)-benzylamino]-2-fluoro-6-nitro-phenol | C19H14ClFN2O3S | D | 404.84 | 405.0 |
| 1143 | 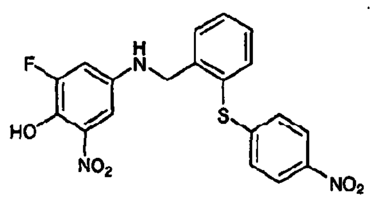 | 2-Fluoro-6-nitro-4-[2-(4-nitro-phenylsulfanyl)-benzylamino]-phenol | C19H14FN3O5S | D | 415.40 | 415.8 |

| | | | | | | |
|---|---|---|---|---|---|---|
| 1144 | 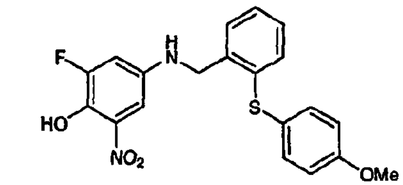 | 2-Fluoro-4-[2-(4-methoxy-phenylsulfanyl)-benzylamino]-6-nitro-phenol | C20H17FN2O4S | D | 400.43 | 400.9 |
| 1145 | 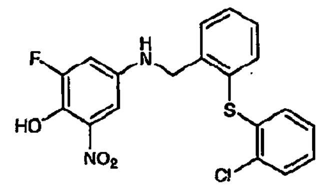 | 4-[2-(2-Chloro-phenylsulfanyl)-benzylamino]-2-fluoro-6-nitro-phenol | C19H14ClFN2O3S | D | 404.84 | 405.0 |
| 1146 | 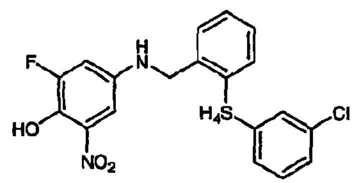 | 4-[2-(3-Chloro-phenylsulfanyl)-benzylamino]-2-fluoro-6-nitro-phenol | C19H14ClFN2O3S | D | 404.84 | 405.0 |
| 1147 | 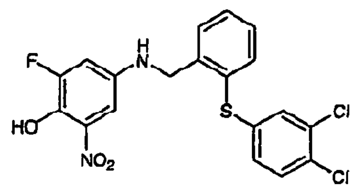 | 4-[2-(3,4-Dichloro-phenylsulfanyl)-benzylamino]-2-fluoro-6-nitro-phenol | C19H13Cl2FN2O3S | D | 439.29 | 439.9 |
| 1148 | 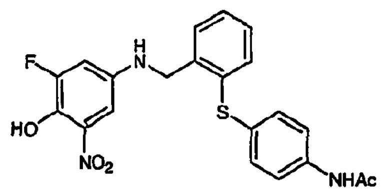 | N-(4-{2-[(3-Fluoro-4-hydroxy-5-nitro-phenylamino)-methyl]-phenylsulfanyl}-phenyl)-acetamide | C21H18FN3O4S | D | 427.45 | 427.9 |

| | | | | | | |
|---|---|---|---|---|---|---|
| 1149 | 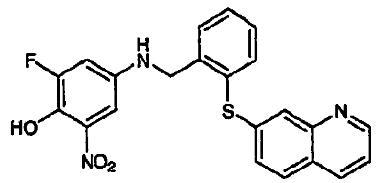 | 2-Fluoro-6-nitro-4-[2-(quinolin-7-ylsulfanyl)-benzylamino]-phenol | C22H16FN3O3S | D | 421.45 | 421.6 |
| 1150 | 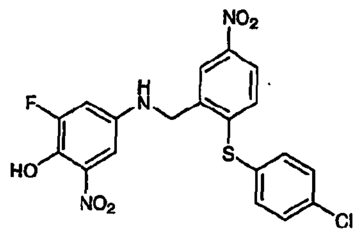 | 4-[2-(4-Chloro-phenylsulfanyl)-5-nitro-benzylamino]-2-fluoro-6-nitro-phenol | C19H13ClFN3O5S | D | 449.84 | 450.1 |
| 1151 | 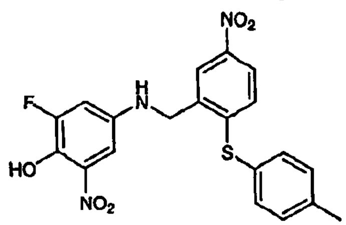 | 2-Fluoro-6-nitro-4-(5-nitro-2-p-tolylsulfanyl-benzylamino)-phenol | C20H16FN3O4S | D | 429.42 | 429.8 |
| 1152 | 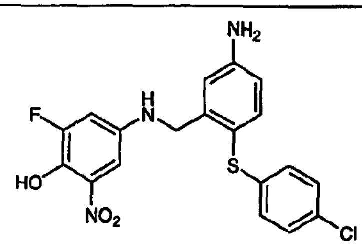 | 4-[5-Amino-2-(4-chloro-phenylsulfanyl)-benzylamino]-2-fluoro-6-nitro-phenol | C19H15ClFN3O2S | D | 419.86 | 420.2 |

| | | | | | | |
|---|---|---|---|---|---|---|
| 1153 | 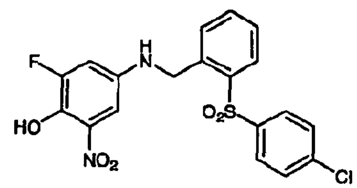 | 4-[2-(4-Chloro-benzenesulfonyl)-benzylamino]-2-fluoro-6-nitro-phenol | C19H15ClFN2O5S | D | 436.84 | 437.1 |
| 1154 | 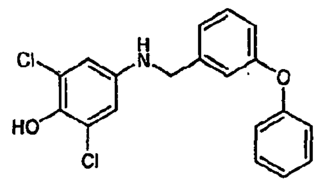 | 2,6-Dichloro-4-(3-phenoxy-benzylamino)-phenol | C19H15Cl2NO2 | D | 360.23 | 360.5 |
| 1155 | 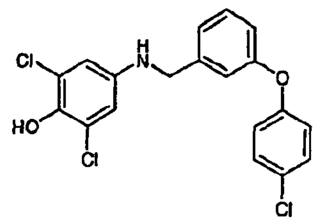 | 2,6-Dichloro-4-[3-(4-chloro-phenoxy)-benzylamino]-phenol | C19H15Cl3NO2 | D | 394.68 | 395.0 |
| 1156 | 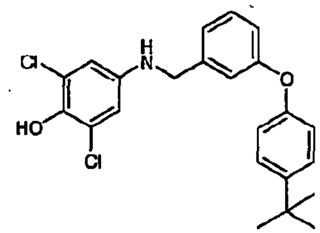 | 4-[3-(4-tert-Butyl-phenoxy)-benzylamino]-2,6-dichloro-phenol | C23H23Cl2NO2 | D | 416.34 | 416.8 |

| | | | | | | |
|---|---|---|---|---|---|---|
| 1157 | 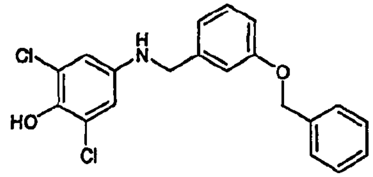 | 4-(3-Benzyloxy-benzylamino)-2,6-dichloro-phenol | C20H17Cl2NO2 | D | 374.26 | 374.6 |
| 1158 | 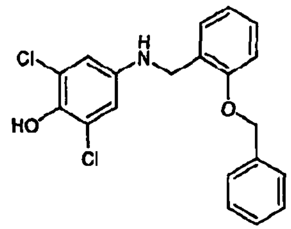 | 4-(2-Benzyloxy-benzylamino)-2,6-dichloro-phenol | C20H17Cl2NO2 | D | 374.26 | 374.6 |
| 1159 | 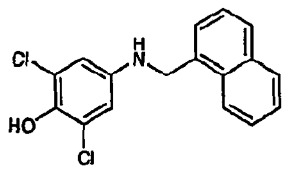 | 2,6-Dichloro-4-[(naphthalen-1-ylmethyl)-amino]-phenol | C17H13Cl2NO | D | 318.20 | 318.6 |
| 1160 | 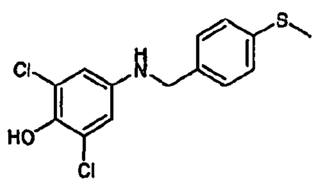 | 2,6-Dichloro-4-(4-methylsulfanyl-benzylamino)-phenol | C14H13Cl2NOS | D | 314.23 | 314.7 |
| 1161 | 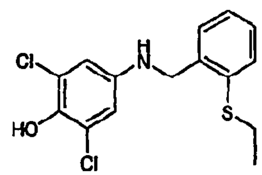 | 2,6-Dichloro-4-(2-ethylsulfanyl-benzylamino)-phenol | C15H15Cl2NOS | D | 328.26 | 328.6 |

| | | | | | | |
|---|---|---|---|---|---|---|
| 1162 | 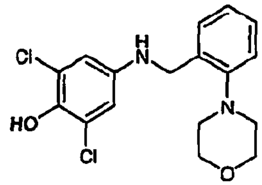 | 2,6-Dichloro-4-(2-morpholin-4-yl-benzylamino)-phenol | C17H18Cl2N2O2 | D | 353.24 | 353.6 |
| 1163 | 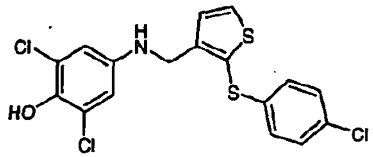 | 2,6-Dichloro-4-{[2-(4-chloro-phenylsulfanyl)-thiophen-3-ylmethyl]-amino}-phenol | C17H12Cl3NOS2 | D | 416.77 | 416.9 |
| 1164 | 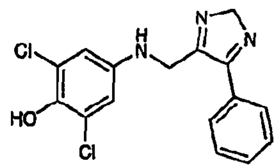 | 2,6-Dichloro-4-[(5-phenyl-2H-imidazol-4-ylmethyl)-amino]-phenol | C16H13Cl2N3O | D | 334.20 | 334.6 |
| 1165 | 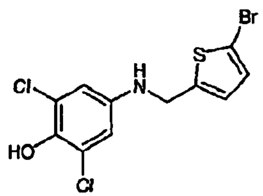 | 4-[(5-Bromo-thiophen-2-ylmethyl)-amino]-2,6-dichloro-phenol | C11H8BrCl2NOS | D | 353.06 | 353.4 |
| 1166 | 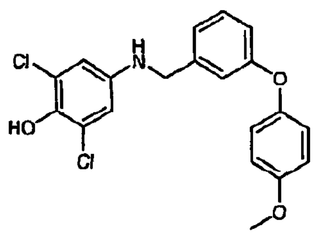 | 2,6-Dichloro-4-[3-(4-methoxy-phenoxy)-benzylamino]-phenol | C20H17Cl2NO3 | D | 390.26 | 390.6 |

| | | | | | | |
|---|---|---|---|---|---|---|
| 1167 | 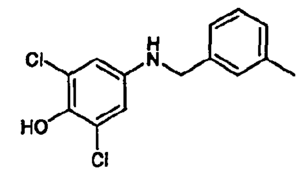 | 2,6-Dichloro-4-(3-methyl-benzylamino)-phenol | C14H13Cl2NO | D | 282.17 | 282.4 |
| 1168 | 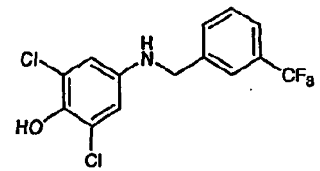 | 2,6-Dichloro-4-(3-trifluoromethyl-benzylamino)-phenol | C14H10Cl2F3NO | D | 336.14 | 336.5 |
| 1169 | 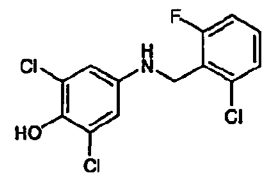 | 2,6-Dichloro-6-(2-chloro-6-fluoro-benzylamino)-phenol | C13H9Cl3FNO | D | 320.57 | 320.8 |
| 1170 | 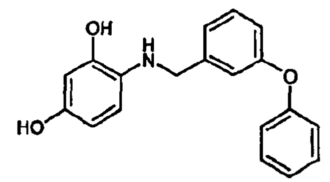 | 4-(3-Phenoxy-benzylamino)-benzene-1,3-diol | C19H17NO3 | D | 307.34 | 307.8 |
| 1171 | 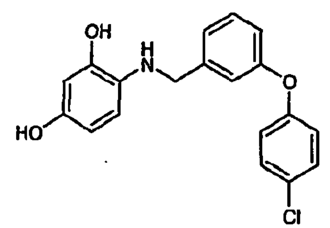 | 4-[3-(4-Chloro-phenoxy)-benzylamino]-benzene-1,3-diol | C19H16ClNO3 | D | 341.79 | 342.1 |

| | | | | | | |
|---|---|---|---|---|---|---|
| 1172 | 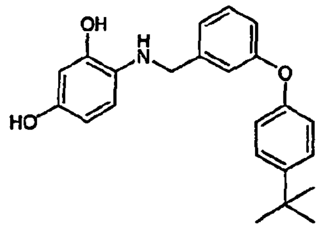 | 4-[3-(4-tert-Butyl-phenoxy)-benzylamino]-benzene-1,3-diol | C23H25NO3 | D | 363.45 | 363.9 |
| 1173 | 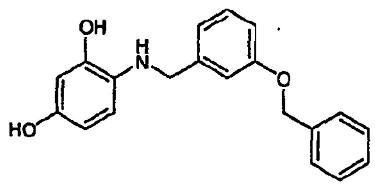 | 4-(3-Benzyloxy-benzylamino)-benzene-1,3-diol | C20H19NO3 | D | 321.37 | 321.6 |
| 1174 | 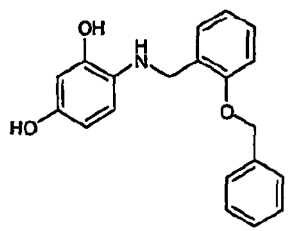 | 4-(2-Benzyloxy-benzylamino)-benzene-1,3-diol | C20H19NO3 | D | 321.37 | 321.5 |
| 1175 | 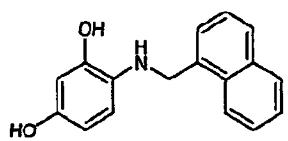 | 4-[(Naphthalen-1-ylmethyl)-amino]-benzene-1,3-diol | C17H15NO2 | D | 265.31 | 265.7 |
| 1176 | 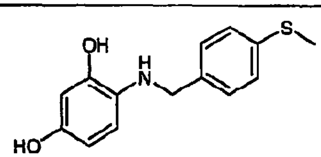 | 4-(4-Methylsulfanyl-benzylamino)-benzene-1,3-diol | C14H15NO2S | D | 261.34 | 261.7 |

| | | | | | | |
|---|---|---|---|---|---|---|
| 1177 | 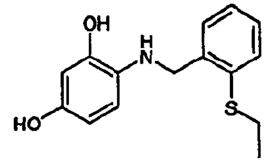 | 4-(2-Ethylsulfanyl-benzylamino)-benzene-1,3-diol | C15H17NO2S | D | 275.37 | 275.9 |
| 1178 | 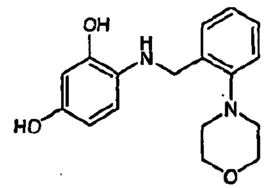 | 4-(2-Morpholin-4-yl-benzylamino)-benzene-1,3-diol | C17H20N2O3 | D | 300.35 | 300.8 |
| 1179 | 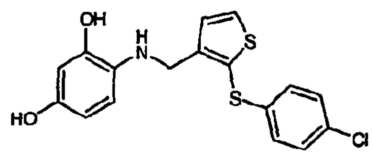 | 4-{[2-(4-Chloro-phenylsulfanyl)-thiophen-3-ylmethyl]-amino}-benzene-1,3-diol | C17H14ClNO2S2 | D | 363.88 | 364.1 |
| 1180 | 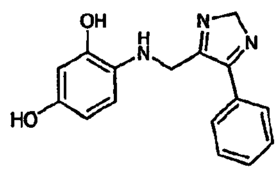 | 4-[(5-Phenyl-2H-imidazol-4-ylmethyl)-amino]-benzene-1,3-diol | C16H15N3O2 | D | 281.31 | 281.6 |
| 1181 | 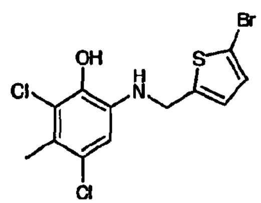 | 2-[(5-Bromo-thiophen-2-ylmethyl)-amino]-4,6-dichloro-3-methyl-phenol | C12H10BrCl2NOS | D | 367.09 | 367.4 |

| | | | | | | |
|---|---|---|---|---|---|---|
| 1182 | 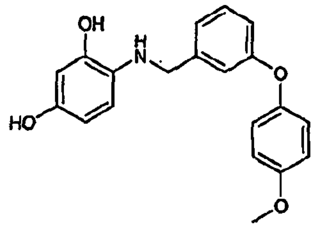 | 4-[3-(4-Methoxy-phenoxy)-benzylamino]-benzene-1,3-diol | C20H19NO4 | D | 337.37 | 337.8 |
| 1183 | 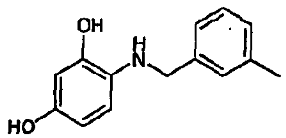 | 4-(3-Methyl-benzylamino)-benzene-1,3-diol | C14H15NO2 | D | 229.27 | 229.6 |
| 1184 | 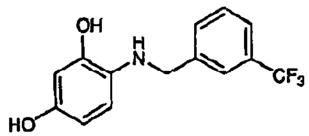 | 4-(3-Trifluoromethyl-benzylamino)-benzene-1,3-diol | C14H12F3NO2 | D | 283.25 | 283.6 |
| 1185 | 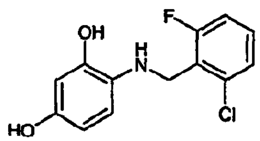 | 4-(2-Chloro-6-fluoro-benzylamino)-benzene-1,3-diol | C13H11ClFNO2 | D | 267.68 | 268.0 |
| 1186 | 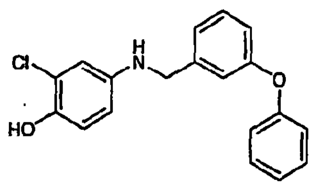 | 2-Chloro-4-(3-phenoxy-benzylamino)-phenol | C19H16ClNO2 | D | 325.79 | 326.1 |

| | | | | | | |
|---|---|---|---|---|---|---|
| 1187 | 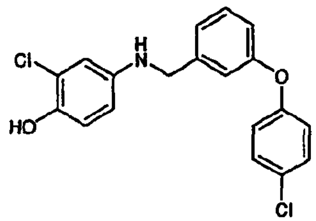 | 2-Chloro-4-[3-(4-chloro-phenoxy)-benzylamino]-phenol | C19H15Cl2NO2 | D | 360.23 | 360.6 |
| 1188 | 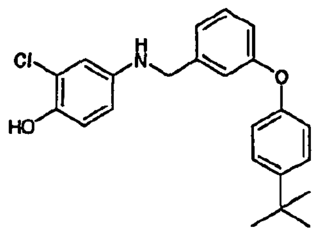 | 4-[3-(4-tert-Butyl-phenoxy)-benzylamino]-2-chloro-phenol | C23H24ClNO2 | D | 381.90 | 382.2 |
| 1189 | 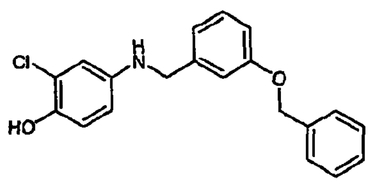 | 4-(3-Benzyloxy-benzylamino)-2-chloro-phenol | C20H18ClNO2 | D | 339.82 | 340.1 |
| 1190 | 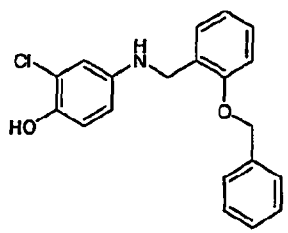 | 4-(2-Benzyloxy-benzylamino)-2-chloro-phenol | C20H18ClNO2 | D | 339.82 | 340.1 |

| | | | | | | |
|---|---|---|---|---|---|---|
| 1191 | 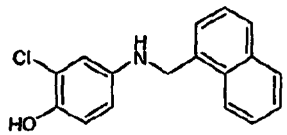 | 2-Chloro-4-[(naphthalen-1-ylmethyl)-amino]-phenol | C17H14ClNO | D | 283.75 | 284.0 |
| 1192 | 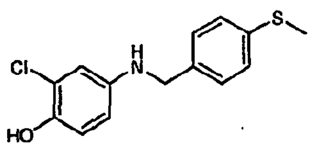 | 2-Chloro-4-(4-methylsulfanyl-benzylamino)-phenol | C14H14ClNOS | D | 279.79 | 280.2 |
| 1193 | 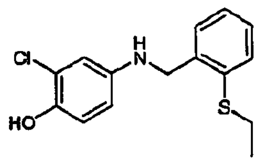 | 2-Chloro-4-(2-ethylsulfanyl-benzylamino)-phenol | C15H16ClNOS | D | 293.81 | 294.3 |
| 1194 | 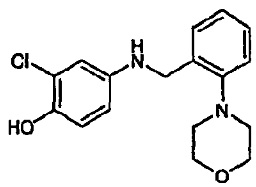 | 2-Chloro-4-(2-morpholin-4-yl-benzylamino)-phenol | C17H19ClN2O2 | D | 318.80 | 319.1 |
| 1195 | 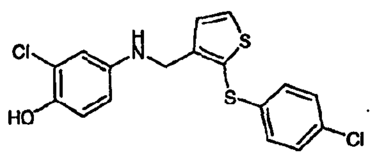 | 2-Chloro-4-{[2-(4-chloro-phenylsulfanyl)-thiophen-3-ylmethyl]-amino}-phenol | C17H13Cl2NOS2 | D | 382.33 | 382.6 |

| | | | | | | |
|---|---|---|---|---|---|---|
| 1196 | 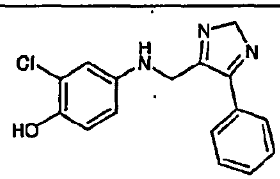 | 2-Chloro-4-[(5-phenyl-2H-imidazol-4-ylmethyl)-amino]-phenol | C16H14ClN3O | D | 299.75 | 300.2 |
| 1197 | 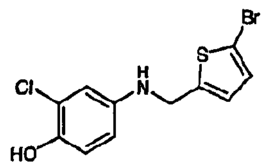 | 4-[(5-Bromo-thiophen-2-ylmethyl)-amino]-2-chloro-phenol | C11H9BrClNOS | D | 318.62 | 319.5 |
| 1198 | 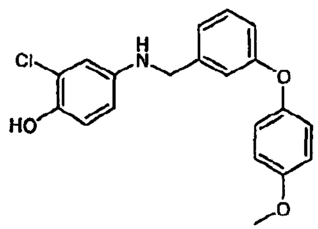 | 2-Chloro-4-[3-(4-methoxy-phenoxy)-benzylamino]-phenol | C20H18ClNO3 | D | 355.81 | 356.5 |
| 1199 | 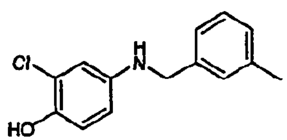 | 2-Chloro-4-(3-methyl-benzylamino)-phenol | C14H14ClNO | D | 247.72 | 248.5 |

| | Structure | Chemical name | Formula | Synthesis methods | MolWeight | MS data |
|---|---|---|---|---|---|---|
| 1200 | 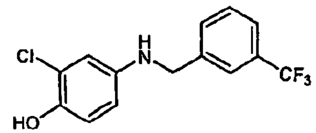 | 2-Chloro-4-(3-trifluoromethyl-benzylamino)-phenol | C14H11ClF3NO | D | 301.69 | 302.2 |
| 1201 | 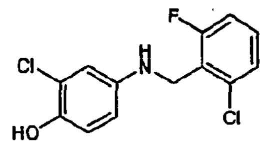 | 2-Chloro-4-(2-chloro-6-fluoro-benzylamino)-phenol | C13H10Cl2FNO | D | 286.13 | 286.6 |
| 1202 | 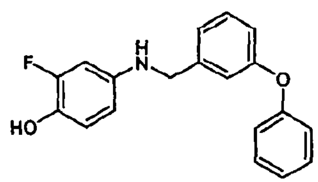 | 2-Fluoro-4-(3-phenoxy-benzylamino)-phenol | C19H16FNO2 | D | 309.33 | 309.8 |
| 1203 | 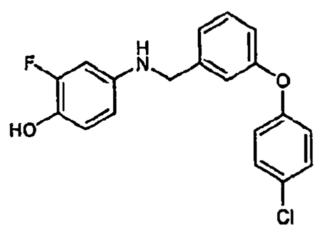 | 2-Fluoro-4-[3-(4-chloro-phenoxy)-benzylamino]-phenol | C19H15ClFNO2 | D | 343.77 | 344.0 |

| | | | | | | |
|---|---|---|---|---|---|---|
| 1204 | 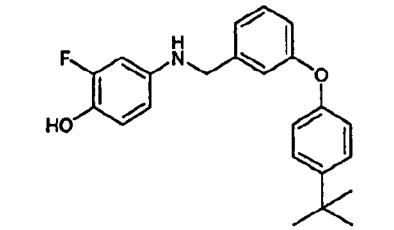 | 4-[3-(4-tert-Butyl-phenoxy)-benzylamino]-2-fluoro-phenol | C23H24FNO2 | D | 365.44 | 366.0 |
| 1205 | 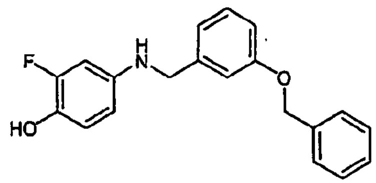 | 4-(3-Benzyloxy-benzylamino)-2-fluoro-phenol | C20H18FNO2 | D | 323.36 | 323.8 |
| 1206 | 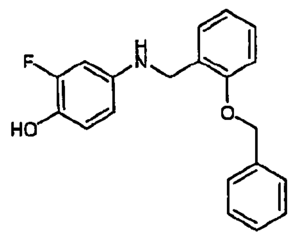 | 4-(2-Benzyloxy-benzylamino)-2-fluoro-phenol | C20H18FNO2 | D | 323.36 | 323.8 |
| 1207 | 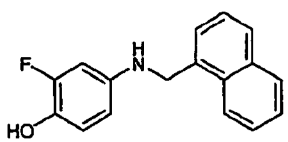 | 2-Fluoro-4-[(naphthalen-1-ylmethyl)-amino]-phenol | C17H14FNO | D | 267.30 | 267.7 |
| 1208 | 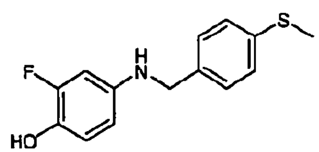 | 2-Fluoro-4-(4-methylsulfanyl-benzylamino)-phenol | C14H14FNOS | D | 263.33 | 263.5 |

| | | | | | | |
|---|---|---|---|---|---|---|
| 1209 | 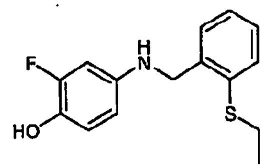 | 2-Fluoro-4-(2-ethylsulfanyl-benzylamino)-phenol | C15H16FNOS | D | 277.36 | 277.8 |
| 1210 | 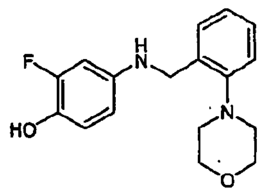 | 2-Fluoro-4-(2-morpholin-4-yl-benzylamino)-phenol | C17H19FN2O2 | D | 302.34 | 302.8 |
| 1211 | 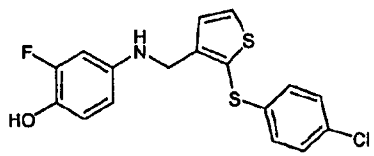 | 2-Fluoro-4-{[2-(4-chloro-phenylsulfanyl)-thiophen-3-ylmethyl]-amino}-phenol | C17H13ClFNOS2 | D | 365.87 | 366.0 |
| 1212 | 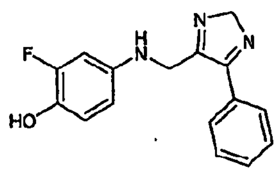 | 2-Fluoro-4-[(5-phenyl-2H-imidazol-4-ylmethyl)-amino]-phenol | C16H14FN3O | D | 283.30 | 283.8 |
| 1213 | 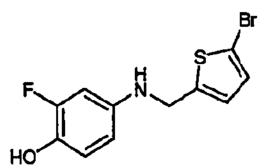 | 4-[(5-Bromo-thiophen-2-ylmethyl)-amino]-2-fluoro-phenol | C11H9BrFNOS | D | 302.16 | 302.5 |

| | | | | | | |
|---|---|---|---|---|---|---|
| 1214 | 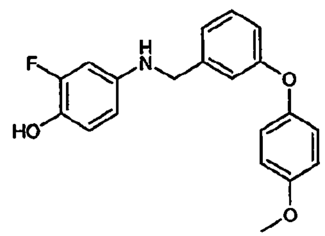 | 2-Fluoro-4-[3-(4-methoxy-phenoxy)-benzylamino]-phenol | C20H18FNO3 | D | 339.36 | 339.8 |
| 1215 | 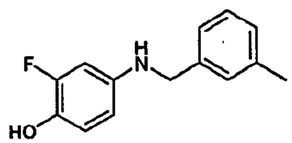 | 2-Fluoro-4-(3-methyl-benzylamino)-phenol | C14H14FNO | D | 231.27 | 231.4 |
| 1216 | 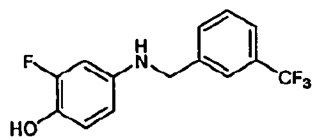 | 2-Fluoro-4-(3-trifluoromethyl-benzylamino)-phenol | C14H11F4NO | D | 285.24 | 285.5 |
| 1217 | 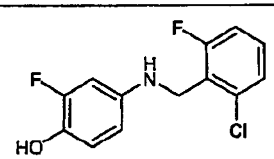 | 2-Fluoro-4-(2-chloro-6-fluoro-benzylamino)-phenol | C13H10ClF2NO | D | 269.67 | 270.1 |
| 1218 | 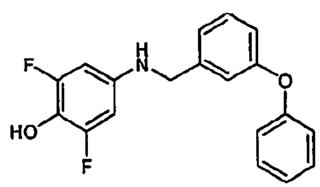 | 2,6-Difluoro-4-(3-phenoxy-benzylamino)-phenol | C19H15F2NO2 | D | 327.32 | 327.6 |

| | | | | | | |
|---|---|---|---|---|---|---|
| 1219 | 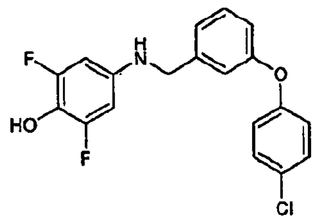 | 2,6-Difluoro-4-[3-(4-chloro-phenoxy)-benzylamino]-phenol | C19H14ClF2NO2 | D | 361.76 | 362.0 |
| 1220 | 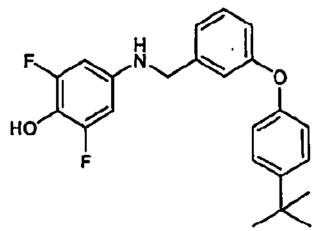 | 4-[3-(4-tert-Butyl-phenoxy)-benzylamino]-2,6-difluoro-phenol | C23H23F2NO2 | D | 383.43 | 383.8 |
| 1221 | 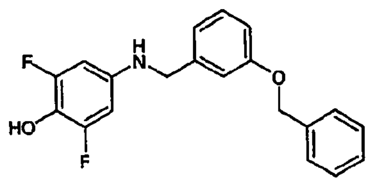 | 4-(3-Benzyloxy-benzylamino)-2,6-difluoro-phenol | C20H17F2NO2 | D | 341.35 | 341.7 |
| 1222 | 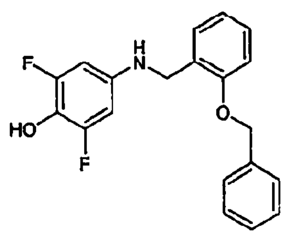 | 4-(2-Benzyloxy-benzylamino)-2,6-difluoro-phenol | C20H17F2NO2 | D | 341.35 | 341.7 |

| | | | | | | |
|---|---|---|---|---|---|---|
| 1223 | 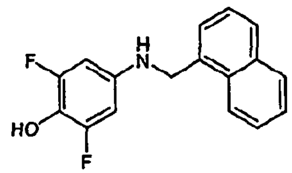 | 2,6-Difluoro-4-[(naphthalen-1-ylmethyl)-amino]-phenol | C17H13F2NO | D | 285.29 | 285.6 |
| 1224 | 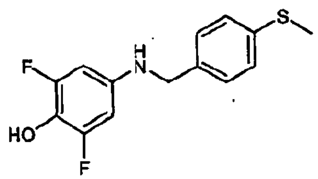 | 2,6-Difluoro-4-(4-methylsulfanyl-benzylamino)-phenol | C14H13F2NOS | D | 281.32 | 281.6 |
| 1225 | 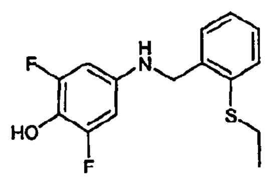 | 2,6-Difluoro-4-(2-ethylsulfanyl-benzylamino)-phenol | C15H15F2NOS | D | 295.35 | 295.7 |
| 1226 | 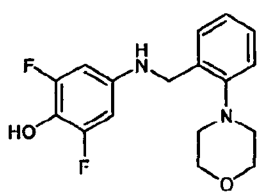 | 2,6-Difluoro-4-(2-morpholin-4-yl-benzylamino)-phenol | C17H18F2N2O2 | D | 320.33 | 320.8 |
| 1227 | 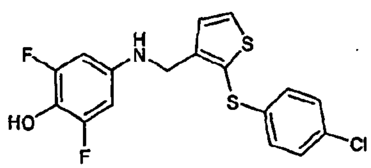 | 2,6-Difluoro-4-{[2-(4-chloro-phenylsulfanyl)-thiophen-3-ylmethyl]-amino}-phenol | C17H12ClF2NOS2 | D | 383.86 | 384.2 |

| | | | | | | |
|---|---|---|---|---|---|---|
| 1228 | 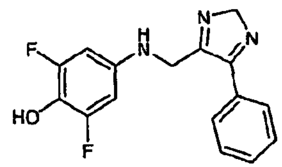 | 2,6-Difluoro-4-[(5-phenyl-2H-imidazol-4-ylmethyl)-amino]-phenol | C16H13F2N3O | D | 301.29 | 301.6 |
| 1229 | 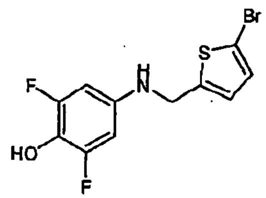 | 4-[(5-Bromo-thiophen-2-ylmethyl)-amino]-2,6-difluoro-phenol | C11H8BrF2NOS | D | 320.15 | 320.4 |
| 1230 | 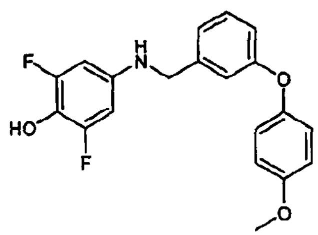 | 2,6-Difluoro-4-[3-(4-methoxy-phenoxy)-benzylamino]-phenol | C20H17F2NO3 | D | 357.35 | 357.6 |
| 1231 | 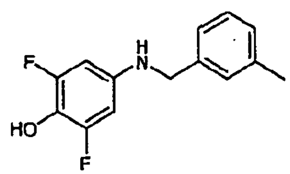 | 2,6-Difluoro-4-(3-methyl-benzylamino)-phenol | C14H13F2NO | D | 249.26 | 249.6 |
| 1232 | 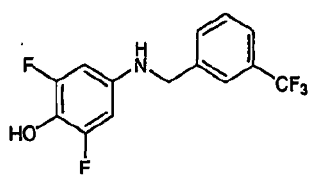 | 2,6-Difluoro-4-(3-trifluoromethyl-benzylamino)-phenol | C14H10F5NO | D | 303.23 | 303.7 |

| | | | | | | |
|---|---|---|---|---|---|---|
| 1233 | 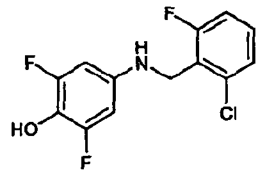 | 2,6-Difluoro-4-(2-chloro-6-fluoro-benzylamino)-phenol | C13H9ClF3NO | D | 287.66 | 288.1 |
| 1234 | 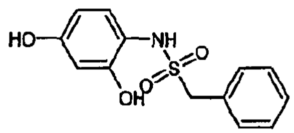 | N-(2,4-Dihydroxy-phenyl)-C-phenyl-methanesulfonamide | C13H13NO4S | B | 279.31 | 279.8 |
| 1235 | 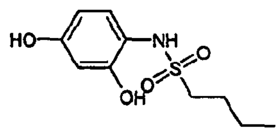 | Butane-1-sulfonic acid (2,4-dihydroxy-phenyl)-amide | C10H15NO4S | B | 245.30 | 245.6 |
| 1236 | 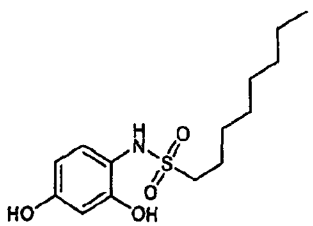 | Octane-1-sulfonic acid (2,4-dihydroxy-phenyl)-amide | C14H23NO4S | B | 301.40 | 301.8 |
| 1237 | 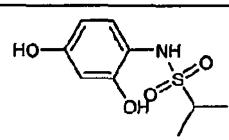 | Propane-2-sulfonic acid (2,4-dihydroxy-phenyl)-amide | C9H13NO4S | B | 231.27 | 231.7 |

| | | | | | | |
|---|---|---|---|---|---|---|
| 1238 | 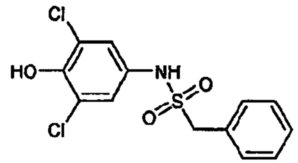 | N-(3,5-Dichloro-4-hydroxy-phenyl)-C-phenyl-methanesulfonamide | C13H11Cl2NO3S | B | 332.20 | 332.4 |
| 1239 | 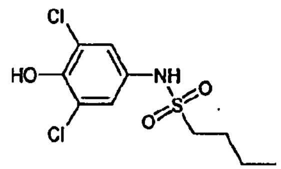 | Butane-1-sulfonic acid (3,5-dichloro-4-hydroxy-phenyl)-amide | C10H13Cl2NO3S | B | 298.19 | 298.5 |
| 1240 | 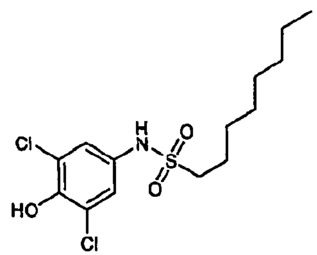 | Octane-1-sulfonic acid (3,5-dichloro-4-hydroxy-phenyl)-amide | C14H21Cl2NO3S | B | 354.29 | 354.6 |
| 1241 | 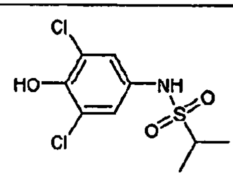 | Propane-2-sulfonic acid (3,5-dichloro-4-hydroxy-phenyl)-amide | C9H11Cl2NO3S | B | 284.16 | 284.5 |
| 1242 | 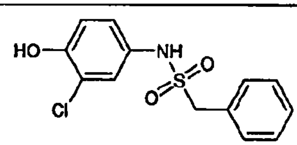 | N-(3-Chloro-4-hydroxy-phenyl)-C-phenyl-methanesulfonamide | C13H12ClNO3S | B | 297.76 | 297.9 |

| | | | | | | |
|---|---|---|---|---|---|---|
| 1243 | 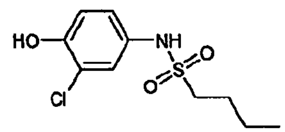 | Butane-1-sulfonic acid (3-chloro-4-hydroxy-phenyl)-amide | C10H14ClNO3S | B | 263.74 | 264.0 |
| 1244 | 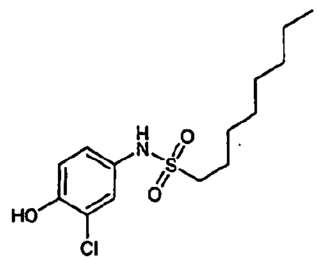 | Octane-1-sulfonic acid (3-chloro-4-hydroxy-phenyl)-amide | C14H22ClNO3S | B | 319.85 | 320.3 |
| 1245 | 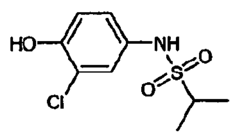 | Propane-2-sulfonic acid (3-chloro-4-hydroxy-phenyl)-amide | C9H12ClNO3S | B | 249.71 | 250.4 |
| 1246 | 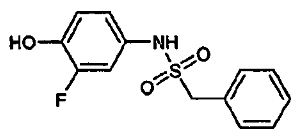 | N-(3-Fluoro-4-hydroxy-phenyl)-C-phenyl-methanesulfonamide | C13H12FNO3S | B | 281.30 | 281.8 |
| 1247 | 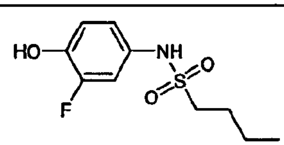 | Butane-1-sulfonic acid (3-fluoro-4-hydroxy-phenyl)-amide | C10H14FNO3S | B | 247.29 | 247.8 |

| | | | | | | |
|---|---|---|---|---|---|---|
| 1248 | 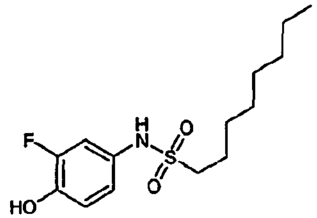 | Octane-1-sulfonic acid (3-fluoro-4-hydroxy-phenyl)-amide | C14H22FNO3S | B | 303.39 | 303.8 |
| 1249 | 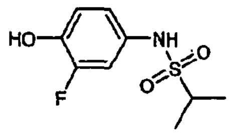 | Propane-2-sulfonic acid (3-fluoro-4-hydroxy-phenyl)-amide | C9H12FNO3S | B | 233.26 | 233.7 |
| 1250 | 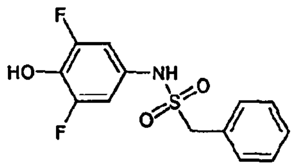 | N-(3,5-Difluoro-4-hydroxy-phenyl)-C-phenyl-methanesulfonamide | C13H11F2NO3S | B | 299.29 | 300.8 |
| 1251 | 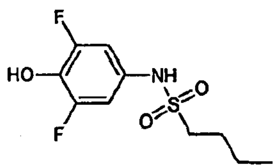 | Butane-1-sulfonic acid (3,5-difluoro-4-hydroxy-phenyl)-amide | C10H13F2NO3S | B | 265.28 | 265.7 |
| 1252 | 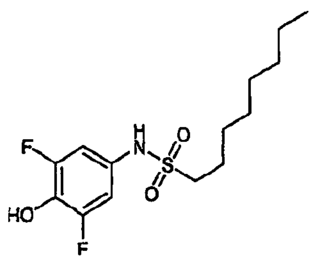 | Octane-1-sulfonic acid (3,5-difluoro-4-hydroxy-phenyl)-amide | C14H21F2NO3S | B | 321.38 | 321.8 |

| | | | | | | |
|---|---|---|---|---|---|---|
| 1253 | 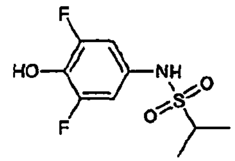 | Propane-2-sulfonic acid (3,5-difluoro-4-hydroxy-phenyl)-amide | C9H11F2NO3S | B | 251.25 | 251.6 |
| 1254 | 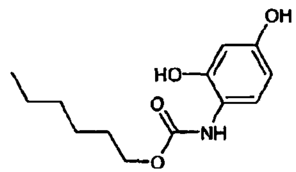 | (2,4-Dihydroxy-phenyl)-carbamic acid hexyl ester | C13H19NO4 | F, G, H | 253.29 | 253.7 |
| 1255 | 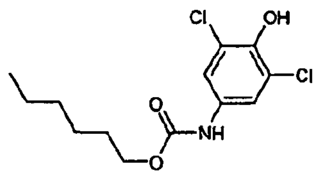 | (3,5-Dichloro-4-hydroxy-phenyl)-carbamic acid hexyl ester | C13H17Cl2NO3 | F, G, H | 306.18 | 306.4 |
| 1256 | 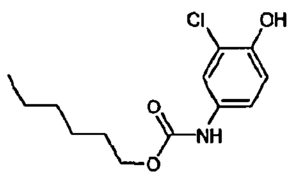 | (3-Chloro-4-hydroxy-phenyl)-carbamic acid hexyl ester | C13H18ClNO3 | F, G, H | 271.74 | 272.1 |
| 1257 | 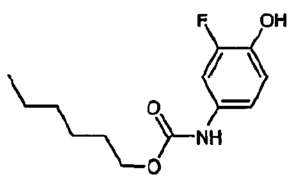 | (3-Fluoro-4-hydroxy-phenyl)-carbamic acid hexyl ester | C13H18FNO3 | F, G, H | 255.29 | 255.6 |

| | | | | | | |
|---|---|---|---|---|---|---|
| 1258 | 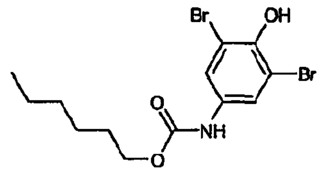 | (3,5-Dibromo-4-hydroxy-phenyl)-carbamic acid hexyl ester | C13H17Br2NO3 | F, G, H | 395.09 | 395.3 |
| 1259 | 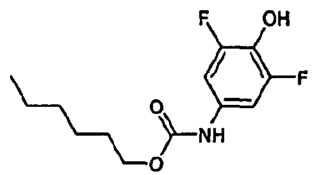 | (3,5-Difluoro-4-hydroxy-phenyl)-carbamic acid hexyl ester | C13H17F2NO3 | F, G, H | 273.28 | 273.6 |
| 1260 | 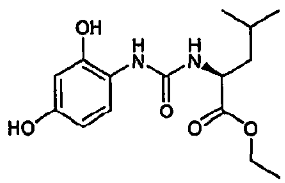 | -[3-(2,4-Dihydroxy-phenyl)-ureido]-4-methyl-pentanoic acid ethyl ester | C15H22N2O5 | A | 310.35 | 310.7 |
| 1261 | 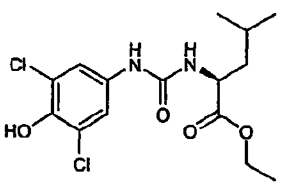 | 2-[3-(3,5-Dichloro-4-hydroxy-phenyl)-ureido]-4-methyl-pentanoic acid ethyl ester | C15H20Cl2N2O4 | A | 363.24 | 363.6 |
| 1262 | 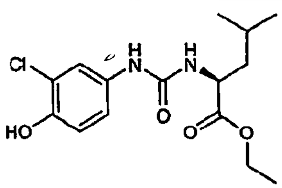 | 2-[3-(3-Chloro-4-hydroxy-phenyl)-ureido]-4-methyl-pentanoic acid ethyl ester | C15H21ClN2O4 | A | 328.79 | 329.1 |

| | | | | | | |
|---|---|---|---|---|---|---|
| 1263 | 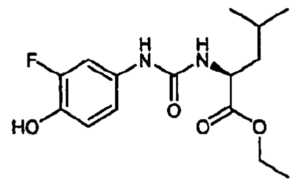 | 2-[3-(3-Fluoro-4-hydroxy-phenyl)-ureido]-4-methyl-pentanoic acid ethyl ester | C15H21FN2O4 | A | 312.34 | 312.8 |
| 1264 | 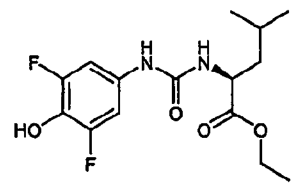 | 2-[3-(3,5-Difluoro-4-hydroxy-4-phenyl)-ureido]-4-methyl-pentanoic acid ethyl ester | C15H20F2N2O4 | A | 330.33 | 330.8 |
| 1265 | 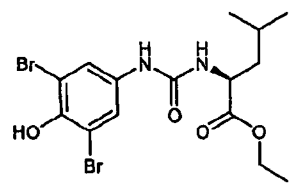 | 2-[3-(3,5-Dibromo-4-hydroxy-4-methyl-phenyl)-ureido]-4-methyl-pentanoic acid ethyl ester | C15H20Br2N2O4 | A | 452.14 | 452.3 |
| 1266 | 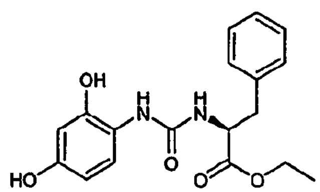 | 2-[3-(2,4-Dihydroxy-phenyl)-ureido]-3-phenyl-propionic acid ethyl ester | C18H20N2O5 | A | 344.36 | 344.8 |
| 1267 | 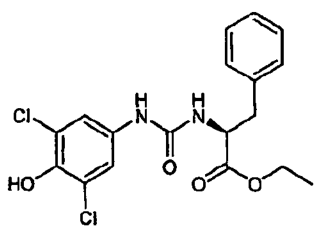 | 2-[3-(3,5-Dichloro-4-hydroxy-phenyl)-ureido]-3-phenyl-propionic acid ethyl ester | C18H18Cl2N2O4 | A | 397.25 | 397.6 |

| | | | | | | |
|---|---|---|---|---|---|---|
| 1268 | 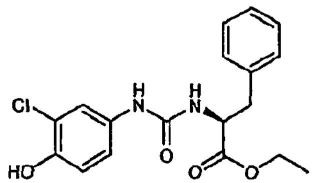 | 2-[3-(3-Chloro-4-hydroxy-phenyl)-ureido]-3-phenyl-propionic acid ethyl ester | C18H19ClN2O4 | A | 362.81 | 363.0 |
| 1269 | 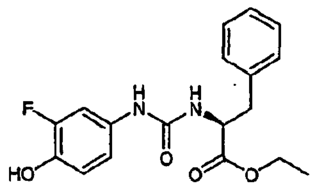 | 2-[3-(3-Fluoro-4-hydroxy-phenyl)-ureido]-3-phenyl-propionic acid ethyl ester | C18H19FN2O4 | A | 346.35 | 346.7 |
| 1270 | 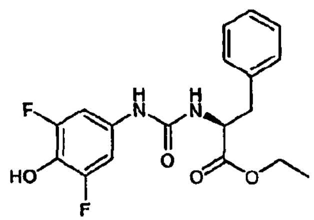 | 2-[3-(3,5-Difluoro-4-hydroxy-phenyl)-ureido]-3-phenyl-propionic acid ethyl ester | C18H18F2N2O4 | A | 364.34 | 364.8 |
| 1271 | 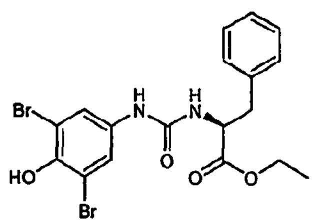 | 2-[3-(3,5-Dibromo-3-fluoro-4-hydroxy-phenyl)-ureido]-3-phenyl-propionic acid ethyl ester | C18H18Br2N2O4 | A | 486.15 | 486.2 |

| | | | | | | |
|---|---|---|---|---|---|---|
| 1272 | 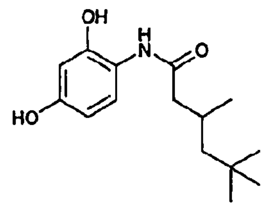 | 3,5,5-Trimethyl-hexanoic acid (2,4-dihydroxy-phenyl)-amide | C15H23NO3 | C | 265.35 | 265.7 |
| 1273 | 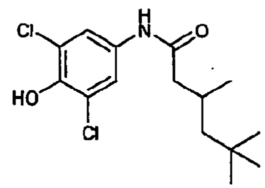 | 3,5,5-Trimethyl-hexanoic acid (3,5-dichloro-4-hydroxy-phenyl)-amide | C15H21Cl2NO2 | C | 318.24 | 318.5 |
| 1274 | 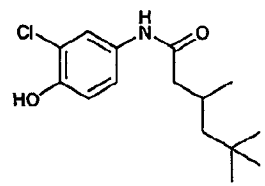 | 3,5,5-Trimethyl-hexanoic acid (3-chloro-4-hydroxy-phenyl)-amide | C15H22ClNO2 | C | 283.79 | 284.0 |
| 1275 | 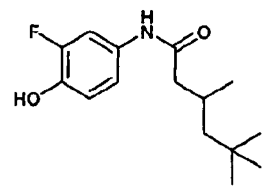 | 3,5,5-Trimethyl-hexanoic acid (3-fluoro-4-hydroxy-phenyl)-amide | C15H21FNO2 | C | 267.34 | 267.7 |
| 1276 | 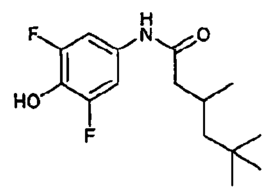 | 3,5,5-Trimethyl-hexanoic acid (3,5-difluoro-4-hydroxy-phenyl)-amide | C15H21F2NO2 | C | 285.33 | 285.7 |

| | | | | | | |
|---|---|---|---|---|---|---|
| 1277 | 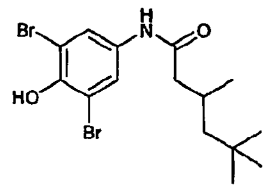 | 3,5,5-Trimethyl-hexanoic acid (3,5-dibromo-4-hydroxy-phenyl)-amide | C15H21Br2NO2 | C | 407.14 | 407.3 |
| 1278 | 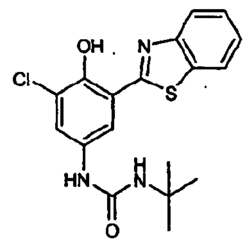 | 1-(3-Benzothiazol-2-yl-5-chloro-4-hydroxy-phenyl)-3-tert-butyl-urea | C18H18ClN3O2S | K followed by A | 375.9 | 376.1 |
| 1279 | 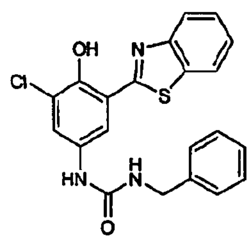 | 1-(3-Benzothiazol-2-yl-5-chloro-4-hydroxy-phenyl)-3-benzyl-urea | C21H16ClN3O2S | K followed by A | 409.9 | 410.1 |
| 1280 | 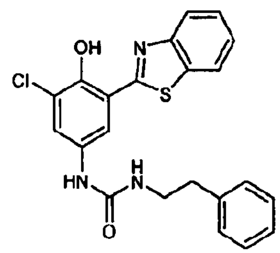 | 1-(3-Benzothiazol-2-yl-5-chloro-4-hydroxy-phenyl)-3-phenethyl-urea | C22H18ClN3O2S | K followed by A | 423.9 | 424.1 |

| | | | | | | |
|---|---|---|---|---|---|---|
| 1281 | 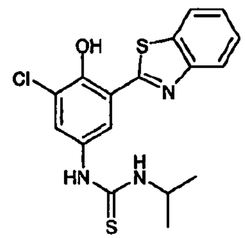 | 1-(3-Benzothiazol-2-yl-5-chloro-4-hydroxy-phenyl)-3-isopropyl-thiourea | C17H16ClN3OS2 | K followed by I | 377.9 | 378.1 |
| 1282 | 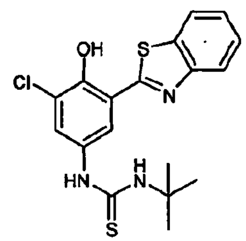 | 1-(3-Benzothiazol-2-yl-5-chloro-4-hydroxy-phenyl)-3-tert-butyl-thiourea | C18H18ClN3OS2 | K followed by I | 391.9 | 392.1 |
| 1283 | 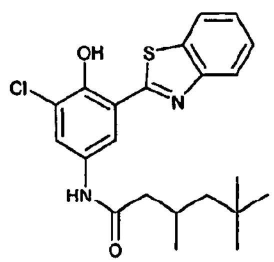 | 3,5,5-Trimethyl-hexanoic acid (3-benzothiazol-2-yl-5-chloro-4-hydroxy-phenyl)-amide | C22H25ClN2O2S | K followed by C | 417.0 | 417.2 |

| | | | | | | |
|---|---|---|---|---|---|---|
| 1284 | 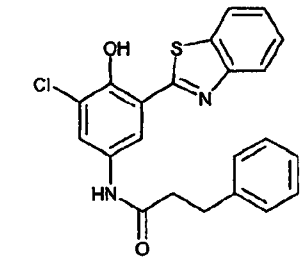 | N-(3-Benzothiazol-2-yl-5-chloro-4-hydroxy-phenyl)-3-phenyl-propionamide | C22H17ClN2O2S | K followed by C | 408.9 | 409.2 |
| 1285 | 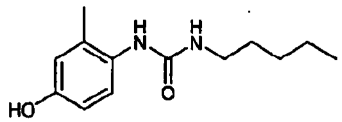 | 1-(4-Hydroxy-2-methyl-phenyl)-3-pentyl-urea | C13H20N2O2 | A | 263.31 | 256.9 |
| 1286 | 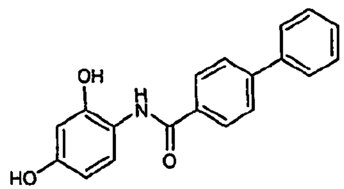 | Biphenyl-4-carboxylic acid (2,4-dihydroxy-phenyl)-amide | C19H15NO3 | C, E | 305.33 | 305.6 |
| 1287 | 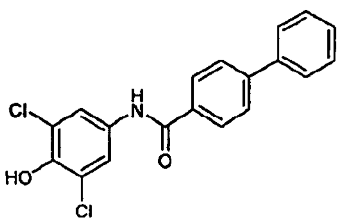 | Biphenyl-4-carboxylic acid (3,5-dichloro-4-hydroxy-phenyl)-amide | C20H15Cl2NO2 | C, E | 358.22 | 358.5 |
| 1288 | 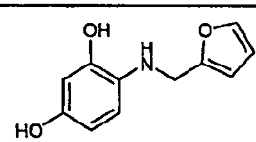 | 4-[(Furan-2-ylmethyl)-amino]-benzene-1,3-diol | C11H11NO3 | D | 205.21 | 205.7 |

| | | | | | | |
|---|---|---|---|---|---|---|
| 1289 | 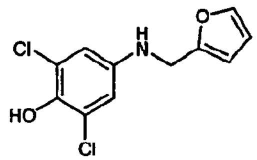 | 2,6-Dichloro-4-[(furan-2-ylmethyl)-amino]-phenol | C11H9Cl2NO2 | D | 258.10 | 258.4 |
| 1290 | 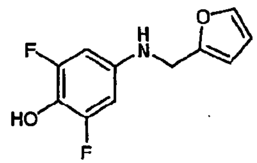 | 2,6-Difluoro-4-[(furan-2-ylmethyl)-amino]-phenol | C11H9F2NO2 | D | 225.19 | 225.7 |
| 1291 | 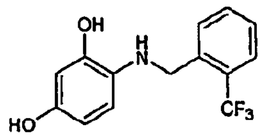 | 4-(2-Trifluoromethyl-benzylamino)-benzene-1,3-diol | C14H12F3NO2 | D | 283.25 | 283.5 |
| 1292 | 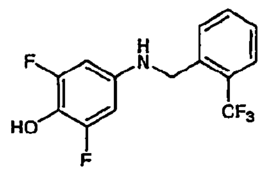 | 2,6-Difluoro-4-(2-trifluoromethyl-benzylamino)-phenol | C14H10F5NO | D | 303.23 | 303.6 |
| 1293 | 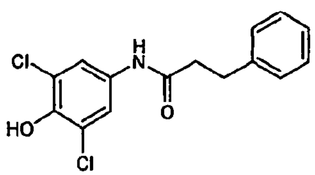 | N-(3,5-Dichloro-4-hydroxy-phenyl)-3-phenyl-propionamide | C15H13Cl2NO2 | C, E | 310.18 | 310.4 |

| | | | | | | |
|---|---|---|---|---|---|---|
| 1294 | 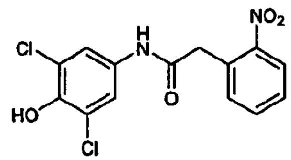 | N-(3,5-Dichloro-4-hydroxy-phenyl)-2-(2-nitro-phenyl)-acetamide | C14H10Cl2N2O4 | C, E | 341.15 | 341.3 |
| 1295 | 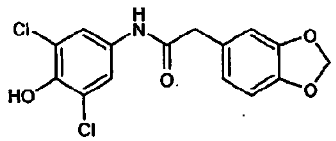 | 2-Benzo[1,3]dioxol-5-yl-N-(3,5-dichloro-4-hydroxy-phenyl)-acetamide | C15H11Cl2NO4 | C, E | 340.16 | 340.3 |
| 1296 | 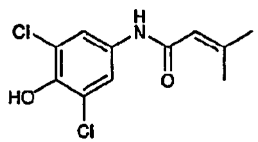 | 3-Methyl-but-2-enoic acid (3,5-dichloro-4-hydroxy-phenyl)-amide | C11H11Cl2NO2 | C | 260.12 | 260.2 |
| 1297 | 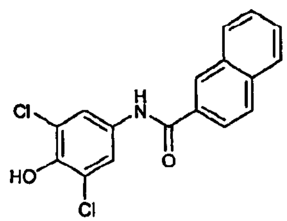 | Naphthalene-2-carboxylic acid (3,5-dichloro-4-hydroxy-phenyl)-amide | C17H11Cl2NO2 | C, E | 332.18 | 332.4 |
| 1298 | 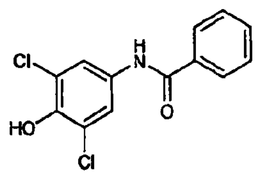 | N-(3,5-Dichloro-4-hydroxy-phenyl)-benzamide | C13H9Cl2NO2 | C, E | 282.12 | 282.3 |

| | | | | | | |
|---|---|---|---|---|---|---|
| 1299 | 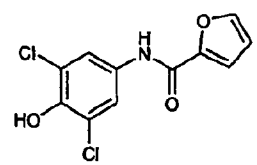 | Furan-2-carboxylic acid (3,5-dichloro-4-hydroxy-phenyl)-amide | C11H7Cl2NO3 | C, E | 272.08 | 272.3 |
| 1300 | 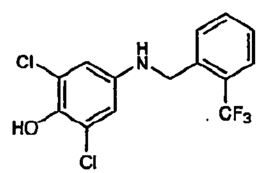 | 2,6-Dichloro-4-(2-trifluoromethyl-benzylamino)-phenol | C14H10Cl2F3NO | D | 336.14 | 336.5 |

or a pharmaceutical acceptable salt or prodrug thereof.

Even more preferred compounds according to the present invention are those mentioned in any of the tables herein and those further disclosed and/or characterized in the examples.

As used herein, each of the following terms, used alone or in conjunction with other terms, are preferably used in the following meaning (except where noted to the contrary):

The term "alkyl" refers to a saturated aliphatic radical containing from one to ten carbon atoms or a mono- or polyunsaturated aliphatic hydrocarbon radical containing from two to twelve carbon atoms, containing at least one double and triple bound, respectively. "Alkyl" refers to both branched and unbranched alkyl groups. Preferred alkyl groups are straight chain alkyl groups containing from one to eight carbon atoms. More preferred alkyl groups are straight chain alkyl groups containing from one to six carbon atoms and branched alkyl groups containing from three to six carbon atoms. It should be understood that any combination term using an "alk" or "alkyl" prefix refers to analogs according to the above definition of "alkyl". For example, terms such as "alkoxy", "alkylthio" refer to alkyl group linked to a second group via an oxygen or sulfur atom. "Alkanoyl" refers to an alkyl group linked to a carboxyl group (C=O). "Substituted alkyl" refers to alkyl groups straight or branched further bearing one or more substituents. One substituent also means mono-substituted and more substitutents mean poly-substituted. It should be understood that any combination term using a "substituted alkyl" prefix refers to analogs according to the above definition of "substituted alkyl". For example, a term such as "substituted alkylaryl" refers to substituted alkyl group linked to an aryl group.

The term "cycloalkyl" refers to the cyclic analog of an alkyl group, as defined above, optionally unsaturated and/or substituted. Preferred cycloalkyl groups are saturated cycloalkyl groups, more particularly those containing from three to eight carbon atoms, and even more preferably three to six carbon atoms. "Substituted cycloalkyl" refers to cycloalkyl groups further bearing one or more substituents. "Mono-unsaturated cycloalkyl," refers to cycloalkyl containing one double bond or one triple bond. "Poly-unsaturated cycloalkyl" refers to cycloalkyl containing at least two double bonds or two triple bonds or a combination of at least one double bond and one triple bond.

The term "alkenyl" refers to an unsaturated hydrocarbon group containing at least one carbon-carbon double bond, including straight-chain, branched-chain, and cyclic groups. Preferred alkenyl groups have one to twelve carbons. More preferred alkenyl groups have one to six carbons. "Substituted alkenyl" refers to alkenyl groups further bearing one or more substitutents.

The term "cycloalkenyl" refers to the cyclic analog of an alkenyl group, as defined above, optionally substituted. Preferred cycloalkenyl groups are containing from four to eight carbon atoms. "Substituted cycloalkenyl" refers to cycloalkenyl groups further bearing one or more substituents. "Mono-unsaturated cycloalkenyl" refers to cycloalkenyl containing one double bond. "Poly-unsaturated cycloalkenyl" refers to cycloalkenyl containing at least two double bonds.

The term "alkynyl" refers to an unsaturated hydrocarbon group containing at least one carbon-carbon triple bond, including straight-chain, branched-chain, and cyclic groups. Preferred alkynyl groups have one to twelve carbons. More preferred alkynyl groups have one to six carbons. "Substituted alkynyl" refers to alkynyl groups further bearing one or more substitutents.

The term "aryl" refers to aromatic groups having in the range of 6 to 14 carbon atoms and "substituted aryl" refers to aryl groups further bearing one or more substituents. It should be understood that any combination term using an "ar" or "aryl" prefix refers to analogs according to the above definition of "aryl". For example, a term such as "aryloxy" refers to aryl group linked to a second group via an oxygen.

Each of the above defined "alkyl", "cycloalkyl", and "aryl" shall be understood to include their halogenated analogs, whereby the halogenated analogs may comprise one or several halogen atoms. The halogenated analogs thus comprise any halogen radical as defined in the following.

The term "halo" refers to a halogen radical selected from fluoro, chloro, bromo, iodo. Preferred halo groups are fluoro, chloro and bromo.

The term "heteroaryl" refers to a stable 5 to 8 membered, preferably 5 or 6 membered monocyclic or 8 to 11 membered bicyclic aromatic heterocycle radical. Each heterocycle consists of carbon atoms and from 1 to 4 heteroatoms selected from the group consisting of nitrogen, oxygen, sulfur. The heterocycle may be attached by any atom of the cycle, which preferably results in the creation of a stable structure. Preferred heteroaryl radicals as used herein include, for example, furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzthiazolyl, benzoxazolyl, purinyl, quinolizinyl, quinolinyl, isoquinolinyl, cinnolinyl, phthalazinyl, quinazolinyl, quinoxalinyl, naphthridinyl, pteridinyl, carbazolyl, acridinyl, phenazinyl, phenothiazinyl and phenoxazinyl. "Substituted heteroaryl" refers to heteroaryl groups further bearing one or more substituents.

The term "heterocyclyl" refers to a stable 5 to 8 membered, preferably 5 or 6 membered monocyclic or 8 to 11 membered bicyclic heterocycle radical which may be either saturated or unsaturated, and is non-aromatic. Each heterocycle consists of carbon atom(s) and from 1 to 4 heteroatoms selected from the group consisting of nitrogen, oxygen and sulfur. The heterocycle may be attached by any atom of the cycle, which preferably results in the creation of a stable structure. Preferred heterocycle radicals as used herein include, for example, pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione. "Mono-unsaturated heterocyclyl" refers to heterocyclyl containing one double bond or one triple bond. "Poly-unsaturated heterocyclyl" refers to heterocyclyl containing at least two double bonds or two triple bonds or a combination of at least one double bond and one triple bond.

"Substituted heterocyclyl" refers to heterocyclyl groups further bearing one or more substituents.

The terms "heterocyclyl", "heteroaryl" and "aryl", when associated with another moiety, unless otherwise specified, shall have the same meaning as given above. For example, "aroyl" refers to phenyl or naphthyl linked to a carbonyl group (C=O).

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydranaphthyl.

As used herein above and throughout this application, "nitrogen" or "N"and "sulfur" or "S" include any oxidized form of nitrogen and sulfur and the quaternized form of any basic nitrogen sulfoxide, sulfone, nitrone, N-oxide.

As used herein a wording defining the limits of a range of length such as e. g. "from 1 to 5" means any integer from 1 to 5, i. e. 1, 2, 3, 4 and 5. In other words, any range defined by two integers explicitly mentioned is meant to comprise any integer defining said limits and any integer comprised in said range.

As used herein the term substituted shall mean that one or more H atom of the group or compound which is substituted, is replaced by a different atom, a group of atoms, a molecule or a molecule moiety. Such atom, group of atoms, molecule or molecule moiety is also referred to herein as substituent.

The substituent can be selected from the group comprising hydroxy, alkoxy, mercapto, cycloalkyl, heterocyclic, aryl, heteroaryl, aryloxy, halogen, trifluoromethyl, difluoromethyl, cyano, nitrone, amino, amido, -C(O)H, acyl, oxyacyl, carboxyl, carbamate, sulfonyl, sulphonamide and sulfuryl. Any of the substituents may be substituted itself by any of the aforementioned substituents. This applies preferably to cycloalkyl, heterocylic, aryl, heteroaryl and aryloxy. It is also preferred that alkoxy and mercapto are those of a lower alkyl group. It is to be acknowledged that any of the definition provided herein also applies to any substituent.

A substituent can also be any of $R^a$, $R^b$, $R^c$, $R^d$, $R^e$, $R^f$, and $R^g$ and/or any of $R_1$ to $R_{21}$. It is also within the present invention that any substitutent may in turn be substituted by a substituent. A group, structure, moiety or the like which is substituted may comprise several substituents which may either be different or the same.

As used herein =T can mean in any embodiment of the various aspects of the present invention that with =T is selected from electron withdrawing groups, whereby preferably the electron withdrawing groups are selected from =O, =N-$R^e$, =N-CN, =N-$NO_2$ and =CH-$NO_2$, and =S,

It is within the present invention that any thiourea moieties and derivates therefrom, particularly those described herein, can, in principle be replaced by a cyanoguanidine moiety or residue and respective derivates therefrom as described in J. Med. Chem 1977, 20, 901 - 906. In Addition to being weakly basic cyanoguanidine and thiourea are also weakly acidic and both are therefore neutral and weakly amphoteric compounds. Cyanoguanidine is also similar to thiourea in its geometry since both are planar structures with almost identical C-N bond lengths and bond angles. Another property common to thioureas and cyanoguanidines is conformational isomerism resulting from restricted C-N bond rotation. Cyanoguanidine and thiourea are similar in their hydrophilicity and hydrogen-bonding properties; they have comparably low octanol-water partition coefficients (P) and are both reasonably soluble in water.

As used herein in connection with an embodiment of the various aspects of the present invention the term "wherein $R_1$ and $R_2$, $R_2$ and $R_3$, $R_3$ and $R_4$ , $R_1$ and $R_3$, $R_1$ and $R_4$, or $R_2$ and $R_4$ may be linked so as to form a ring comprising 4 to 12 members, preferably 5 to 10 members" shall mean that any of the two residues R, such as, for example, $R_1$ and $R_2$ or $R_2$ and $R_3$, are linked to each through a covalent bond, a non-covalent bond or any combination thereof. The formation of the ring may be the result of one or several of this kind of bonds. It is to be understood that the molecule may comprise one or more of those rings formed by two residues R.

In a preferred embodiment wherein $R_1$ and $R_2$, $R_2$ and $R_3$, $R_3$ and $R_4$ , $R_1$ and $R_3$, $R_1$ and $R_4$, or $R_2$ and $R_4$ linked so as to form a ring comprising 4 to 12 members, preferably 5 to 10 members, more preferably 5 or 6 members, $R_1$, $R_2$, $R_3$ and $R_4$ are each and independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl;

The ring may be cycloalkyl, heterocyclyl, aryl, or heteroaryl. The cycloalkyl or heterocyclyl ring can be mono-unsaturated or poly-unsaturated. The ring can be substituted by one or more substituents as defined herein

As used herein in connection with an embodiment of the various aspects of the present invention the term "each and independently selected from a group" or "are independently from each other selected from the group" refers to two or more atoms, groups, substituents, moieties or whatsoever and describes that the single atom, group etc.

mentioned can be selected from the group. The wording used is a truncation which avoids unnecessary repetition as otherwise for each of the atoms, groups etc. the same group definition would have to be repeated.

As used herein in connection with an embodiment of the various aspects of the present invention the term "each and individually absent" refers to two or more atoms, groups, substituents, moieties or whatsoever and describes that the single atom, group etc. mentioned can be absent regardless whether any of the other atoms, groups etc. mentioned is absent. The wording used is a truncation which avoids unnecessary repetition as otherwise for each of the atoms, groups etc. the fact that it may be absent in an embodiment of the invention would have to be repeated.

In connection with the present invention some groups such as, e.g., $-(CR^aR^b)-$ or $-(CR^fR^g)-$are repeated, i.e. are repeatedly present in a compound according to the present invention. Typically such repetition occurs in such a manner that, e.g., $-(CR^aR^b)-$ is repeated one or several times. In case, e.g., $-(CR^aR^b)-$ is repeated one time which means that there are two consecutive groups of $-(CR^aR^b)-$, these two forms of $-(CR^aR^b)-$ can be either the same or they may be different in a different embodiment which means that either $R^a$ or $R^b$ or both of them are different between said two $-(CR^aR^b)-$ groups. If there are three or more of these groups such as , e.g., $-(CR^aR^b)-$, it is possible that all of them are different or only some or different whereas others are the same in the sense defined above. Any permutation for the arrangement for such identical or different groups is within the present invention.

The same applies to the design and arrangement of the spacer -M1-L1-K-L2-M2-.

In connection with any of the compounds according to the present invention and more particularly in connection with the compounds according to formulae VI to XIII and XIX to XXVI it is to be noted that the formation of a ring structure through E between the cyclic moiety A such as the phenol moiety and the spacer X can occur between the cyclic moiety A such as the phenol moiety and any of the spacer moieties -M1-L1-K-L2-M2-, regardless whether they are of a linear or any of the cyclic structures themselves. It is therefore within the present invention that in case of a repetition of the above described spacer moiety -M1-L1-K-L2-M2- the ring formation can either occur at the -M1-L1-K-L2-M2- moiety which is next or closest to the ring as represented in the respective formulae, or at the or any of the further -M1-L1-K-L2-M2- moieties present in the particular compound.

As used herein in connection with an embodiment of the various aspects of the present invention the term C=T shall represent a C atom having a double bond with T which represents certain atoms, groups, substituents, moieties or the like as further defined herein.

It is within the present invention that the features of the various embodiments of the present invention can be realized either alone or in combination with the features of any other embodiment(s) of the present invention. Thus any combination of an/the individual feature or the combination of features of an embodiment of the present invention with an/the individual feature(s) or the combination of features of any other embodiment(s), either alone or in combination with other embodiments, shall be disclosed by the present specification.

As used herein in connection with an embodiment of the various aspects of the present invention the term referring to a group, substituent, moiety, spacer or the like specifying that it "can be inserted in any orientation into any of the preceding formulae" means that the group etc. can be attached to another atom, group, substitutent, moiety spacer or the like of any of the compounds according to the present invention or any of the formulae disclosed herein via any of its ends an in particular through any of the atoms arranged at the ends of said group, substituent, moiety, spacer or the like.

In a further aspect the present invention is related to the use of a compound according to any of the aspects of the present invention as an inhibitor to or for a rotamase.

In an embodiment the rotamase regulates a part of the cell cycle.

In a preferred embodiment the rotamase regulates a part of the cell cycle, whereby preferably the part of the cell cycle is mitosis.

In an even more preferred embodiment the rotamase is a mammalian rotamase, preferably a human rotamase, more preferably hPin1.

In a further aspect the present invention is related to the use of the compounds according to the present invention as a pharmaceutical or in a pharmaceutical composition or for the manufacture of such pharmaceutical composition which is preferably for the prophylaxis and/or treatment of a disease, whereby the disease involves a rotamase, whereby the rotamase is a mammalian rotamase, preferably a human rotamase, more preferably hPin1.

In connection with the further aspect of the present invention related to the use of any of the aforementioned compounds according to the present invention as an inhibitor to rotamases the following will be acknowledged by the one skilled in the art. In view of the characteristics of the compounds according to the present invention to be active as an inhibitor of (a) rotamase(s), it is sufficient that the respective compound is at least suitable to inhibit at least one rotamase. The compounds according to the present invention which may be used as inhibitors, are also referred to as rotamase inhibitors herein.

Rotamases as such are known in the art and, for example, described in the introductory part of this specification which is incorporated by reference. Rotamases as used herein shall mean cyclophilins, FK-506 binding proteins and the rotamases of the Pin1/parvulin class. The Pinl/parvulin class includes Pins 1 to 3, PinL/parvulin, dodo, and Es1/Pft1.

Suitable assays to determine whether a compound is suitable to inhibit a rotamase are known to the one skilled in the art and also described in the present examples. Basically, a rotamase is provided the activity of which or non-activity of which may be determined. A candidate inhibitor, i. e. a compound which is to be tested whether it is active as an inhibitor to rotamase, is added to the rotamase and tested whether upon the addition and/or influence of the candidate inhibitor the activity of the rotamase is changed relative to the activity without candidate rotamase inhibitor. If the rotamase activity is decreased by the candidate rotamase inhibitor, said candidate rotamase inhibitor is a rotamase inhibitor according to the present invention.

In another aspect of the present invention the compounds according to the present invention may be used in a method for inhibiting a rotamase. In such case a rotamase is provided and a candidate rotamase inhibitor is added thereto whereupon the activity of rotamase is decreased. Optionally, such decrease in rotamase activity is measured. The techniques used theretofore are basically the same as outlined in connection with the use of the compounds according to the present invention as rotamase inhibitors.

The compounds according to the present invention are preferably reversible rotamase inhibitors.

By "reversible" herein is meant that the inhibitor binds non-covalently to the enzyme, and is to be distinguished from irreversible inhibition. See Walsh, Enzymatic Reaction Mechanisms, Freeman & Co., N.Y., 1979. "Reversible" in this context is a term understood by those skilled in the art. Preferably the rotamase inhibitors according to the present invention are competitive inhibitors, that is, they compete with substrate in binding reversibly to the enzyme, with the binding of inhibitor and substrate being mutually exclusive.

In a preferred embodiment of the compounds according to the present invention being active as a rotamase inhibitor, the dissociation constant for inhibition of a rotamase with the inhibitor, generally referred to and characterized by those in the art as $K_i$, is at most about 100μ M. By the term "binding constant" or "dissociation constant" or grammatical equivalents herein is meant the equilibrium dissociation constant for the reversible association of inhibitor with enzyme. The dissociation constants are defined and determined as described below. The determination of dissociation constants is known in the art. For example, for reversible inhibition reactions such as those of the present invention, the reaction scheme is as follows:

$$E{+}I \underset{k_2}{\overset{k_1}{\rightleftharpoons}} E{*}I \quad \text{(Equation 1)}$$

The enzyme (E) and the inhibitor (I) combine to give an enzyme-inhibitor complex (E*I). This step is assumed to be rapid and reversible, with no chemical changes taking place; the enzyme and the inhibitor are held together by non-covalent forces. In this reaction, $k_1$ is the second order rate constant for the formation of the E*I reversible complex. $k_2$ is the first order rate constant for the dissociation of the reversible E*I complex. In this reaction, $Ki = k_2/k_1$.

The measurement of the equilibrium constant $K_i$ proceeds according to techniques well known in the art, as described in the examples. For example, assays generally use synthetic chromogenic or fluorogenic substrates. The respective $K_i$ values may be estimated using the Dixon plot as described by Irwin Segel in Enzyme Kinetics: Behavior and analysis of rapid equilibrium and steady-state enzyme systems, 1975, Wiley-Interscience Publication, John Wiley & Sons, New York, or for competitive binding inhibitors from the following calculation:

$$1-(v_i/v_o)=[I]/[I]+K_i\ (1+([S]/K_m))) \quad \text{(Equation 2)}$$

wherein $v_o$ is the rate of substrate hydrolysis in the absence of inhibitor, and $v_i$ is the rate in the presence of competitive inhibitor.

It is to be understood that dissociation constants are a particularly useful way of quantifying the efficiency of an enzyme with a particular substrate or inhibitor, and are frequently used in the art as such. If an inhibitor exhibits a very low $K_i$ value, it is an efficient inhibitor. Accordingly, the rotamase inhibitors of the present invention have dissociation constants, $K_i$, of at most about 100 μM. Preferably, the rotamase inhibitors according to the present invention exhibit dissociation constants of at most about 10 μM, more preferably about 1 μM, most preferably of at most about 100 nM.

The rotamase inhibitors of the present invention may be easily screened for their inhibitory effect. The inhibitor is first tested against different classes of rotamases for which the targeting group of the inhibitor was chosen, as outlined above. The activity of rotamases is typically measured by using a protease coupled assay with chromogenic substrates and conformer specific proteases. Basically, upon the conformer specific protease activity the chromogenic substrate

is converted into a compound which has an absorption characteristic which is different from the starting chromogenic substrate and may thus be selectively measured. This reaction is accelerated in presence of the rotamase and decelerated in the presence of rotamase-inhibitors. Alternatively, many rotamases and their corresponding chromogenic substrates are commercially available. Thus, a variety of rotamases are routinely assayed with synthetic chromogenic substrates in the presence and absence of the rotamase inhibitor, to confirm the inhibitory action of the compound, using techniques well known in the art. The effective inhibitors are then subjected to kinetic analysis to calculate the $K_i$ values, and the dissociation constants determined.

If a compound inhibits at least one rotamase, it is a rotamase inhibitor for the purposes of the present invention. Preferred embodiments of the rotamase inhibitors according to the present invention are compounds and inhibitors, respectively, that exhibit the correct kinetic parameters Ki below 100 $\mu$M against the targeted rotamases.

In a further aspect of the present invention any of the compounds used as rotamase inhibitors or as a medicament may be labelled.

By a "labelled rotamase inhibitor" herein is meant a rotamase inhibitor that has at least one element, isotope or chemical compound attached to enable the detection of the rotamase inhibitor or the rotamase inhibitor bound to a rotamase. In general, labels as used herein, fall into three classes: a) isotopic labels, which may be radioactive or heavy isotopes; b) immune labels, which may be antibodies or antigens; and c) colored or fluorescent dyes. The labels may be incorporated into the rotamase inhibitor at any position. Examples of useful labels include $^{14}C$, $^{13}C$, $^{15}N$, $^{3}H$, biotin, and fluorescent labels as are well known in the art.

In a further aspect the compounds according to the present invention, particularly those having rotamase inhibitory activity, may be used for removing, identifying and/or inhibiting contaminating rotamases in a sample.

Therefore, the rotamase inhibitors of the present invention are, for example, added to a sample where the catalytic activity by contaminating rotamases is undesirable. Alternatively, the rotamase inhibitors of the present invention may be bound to a chromatographic support, using techniques well known in the art, to form an affinity chromatography column. A sample containing an undesirable rotamase is run through the column to remove the rotamase. Alternatively, the same methods may be used to identify new rotamases. In doing so, a new rotamase contained in a sample may bind to the rotamase inhibitor bound to the chromatographic support and upon elution, preferably a specific elution, from said chromatographic support, characterized and compared to other rotamase activities with regard to, among others, specificities. The characterization of the rotamase as such is known to the one skilled in the art.

In a further aspect the present invention is related to a pharmaceutical composition comprising a compound according to any of the aspects of the present invention and a pharmaceutically acceptable carrier, diluent or excipient.

In an embodiment the composition comprises a further pharmaceutically active compound, preferably such further pharmaceutically active compound is a chemotherapeutic agent.

In a preferred embodiment of the composition the compound is present as a pharmaceutically acceptable salt or a pharmaceutically active solvate.

In an even more preferred embodiment the pharmaceutically active compound is either alone or in combination with any of the ingredients of the composition present in a multitude of individualized dosages and/or administration forms.

The use of the compounds according to the present invention for the manufacture of a medicament is based on the fact that the compounds according to the present invention are inhibitors of rotamases and rotamases in turn have been identified in both procaryotic and eucaryotic cells such as in bacteria, fungi, insect and mammalian cells. In this cellular environment rotamases are known to have an impact on cell proliferation and mitosis, respectively. Because of this, rotamase inhibitors may be used for the treatment of a wide variety of disorders involving cell cycle regulation, both procaryotic and eucaryotic cell cycle regulation. The term "treatment" as used herein comprises both treatment and prevention of a disease. It also comprises follow-up treatment of a disease. Follow-up treatment is realized upon a treatment of a disease using compounds preferably different from the one according to the present invention. For example, after stimulating the growth of a cell, tissue or the like by the application of a respective compound such as, e. g., erythropoietin, it might be necessary to stop an overshooting reaction of cell proliferation which may be obtained using the compounds according to the present invention.

In a further aspect the present invention is related to the use of the compounds according to the present invention as a medicament and for the manufacture of a medicament, respectively. It is to be understood that any of the compounds according to the present invention can be used for the treatment of or for the manufacture of a medicament for the treatment of any of the diseases disclosed herein, irrespective of the mode of action or the causative agent involved as may be specified herein. Of Course, it may particularly be used for any form of such disease where the particular causative agent is involved. Causative agent as used herein also means any agent which is observed in connection with the particular disease described and such agent is not necessarily causative in the sense that is causes the observed diseases or diseased condition.

In an embodiment the medicament is for the treatment or prevention of a disease, whereby the disease involves an undesired cell proliferation.

This use of the compounds according to the present invention is based on the fact that the compounds according

to the present invention are suitable to inhibit undesired cell proliferation. Undesired cell proliferation comprises the undesired cell proliferation of procaryotic cells as well as undesired cell proliferation of eucaryotic cells. The term undesired cell proliferation also covers the phenomenon of abnormal cell proliferation, abnormal mitosis and undesired mitosis. Abnormal cell proliferation means any form of cell proliferation which occurs in a manner different from the normal cell proliferation. Normal cell proliferation is a cell proliferation observed under normal circumstances by the majority of cells and organisms, respectively. The same basic definition applies to abnormal mitosis.

More particularly, undesired cell proliferation and undesired mitosis mean a proliferation and a mitosis, respectively, which may be either a normal or an abnormal cell proliferation, however, in any case it is not a cell proliferation or mitosis which is desired. Desired may thus be defined by an individual such as a human being and in particular a physician, and defined within certain boundaries whereby the boundaries as such may reflect the extent of proliferation and mitosis, respectively, observed under usual conditions or in the majority of cells and organisms, respectively, or may be arbitrarily fixed or defined. Cell proliferation as used herein refers preferably to the proliferation of cells forming the organism to be treated or to which a compound according to the present invention shall be administered which is also referred to herein as the first organism. Cell proliferation as used herein also means the proliferation of cells which are different from the cells forming a first organism or species but are the cells forming a second organism or second species. Typically, the second organism enters in or has a relationship with the first organism. Preferably, the first organism is a human being or an animal or plant, also referred to herein as patient, and the second organism is a parasite and pathogen, respectively, to said first organism. Mitosis as used herein, preferably means the cell division of cells being subject to said cell proliferation whereby even more preferably mitosis is the process of cell division whereby a complete set of chromosomes is distributed to the daughter cells.

Without wishing to be bound by any theory, it seems that the compounds according to the present invention act on cells and thus influence their proliferation and mitosis, respectively, by being inhibitors to some enzymatic activity. Preferably, the inhibition is reversible. This activity is shown by the compounds according to the present invention with regard to bacteria, fungi, insect and mammalian cells.

Because of this, the compounds according to the present invention may be used for the treatment of a wide variety of disorders involving cell cycle regulation, both procaryotic and eucaryotic cell cycle regulation. The term "treatment" as used herein comprises both treatment and prevention of a disease. It also comprises follow-up treatment of a disease. Follow-up treatment is realized upon a treatment of a disease using compounds preferably different from the one according to the present invention. For example, after stimulating the growth of a cell, tissue or the like by the application of a respective compound such as, e. g., erythropoietin, it might be necessary to stop an overshooting reaction of cell proliferation which may be obtained using the compounds according to the present invention.

By "reversible" herein is meant that the inhibitor binds non-covalently to the respective enzyme, and is to be distinguished from irreversible inhibition. See Walsh, Enzymatic Reaction Mechanisms, Freeman & Co., N.Y., 1979. "Reversible" in this context is a term understood by those skilled in the art. Preferably the compounds according to the present invention are competitive inhibitors, that is, they compete with substrate in binding reversibly to the enzyme, with the binding of inhibitor and substrate being mutually exclusive.

In a preferred embodiment of the compounds according to the present invention the dissociation constant for inhibition of the enzyme(s) with the inhibitor, i. e. the compound according to the present invention, generally referred to and characterized by those in the art as $K_i$, is at most about 100 $\mu$M. By the term "binding constant" or "dissociation constant" or grammatical equivalents herein is meant the equilibrium dissociation constant for the reversible association of inhibitor with enzyme. The dissociation constants are defined and determined as described below. The determination of dissociation constants is known in the art. For example, for reversible inhibition reactions such as those of the present invention, the reaction scheme is as follows:

$$E+I \underset{k_2}{\overset{k_1}{\rightleftharpoons}} E^*I \quad \text{(Equation 1)}$$

The enzyme (E) and the inhibitor (I) combine to give an enzyme-inhibitor complex (E*I). This step is assumed to be rapid and reversible, with no chemical changes taking place; the enzyme and the inhibitor are held together by non-covalent forces. In this reaction, $k_1$ is the second order rate constant for the formation of the E*I reversible complex. $k_2$ is the first order rate constant for the dissociation of the reversible E*I complex. In this reaction, $Ki = k_2/k_1$.

The measurement of the equilibrium constant $K_i$ proceeds according to techniques well known in the art. For example, assays generally use synthetic chromogenic or fluorogenic substrates. The respective $K_i$ values may be estimated using the Dixon plot as described by Irwin Segel in Enzyme Kinetics: Behavior and analysis of rapid equilibrium and steady-state enzyme systems, 1975, Wiley-Interscience Publication, John Wiley & Sons, New York, or for competitive

binding inhibitors from the following calculation:

$$1-(\nu_i/\nu_o)=[I]/[I]+K_i\,(1+([S]/K_m))\quad \text{(Equation 2)}$$

wherein $\nu_o$ is the rate of substrate hydrolysis in the absence of inhibitor, and $\nu_i$ is the rate in the presence of competitive inhibitor.

The compounds according to the present invention may be easily screened for their efficacy in relation to the various uses disclosed herein

By a "labelled compound according to the present invention" herein is meant a compound according to the present invention that has at least one element, isotope or chemical compound attached to enable the detection of the compound or the compound bound to a target such as an enzyme. In general, labels as used herein, fall into three classes: a) isotopic labels, which may be radioactive or heavy isotopes; b) immune labels, which may be antibodies or antigens; and c) colored or fluorescent dyes. The labels may be incorporated into the compound at any position. Examples of useful labels include $^{14}C$, $^{13}C$, $^{15}N$, $^{3}H$, biotin, and fluorescent labels as are well known in the art.

As used herein, the term "disease" describes any disease, diseased condition or pathological condition. Such disease may also be defined as abnormal condition. Also, in case of a pathogen, disease means a condition where a pathogen or an unwanted organism is present or present in a concentration or compartment where it is undesired and thus subject to reduction in numbers, removal, elimination and/or destruction by using the compounds according to the present invention.

The compounds according to the present invention may be used as a medicament and for the manufacture of a medicament, respectively, whereby the medicament is for the treatment of cell proliferative disorders and any of the diseases specified herein. Cell proliferated disorders as used herein, typically involve an abnormal cell proliferation, an undesired cell proliferation, an abnormal mitosis and/or an undesired mitosis.

Cell proliferative disorders contemplated for treatment using the compounds according to the present invention and for the methods disclosed herein include also disorders characterized by unwanted or undesired, inappropriate or uncontrolled cell growth. Preferably, the disease is selected from the group comprising neurodegenerative diseases, stroke, inflammatory diseases, immune based disorders, infectious diseases, heart diseases, fibrotic disorders, cardiovascular diseases and cell proliferative diseases. Rotamases comprise families of ubiquitous and highly conserved enzymes who have been reported to play important roles in biological processes like protein folding, proteolysis, protein dephosphorylation, peptide transport function, cell cycle regulation, protein synthesis. Furthermore various isomerases have been shown to have regulatory functions as stable or dynamic part of heterooligomeric complexes containing physiologically relevant proteins e.g. hormone receptors, ion channels, kinases, and growth factor receptors.

Preferably, the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases and motor neuron diseases.

The compounds according to the present invention are additionally useful in inhibiting cell cycle (mitosis) or cell division in pathogenic organisms and are, therefore, useful for treating infectious diseases.

In a preferred embodiment the infectious is selected from the group comprising fungal, viral, bacterial and parasite infection.

Fungal infections contemplated for treatment using the compounds and methods according to the present invention include systemic fungal infections, dermatophytoses and fungal infections of the genito-urinary tract. Fungal infections, preferably systemic fungal infections, include those caused by *Histoplasma, Coccidioides, Cryptococcus, Blastomyces, Paracoccidioides, Aspergillus, Nocardia, Sporothrix, Rhizopus, Absidia, Mucor, Hormodendrum, Phialophora, Rhinosporidium,* and the like. Dermatophyte infections include those caused by *Microsporum, Trichophyton, Epidermophyton, Candida, Pityrosporum*, and the like. Fungal disorders of the genito-urinary tract include infections caused by *Candida, Cryptococcus, Aspergillus, Zygomycodoides,* and the like. Infection by such organisms causes a wide variety of disorders such as ringworm, thrush or candidiasis, San Joaquin fever or Valley fever or coccidiodomycosis, Gilchrist's disease or blastomycosis, aspergillosis, cryptococcosis, histioplasmosis, paracoccidiomycosis, zygomycosis, mycotic keratitis, nail hair and skin disease, Lobo's disease, lobomycosis, chromoblastomycosis, mycetoma, and the like. These infections can be particularly serious, and even fatal, in patients with a depressed immune system such as organ transplant recipients and persons with acquired immunodefficiency syndrome (AIDS). Insofar a patient group which can be treated using the inhibitors according to the present invention are persons with AIDS, particularly those suffering from any of the infectious diseases described herein.

In a further embodiment the bacterial infection is selected from the group comprising infections caused by both

Gram-positive and Gram-negative bacteria, including infections caused by *Staphylococcus, Clostridium, Streptococcus, Enterococcus, Diplococcus, Hemophilus, Neisseria, Erysipelothricosis, Listeria, Bacillus, Salmonella, Shigella, Escherichia, Klebsiella, Enterobacter, Serratia, Proteus, Morganella, Providencia, Yersinia, Camphylobacter, Mycobacteria, Flelicobacter, Legionalla, Nocardia* and the like.

In a preferred embodiment the bacterial infection causes a wide variety of diseases. Said disorders are selected, among others, from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

In another embodiment the disease is a viral infection, more particularly a viral infection caused by a virus selected from the group comprising retrovirus, HIV, Papilloma virus, Polio virus, Epstein-Barr, Herpes virus, Hepatitis virus, Papova virus, Influenza virus, Rabies, JC, encephalitis causing virus, hemorrhagic fever causing virus such as Ebola Virus and Marburg Virus.

In a further embodiment the parasite infection is selected from the group comprising infections caused by *Trypanosoma, Leishmania, Trichinella, Echinococcus, Nematodes, Classes Cestoda, Trematoda, Monogenea, Toxoplasma, Giardia, Balantidium, Paramecium, Plasmodium or Entamoeba.*

The disease may further be a cell proliferative disorder which preferably is selected from the group characterized by unwanted, inappropriate or uncontrolled cell growth. Particular examples include cancer, fibrotic disorders, non-neoplastic growths. The neoplastic cell proliferative disorder is preferably selected from the group comprising solid tumors, and hematopoeitic cancers such as lymphoma and leukemia.

More preferably, the solid tumor is selected from the group comprising carcinoma, sarcoma, osteoma, fibrosarcoma, and chondrosarcoma.

More preferably, the cell proliferative disorder is selected from the group comprising breast cancer, prostate cancer, colon cancer, brain cancer, lung cancer, pancreatic cancer, gastric cancer, bladder cancer, kidney cancer and head and neck cancer. Preferably, the lung cancer is non-small lung cancer and small lung cancer.

In case the disease is a non-neoplastic cell proliferative disorder, it is preferably selected from the group comprising fibrotic disorder. Preferably, the fibrotic disorder is fibrosis.

The disease may also be a non-neoplastic cell proliferative disorder which is selected from the group comprising prostatic hypertrophy, preferably benign prostatic hypertrophy, endometriosis, psoriasis, tissue repair and wound healing. Fibrotic disorders which may be treated using the compounds according to the present invention are generally characterized by inappropriate overproliferation of non-cancerous fibroblasts. Examples thereof include fibromyalgia, fibrosis, more particularly cystic, hepatic, idopathic pulmonary, and pericardial fibrosis and the like, cardiac fibromas, fibromuscular hyperplasia, restenosis, atherosclerosis, fibromyositis, and the like.

In another embodiment the immune based and/or inflammatory disease is an autoimmune disease or autoimmune disorder. In a further embodiment, the immune based and/or inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease, and dermatomyositis.

In a further preferred embodiment the immune based and/or inflammatory disease is an inflammatory condition.

In a still further embodiment the immune based and/or inflammatory disease is selected from the group comprising inflammation associated with burns, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

It is also within the present invention that the compounds according to the present invention may be used for the treatment of a patient suffering from a disease or diseased condition as defined above. Such treatment comprises the administration of one or several of the compounds according to the present invention or a medicament or pharmaceutical composition described herein.

Toxicity and therapeutic efficacy of a compound can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Compounds which exhibit large therapeutic indices are preferred. The data obtained from these cell culture assays and animal studies can be used in formulating a range of dosages suitable for use in humans. The dosage may vary within this range depending upon a variety of factors, e.g., the dosage form employed, the route of administration utilized, the condition of the subject, and the like

For any compound according to the present invention, the therapeutically effective dose can be estimated initially from cell culture assays by determining an $IC_{50}$ (i.e., the concentration of the test substance which achieves a

half-maximal inhibition of cell proliferation). A dose can then be formulated in animal models to achieve a circulating plasma concentration range that includes the $IC_{50}$ as determined in cell culture. Such information can be used to more accurately determine useful doses in humans. Levels in plasma may be measured, for example by HPLC or LC/MS.

It should be noted that the attending physician would know how to and when to terminate, interrupt, or adjust administration due to toxicity, to organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient. Typically, the dose will be between about 1-10 mg/kg of body weight. About 1 mg to about 50 mg will preferably be administered to a child, and between 25 mg and about 1000 mg will preferably be administered to an adult.

A program comparable to that discussed above may be used in veterinary medicine. The exact dose will depend on the disorder to be treated and will be ascertainable by one skilled in the art using known techniques.

Depending on the specific conditions to be treated, such compounds may be formulated and administrated systemically or locally. Techniques for formulation and administration may be found in "Remington's Pharmaceutical Sciences", 1990, 18th ed., Mack Publishing Co., Easton, PA. The administration of a compound according to the present invention can be done in a variety of ways, including, but not limited to, orally, subcutaneously, intravenously, intranasally, transdermally, intraperitoneally, intramuscularly, intrapulmonary, vaginally, rectally, or intraocularly, just to name a few. In some instances, for example, in the treatment of wounds and inflammation, the compound according to the present invention may be directly applied as a solution or spray.

In a further aspect the present invention is related to a medicament or a pharmaceutical composition comprising at least one active compound and at least one pharmaceutically acceptable carrier, excipient or diluent. As used herein, the active compound is a compound according to the present invention, a pharmaceutically salt or base thereof or a prodrug thereof, if not indicated to the contrary.

For injection, compounds of the invention may be formulated in aqueous solution, preferably in physiologically compatible buffers such as Hank's solution, Ringer's solution, or physiologically saline buffer. For transmucosal administration, penetrants appropriate to the barrier to be permeated are used in the formulation. Such penetrants are generally known in the art.

The use of pharmaceutical acceptable carriers to formulate the compounds according to the present invention into dosages or pharmaceutical compositions suitable for systemic administration is within the scope of the present invention. With proper choice of carrier and suitable manufacturing practice, the compositions of the present invention, in particular those formulated as solutions, may be administered parenterally, such as by intravenous injection. The compounds can be readily formulated using pharmaceutically acceptable carriers well known in the art into dosages suitable for oral administration. Such carriers enable the compounds according to the present invention to be formulated as tablets, pills, capsules, dragees, liquids, gels, syrups, slurries, suspensions and the like, for oral ingestion by a subject to be treated.

Compounds according to the present invention or medicaments comprising them, intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Delivery systems involving liposomes are disclosed in International Patent Publication No. WO 91/19501, as well as U.S. Patent No. 4,880,635 to Janoff et al. The publications and patents provide useful descriptions of techniques for liposome drug delivery and are incorporated by reference herein in their entirety.

Pharmaceutical compositions comprising a compound according to the present invention for parenteral administration include aqueous solutions of the active compound(s) in watersoluble form. Additionally, suspensions of the active compounds may be prepared as appropriate oily injection suspensions. Suitable lipophilic solvents or vehicles include fatty oils such as sesame oil or castor oil, or synthetic fatty acid esters, such as ethyl oleate or triglycerides, or liposomes. Aqueous injections suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions.

Pharmaceutical compositions comprising a compound according to the present invention for oral use can be obtained by combining the active compound(s) with solid excipient, optionally grinding the resulting mixture, and processing the mixture of granules, after adding suitable auxiliaries, if desired, to obtain tablets or dragee cores.

Suitable excipients are, in particular, fillers such as sugars, including lactose, sucrose, mannitol, sorbitol, and

the like; cellulose preparations, such as, for example, maize starch wheat starch, rice starch, potato starch, gelatin, gum tragacanth, methyl cellulose, hydroxypropylmethyl cellulose, sodium carboxymethyl cellulose, polyvinylpyrrolidone (PVP) and the like, as well as mixtures of any two or more thereof. If desired, disintegrating agents may be added, such as cross-linked polyvinyl pyrrolidone, agar, alginic acid or a salt thereof such as sodium alginate, and the like.

Dragee cores as a pharmaceutical composition comprising a compound according to the present invention are provided with suitable coatings. For this purpose, concentrated sugar solutions may be used, which may optionally contain gum arabic, talc, polyvinyl pyrrolidone, carbopol gel, polyethylene glycol, titanium dioxide, lacquer solutions, suitable organic solvents or solvent mixtures, and the like. Dyestuffs or pigments may be added to the tablets or dragee coatings for identification or to characterize different combinations of active compound doses.

Pharmaceutical preparations comprising a compound according to the present invention which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

A "patient" for the purposes of the present invention, i. e. to whom a compound according to the present invention or a pharmaceutical composition according to the present invention is administered, includes both humans and other animals and organisms. Thus the compounds, pharmaceutical compositions and methods are applicable to or in connection with both human therapy and veterinary applications including diagnostic(s), diagnostic procedures and methods as well as staging procedures and methods. For example, the veterinary applications include, but are not limited to, canine, bovine, feline, porcine, caprine, equine, and ovine animals, as well as other domesticated animals including reptiles, such as iguanas, turtles and snakes, birds such as finches and members of the parrot family, lagomorphs such as rabbits, rodents such as rats, mice, guinea pigs and hamsters, amphibians, fish, and arthropods. Valuable non-domesticated animals, such as zoo animals, may also be treated. In the preferred embodiment the patient is a mammal, and in the most preferred embodiment the patient is human.

The pharmaceutical composition according to the present invention comprises at least one compound according to the present invention in a form suitable for administration to a patient. Preferably, a compound according to the present application is in a water soluble form, such as being present as a pharmaceutically acceptable salt, which is meant to include both acid and base addition salts which are also generally referred to herein as pharmaceutically acceptable salts. "Acid addition salt", and more particularly "pharmaceutically acceptable acid addition salts" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Base addition salts" and more particularly "pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The pharmaceutical compositions according to the present invention may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds according to the present invention are, in a further embodiment, administered to a subject either alone or in a pharmaceutical composition where the compound(s) is mixed with suitable carriers or excipient(s). In treating a subject, a therapeutically effective dose of compound (i.e. active ingredient) is administered. A therapeutically effective dose refers to that amount of the active ingredient that produces amelioration of symptoms or a prolongation of survival of a subject which can be determined by the one skilled in the art doing routine testing.

On the other hand, the compounds according to the present invention may as such or contained in a pharmaceutical composition according to the present invention be used in drug potential applications.

For example, therapeutic agents such as antibiotics or antitumor drugs can be inactivated through the catalytic action of endogenous enzymes, thus rendering the administered drug less effective or inactive. Accordingly, the compound(s) according to the present invention may be administered to a patient in conjunction with a therapeutic agent in order to potentiate or increase the activity of the drug. This co-administration may be by simultaneous administration, such as a mixture of the compound(s) according to the present invention and the drug, or by separate simultaneous or sequential administration.

According to the present invention the compounds disclosed herein, referred to as compounds according to the present invention, may be used as a medicament or for the manufacture of medicament or in a method of treatment of a patient in need thereof. Insofar any of these compounds constitute a pharmaceutical compound. The use of this kind of compound also comprises the use of pharmaceutically acceptable derivatives of such compounds.

In addition, the compounds according to the present invention may be transformed upon application to an organism such as a patient, into the pharmaceutically active compound. Insofar the compounds according to the present invention may be prodrugs which, however, are nevertheless used for the manufacture of the medicaments as disclosed herein given the fact that at least in the organism they are changed in a form which allows the desired.

It is to be understood that any of the pharmaceutical compositions according to the present invention may be used for any of the diseases described herein.

The pharmaceutical compositions according to the present invention may be manufactured in a manner that is known as such, e.g., by means of conventional mixing, dissolving, granulating, dragee-mixing, levigating, emulsifying, encapsulating, entrapping, lyophilizing, processes, or the like.

In a further aspect of the present invention the compounds of the present invention may be used as insecticides as they may prevent cell cycle mitosis in insect cells and thus can be used to control the growth and proliferation of a variety of insect pests. This aspect of the present invention has important applications in agriculture, such as in the field, in the storage of agricultural products and the like. Additionally, the compounds according to the present invention are useful for controlling insect populations, preferably in places inhabited by men, such as homes, offices and the like.

Any of the compounds according to the present invention containing one or more asymmetric carbon atoms may occur as racemates and racemic mixtures, single enantiomers, diastereomeric mixtures and individual diastereomers. All such isomeric forms of these compounds are expressly included in the present invention. Each stereogenic carbon may be in the R or S configuration, or a combination of configurations.

It shall be understood by the one of ordinary skill in the art that all compounds of the invention are preferably those which are chemically stable. This applies to any of the various uses of the compounds according to the present invention disclosed herein.

In determining the suitability of any of the compounds according to the present applications for the various uses, besides the particular use-specific profile to be met by such a compound, also it has to be checked whether it is stable to proteolytic degradation. The resistance of the compound used as a pharmaceutical may be tested against a variety of non-commercially available proteases *in vitro* to determine its proteolytic stability. Promising candidates may then be routinely screened in animal models, for example using labelled inhibitors, to determine the *in vivo* stability and efficacy. In any of the aforementioned uses the compound may be present in a crude or purified form. Methods for purifying the compounds according to the present invention are known to the one skilled in the art. The problem underlying the present invention is also solved by the technical teaching according to the attached independent claims. Preferred embodiments thereof may be taken from the dependent claims.

The invention is now further illustrated by reference to the following figures and examples from which further advantages, features and embodiments may be taken. It is understood that these examples are given for purpose of illustration only and not for purpose of limitation. All references cited herein are incorporated by reference.

Fig. 1 shows FACS results of compound 703,

Fig. 2 show a FACS analysis of the effect of various compounds on HL 60 cells as a measure for the apoptotic activity of the compounds; and

Fig. 3 shows fluorescence microscope photographs of DAPI stained HeLa cells after treatment with various compounds

Example 1: Material and Methods

In order that the invention herein described may be more fully understood, the following detailed description is set forth. As used herein, the following abbreviations are used:

Ar is argon;
D is doublet;
DCM is dichloromethane;
DIPEA is *N*,*N*-diisopropylethylamine;
DMF is *N*,*N*-dimethylformamide;
DMSO is *N*,*N*-dimethylsulfoxide;
eq is equivalent;

$Et_3N$ is triethylamine;
HCl is chlorhydric acid;
HPLC is high performance liquid chromatography;
h is hour;
Hz is hertz;
m is multiplet;
mL is milliliter;
$NaHCO_3$ is sodium hydrogencarbonate;
s is singulet;
THF is tetrahydrofuran.

**Method A: Urea formation in solution**

Amine salt and DIPEA (1 eq each) or amine (1 eq) was dissolved in dry dioxan and a solution of the isocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40˚C, the solution was filtered off and the solvent was removed under reduced pressure. The obtained crude ureas were purified by HPLC.

**Method B: Coupling of substituted anilines with sulfonyl chlorides**

Aniline (30 mg) and $NEt_3$ (1.2 eq, 2.2 eq. when HCl salt) were dissolved in dry acetonitrile (0.5 mL). Sulfonyl chloride (1 eq) was dissolved in dry acetonitrile (0.5 mL) and added to the solution. The reaction mixture was stirred under argon for 12 h at 40˚C. Tris-(2-aminoethyl)-amine polystyrene (30 mg), (polystyrylmethyl) trimethylammomum bicarbonate (30 mg), *N*-(2-mercaptoethyl)amino methyl polystyrene (30 mg), and methylisocyanate polystyrene (30 mg) were given to the solution and stirred for additional 12 h at 40˚C. After filtration the solvent was removed under reduced pressure. The crude reaction product was purified by preparative HPLC using acetonitrile and water as mobile phase.

**Method C: Coupling of substituted anilines with acid chlorides**

Aniline (30 mg) and $NEt_3$ (1 eq, 2 eq. when HCl salt) were dissolved at 0˚C in dry DCM (0.5 mL) with 10% DMSO. Acid chloride (1 eq) was dissolved at 0˚C in dry DCM (0.5 mL) and added to the solution. The reaction mixture was stirred under argon for 2 h while slowly warming up to room temperature. Work up was performed by pouring the reaction mixture with DCM over incubated HYDROMATRIX layers. 2 mL basic layer (saturated $NaHCO_3$ solution 2 ml/g HYDROMATRIX), 2 mL acidic layer (2M HCl 2 ml/g HYDROMATRIX), and 2 mL of dry HYDROMATRIX layer in a 10 ml syringe were used. The solvent was removed under reduced pressure. The crude reaction product was purified by preparative HPLC using acetonitrile and water as mobile phase.

**Method D: Reductive amination of aldehydes with hydroxyl anilines**

Amine hydrochloride (0.11 mmol, 1 eq), aldehyde (0.11 mmol, 1 eq), and DIPEA (0.11 mmol, 1 eq) were dissolved in anhydrous THF (1 mL), and molecular sieves 4A (10 mg) was added to the solution. After shaking for 1.5 h at room temperature, (polystyrylmethyl)trimethylammonium cyanoborohydride (4.3 mmol/g, 0.22 mmol) was added to the reaction mixture. After shaking for 8 h at room temperature, 4-benzyloxybenzaldehyde polystyrene (3 mmol/g, 0.22 mmol), 3-(4-(hydrazinosufonyl)phenyl)propionyl AM resin (1.5 mmol/g, 0.22 mmol), and N-(2-mercaptoethyl)aminomethyl polystyrene (2.1 mmol/g, 0.22 mmol) were added, after which the reaction mixture was shaken at room temperature for 18 h. Filtration, washing with DCM, and evaporation of the solvent in vacuo afforded the crude product, which was purified by reversed phase HPLC.

**Method E: Condensation of amines with hydroxyl carboxylic acids**

1-Ethyl-3(3'-dimethylaminophropyl)carbodiimide hydrochloride (0.15 mmol, 1.36 eq), hydroxyl carboxylic acid (0.15 mmol, 1.36 eq), and 1-hydroxy-7-azabenzotriazole (0.15 mmol, 1.36 eq) were dissolved in DMF (0.7 mL). After shaking for 30 min at room temperature, a solution of amine (0.11 mmol) in DMF (0.7 mL) was added to the reaction mixture. After shaking for 2 h at room temperature, amine (0.22 mmol) was added, after which the reaction mixture was shaken at 60 ˚C overnight. Then the solvent was evaporated in vacuo, and the residue was dissolved in DCM (7 mL). HYDROMATRIX™ (0.3 g) which was previously treated with HCl (2N, 0.6 mL) was added to the solution, and the mixture

was shaken for 30 min. Filtration, washing with DCM, and evaporation of the solvent in vacuo afforded the crude product, which was purified by reversed phase HPLC.

**Method F: Carbamate formation in solution**

Amine or amine salt (1 eq) and sodium bicarbonate (1 or 2 eq) were dissolved in a mixture of MeOH / $H_2O$ (3: 1). The mixture was treated with chloroformate (1 eq), which was added in three portions over 10 minutes. After 30 minutes at room temperature, the precipitating product was collected by filtration and washed with water. The obtained crude carbamates were purified by HPLC.

**Method G: Carbamate formation in solution**

To an ice cooled mixture of amine or amine salt (1 eq) and DIPEA (1.1 eq or 2.2 eq) in dry DCM was added an ice cooled solution of chloroformate (1.1 eq) in DCM in one portion. After 1.5-8 h at room temperature the solvent was removed under reduced pressure. The obtained crude carbamates were purified by HPLC.

**Method H: Carbamate formation in solution**

To a mixture of amine or amine salt (1 eq) and sodium bicarbonate (1 or 2.5 eq) in dry DCM was added chloroformate (1 eq) in one portion. After 4 hours at room temperature the sodium bicarbonate was filtered off and the solvent was removed under reduced pressure. The obtained crude carbamates were purified by HPLC.

**Method I: Thiourea formation in solution**

Amine salt and DIPEA (1 eq each) or amine (1 eq) was dissolved in dry dioxan and a solution of the thioisocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40˚C, the solution was filtered off and the solvent was removed under reduced pressure. The obtained crude thioureas were purified by HPLC.

**Method J: Synthesis of (3-amino-5-chloro-4-hydroxy-phenyl)-ureas.**

Isocyanate (5.0 mmol) was added to a stirred solution of 4-Amino-2-chloro-phenol (5.0 mmol) in anhydrous $CH_2Cl_2$ (23 mL) and THF (4 mL) at room temperature. After stirring for 12 h, the solvent was evaporated in vacuo. The residue was then dissolved in HOAc (95 mL) and added in one single portion to a stirred solution of $NaNO_2$ (1.17 g, 17.0 mmol) in $H_2O$ (8.4 mL). The flask was sealed with a stopper and the reaction mixture was stirred for 1.5 min at room temperature. The reaction was stopped by the addition of saturated aqueous $NaHCO_3$ (190 mL). After stirring for 10 min at room temperature, the yellow precipitate was filtered, washed with $H_2O$ (3 x 30 mL), and dried in vacuo. The yellow residue was then dissolved in a mixture of toluene (75 mL) and MeOH (90 mL). Raney nickel (0.5 g) was washed with MeOH (5 x 10 mL) and added to the reaction mixture. Then the reaction mixture was vigorously stirred under a hydrogen atmosphere at 1 bar at room temperature for 2 h. Filtration through a pad of Celite and evaporation of the solvent afforded the aniline, which was converted into the corresponding diureas by method A, the corresponding anilines by method D, the corresponding sulfonamides by method B, the corresponding amides by method C or E, or the corresponding thioureas by method I.

**Method K: Synthesis of (5-benzothiazol-2-yl-3-chloro-2-hydroxy-phenyl)-ureas, (5-benzothiazol-2-yl-3-chloro-2-hydroxy-phenyl)-thioureas, (3-benzothiazol-2-yl-5-chloro-4-hydroxy-phenylrureas, (3-benzothiazol-2-yl-5-chloro-4-hydroxy-phenyl)-thioureas, (5-benzothiazol-2-yl-3-chloro-2-hydroxy-phenyl)-amides, and (3-benzothiazol-2-yl-5-chloro-4-hydroxy-phenyl)-amides.**

3-Chloro-2-hydroxy-5-nitro-benzoic acid or 3-chloro-4-hydroxy-5-nitro-benzoic acid (0.41 g, 1.9 mmol) was dissolved in polyphosphoric acid (12.3 g) at 110 ˚C. 2-Aminothiophenol (0.36 mg, 2.9 mmol) was added and the resulting solution was stirred at 110 ˚C for 5 h. After cooling, ammonia (35% in $H_2O$, 12 mL) was added to the reaction mixture. The precipitate was filtered, washed with $H_2O$ (3 x 10 mL) and dried in vacuo. The residue was then dissolved in a mixture of MeOH (30 mL) and THF (70 mL). Raney nickel (0.5 g) was washed with MeOH (5 x 10 mL) and added to the reaction mixture. Then the reaction mixture was vigorously stirred under a hydrogen atmosphere at 1 bar at room temperature for 1 h. Filtration through a pad of Celite and evaporation of the solvent afforded the aniline, which was

converted into the corresponding ureas by method A, the corresponding amides by method C or E, or the corresponding thioureas by method I.

**Method L: Synthesis of (3-amino-5-chloro-4-hydroxy-phenyl)-amides.**

Acyl chloride (5.0 mmol) was added to a stirred solution of 4-Amino-2-chloro-phenol (5.0 mmol) in anhydrous $CH_2Cl_2$ (23 mL) and THF (4 mL) at O˚C. After stirring for 12 h, the solvent was evaporated in vacuo. The residue was then dissolved in HOAc (95 mL) and added in one single portion to a stirred solution of $NaNO_2$ (1.17 g, 17.0 mmol) in $H_2O$ (8.4 mL). The flask was sealed with a stopper and the reaction mixture was stirred for 1.5 min at room temperature. The reaction was stopped by the addition of saturated aqueous $NaHCO_3$ (190 mL). After stirring for 10 min at room temperature, the yellow precipitate was filtered, washed with $H_2O$ (3 x 30 mL), and dried in vacuo. The yellow residue was then dissolved in a mixture of toluene (75 mL) and MeOH (90 mL). Raney nickel (0.5 g) was washed with MeOH (5 x 10 mL) and added to the reaction mixture. Then the reaction mixture was vigorously stirred under a hydrogen atmosphere at 1 bar at room temperature for 2 h. Filtration through a pad of Celite and evaporation of the solvent afforded the aniline, which was converted into the corresponding ureas by method A, the corresponding anilines by method D, the corresponding sulfonamides by method B, the corresponding diamides by method C or E, or the corresponding thioureas by method I.

Example 2: 1-Adamantan-1-yl-3-(3,5-dichloro-2-hydroxy-4-methyl-phenyl)-urea

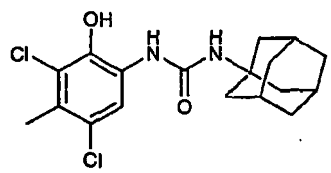

To a solution of 6-amino-2,4-dichloro-3-methyl-phenol hydrochloride (113.5 mg, 1 eq) and DIPEA (48 μL, 1 eq) in dioxan (1.1 mL) was added 1-adamantylisocyanate (88.5 mg, 1 eq) in 580 μL DMSO in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and *N*-(2-mercaptoethyl)aminomethyl polystyrene (3 eq)) were added. After 18 h at 40˚C the solution was filtered off and the solvent was removed under reduced pressure. The crude product was purified by HPLC to obtain 118 mg (64 %) of the title compound as a white powder.
NMR-$^1$H (DMSO-$d_6$) δ =1.62 ($s_b$, 6H), 1.92 ($s_b$, 6H), 2.05 ($s_b$, 3H), 2.29 (s, 3H), 6.79 (s, 1H), 7.95 (s, 1H), 8.15 (s, 1H), 9.82 (s, 1H).
NMR-$^{13}$C (DMSO-$d_6$) δ =17.1, 29.3, 36.1, 41.5, 50.3, 116.3, 122.1, 123.8, 124.7, 129.9, 140.6, 154.3; MS (m/z): 369.2 [M+H$^+$].

Example 3: 1-(3,5-Dichloro-4-hydroxy-phenyl)-3-(1,1,3,3-tetramethyl-butyl)-urea

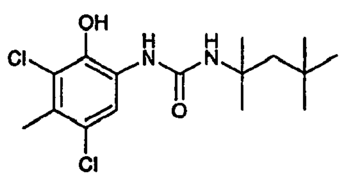

To a solution of 4-amino-2,6-dichloro-phenol (44.5 mg,1 1 eq) in dioxan (2.0 mL) was added 2-isocyanato-2,4,4-trimethyl-pentane (38 mg, 1 eq) in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added. After 18 h at 35˚C the solution was filtered off and the solvent was removed under reduced pressure. The crude material was purified by HPLC to

obtain 67 mg (81%) of the title compound as a white powder.

NMR-$^1$H (DMSO-$d_6$) $\delta$ = 0.95 (s, 9H), 1.30 (s, 6H), 1.70 (s, 2H), 2.30 (s, 3H), 6.80 (s, 1H), 8.00 (s, 1H), 8.08 (s, 1H), 9.83 ($s_b$, 1H).
NMR-$^{13}$C (DMSO-$d_6$) $\delta$ = 29.7, 31.2, 50.5, 53.2, 117.3, 122.4, 134.0, 142.8, 153.9; MS (m/z): 333.2 [M+H$^+$].

Example 4: 2 *N*-(2-Hydroxy-4-methyl-phenyl)-C-phenyl-methanesulfonamide

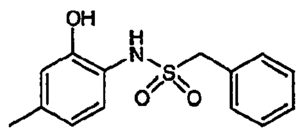

The compound was obtained in 32% yield (21.2 mg) using the protocol described in method A.
NMR-$^1$H (DMSO-$d_6$) $\delta$ = 2.13 (s, 3H), 4.97 (s, 2H), 6.70 (d, 1H, *J* = 8.2 Hz), 6.78 (s, 1H), 6.83 (d, 1H, *J* = 8.2 Hz), 7.43 (m, 2H), 7.49 (m, 2H); MS (*m*/*z*): 278.1 [M$^+$].

Example 5: Propane-2-sulfonic acid (3,5-dichloro-2-hydroxy-4-methyl-phenyl)-amide

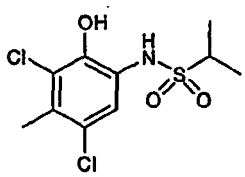

The compound was obtained in 47% yield (22.1 mg) using the protocol described in method B.
NMR-$^1$H (DMSO-$d_6$) $\delta$ = 2.25 (d, 6H, *J*= 6.8), 2.27 (s, 3H), 3.97 (hep, 1H, *J*= 6.8), 5.39 (s, 1H), 6.91 (s, 1H); MS (*m*/*z*): 298.1 [MH$^+$].

Example 6: N-(3,5-Dichloro-2-hydroxy-phenyl)-2-phenyl-acetamide

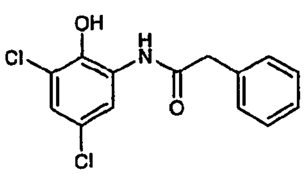

The compound was obtained in 22% yield (11.0 mg) using the protocol described in method C.
NMR-$^1$H (DMSO-$d_6$) $\delta$ = 3.06 (s, 2H), 7.25 (m, 1H), 7.31 (m, 2H), 7.32 (m, 2H), 7.61 (m, 2H), 9.87 (m, 1H); MS (*m*/*z*): 296.2 [MH$^+$].

Example 7: N-(3,5-Dichloro-2-hydroxy-4-methyl-phenyl)-N-methyl-2-trifluoromethyl-benzamide

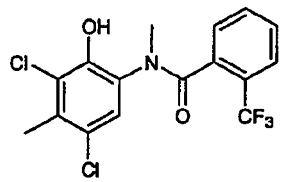

Aniline HCL salt (300 mg, 1.3 mmol) and $K_2CO_3$ (500 mg) were dissolved in DMSO (5 mL). $CH_3I$ (187 mg, 1.3 mmol) was added and the suspension was stirred for 48 h at room temperature. After filtration, the solvent was removed under reduced pressure. The crude reaction product was purified by preparative HPLC using acetonitrile and water as mobile phase to give the *N*-methyl amino phenol derivative (130 mg, 31 %).
The amide was obtained in 56% yield (19.7 mg) using the protocol described in method C.
NMR-$^1$H (DMSO-$d_6$) δ = 2.41 (s, 3H), 3.75 (s, 3H), 7.70-7.85 (m, 4H), 8.05 (d, 1H, *J*= 5.0); MS (*m*/*z*): 377.9 [$MH^+$].

Example 8: 2,4-Dichloro-3-methyl-6-(3-methyl-benzylamino)-phenol

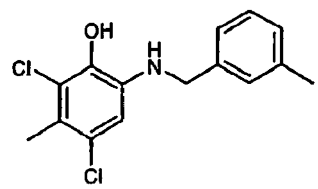

The compound was obtained in 49 % yield (16 mg) using the protocol described in method D. According to method D.
NMR-$^1$H (DMSO-$d_6$) δ 2.21 (s, 3 H), 2.27 (s, 3 H), 4.27 (s, 2 H), 5.80 (br s, 1 H), 6.35 (s, 1 H), 7.02-7.24 (m, 4 H), 9.18 (br s, 1 H); MS (*m*/*z*): 296.2 [$M+H^+$].

Example 9: 1-{5-Chloro-3-[2-(4-chloro-phenylsulfanyl)-benzylamino]-2-hydroxy-phenyl}-ethanone

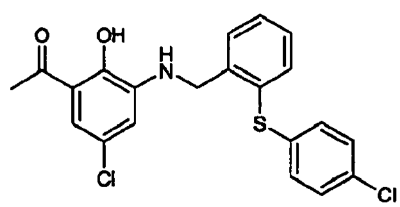

The compound was obtained in 18 % yield (12 mg) using the protocol described in method D.
NMR-$^1$H (DMSO-$d_6$) δ 2.60 (s, 3 H), 4.40 (s, 2 H), 6.24 (d, *J* = 2.0 Hz, 1H), 6.41 (m, 1H), 7.10 (d, *J*= 2 Hz, 1H), 7.18-7.24 (m, 2 H), 7.29-7.44 (m, 6 H), 12.66 (s, 1 H); MS: *m*/*z*: 418.5 [$M+H^+$].

Example 10: 4-Chloro-2-[2-(4-chloro-phenylsulfanyl)-benzylamino]-6-(1-hydroxy-ethyl)-phenol

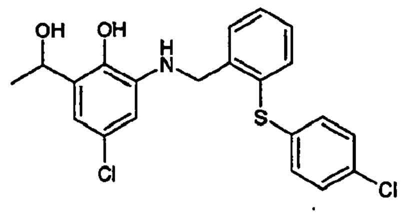

The compound was obtained in 34 % yield (23 mg) using the protocol described in method D. NMR-$^1$H (DMSO-$d_6$) δ 1.27 (d, *J* = 6.4 Hz, 3 H), 4.33 (s, 2 H), 4.97 (q, *J* = 6.4 Hz, 1 H), 5.96 .(d, *J* = 2.4 Hz, 1 H), 6.47 (d, *J* = 2.4 Hz, 1 H), 7.19-7.47 (m, 8 H), 8.69 (br. S, 1 H); MS: *m/z*: 420.7 [M+H$^+$].

Example 11: (3,5-Dichloro-2-hydroxy-phenyl)-carbamic acid phenyl ester

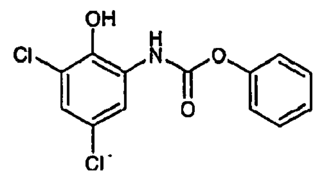

The compound was obtained in 26 % yield (11 mg) using the protocol described in method F.
NMR-$^1$H (DMSO-$d_6$) δ= 7.25 (m, 3H), 7.43 (m, 2 H), 7.50 (s, 2H)
NMR-$^3$C (DMSO-$d_6$) δ= 109.1, 118.8, 121.4, 128.2, 129.4, 132.6, 153.2, 157.3

Example 12: (3,5-Dichloro-2-hydroxy-4-methyl-phenyl)-carbamic acid phenyl ester

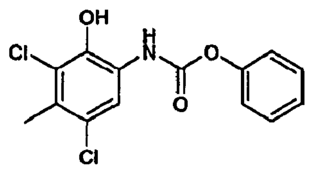

The compound was obtained in 31 % yield (24 mg) using the protocol described in method F.
NMR-$^1$H (DMSO-$d_6$) δ = 2.38 (s, 3 H), 6.76 (m, 3H), 7.15 (m, 3H)
NMR-$^{13}$C (DMSO-$d_6$) δ = 16.5, 109.1, 115.2, 118.8, 126.7, 128.5, 129.5, 129.7, 139.4, 153.4, 157.4

Example 13: (3,5-Dichloro-2-hydroxy-phenyl)-carbamic acid benzyl ester

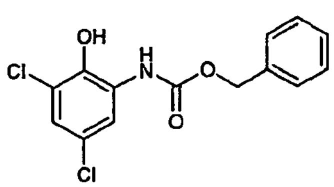

According to method F 2-amino-4,6-dichloro-phenol (25 mg, 1 eq) and phenylchloroformate (23 μL, 1 eq) gave 10.9 mg (26 %) of the title compound as a white solid.
NMR-$^1$H (DMSO-$d_6$) δ = 5.30 (d, 2 H), 7.43 (m, 2 H), 7.25 (m, 2H), 7.50 (s, 2H)

Example 14: (3,5-Dichloro-2-hydroxy-4-methyl-phenyl)-carbamic acid 2-isopropyl-5-methyl-cyclohexyl ester

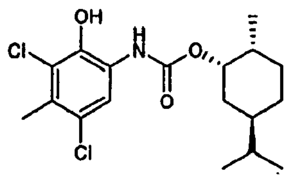

The compound was obtained in 39% yield (22 mg) using the protocol described in method G.
NMR-$^1$H (DMSO-$d_6$), δ = 0.84-0.90 (m, 3H), 1.22-1.41 (m, 6H), 2.35 (s, 3H), 3.28-3.33 (m, 2H), 4.06 (t, 2H), 7.57 (s, 1H), 8.74 (s, 1H).

Example 15: (3,5-Dichloro-2-hydroxy-phenyl)-carbamic acid 2-isopropyl-5-methylcyclohexyl ester

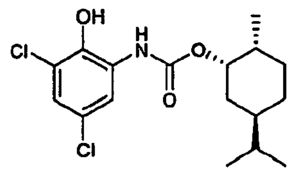

The compound was obtained in 54% yield (44 mg) using the protocol described in method G.
NMR-$^1$H (DMSO-$d_6$) δ = 0.75 (d, 3H), 0.82-093 (m, 7H), 0.96-1.14 (m, 2H), 1.29-1.53 (m, 2H), 1.59-1.71 (m, 2H), 1.88-2.02 (m, 2H), 4.54 (ddd, 1H), 7.47 (s, 2H), 9.67 (s, 1H), 9.73 (s, 1H)

Example 16: (3,5-Dichloro-2-hydroxy-4-methyl-phenyl)-carbamic acid hexyl ester

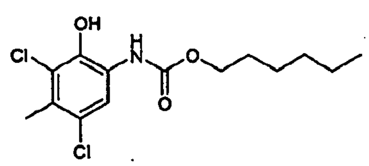

The compound was obtained in 21% yield (10 mg) using the protocol described in method G.
NMR-$^1$H (DMSO-$d_6$) δ = 0.87 (t, 3H), 1.25-1.40 (m, 6H), 1.55-1.66 (m, 2H), 2.35 (s, 3H), 4.06 (t, 2H), 7.56 (s, 1H), 8.74 (s, 1H), 9.73 (s, 1H).

Example 17: (3,5-Dichloro-2-hydroxy-phenyl)-carbamic acid hexyl ester

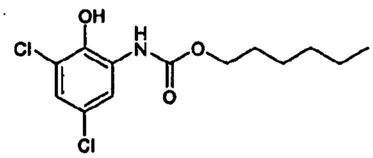

The compound was obtained in 43% yield (30 mg) using the protocol described in method G. NMR-$^1$H (DMSO-$d_6$) δ = 0.87 (t, 3H), 1.21-1.40 (m, 6H), 1.54-1.66 (m, 2H), 4.06 (t, 2H), 7.46 (s, 2H), 9.67 (s, 1H), 9.73 (s, 1H).

Example 18: 1-[3-Chloro-5-(3-cyclohexyl-ureido)-2-hydroxy-phenyl]-3-(1,1,3,3-tetramethyl-butyl)-urea

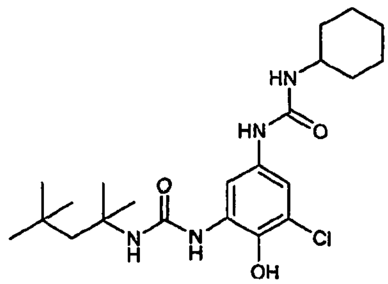

The compound was obtained in 31% yield (16 mg) using the protocol described in method J. NMR-$^1$H (DMSO-$d_6$) δ 1.06 (s, 9 H), 1.08-1.28 (m, 5 H), 1.35 (s, 6 H), 1.45-1.76 (m, 7 H), 3.30-3.42 (m, 1 H), 6.84 (s, 1 H), 7.24 (s, 1 H), 7.73 (s, 1 H), 8.20 (br. s, 1 H), 8.82 (s, 1 H), 8.97 (s, 1 H), 9.11 (s, 1 H); MS: *m*/*z*: 439.2 [M+H$^+$].

Example 19: *N*-[3-Chloro-5-(3-cyclohexyl-ureido)-2-hydroxy-phenyl]-2-nitro-benzenesulfonamide

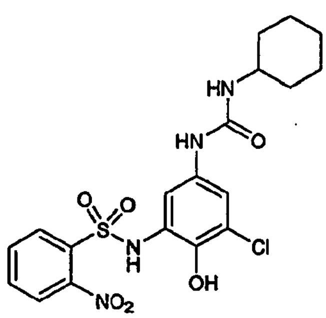

According to method J 1-(3-amino-5-chloro-4-hydroxy-phenyl)-3-cyclohexyl-urea (19 mg, 67 μmol) and 2-nitrobenzenesulfonyl chloride (15 mg, 67 μmol) gave *N*-[3-Chloro-5-(3-cyclohexyl-ureido)-2-hydroxy-phenyl]-2-nitro-benzenesulfonamide (15 mg, 48%).
NMR-$^1$H (DMSO-$d_6$) δ 1.05-1.37 (m, 5 H), 1.49-1.83 (m, 5 H), 3.42-3.47 (m, 1 H), 5.94 (d, *J* = 7.9 Hz, 1 H), 6.99 (d, *J* = 2.4 Hz, 1 H), 7.46 (d, *J* = 2.4 Hz, 1 H), 7.78-7.90 (m 2 H), 7.93-8.03 (m, 2 H), 8.27 (s, 1 H), 9.19 (br s, 1 H), 9.70 (s, 1 H); MS: *m*/*z*: 469.1 [M+H$^+$].

Example 20: 1-(3-Benzothiazol-2-yl-5-chloro-4-hydroxy-phenyl)-3-cyclohexyl-urea

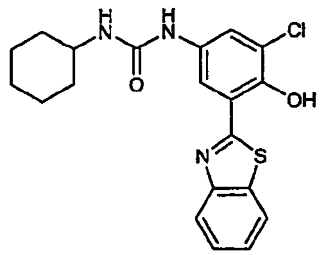

According to method K 4-amino-2-benzothiazol-2-yl-6-chloro-phenol (21 mg, 76 μmol) and isocyanato-cyclohexane (9.5 mg, 76 μmol) gave 1-(3-Benzothiazol-2-yl-5-chlom-4-hydroxyphenyl)-3-cyclohexyl-urea (3.0 mg, 10%). NMR-$^1$H (DMSO-$d_6$) δ 1.09-1.41 (m, 5 H), 1.49-1.87 (m, 5 H), 3.41-3.47 (m, 1H), 6.12 (d, *J* = 7.8 Hz, 1 H), 7.51 (t, *J* = 7.8 Hz, 1 H), 7.60 (t, *J* = 7.1 Hz, 1 H), 7.68 (d, *J* = 2.4 Hz, 1 H), 8.06 (d, *J* = 2.4 Hz, 1 H), 8.11 (d, *J* = 8.3 Hz, 1 H), 8.21 (d, *J* = 7.3 Hz, 1 H), 8.52 (s, 1 H), 11.92 (s, 1 H); MS: *m*/*z*: 402.2 [M+H$^+$].

Example 21: 1-(5-Benzothiazol-2-yl-3-chloro-2-hydroxy-phenyl)-3-(2-trifluoromethylphenyl)-thiourea

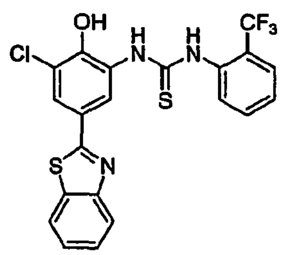

According to method K 2-Amino-4-benzothiazol-2-yl-6-chloro-phenol (20 mg, 72 μmol) and 1-isothiocyanato-2-trifluoromethyl-benzene (15 mg, 72 μmol) gave 1-(5-benzothiazol-2-yl-3-chloro-2-hydroxy-phenyl)-3-(2-trifluoromethyl-phenyl)-thiourea (12 mg, 35%).
NMR-$^1$H (DMSO-$d_6$) δ 7.40-7.56 (m, 3 H), 7.66-7.79 (m, 3 H), 7.92 (d, *J*= 2.4 Hz, 1 H), 8.03 (d, *J* = 7.8 Hz, 1 H), 8.12 (d, *J* = 8.8 Hz, 1 H), 8.68 (br s, 1 H), 9.65 (s, 1 H), 9.94 (s, 1 H), 10.65 (s, 1 H); MS: *m*/*z*: 480.0 [M+H$^+$].

Example 22: 1-(3,5-Dichloro-2-hydroxy-4-methyl-phenyl)-3-(4-trifluoromethyl-phenyl)-thiourea

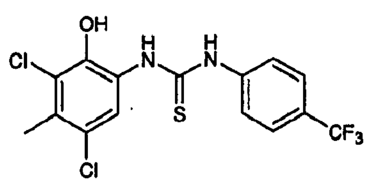

The compound was obtained in 57% yield (59 mg) using the protocol described in method L
NMR-$^1$H (DMSO-$d_s$) δ = 2.38 (s, 3 H), 7.68-7.85 (m, 5 H), 9.43 (s, 1 H), 9.90 (s, 1 H), 10.37 (s, 1 H); MS (*m*/*z*): 278.1 [M$^+$].

Example 23: 1-Adamantan-1-yl-3-(3,5-dichloro-4-hydroxy-phenyl)-urea

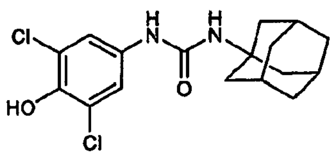

The compound was obtained in 60% yield (35 mg) using the protocol described in method A. NMR-$^1$H (DMSO-$d_6$) δ = 1.60 ($s_b$, 6H), 1.95 ($s_b$, 6H), 2.08 ($s_b$, 3H), 6.79 (s, 1H), 7.95 (s, 1H), 8.18 (s, 1H), 9.82 (s, 1H). NMR-$^{13}$C (DMSO-$d_6$) δ =17.2, 29.5, 36.1, 41.7, 50.3, 116.2, 122.2, 123.8, 124.8, 140.6, 154.3; MS (*m*/*z*): 369.2 [M+H$^+$].

Example 24: 1-(3,5-Dichloro-4-hydroxy-phenyl)-3-(1,1,3,3-tetramethyl-butyl)-urea

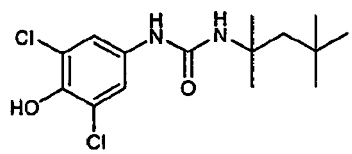

To a solution of 4-amino-2,6-dichloro-phenol (44.5 mg, 1 eq) in dioxan (2.0 mL) was added 2-isocyanato-2,4,4-trimethyl-pentane (38 mg, 1 eq) in one portion. The solution was stirred at room temperature for 3 h. The solution was diluted with 2 mL DCM and scavenger resins (tris-(2-amimoethyl)-amine polystyrene (3 eq), methylisocyanate polystyrene (3 eq) and N-(2-mercaptoethyl)aminomethyl polystyrene (3 eq)) were added. After 18 h at 35˚C the solution was filtered off and the solvent was removed under reduced pressure. The crude material was purified by HPLC to obtain 67 mg (81 %) of the title compound as a white powder.

NMR-$^1$H (DMSO-$d_6$) δ = 0.95 (s, 9H), 1.28 (s, 6H), 1.69 (s, 2H), 5.90 (s, 1H), 7.33 (s, 2H), 8.22 (s, 1H), 9.50 ($s_b$, 1H).
NMR-$^{13}$C (DMSO-$d_6$) δ = 29.9, 31.2, 50.9, 54.2, 117.3, 121.4, 134.0, 142.8, 152.9; MS (m/z): 333.2 [M+H$^+$].

Example 25: 1-(3,5-Dichloro-4-hydroxy-phenyl)-3-(4-trifluoromethyl-phenyl)-thiourea

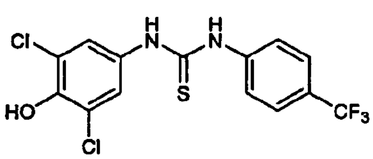

The compound was obtained in 58% yield (34.8 mg) using the protocol described in method I.
NMR-$^1$H (DMSO-$d_6$) δ = 6.32 (m, 2 H), 6.80 (m, 2 H), 7.42 (m, 2 H), MS (*m*/*z*): 381.2 [M$^+$].

Example 26: Propane-2-sulfonic acid (3,5-dichloro-2-hydroxy-4-methyl-phenyl)-amide

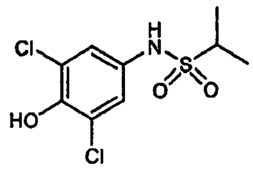

The compound was obtained in 40% yield (21 mg) using the protocol described in method B. NMR-$^1$H (DMSO-$d_6$) δ = 2.43 (d, 6H), 2.51 (s, 3H), 3.97 (m, 1H), 5.43 (s, 1H), 6.8 (s, 1H); MS (*m*/*z*): 284.1 [$MH^+$].

Example 27: *N*-(3,5-Dichloro-4-hydroxy-phenyl)-2-phenyl-acetamide

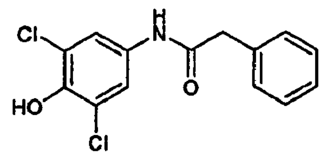

The compound was obtained in 22% yield (11.0 mg) using the protocol described in method C. NMR-$^1$H (DMSO-$d_6$) δ = 3.12 (s, 2H), 7.24 (m, 1H), 7.36 (m, 2H), 7.37 (m, 2H), 7.61 (m, 2H), 9.91 (m, 1H); MS (*m*/*z*): 296.2 [$MH^+$].

Example 28: 2,6-Dichloro-4-(3-methyl-benzylamino)-phenol

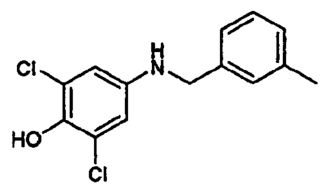

The compound was obtained in 55% yield (15.0 mg) using the protocol described in method D. NMR-$^1$H (DMSO-$d_6$) δ 2.26 (s, 3 H), 4.29 (s, 2 H), 5.92 (s, 1 H), 6.50 (s, 1 H), 7.00-7.28 (m, 4 H), 9.20 (s, 1 H); MS (*m*/*z*): 282.2 [$M+H^+$].

Example 29: (3,5-Dichloro-4-hydroxy-phenyl)-carbamic acid phenyl ester

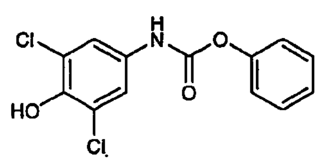

The compound was obtained in 49% yield (55.7 mg) using the protocol described in method F.
NMR-$^1$H (DMSO-$d_6$) δ = 7.22 (m, 2H), 7.36 (m, 2 H), 7.50 (s, 2H)
NMR-$^{13}$C (DMSO-<$d_6$) δ = 118.5, 121.9, 122.5, 125.5, 129.4, 131.7, 144.7, 150.3, 151.6

Example 30: (3-Chloro-4-hydroxy-phenyl)-carbamic acid phenyl ester

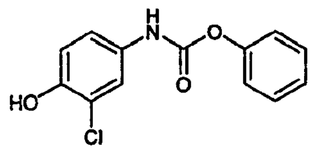

The compound was obtained in 46% yield (32.1 mg) using the protocol described in method F. NMR-$^1$H (DMSO-$d_6$) $\delta$ = 2.38 (s, 3 H), 6.76 (s, 1H), 7.15 (m, 5H), 7.32 (m, 2 H). NMR-$^{13}$C (DMSO-$d_6$) $\delta$ = 16.5, 109.1, 115.2, 118.8, 126.7, 128.5, 129.5, 129.7, 139.4, 153.4, 157.4

Example 31: (3,5-Dibromo-4-hydroxy-phenyl)-carbamic acid phenyl ester

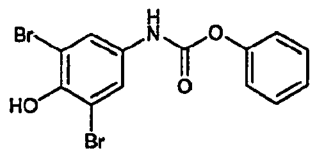

The compound was obtained in 25 % yield (10.2 mg) using the protocol described in method F. NMR-$^1$H (DMSO-$d_6$) $\delta$ = 7.33 (m, 2 H), 7.45 (m, 2H), 7.50 (s, 2H)

Example 32: (3,5-Dichloro-4-hydroxy-phenyl)-carbamic acid 2-isopropyl-5-methylcyclohexyl ester

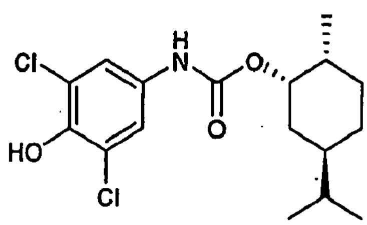

According to method G 4-amino-2,6-dichloro-phenol (40 mg, 1 eq) and (-)-menthylchloroformate (54 μl, 1.1 eq) in DCM gave 44.1 mg (54 %) of the title compound as a white solid. NMR-$^1$H (DMSO-$d_b$) $\delta$ = 0.71 (d, 3H), 0.78-0.96 (m, 7H), 0.99-1.16 (m, 2H), 1.29-1.58(m, 2H), 1.59-1.71 (m, 2H), 2.0-2.15 (m, 2H), 4.58 (m, 1H), 7.49 (s, 2H), 9.68 (s, 1H), 9.73 (s, 1H)

Example 33: (3,5-Dichloro-4-hydroxy-phenyl)-carbamic acid hexyl ester

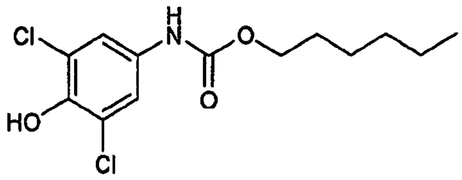

According to method G 4-amino-2,6-dichloro-phenol (40 mg, 1 eq) and hexylchloroformate (41 μL, 1.1 eq) gave 30 mg (43 %) of the title compound as a white solid.
NMR-$^1$H (DMSO-$d_6$) δ = 0.75 (m, 3H), 1.21-1.40 (m, 6H), 1.48-1.62 (m, 2H), 4.22 (m, 2H), 7.32 (s, 2H), 9.69 (s, 1H), 9.75 (s, 1H).

Example 34: 1-[3-Chloro-5-(3-cyclohexyl-ureido)-2-hydroxy-phenyl]-3-(2-trifluoromethylphenyl)-urea

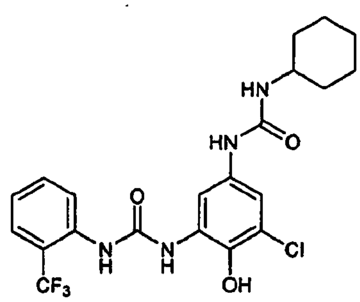

According to method J 1-(3-amino-5-chloro-4-hydroxy-phenyl)-3-cyclohexyl-urea (30 mg, 0.11 mmol) and 1-isocyanato-2-trifluoromethyl-benzene (21 mg, 0.11 mmol) gave 1-[3-chloro-5-(3-cyclohexyl-ureido)-2-hydroxy-phenyl]-3-(2-trifluoromethyl-phenyl)-urea (16 mg, 31%).
NMR-$^1$H (DMSO-$d_6$) δ 0.98-1.28 (m, 5 H), 1.39-1.76 (m, 5 H), 3.30-3.42 (m, 1 H), 5.84 (d, *J* = 7.8 Hz, 1 H), 7.24 (d, *J* = 7.8 Hz, 1 H), 7.31 (d, *J* = 2.4 Hz, 1 H), 7.52-7.67 (m, 2 H), 7.71-7.75 (m, 2 H), 8.20 (br. s, 1 H), 8.80-8.82 (m, 1 H), 8.97-9.04 (m, 1 H), 9.11 (s, 1 H); MS: *m/z*: 471.1 [M+H$^+$].

Example 35: Specificity of inhibition of certain enzymes by compounds according to the present invention

In order to characterize the specificity of various compounds the following assays were performed. PPIase activity of hPin1, hCyp18, LpCyp18, hFKBP12 and EcParvulin was measured using the protease-coupled PPIase assay according to Fischer et al. (Fischer, G.; Bang, H.; Mech, C. Determination of enzymatic catalysis fort he cis-trans-isomerization of peptide binding in proline-containing peptides. [German] Biomed. Biochem. Acta 1984, 43, 1101-1111; Hennig et al., Selective Inactivation of Parvulin-like peptidyl-prolyl cis/trans isomerases by Juglon, Biochemistry. 1998, 37(17):5953-5960). For hPin1 measurements Ac-Ala-Ala-Ser($PO_3H_2$) -Pro-Arg-pNA was used as a substrate and trypsin (final concentration 190 μg/ml) as an isomer-specific protease. Activity measurements of other PPIases were made with the substrate peptide Suc-Ala-Phe-Pro-Phe-pNA and the protease -chymotrypsin (final concentration 470 μg/ml). The assays were performed in a final reaction volume of 150 μl at final concentrations of 6 nM hPin1, 10 nM hCyp18, 5 nM LpCyp18, 20 nM EcParvulin and 20 nM hFKBP12, respectively, and 120 μM substrate peptide in 35 mM HEPES (pH 7.8). For inhibition experiments 100-0.01 μM of effector freshly diluted from a DMSO stock solution were added. The amount of solvent was kept constant within each experiment, usually below 0.3% (v/v). All reactions were started by addition of protease. The test was performed by observing the released 4-nitroaniline at 390 nm with a MR5000 UV/Vis spectrophotometer (Dynex) at 6°C. Data were evaluated by calculation of pseudo-first-order rate constants $k_{obs}$ in presence of PPIase and PPIase/effector, respectively, and corrected for the contribution of the non-catalyzed reaction ($k_0$). Inhibition constants $IC_{50}$ were calculated using SigmaPlot 8.0 (SPSS).
The following target enzymes which are all rotamases belonging to different classes of rotamases were used:

T-1: Protein interacting with NIMA (-kinase), hPin1
T-2: First described human Rapamycin receptor, hFKBP12

T-3: Human Cyclosporin A receptor with 18 kDa molecular weight, hCyp18
T-4: Leishmonia pneumophila virulence Cyclosporin A receptor with 18 kDa molecular weight, LpCyp18
T-5: Bacterial Juglon sensitive non proteolytic enzyme, EcParv

These rotamases are known in the art. Their production and characteristics may be taken from the following references.

**Review about all PPIase families**

Gothel, S. F.; Marahiel, M. A. TI Peptidyl-prolyl cis-trans isomerases, a superfamily of ubiquitous folding catalysts [Review]. Cell. Molec. Life Sci. 1999, 55, 423-436

**Pin1**

Lu, K. P.; Hanes, S. D.; Hunter, T. (1996) A human peptidyl-prolyl isomerase essential for regulation of mitosis. Nature 1996, 380, 544-547

Yaffe, M. B.; Schutkowski, M.; Shen, M. H.; Zhou, X. Z.; Stukenberg, P. T.; Rahfeld, J. U.; Xu, J.; Kuang, J.; Kirschner, M. W.; Fischer, G.; Cantley, L. C.; Lu K. P. SEQUENCE-SPECIFIC AND PHOSPHORYLATION-DEPENDENT PROLINE ISOMERIZATION - A POTENTIAL MITOTIC REGULATORY MECHANISM. Science 1997, 278, 1957-1960
Shen, M.; Stukenberg, P. T.; Kirschner, M. W.; Lu, K. P. The essential mitotic peptidyl-prolyl isomerase Pinl binds and regulates mitosis-specific phosphoproteins. Genes Developm. 1998, 12, 706-720.

**EcParvulin**

Rahfeld JU. Schierhorn A. Mann K. Fischer G. A novel peptidyl-prolyl cis/trans isomerase from Escherichia coli. FEBS Letters. 1994, 343, 65-69

Rahfeld JU. Rucknagel KP. Schelbert B. Ludwig B. Hacker J. Mann K. Fischer G. Confirmation of the existence of a third family among peptidyl-prolyl cis/trans isomerases. Amino acid sequence and recombinant production of parvulin. FEBS Letters. 1994, 352, 180-184

**FKBPs (including FKBP12) and Cyclophilins (including Cyp18)**

For recent reviews on cyclophilins and FKBPs and their effectors, see: (a) Fischer, G. Peptidyl-prolyl cis/trans isomerases and their effectors. Angew. Chem., Int. Ed. Engl. 1994, 33, 1415-1436. (b) Galat, A.; Metcalfe, S. M. Peptidylproline cis/trans isomerases. Prog. Biophys. Molec. Biol. 1995, 63, 67-118.

**LpCyp18**

Schmidt B. Tradler T. Rahfeld JU. Ludwig B. Jain B. Mann K. Rucknagel KP. Janowski B. Schierhorn A. Kullertz G. Hacker J. Fischer G. A cyclophilin-like peptidyl-prolyl cis/trans isomerase from Legionella pneumophila--characterization, molecular cloning and overexpression. Mol. Microbiol. 1996, 21,1147-1160

In order to cluster the various rotamase inhibitors the following classes were defined with "A" indicating the most potent rotamase inhibitor.

A: IC50 < 1 μM
B: 1 μM < IC50 < 10 μM
C: 10 μM < IC50 < 50 μM
D: 50 μM < IC50 < 100 μM
E: IC50 > 100 μM

Table 2

Specificity of the inhibition with rotamases

A: IC50 < 1 μM
B: 1 μM < IC50 < 10 μM
C: 10 μM < IC50 < 50 μM
D: 50 μM < IC50 < 100 μM
E: IC50 > 100 μM

Table 2

| Specificity of the inhibition with rotamases | | | | | | |
|---|---|---|---|---|---|---|
| **Compound** | **N°** | **Target** | | | | |
| | | **T-1** | **T-2** | **T-3** | **T-4** | **T-5** |
| 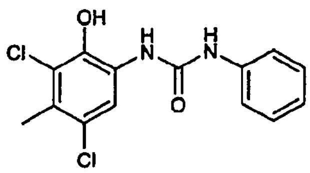 | 120 | C | D | C | E | - |
| 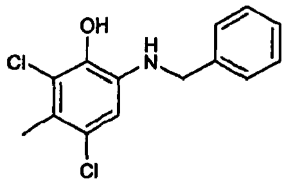 | 655 | C | E | E | E | C |
| 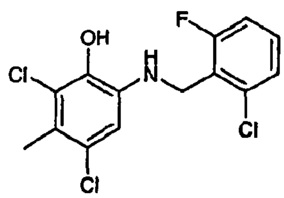 | 512 | C | E | D | E | C |
| 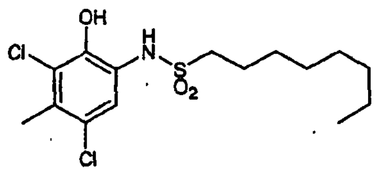 | 563 | B | E | E | E | E |
| 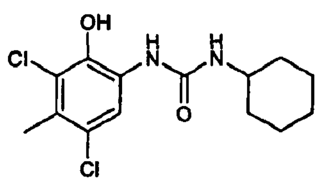 | 109 | A | E | C | E | C |
| 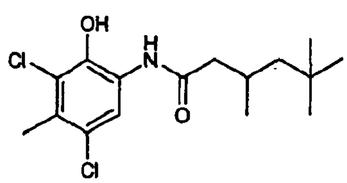 | 599 | B | E | E | D | C |
| 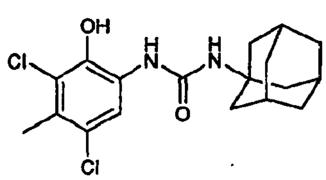 | 118 | B | D | C | C | B |
| 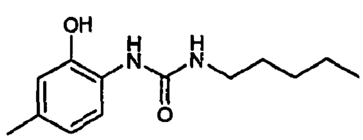 | 643 | C | E | E | E | E |

(continued)

| Specificity of the inhibition with rotamases | | | | | | |
|---|---|---|---|---|---|---|
| **Compound** | **N°** | **Target** | | | | |
| | | **T-1** | **T-2** | **T-3** | **T-4** | **T-5** |
| 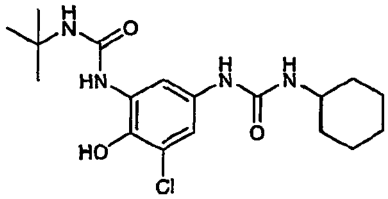 | 605 | A | E | C | E | B |
| 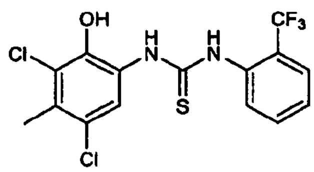 | 266 | A | E | E | E | B |
| 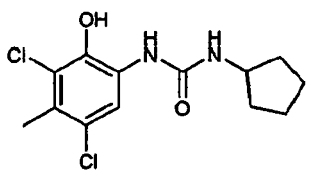 | 629 | A | E | C | E | B |
| 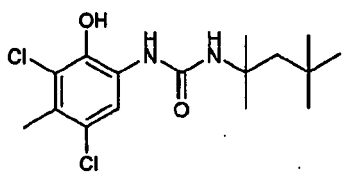 | 102 | B | E | C | C | B |
| 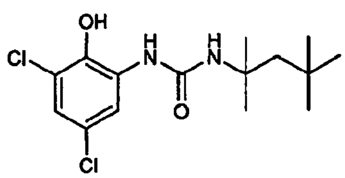 | 30 | B | E | E | E | E |
| 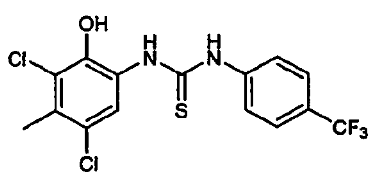 | 264 | A | - | - | - | - |
| 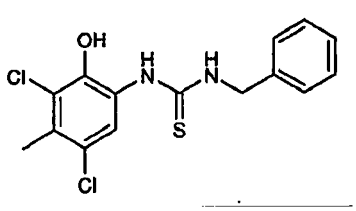 | 254 | A | - | - | - | - |

(continued)

| Specificity of the inhibition with rotamases | | | | | | |
|---|---|---|---|---|---|---|
| Compound | N° | Target | | | | |
| | | T-1 | T-2 | T-3 | T-4 | T-5 |
| 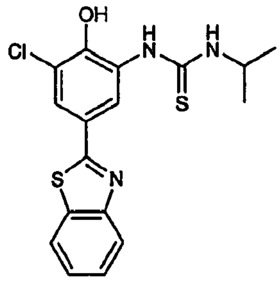 | 639 | A | - | - | - | - |
| 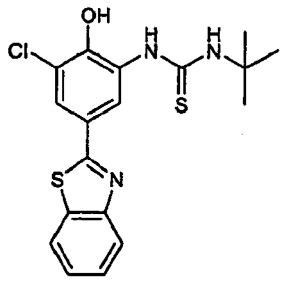 | 640 | B | - | - | - | - |
| 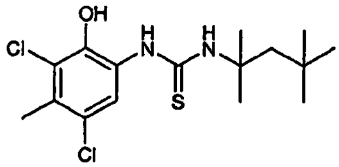 | 257 | A | - | - | - | - |
| 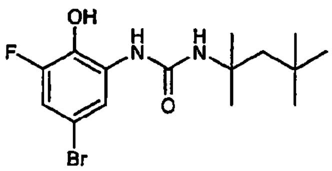 | 126 | A | - | - | - | - |
| 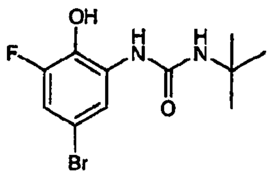 | 127 | B | - | - | - | - |
| 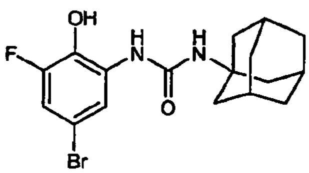 | 142 | A | - | - | - | - |
| 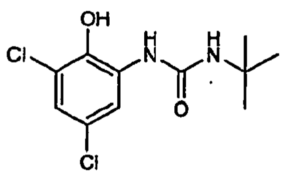 | 31 | A | - | - | - | - |

(continued)

| Specificity of the inhibition with rotamases | | | | | | |
|---|---|---|---|---|---|---|
| **Compound** | **N°** | **Target** | | | | |
| | | **T-1** | **T-2** | **T-3** | **T-4** | **T-5** |
| 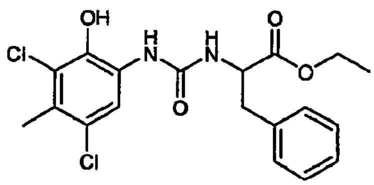 | 593 | B | - | - | - | - |
| 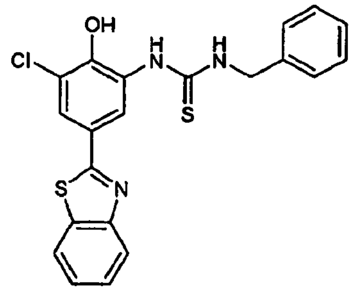 | 637 | B | - | - | - | - |
| 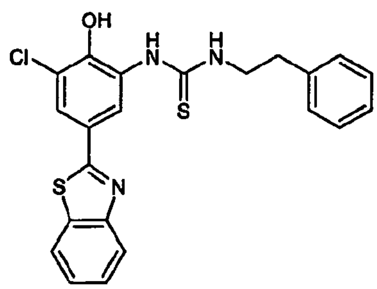 | 638 | B | - | - | - | - |
| 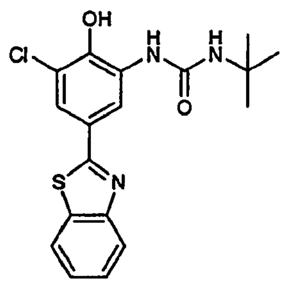 | 636 | A | - | - | - | - |
| 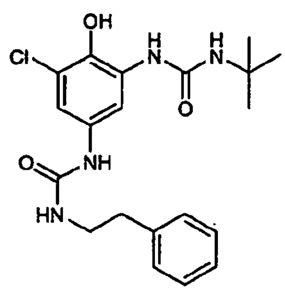 | 622 | A | - | - | - | - |

(continued)

| Specificity of the inhibition with rotamases | | | | | | |
|---|---|---|---|---|---|---|
| Compound | N° | Target | | | | |
| | | T-1 | T-2 | T-3 | T-4 | T-5 |
| 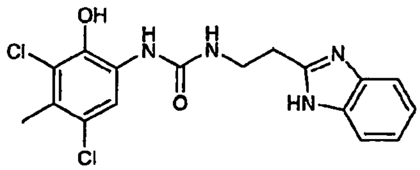 | 656 | A | - | - | - | - |
| 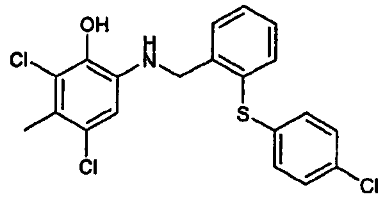 | 371 | A | E | C | E | C |
| 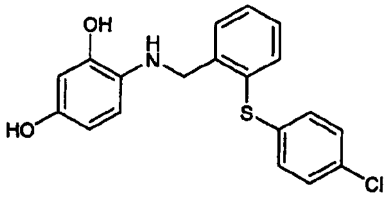 | 672 | A | C | B | C | C |
| 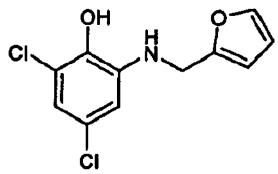 | 647 | B | E | E | E | E |
| 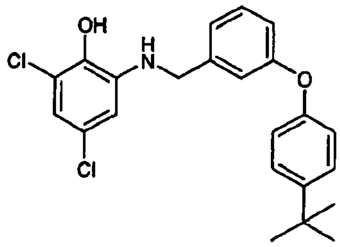 | 483 | B | E | C | E | D |
| 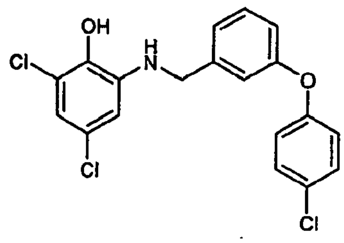 | 482 | B | E | C | E | E |
| 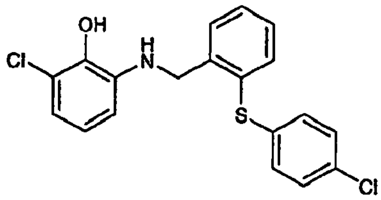 | 343 | B | E | C | E | E |

(continued)

| Specificity of the inhibition with rotamases | | | | | | |
|---|---|---|---|---|---|---|
| **Compound** | **N°** | **Target** | | | | |
| | | **T-1** | **T-2** | **T-3** | **T-4** | **T-5** |
| 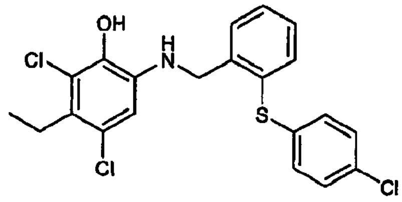 | 441 | B | E | C | E | E |
| 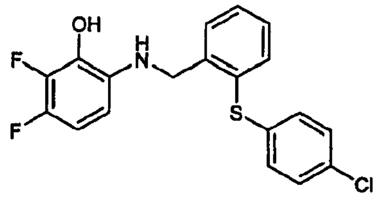 | 399 | A | E | D | D | C |
| 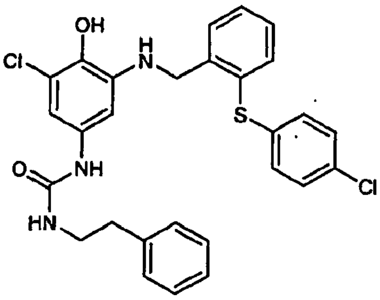 | 610 | A | - | - | - | - |
| 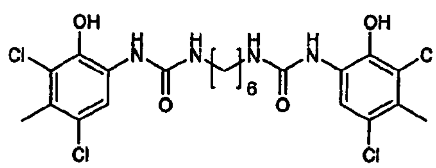 | 673 | A | - | - | - | - |
| 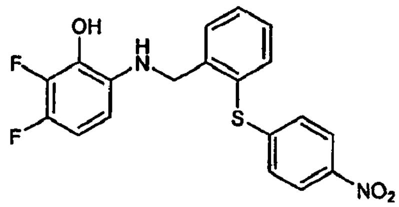 | 400 | A | - | - | - | - |
| 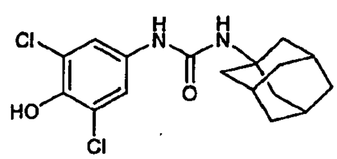 | 719 | A | E | D | C | - |
| 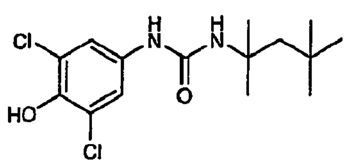 | 703 | A | E | E | E | B |

(continued)

| Specificity of the inhibition with rotamases | | | | | | |
|---|---|---|---|---|---|---|
| Compound | N° | Target | | | | |
| | | T-1 | T-2 | T-3 | T-4 | T-5 |
| 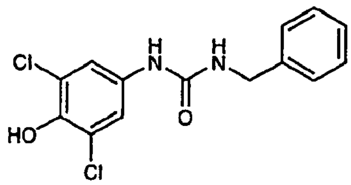 | 700 | A | E | E | E | C |
| 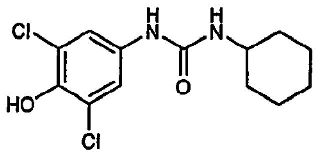 | 710 | A | E | E | E | E |
| 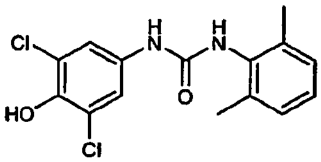 | 716 | B | E | E | E | C |
| 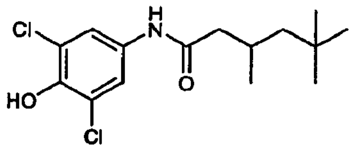 | 1273 | A | E | E | E | B |
| 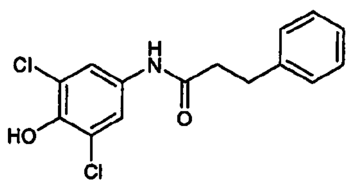 | 1293 | B | E | E | E | C |
| 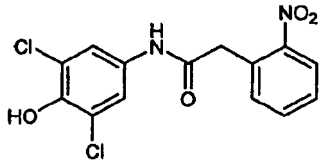 | 1294 | A | E | E | E | B |
| 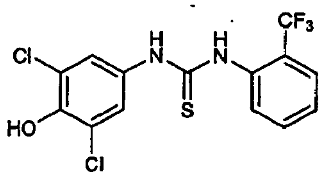 | 894 | A | E | E | E | B |
| 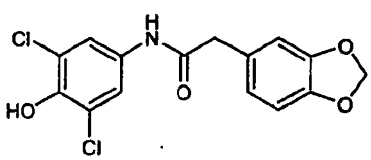 | 1295 | B | E | E | E | B |

(continued)

| Specificity of the inhibition with rotamases | | | | | | |
|---|---|---|---|---|---|---|
| Compound | N° | Target | | | | |
| | | T-1 | T-2 | T-3 | T-4 | T-5 |
| 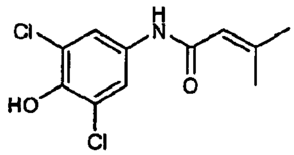 | 1296 | B | E | C | E | C |
| 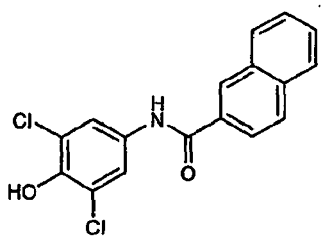 | 1297 | A | E | E | E | C |
| 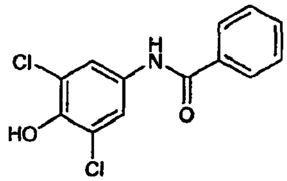 | 1298 | A | E | E | E | C |
| 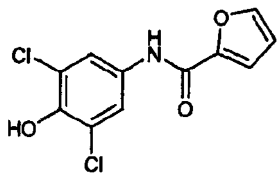 | 1299 | A | E | A | E | C |
| 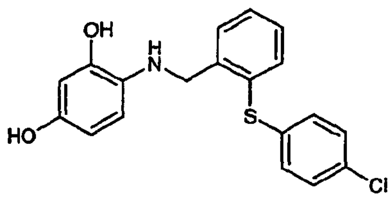 | 1044 | A | C | C | B | C |

As may be taken from the above table 2 the following compounds 24, 88, 89, 110, 169, 170, 298, 342, 344, 377, 378, 700, 703, 710, 894, 1273, 1294, 1297 are of class A and are thus extremely specific for hPin1.

Example 36: Specificity of inhibition of proteases

In order to investigate the impact of some of the inventive compounds on the activity of key proteases the following assay was performed: Protease activities were measured spectrophotometrically at 30°C according to Schomburg and Salzmann (Schomburg, B.; Salzmann M. GBF: Enzyme Handbook. Springer Verlag, Berlin Heidelberg, 1991) and Bergmeyer et al. (Bergmeyer, H. U.; Bergmeyer, J.; Graß1, M. Methods of Enzymatic Analysis, Vol. V Enzymes 3: Peptides, Proteinases and Their Inhibitors, pp 55 - 371, VCH, Weinheim, 1988). The release of 4-nitroaniline was determined at 390 nm with a Spectramax Plus UV/Vis spectrophotometer (Molecular Devices). The cathepsin B assay was performed in a reaction mixture containing 0.2 μg/ml cathepsin B, 2 mM Z-Arg-Arg-pNA in 88 mM $KH_2PO_4$, 12 mM $Na_2HPO_4$, 1.33 mM EDTA, 0.03% Brij 35 (pH 5.8). The trypsin assay was carried out in a reaction mixture containing 0.1 μg/ml trypsin and 120 μM Ac-Ala-Ala-Ser($PO_3H_2$) -Pro-Arg-pNA in 35 mM HEPES (pH 7.8) and the papain assay in a mixture consisting of 16 μg/ml papain and 2 mM Bz-DL-Arg-pNA in 10 mM $Na_2HPO_4$, 2 mM L-Cys, 5 mM EDTA (pH 6.5). In general, reactions were started by addition of peptide substrate after a 30 min incubation of 1-100 μM effector

with given concentrations of enzyme.

The key proteases used were the following:

T-6: Papain
T-7: Trypsin
T-8: Cathepsin

In order to cluster the various compounds the following classes of activity were defined.

A: IC50 < 1 μM
B: 1 μM < IC50 < 10 μM
C: 10 μM < IC50 < 50 μM
D: 50 μM < IC50 < 100 μM
E: IC50 > 100 μM

Table 3

| Specificity of the inhibition of some proteases | | | | |
|---|---|---|---|---|
| **Compound** | **N°** | **Target** | | |
| | | **T-6** | **T-7** | **T-8** |
| 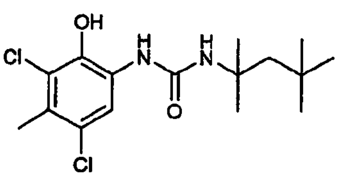 | 102 | D | C | D |
| 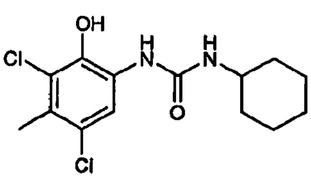 | 109 | E | - | - |
| 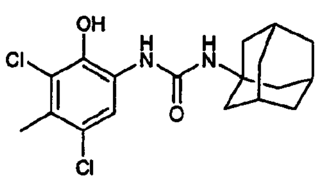 | 118 | E | - | - |
| 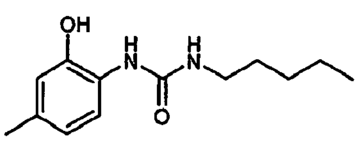 | 263 | E | - | - |
| 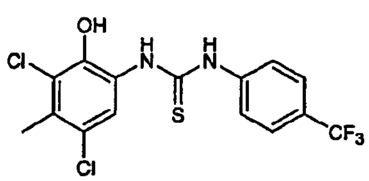 | 264 | E | E | E |
| 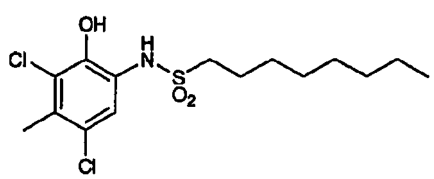 | 563 | E | - | - |

(continued)

| Specificity of the inhibition of some proteases | | | | |
|---|---|---|---|---|
| **Compound** | **N°** | **Target** | | |
| | | **T-6** | **T-7** | **T-8** |
| 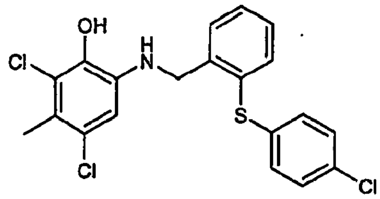 | 371 | E | - | - |
| 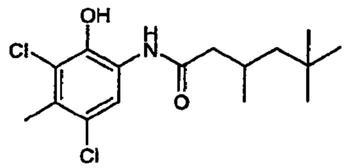 | 599 | E | - | D |
| 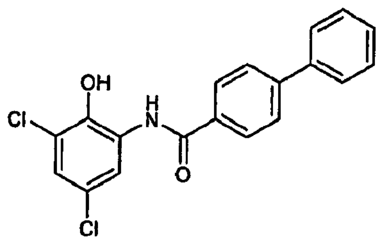 | 645 | E | - | - |
| 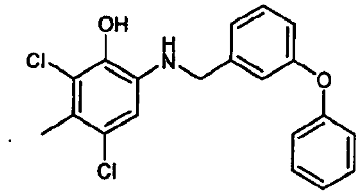 | 497 | E | - | C |
| 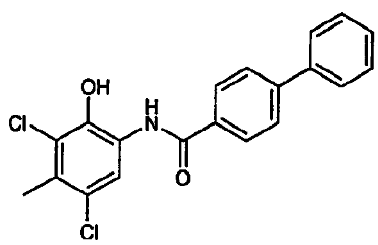 | 644 | E | - | - |
| 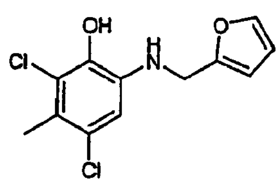 | 646 | E | - | E |
| 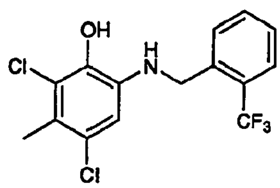 | 649 | E | - | D |

(continued)

| Specificity of the inhibition of some proteases | | | | |
|---|---|---|---|---|
| Compound | N° | Target | | |
| | | T-6 | T-7 | T-8 |
| 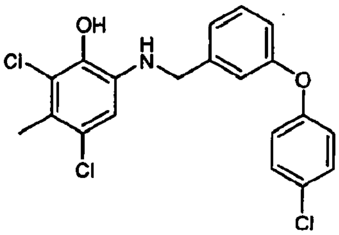 | 498 | E | - | - |
| 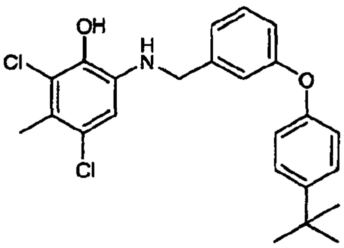 | 499 | E | - | - |
| 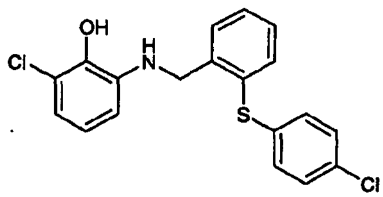 | 343 | E | - | - |
| 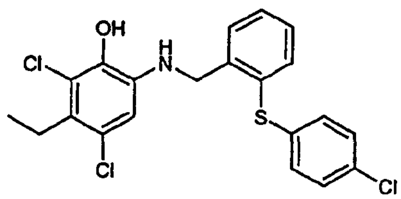 | 441 | E | - | - |
| 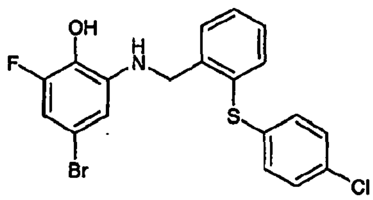 | 385 | E | - | - |
| 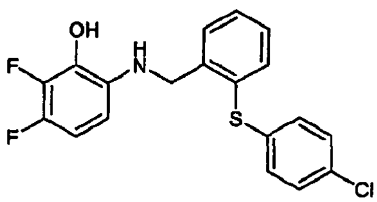 | 399 | E | - | C |
| 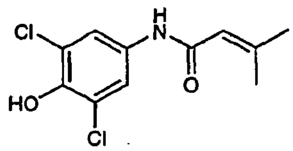 | 1296 | E | E | E |

(continued)

| Specificity of the inhibition of some proteases | | | | |
|---|---|---|---|---|
| **Compound** | **N°** | **Target** | | |
| | | **T-6** | **T-7** | **T-8** |
| 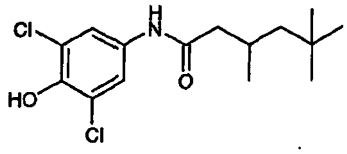 | 1273 | E | E | E |
| 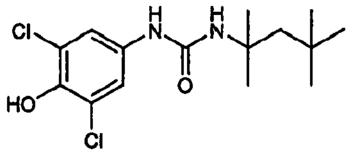 | 703 | E | D | E |
| 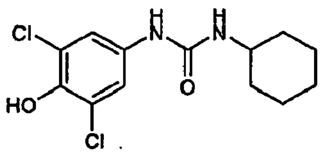 | 710 | E | - | - |
| 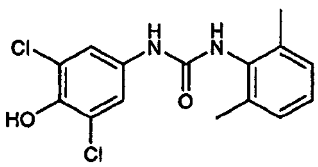 | 716 | E | - | - |

As may be taken from table 3 none of the tested compound is a strong inhibitor of any of the key proteases tested.

Example 37: Cytotoxic effects on tumor cell lines

In order to show that the compounds according to the present invention are actually useful in the treatment of tumors, the cytotoxic effects of some of said compounds on tumor cell lines were determined.

For this cytotoxic evaluation of the compounds the commercial available WST-1 assay (Roche) was used according to the manufacturer's instructions. The assay is based on the cleavage of the tetrazolium salt WST-1 by mitochondrial dehydrogenases found in viable cells. In general compounds were added to cells cultured in 96-well plates at 37°C. After 48 h of incubation 10 µl of WST-1 solution was added. The formazan dye was analyzed with an ELISA plate reader at (450 vs. 620) nm.

The following tumor cell lines were used in this assay:

CL-1: human acute myeloid leukemia, HL-60
CL-2: human cervix carcinoma, HeLa
CL-3: human prostate carcinoma, PC-3
CL-4: human colon adenocarcinoma, Caco-2
CL-5: human breast adenocarcinoma, MCF-7

In order to cluster the efficacy of the various compounds the following classes in terms of EC50 were defined.

A: EC50 < 10 µM
B: 10 µM < EC50 < 50 µM
C: 50 µM < EC50 < 100 µM
D: 100 µM < EC50 < 200 µM
E: EC50 > 200 µM

Table 4

| Cytotoxic effects on tumor cell lines | | | | | | |
|---|---|---|---|---|---|---|
| Compound | N° | Target | | | | |
| | | CL-1 | CL-2 | CL-3 | CL-4 | CL-5 |
| 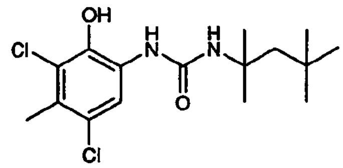 | 102 | A | A | A | A | A |
| 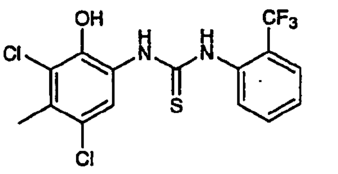 | 261 | A | B | B | B | B |
| 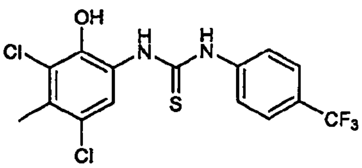 | 264 | A | A | A | A | A |
| 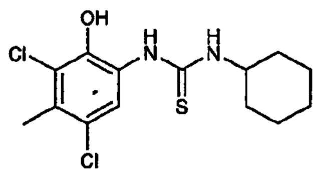 | 109 | B | A | B | - | A |
| 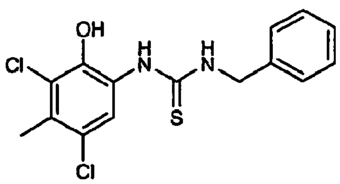 | 254 | B | B | B | - | B |
| 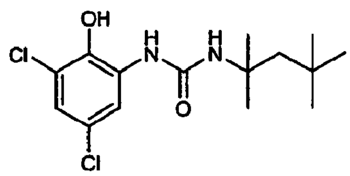 | 30 | A | - | - | A | - |
| 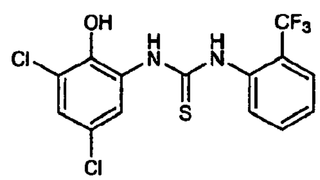 | 221 | B | - | - | - | B |
| 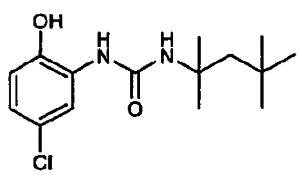 | 6 | B | - | - | - | A |

(continued)

| Cytotoxic effects on tumor cell lines | | | | | | |
|---|---|---|---|---|---|---|
| Compound | N° | Target | | | | |
| | | CL-1 | CL-2 | CL-3 | CL-4 | CL-5 |
| 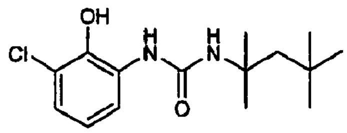 | 54 | B | - | - | - | A |
| 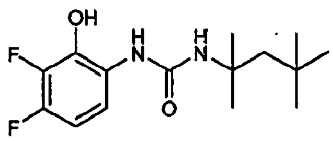 | 150 | B | - | - | - | A |
| 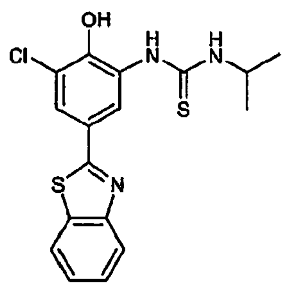 | 639 | A | - | - | - | B |
| 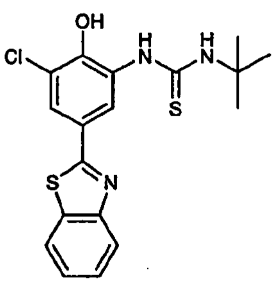 | 640 | B | - | - | - | B |
| 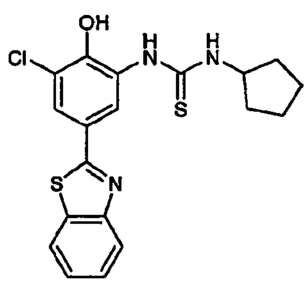 | 653 | A | - | - | - | B |
| 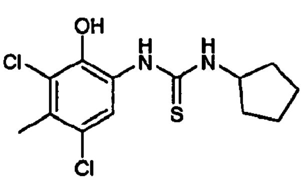 | 629 | A | A | A | - | A |

(continued)

| Cytotoxic effects on tumor cell lines | | | | | | |
|---|---|---|---|---|---|---|
| Compound | N° | Target | | | | |
| | | CL-1 | CL-2 | CL-3 | CL-4 | CL-5 |
| 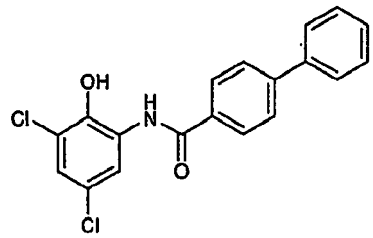 | 645 | A | A | A | - | A |
| 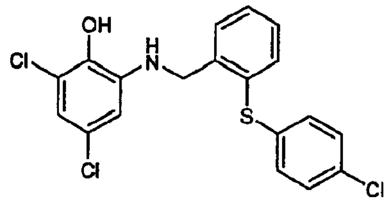 | 329 | B | B | B | - | B |
| 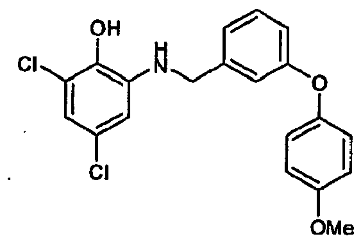 | 493 | B | A | A | - | - |
| 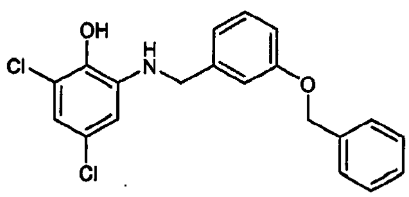 | 484 | B | A | A | - | - |
| 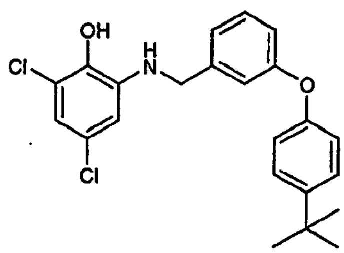 | 483 | - | B | B | - | - |
| 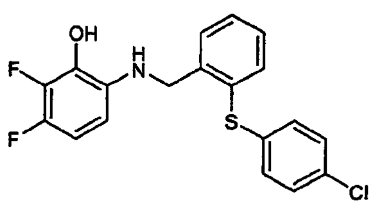 | 399 | A | A | A | - | A |

(continued)

| Cytotoxic effects on tumor cell lines | | | | | | |
|---|---|---|---|---|---|---|
| **Compound** | **N°** | **Target** | | | | |
| | | **CL-1** | **CL-2** | **CL-3** | **CL-4** | **CL-5** |
| 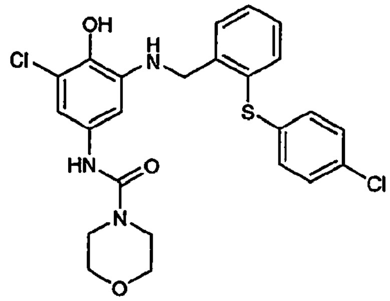 | 654 | - | B | - | - | A |
| 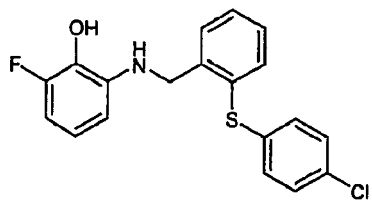 | 357 | A | A | A | - | - |
| 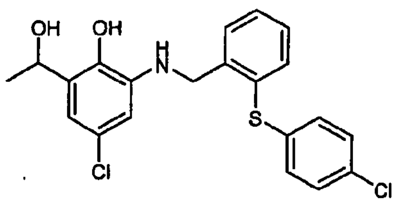 | 413 | B | A | B | - | - |
| 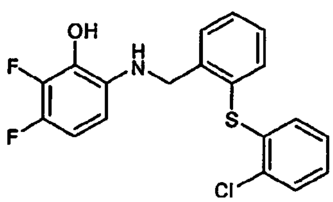 | 402 | B | B | B | - | - |
| 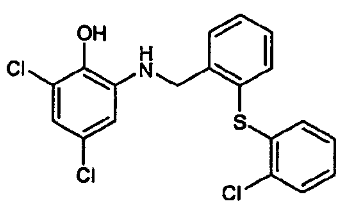 | 332 | B | B | B | - | - |
| 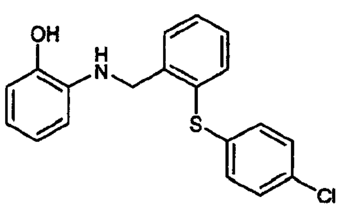 | 630 | B | A | A | - | - |

(continued)

| Cytotoxic effects on tumor cell lines | | | | | | |
|---|---|---|---|---|---|---|
| Compound | N° | Target | | | | |
| | | CL-1 | CL-2 | CL-3 | CL-4 | CL-5 |
| 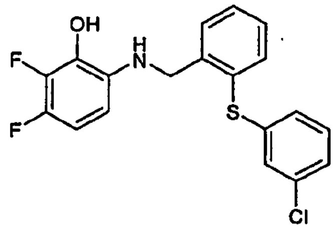 | 403 | B | A | A | - | - |
| 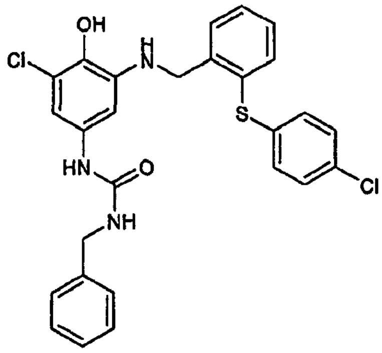 | 631 | A | - | B | - | - |
| 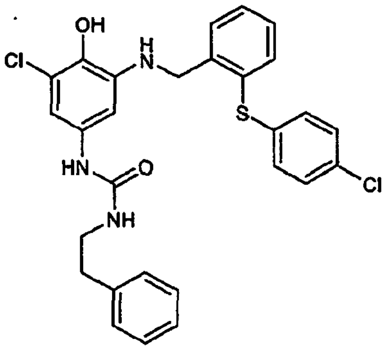 | 632 | A | - | B | - | - |
| 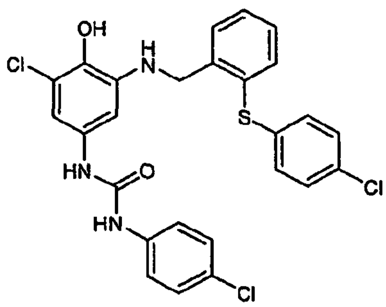 | 633 | A | - | A | - | - |
| 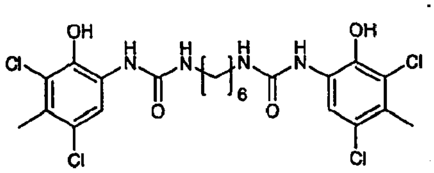 | 673 | - | - | - | - | A |

(continued)

| Cytotoxic effects on tumor cell lines | | | | | | |
|---|---|---|---|---|---|---|
| Compound | N° | Target | | | | |
| | | CL-1 | CL-2 | CL-3 | CL-4 | CL-5 |
| 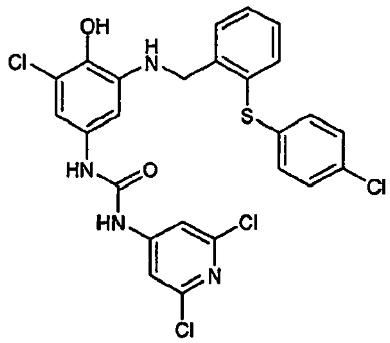 | 652 | A | - | A | - | - |
| 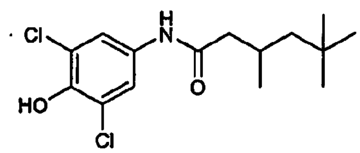 | 1273 | B | C | B | B | - |
| 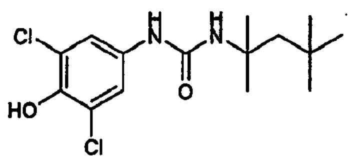 | 703 | B | B | B | B | - |
| 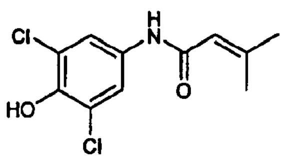 | 1296 | C | - | - | - | - |
| 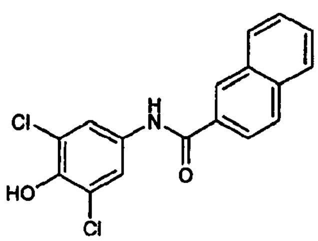 | 1297 | C | - | - | - | - |
| 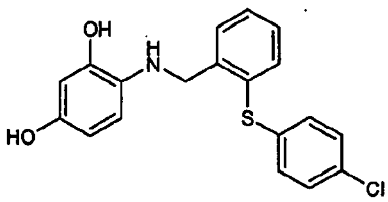 | 1044 | B | B | B | - | - |

As may be taken from table 4 all of the tested compounds are highly efficient in exhibiting a cytotoxic effect on at least one of the various tumor cell lines tested. Of particular relevance are compounds 102, 264, 357, 399, 629, 633, 645, 652, 673, 703, 1044, 1273.

Example 38: FACS measurements and TUNEL assay

In order to show that the compounds according to the present invention are actually useful for inducing apoptosis in tumor cells, FACS measurements and TUNEL assay were performed. Enari M. Sakahira H. Yokoyama H. Okawa K. Iwamatsu A. Nagata S. A caspase-activated DNase that degrades DNA during apoptosis, and its inhibitor ICAD [erratum appears in Nature 1998 May 28;393(6683):396.]. Nature. 391:43-50, 1998

Darzynkiewicz Z. Juan G. Li X. Gorczyca W. Murakami T. Traganos F. Cytometry in cell necrobiology: analysis of apoptosis and accidental cell death (necrosis). Cytometry. 27:1-20, 1997

Apoptotic HL-60 cells were detected by FACS analysis of FITC-dUTP-labelled DNA breaks using the Apo-Direct kit (BD-Pharmingen) according to the manufacturer's protocol.

As may be taken from Fig. 2 compounds 102 and 264 induce apoptosis in tumor cells.

Example 39: Cyclin D1 down regulation

In order to show that the compounds according to the present invention are actually acting the expected way and induces cyclin D1 down regulation, cyclin D1 marker analysis were performed

MCF-7 ($5\times10^5$ cells/well) and HeLa cells ($1.5\times10^5$ cells/well) were seeded in 6-well plates and incubated at 37°C over night. Compounds or DMSO (final solvent concentration 0.1%) were added to the cells and incubated for different times as indicated. Subsequently, cells were lysed in RIPA buffer for 30 min on ice and centrifuged for 20 min at 4°C. After addition of electrophoresis sample buffer (4×) and 50 mM DTT to the supernatant, samples were boiled for 4 min at 95°C. Samples (equivalent to $2\times10^5$ cells/well) were run on a 15% SDS gel followed by blotting onto PVDF membrane.

The membrane was blocked for 1 h in 10 mM Tris (pH 7.5), 100 mM NaCl, 0.1% Tween-20 and 5% non-fat dry milk (blocking buffer) and incubated for 1 h with mouse anti-hCyclin D1 monoclonal antibody (clone DCS-6, BD Biosciences) diluted to 1 μg/ml in blocking buffer. Blots were washed 3 × 10 min with 10 mM Tris (pH 7.5), 100 mM NaCl, 0.1% Tween-20 (washing buffer) and incubated with 0.7 μg/ml peroxidase-conjugated sheep anti-mouse IgG (Sigma) in blocking buffer for 1 h. After washing 3 × 10 min with washing buffer, the plot was developed with the ECL+ detection kit (Amersham Biosciences).

Of particular relevance are compounds 30, 102, 264, 399, 629, 639, 657, 673.

Example 40: DAPI staining

In order to show that the compounds according to the present invention are actually useful for inducing apoptosis in tumor cells, DAPI staining was performed.

Hela cells grown on poly-L-Lys-coated coverslips were fixed with 2% paraformaldehyde/MeOH.

Cellular DNA was stained with DAPI staining buffer (100 mM Tris (pH 7.4), 150 mM NaCl, 1 mM CaCl2, 0.5 mM MgCl2, 0.1% nonidet P-40, 1 μg/ml DAPI (Molecular Probes)). All the steps were performed at room temperature, and cells were washed two times with PBS after each step. Finally, cells were mounted in 80% glycerol/PBS.

As may be taken from Fig. 3 compounds 30, 102, 264 and 399 induce apoptosis in tumor cells.

**EMBODIMENTS**

Embodiment 1: A compound of the formula (I), (II), (III), (IV), (V):

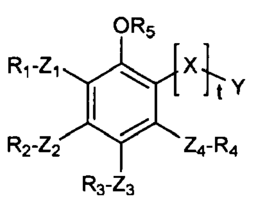 I ,

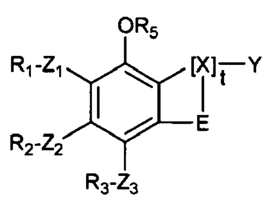 II ,

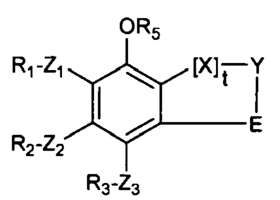 III ,

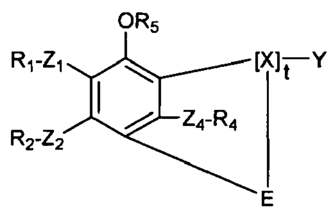 IV or 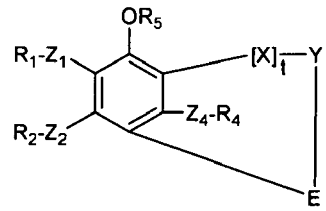 V

wherein $R_1$, $R_2$, $R_3$ and $R_4$ are each independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl;

wherein $R_1$ and $R_2$, $R_2$ and $R_3$, $R_3$ and $R_4$ , $R_1$ and $R_3$, $R_1$ and $R_4$, and $R_2$ and $R_4$ may be linked so as to form a ring comprising 4 to 12 members, preferably 5 to 10 members,

wherein $Z_1$, $Z_2$, $Z_3$ and $Z_4$ are each and independently selected from the group comprising - C(O)-, -C(S)-, -C (OYNR$_{10}$-, -C(S)-NR$_{11}$-, -C(N-CN)-NR$_{12}$-, -S(O)-, -S(O$_2$)-, -S(O)-NR$_{13}$-, and -S(O$_2$)-NR$_{14}$-, -O-, -S- or are each and individually absent;

$R_5$ is selected from the group comprising H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, substituted alkylheteroaryl and -C(O)-Q;

wherein Q is selected from the group comprising H, $NHR_{15}$, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, and substituted alkylheteroaryl; and

$R_6$, $R_7$, $R_8$, $R_9$, $R_{10}$, $R_{11}$, $R_{12}$, $R_{13}$, $R_{14}$ and $R_{15}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

X is a spacer and is independently selected from the group comprising
-M1-L1-K-L2-M2-,

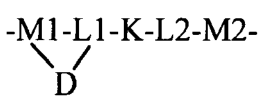 , 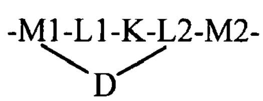 , 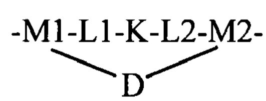 , 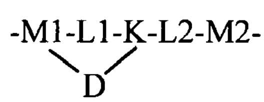 ,

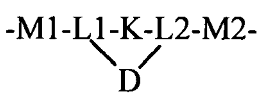 , 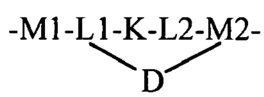 , 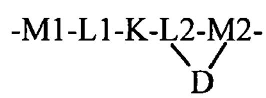 or 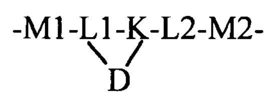

wherein

K is selected from the group comprising

C=T,
O, S, S(O) and $S(O_2)$,
or is absent,
with =T being selected from the group comprising
= O, =S, $=N-R^e$, =N-CN, $=N-NO_2$ and $=CH-NO_2$,

L1 and L2 are each and independently selected from the group comprising O, S and primary amines, more particularly $NR^c$, $NR^d$; or being individually and independent from each other absent

M1 and M2 are each and independently selected from the group comprising
$-(CR^aR^b)n-$,
$-(CR^fR^g)m-$,
cycloalkyl, substituted cycloakyl, heterocyclyl, substituted heterocyclyl, aryl, substituted aryl, heteroaryl and substituted heteroaryl heteroaryl], or being individually and independent from each other absent,

wherein D is straight $C_1$-$C_6$ alkyl, straight $C_1$-$C_6$ alkenyl, straight $C_1$-$C_6$ alkynyl, whereby any of the alkyl, alkenyl and alkynyl may individually and independently comprise from 0 to 3 heteroatoms, and/or whereby any of the alkyl, alkenyl and alkynyl can be individually and independently substituted by 1 or 2 substituent(s) each independently selected from H, halo, $OR_{16}$, alkyl, and substituted alkyl

wherein n and m are each and independently selected from each other and are each any integer from 0 to 10,

whereby if n is 2 or more, the group(s) $-(CR^aR^b)-$ which is/are repeated, can be the same or different from any of the group(s) $-(CR^aR^b)-$,

whereby any individual group can be linked to any other group or any moiety of the compound through a bond selected from the group comprising single bonds, double bonds and triple bonds,

whereby if m is 2 or more, the group(s) $-(CR^fR^g)-$ which is/are repeated, can be the same or different from any of the group(s) $-(CR^fR^g)-$,

whereby any individual group can be linked to any other group or any moiety of the compound through a bond selected from the group comprising single bonds, double bonds and triple bonds,

wherein t is independently selected from n and/or m and is any integer from 0 to 10,

whereby if t is 2 or more any of the spacer -M1-L1-K-L2-M2- can be the same or different from any of the spacer(s) X repeated,

wherein

$R^c$, $R^d$ and $R^e$ are independently from each other selected from the group H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl; and

$R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

wherein E is straight $C_1$-$C_6$ alkyl, straight $C_1$-$C_6$ alkenyl, straight $C_1$-$C_6$ alkynyl, whereby any of the alkyl, alkenyl and alkynyl may comprise individually and independently from 0 to 3 heteroatoms, and/or whereby any of the alkyl, alkenyl and alkynyl can be individually and independently substituted by 1 or 2 substituent(s) each independently selected from the group comprising H, halo, $OR_{21}$, alkyl, and substituted alkyl.

$R_{16}$, $R_{17}$, $R_{18}$, $R_{19}$, $R_{20}$ and $R_{21}$ are each and independently selected from the group comprising H, alkyl,

substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl, wherein Y is different from a peptide or is absent;

Embodiment 2: The compound according to embodiment 1, wherein the phenol moiety forms a cyclic structure with the spacer X and/or Y.

Embodiment 3: The compound according to embodiment 1 or 2, wherein the compound is

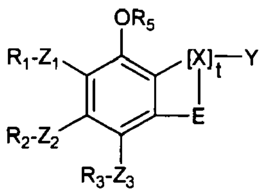 II ,

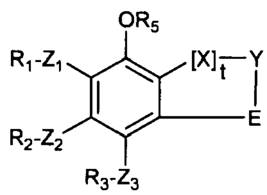 III ,

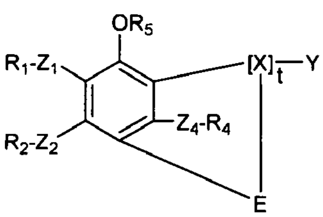 IV or

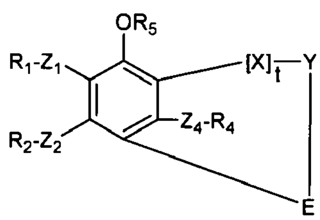 V

Embodiment 4: The compound according to any of embodiments 1 to 3, wherein the compound is

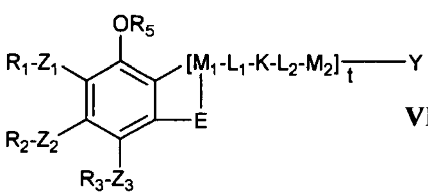 VI ,

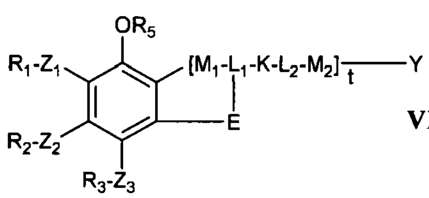 VII ,

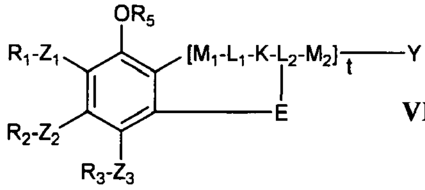

VIII,

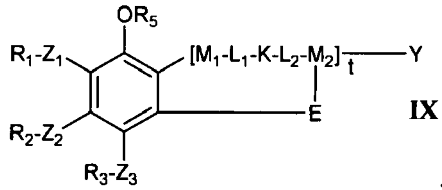

IX,

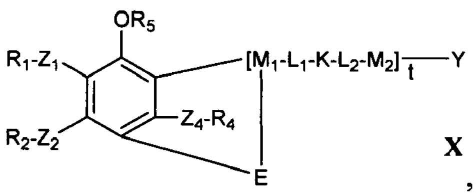

X,

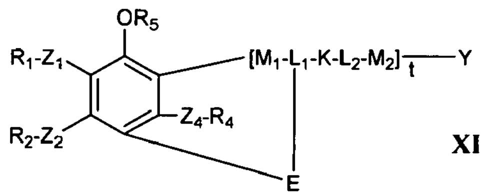

XI,

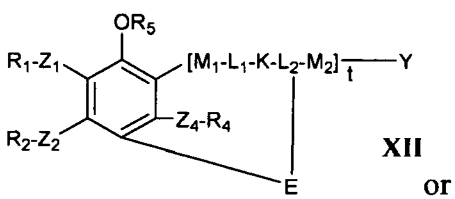

XII

or

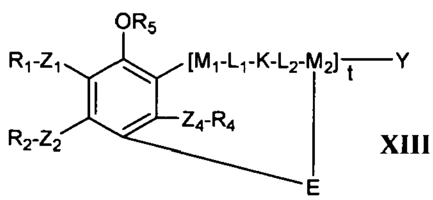

XIII

Embodiment 5: The compound according to any of embodiments 1 to 4, wherein K is C=T.

Embodiment 6: The compound according to embodiment 5, wherein T is selected from the group comprising O and S.

Embodiment 7: The compound according to embodiment 6, wherein T is O.

Embodiment 8: The compound according to embodiment 6, wherein T is S.

Embodiment 9: The compound according to embodiment 6, wherein T is N-CN, $N-NO_2$, $CH-NO_2$ or $N-R^e$.

Embodiment 10: The compound according to any of embodiments 1 to 9, preferably embodiments 7 and 8, wherein L1 and L2 are each and independently a primary amine, preferably $NR^c$ and/or $NR^d$.

Embodiment 11: The compound according to any of embodiments 1 to 10, wherein n = 0 and m is any integer from 0 to 10.

Embodiment 12: The compound according to any of embodiments 1 to 11, wherein $R_1$ and/or $R_3$ are selected from the group comprising halo, alkyl, substituted alkyl, heterocyclyl, substituted heterocyclyl, heteroaryl and substituted heteroaryl, preferably $R_1$ is halo.

Embodiment 13: The compound according to any of embodiments 1 to 12, wherein $R_5$ is selected from the group comprising H and -C(O)-Q,
wherein preferably Q is selected from alkylheterocyclyl and substituted alkylheterocyclyl, preferably N-acylated morpholino- and/or N-acylated piperazino- and/or N-acyl-derivatives.

Embodiment 14: The compound according to any of embodiments 1 to 13, wherein $R_6$ is alkyl or substituted alkyl.

Embodiment 15: The compound according to any of embodiments 1 to 14, wherein $R_8$ and $R_9$ are individually and separately selected from the group comprising H, alkyl and substituted alkyl.

Embodiment 16: The compound according to any of embodiments 1 to 15, wherein n and m are individually and independently any integer from 1 to 3.

Embodiment 17: The compound according to any of embodiments 1 to 15, wherein n is any integer from 0 to 3 and is preferably 0 or 1.

Embodiment 18: The compound according to any of embodiments 1 to 15, wherein n and m are both 0.

Embodiment 19: The compound according to any of embodiments 1 to 18, wherein t is 1 or 2.

Embodiment 20: The compound according to any of embodiments 1 to 19, wherein $R^c$ and/or $R^d$ are each and independently from each other selected from the group comprising alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

Embodiment 21: The compound according to any of embodiments 1 to 19, wherein $R^a$, $R^b$, $R^f$ and $R^g$ are each individually and independently from each other selected from the group comprising H, $OR_{17}$, $SR_{18}$, $NR_{19}R_{20}$, halo, alkyl and substituted alkyl.

Embodiment 22: The compound according to any of embodiments 1 to 21, wherein Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkylnyl and substituted branched alkynyl.

Embodiment 23: The compound according to any of embodiments 1 to 21, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 24: The compound according to any of the preceding embodiments, wherein X is
$-(CR^aR^b)_n-NR^c-CZ-NR^d-(CR^fR^g)_m-$
and Z is selected from the group comprising O, S, N-CN, $N-NO_2$ and $CH-NO_2$.

Embodiment 25: The compound according to embodiment 24, wherein m is any integer from 1 to 10.

Embodiment 26: The compound according to embodiment 24, wherein $R_5$ is selected from the group comprising H and -C(O)-Q.

Embodiment 27: The compound according to embodiment 26, wherein $R_5$ is H.

Embodiment 28: The compound according to embodiment 26 or 27, wherein n is 0.

Embodiment 29: The compound according to embodiment 26 or 27, wherein n is any integer from 1 to 10.

Embodiment 30: The compound according to any of embodiments 24 to 29, wherein t is 1.

Embodiment 31: The compound according to any of embodiments 1 to 30, preferably 24 to 30, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl and substituted branched alkynyl.

Embodiment 32: The compound according to any embodiments 1 to 30, preferably 24 to 30, wherein Y is selected from the group comprising cycloalkyl, substituted cycloalkyl, cycloalkenyl, substituted cycloalkenyl, heterocyclyl,

substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or wherein Y is absent.

Embodiment 33: The compound according to any of embodiments 24 to 32 wherein $R^c$ and/or $R^d$ are independently from each other selected from the group alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

Embodiment 34: A compound according to any of embodiments 1 to 23, wherein X is
$-(CR^aR^b)_n-NR^c-(CR^fR^g)_m-$

Embodiment 35: The compound according to embodiment 34, wherein $R_5$ is selected from the group comprising H and -C(O)-Q.

Embodiment 36: The compound according to embodiment 35, wherein $R_5$ is H.

Embodiment 37: The compound according to embodiment 34, wherein m is any integer between 1 and 10.

Embodiment 38: The compound according to embodiment 37, wherein n is 0.

Embodiment 39: The compound according to embodiment 37 or 38, wherein $R_5$ is selected from the group comprising H and -C(O)-Q.

Embodiment 40: The compound according to embodiment 39, wherein $R_5$ is H.

Embodiment 41: A compound according to embodiment 34, wherein X is
$-(CR^aR^b)_n-NR^c-(CR^fR^g)_m-$, and
wherein t is 1.

Embodiment 42: The compound according to embodiment 41, wherein Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, and substituted branched alkynyl.

Embodiment 43: The compound according to embodiment 42, wherein $R_5$ is selected from the group comprising H and -C(O)-Q.

Embodiment 44: The compound according to embodiment 43, wherein $R_5$ is H.

Embodiment 45: The compound according to any of embodiments 42 to 44, wherein n is 0.

Embodiment 46: The compound according to embodiment 41, wherein m is any integer between 1 and 10.

Embodiment 47: The compound according to embodiment 41, wherein m is any integer between 2 and 10.

Embodiment 48: The compound according to embodiment 46 or 47, wherein $R_5$ is selected from the group comprising H and -C(O)-Q.

Embodiment 49: The compound according to embodiment 48, wherein $R_5$ is H.

Embodiment 50: The compound according to embodiment 46 or 47, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent..

Embodiment 51: The compound according to embodiment 50, wherein $R_5$ is selected from the group comprising H and -C(O)-Q.

Embodiment 52: The compound according to embodiment 51, wherein $R_5$ is H.

Embodiment 53: The compound according to any of embodiments 50 to 52, wherein n is 0.

Embodiment 54: A compound according to any of embodiments 1 to 23, wherein X is $-(CR^aR^b)_n-NR^c-Z-(CR^fR^g)_m-$ and can be inserted in any orientation into any of the preceding formulae, and wherein Z is selected from the group comprising C(O), C(S), $S(O_2)$, C(O)-O, and C(O)-S.

Embodiment 55: The compound according to embodiment 54, wherein $R_5$ is selected from the group comprising H and -C(O)-Q.

Embodiment 56: The compound according to embodiment 50, wherein $R_5$ is H.

Embodiment 57: The compound according to embodiment 55 or 56, wherein n is 0.

Embodiment 58: The compound according to embodiment 54, wherein X is $-(CR^aR^b)_n-NR^c-Z-(CR^fR^g)_m-$ and can be inserted in any orientation into any of the preceding formulae, and Z is selected from the group comprising C(O), C(S), $S(O_2)$, C(O)-O, and C(O)-S, and wherein preferably t is 1.

Embodiment 59: The compound according to embodiment 58, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, and substituted branched alkynyl.

Embodiment 60: The compound according to embodiment 59, wherein $R_5$ is selected from the group comprising H and -C(O)-Q.

Embodiment 61: The compound according to embodiment 60, wherein $R_5$ is H.

Embodiment 62: The compound according to any of embodiments 59 to 61, wherein n is 0.

Embodiment 63: The compound according to embodiment 58, wherein m is any integer between 1 and 10.

Embodiment 64: The compound according to embodiment 63, wherein $R_5$ is selected from the group comprising H and -C(O)-Q.

Embodiment 65: The compound according to embodiment 64, wherein $R_5$ is H.

Embodiment 66: The compound according to embodiment 63, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 67: The compound according to embodiment 66, wherein $R_5$ is selected from the group comprising H and -C(O)-Q.

Embodiment 68: The compound according to embodiment 67, wherein $R_5$ is H.

Embodiment 69: The compound according to any of embodiments 66 to 68, wherein n is 0.

Embodiment 70: The compound according to embodiment 66, wherein m is any integer between 2 and 10.

Embodiment 71: The compound according to embodiment 70, wherein $R_5$ is selected from the group comprising H

and -C(O)-Q.

Embodiment 72: The compound according to embodiment 71, wherein $R_5$ is H.

Embodiment 73: The compound according to any of embodiments 70 to 72, wherein n is 0.

Embodiment 74: A compound of the formula (XIV), (XV) or (XVI):

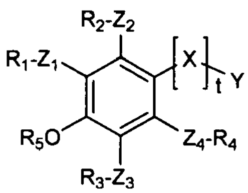

XIV ,

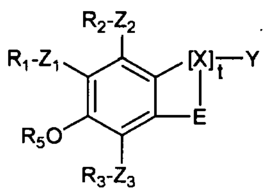

XV ,

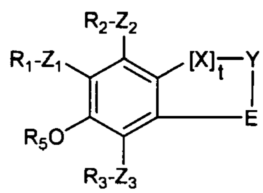

XVI ,

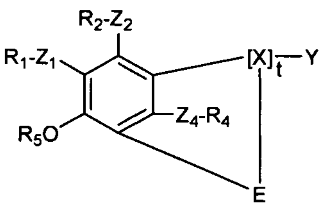

XVII

or

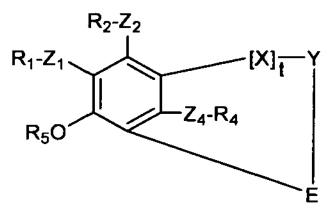

XVIII

wherein $R_1$, $R_2$, $R_3$ and $R_4$ are each independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; and

wherein $R_1$ and $R_2$, $R_2$ and $R_3$, $R_3$ and $R_4$ , $R_1$ and $R_3$, $R_1$ and $R_4$, and $R_2$ and $R_4$ may be linked so as to form a ring comprising 4 to 12 members, preferably 5 to 10 members,

wherein $Z_1$, $Z_2$, $Z_3$ and $Z_4$ are each and independently selected from the group comprising - C(O)-, -C(S)-, -C (O)-$NR_{10}$-, -C(S)-$NR_{11}$-, -C(N-CN)-$NR_{12}$-, -S(O)-, -S($O_2$)-, -S(O)-$NR_{13}$-, and -S($O_2$)-$NR_{14}$-, -O-, -S-, or are each and individually absent;

$R_5$ is selected from the group comprising H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, substituted alkylheteroaryl and -C(O)-Q;

> wherein Q is selected from the group comprising H, $NHR_{15}$, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, and substituted alkylheteroaryl; and

$R_6$, $R_7$, $R_8$, $R_9$, $R_{10}$, $R_{11}$, $R_{12}$, $R_{13}$, $R_{14}$ and $R_{15}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

X is a spacer and is independently selected from the group comprising

-M1-L1-K-L2-M2-,

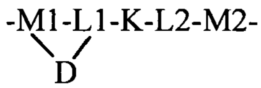 , 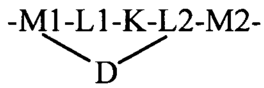 , 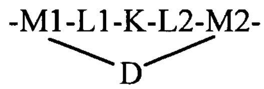 , 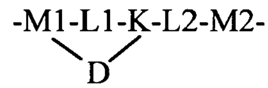 ,

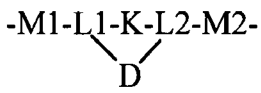 , 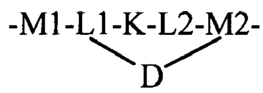 , 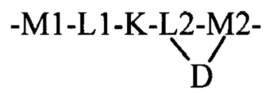 or 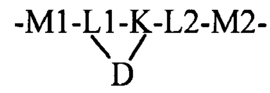

wherein

K is selected from the group comprising

C=T,
O, S, S(O) and S(O)2,
or is absent,
with =T being selected from the group comprising
=O, =S, =N-$R^e$, =N-CN, =N-$NO_2$ and =CH-$NO_2$,

L1 and L2 are each and independently selected from the group comprising O, S and primary amines, more particularly $NR^c$, $NR^d$; or being individually and independent from each other absent

M1 and M2 are each and independently selected from the group comprising
-($CR^aR^b$)n,
-($CR^fR^g$)m-,
cycloalkyl, substituted cycloakyl, heterocyclyl, substituted heterocyclyl, aryl, substituted aryl, heteroaryl and substituted heteroaryl;
or being individually and independent from each other absent,

wherein D is straight $C_1$-$C_6$ alkyl, straight $C_1$-$C_6$ alkenyl, straight $C_1$-$C_6$ alkynyl, whereby any of the alkyl, alkenyl and alkynyl may individually and independently comprise from 0 to 3 heteroatoms, and/or whereby any of the alkyl, alkenyl and alkynyl can be individually and independently substituted by 1 or 2 substituent(s) each independently selected from the group comprising H, halo, $OR_{16}$, alkyl, and substituted alkyl,

wherein n and m are each and independently selected from each other and are each and any integer from 0 to 10,

whereby if n is 2 or more, the group(s) -($CR^aR^b$)- which is/are repeated, can be the same or different from any of the group(s) -($CR^aR^b$)-,

whereby any individual group can be linked to any other group or any moiety of the compound through a bond selected from the group comprising single bonds, double bonds and triple bonds,

whereby if m is 2 or more, the group(s) -($CR^fR^g$)- which is/are repeated, can be the same or different from any of the group(s) -($CR^fR^g$)-,

whereby any individual group can be linked to any other group or any moiety of the compound through a bond selected from the group comprising single bonds, double bonds and triple bonds,

wherein t is independently selected from n and/or m and is any integer from 0 to 10,

whereby if t is 2 or more any of the spacer -M1-L1-K-L2-M2- can be the same or different from any of the spacer(s) X repeated,

wherein

$R^c$, $R^d$ and $R^e$ are independently from each other selected from the group H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl; and

$R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

wherein E is straight $C_1$-$C_6$ alkyl, straight $C_1$-$C_6$ alkenyl, straight $C_1$-$C_6$ alkynyl, whereby any of the alkyl, alkenyl and alkynyl may comprise individually and independently from 0 to 3 heteroatoms, and/or whereby any of the alkyl, alkenyl and alkynyl can be individually and independently substituted by 1 or 2 substituent(s) each independently selected from the group comprising H, halo, $OR_{21}$, alkyl, and substituted alkyl.

$R_{16}$, $R_{17}$, $R_{18}$, $R_{19}$, $R_{20}$ and $R_{21}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;

wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl, wherein Y is different from a peptide or is absent.

Embodiment 75: The compound according to embodiment 74, wherein the phenol moiety forms a cyclic structure with the spacer X and/or Y.

Embodiment 76: The compound according to embodiment 73 or 74, wherein the compound is

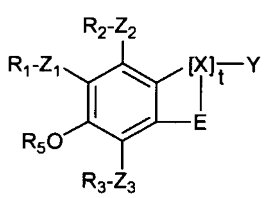 XV ,

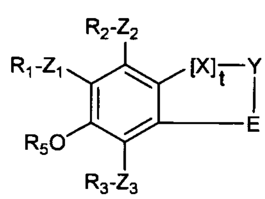 XVI ,

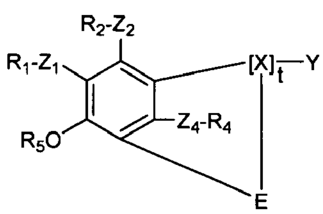 XVII

or

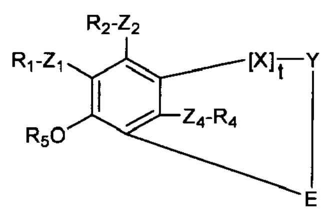 XVIII

Embodiment 77: The compound according to any of embodiments 74 to 76, wherein the compound is

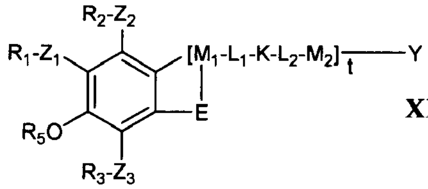 XIX , 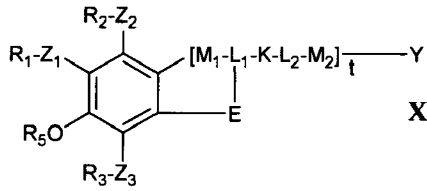 XX ,

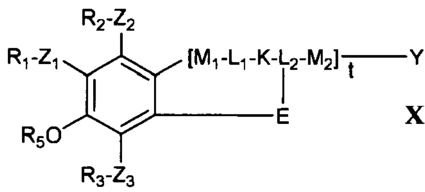 XXI , 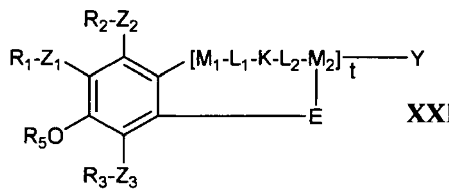 XXII ,

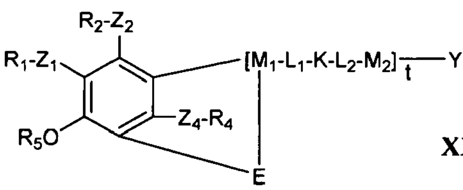 XXIII , 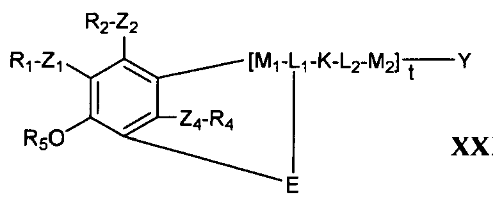 XXIV ,

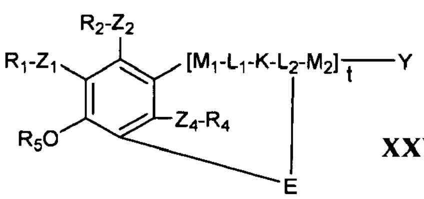 XXV or 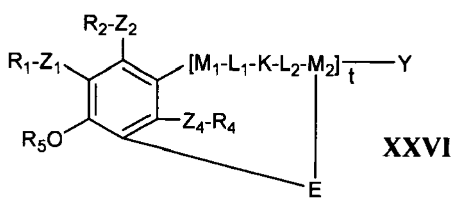 XXVI

Embodiment 78: The compound according to any of embodiments 73 to 77, wherein K is C=T.

Embodiment 79: The compound according to embodiment 78, wherein T is selected from the group comprising O and S.

Embodiment 80: The compound according to embodiment 79, wherein T is O.

Embodiment 81: The compound according to embodiment 79, wherein T is S.

Embodiment 82: The compound according to embodiment 79, wherein T is N-CN, $N-NO_2$, CH-NO2 or $N-R^e$.

Embodiment 83: The compound according to any of embodiments 74 to 82, preferably embodiments 80 and 81, wherein L1 and L2 are each and independently a primary amine, preferably $NR^c$ and/or $NR^d$.

Embodiment 84: The compound according to any of embodiments 74 to 83, wherein n = 0 and m is any integer from 0 to 10.

Embodiment 85: The compound according to any of embodiments 74 to 84, wherein $R_1$ and/or $R_3$, are selected from the group comprising halo, alkyl, substituted alkyl, heterocyclyl, substituted heterocyclyl, heteroaryl and substituted heteroaryl, preferably R1 is halo.

Embodiment 86: The compound according to any of embodiments 74 to 85, wherein $R_5$ is selected from the group comprising H and -C(O)-Q;
wherein preferably Q is selected from alkylheterocyclyl and substituted alkylheterocyclyl, preferably N-acylated morpholino- and/or N-acylated piperazino- and/or N-acyl-derivatives.

Embodiment 87: The compound according to any of embodiments 74 to 86, wherein $R_6$ is alkyl or substituted alkyl.

Embodiment 88: The compound according to any of embodiments 74 to 87, wherein $R_8$ and $R_9$ are individually and separately selected from the group comprising H, alkyl and substituted alkyl.

Embodiment 89: The compound according to any of embodiments 74 to 88, wherein n and m are individually and independently any integer from 1 to 3.

Embodiment 90: The compound according to any of embodiments 74 to 88, wherein n is any integer from 0 to 3 and is preferably 0 or 1.

Embodiment 91: The compound according to any of embodiments 74 to 88, wherein n and m are both 0.

Embodiment 92: The compound according to any of embodiments 74 to 91, wherein t is 1 or 2.

Embodiment 93: The compound according to any of embodiments 74 to 92, wherein $R^c$ and/or $R^d$ are each and independently from each other selected from the group comprising alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

Embodiment 94: The compound according to any of embodiments 1 to 19, wherein $R^a$, $R^b$, $R^f$ and $R^g$ are each individually and independently from each other selected from the group comprising H, $OR_{17}$, $SR_{18}$, $NR_{19}R_{20}$, halo, alkyl and substituted alkyl.

Embodiment 95: The compound according to any of embodiments 74 to 94, wherein wherein Y is selected from the group comprising alkyl, substituted alkyl, straight alkyl, substituted straight alkyl, branched alkyl, substituted branched alkyl, straight alkenyl, substituted straight alkenyl, branched alkenyl, substituted branched alkenyl, straight alkynyl, substituted straight alkynyl, branched alkynyl and substituted branched alkynyl.

Embodiment 96: The compound according to any of embodiments 74 to 94, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 97: The compound according to any embodiments 74 to 96, wherein X is
$-(CR^aR^b)_n-NR^c-CZ-NR^d-(CR^fR^g)_m-$
and Z is selected from the group comprising O, S, N-CN, $N-NO_2$ and $CH-NO_2$.

Embodiment 98: The compound according to embodiment 97, wherein m is any integer from 1 to 10.

Embodiment 99: The compound according to embodiment 97, wherein $R_5$ is selected from the group comprising H and -C(O)-Q.

Embodiment 100: The compound according to embodiment 99, wherein $R_5$ is H.

Embodiment 101: The compound according to embodiment 99 or 100, wherein n is 0.

Embodiment 102: The compound according to embodiment 99 or 100, wherein n is any integer from 1 to 10.

Embodiment 103: The compound according to any of embodiments 97 to 102, wherein t is 1.

Embodiment 104: The compound according to any of embodiments 74 to 103, preferably 97 to 103, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl and substituted branched alkynyl.

Embodiment 105: The compound according to any embodiments 74 to 103, preferably 97 to 103, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 106: The compound according to any of embodiments 97 to 105, wherein $R^c$ and/or $R^d$ are independently from each other selected from the group alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl.

Embodiment 107: A compound according to any of embodiments 74 to 96, wherein X is
$-(CR^aR^b)_n-NR^c-(CR^fR^g)_m-$
wherein

preferably $R^a$, $R^b$, $R^c$, $R^d$, $R^e$, $R^f$ and $R^g$ are independently from each other selected from the group H, alkyl, substituted alkyl, cycloalkyl, substituted cycloalkyl, aryl, substituted aryl, alkylaryl, substituted alkylaryl, heterocyclyl, substituted heterocyclyl, heteroaryl, substituted heteroaryl.

Embodiment 108: The compound according to embodiment 107, wherein $R_5$ is selected from the group comprising H and -C(O)-Q.

Embodiment 109: The compound according to embodiment 108, wherein $R_5$ is H.

Embodiment 110: The compound according to embodiment 107, wherein m is any integer between 1 and 10.

Embodiment 111: The compound according to embodiment 110, wherein n is 0.

Embodiment 112: The compound according to embodiment 110 or 111, wherein $R_5$ is selected from the group comprising H and -C(O)-Q.

Embodiment 113: The compound according to embodiment 112, wherein $R_5$ is H.

Embodiment 114: A compound according to embodiment 107, wherein X is
$-(CR^aR^b)_n-NR^c-(CR^fR^g)_m-$, and
wherein t is 1.

Embodiment 115: The compound according to embodiment 114, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, and substituted branched alkynyl.

Embodiment 116: The compound according to embodiment 115, wherein $R_5$ is selected from the group comprising H and -C(O)-Q.

Embodiment 117: The compound according to embodiment 116, wherein $R_5$ is H.

Embodiment 118: The compound according to any of embodiments 115 to 117, wherein n is 0.

Embodiment 119: The compound according to embodiment 114, wherein m is any integer between 1 and 10.

Embodiment 120: The compound according to embodiment 114, wherein m is any integer between 2 and 10.

Embodiment 121: The compound according to embodiment 119 or 120, wherein $R_5$ is selected from the group comprising H and -C(O)-Q.

Embodiment 122: The compound according to embodiment 121, wherein $R_5$ is H.

Embodiment 123: The compound according to embodiment 119 or 120, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 124: The compound according to embodiment 123, wherein $R_5$ is selected from the group comprising H and -C(O)-Q.

Embodiment 125: The compound according to embodiment 124, wherein $R_5$ is H.

Embodiment 126: The compound according to any of embodiments 123 to 125, wherein n is 0.

Embodiment 127: A compound according to any of embodiments 74 to 96, wherein X is $-(CR^aR^b)_n-NR^c-Z-(CR^fR^g)_m-$ and can be inserted in any orientation into any of the preceding formulae, and wherein Z is selected from the group comprising C(O), C(S), $S(O_2)$, C(O)-O, and C(O)-S.

Embodiment 128: The compound according to embodiment 127, wherein $R_5$ is selected from the group comprising H and -C(O)-Q.

Embodiment 129: The compound according to embodiment 123, wherein $R_5$ is H.

Embodiment 130: The compound according to embodiment 128 or 129, wherein n is 0.

Embodiment 131: The compound according to embodiment 127, wherein X is $-(CR^aR^b)_n-NR^c-Z-(CR^fR^g)_m-$ and can be inserted in any orientation into any of the preceding formulae, and Z is selected from the group comprising C(O), C(S), $S(O_2)$ C(O)-O, and C(O)-S, and wherein preferably t is 1.

Embodiment 132: The compound according to embodiment 131, wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, and substituted branched alkynyl.

Embodiment 133: The compound according to embodiment 132, wherein $R_5$ is selected from the group comprising H and -C(O)-Q.

Embodiment 134: The compound according to embodiment 133, wherein $R_5$ is H.

Embodiment 135: The compound according to any of embodiments 132 to 134, wherein n is 0.

Embodiment 136: The compound according to embodiment 131, wherein m is any integer between 1 and 10.

Embodiment 137: The compound according to embodiment 136, wherein $R_5$ is selected from the group comprising H and -C(O)-Q.

Embodiment 138: The compound according to embodiment 137, wherein $R_5$ is H.

Embodiment 139: The compound according to embodiment 63, wherein Y is selected from the group comprising cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide or is absent.

Embodiment 140: The compound according to embodiment 139, wherein $R_5$ is selected from the group comprising H and -C(O)-Q.

Embodiment 141: The compound according to embodiment 140, wherein $R_5$ is H

Embodiment 142: The compound according to any of embodiments 139 to 141, wherein n is 0.

Embodiment 143: The compound according to embodiment 139, wherein m is any integer between 2 and 10.

Embodiment 144: The compound according to embodiment 143, wherein $R_5$ is selected from the group comprising H and -C(O)-Q.

Embodiment 145: The compound according to embodiment 144, wherein $R_5$ is H.

Embodiment 146: The compound according to any of embodiments 143 to 145, wherein n is 0.

Embodiment 147: Compound, preferably a compound according to any of the preceding embodiments, selected from:

3-[3-(5-Chloro-2-hydroxy-phenyl)-ureido]-propionic acid ethyl ester
1-(5-Chloro-2-hydroxy-phenyl)-3-pentyl-urea
1-Benzyl-3-(5-chloro-2-hydroxy-phenyl)-urea
1-(5-Chloro-2-hydroxy-phenyl)-3-(2-methyl-benzyl)-urea
1-(5-Chloro-2-hydroxy-phenyl)-3-phenethyl-urea
1-(5-Chloro-2-hydroxy-phenyl)-3-(1,1,3,3-tetramethyl-butyl)-urea
1-tert-Butyl-3-(5-chloro-2-hydroxy-phenyl)-urea
1-(5-Chloro-2-hydroxy-phenyl)-3-cyclohexylmethyl-urea
1-(5-Chloro-2-hydroxy-phenyl)-3-(4-trifluoromethyl-benzyl)-urea
1-(5-Chloro-2-hydroxy-phenyl)-3-(3,5-dichloro-phenyl)-urea
1-(5-Chloro-2-hydroxy-phenyl)-3-(4-chloro-phenyl)-urea
1-(5-Chloro-2-hydroxy-phenyl)-3-(4-trifluoromethyl-phenyl)-urea
1-(5-Chloro-2-hydroxy-phenyl)-3-cyclohexyl-urea
1-(5-Chloro-2-hydroxy-phenyl)-3-(4-trifluoromethoxy-phenyl)-urea
1-(5-Chloro-2-hydroxy-phenyl)-3-(4-cyano-phenyl)-urea
1-Benzo[1,3]dioxol-5-yl-3-(5-chloro-2-hydroxy-phenyl)-urea
1-(5-Chloro-2-hydroxy-phenyl)-3-o-tolyl-urea
1-(5-Chloro-2-hydroxy-phenyl)-3-(3-methoxy-phenyl)-urea
1-(5-Chloro-2-hydroxy-phenyl)-3-(2,6-dimethyl-phenyl)-urea
1-(5-Chloro-2-hydroxy-phenyl)-3-(3,4,5-trimethoxy-phenyl)-urea
1-(5-Chloro-2-hydroxy-phenyl)-3-naphthalen-1-yl-urea
1-Adamantan-1-yl-3-(5-chloro-2-hydroxy-phenyl)-urea
1-(5-Chloro-2-hydroxy-phenyl)-3-(4-phenoxy-phenyl)-urea
1-(5-Chloro-2-hydroxy-phenyl)-3-phenyl-urea
3-[3-(3,5-Dichloro-2-hydroxy-phenyl)-ureido]-propionic acid ethyl ester
1-(3,5-Dichloro-2-hydroxy-phenyl)-3-pentyl-urea
1-Benzyl-3-(3,5-dichloro-2-hydroxy-phenyl)-urea
1-(3,5-Dichloro-2-hydroxy-phenyl)-3-(2-methyl-benzyl)-urea
1-(3,5-Dichloro-2-hydroxy-phenyl)-3-phenethyl-urea
1-(3,5-Dichloro-2-hydroxy-phenyl)-3-(1,1,3,3-tetramethyl-butyl)-urea
1-tert-Butyl-3-(3,5-dichloro-2-hydroxy-phenyl)-urea
1-(3,5-Dichloro-2-hydroxy-phenyl)-3-cyclohexylmethyl-urea
1-(3,5-Dichloro-2-hydroxy-phenyl)-3-(4-trifluoromethyl-benzyl)-urea
1-(3,5-Dichloro-2-hydroxy-phenyl)-3-(3,5-dichloro-phenyl)-urea
1-(3,5-Dichloro-2-hydroxy-phenyl)-3-(4-chloro-phenyl)-urea
1-(3,5-Dichloro-2-hydroxy-phenyl)-3-(4-trifluoromethyl-phenyl)-urea
1-(3,5-Dichloro-2-hydroxy-phenyl)-3-cyclohexyl-urea

1-(3,5-Dichloro-2-hydroxy-phenyl)-3-(4-trifluoromethoxy-phenyl)-urea
1-(3,5-Dichloro-2-hydroxy-phenyl)-3-(4-cyano-phenyl)-urea
1-Benzo[1,3]dioxol-5-yl-3-(3,5-dichloro-2-hydroxy-phenyl)-urea
1-(3,5-Dichloro-2-hydroxy-phenyl)-3-o-tolyl-urea
1-(3,5-Dichloro-2-hydroxy-phenyl)-3-(3-methoxy-phenyl)-urea
1-(3,5-Dichloro-2-hydroxy-phenyl)-3-(2,6-dimethyl-phenyl)-urea
1-(3,5-Dichloro-2-hydroxy-phenyl)-3-(3,4,5-trimethoxy-phenyl)-urea
1-(3,5-Dichloro-2-hydroxy-phenyl)-3-naphthalen-1-yl-urea
1-Adamantan-1-yl-3-(3,5-dichloro-2-hydroxy-phenyl)-urea
1-(3,5-Dichloro-2-hydroxy-phenyl)-3-(4-phenoxy-phenyl)-urea
1-(3,5-Dichloro-2-hydroxy-phenyl)-3-phenyl-urea
3-[3-(3-Chloro-2-hydroxy-phenyl)-ureido]-propionic acid ethyl ester
1-(3-Chloro-2-hydroxy-phenyl)-3-pentyl-urea
1- -Benzyl-3-(3-chloro-2-hydroxy-phenyl)-urea
1-(3-Chloro-2-hydroxy-phenyl)-3-(2-methyl-benzyl)-urea
1-(3-Chloro-2-hydroxy-phenyl)-3-phenethyl-urea
1-(3-Chloro-2-hydroxy-phenyl)-3-(1,1,3,3-tetramethyl-butyl)-urea
1-tert-Butyl-3-(3-chloro-2-hydroxy-phenyl)-urea
1-(3-Chloro-2-hydroxy-phenyl)-3-cyclohexylmethyl-urea
1-(3-Chloro-2-hydroxy-phenyl)-3-(4-trifluoromethyl-benzyl)-urea
1-(3-Chloro-2-hydroxy-phenyl)-3-(3,5-dichloro-phenyl)-urea
1-(3-Chloro-2-hydroxy-phenyl)-3-(4-chloro-phenyl)-urea
1-(3-Chloro-2-hydroxy-phenyl)-3-(4-trifluoromethyl-phenyl)-urea
1-(3-Chloro-2-hydroxy-phenyl)-3-cyclohexyl-urea
1-(3-Chloro-2-hydroxy-phenyl)-3-(4-trifluoromethoxy-phenyl)-urea
1-(3-Chloro-2-hydroxy-phenyl)-3-(4-cyano-phenyl)-urea
1-Benzo[1,3]dioxol-5-yl-3-(3-chloro-2-hydroxy-phenyl)-urea
1-(3-Chloro-2-hydroxy-phenyl)-3-o-tolyl-urea
1-(3-Chloro-2-hydroxy-phenyl)-3-(3-methoxy-phenyl)-urea
1-(3-Chloro-2-hydroxy-phenyl)-3-(2,6-dimethyl-phenyl)-urea
1-(3-Chloro-2-hydroxy-phenyl)-3-(3,4,5-trimethoxy-phenyl)-urea
1-(3-Chloro-2-hydroxy-phenyl)-3-naphthalen-1-yl-urea
1-Adamantan-1-yl-3-(3-chloro-2-hydroxy-phenyl)-urea
1-(3-Chloro-2-hydroxy-phenyl)-3-(4-phenoxy-phenyl)-urea
1-(3-Chloro-2-hydroxy-phenyl)-3-phenyl-urea
3-[3-(3-Fluoro-2-hydroxy-phenyl)-ureido]-propionic acid ethyl ester
1-(3-Fluoro-2-hydroxy-phenyl)-3-pentyl-urea
1-Benzyl-3-(3-fluoro-2-hydroxy-phenyl)-urea
1-(3-Fluoro-2-hydroxy-phenyl)-3-(2-methyl-benzyl)-urea
1-(3-Fluoro-2-hydroxy-phenyl)-3-phenethyl-urea
1-(3-Fluoro-2-hydroxy-phenyl)-3-(1,1,3,3-tetramethyl-butyl)-urea
1-tert-Butyl-3-(3-fluoro-2-hydroxy-phenyl)-urea
1-(3-Fluoro-2-hydroxy-phenyl)-3-cyclohexylmethyl-urea
1-(3-Fluoro-2-hydroxy-phenyl)-3-(4-trifluoromethyl-benzyl)-urea
1-(3-Fluoro-2-hydroxy-phenyl)-3-(3,5-dichloro-phenyl)-urea

1-(3-Fluoro-2-hydroxy-phenyl)-3-(4-chloro-phenyl)-urea
1-(3-Fluoro-2-hydroxy-phenyl)-3-(4-trifluoromethyl-phenyl)-urea
1-(3-Fluoro-2-hydroxy-phenyl)-3-cyclohexyl-urea
1-(3-Fluoro-2-hydroxy-phenyl)-3-(4-trifluoromethoxy-phenyl)-urea
1-(3-Fluoro-2-hydroxy-phenyl)-3-(4-cyano-phenyl)-urea
1-Benzo[1,3]dioxol-5-yl-3-(3-fluoro-2-hydroxy-phenyl)-urea
1-(3-Fluoro-2-hydroxy-phenyl)-3-o-tolyl-urea
1-(3-Fluoro-2-hydroxy-phenyl)-3-(3-methoxy-phenyl)-urea
1-(3-Fluoro-2-hydroxy-phenyl)-3-(2,6-dimethyl-phenyl)-urea
1-(3-Fluoro-2-hydroxy-phenyl)-3-(3,4,5-trimethoxy-phenyl)-urea
1-(3-Fluoro-2-hydroxy-phenyl)-3-naphthalen-1-yl-urea
1-Adamantan-1-yl-3-(3-fluoro-2-hydroxy-phenyl)-urea

1-(3-Fluoro-2-hydroxy-phenyl)-3-(4-phenoxy-phenyl)-urea
1-(3-Fluoro-2-hydroxy-phenyl)-3-phenyl-urea
3-[3-(3,5-Dichloro-2-hydroxy-4-methyl-phenyl)-ureido]-propionic acid ethyl ester
1-(3,5-Dichloro-2-hydroxy-4-methyl-phenyl)-3-pentyl-urea
1-Benzyl-3-(3,5-dichloro-2-hydroxy-4-methyl-phenyl)-urea
1-(3,5-Dichloro-2-hydroxy-4-methyl-phenyl)-3-(2-methyl-benzyl)-urea
1-(3,5-Dichloro-2-hydroxy-4-methyl-phenyl)-3-phenethyl-urea
1-(3,5-Dichloro-2-hydroxy-4-methyl-phenyl)-3-(1,1,3,3-tetramethyl-butyl)-urea
1-tert-Butyl-3-(3,5-dichloro-2-hydroxy-4-methyl-phenyl)-urea
1-Cyclohexylmethyl-3-(3,5-dichloro-2-hydroxy-4-methyl-phenyl)-urea
1-(3,5-Dichloro-2-hydroxy-4-methyl-phenyl)-3-(4-trifluoromethyl-benzyl)-urea
1-(3,5-Dichloro-2-hydroxy-4-methyl-phenyl)-3-(3,5-dichloro-phenyl)-urea
1-(4-Chloro-phenyl)-3-(3,5-dichloro-2-hydroxy-4-methyl-phenyl)-urea
1-(3,5-Dichloro-2-hydroxy-4-methyl-phenyl)-3-(4-trifluoromethyl-phenyl)-urea
1-Cyclohexyl-3-(3,5-dichloro-2-hydroxy-4-methyl-phenyl)-urea
1-(3,5-Dichloro-2-hydroxy-4-methyl-phenyl)-3-(4-trifluoromethoxy-phenyl)-urea
1-(4-Cyano-phenyl)-3-(3,5-dichloro-2-hydroxy-4-methyl-phenyl)-urea
1-Benzo[1,3]dioxol-5-yl-3-(3,5-dichloro-2-hydroxy-4-methyl-phenyl)-urea
1-(3,5-Dichloro-2-hydroxy-4-methyl-phenyl)-3-o-tolyl-urea
1-(3,5-Dichloro-2-hydroxy-4-methyl-phenyl)-3-(3-methoxy-phenyl)-urea
1-(3,5-Dichloro-2-hydroxy-4-methyl-phenyl)-3-(2,6-dimethyl-phenyl)-urea
1-(3,5-Dichloro-2-hydroxy-4-methyl-phenyl)-3-(3,4,5-trimethoxy-phenyl)-urea
1-(3,5-Dichloro-2-hydroxy-4-methyl-phenyl)-3-naphthalen-1-yl-urea
1-Adamantan-1-yl-3-(3,5-dichloro-2-hydroxy-4-methyl-phenyl)-urea
1-(3,5-Dichloro-2-hydroxy-4-methyl-phenyl)-3-(4-phenoxy-phenyl)-urea
1-(3,5-Dichloro-2-hydroxy-4-methyl-phenyl)-3-phenyl-urea
3-[3-(5-Bromo-3-fluoro-2-hydroxy-phenyl)-ureido]-propionic acid ethyl ester
1-(5-Bromo-3-fluoro-2-hydroxy-phenyl)-3-pentyl-urea
1-Benzyl-3-(5-bromo-3-fluoro-2-hydroxy-phenyl)-urea
1-(5-Bromo-3-fluoro-2-hydroxy-phenyl)-3-(2-methyl-benzyl)-urea
1-(5-Bromo-3-fluoro-2-hydroxy-phenyl)-3-phenethyl-urea
1-(5-Bromo-3-fluoro-2-hydroxy-phenyl)-3-(1,1,3,3-tetramethyl-butyl)-urea
1-tert-Butyl-3-(5-bromo-3-fluoro-2-hydroxy-phenyl)-urea
1-(5-Bromo-3-fluoro-2-hydroxy-phenyl)-3-cyclohexylmethyl-urea
1-(5-Bromo-3-fluoro-2-hydroxy-phenyl)-3-(4-trifluoromethyl-benzyl)-urea
1-(5-Bromo-3-fluoro-2-hydroxy-phenyl)-3-(3,5-dichloro-phenyl)-urea
1-(5-Bromo-3-fluoro-2-hydroxy-phenyl)-3-(4-chloro-phenyl)-urea
1-(5-Bromo-3-fluoro-2-hydroxy-phenyl)-3-(4-trifluoromethyl-phenyl)-urea
1-(5-Bromo-3-fluoro-2-hydroxy-phenyl)-3-cyclohexyl-urea
1-(5-Bromo-3-fluoro-2-hydroxy-phenyl)-3-(4-trifluoromethoxy-phenyl)-urea
1-(5-Bromo-3-fluoro-2-hydroxy-phenyl)-3-(4-cyano-phenyl)-urea
1-Benzo[1,3]dioxol-5-yl-3-(5-bromo-3-fluoro-2-hydroxy-phenyl)-urea
1-(5-Bromo-3-fluoro-2-hydroxy-phenyl)-3-o-tolyl-urea
1-(5-Bromo-3-fluoro-2-hydroxy-phenyl)-3-(3-methoxy-phenyl)-urea
1-(5-Bromo-3-fluoro-2-hydroxy-phenyl)-3-(2,6-dimethyl-phenyl)-urea
1-(5-Bromo-3-fluoro-2-hydroxy-phenyl)-3-(3,4,5-trimethoxy-phenyl)-urea
1-(5-Bromo-3-fluoro-2-hydroxy-phenyl)-3-naphthalen-1-yl-urea
1-Adamantan-1-yl-3-(5-Bromo-3-fluoro-2-hydroxy-phenyl)-urea
1-(5-Bromo-3-fluoro-2-hydroxy-phenyl)-3-(4-phenoxy-phenyl)-urea
1-(5-Bromo-3-fluoro-2-hydroxy-phenyl)-3-phenyl-urea
3-[3-(3,5-Difluoro-2-hydroxy-phenyl)-ureido]-propionic acid ethyl ester
1-(3,5-Difluoro-2-hydroxy-phenyl)-3-pentyl-urea
1-Benzyl-3-(3,5-difluoro-2-hydroxy-phenyl)-urea
1-(3,5-Difluoro-2-hydroxy-phenyl)-3-(2-methyl-benzyl)-urea
1-(3,5-Difluoro-2-hydroxy-phenyl)-3-phenethyl-urea
1-(3,5-Difluoro-2-hydroxy-phenyl)-3-(1,1,3,3-tetramethyl-butyl)-urea
1-tert-Butyl-3-(3,5-difluoro-2-hydroxy-phenyl)-urea
1-(3,5-Difluoro-2-hydroxy-phenyl)-3-cyclohexylmethyl-urea

1-(3,5-Difluoro-2-hydroxy-phenyl)-3-(4-trifluoromethyl-benzyl)-urea
1-(3,5-Difluoro-2-hydroxy-phenyl)-3-(3,5-dichloro-phenyl)-urea
1-(3,5-Difluoro-2-hydroxy-phenyl)-3-(4-chloro-phenyl)-urea
1-(3,5-Difluoro-2-hydroxy-phenyl)-3-(4-trifluoromethyl-phenyl)-urea
1-(3,5-Difluoro-2-hydroxy-phenyl)-3-cyclohexyl-urea
1-(3,5-Difluoro-2-hydroxy-phenyl)-3-(4-trifluoromethoxy-phenyl)-urea
1-(3,5-Difluoro-2-hydroxy-phenyl)-3-(4-cyano-phenyl)-urea
1-Benzo[1,3]dioxol-5-yl-3-(3,5-difluoro-2-hydroxy-phenyl)-urea
1-(3,5-Difluoro-2-hydroxy-phenyl)-3-o-tolyl-urea
1-(3,5-Difluoro-2-hydroxy-phenyl)-3-(3-methoxy-phenyl)-urea
1-(3,5-Difluoro-2-hydroxy-phenyl)-3-(2,6-dimethyl-phenyl)-urea
1-(3,5-Difluoro-2-hydroxy-phenyl)-3-(3,4,5-trimethoxy-phenyl)-urea
1-(3,5-Difluoro-2-hydroxy-phenyl)-3-naphthalen-1-yl-urea
1-Adamantan-1-yl-3-(3,5-difluoro-2-hydroxy-phenyl)-urea
1-(3,5-Difluoro-2-hydroxy-phenyl)-3-(4-phenoxy-phenyl)-urea
1-(3,5-Difluoro-2-hydroxy-phenyl)-3-phenyl-urea
3-{3-[5-Chloro-2-hydroxy-3-(1-hydroxy-ethyl)-phenyl]-ureido}-propionic acid ethyl ester
1-[5-Chloro-2-hydroxy-3-(1-hydroxy-ethyl)-phenyl]-3-pentyl-urea
1-[5-Chloro-2-hydroxy-3-(1-hydroxy-ethyl)-phenyl]-3-pentyl-urea
1-[5-Chloro-2-hydroxy-3-(1-hydroxy-ethyl)-phenyl]-3-(2-methyl-benzyl)-urea
1-[5-Chloro-2-hydroxy-3-(1-hydroxy-ethyl)-phenyl]-3-phenethyl-urea
1-[5-Chloro-2-hydroxy-3-(1-hydroxy-ethyl)-phenyl]-3-(1,1,3,3-tetramethyl-butyl)-urea
1-tert-Butyl-3-[5-chloro-2-hydroxy-3-(1-hydroxy-ethyl)-phenyl]-urea
1-[5-Chloro-2-hydroxy-3-(1-hydroxy-ethyl)-phenyl]-3-cyclohexylmethyl-urea
1-[5-Chloro-2-hydroxy-3-(1-hydroxy-ethyl)-phenyl]-3-(4-trifluoromethyl-benzyl)-urea
1-[5-Chloro-2-hydroxy-3-(1-hydroxy-ethyl)-phenyl]-3-(4-chloro-phenyl)-urea
1-[5-Chloro-2-hydroxy-3-(1-hydroxy-ethyl)-phenyl]-3-(4-chloro-phenyl)-urea
1-[5-Chloro-2-hydroxy-3-(1-hydroxy-ethyl)-phenyl]-3-(4-trifluoromethyl-phenyl)-urea
1-[5-Chloro-2-hydroxy-3-(-hydroxy-ethyl)-phenyl]-3-cyclohexyl-urea
1-[5-Chloro-2-hydroxy-3-(1-hydroxy-ethyl)-phenyl]-3-(4-trifluoromethoxy-phenyl)-urea
1-[5-Chloro-2-hydroxy-3-(1-hydroxy-ethyl)-phenyl]-3-(4-cyano-phenyl)-urea
1-Benzo[1,3]dioxol-5-yl-3-[5-chloro-2-hydroxy-3-(1-hydroxy-ethyl)-phenyl]-urea
1-[5-Chloro-2-hydroxy-3-(1-hydroxy-ethyl)-phenyl]-3-o-tolyl-urea
1-[5-Chloro-2-hydroxy-3-(1-hydroxy-ethyl)-phenyl]-3-(3-methoxy-phenyl)-urea
1-[5-Chloro-2-hydroxy-3-(1-hydroxy-ethyl)-phenyl]-3-(2,6-dimethyl-phenyl)-urea
1-[5-Chloro-2-hydroxy-3-(1-hydroxy-ethyl)-phenyl]-3-(3,4,5-trimethoxy-phenyl)-urea
1-[5-Chloro-2-hydroxy-3-(1-hydroxy-ethyl)-phenyl]-3-naphthalen-1-yl-urea
1-Adamantan-1-yl-3-[5-chloro-2-hydroxy-3-(1-hydroxy-ethyl)-phenyl]-urea

1-[5-Chloro-2-hydroxy-3-(1-hydroxy-ethyl)-phenyl]-3-(4-phenoxy-phenyl)-urea
1-[5-Chloro-2-hydroxy-3-(-hydroxy-ethyl)-phenyl]-3-phenyl-urea
1-(5-Chloro-2-hydroxy-phenyl)-3-pentyl-thiourea
1-Benzyl-3-(5-chloro-2-hydroxy-phenyl)-thiourea
1-(5-Chloro-2-hydroxy-phenyl)-3-(2-methyl-benzyl)-thiourea
1-(5-Chloro-2-hydroxy-phenyl)-3-phenethyl-thiourea
1-(5-Chloro-2-hydroxy-phenyl)-3-(1,1,3,3-tetramethyl-butyl)-thiourea
1-tert-Butyl-3-(5-chloro-2-hydroxy-phenyl)-thiourea
1-(5-Chloro-2-hydroxy-phenyl)-3-isopropyl-thiourea
1-(5-Chloro-2-hydroxy-phenyl)-3-cyclohexylmethyl-thiourea
1-(5-Chloro-2-hydroxy-phenyl)-3-(4-trifluoromethyl-benzyl)-thiourea
1-(5-Chloro-2-hydroxy-phenyl)-3-(3,5-dichloro-phenyl)-thiourea
1-(5-Chloro-2-hydroxy-phenyl)-3-(4-chloro-phenyl)-thiourea
1-(5-Chloro-2-hydroxy-phenyl)-3-(4-trifluoromethyl-phenyl)-thiourea
1-(5-Chloro-2-hydroxy-phenyl)-3-cyclohexyl-thiourea
1-(5-Chloro-2-hydroxy-phenyl)-3-(2-trifluoromethyl-phenyl)-thiourea
1-(5-Chloro-2-hydroxy-phenyl)-3-phenyl-thiourea
1-(3,5-Dichloro-2-hydroxy-phenyl)-3-pentyl-thiourea
1-Benzyl-3-(3,5-dichloro-2-hydroxy-phenyl)-thiourea

1-(3,5-Dichloro-2-hydroxy-phenyl)-3-(2-methyl-benzyl)-thiourea
1-(3,5-Dichloro-2-hydroxy-phenyl)-3-phenethyl-thiourea
1-(3,5-Dichloro-2-hydroxy-phenyl)-3-(1,1,3,3-tetramethyl-butyl)-thiourea
1-tert-Butyl-3-(3,5-dichloro-2-hydroxy-phenyl)-thiourea
1-(5-Chloro-2-hydroxy-phenyl)-3-isopropyl-thiourea
1-(3,5-Dichloro-2-hydroxy-phenyl)-3-cyclohexylmethyl-thiourea
1-(3,5-Dichloro-2-hydroxy-phenyl)-3-(4-trifluoromethyl-benzyl)-thiourea
1-(3,5-Dichloro-2-hydroxy-phenyl)-3-(3,5-dichloro-phenyl)-thiourea
1-(3,5-Dichloro-2-hydroxy-phenyl)-3-(4-chloro-phenyl)-thiourea
1-(3,5-Dichloro-2-hydroxy-phenyl)-3-(4-trifluoromethyl-phenyl)-thiourea
1-(3,5-Dichloro-2-hydroxy-phenyl)-3-cyclohexyl-thiourea
1-(3,5-Dichloro-2-hydroxy-phenyl)-3-(2-trifluoromethyl-phenyl)-thiourea
1-(3,5-Dichloro-2-hydroxy-phenyl)-3-phenyl-thiourea
1-(3-Chloro-2-hydroxy-phenyl)-3-pentyl-thiourea
1-Benzyl-3-(3-chloro-2-hydroxy-phenyl)-thiourea
1-(3-Chloro-2-hydroxy-phenyl)-3-(2-methyl-benzyl)-thiourea
1-(3-Chloro-2-hydroxy-phenyl)-3-phenethyl-thiourea
1-(3-Chloro-2-hydroxy-phenyl)-3-(1,1,3,3-tetramethyl-butyl)-thiourea
1-tert-Butyl-3-(3-Chloro-2-hydroxy-phenyl)-thiourea
1-(3-Chloro-2-hydroxy-phenyl)-3-isopropyl-thiourea
1-(3-Chloro-2-hydroxy-phenyl)-3-cyclohexylmethyl-thiourea
1-(3-Chloro-2-hydroxy-phenyl)-3-(4-trifluoromethyl-benzyl)-thiourea
1-(3-Chloro-2-hydroxy-phenyl)-3-(3,5-dichloro-phenyl)-thiourea
1-(3-Chloro-2-hydroxy-phenyl)-3-(4-chloro-phenyl)-thiourea
1-(3-Chloro-2-hydroxy-phenyl)-3-(4-trifluoromethyl-phenyl)-thiourea
1-(3-Chloro-2-hydroxy-phenyl)-3-cyclohexyl-thiourea
1-(3-Chloro-2-hydroxy-phenyl)-3-(2-trifluoromethyl-phenyl)-thiourea
1-(3-Chloro-2-hydroxy-phenyl)-3-phenyl-thiourea
1-(3-Fluoro-2-hydroxy-phenyl)-3-pentyl-thiourea
1-Benzyl-3-(3-fluoro-2-hydroxy-phenyl)-thiourea
1-(3-Fluoro-2-hydroxy-phenyl)-3-(2-methyl-benzyl)-thiourea
1-(3-Fluoro-2-hydroxy-phenyl)-3-phenethyl-thiourea
1-(3-Fluoro-2-hydroxy-phenyl)-3-(1,1,3,3-tetramethyl-butyl)-thiourea
1-tert-Butyl-3-(3-fluoro-2-hydroxy-phenyl)-thiourea
1-(3-Fluoro-2-hydroxy-phenyl)-3-isopropyl-thiourea
1-(3-Fluoro-2-hydroxy-phenyl)-3-cyclohexylmethyl-thiourea
1-(3-Fluoro-2-hydroxy-phenyl)-3-(4-trifluoromethyl-benzyl)-thiourea
1-(3-Fluoro-2-hydroxy-phenyl)-3-(3,5-dichloro-phenyl)-thiourea
1-(3-Fluoro-2-hydroxy-phenyl)-3-(4-chloro-phenyl)-thiourea
1-(3-Fluoro-2-hydroxy-phenyl)-3-(4-trifluoromethyl-phenyl)-thiourea
1-(3-Fluoro-2-hydroxy-phenyl)-3-cyclohexyl-thiourea
1-(3-Fluoro-2-hydroxy-phenyl)-3-(2-trifluoromethyl-phenyl)-thiourea
1-(3-Fluoro-2-hydroxy-phenyl)-3-phenyl-thiourea
1-(3,5-Dichloro-2-hydroxy-4-methyl-phenyl)-3-pentyl-thiourea
1-Benzyl-3-(3,5-dichloro-2-hydroxy-4-methyl-phenyl)-thiourea
1-(3,5-Dichloro-2-hydroxy-4-methyl-phenyl)-3-(2-methyl-benzyl)-thiourea
1-(3,5-Dichloro-2-hydroxy-4-methyl-phenyl)-3-phenethyl-thiourea
1-(3,5-Dichloro-2-hydroxy-4-methyl-phenyl)-3-(1,1,3,3-tetramethyl-butyl)-thiourea
1-tert-Butyl-3-(3,5-dichloro-2-hydroxy-4-methyl-phenyl)-thiourea
1-(5-Chloro-2-hydroxy-4-methyl-phenyl)-3-isopropyl-thiourea
1-(3,5-Dichloro-2-hydroxy-4-methyl-phenyl)-3-cyclohexylmethyl-thiourea
1-(3,5-Dichloro-2-hydroxy-4-methyl-phenyl)-3-(4-trifluoromethyl-benzyl)-thiourea
1-(3,5-Dichloro-2-hydroxy-4-methyl-phenyl)-3-(3,5-dichloro-phenyl)-thiourea
1-(3,5-Dichloro-2-hydroxy-4-methyl-phenyl)-3-(4-chloro-phenyl)-thiourea
1-(3,5-Dichloro-2-hydroxy-4-methyl-phenyl)-3-(4-trifluoromethyl-phenyl)-thiourea
1-(3,5-Dichloro-2-hydroxy-4-methyl-phenyl)-3-cyclohexyl-thiourea
1-(3,5-Dichloro-2-hydroxy-4-methyl-phenyl)-3-(2-trifluoromethyl-phenyl)-thiourea
1-(3,5-Dichloro-2-hydroxy-4-methyl-phenyl)-3-phenyl-thiourea

1-(5-Bromo-3-fluoro-2-hydroxy-phenyl)-3-pentyl-thiourea
1-Benzyl-3-(5-bromo-3-fluoro-2-hydroxy-phenyl)-thiourea
1-(5-Bromo-3-fluoro-2-hydroxy-phenyl)-3-(2-methyl-benzyl)-thiourea
1-(5-Bromo-3-fluoro-2-hydroxy-phenyl)-3-phenethyl-thiourea
1-(5-Bromo-3-fluoro-2-hydroxy-phenyl)-3-(1,1,3,3-tetramethyl-butyl)-thiourea
1-tert-Butyl-3-(5-bromo-3-fluoro-2-hydroxy-phenyl)-thiourea
1-(5-Bromo-3-fluoro-2-hydroxy-phenyl)-3-isopropyl-thiourea
1-(5-Bromo-3-fluoro-2-hydroxy-phenyl)-3-cyclohexylmethyl-thiourea
1-(5-Bromo-3-fluoro-2-hydroxy-phenyl)-3-(4-trifluoromethyl-benzyl)-thiourea
1-(5-Bromo-3-fluoro-2-hydroxy-phenyl)-3-(3,5-dichloro-phenyl)-thiourea
1-(5-Bromo-3-fluoro-2-hydroxy-phenyl)-3-(4-chloro-phenyl)-thiourea
1-(5-Bromo-3-fluoro-2-hydroxy-phenyl)-3-(4-trifluoromethyl-phenyl)-thiourea
1-(5-Bromo-3-fluoro-2-hydroxy-phenyl)-3-cyclohexyl-thiourea
1-(5-Bromo-3-fluoro-2-hydroxy-phenyl)-3-(2-trifluoromethyl-phenyl)-thiourea
1-(5-Bromo-3-fluoro-2-hydroxy-phenyl)-3-phenyl-thiourea
1-(3,4-Difluoro-2-hydroxy-phenyl)-3-pentyl-thiourea
1-Benzyl-3-(3,4-difluoro-2-hydroxy-phenyl)-thiourea
1-(3,4-Difluoro-2-hydroxy-phenyl)-3-(2-methyl-benzyl)-thiourea
1-(3,4-Difluoro-2-hydroxy-phenyl)-3-phenethyl-thiourea
1-(3,4-Difluoro-2-hydroxy-phenyl)-3-(1,1,3,3-tetramethyl-butyl)-thiourea
1-tert-Butyl-3-(3,4-Difluoro-2-hydroxy-phenyl)-thiourea
1-(3,4-Difluoro-2-hydroxy-phenyl)-3-isopropyl-thiourea
1-(3,4-Difluoro-2-hydroxy-phenyl)-3-cyclohexylmethyl-thiourea
1-(3,4-Difluoro-2-hydroxy-phenyl)-3-(4-trifluoromethyl-benzyl)-thiourea
1-(3,4-Difluoro-2-hydroxy-phenyl)-3-(3,5-dichloro-phenyl)-thiourea
1-(3,4-Difluoro-2-hydroxy-phenyl)-3-(4-chloro-phenyl)-thiourea
1-(3,4-Difluoro-2-hydroxy-phenyl)-3-(4-trifluoromethyl-phenyl)-thiourea
1-(3,4-Difluoro-2-hydroxy-phenyl)-3-cyclohexyl-thiourea
1-(3,4-Difluoro-2-hydroxy-phenyl)-3-(2-trifluoromethyl-phenyl)-thiourea
1-(3,4-Difluoro-2-hydroxy-phenyl)-3-phenyl-thiourea
1-[5-Chloro-2-hydroxy-3-(-hydroxy-ethyl)-phenyl]-3-pentyl-thiourea

1-Benzyl-3-[5-chloro-2-hydroxy-3-(1-hydroxy-ethyl)-phenyl]-thiourea
1-[5-Chloro-2-hydroxy-3-(1-hydroxy-ethyl)-phenyl]-3-(2-methyl-benzyl)-thiourea
1-[5-Chloro-2-hydroxy-3-(1-hydroxy-ethyl)-phenyl]-3-phenethyl-thiourea
1-[5-Chloro-2-hydroxy-3-(1-hydroxy-ethyl)-phenyl]-3-(1,1,3,3-tetramethyl-butyl)-thiourea
1-tert-Butyl-3-[5-chloro-2-hydroxy-3-(1-hydroxy-ethyl)-phenyl]-thiourea
1-[5-Chloro-2-hydroxy-3-(1-hydroxy-ethyl)-phenyl]-3-isopropyl-thiourea
1-[5-Chloro-2-hydroxy-3-(1-hydroxy-ethyl)-phenyl]-3-cyclohexylmethyl-thiourea
1-[5-Chloro-2-hydroxy-3-(1-hydroxy-ethyl)-phenyl]-3-(4-trifluoromethyl-benzyl)-thiourea
1-[5-Chloro-2-hydroxy-3-(1-hydroxy-ethyl)-phenyl]-3-(3,5-dichloro-phenyl)-thiourea
1-[5-Chloro-2-hydroxy-3-(1-hydroxy-ethyl)-phenyl]-3-(4-chloro-phenyl)-thiourea
1-[5-Chloro-2-hydroxy-3-(1-hydroxy-ethyl)-phenyl]-3-(4-trifluoromethyl-phenyl)-thiourea
1-[5-Chloro-2-hydroxy-3-(1-hydroxy-ethyl)-phenyl]-3-cyclohexyl-thiourea
1-[5-Chloro-2-hydroxy-3-(1-hydroxy-ethyl)-phenyl]-3-(2-trifluoromethyl-phenyl)-thiourea
1-[5-Chloro-2-hydroxy-3-(1-hydroxy-ethyl)-phenyl]-3-phenyl-thiourea
4-Chloro-2-(2-phenylsulfanyl-benzylamino)-phenol
4-Chloro-2-(2-p-tolylsulfanyl-benzylamino)-phenol
4-Chloro-2-[2-(4-chloro-phenylsulfanyl)-benzylamino]-phenol
4-Chloro-2-[2-(4-nitro-phenylsulfanyl)-benzylamino]-phenol
4-Chloro-2-[2-(4-methoxy-phenylsulfanyl)-benzylamino]-phenol
4-Chloro-2-[2-(2-chloro-phenylsulfanyl)-benzylamino]-phenol
4-Chloro-2-[2-(3-chloro-phenylsulfanyl)-benzylamino]-phenol
4-Chloro-2-[2-(3,4-dichloro-phenylsulfanyl)-benzylamino]-phenol
N-(4-{2-[(5-Chloro-2-hydroxy-phenylamino)-methyl]-phenylsulfanyl}-phenyl)-acetamide
4-Chloro-2-[2-(quinolin-7-ylsulfanyl)-benzylamino]-phenol
4-Chloro-2-[2-(4-chloro-phenylsulfanyl)-5-nitro-benzylamino]-phenol
4-Chloro-2-(5-nitro-2-p-tolylsulfanyl-benzylamino)-phenol

2-[5-Amino-2-(4-chloro-phenylsulfanyl)-benzylamino]-4-chloro-phenol
4-Chloro-2-[2-(4-chloro-benzenesulfonyl)-benzylamino]-phenol
2,4-Dichloro-6-(2-phenylsulfanyl-benzylamino)-phenol
2,4-Dichloro-6-(2-p-tolylsulfanyl-benzylamino)-phenol
2,4-Dichloro-6-[2-(4-chloro-phenylsulfanyl)-benzylamino]-phenol
2,4-Dichloro-6-[2-(4-nitro-phenylsulfanyl)-benzylamino]-phenol
2,4-Dichloro-6-[2-(4-methoxy-phenylsulfanyl)-benzylamino]-phenol
2,4-Dichloro-6-[2-(2-chloro-phenylsulfanyl)-benzylamino]-phenol
2,4-Dichloro-6-[2-(3-chloro-phenylsulfanyl)-benzylamino]-phenol
2,4-Dichloro-6-[2-(3,4-dichloro-phenylsulfanyl)-benzylamino]-phenol
N-(4-{2-[(3,5-Dichloro-2-hydroxy-phenylamino)-methyl]-phenylsulfanyl}-phenyl)-acetamide
2,4-Dichloro-6-[2-(quinolin-7-ylsulfanyl)-benzylamino]-phenol
2,4-Dichloro-6-[2-(4-chloro-phenylsulfanyl)-5-nitro-benzylamino]-phenol
2,4-Dichloro-6-(5-nitro-2-p-tolylsulfanyl-benzylamino)-phenol
2-[5-Amino-2-(4-chloro-phenylsulfanyl)-benzylamino]-4,6-dichloro-phenol
2,4-Dichloro-6-[2-(4-chloro-benzenesulfonyl)-benzylamino]-phenol
2-Chloro-6-(2-phenylsulfanyl-benzylamino)-phenol
2-Chloro-6-(2-p-tolylsulfanyl-benzylamino)-phenol
2-Chloro-6-[2-(4-chloro-phenylsulfanyl)-benzylamino]-phenol
2-Chloro-6-[2-(4-nitro-phenylsulfanyl)-benzylamino]-phenol
2-Chloro-6-[2-(4-methoxy-phenylsulfanyl)-benzylamino]-phenol
2-Chloro-6-[2-(2-chloro-phenylsulfanyl)-benzylamino]-phenol
2-Chloro-6-[2-(3-chloro-phenylsulfanyl)-benzylamino]-phenol
2-Chloro-6-[2-(3,4-dichloro-phenylsulfanyl)-benzylamino]-phenol
N-(4-{2-[(3-Chloro-2-hydroxy-phenylamino)-methyl]-phenylsulfanyl}-phenyl)-acetamide
2-Chloro-6-[2-(quinolin-7-ylsulfanyl)-benzylamino]-phenol
2-Chloro-6-[2-(4-chloro-phenylsulfanyl)-5-nitro-benzylamino]-phenol
2-Chloro-6-(5-nitro-2-p-tolylsulfanyl-benzylamino)-phenol
2-[5-Amino-2-(4-chloro-phenylsulfanyl)-benzylamino]-6-chloro-phenol
2-Chloro-6-[2-(4-chloro-benzenesulfonyl)-benzylamino]-phenol
2-Fluoro-6-(2-phenylsulfanyl-benzylamino)-phenol
2-Fluoro-6-(2-p-tolylsulfanyl-benzylamino)-phenol
2-Fluoro-6-[2-(4-chloro-phenylsulfanyl)-benzylamino]-phenol
2-Fluoro-6-[2-(4-nitro-phenylsulfanyl)-benzylamino]-phenol
2-Fluoro-6-[2-(4-methoxy-phenylsulfanyl)-benzylamino]-phenol
2-Fluoro-6-[2-(2-chloro-phenylsulfanyl)-benzylamino]-phenol
2-Fluoro-6-[2-(3-chloro-phenylsulfanyl)-benzylamino]-phenol
2-Fluoro-6-[2-(3,4-dichloro-phenylsulfanyl)-benzylamino]-phenol
N-(4-{2-[(3-Fluoro-2-hydroxy-phenylamino)-methyl]-phenylsulfanyl}-phenyl)-acetamide
2-Fluoro-6-[2-(quinolin-7-ylsulfanyl)-benzylamino]-phenol
2-Fluoro-6-[2-(4-chloro-phenylsulfanyl)-5-nitro-benzylamino]-phenol
2-Fluoro-6-(5-nitro-2-p-tolylsulfanyl-benzylamino)-phenol
2-[5-Amino-2-(4-chloro-phenylsulfanyl)-benzylamino]-6-fluoro-phenol
2-Fluoro-6-[2-(4-chloro-benzenesulfonyl)-benzylamino]-phenol
2,4-Dichloro-3-methyl-6-(2-phenylsulfanyl-benzylamino)-phenol
2,4-Dichloro-3-methyl-6-(2-p-tolylsulfanyl-benzylamino)-phenol
2,4-Dichloro-3-methyl-6-[2-(4-chloro-phenylsulfanyl)-benzylamino]-phenol
2,4-Dichloro-3-methyl-6-[2-(4-nitro-phenylsulfanyl)-benzylamino]-phenol
2,4-Dichloro-3-methyl-6-[2-(4-methoxy-phenylsulfanyl)-benzylamino]-phenol
2,4-Dichloro-3-methyl-6-[2-(2-chloro-phenylsulfanyl)-benzylamino]-phenol
2,4-Dichloro-3-methyl-6-[2-(3-chloro-phenylsulfanyl)-benzylamino]-phenol
2,4-Dichloro-3-methyl-6-[2-(3,4-dichloro-phenylsulfanyl)-benzylamino]-phenol
N-(4-{2-[(3,5-Dichloro-2-hydroxy-4-methyl-phenylamino)-methyl]-phenylsulfanyl}-phenyl)-acetamide
2,4-Dichloro-3-methyl-6-[2-(quinolin-7-ylsulfanyl)-benzylamino]-phenol
2,4-Dichloro-3-methyl-6-[2-(4-chloro-phenylsulfanyl)-5-nitro-benzylamino]-phenol
2,4-Dichloro-3-methyl-6-(5-nitro-2-p-tolylsulfanyl-benzylamino)-phenol
2-[5-Amino-2-(4-chloro-phenylsulfanyl)-benzylamino]-4,6-dichloro-5-methyl-phenol
2,4-Dichloro-3-methyl-6-[2-(4-chloro-benzenesulfonyl)-benzylamino]-phenol

4-Bromo-2-fluoro-6-(2-phenylsulfanyl-benzylamino)-phenol
4-Bromo-2-fluoro-6-(2-p-tolylsulfanyl-benzylamino)-phenol
4-Bromo-2-[2-(4-chloro-phenylsulfanyl)-benzylamino]-6-fluoro-phenol
4-Bromo-2-fluoro-6-[2-(4-nitro-phenylsulfanyl)-benzylamino]-phenol
4-Bromo-2-fluoro-6-[2-(4-methoxy-phenylsulfanyl)-benzylamino]-phenol
4-Bromo-2-[2-(2-chloro-phenylsulfanyl)-benzylamino]-6-fluoro-phenol
4-Bromo-2-[2-(3-chloro-phenylsulfanyl)-benzylamino]-6-fluoro-phenol
4-Bromo-2-[2-(3,4-dichloro-phenylsulfanyl)-benzylamino]-6-fluoro-phenol
N-(4-{2-[(5-Bromo-3-fluoro-2-hydroxy-phenylamino)-methyl]-phenylsulfanyl}-phenyl)-acetamide
4-Bromo-2-fluoro-6-[2-(quinolin-7-ylsulfanyl)-benzylamino]-phenol
4-Bromo-2-[2-(4-chloro-phenylsulfanyl)-5-nitro-benzylamino]-6-fluoro-phenol
4-Bromo-2-fluoro-6-(5-nitro-2-p-tolylsulfanyl-benzylamino)-phenol
2-[5-Amino-2-(4-chloro-phenylsulfanyl)-benzylamino]-4-bromo-6-fluoro-phenol
4-Bromo-2-[2-(4-chloro-benzenesulfonyl)-benzylamino]-6-fluoro-phenol
2,3-Difluoro-6-(2-phenylsulfanyl-benzylamino)-phenol
2,3-Difluoro-6-(2-p-tolylsulfanyl-benzylamino)-phenol
6-[2-(4-Chloro-phenylsulfanyl)-benzylamino]-2,3-difluoro-phenol
2,3-Difluoro-6-[2-(4-nitro-phenylsulfanyl)-benzylamino]-phenol
2,3-Difluoro-6-[2-(4-methoxy-phenylsulfanyl)-benzylamino]-phenol
6-[2-(2-Chloro-phenylsulfanyl)-benzylamino]-2,3-difluoro-phenol
6-[2-(3-Chloro-phenylsulfanyl)-benzylamino]-2,3-difluoro-phenol
6-[2-(3,4-Dichloro-phenylsulfanyl)-benzylamino]-2,3-difluoro-phenol
N-(4-{2-[(3,4-Difluoro-2-hydroxy-phenylamino)-methyl]-phenylsulfanyl}-phenyl)-acetamide

2,3-Difluoro-6-[2-(quinolin-7-ylsulfanyl)-benzylamino]-phenol
6-[2-(4-Chloro-phenylsulfanyl)-5-nitro-benzylamino]-2,3-difluoro-phenol
2,3-Difluoro-6-(5-nitro-2-p-tolylsulfanyl-benzylamino)-phenol
6-[5-Amino-2-(4-chloro-phenylsulfanyl)-benzylamino]-2,3-difluoro-phenol
6-[2-(4-Chloro-benzenesulfonyl)-benzylamino]-2,3-difluoro-phenol
4-Chloro-2-(1-hydroxy-ethyl)-6-(2-phenylsulfanyl-benzylamino)-phenol
4-Chloro-2-(1-hydroxy-ethyl)-6-(2-p-tolylsulfanyl-benzylamino)-phenol
4-Chloro-2-[2-(4-chloro-phenylsulfanyl)-benzylamino]-6-(1-hydroxy-ethyl)-phenol
4-Chloro-2-(1-hydroxy-ethyl)-6-[2-(4-nitro-phenylsulfanyl)-benzylamino]-phenol
4-Chloro-2-(1-hydroxy-ethyl)-6-[2-(4-methoxy-phenylsulfanyl)-benzylamino]-phenol
4-Chloro-2-[2-(2-chloro-phenylsulfanyl)-benzylamino]-6-(1-hydroxy-ethyl)-phenol
4-Chloro-2-[2-(3-chloro-phenylsulfanyl)-benzylamino]-6-(1-hydroxy-ethyl)-phenol
4-Chloro-2-[2-(3,4-dichloro-phenylsulfanyl)-benzylamino]-6-(1-hydroxy-ethyl)-phenol
N-[4-(2-{[5-Chloro-2-hydroxy-3-(1-hydroxy-ethyl)-phenylamino]-methyl}-phenylsulfanyl)-phenyl]-acetamide
4-Chloro-2-(1-hydroxy-ethyl)-6-[2-(quinolin-7-ylsulfanyl)-benzylamino]-phenol
4-Chloro-2-[2-(4-chloro-phenylsulfanyl)-5-nitro-benzylamino]-6-(1-hydroxy-ethyl)-phenol
4-Chloro-2-(1-hydroxy-ethyl)-6-(5-nitro-2-p-tolylsulfanyl-benzylamino)-phenol
2-[5-Amino-2-(4-chloro-phenylsulfanyl)-benzylamino]-4-chloro-6-(1-hydroxy-ethyl)-phenol
4-Chloro-2-[2-(4-chloro-benzenesulfonyl)-benzylamino]-6-(1-hydroxy-ethyl)-phenol
2-Hydroxymethyl-6-(2-phenylsulfanyl-benzylamino)-phenol
2-Hydroxymethyl-6-(2-p-tolylsulfanyl-benzylamino)-phenol
2-[2-(4-Chloro-phenylsulfanyl)-benzylamino]-6-hydroxymethyl-phenol
2-Hydroxymethyl-6-[2-(4-nitro-phenylsulfanyl)-benzylamino]-phenol
2-Hydroxymethyl-6-[2-(4-methoxy-phenylsulfanyl)-benzylamino]-phenol
2-[2-(2-Chloro-phenylsulfanyl)-benzylamino]-6-hydroxymethyl-phenol
2-[2-(3-Chloro-phenylsulfanyl)-benzylamino]-6-hydroxymethyl-phenol
2-[2-(3,4-Dichloro-phenylsulfanyl)-benzylamino]-6-hydroxymethyl-phenol
N-(4-{2-[(2-Hydroxy-3-hydroxymethyl-phenylamino)-methyl]-phenylsulfanyl}-phenyl)-acetamide
2-Hydroxymethyl-6-[2-(quinolin-7-ylsulfanyl)-benzylamino]-phenol
2-[2-(4-Chloro-phenylsulfanyl)-5-nitro-benzylamino]-6-hydroxymethyl-phenol
2-Hydroxymethyl-6-(5-nitro-2-p-tolylsulfanyl-benzylamino)-phenol
2-[5-Amino-2-(4-chloro-phenylsulfanyl)-benzylamino]-6-hydroxymethyl-phenol
2-[2-(4-Chloro-benzenesulfonyl)-benzylamino]-6-hydroxymethyl-phenol
2,4-Dichloro-3-ethyl-6-(2-phenylsulfanyl-benzylamino)-phenol

2,4-Dichloro-3-ethyl-6-(2-p-tolylsulfanyl-benzylamino)-phenol
2,4-Dichloro-3-ethyl-6-[2-(4-chloro-phenylsulfanyl)-benzylamino]-phenol
2,4-Dichloro-3-ethyl-6-[2-(4-nitro-phenylsulfanyl)-benzylamino]-phenol
2,4-Dichloro-3-ethyl-6-[2-(4-methoxy-phenylsulfanyl)-benzylamino]-phenol
2,4-Dichloro-3-ethyl-6-[2-(2-chloro-phenylsulfanyl)-benzylamino]-phenol
2,4-Dichloro-3-ethyl-6-[2-(3-chloro-phenylsulfanyl)-benzylamino]-phenol
2,4-Dichloro-3-ethyl-6-[2-(3,4-dichloro-phenylsulfanyl)-benzylamino]-phenol
N-(4-{2-[(3,5-Dichloro-4-ethyl-2-hydroxy-phenylamino)-methyl]-phenylsulfanyl}-phenyl)-acetamide
2,4-Dichloro-3-ethyl-6-[2-(quinolin-7-ylsulfanyl)-benzylamino]-phenol
2,4-Dichloro-3-ethyl-6-[2-(4-chloro-phenylsulfanyl)-5-nitro-benzylamino]-phenol
2,4-Dichloro-3-ethyl-6-(5-nitro-2-p-tolylsulfanyl-benzylamino)-phenol
2-[5-Amino-2-(4-chloro-phenylsulfanyl)-benzylamino]-4,6-dichloro-5-ethyl-phenol
2,4-Dichloro-3-ethyl-6-[2-(4-chloro-benzenesulfonyl)-benzylamino]-phenol
2-Hydroxy-3-(2-phenylsulfanyl-benzylamino)-benzoic acid
2-Hydroxy-3-(2-p-tolylsulfanyl-benzylamino)-benzoic acid
3-[2-(4-Chloro-phenylsulfanyl)-benzylamino]-2-hydroxy-benzoic acid
2-Hydroxy-3-[2-(4-nitro-phenylsulfanyl)-benzylamino]-benzoic acid
2-Hydroxy-3-[2-(4-methoxy-phenylsulfanyl)-benzylamino]-benzoic acid
3-[2-(2-Chloro-phenylsulfanyl)-benzylamino]-2-hydroxy-benzoic acid
3-[2-(3-Chloro-phenylsulfanyl)-benzylamino]-2-hydroxy-benzoic acid
3-[2-(3,4-Dichloro-phenylsulfanyl)-benzylamino]-2-hydroxy-benzoic acid
3-[2-(4-Acetylamino-phenylsulfanyl)-benzylamino]-2-hydroxy-benzoic acid
2-Hydroxy-3-[2-(quinolin-7-ylsulfanyl)-benzylamino]-benzoic acid
3-[2-(4-Chloro-phenylsulfanyl)-5-nitro-benzylamino]-2-hydroxy-benzoic acid
2-Hydroxy-3-(5-nitro-2-p-tolylsulfanyl-benzylamino)-benzoic acid
2-[5-Amino-2-(4-chloro-phenylsulfanyl)-benzylamino]-6-hydroxymethyl-phenol
3-[2-(4-Chloro-benzenesulfonyl)-benzylamino]-2-hydroxy-benzoic acid
2-Fluoro-4-nitro-6-(2-phenylsulfanyl-benzylamino)-phenol
2-Fluoro-4-nitro-6-(2-p-tolylsulfanyl-benzylamino)-phenol
2-[2-(4-Chloro-phenylsulfanyl)-benzylamino]-6-fluoro-4-nitro-phenol
2-Fluoro-4-nitro-6-[2-(4-nitro-phenylsulfanyl)-benzylamino]-phenol
2-Fluoro-6-[2-(4-methoxy-phenylsulfanyl)-benzylamino]-4-nitro-phenol
2-[2-(2-Chloro-phenylsulfanyl)-benzylamino]-6-fluoro-4-nitro-phenol
2-[2-(3-Chloro-phenylsulfanyl)-benzylamino]-6-fluoro-4-nitro-phenol
2-[2-(3,4-Dichloro-phenylsulfanyl)-benzylamino]-6-fluoro-4-nitro-phenol
N-(4-{2-[(3-Fluoro-2-hydroxy-5-nitro-phenylamino)-methyl]-phenylsulfanyl}-phenyl)-acetamide
2-Fluoro-4-nitro-6-[2-(quinolin-7-ylsulfanyl)-benzylamino]-phenol
2-[2-(4-Chloro-phenylsulfanyl)-5-nitro-benzylamino]-6-fluoro-4-nitro-phenol
2-Fluoro-4-nitro-6-(5-nitro-2-p-tolylsulfanyl-benzylamino)-phenol
2-[S-Amino-2-(4-chloro-phenylsulfanyl)-benzylamino]-6-fluoro-4-nitro-phenol
2-[2-(4-Chloro-benzenesulfonyl)-benzylamino]-6-fluoro-4-nitro-phenol
2,4-Dichloro-6-(3-phenoxy-benzylamino)-phenol
2,4-Dichloro-6-[3-(4-chloro-phenoxy)-benzylamino]-phenol
2-[3-(4-tert-Butyl-phenoxy)-benzylamino]-4,6-dichloro-phenol
2-(3-Benzyloxy-benzylamino)-4,6-dichloro-phenol
2-(2-Benzyloxy-benzylamino)-4,6-dichloro-phenol
2,4-Dichloro-6-[(naphthalen-1-ylmethyl)-amino]-phenol
2,4-Dichloro-6-(4-methylsulfanyl-benzylamino)-phenol
2,4-Dichloro-6-(2-ethylsulfanyl-benzylamino)-phenol
2,4-Dichloro-6-(2-morpholin-4-yl-benzylamino)-phenol
2,4-Dichloro-6-{[2-(4-chloro-phenylsulfanyl)-thiophen-3-ylmethyl]-amino}-phenol
2,4-Dichloro-6-[(5-phenyl-2H-imidazol-4-ylmethyl)-amino]-phenol
2-[(5-Bromo-thiophen-2-ylmethyl)-amino]-4,6-dichloro-phenol
2,4-Dichloro-6-[3-(4-methoxy-phenoxy)-benzylamino]-phenol
2,4-Dichloro-6-(3-methyl-benzylamino)-phenol
2,4-Dichloro-6-(3-trifluoromethyl-benzylamino)-phenol
2,4-Dichloro-6-(2-chloro-6-fluoro-benzylamino)-phenol
2,4-Dichloro-3-methyl-6-(3-phenoxy-benzylamino)-phenol

2,4-Dichloro-3-methyl-6-[3-(4-chloro-phenoxy)-benzylamino]-phenol
2-[3-(4-tert-Butyl-phenoxy)-benzylamino]-4,6-dichloro-3-methyl-phenol
2-(3-Benzyloxy-benzylamino)-4,6-dichloro-3-methyl-phenol
2-(2-Benzyloxy-benzylamino)-4,6-dichloro-3-methyl-phenol
2,4-Dichloro-3-methyl-6-[(naphthalen-1-ylmethyl)-amino]-phenol
2,4-Dichloro-3-methyl-6-(4-methylsulfanyl-benzylamino)-phenol
2,4-Dichloro-3-methyl-6-(2-ethylsulfanyl-benzylamino)-phenol
2,4-Dichloro-3-methyl-6-(2-morpholin-4-yl-benzylamino)-phenol
2,4-Dichloro-3-methyl-6-{[2-(4-chloro-phenylsulfanyl)-thiophen-3-ylmethyl]-amino}-phenol
2,4-Dichloro-3-methyl-6-[(5-phenyl-2H-imidazol-4-ylmethyl)-amino]-phenol
2-[(5-Bromo-thiophen-2-ylmethyl)-amino]-4,6-dichloro-3-methyl-phenol
2,4-Dichloro-6-[3-(4-methoxy-phenoxy)-benzylamino]-3-methyl-phenol
2,4-Dichloro-6-(3-methyl-benzylamino)-3-methyl-phenol
2,4-Dichloro-3-methyl-6-(3-trifluoromethyl-benzylamino)-phenol

2,4-Dichloro-3-methyl-6-(2-chloro-6-fluoro-benzylamino)-phenol
2-Chloro-6-(3-phenoxy-benzylamino)-phenol
2-Chloro-6-[3-(4-chloro-phenoxy)-benzylamino]-phenol
2-[3-(4-tert-Butyl-phenoxy)-benzylamino]-6-chloro-phenol
2-(3-Benzyloxy-benzylamino)-6-chloro-phenol
2-(2-Benzyloxy-benzylamino)-6-chloro-phenol
2-Chloro-6-[(naphthalen-1-ylmethyl)-amino]-phenol
2-Chloro-6-(4-methylsulfanyl-benzylamino)-phenol
2-Chloro-6-(2-ethylsulfanyl-benzylamino)-phenol
2-Chloro-6-(2-morpholin-4-yl-benzylamino)-phenol
2-Chloro-6-{[2-(4-chloro-phenylsulfanyl)-thiophen-3-ylmethyl]-amino}-phenol
2-Chloro-6-[(5-phenyl-2H-imidazol-4-ylmethyl)-amino]-phenol
2-[(5-Bromo-thiophen-2-ylmethyl)-amino]-6-chloro-phenol
2-Chloro-6-[3-(4-methoxy-phenoxy)-benzylamino]-phenol
2-Chloro-6-(3-methyl-benzylamino)-phenol
2-Chloro-6-(3-trifluoromethyl-benzylamino)-phenol
2-Chloro-6-(2-chloro-6-fluoro-benzylamino)-phenol
2-Fluoro-6-(3-phenoxy-benzylamino)-phenol
2-Fluoro-6-[3-(4-chloro-phenoxy)-benzylamino]-phenol
2-[3-(4-tert-Butyl-phenoxy)-benzylamino]-6-fluoro-phenol
2-(3-Benzyloxy-benzylamino)-6-fluoro-phenol
2-(2-Benzyloxy-benzylamino)-6-fluoro-phenol
2-Fluoro-6-[(naphthalen-1-ylmethyl)-amino]-phenol
2-Fluoro-6-(4-methylsulfanyl-benzylamino)-phenol
2-Fluoro-6-(2-ethylsulfanyl-benzylamino)-phenol
2-Fluoro-6-(2-morpholin-4-yl-benzylamino)-phenol
2-Fluoro-6-{[2-(4-chloro-phenylsulfanyl)-thiophen-3-ylmethyl]-amino}-phenol
2-Fluoro-6-[(5-phenyl-2H-imidazol-4-ylmethyl)-amino]-phenol
2-[(5-Bromo-thiophen-2-ylmethyl)-amino]-6-fluoro-phenol
2-Fluoro-6-[3-(4-methoxy-phenoxy)-benzylamino]-phenol
2-Fluoro-6-(3-methyl-benzylamino)-phenol
2-Fluoro-6-(3-trifluoromethyl-benzylamino)-phenol
2-Fluoro-6-(2-chloro-6-fluoro-benzylamino)-phenol
2,3-Difluoro-6-(3-phenoxy-benzylamino)-phenol
2,3-Difluoro-6-[3-(4-chloro-phenoxy)-benzylamino]-phenol
2-[3-(4-tert-Butyl-phenoxy)-benzylamino]-5,6-difluoro-phenol
2-(3-Benzyloxy-benzylamino)-5,6-difluoro-phenol
2-(2-Benzyloxy-benzylamino)-5,6-difluoro-phenol
2,3-Difluoro-6-[(naphthalen-1-ylmethyl)-amino]-phenol
2,3-Difluoro-6-(4-methylsulfanyl-benzylamino)-phenol
2,3-Difluoro-6-(2-ethylsulfanyl-benzylamino)-phenol
2,3-Difluoro-6-(2-morpholin-4-yl-benzylamino)-phenol
2,3-Difluoro-6-{[2-(4-chloro-phenylsulfanyl)-thiophen-3-ylmethyl]-amino}-phenol

2,3-Difluoro-6-[(5-phenyl-2H-imidazol-4-ylmethyl)-amino]-phenol
2-[(5-Bromo-thiophen-2-ylmethyl)-amino]-5,6-difluoro-phenol
2,3-Difluoro-6-[3-(4-methoxy-phenoxy)-benzylamino]-phenol
2,3-Difluoro-6-(3-methyl-benzylamino)-phenol
2,3-Difluoro-6-(3-trifluoromethyl-benzylamino)-phenol
2,3-Difluoro-6-(2-chloro-6-fluoro-benzylamino)-phenol
N-(3,5-Dichloro-2-hydroxy-4-methyl-phenyl)-C-phenyl-methanesulfonamide
Butane-1-sulfonic acid (3,5-dichloro-2-hydroxy-4-methyl-phenyl)-amide
Octane-1-sulfonic acid (3,5-dichloro-2-hydroxy-4-methyl-phenyl)-amide
Propane-2-sulfonic acid (3,5-dichloro-2-hydroxy-4-methyl-phenyl)-amide
N-(3,5-Dichloro-2-hydroxy-phenyl)-C-phenyl-methanesulfonamide
Butane-1-sulfonic acid (3,5-dichloro-2-hydroxy-phenyl)-amide
Octane-1-sulfonic acid (3,5-dichloro-2-hydroxy-phenyl)-amide
Propane-2-sulfonic acid (3,5-dichloro-2-hydroxy-phenyl)-amide
N-(3-Chloro-2-hydroxy-phenyl)-C-phenyl-methanesulfonamide
Butane-1-sulfonic acid (3-chloro-2-hydroxy-phenyl)-amide
Octane-1-sulfonic acid (3-chloro-2-hydroxy-phenyl)-amide
Propane-2-sulfonic acid (3-chloro-2-hydroxy-phenyl)-amide
N-(3-Fluoro-2-hydroxy-phenyl)-C-phenyl-methanesulfonamide
Butane-1-sulfonic acid (3-fluoro-2-hydroxy-phenyl)-amide
Octane-1-sulfonic acid (3-fluoro-2-hydroxy-phenyl)-amide
Propane-2-sulfonic acid (3-fluoro-2-hydroxy-phenyl)-amide
N-(3,4-Difluoro-2-hydroxy-phenyl)-C-phenyl-methanesulfonamide
Butane-1-sulfonic acid (3,4-difluoro-2-hydroxy-phenyl)-amide
Octane-1-sulfonic acid (3,4-difluoro-2-hydroxy-phenyl)-amide
Propane-2-sulfonic acid (3,4-difluoro-2-hydroxy-phenyl)-amide
(3,5-Dichloro-2-hydroxy-4-methyl-phenyl)-carbamic acid hexyl ester
(3,5-Dichloro-2-hydroxy-phenyl)-carbamic acid hexyl ester
(3-Chloro-2-hydroxy-phenyl)-carbamic acid hexyl ester
(3-Fluoro-2-hydroxy-phenyl)-carbamic acid hexyl ester
(5-Bromo-3-fluoro-2-hydroxy-phenyl)-carbamic acid hexyl ester
(3,4-Difluoro-2-hydroxy-phenyl)-carbamic acid hexyl ester
2-[3-(3,5-Dichloro-2-hydroxy-4-methyl-phenyl)-ureido]-4-methyl-pentanoic acid ethyl ester
2-[3-(3,5-Dichloro-2-hydroxy-phenyl)-ureido]-4-methyl-pentanoic acid ethyl ester
2-[3-(3-Chloro-2-hydroxy-phenyl)-ureido]-4-methyl-pentanoic acid ethyl ester
2-[3-(3-Fluoro-2-hydroxy-phenyl)-ureido]-4-methyl-pentanoic acid ethyl ester
2-[3-(3,4-Difluoro-2-hydroxy-4-phenyl)-ureido]-4-methyl-pentanoic acid ethyl ester
2-[3-(5-Bromo-3-fluoro-2-hydroxy-4-methyl-phenyl)-ureido]-4-methyl-pentanoic acid ethyl ester
2-[3-(3,5-Dichloro-2-hydroxy-4-methyl-phenyl)-ureido]-3-phenyl-propionic acid ethyl ester
2-[3-(3,5-Dichloro-2-hydroxy-phenyl)-ureido]-3-phenyl-propionic acid ethyl ester
2-[3-(3-Chloro-2-hydroxy-phenyl)-ureido]-3-phenyl-propionic acid ethyl ester
2-[3-(3-Fluoro-2-hydroxy-phenyl)-ureido]-3-phenyl-propionic acid ethyl ester
2-[3-(3,4-Difluoro-2-hydroxy-phenyl)-ureido]-3-phenyl-propionic acid ethyl ester
2-[3-(5-Bromo-3-fluoro-2-hydroxy-phenyl)-ureido]-3-phenyl-propionic acid ethyl ester
3,5,5-Trimethyl-hexanoic acid (3,5-dichloro-2-hydroxy-4-methyl-phenyl)-amide
3,5,5-Trimethyl-hexanoic acid (3,5-dichloro-2-hydroxy-phenyl)-amide
3,5,5-Trimethyl-hexanoic acid (3-chloro-2-hydroxy-phenyl)-amide
3,5,5-Trimethyl-hexanoic acid (3-fluoro-2-hydroxy-phenyl)-amide
3,5,5-Trimethyl-hexanoic acid (3,4-difluoro-2-hydroxy-phenyl)-amide
3,5,5-Trimethyl-hexanoic acid (5-bromo-3-fluoro-2-hydroxy-phenyl)-amide
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-{3-Chloro-5-[2-(4-chloro-phenylsulfanyl)-benzylamino]-4-hydroxy-phenyl}-3-cyclohexyl-urea
1-{3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
1-tert-Butyl-3-[3-chloro-2-hydroxy-5-(3-phenethyl-ureido)-phenyl]-urea
[3-Chloro-2-hydroxy-5-(3-phenethyl-ureido)-phenyl]-carbamic acid isobutyl ester
[3-Chloro-2-hydroxy-5-(3-phenethyl-ureido)-phenyl]-carbamic acid sec-butyl ester

Cyclopentanecarboxylic acid [3-chloro-2-hydroxy-5-(3-phenethyl-ureido)-phenyl]-amide
Cyclohexanecarboxylic acid [3-chloro-2-hydroxy-5-(3-phenethyl-ureido)-phenyl]-amide
1-tert-Butyl-3-{3-chloro-2-hydroxy-5-[3-(1,1,3,3-tetramethyl-butyl)-ureido]-phenyl}-urea
{3-Chloro-2-hydroxy-5-[3-(1,1,3,3-tetramethyl-butyl)-ureido]-phenyl}-carbamic acid isobutyl ester

{3-Chloro-2-hydroxy-5-[3-(1,1,3,3-tetramethyt-butyl)-ureido]-phenyl}-carbamic acid sec-butyl ester
Cyclopropanecarboxylic acid {3-chloro-2-hydroxy-5-[3-(1,1,3,3-tetramethyl-butyl)-ureido]-phenyl}-amide
Cyclobutanecarboxylic acid {3-chloro-2-hydroxy-5-[3-(1,1,3,3-tetramethyl-butyl)-ureido]-phenyl}-amide
Cyclopentanecarboxylic acid {3-chloro-2-hydroxy-5-[3-(1,1,3,3-tetramethyl-butyl)-ureido]-phenyl}-amide
Cyclohexanecarboxylic acid {3-chloro-2-hydroxy-5-[3-(1,1,3,3-tetramethyl-butyl)-ureido]-phenyl}-amide
N-[3-(3-tert-Butyl-ureido)-5-chloro-4-hydroxy-phenyl]-3-phenyl-propionamide
[3-Chloro-2-hydroxy-5-(3-phenyl-propionylamino)-phenyl]-carbamic acid isobutyl ester
[3-Chloro-2-hydroxy-5-(3-phenyl-propionylamino)-phenyl]-carbamic acid sec-butyl ester
Cyclopropanecarboxylic acid [3-chloro-2-hydroxy-5-(3-phenyl-propionylamino)-phenyl]-amide
Cyclobutanecarboxylic acid [3-chloro-2-hydroxy-5-(3-phenyl-propionylamino)-phenyl]-amide
Cyclopentanecarboxylic acid [3-chloro-2-hydroxy-5-(3-phenyl-propionylamino)-phenyl]-amide
Cyclohexanecarboxylic acid [3-chloro-2-hydroxy-5-(3-phenyl-propionylamino)-phenyl]-amide
1-Cyclopentyl-3-(3,5-dichloro-2-hydroxy-4-methyl-phenyl)-thiourea
2-[2-(4-Chloro-phenylsulfanyl)-benzylamino]-phenol
1-Benzyl-3-{3-chloro-5-[2-(4-chloro-phenylsulfanyl)-benzylamino]-4-hydroxy-phenyl}-urea
1-{3-Chloro-5-[2-(4-chloro-phenylsulfanyl)-benzylamino]-4-hydroxy-phenyl}-3-phenethyl-urea
1-{3-Chloro-5-[2-(4-chloro-phenylsulfanyl)-benzylamino]-4-hydroxy-phenyl}-3-(4-chlorophenyl)-urea
Ethanesulfonic acid [3-chloro-5-(3-cyclohexyl-ureido)-2-hydroxy-phenyl]-amide
N-[3-Chloro-5-(3-cyclohexyl-ureido)-2-hydroxy-phenyl]-3,3-dimethyl-butyramide
1-(5-Benzothiazol-2-yl-3-chloro-2-hydroxy-phenyl)-3-tert-butyl-urea
1-(5-Benzothiazol-2-yl-3-chloro-2-hydroxy-phenyl)-3-benzyl-urea
1-(5-Benzothiazol-2-yl-3-chloro-2-hydroxy-phenyl)-3-phenethyl-urea
1-(5-Benzothiazol-2-yl-3-chloro-2-hydroxy-phenyl)-3-isopropyl-thiourea
1-(5-Benzothiazol-2-yl-3-chloro-2-hydroxy-phenyl)-3-tert-butyl-thiourea
3,5,5-Trimethyl-hexanoic acid (5-benzothiazol-2-yl-3-chloro-2-hydroxy-phenyl)-amide
N-(5-Benzothiazol-2-yl-3-chloro-2-hydroxy-phenyl)-3-phenyl-propionamide
1-(2-Hydroxy-4-methyl-phenyl)-3-pentyl-urea
Biphenyl-4-carboxylic acid (3,5-dichloro-2-hydroxy-4-methyl-phenyl)-amide
Biphenyl-4-carboxylic acid (3,5-dichloro-2-hydroxy-phenyl)-amide
2,4-Dichloro-6-[(furan-2-ylmethyl)-amino]-3-methyl-phenol
2,4-Dichloro-6-[(furan-2-ylmethyl)-amino]-phenol
2,3-Difluoro-6-[(furan-2-ylmethyl)-amino]-phenol
2,4-Dichloro-3-methyl-6-(2-trifluoromethyl-benzylamino)-phenol
2,4-Dichloro-6-(2-trifluoromethyl-benzylamino)-phenol
2,3-Difluoro-6-(2-trifluoromethyl-benzylamino)-phenol
1-{3-Chloro-5-[2-(4-chloro-phenylsulfanyl)-benzylamino]-4-hydroxy-phenyl}-3-(2,6-dichloro-pyridin-4-yl)-urea
1-(5-Benzothiazol-2-yl-3-chloro-2-hydroxy-phenyl)-3-cyclopentyl-thiourea
1-{3-Chloro-5-[2-(4-chloro-phenylsulfanyl)-benzylamino]-4-hydroxy-phenyl}-3-morpholin-4-yl-urea
6-Benzylamino-2,4-dichloro-3-methyl-phenol
1-[2-(1H-Benzoimidazol-2-yl)-ethyl]-3-(3,5-dichloro-2-hydroxy-4-methyl-phenyl)-urea
1-(5-Benzothiazol-2-yl-3-chloro-2-hydroxy-phenyl)-3-cyclopentyl-thiourea
1-[5-Chloro-2-hydroxy-3-(2-phenylsulfanyl-benzylamino)-phenyl]-ethanone
1-[5-Chloro-2-hydroxy-3-(2-p-tolylsulfanyl-benzylamino)-phenyl]-ethanone
1-{5-Chloro-3-[2-(4-chloro-phenylsulfanyl)-benzylamino]-2-hydroxy-phenyl}-ethanone
1-{5-Chloro-2-hydroxy-3-[2-(4-nitro-phenylsulfanyl)-benzylamino]-phenyl}-ethanone
1-{5-Chloro-2-hydroxy-3-[2-(4-methoxy-phenylsulfanyl)-benzylamino]-phenyl}-ethanone
1-{5-Chloro-3-[2-(2-chloro-phenylsulfanyl)-benzylamino]-2-hydroxy-phenyl}-ethanone
1-{5-Chloro-3-[2-(3-chloro-phenylsulfanyl)-benzylamino]-2-hydroxy-phenyl}-ethanone
1-{5-Chloro-3-[2-(3,4-dichloro-phenylsulfanyl)-benzylamino]-2-hydroxy-phenyl}-ethanone
N-(4-{2-[(3-Acetyl-5-chloro-2-hydroxy-phenylamino)-methyl]-phenylsulfanyl}-phenyl)-acetamide
1-{5-Chloro-2-hydroxy-3-[2-(quinolin-7-ylsulfanyl)-benzylamino]-phenyl}-ethanone
1-{5-Chloro-3-[2-(4-chloro-phenylsulfanyl)-5-nitro-benzylamino]-2-hydroxy-phenyl}-ethanone
1-[5-Chloro-2-hydroxy-3-(5-nitro-2-p-tolylsulfanyl-benzylamino)-phenyl]-ethanone

1-{3-[5-Amino-2-(4-chloro-phenylsulfanyl)-benzylammo]-5-chtoro-2-hydroxy-phenyl}ethanone
1-{5-Chloro-3-[2-(4-chloro-benzenesulfonyl)-benzylamino]-2-hydroxy-phenyl}-ethanone
4-[2-(4-Chloro-phenylsulfanyl)-benzylamino]-benzene-1,3-diol
1,6-Di-(3,5-Dichloro-2-hydroxy-4-methyl-phenyl)-3-hexyl-urea
3-[3-(3-Chloro-4-hydroxy-phenyl)-ureido]-propionic acid ethyl ester
1-(3-Chloro-4-hydroxy-phenyl)-3-pentyl-urea
1-Benzyl-3-(3-chloro-4-hydroxy-phenyl)-urea
1-(3-Chloro-4-hydroxy-phenyl)-3-(2-methyl-benzyl)-urea
1-(3-Chloro-4-hydroxy-phenyl)-3-phenethyl-urea
1-(3-Chloro-4-hydroxy-phenyl)-3-(1,1,3,3-tetramethyl-butyl)-urea
1-tert-Butyl-3-(3-chloro-4-hydroxy-phenyl)-urea
1-(3-Chloro-4-hydroxy-phenyl)-3-cyclohexylmethyl-urea
1-(3-Chloro-4-hydroxy-phenyl)-3-(4-trifluoromethyl-benzyl)-urea
1-(3-Chloro-3-hydroxy-phenyl)-3-(3,5-dichloro-phenyl)-urea
1-(3-Chloro-4-hydroxy-phenyl)-3-(4-chloro-phenyl)-urea
1-(3-Chloro-4-hydroxy-phenyl)-3-(4-trifluoromethyl-phenyl)-urea
1-(3-Chloro-4-hydroxy-phenyl)-3-cyclohexyl-urea
1-(3-Chloro-4-hydroxy-phenyl)-3-(4-trifluoromethoxy-phenyl)-urea
1-(3-Chloro-4-hydroxy-phenyl)-3-(4-cyano-phenyl)-urea
1-Benzo[1,3]dioxol-5-yl-3-(3-chloro-4-hydroxy-phenyl)-urea
1-(3-Chloro-4-hydroxy-phenyl)-3-o-tolyl-urea
1-(3-Chloro-4-hydroxy-phenyl)-3-(3-methoxy-phenyl)-urea
1-(3-Chloro-4-hydroxy-phenyl)-3-(2,6-dimethyl-phenyl)-urea
1-(3-Chloro-4-hydroxy-phenyl)-3-(3,4,5-trimethoxy-phenyl)-urea
1-(3-Chloro-4-hydroxy-phenyl)-3-naphthalen-1-yl-urea
1-Adamantan-1-yl-3-(3-chloro-4-hydroxy-phenyl)-urea
1-(3-Chloro-4-hydroxy-phenyl)-3-(4-phenoxy-phenyl)-urea
1-(3-Chloro-4-hydroxy-phenyl)-3-phenyl-urea
3-[3-(3,5-Dichloro-4-hydroxy-phenyl)-ureido]-propionic acid ethyl ester
1-(3,5-Dichloro-4-hydroxy-phenyl)-3-pentyl-urea
1-Benzyl-3-(3,5-dichloro-4-hydroxy-phenyl)-urea
1-(3,5-Dichloro-4-hydroxy-phenyl)-3-(2-methyl-benzyl)-urea
1-(3,5-Dichloro-4-hydroxy-phenyl)-3-phenethyl-urea
1-(3,5-Dichloro-4-hydroxy-phenyl)-3-(1,1,3,3-tetramethyl-butyl)-urea
1-tert-Butyl-3-(3,5-dichloro-4-hydroxy-phenyl)-urea
1-(3,5-Dichloro-4-hydroxy-phenyl)-3-cyclohexylmethyl-urea
1-(3,5-Dichloro-4-hydroxy-phenyl)-3-(4-trifluoromethyl-benzyl)-urea
1-(3,5-Dichloro-4-hydroxy-phenyl)-3-(3,5-dichloro-phenyl)-urea
1-(3,5-Dichloro-4-hydroxy-phenyl)-3-(4-chloro-phenyl)-urea
1-(3,5-Dichloro-4-hydroxy-phenyl)-3-(4-trifluoromethyl-phenyl)-urea
1-(3,5-Dichloro-4-hydroxy-phenyl)-3-cyclohexyl-urea
1-(3,5-Dichloro-4-hydroxy-phenyl)-3-(4-trifluoromethoxy-phenyl)-urea
1-(3,5-Dichloro-4-hydroxy-phenyl)-3-(4-cyano-phenyl)-urea
1-Benzo[1,3]dioxol-5-yl-3-(3,5-dichloro-4-hydroxy-phenyl)-urea
1-(3,5-Dichloro-4-hydroxy-phenyl)-3-o-tolyl-urea
1-(3,5-Dichloro-4-hydroxy-phenyl)-3-(3-methoxy-phenyl)-urea
1-(3,5-Dichloro-4-hydroxy-phenyl)-3-(2,6-dimethyl-phenyl)-urea
1-(3,5-Dichloro-4-hydroxy-phenyl)-3-(3,4,5-trimethoxy-phenyl)-urea
1-(3,5-Dichloro-4-hydroxy-phenyl)-3-naphthalen-1-yl-urea
1-Adamantan-1-yl-3-(3,5-dichloro-4-hydroxy-phenyl)-urea
1-(3,5-Dichloro-4-hydroxy-phenyl)-3-(4-phenoxy-phenyl)-urea
1-(3,5-Dichloro-4-hydroxy-phenyl)-3-phenyl-urea
3-[3-(4-Hydroxy-3-nitro-phenyl)-ureido]-propionic acid ethyl ester
1-(4-Hydroxy-3-nitro-phenyl)-3-pentyl-urea
1-Benzyl-3-(4-hydroxy-3-nitro-phenyl)-urea
1-(4-Hydroxy-3-nitro-phenyl)-3-(2-methyl-benzyl)-urea
1-(4-Hydroxy-3-nitro-phenyl)-3-phenethyl-urea
1-(4-Hydroxy-3-nitro-phenyl)-3-(1,1,3,3-tetramethyl-butyl)-urea

1-tert-Butyl-3-(4-hydroxy-3-nitro-phenyl)-urea
1-Cyclohexylmethyl-3-(4-hydroxy-3-nitro-phenyl)-urea
1-(4-Hydroxy-3-nitro-phenyl)-3-(4-trifluoromethyl-benzyl)-urea
1-(3,5-Dichloro-phenyl)-3-(4-hydroxy-3-nitro-phenyl)-urea
1-(4-Chloro-phenyl)-3-(4-hydroxy-3-nitro-phenyl)-urea
1-(4-Hydroxy-3-nitro-phenyl)-3-(4-trifluoromethyl-phenyl)-urea
1-Cyclohexyl-3-(4-hydroxy-3-nitro-phenyl)-urea
1-(4-Hydroxy-3-nitro-phenyl)-3-(4-trifluoromethoxy-phenyl)-urea
1-(4-Cyano-phenyl)-3-(4-hydroxy-3-nitro-phenyl)-urea
1-Benzo[1,3]dioxol-5-yl-3-(4-hydroxy-3-nitro-phenyl)-urea
1-(4-Hydroxy-3-nitro-phenyl)-3-o-tolyl-urea
1-(4-Hydroxy-3-nitro-phenyl)-3-(3-methoxy-phenyl)-urea
1-(2,6-Dimethyl-phenyl)-3-(4-hydroxy-3-nitro-phenyl)-urea
1-(4-Hydroxy-3-nitro-phenyl)-3-(3,4,5-trimethoxy-phenyl)-urea
1-(4-Hydroxy-3-nitro-phenyl)-3-naphthalen-1-yl-urea
1-Adamantan-1-yl-3-(4-hydroxy-3-nitro-phenyl)-urea
1-(4-Hydroxy-3-nitro-phenyl)-3-(4-phenoxy-phenyl)-urea
1-(4-Hydroxy-3-nitro-phenyl)-3-phenyl-urea
3-[3-(3-Fluoro-4-hydroxy-phenyl)-ureido]-propionic acid ethyl ester
1-(3-Fluoro-4-hydroxy-phenyl)-3-pentyl-urea
1-Benzyl-3-(3-fluoro-4-hydroxy-phenyl)-urea
1-(3-Fluoro-4-hydroxy-phenyl)-3-(2-methyl-benzyl)-urea
1-(3-Fluoro-4-hydroxy-phenyl)-3-phenethyl-urea
1-(3-Fluoro-4-hydroxy-phenyl)-3-(1,1,3,3-tetramethyl-butyl)-urea
1-tert-Butyl-3-(3-fluoro-4-hydroxy-phenyl)-urea
1-(3-Fluoro-4-hydroxy-phenyl)-3-cyclohexylmethyl-urea

1-(3-Fluoro-4-hydroxy-phenyl)-3-(4-trifluoromethyl-benzyl)-urea
1-(3-Fluoro-4-hydroxy-phenyl)-3-(3,5-dichloro-phenyl)-urea
1-(3-Fluoro-4-hydroxy-phenyl)-3-(4-chloro-phenyl)-urea
1-(3-Fluoro-2-hydroxy-phenyl)-3-(4-trifluoromethyl-phenyl)-urea
1-(3-Fluoro-4-hydroxy-phenyl)-3-cyclohexyl-urea
1-(3-Fluoro-4-hydroxy-phenyl)-3-(4-trifluoromethoxy-phenyl)-urea
1-(3-Fluoro-4-hydroxy-phenyl)-3-(4-cyano-phenyl)-urea
1-Benzo[1,3]dioxol-5-yl-3-(3-fluoro-4-hydroxy-phenyl)-urea
1-(3-Fluoro-4-hydroxy-phenyl)-3-o-tolyl-urea
1-(3-Fluoro-4-hydroxy-phenyl)-3-(3-methoxy-phenyl)-urea
1-(3-Fluoro-4-hydroxy-phenyl)-3-(2,6-dimethyl-phenyl)-urea
1-(3-Fluoro-4-hydroxy-phenyl)-3-(3,4,5-trimethoxy-phenyl)-urea
1-(3-Fluoro-4-hydroxy-phenyl)-3-naphthalen-1-yl-urea
1-Adamantan-1-yl-3-(3-fluoro-4-hydroxy-phenyl)-urea
1-(3-Fluoro-4-hydroxy-phenyl)-3-(4-phenoxy-phenyl)-urea
1-(3-Fluoro-4-hydroxy-phenyl)-3-phenyl-urea
3-[3-(2,4-Dihydroxy-phenyl)-ureido]-propionic acid ethyl ester
1-(2,4-Dihydroxy-phenyl)-3-pentyl-urea
1-Benzyl-3-(2,4-dihydroxy-phenyl)-urea
1-(2,4-Dihydroxy-phenyl)-3-(2-methyl-benzyl)-urea
1-(2,4-Dihydroxy-phenyl)-3-phenethyl-urea
1-(2,4-Dihydroxy-phenyl)-3-(1,1,3,3-tetramethyl-butyl)-urea
1tert-Butyl-3-(2,4-dihydroxy-phenyl)-urea
1-Cyclohexylmethyl-3-(2,4-dihydroxy-phenyl)-urea
1-(2,4-Dihydroxy-phenyl)-3-(4-trifluoromethyl-benzyl)-urea
1-(3,5-Dichloro-phenyl)-3-(2,4-dihydroxy-phenyl)-urea
1-(4-Chloro-phenyl)-3-(2,4-dihydroxy-phenyl)-urea
1-(2,4-Dihydroxy-phenyl)-3-(4-trifluoromethyl-phenyl)-urea
1-Cyclohexyl-3-(2,4-dihydroxy-phenyl)-urea
1-(2,4-Dihydroxy-phenyl)-3-(4-trifluoromethoxy-phenyl)-urea
1-(4-Cyano-phenyl)-3-(2,4-dihydroxy-phenyl)-urea

1-Benzo[1,3]dioxol-5-yl-3-(2,4-dihydroxy-phenyl)-urea
1-(2,4-Dihydroxy-phenyl)-3-o-tolyl-urea
1-(2,4-Dihydroxy-phenyl)-3-(3-methoxy-phenyl)-urea
1-(2,4-Dihydroxy-phenyl)-3-(2,6-dimethyl-phenyl)-urea
1-(2,4-Dihydroxy-phenyl)-3-(3,4,5-trimethoxy-phenyl)-urea
1-(2,4-Dihydroxy-phenyl)-3-naphthalen-1-yl-urea
1-Adamantan-1-yl-3-(2,4-dihydroxy-phenyl)-urea
1-(2,4-Dihydroxy-phenyl)-3-(4-phenoxy-phenyl)-urea
1-(2,4-Dihydroxy-phenyl)-3-phenyl-urea
3-[3-(3,5-Dibromo-4-hydroxy-phenyl)-ureido]-propionic acid ethyl ester
1-(3,5-Dibromo-4-hydroxy-phenyl)-3-pentyl-urea
1-Benzyl-3-(3,5-Dibromo-4-hydroxy-phenyl)-urea
1-(5-Bromo-3-fluoro-2-hydroxy-phenyl)-3-(2-methyl-benzyl)-urea
1-(3,5-Dibromo-4-hydroxy-phenyl)-3-phenethyl-urea
1-(3,5-Dibromo-4-hydroxy-phenyl)-3-(1,1,3,3-tetramethyl-butyl)-urea
1-tert-Butyl-3-(3,5-dibromo-4-hydroxy-phenyl)-urea
1-(3,5-Dibromo-4-hydroxy-phenyl)-3-cyclohexylmethyl-urea
1-(3,5-Dibromo-4-hydroxy-phenyl)-3-(4-trifluoromethyl-benzyl)-urea
1-(3,5-Dibromo-4-hydroxy-phenyl)-3-(3,5-dichloro-phenyl)-urea
1-(3,5-Dibromo-4-hydroxy-phenyl)-3-(4-chloro-phenyl)-urea
1-(3,5-Dibromo-4-hydroxy-phenyl)-3-(4-trifluoromethyl-phenyl)-urea
1-(3,5-Dibromo-4-hydroxy-phenyl)-3-cyclohexyl-urea
1-(3,5-Dibromo-4-hydroxy-phenyl)-3-(4-trifluoromethoxy-phenyl)-urea
1-(3,5-Dibromo-4-hydroxy-phenyl)-3-(4-cyano-phenyl)-urea
1-Benzo[1,3]dioxol-5-yl-3-(3,5-dibromo-4-hydroxy-phenyl)-urea
1-(3,5-Dibromo-4-hydroxy-phenyl)-3-o-tolyl-urea
1-(3,5-Dibromo-4-hydroxy-phenyl)-3-(3-methoxy-phenyl)-urea
1-(3,5-Dibromo-4-hydroxy-phenyl)-3-(2,6-dimethyl-phenyl)-urea
1-(3,5-Dibromo-4-hydroxy-phenyl)-3-(3,4,5-trimethoxy-phenyl)-urea
1-(3,5-Dibromo-4-hydroxy-phenyl)-3-naphthalen-1-yl-urea
1-Adamantan-1-yl-3-(3,5-dibromo-4-hydroxy-phenyl)-urea
1-(3,5-Dibromo-4-hydroxy-phenyl)-3-(4-phenoxy-phenyl)-urea
1-(3,5-Dibromo-4-hydroxy-phenyl)-3-phenyl-urea
3-[3-(3,5-Difluoro-4-hydroxy-phenyl)-ureido]-propionic acid ethyl ester
1-(3,5-Difluoro-4-hydroxy-phenyl)-3-pentyl-urea
1-Benzyl-3-(3,5-difluoro-4-hydroxy-phenyl)-urea
1-(3,5-Difluoro-4-hydroxy-phenyl)-3-(2-methyl-benzyl)-urea
1-(3,5-Difluoro-4-hydroxy-phenyl)-3-phenethyl-urea
1-(3,5-Difluoro-4-hydroxy-phenyl)-3-(1,1,3,3-tetramethyl-butyl)-urea
1-tert-Butyl-3-(3,5-difluoro-4-hydroxy-phenyl)-urea
1-(3,5-Difluoro-4-hydroxy-phenyl)-3-cyclohexylmethyl-urea
1-(3,5-Difluoro-4-hydroxy-phenyl)-3-(4-trifluoromethyl-benzyl)-urea
1-(3,5-Difluoro-4-hydroxy-phenyl)-3-(3,5-dichloro-phenyl)-urea
1-(3,5-Difluoro-4-hydroxy-phenyl)-3-(4-chloro-phenyl)-urea
1-(3,5-Difluoro-4-hydroxy-phenyl)-3-(4-trifluoromethyl-phenyl)-urea
1-(3,5-Difluoro-4-hydroxy-phenyl)-3-cyclohexyl-urea
1-(3,5-Difluoro-4-hydroxy-phenyl)-3-(4-trifluoromethoxy-phenyl)-urea
1-(3,5-Difluoro-4-hydroxy-phenyl)-3-(4-cyano-phenyl)-urea
1-Benzo[1,3]dioxol-5-yl-3-(3,5-difluoro-4-hydroxy-phenyl)-urea
1-(3,5-Difluoro-4-hydroxy-phenyl)-3-o-tolyl-urea
1-(3,5-Difluoro-4-hydroxy-phenyl)-3-(3-methoxy-phenyl)-urea
1-(3,5-Difluoro-4-hydroxy-phenyl)-3-(2,6-dimethyl-phenyl)-urea
1-(3,5-Difluoro-4-hydroxy-phenyl)-3-(3,4,5-trimethoxy-phenyl)-urea
1-(3,5-Difluoro-4-hydroxy-phenyl)-3-naphthalen-1-yl-urea
1-Adamantan-1-yl-3-(3,5-difluoro-4-hydroxy-phenyl)-urea
1-(3,5-Difluoro-4-hydroxy-phenyl)-3-(4-phenoxy-phenyl)-urea
1-(3,5-Difluoro-4-hydroxy-phenyl)-3-phenyl-urea
3-{3-[3-Chloro-4-hydroxy-5-(1-hydroxy-ethyl)-phenyl]-ureido}-propionic acid ethyl ester

1-[3-Chloro-4-hydroxy-5-(1-hydroxy-ethyl)-phenyl]-3-pentyl-urea
1-[3-Chloro-4-hydroxy-5-(1-hydroxy-ethyl)-phenyl]-3-pentyl-urea
1-[3-Chloro-4-hydroxy-5-(1-hydroxy-ethyl)-phenyl]-3-(2-methyl-benzyl)-urea
1-[3-Chloro-4-hydroxy-5-(1-hydroxy-ethyl)-phenyl]-3-phenethyl-urea
1-[3-Chloro-4-hydroxy-5-(1-hydroxy-ethyl)-phenyl]-3-(1,1,3,3-tetramethyl-butyl)-urea
1-tert-Butyl-3-[3-chloro-4-hydroxy-5-(1-hydroxy-ethyl)-phenyl]-urea
1-[3-Chloro-4-hydroxy-5-(1-hydroxy-ethyl)-phenyl]-3-cyclohexylmethyl-ure
1-[3-Chloro-4-hydroxy-5-(1-hydroxy-ethyl)-phenyl]-3-(4-trifluoromethyl-benzyl)-urea
1-[3-Chloro-4-hydroxy-5-(1-hydroxy-ethyl)-phenyl]-3-(4-chloro-phenyl)-urea
1-[3-Chloro-4-hydroxy-5-(1-hydroxy-ethyl)-phenyl]-3-(4-chloro-phenyl)-urea
1-[3-Chloro-4-hydroxy-5-(1-hydroxy-ethyl)-phenyl]-3-(4-trifluoromethyl-phenyl)-urea
1-[3-Chloro-4-hydroxy-5-(1-hydroxy-ethyl)-phenyl]-3-cyclohexyl-urea
1-[3-Chloro-4-hydroxy-5-(1-hydroxy-ethyl)-phenyl]-3-(4-trifluoromethoxy-phenyl)-urea
1-[3-Chloro-4-hydroxy-5-(1-hydroxy-ethyl)-phenyl]-3-(4-cyano-phenyl)-urea
1-Benzo[1,3]dioxol-5-yl-3-[3-chloro-4-hydroxy-5-(1-hydroxy-ethyl)-phenyl]-urea
1-[3-Chloro-4-hydroxy-5-(1-hydroxy-ethyl)-phenyl]-3-o-tolyl-urea
1-[3-Chloro-4-hydroxy-5-(1-hydroxy-ethyl)-phenyl]-3-(3-methoxy-phenyl)-urea
1-[3-Chloro-4-hydroxy-5-(1-hydroxy-ethyl)-phenyl]-3-(2,6-dimethyl-phenyl)-urea
1-[3-Chloro-4-hydroxy-5-(1-hydroxy-ethyl)-phenyl]-3-(3,4,5-trimethoxy-phenyl)-urea
1-[3-Chloro-4-hydroxy-5-(1-hydroxy-ethyl)-phenyl]-3-naphthalen-1-yl-urea
1-Adamantan-1-yl-3-[3-chloro-4-hydroxy-5-(1-hydroxy-ethyl)-phenyl]-urea
1-[3-Chloro-4-hydroxy-5-(1-hydroxy-ethyl)-phenyl]-3-(4-phenoxy-phenyl)-urea
1-[3-Chloro-4-hydroxy-5-(1-hydroxy-ethyl)-phenyl]-3-phenyl-urea
1-(3-Chloro-4-hydroxy-phenyl)-3-pentyl-thiourea
1-Benzyl-3-(3-chloro-4-hydroxy-phenyl)-thiourea
1-(3-Chloro-4-hydroxy-phenyl)-3-(2-methyl-benzyl)-thiourea
1-(3-Chloro-4-hydroxy-phenyl)-3-phenethyl-thiourea
1-(3-Chloro-4-hydroxy-phenyl)-3-(1,1,3,3-tetramethyl-butyl)-thiourea
1-tert-Butyl-3-(3-chloro-4-hydroxy-phenyl)-thiourea
1-(5-Chloro-2-hydroxy-phenyl)-3-isopropyl-thiourea
1-(3-Chloro-4-hydroxy-phenyl)-3-cyclohexylmethyl-thiourea
1-(3-Chloro-4-hydroxy-phenyl)-3-(4-trifluoromethyl-benzyl)-thiourea
1-(3-Chloro-4-hydroxy-phenyl)-3-(3,5-dichloro-phenyl)-thiourea
1-(3-Chloro-4-hydroxy-phenyl)-3-(4-chloro-phenyl)-thiourea
1-(3-Chloro-4-hydroxy-phenyl)-3-(4-trifluoromethyl-phenyl)-thiourea
1-(3-Chloro-4-hydroxy-phenyl)-3-cyclohexyl-thiourea
1-(3-Chloro-4-hydroxy-phenyl)-3-(2-trifluoromethyl-phenyl)-thiourea
1-(3-Chloro-4-hydroxy-phenyl)-3-phenyl-thiourea
1-(3,5-Dichloro-4-hydroxy-phenyl)-3-pentyl-thiourea
1-Benzyl-3-(3,5-dichloro-4-hydroxy-phenyl)-thiourea
1-(3,5-Dichloro-4-hydroxy-phenyl)-3-(2-methyl-benzyl)-thiourea
1-(3,5-Dichloro-4-hydroxy-phenyl)-3-phenethyl-thiourea
1-(3,5-Dichloro-4-hydroxy-phenyl)-3-(1,1,3,3-tetramethyl-butyl)-thiourea
1-tert-Butyl-3-(3,5-dichloro-4-hydroxy-phenyl)-thiourea
1-(5-Chloro-4-hydroxy-phenyl)-3-isopropyl-thiourea
1-(3,5-Dichloro-4-hydroxy-phenyl)-3-cyclohexylmethyl-thiourea
1-(3,5-Dichloro-4-hydroxy-phenyl)-3-(4-trifluoromethyl-benzyl)-thiourea
1-(3,5-Dichloro-4-hydroxy-phenyl)-3-(3,5-dichloro-phenyl)-thiourea
1-(3,5-Dichloro-4-hydroxy-phenyl)-3-(4-chloro-phenyl)-thiourea
1-(3,5-Dichloro-4-hydroxy-phenyl)-3-(4-trifluoromethyl-phenyl)-thiourea
1-(3,5-Dichloro-4-hydroxy-phenyl)-3-cyclohexyl-thiourea
1-(3,5-Dichloro-4-hydroxy-phenyl)-3-(2-trifluoromethyl-phenyl)-thiourea
1-(3,5-Dichloro-4-hydroxy-phenyl)-3-phenyl-thiourea
1-(4-Hydroxy-3-nitro-phenyl)-3-pentyl-thiourea

1-Benzyl-3-(4-hydroxy-3-nitro-phenyl)-thiourea
1-(4-Hydroxy-3-nitro-phenyl)-3-(2-methyl-benzyl)-thiourea
1-(4-Hydroxy-3-nitro-phenyl)-3-phenethyl-thiourea

1-(4-Hydroxy-3-nitro-phenyl)-3-(1,1,3,3-tetramethyl-butyl)-thiourea
1-tert-Butyl-3-(4-hydroxy-3-nitro-phenyl)-thiourea
1-(4-Hydroxy-3-nitro-phenyl)-3-isopropyl-thiourea
1-Cyclohexylmethyl-3-(4-hydroxy-3-nitro-phenyl)-thiourea
1-(4-Hydroxy-3-nitro-phenyl)-3-(4-trifluoromethyl-benzyl)-thiourea
1-(3,5-Dichloro-phenyl)-3-(4-hydroxy-3-nitro-phenyl)-thiourea
1-(4-Chloro-phenyl)-3-(4-hydroxy-3-nitro-phenyl)-thiourea
1-(4-Hydroxy-3-nitro-phenyl)-3-(4-trifluoromethyl-phenyl)-thiourea
1-Cyclohexyl-3-(4-hydroxy-3-nitro-phenyl)-thiourea
1-(4-Hydroxy-3-nitro-phenyl)-3-(2-trifluoromethyl-phenyl)-thiourea
1-(4-Hydroxy-3-nitro-phenyl)-3-phenyl-thiourea
1-(3-Fluoro-4-hydroxy-phenyl)-3-pentyl-thiourea
1-Benzyl-3-(3-fluoro-4-hydroxy-phenyl)-thiowea
1-(3-Fluoro-4-hydroxy-phenyl)-3-(2-methyl-benzyl)-thiourea
1-(3-Fluoro-4-hydroxy-phenyl)-3-phenethyl-thiourea
1-(3-Fluoro-4-hydroxy-phenyl)-3-(1,1,3,3-tetramethyl-butyl)-thiourea
1-tert-Butyl-3-(3-fluoro-4-hydroxy-phenyl)-thiourea
1-(3-Fluoro-4-hydroxy-phenyl)-3-isopropyl-thiourea
1-(3-Fluoro-4-hydroxy-phenyl)-3-cyclohexylmethyl-thiourea
1-(3-Fluoro-4-hydroxy-phenyl)-3-(4-trifluoromethyl-benzyl)-thiourea
1-(3-Fluoro-4-hydroxy-phenyl)-3-(3,5-dichloro-phenyl)-thiourea
1-(3-Fluoro-4-hydroxy-phenyl)-3-(4-chloro-phenyl)-thiowea
1-(3-Fluoro-4-hydroxy-phenyl)-3-(4-trifluoromethyl-phenyl)-thiourea
1-(3-Fluoro-4-hydroxy-phenyl)-3-cyclohexyl-thiourea
1-(3-Fluoro-4-hydroxy-phenyl)-3-(2-trifluoromethyl-phenyl)-thiourea
1-(3-Fluoro-4-hydroxy-phenyl)-3-phenyl-thiourea
1-(2,4-Dihydroxy-phenyl)-3-pentyl-thiourea
1-Benzyl-3-(2,4-dihydroxy-phenyl)-thiourea
1-(2,4-Dihydroxy-phenyl)-3-(2-methyl-benzyl)-thiourea
1-(2,4-Dihydroxy-phenyl)-3-phenethyl-thiourea
1-(2,4-Dihydroxy-phenyl)-3-(1,1,3,3-tetramethyl-butyl)-thiourea
1-tert-Butyl-3-(2,4-dihydroxy-phenyl)-thiourea
1-(2,4-Dihydroxy-phenyl)-3-isopropyl-thiourea
1-Cyclohexylmethyl-3-(2,4-dihydroxy-phenyl)-thiourea
1-(2,4-Dihydroxy-phenyl)-3-(4-trifluoromethyl-benzyl)-thiourea
1-(3,5-Dichloro-phenyl)-3-(2,4-dihydroxy-phenyl)-thiourea
1-(4-Chloro-phenyl)-3-(2,4-dihydroxy-phenyl)-thiourea
1-(2,4-Dihydroxy-phenyl)-3-(4-trifluoromethyl-phenyl)-thiourea
1-Cyclohexyl-3-(2,4-dihydroxy-phenyl)-thiourea
1-(2,4-Dihydroxy-phenyl)-3-(2-trifluoromethyl-phenyl)-thiourea
1-(2,4-Dihydroxy-phenyl)-3-phenyl-thiourea
1-(3,5-Dibromo-4-hydroxy-phenyl)-3-pentyl-thiourea
1-Benzyl-3-(3,5-dibromo-4-hydroxy-phenyl)-thiourea
1-(3,5-Dibromo-4-hydroxy-phenyl)-3-(2-methyl-benzyl)-thiourea
1-(3,5-Dibromo-4-hydroxy-phenyl)-3-phenethyl-thiourea
1-(3,5-Dibromo-4-hydroxy-phenyl)-3-(1,1,3,3-tetramethyl-butyl)-thiourea
1-tert-Butyl-3-(3,5-dibromo-4-hydroxy-phenyl)-thiourea
1-(3,5-Dibromo-4-hydroxy-phenyl)-3-isopropyl-thiourea
1-(3,5-Dibromo-4-hydroxy-phenyl)-3-cyclohexylmethyl-thiourea
1-(3,5-Dibromo-4-hydroxy-phenyl)-3-(4-trifluoromethyl-benzyl)-thiourea
1-(3,5-Dibromo-4-hydroxy-phenyl)-3-(3,5-dichloro-phenyl)-thiourea
1-(3,5-Dibromo-4-hydroxy-phenyl)-3-(4-chloro-phenyl)-thiourea
1-(3,5-Dibromo-4-hydroxy-phenyl)-3-(4-trifluoromethyl-phenyl)-thiourea
1-(3,5-Dibromo-4-hydroxy-phenyl)-3-cyclohexyl-thiourea
1-(3,5-Dibromo-4-hydroxy-phenyl)-3-(2-trifluoromethyl-phenyl)-thiourea
1-(3,5-Dibromo-4-hydroxy-phenyl)-3-phenyl-thiourea
1-(3,5-Difluoro-4-hydroxy-phenyl)-3-pentyl-thiourea
1-Benzyl-3-(3,5-difluoro-4-hydroxy-phenyl)-thiourea

1-(3,5-Difluoro-4-hydroxy-phenyl)-3-(2-methyl-benzyl)-thiourea
1-(3,5-Difluoro-4-hydroxy-phenyl)-3-phenethyl-thiourea
1-(3,5-Difluoro-4-hydroxy-phenyl)-3-(1,1,3,3-tetramethyl-butyl)-thiourea
1-tert-Butyl-3-(3,5-Difluoro-4-hydroxy-phenyl)-thiourea
1-(3,5-Difluoro-4-hydroxy-phenyl)-3-isopropyl-thiourea
1-(3,5-Difluoro-4-hydroxy-phenyl)-3-cyclohexylmethyl-thiourea
1-(3,5-Difluoro-4-hydroxy-phenyl)-3-(4-trifluoromethyl-benzyl)-thiourea
1-(3,5-Difluoro-4-hydroxy-phenyl)-3-(3,5-dichloro-phenyl)-thiourea
1-(3,5-Difluoro-4-hydroxy-phenyl)-3-(4-chloro-phenyl)-thiourea
1-(3,5-Difluoro-4-hydroxy-phenyl)-3-(4-trifluoromethyl-phenyl)-thiourea
1-(3,5-Difluoro-4-hydroxy-phenyl)-3-cyclohexyl-thiourea
1-(3,5-Difluoro-4-hydroxy-phenyl)-3-(2-trifluoromethyl-phenyl)-thiourea
1-(3,5-Difluoro-4-hydroxy-phenyl)-3-phenyl-thiourea
1-[3-Chloro-4-hydroxy-5-(1-hydroxy-ethyl)-phenyl]-3-pentyl-thiourea
1-Benzyl-3-[3-chloro-4-hydroxy-5-(1-hydroxy-ethyl)-phenyl]-thiourea
1-[3-Chloro-4-hydroxy-5-(1-hydroxy-ethyl)-phenyl]-3-(2-methyl-benzyl)-thiourea
1-[3-Chloro-4-hydroxy-5-(1-hydroxy-ethyl)-phenyl]-3-phenethyl-thiourea
1-[3-Chloro-4-hydroxy-5-(1-hydroxy-ethyl)-phenyl]-3-(1,1,3,3-tetramethyl-butyl)-thiourea
1-tert-Butyl-3-[3-chloro-4-hydroxy-5-(1-hydroxy-ethyl)-phenyl]-thiourea
1-[3-Chloro-4-hydroxy-5-(1-hydroxy-ethyl)-phenyl]-3-isopropyl-thiourea
1-[3-Chloro-4-hydroxy-5-(1-hydroxy-ethyl)-phenyl]-3-cyclohexylmethyl-thiourea
1-[3-Chloro-4-hydroxy-5-(1-hydroxy-ethyl)-phenyl]-3-(4-triftuoromethyl-benzyl)-thiourea
1-[3-Chloro-4-hydroxy-5-(1-hydroxy-ethyl)-phenyl]-3-(3,5-dichloro-phenyl)-thiourea
1-[3-Chloro-4-hydroxy-5-(1-hydroxy-ethyl)-phenyl]-3-(4-chloro-phenyl)-thiourea
1-[3-Chloro-4-hydroxy-5-(1-hydroxy-ethyl)-phenyl]-3-(4-trifluoromethyl-phenyl)-thiourea
1-[3-Chloro-4-hydroxy-5-(1-hydroxy-ethyl)-phenyl]-3-cyclohexyl-thiourea
1-[3-Chloro-4-hydroxy-5-(1-hydroxy-ethyl)-phenyl]-3-(2-trifluoromethyl-phenyl)-thiourea
1-[3-Chloro-4-hydroxy-5-(1-hydroxy-ethyl)-phenyl]-3-phenyl-thiourea
2-Chloro-4-(2-phenylsulfanyl-benzylamino)-phenol
2-Chloro-4-(2-p-tolylsulfanyl-benzylamino)-phenol
2-Chloro-4-[2-(4-chloro-phenylsulfanyl)-benzylamino]-phenol
2-Chloro-4-[2-(4-nitro-phenylsulfanyl)-benzylamino]-phenol
2-Chloro-4-[2-(4-methoxy-phenylsulfanyl)-benzylamino]-phenol
2-Chloro-4-[2-(2-chloro-phenylsulfanyl)-benzylamino]-phenol
2-Chloro-4-[2-(3-chloro-phenylsulfanyl)-benzylamino]-phenol
2-Chloro-4-[2-(3,4-dichloro-phenylsulfanyl)-benzylamino]-phenol
N-(4-{2-[(3-Chloro-4-hydroxy-phenylamino)-methyl]-phenylsulfanyl}-phenyl)-acetamide
2-Chloro-4-[2-(quinolin-7-ylsulfanyl)-benzylamino]-phenol
2-Chloro-4-[2-(4-chloro-phenylsulfanyl)-5-nitro-benzylamino]-phenol
2-Chloro-4-(5-nitro-2-p-tolylsulfanyl-benzylamino)-phenol
4-[5-Amino-2-(4-chloro-phenylsulfanyl)-benzylamino]-2-chloro-phenol
2-Chloro-4-[2-(4-chloro-benzenesulfonyl)-benzylamino]-phenol
2,6-Dichloro-4-(2-phenylsulfanyl-benzylamino)-phenol
2,6-Dichloro-4-(2-p-tolylsulfanyl-benzylamino)-phenol
2,6-Dichloro-4-[2-(4-chloro-phenylsulfanyl)-benzylamino]-phenol
2,6-Dichloro-4-[2-(4-nitro-phenylsulfanyl)-benzylamino]-phenol
2,6-Dichloro-4-[2-(4-methoxy-phenylsulfanyl)-benzylamino]-phenol
2,6-Dichloro-4-[2-(2-chloro-phenylsulfanyl)-benzylamino]-phenol
2,6-Dichloro-4-[2-(3-chloro-phenylsulfanyl)-benzylamino]-phenol
2,6-Dichloro-4-[2-(3,4-dichloro-phenylsulfanyl)-benzylamino]-phenol
N-(4-{2-[(3,5-Dichloro-4-hydroxy-phenylamino)-methyl]-phenylsulfanyl}-phenyl)-acetamide
2,6-Dichloro-4-[2-(quinolin-7-ylsulfanyl)-benzylamino]-phenol
2,6-Dichloro-4-[2-(4-chloro-phenylsulfanyl)-5-nitro-benzylamino]-phenol
2,6-Dichloro-4-(5-nitro-2-p-tolylsulfanyl-benzylamino)-phenol
4-[5-Amino-2-(4-chloro-phenylsulfanyl)-benzylamino]-2,6-dichloro-phenol
2,6-Dichloro-4-[2-(4-chloro-benzenesulfonyl)-benzylamino]-phenol
2-Nitro-4-(2-phenylsulfanyl-benzylamino)-phenol
2-Nitro-4-(2-p-tolylsulfanyl-benzylamino)-phenol

4-[2-(4-Chloro-phenylsulfanyl)-benzylamino]-2-nitro-phenol
2-Nitro-4-[2-(4-nitro-phenylsulfanyl)-benzylamino]-phenol
4-[2-(4-Methoxy-phenylsulfanyl)-benzylamino]-2-nitro-phenol
4-[2-(2-Chloro-phenylsulfanyl)-benzylamino]-2-nitro-phenol
4-[2-(3-Chloro-phenylsulfanyl)-benzylamino]-2-nitro-phenol
4-[2-(3,4-Dichloro-phenylsulfanyl)-benzylamino]-2-nitro-phenol
N-(4-{2-[(4-Hydroxy-3-nitro-phenylamino)-methyl]-phenylsulfanyl}-phenyl)-acetamide
2-Nitro-4-[2-(quinolin-7-ylsulfanyl)-benzylamino]-phenol
4-[2-(4-Chloro-phenylsulfanyl)-5-nitro-benzylamino]-2-nitro-phenol
2-Nitro-4-(5-nitro-2-p-tolylsulfanyl-benzylamino)-phenol
N-{4-(4-Chloro-phenylsulfanyl)-3-[(4-hydroxy-3-nitro-phenylamino)-methyl]-phenyl}acetamide
4-[2-(4-Chloro-benzenesulfonyl)-benzylamino]-2-nitro-phenol
2-Fluoro-4-(2-phenylsulfanyl-benzylamino)-phenol
2-Fluoro-4-(2-p-tolylsulfanyl-benzylamino)-phenol
2-Fluoro-4-[2-(4-chloro-phenylsulfanyl)-benzylamino]-phenol
2-Fluoro-4-[2-(4-nitro-phenylsulfanyl)-benzylamino]-phenol
2-Fluoro-4-[2-(4-methoxy-phenylsulfanyl)-benzylamino]-phenol
2-Fluoro-4-[2-(2-chloro-phenylsulfanyl)-benzylamino]-phenol
2-Fluoro-4-[2-(3-chloro-phenylsulfanyl)-benzylamino]-phenol
2-Fluoro-4-[2-(3,4-dichloro-phenylsulfanyl)-benzylamino]-phenol
N-(4-{2-[(3-Fluoro-4-hydroxy-phenylamino)-methyl]-phenylsulfanyl}-phenyl)-acetamide
2-Fluoro-4-[2-(quinolin-7-ylsulfanyl)-benzylamino]-phenol
2-Fluoro-4-[2-(4-chloro-phenylsulfanyl)-5-nitro-benzylamino]-phenol
2-Fluoro-4-(5-nitro-2-p-tolylsulfanyl-benzylamino)-phenol

4-[5-Amino-2-(4-chloro-phenylsulfanyl)-benzylamino]-2-fluoro-phenol
2-Fluoro-4-[2-(4-chloro-benzenesulfonyl)-benzylamino]-phenol
4-(2-Phenylsulfanyl-benzylamino)-benzene-1,3-diol
4-(2-p-Tolylsulfanyl-benzylamino)-benzene-1,3-diol
4-[2-(4-Chloro-phenylsulfanyl)-benzylamino]-benzene-1,3-diol
4-[2-(4-Nitro-phenylsulfanyl)-benzylamino]-benzene-1,3-diol
4-[2-(4-Methoxy-phenylsulfanyl)-benzylamino]-benzene-1,3-diol
4-[2-(2-Chloro-phenylsulfanyl)-benzylamino]-benzene-1,3-diol
4-[2-(3-Chloro-phenylsulfanyl)-benzylamino]-benzene-1,3-diol
4-[2-(3,4-Dichloro-phenylsulfanyl)-benzylamino]-benzene-1,3-diol
N-(4-{2-[(2,4-Dihydroxy-phenylamino)-methyl]-phenylsulfanyl}-phenyl)-acetamide
4-[2-(Quinolin-7-ylsulfanyl)-benzylamino]-benzene-1,3-diol
4-[2-(4-Chloro-phenylsulfanyl)-5-nitro-benzylamino]-benzene-1,3-diol
4-(5-Nitro-2-p-tolylsulfanyl-benzylamino)-benzene-1,3-diol
4-[5-Amino-2-(4-chloro-phenylsulfanyl)-benzylamino]-benzene-1,3-diol
4-[2-(4-Chloro-benzenesulfonyl)-benzylamino]-benzene-1,3-diol
2,6-Dibromo-4-(2-phenylsulfanyl-benzylamino)-phenol
2,6-Dibromo-4-(2-p-tolylsulfanyl-benzylamino)-phenol
2,6-Dibromo-4-[2-(4-chloro-phenylsulfanyl)-benzylamino]-phenol
2,6-Dibromo-4-[2-(4-nitro-phenylsulfanyl)-benzylamino]-phenol
2,6-Dibromo-4-[2-(4-methoxy-phenylsulfanyl)-benzylamino]-phenol
2,6-Dibromo-4-[2-(2-chloro-phenylsulfanyl)-benzylamino]-phenol
2,6-Dibromo-4-[2-(3-chloro-phenylsulfanyl)-benzylamino]-phenol
2,6-Dibromo-4-[2-(3,4-dichloro-phenylsulfanyl)-benzylamino]-phenol
N-(4-{2-[(3,5-Dibromo-4-hydroxy-phenylamino)-methyl]-phenylsulfanyl}-phenyl)-acetamide
2,6-Dibromo-4-[2-(quinolin-7-ylsulfanyl)-benzylamino]-phenol
2,6-Dibromo-4-[2-(4-chloro-phenylsulfanyl)-5-nitro-benzylamino]-phenol
2,6-Dibromo-4-(5-nitro-2-p-tolylsulfanyl-benzylamino)-phenol
4-[5-Amino-2-(4-chloro-phenylsulfanyl)-benzylamino]-2,6-dibromo-phenol
2,6-Dibromo-4-[2-(4-chloro-benzenesulfonyl)-benzylamino]-phenol
2,6-Difluoro-4-(2-phenylsulfanyl-benzylamino)-phenol
2,6-Difluoro-4-(2-p-tolylsulfanyl-benzylamino)-phenol
4-[2-(4-Chloro-phenylsulfanyl)-benzylamino]-2,6-difluoro-phenol

2,6-Difluoro-4-[2-(4-nitro-phenylsulfanyl)-benzylamino]-phenol
2,6-Difluoro-4-[2-(4-methoxy-phenylsulfanyl)-benzylamino]-phenol
4-[2-(2-Chloro-phenylsulfanyl)-benzylamino]-2,6-difluoro-phenol
4-[2-(3-Chloro-phenylsulfanyl)-benzylamino]-2,6-difluoro-phenol
4-[2-(3,4-Dichloro-phenylsulfanyl)-benzylamino]-2,6-difluoro-phenol
N-(4-{2-[(3,5-Difluoro-4-hydroxy-phenylamino)-methyl]-phenylsulfanyl}-phenyl)-acetamide
2,6-Difluoro-4-[2-(quinolin-7-ylsulfanyl)-benzylamino]-phenol
4-[2-(4-Chloro-phenylsulfanyl)-5-nitro-benzylamino]-2,6-difluoro-phenol
2,6-Difluoro-4-(5-nitro-2-p-tolylsulfanyl-benzylamino)-phenol
4-[5-Amino-2-(4-chloro-phenylsulfanyl)-benzylamino]-2,6-difluoro-phenol
4-[2-(4-Chloro-benzenesulfonyl)-benzylamino]-2,6-difluoro-phenol
2-Chloro-6-(1-hydroxy-ethyl)-4-(2-phenylsulfanyl-benzylamino)-phenol
2-Chloro-6-(1-hydroxy-ethyl)-4-(2-p-tolylsulfanyl-benzylamino)-phenol
2-Chloro-4-[2-(4-chloro-phenylsulfanyl)-benzylamino]-6-(1-hydroxy-ethyl)-phenol
2-Chloro-6-(1-hydroxy-ethyl)-4-[2-(4-nitro-phenylsulfanyl)-benzylamino]-phenol
2-Chloro-6-(1-hydroxy-ethyl)-4-[2-(4-methoxy-phenylsulfanyl)-benzylamino]-phenol
2-Chloro-4-[2-(2-chloro-phenylsulfanyl)-benzylamino]-6-(1-hydroxy-ethyl)-phenol
2-Chloro-4-[2-(3-chloro-phenylsulfanyl)-benzylamino]-6-(1-hydroxy-ethyl)-phenol
2-Chloro-4-[2-(3,4-dichloro-phenylsulfanyl)-benzylamino]-6-(1-hydroxy-ethyl)-phenol
N-[4-(2-{[3-Chloro-4-hydroxy-5-(1-hydroxy-ethyl)-phenylamino]-methyl}-phenylsulfanyl)-phenyl]-acetamide
2-Chloro-6-(1-hydroxy-ethyl)-4-[2-(quinolin-7-ylsulfanyl)-benzylamino]-phenol
2-Chloro-4-[2-(4-chloro-phenylsulfanyl)-5-nitro-benzylamino]-6-(1-hydroxy-ethyl)-phenol
2-Chloro-4-(1-hydroxy-ethyl)-6-(5-nitro-2-p-tolylsulfanyl-benzylamino)-phenol
4-[5-Amino-2-(4-chloro-phenylsulfanyl)-benzylamino]-2-chloro-6-(1-hydroxy-ethyl)-phenol
2-Chloro-4-[2-(4-chloro-benzenesulfonyl)-benzylamino]-6-(1-hydroxy-ethyl)-phenol
2-Hydroxymethyl-4-(2-phenylsulfanyl-benzylamino)-phenol
2-Hydroxymethyl-4-(2-p-tolylsulfanyl-benzylamino)-phenol
4-[2-(4-Chloro-phenylsulfanyl)-benzylamino]-2-hydroxymethyl-phenol
2-Hydroxymethyl-4-[2-(4-nitro-phenylsulfanyl)-benzylalnino]-phenol
2-Hydroxymethyl-4-[2-(4-methoxy-phenylsulfanyl)-benzylamino]-phenol
4-[2-(2-Chloro-phenylsulfanyl)-benzylamino]-2-hydroxymethyl-phenol
4-[2-(3-Chloro-phenylsulfanyl)-benzylamino]-2-hydroxymethyl-phenol
4-[2-(3,4-Dichloro-phenylsulfanyl)-benzylamino]-2-hydroxymethyl-phenol
N-(4-{2-[(4-Hydroxy-3-hydroxymethyl-phenylamino)-methyl]-phenylsulfanyl}-phenyl)-acetamide
2-Hydroxymethyl-4-[2-(quinolin-7-ylsulfanyl)-benzylamino]-phenol
4-[2-(4-Chloro-phenylsulfanyl)-5-nitro-benzylamino]-2-hydroxymethyl-phenol
2-Hydroxymethyl-4-(5-nitro-2-p-tolylsulfanyl-benzylamino)-phenol
4-[5-Amino-2-(4-chloro-phenylsulfanyl)-benzylamino]-2-hydroxymethyl-phenol
4-[2-(4-Chloro-benzenesulfonyl)-benzylamino]-2-hydroxymethyl-phenol
1-[3-Chloro-2-hydroxy-5-(2-phenylsulfanyl-benzylamino)-phenyl]-ethanone
1-[3-Chloro-2-hydroxy-5-(2-p-tolylsulfanyl-benzylamino)-phenyl]-ethanone
1-{3-Chloro-5-[2-(4-chloro-phenylsulfanyl)-benzylamino]-2-hydroxy-phenyl}-ethanone
1-{3-Chloro-2-hydroxy-5-[2-(4-nitro-phenylsulfanyl)-benzylamino]-phenyl}-ethanone
1-{3-Chloro-2-hydroxy-5-[2-(4-methoxy-phenylsulfanyl)-benzylamino]-phenyl}-ethanone
1-{3-Chloro-5-[2-(2-chloro-phenylsulfanyl)-benzylamino]-2-hydroxy-phenyl}-ethanone
1-{3-Chloro-5-[2-(3-chloro-phenylsulfanyl)-benzylamino]-2-hydroxy-phenyl}-ethanone
1-{3-Chloro-5-[2-(3,4-dichloro-phenylsulfanyl)-benzylamino]-2-hydroxy-phenyl}-ethanone
N-(4-{2-[(3-Acetyl-5-chloro-4-hydroxy-phenylamino)-methyl]-phenylsulfanyl}-phenyl)-acetamide
1-{3-Chloro-2-hydroxy-5-[2-(quinolin-7-ylsulfanyl)-benzylamino]-phenyl}-ethanone
1-{3-Chloro-5-[2-(4-chloro-phenylsulfanyl)-5-nitro-benzylamino]-2-hydroxy-phenyl}-ethanone
1-[3-Chloro-2-hydroxy-5-(5-nitro-2-p-tolylsulfanyl-benzylamino)-phenyl]-ethanone
1-{5-[5-Amino-2-(4-chloro-phenylsulfanyl)-benzylamino]-3-chloro-2-hydroxy-phenyl}-ethanone
1-{3-Chloro-5-[2-(4-chloro-benzenesulfonyl)-benzylamino]-2-hydroxy-phenyl}-ethanone
2-Hydroxy-5-(2-phenylsulfanyl-benzylamino)-benzoic acid
2-Hydroxy-5-(2-p-tolylsulfanyl-benzylamino)-benzoic acid
5-[2-(4-Chloro-phenylsulfanyl)-benzylamino]-2-hydroxy-benzoic acid
2-Hydroxy-5-[2-(4-nitro-phenylsulfanyl)-benzylamino]-benzoic acid
2-Hydroxy-5-[2-(4-methoxy-phenylsulfanyl)-benzylamino]-benzoic acid

5-[2-(2-Chloro-phenylsulfanyl)-benzylamino]-2-hydroxy-benzoic acid
5-[2-(3-Chloro-phenylsulfanyl)-benzylamino]-2-hydroxy-benzoic acid
5-[2-(3,4-Dichloro-phenylsulfanyl)-benzylamino]-2-hydroxy-benzoic acid
5-[2-(4-Acetylamino-phenylsulfanyl)-benzylamino]-2-hydroxy-benzoic acid
2-Hydroxy-5-[2-(quinolin-7-ylsulfanyl)-benzylamino]-benzoic acid
5-[2-(4-Chloro-phenylsulfanyl)-5-nitro-benzylamino]-2-hydroxy-benzoic acid
2-Hydroxy-5-(5-nitro-2-p-tolylsulfanyl-benzylamino)-benzoic acid
5-[5-Amino-2-(4-chloro-phenylsulfanyl)-benzylamino]-2-hydroxymethyl-phenol
5-[2-(4-Chloro-benzenesulfonyl)-benzylamino]-2-hydroxy-benzoic acid
2-Fluoro-6-nitro-4-(2-phenylsulfanyl-benzylamino)-phenol
2-Fluoro-6-nitro-4-(2-p-tolylsulfanyl-benzylamino)-phenol
4-[2-(4-Chloro-phenylsulfanyl)-benzylamino]-2-fluoro-6-nitro-phenol
2-Fluoro-6-nitro-4-[2-(4-nitro-phenylsulfanyl)-benzylamino]-phenol
2-Fluoro-4-[2-(4-methoxy-phenylsulfanyl)-benzylamino]-6-nitro-phenol
4-[2-(2-Chloro-phenylsulfanyl)-benzylamino]-2-fluoro-6-nitro-phenol
4-[2-(3-Chloro-phenylsulfanyl)-benzylamino]-2-fluoro-6-nitro-phenol
4-[2-(3,4-Dichloro-phenylsulfanyl)-benzylamino]-2-fluoro-6-nitro-phenol
N-(4-{2-[(3-Fluoro-4-hydroxy-5-nitro-phenylamino)-methyl]-phenylsulfanyl}-phenyl)-acetamide
2-Fluoro-6-nitro-4-[2-(quinolin-7-ylsulfanyl)-benzylamino]-phenol
4-[2-(4-Chloro-phenylsulfanyl)-5-nitro-benzylamino]-2-fluoro-6-nitro-phenol
2-Fluoro-6-nitro-4-(5-nitro-2-p-tolylsulfanyl-benzylamino)-phenol
4-[5-Amino-2-(4-chloro-phenylsulfanyl)-benzylamino]-2-fluoro-6-nitro-phenol
4-[2-(4-Chloro-benzenesulfonyl)-benzylamino]-2-fluoro-6-nitro-phenol
2,6-Dichloro-4-(3-phenoxy-benzylamino)-phenol
2,6-Dichloro-4-[3-(4-chloro-phenoxy)-benzylamino]-phenol
4-[3-(4-tert-Butyl-phenoxy)-benzylamino]-2,6-dichloro-phenol
4-(3-Benzyloxy-benzylamino)-2,6-dichloro-phenol
4-(2-Benzyloxy-benzylamino)-2,6-dichloro-phenol
2,6-Dichloro-4-[(naphthalen-1-ylmethyl)-amino]-phenol
2,6-Dichloro-4-(4-methylsulfanyl-benzylamino)-phenol
2,6-Dichloro-4-(2-ethylsulfanyl-benzylamino)-phenol
2,6-Dichloro-4-(2-morpholin-4-yl-benzylamino)-phenol
2,6-Dichloro-4-{[2-(4-chloro-phenylsulfanyl)-thiophen-3-ylmethyl]-amino}-phenol
2,6-Dichloro-4-[(5-phenyl-2H-imidazol-4-ylmethyl)-amino]-phenol
4-[(5-Bromo-thiophen-2-ylmethyl)-amino]-2,6-dichloro-phenol
2,6-Dichloro-4-[3-(4-methoxy-phenoxy)-benzylamino]-phenol
2,6-Dichloro-4-(3-methyl-benzylamino)-phenol
2,6-Dichloro-4-(3-trifluoromethyl-benzylamino)-phenol
2,6-Dichloro-6-(2-chloro-6-fluoro-benzylamino)-phenol
4-(3-Phenoxy-benzylamino)-benzene-1,3-diol
4-[3-(4-Chloro-phenoxy)-benzylamino]-benzene-1,3-diol
4-[3-(4-tert-Butyl-phenoxy)-benzylamino]-benzene-1,3-diol
4-(3-Benzyloxy-benzylamino)-benzene-1,3-diol
4-(2-Benzyloxy-benzylamino)-benzene-1,3-diol
4-[(Naphthalen-1-ylmethyl)-amino]-benzene-1,3-diol
4-(4-Methylsulfanyl-benzylamino)-benzene-1,3-diol
4-(2-Ethylsulfanyl-benzylamino)-benzene-1,3-diol
4-(2-Morpholin-4-yl-benzylamino)-benzene-1,3-diol
4-{[2-(4-Chloro-phenylsulfanyl)-thiophen-3-ylmethyl]-amino}-benzene-1,3-diol
4-[(5-Phenyl-2H-imidazol-4-ylmethyl)-amino]-benzene-1,3-diol
2-[(5-Bromo-thiophen-2-ylmethyl)-amino]-4,6-dichloro-3-methyl-phenol
4-[3-(4-Methoxy-phenoxy)-benzylamino]-benzene-1,3-diol
4-(3-Methyl-benzylamino)-benzene-1,3-diol
4-(3-Trifluoromethyl-benzylamino)-benzene-1,3-diol
4-(2-Chloro-6-fluoro-benzylamino)-benzene-1,3-diol
2-Chloro-4-(3-phenoxy-benzylamino)-phenol
2-Chloro-4-[3-(4-chloro-phenoxy)-benzylamino]-phenol
4-[3-(4-tert-Butyl-phenoxy)-benzylamino]-2-chloro-phenol

4-(3-Benzyloxy-benzylamino)-2-chloro-phenol
4-(2-Benzyloxy-benzylamino)-2-chloro-phenol
2-Chloro-4-[(naphthalen-1-ylmethyl)-amino]-phenol
2-Chloro-4-(4-methylsulfanyl-benzylamino)-phenol
2-Chloro-4-(2-ethylsulfanyl-benzylamino)-phenol
2-Chloro-4-(2-morpholin-4-yl-benzylamino)-phenol
2-Chloro-4-{[2-(4-chloro-phenylsulfanyl)-thiophen-3-ylmethyl]-amino}-phenol
2-Chloro-4-[(5-phenyl-2H-imidazol-4-ylmethyl)-amino]-phenol
4-[(5-Bromo-thiophen-2-ylmethyl)-amino]-2-chloro-phenol
2-Chloro-4-[3-(4-methoxy-phenoxy)-benzylamino]-phenol
2-Chloro-4-(3-methyl-benzylamino)-phenol
2-Chloro-4-(3-trifluoromethyl-benzylamino)-phenol
2-Chloro-4-(2-chloro-6-fluoro-benzylamino)-phenol
2-Fluoro-4-(3-phenoxy-benzylamino)-phenol
2-Fluoro-4-[3-(4-chloro-phenoxy)-benzylamino]-phenol
4-[3-(4-tert-Butyl-phenoxy)-benzylamino]-2-fluoro-phenol
4-(3-Benzyloxy-benzylamino)-2-fluoro-phenol
4-(2-Benzyloxy-benzylamino)-2-fluoro-phenol
2-Fluoro-4-[(naphthalen- I -ylmethyl)-amino] -phenol
2-Fluoro-4-(4-methylsulfanyl-benzylamino)-phenol
2-Fluoro-4-(2-ethylsulfanyl-benzylamino)-phenol
2-Fluoro-4-(2-morpholin-4-yl-benzylamino)-phenol
2-Fluoro-4-{[2-(4-chloro-phenylsulfanyl)-thiophen-3-ylmethyl]-amino}-phenol
2-Fluoro-4-[(5-phenyl-2H-imidazol-4-ylmethyl)-amino]-phenol
4-[(5-Bromo-thiophen-2-ylmethyl)-amino]-2-fluoro-phenol
2-Fluoro-4-[3-(4-methoxy-phenoxy)-benzylamino]-phenol
2-Fluoro-4-(3-methyl-benzylamino)-phenol
2-Fluoro-4-(3-trifluoromethyl-benzylamino)-phenol

2-Fluoro-4-(2-chloro-6-fluoro-benzylamino)-phenol
2,6-Difluoro-4-(3-phenoxy-benzylamino)-phenol
2,6-Difluoro-4-[3-(4-chloro-phenoxy)-benzylamino]-phenol
4-[3-(4-tert-Butyl-phenoxy)-benzylamino]-2,6-difluoro-phenol
4-(3-Benzyloxy-benzylamino)-2,6-difluoro-phenol
4-(2-Benzyloxy-benzylamino)-2,6-difluoro-phenol
2,6-Difluoro-4-[(naphthalen-1-ylmethyl)-amino]-phenol
2,6-Difluoro-4-(4-methylsulfanyl-benzylamino)-phenol
2,6-Difluoro-4-(2-ethylsulfanyl-benzylamino)-phenol
2,6-Difluoro-4-(2-morpholin-4-yl-benzylamino)-phenol
2,6-Difluoro-4-{[2-(4-chloro-phenylsulfanyl)-thiophen-3-ylmethyl]-amino}-phenol
2,6-Difluoro-4-[(5-phenyl-2H-imidazol-4-ylmethyl)-amino]-phenol
4-[(5-Bromo-thiophen-2-ylmethyl)-amino]-2,6-difluoro-phenol
2,6-Difluoro-4-[3-(4-methoxy-phenoxy)-benzylamino]-phenol
2,6-Difluoro-4-(3-methyl-benzylamino)-phenol
2,6-Difluoro-4-(3-trifluoromethyl-benzylamino)-phenol
2,6-Difluoro-4-(2-chloro-6-fluoro-benzylamino)-phenol
N-(2,4-Dihydroxy-phenyl)-C-phenyl-methanesulfonamide
Butane-1-sulfonic acid (2,4-dihydroxy-phenyl)-amide
Octane-1-sulfonic acid (2,4-dihydroxy-phenyl)-amide
Propane-2-sulfonic acid (2,4-dihydroxy-phenyl)-amide
N-(3,5-Dichloro-4-hydroxy-phenyl)-C-phenyl-methanesulfonamide
Butane-1-sulfonic acid (3,5-dichloro-4-hydroxy-phenyl)-amide
Octane-1-sulfonic acid (3,5-dichloro-4-hydroxy-phenyl)-amide
Propane-2-sulfonic acid (3,5-dichloro-4-hydroxy-phenyl)-amide
N-(3-Chloro-4-hydroxy-phenyl)-C-phenyl-methanesulfonamide
Butane-1-sulfonic acid (3-chloro-4-hydroxy-phenyl)-amide
Octane-1-sulfonic acid (3-chloro-4-hydroxy-phenyl)-amide
Propane-2-sulfonic acid (3-chloro-4-hydroxy-phenyl)-amide

N-(3-Fluoro-4-hydroxy-phenyl)-C-phenyl-methanesulfonamide
Butane-1-sulfonic acid (3-fluoro-4-hydroxy-phenyl)-amide
Octane-1-sulfonic acid (3-fluoro-4-hydroxy-phenyl)-amide
Propane-2-sulfonic acid (3-fluoro-4-hydroxy-phenyl)-amide
N-(3,5-Difluoro-4-hydroxy-phenyl)-C-phenyl-methanesulfonamide
Butane-1-sulfonic acid (3,5-difluoro-4-hydroxy-phenyl)-amide
Octane-1-sulfonic acid (3,5-difluoro-4-hydroxy-phenyl)-amide
Propane-2-sulfonic acid (3,5-difluoro-4-hydroxy-phenyl)-amide
(2,4-Dihydroxy-phenyl)-carbamic acid hexyl ester
(3,5-Dichloro-4-hydroxy-phenyl)-carbamic acid hexyl ester
(3-Chloro-4-hydroxy-phenyl)-carbamic acid hexyl ester
(3-Fluoro-4-hydroxy-phenyl)-carbamic acid hexyl ester
(3,5-Dibromo-4-hydroxy-phenyl)-carbamic acid hexyl ester
(3,5-Difluoro-4-hydroxy-phenyl)-carbamic acid hexyl ester
-[3-(2,4-Dihydroxy-phenyl)-ureido]-4-methyl-pentanoic acid ethyl ester
2-[3-(3,5-Dichloro-4-hydroxy-phenyl)-ureido]-4-methyl-pentanoic acid ethyl ester
2-[3-(3-Chloro-4-hydroxy-phenyl)-ureido]-4-methyl-pentanoic acid ethyl ester
2-[3-(3-Fluoro-4-hydroxy-phenyl)-ureido]-4-methyl-pentanoic acid ethyl ester
2-[3-(3,5-Difluoro-4-hydroxy-4-phenyl)-ureido]-4-methyl-pentanoic acid ethyl ester
2-[3-(3,5-Dibromo-4-hydroxy-4-methyl-phenyl)-ureido]-4-methyl-pentanoic acid ethyl ester 2-[3-(2,4-Dihydroxy-phenyl)-ureido]-3-phenyl-propionic acid ethyl ester
2-[3-(3,5-Dichloro-4-hydroxy-phenyl)-ureido]-3-phenyl-propionic acid ethyl ester
2-[3-(3-Chloro-4-hydroxy-phenyl)-ureido]-3-phenyl-propionic acid ethyl ester
2-[3-(3-Fluoro-4-hydroxy-phenyl)-ureido]-3-phenyl-propionic acid ethyl ester
2-[3-(3,5-Difluoro-4-hydroxy-phenyl)-ureido]-3-phenyl-propionic acid ethyl ester
2-[3-(3,5-Dibromo-3-fluoro-4-hydroxy-phenyl)-ureido]-3-phenyl-propionic acid ethyl ester
3,5,5-Trimethyl-hexanoic acid (2,4-dihydroxy-phenyl)-amide
3,5,5-Trimethyl-hexanoic acid (3,5-dichloro-4-hydroxy-phenyl)-amide
3,5,5-Trimethyl-hexanoic acid (3-chloro-4-hydroxy-phenyl)-amide
3,5,5-Trimethyl-hexanoic acid (3-fluoro-4-hydroxy-phenyl)-amide
3,5,5-Trimethyl-hexanoic acid (3,5-difluoro-4-hydroxy-phenyl)-amide
3,5,5-Trimethyl-hexanoic acid (3,5-dibromo-4-hydroxy-phenyl)-amide
1-(3-Benzothiazol-2-yl-5-chloro-4-hydroxy-phenyl)-3-tert-butyl-urea
1-(3-Benzothiazol-2-yl-5-chloro-4-hydroxy-phenyl)-3-benzyl-urea
1-(3-Benzothiazol-2-yl-5-chloro-4-hydroxy-phenyl)-3-phenethyl-urea
1-(3-Benzothiazol-2-yl-5-chloro-4-hydroxy-phenyl)-3-isopropyl-thiourea
1-(3-Benzothiazol-2-yl-5-chloro-4-hydroxy-phenyl)-3-tert-butyl-thiourea
3,5,5-Trimethyl-hexanoic acid (3-benzothiazol-2-yl-5-chloro-4-hydroxy-phenyl)-amide
N-(3-Benzothiazol-2-yl-5-chloro-4-hydroxy-phenyl)-3-phenyl-propionamide
1-(4-Hydroxy-2-methyl-phenyl)-3-pentyl-urea
Biphenyl-4-carboxylic acid (2,4-dihydroxy-phenyl)-amide
Biphenyl-4-carboxylic acid (3,5-dichloro-4-hydroxy-phenyl)-amide
4-[(Furan-2-ylmethyl)-amino]-benzene-1,3-diol
2,6-Dichloro-4-[(furan-2-ylmethyl)-amino]-phenol
2,6-Difluoro-4-[(furan-2-ylmethyl)-amino]-phenol
4-(2-Trifluoromethyl-benzylamino)-benzene-1,3-diol
2,6-Difluoro-4-(2-trifluoromethyl-benzylamino)-phenol
N-(3,5-Dichloro-4-hydroxy-phenyl)-3-phenyl-propionamide
N-(3,5-Dichloro-4-hydroxy-phenyl)-2-(2-nitro-phenyl)-acetamide
2-Benzo[1,3]dioxol-5-yl-N-(3,5-dichloro-4-hydroxy-phenyl)-acetamide
3-Methyl-but-2-enoic acid (3,5-dichloro-4-hydroxy-phenyl)-amide
Naphthalene-2-carboxylic acid (3,5-dichloro-4-hydroxy-phenyl)-amide
N-(3,5-Dichloro-4-hydroxy-phenyl)-benzamide
Furan-2-carboxylic acid (3,5-dichloro-4-hydroxy-phenyl)-amide
2,6-Dichloro-4-(2-trifluoromethyl-benzylamino)-phenol

Embodiment 148: A pharmaceutical composition comprising a compound according to any of embodiments 1 to 147 and a pharmaceutically acceptable carrier, diluent or excipient.

Embodiment 149: The pharmaceutical composition according to embodiment 148 comprising a further pharmaceutically active compound.

Embodiment 150: The pharmaceutical composition according to embodiment 148 or 149, wherein the compound is present as a pharmaceutically acceptable salt or a pharmaceutically active solvate.

Embodiment 151: The pharmaceutically active composition according to any of embodiments 148 to 150, wherein the pharmaceutically active compound is either alone or in combination with any of the ingredients of the composition present in a multitude of individualized dosages and/or administration forms.

Embodiment 152: Use of a compound according to any of the preceding embodiments for the manufacture of a medicament.

Embodiment 153: Use of a compound for the manufacture of a medicament for the treatment of a disease, whereby the disease involves an abnormal cell proliferation, an undesired cell proliferation, an abnormal mitosis and/or an undesired mitosis, whereby the compound is a compound according to any of the preceding embodiments.

Embodiment 154: The use according to embodiment 153, wherein the compound is acting on an enzymatic activity involved in the regulation of cell division and/or cell cycle or part thereof, preferably the part of the cell cycle is mitosis.

Embodiment 155: The use according to embodiment 153 or 154, wherein the disease is selected from the group comprising neurodegenerative diseases, stroke, inflammatory diseases, immune based disorders, infectious diseases, heart diseases, cardiovascular diseases and cell proliferative diseases.

Embodiment 156: The use according to embodiment 155, wherein the neurodegenerative disease is selected from the group comprising Alzheimer’s disease, Huntington’s disease, Parkinson’s disease, peripheral neuropathy, progressive supranuclear palsy, corticobasal degeneration, frontotemporal dementia, synucleinopathies, multiple system atrophy, amyotrophic lateral atrophy, prion diseases and motor neuron diseases.

Embodiment 157: The use according to embodiment 155, wherein the infectious disease is selected from the group comprising fungal, viral, bacterial and parasite infection.

Embodiment 158: The use according to embodiment 157, wherein the fungal infection is selected from the group comprising gynaecological and dermatological infection.

Embodiment 159: The use according to embodiment 157, wherein the fungal infection is caused by or involves *Histoplasma, Coccidioides, Cryptococcus, Blastomyces, Paracoccidioides, Aspergillus, Sporothrix, Rhizopus, Absidia, Mucor, Hormodendrum, Phialophora* Microsporum, Epidermophyton, *Rhinosporidum or by a yeast, preferably Candida or Cryptococcus.*

Embodiment 160: The use according to embodiment 155 or 157, wherein the infectious disease is selected from or the fungal infection causes a disorder selected from the group comprising ringworm, candidiasis, coccidioidomycosis, blastomycosis, aspergillosis, cryptococcosis, histioplasmosis, paracoccidiomycosis, zygomycosis, sporotrichiosis, mycotic keratitis, nail hair and skin disease, lobomycosis, chromoblastomycosis, mycetoma.

Embodiment 161: The use according to embodiment 157, wherein the bacterial infection is selected from the group comprising infections caused by Gram-positive and by Gram-negative bacteria.

Embodiment 162: The use according to embodiment 161, wherein the bacterial infection is caused by or involves *Staphylococcus, Clostridium, Streptococcus, Listeria, Salmonella, Bacillus, Escherichia, Mycobacteria, Serratia, Enterobacter, Enterococcus,* Nocardia, *Hemophilus, Neisseria, Proteus, Yersinia, Helicobacter or Legionella.*

Embodiment 163: The use according to embodiment 155 or 157, wherein the infectious disease is selected from or the bacterial infection causes a disorder selected from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

Embodiment 164: The use according to embodiment 157, wherein the viral infection is selected from the group

comprising infections caused by or involving retrovirus, HIV, Papilloma virus, Polio virus, Epstein-Barr, Herpes virus, Hepatitis virus, Papova virus, Influenza virus, Rabies, JC, encephalitis causing virus or hemorrhagic fever causing virus.

Embodiment 165: The use according to embodiment 157, wherein the parasite infection is selected from the group comprising infections caused by or involving *Trypanosoma, Leishmania, Trichinella, Echinococcus, Nematodes, Classes Cestoda Trematoda, Monogenea, Toxoplasma, Giardia, Balantidium, Paramecium, Plasmodium, or Entamoeba.*

Embodiment 166: The use according to embodiment 155, wherein the cell proliferative disorder is selected from the group comprising neoplastic and non-neoplastic disorders.

Embodiment 167: The use according to embodiment 166, wherein the neoplastic cell proliferative disorder is selected from the group comprising solid tumor, lymphoma and leukemia.

Embodiment 168: The use according to embodiment 167, wherein the solid tumor is selected from the group comprising carcinoma, sarcoma, osteoma, fibrosarcoma, and chondrosarcoma.

Embodiment 169: The use according to embodiment 166, wherein the neoplastic cell proliferative disorder is selected from the group comprising breast cancer, prostate cancer, colon cancer, brain cancer, lung cancer, pancreatic cancer, gastric cancer, bladder cancer and kidney cancer.

Embodiment 170: The use according to embodiment 166, wherein the non-neoplastic cell proliferative disorder is a fibrotic disorder, preferably the fibrotic disorder is fibrosis.

Embodiment 171: The use according to embodiment 166, wherein the non-neoplastic cell proliferative disorder is selected from the group comprising prostatic hypertrophy, endometriosis, psoriasis, tissue repair and wound healing.

Embodiment 172: The use according to embodiment 155, wherein the immune based/inflammatory disease is an autoimmune disease or disorder.

Embodiment 173: The use according to embodiment 155, wherein the immune based/inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease and dermatomyositis.

Embodiment 174: The use according to embodiment 155, wherein the immune based and/or inflammatory disease is an inflammatory condition.

Embodiment 175: The use according to embodiment 155, wherein the immune based and/or inflammatory disease is selected from the group comprising inflammation associated with bums, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

Embodiment 176: The use according to any of embodiments 153 to 175, wherein the medicament is for administration via an administration route which is selected from the group comprising oral, subcutaneous, intravenous, intranasal, transdermal, intraperitoneal, intramuscular, intrapulmonar, vaginal, rectal, and intraocular administration.

Embodiment 177: The use according to any of embodiments 153 to 176, wherein the medicament is for the administration to a mammal, preferably to a human being.

Embodiment 178: The use according to any of embodiments 152 to 176, wherein the medicament is or comprises a pharmaceutical composition according to any of embodiments 148 to 151.

Embodiment 179: Use of a compound according to any of embodiments 1 to 146 as an inhibitor to a rotamase.

Embodiment 180: Use according to embodiment 179, wherein the rotamase regulates a part of the cell cycle.

Embodiment 181: Use according to embodiment 179 or 180, whererin the rotamase regulates a part of the cell cycle, whereby preferably the part of the cell cycle is mitosis.

Embodiment 182: Use according to any of embodiments 179 to 181, wherein the rotamase is a mammalian rotamase, preferably a human rotamase, more preferably hPin1.

Embodiment 183: Use according to any of embodiments 147 to 182, wherein the disease involves a rotamase, whereby the rotamase is a mammalian rotamase, preferably a human rotamase, more preferably hPin1.

The features of the present invention disclosed in the specification, the claims and/or the drawing may both separately and in any combination thereof be material for realizing the invention in various forms thereof.

## Claims

**1.** A compound of the formula (I):

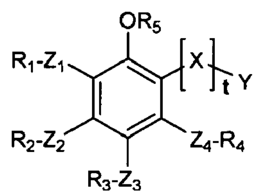

I

wherein $R_1$, $R_2$, $R_3$ and $R_4$ are each independently selected from the group comprising H, $OR_6$, $SR_7$, $NR_8R_9$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl;
wherein $R_1$ and $R_2$, $R_2$ and $R_3$, $R_3$ and $R_4$, $R_1$ and $R_3$, $R_1$ and $R_4$, and $R_2$ and $R_4$ may be linked so as to form a ring comprising 4 to 12 members, preferably 5 to 10 members,
wherein $Z_1$, $Z_2$, $Z_3$ and $Z_4$ are each and independently selected from the group comprising - C(O)-, -C(S)-, -C (O)-$NR_{10}$-, -C(S)-$NR_{11}$-, -C(N-CN)-$NR_{12}$-, -S(O)-, -S($O_2$)-, -S(C)-$NR_{13}$-, and -S($O_2$)-$NR_{14}$-, -O-, -S- or are each and individually absent;
$R_5$ is selected from the group comprising H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, substituted alkylheteroaryl and -C(O)-Q;

wherein Q is selected from the group comprising H, $NHR_{15}$, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl, and substituted alkylheteroaryl; and

$R_6$, $R_7$, $R_8$, $R_9$, $R_{10}$, $R_{11}$, $R_{12}$, $R_{13}$, $R_{14}$ and $R_{15}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;
X is a spacer and is independently selected from the group comprising
-M1-L1-K-L2-M2-,

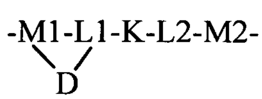 , 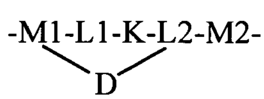 , 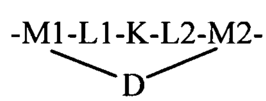 , 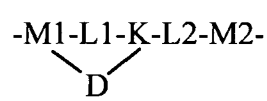 ,

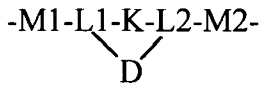 , 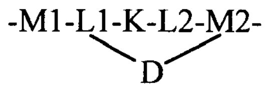 , 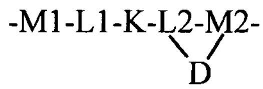 or 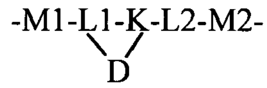

wherein

K is selected from the group comprising

C=T,
O, S, S(O) and $S(O_2)$,
or is absent,
with =T being selected from the group comprising
= O, =S, $=N-R^e$, =N-CN, $=N-NO_2$ and $=CH-NO_2$,

L1 and L2 are each and independently selected from the group comprising O, S and primary amines, more particularly $NR^c$, $NR^d$; or being individually and independent from each other absent
M1 and M2 are each and independently selected from the group comprising
$-(CR^aR^b)n-$,
$-(CR^fR^g)m-$,
cycloalkyl, substituted cycloakyl, heterocyclyl, substituted heterocyclyl, aryl, substituted aryl, heteroaryl and substituted heteroaryl heteroaryl], or being individually and independent from each other absent,

wherein D is straight $C_1$-$C_6$ alkyl, straight $C_1$-$C_6$ alkenyl, straight $C_1$-$C_6$ alkynyl, whereby any of the alkyl, alkenyl and alkynyl may individually and independently comprise from 0 to 3 heteroatoms, and/or whereby any of the alkyl, alkenyl and alkynyl can be individually and independently substituted by 1 or 2 substituent(s) each independently selected from H, halo, $OR_{16}$, alkyl, and substituted alkyl
wherein n and m are each and independently selected from each other and are each any integer from 0 to 10,

whereby if n is 2 or more, the group(s) $-(CR^aR^b)-$ which is/are repeated, can be the same or different from any of the group(s) $-(CR^aR^b)-$,

whereby any individual group can be linked to any other group or any moiety of the compound through a bond selected from the group comprising single bonds, double bonds and triple bonds,

whereby if m is 2 or more, the group(s) $-(CR^fR^g)-$ which is/are repeated, can be the same or different from any of the group(s) $-(CR^fR^g)-$,

whereby any individual group can be linked to any other group or any moiety of the compound through a bond selected from the group comprising single bonds, double bonds and triple bonds,

wherein t is independently selected from n and/or m and is any integer from 0 to 10,

whereby if t is 2 or more any of the spacer -M1-L1-K-L2-M2- can be the same or different from any of the spacer(s) X repeated,

wherein
$R^c$, $R^d$ and $R^e$ are independently from each other selected from the group H, alkyl, substituted alkyl, cycloalkyl, substituted cycloalkyl, alkylcycloalkyl, substituted alkylcycloalkyl, aryl, substituted aryl, alkylaryl, substituted alkylaryl, heterocyclyl, substituted heterocyclyl, alkylheterocyclyl, substituted alkylheterocyclyl, heteroaryl, substituted heteroaryl, alkylheteroaryl and substituted alkylheteroaryl; and
$R^a$, $R^b$, $R^f$ and $R^g$ are independently from each other selected from the group H, $OR_{17}$, $SR_{18}$, $NR_{19}R_{20}$, halo, alkyl, substituted alkyl, alkylaryl, substituted alkylaryl, cycloalkyl, substituted cycloalkyl, alkylcycloalkyl, substituted alkylcycloalkyl, aryl, substituted aryl, heterocyclyl, substituted heterocyclyl, alkylheterocyclyl, substituted alkylheterocyclyl, heteroaryl, substituted heteroaryl, alkylheteroaryl and substituted alkylheteroaryl; or may be independently from each other absent, and

$R_{16}$, $R_{17}$, $R_{18}$, $R_{19}$, and $R_{20}$ are each and independently selected from the group comprising H, alkyl, substituted alkyl, aryl, substituted aryl, alkylaryl, substituted alkylaryl, alkoxy, substituted alkoxy, aryloxy, substituted aryloxy, alkylamino, substituted alkylamino, arylamino and substituted arylamino;
wherein Y is selected from the group comprising alkyl, substituted alkyl, straight alkyl, substituted straight alkyl, branched alkyl, substituted branched alkyl, straight alkenyl, substituted straight alkenyl, branched alkenyl , substituted branched alkenyl, straight alkynyl, substituted straight alkynyl, branched alkynyl, substituted branched alkynyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, mono-substituted mono-unsaturated heterocyclyl, poly-substituted mono-unsaturated heterocyclyl, aryl, substituted aryl, heteroaryl and substituted heteroaryl, wherein Y is different from a peptide or is absent;

**2.** The compound according to claim 1, wherein K is C=T and T is selected from the group comprising O and S.

**3.** The compound according to any of claims 1 to 2, wherein n and m are individually and independently any integer from 1 to 3 or wherein n is any integer from 0 to 3 and is preferably 0 or 1.

**4.** The compound according to any of the preceding claims, wherein X is
$-(CR^aR^b)_n-NR^c-CZ-NR^d-(CR^fR^g)_m-$
and Z is selected from the group comprising O, S, N-CN, $N-NO_2$ and $CH-NO_2$.

**5.** A compound according to any of claims 1 to 3, wherein X is
$-(CR^aR^b)_n-NR^c-(CR^fR^g)_m-$.

**6.** A compound according to any of claims 1 to 3, wherein X is
$-(CR^aR^b)_n-NR^c-Z-(CR^fR^g)_m-$ and can be inserted in any orientation into any of the preceding formulae.

**7.** The compound according to claim 6, wherein X is
$-(CR^aR^b)_n-NR^c-Z-(CR^fR^g)_m-$ and can be inserted in any orientation into any of the preceding formulae,

and Z is selected from the group comprising C(O), C(S), $S(O_2)$, C(O)-O, and C(O)-S, and
wherein preferably t is 1.

**8.** Compound, preferably a compound according to any of the preceding claims, selected from:

3-[3-(5-Chloro-2-hydroxy-phenyl)-ureido]-propionic acid ethyl ester
1-(5-Chloro-2-hydroxy-phenyl)-3-pentyl-urea
1-Benzyl-3-(5-chloro-2-hydroxy-phenyl)-urea
1-(5-Chloro-2-hydroxy-phenyl)-3-(2-methyl-benzyl)-urea
1-(5-Chloro-2-hydroxy-phenyl)-3-phenethyl-urea
1-(5-Chloro-2-hydroxy-phenyl)-3-(1,1,3,3-tetramethyl-butyl)-urea
1-tert-Butyl-3-(5-chloro-2-hydroxy-phenyl)-urea
1-(5-Chloro-2-hydroxy-phenyl)-3-cyclohexylmethyl-urea
1-(5-Chloro-2-hydroxy-phenyl)-3-(4-trifluoromethyl-benzyl)-urea
1-(5-Chloro-2-hydroxy-phenyl)-3-(3,5-dichloro-phenyl)-urea
1-(5-Chloro-2-hydroxy-phenyl)-3-(4-chloro-phenyl)-urea
1-(5-Chloro-2-hydroxy-phenyl)-3-(4-trifluoromethyl-phenyl)-urea
1-(5-Chloro-2-hydroxy-phenyl)-3-cyclohexyl-urea
1-(5-Chloro-2-hydroxy-phenyl)-3-(4-trifluoromethoxy-phenyl)-urea
1-(5-Chloro-2-hydroxy-phenyl)-3-(4-cyano-phenyl)-urea
1-Benzo[1,3]dioxol-5-yl-3-(5-chloro-2-hydroxy-phenyl)-urea
1-(5-Chloro-2-hydroxy-phenyl)-3-o-tolyl-urea
1-(5-Chloro-2-hydroxy-phenyl)-3-(3-methoxy-phenyl)-urea
1-(5-Chloro-2-hydroxy-phenyl)-3-(2,6-dimethyl-phenyl)-urea
1-(5-Chloro-2-hydroxy-phenyl)-3-(3,4,5-trimethoxy-phenyl)-urea
1-(5-Chloro-2-hydroxy-phenyl)-3-naphthalen-1-yl-urea
1-Adamantan-1-yl-3-(5-chloro-2-hydroxy-phenyl)-urea
1-(5-Chloro-2-hydroxy-phenyl)-3-(4-phenoxy-phenyl)-urea

1-(5-Chloro-2-hydroxy-phenyl)-3-phenyl-urea
3-[3-(3,5-Dichloro-2-hydroxy-phenyl)-ureido]-propionic acid ethyl ester
1-(3,5-Dichloro-2-hydroxy-phenyl)-3-pentyl-urea
1-Benzyl-3-(3,5-dichloro-2-hydroxy-phenyl)-urea
1-(3,5-Dichloro-2-hydroxy-phenyl)-3-(2-methyl-benzyl)-urea
1-(3,5-Dichloro-2-hydroxy-phenyl)-3-phenethyl-urea
1-(3,5-Dichloro-2-hydroxy-phenyl)-3-(1,1,3,3-tetramethyl-butyl)-urea
1-tert-Butyl-3-(3,5-dichloro-2-hydroxy-phenyl)-urea
1-(3,5-Dichloro-2-hydroxy-phenyl)-3-cyclohexylmethyl-urea
1-(3,5-Dichloro-2-hydroxy-phenyl)-3-(4-trifluoromethyl-benzyl)-urea
1-(3,5-Dichloro-2-hydroxy-phenyl)-3-(3,5-dichloro-phenyl)-urea
1-(3,5-Dichloro-2-hydroxy-phenyl)-3-(4-chloro-phenyl)-urea
1-(3,5-Dichloro-2-hydroxy-phenyl)-3-(4-trifluoromethyl-phenyl)-urea
1-(3,5-Dichloro-2-hydroxy-phenyl)-3-cyclohexyl-urea
1-(3,5-Dichloro-2-hydroxy-phenyl)-3-(4-trifluoromethoxy-phenyl)-urea
1-(3,5-Dichloro-2-hydroxy-phenyl)-3-(4-cyano-phenyl)-urea
1-Benzo[1,3]dioxol-5-yl-3-(3,5-dichloro-2-hydroxy-phenyl)-urea
1-(3,5-Dichloro-2-hydroxy-phenyl)-3-o-tolyl-urea
1-(3,5-Dichloro-2-hydroxy-phenyl)-3-(3-methoxy-phenyl)-urea
1-(3,5-Dichloro-2-hydroxy-phenyl)-3-(2,6-dimethyl-phenyl)-urea
1-(3,5-Dichloro-2-hydroxy-phenyl)-3-(3,4,5-trimethoxy-phenyl)-urea
1-(3,5-Dichloro-2-hydroxy-phenyl)-3-naphthalen-1-yl-urea
1-Adamantan-1-yl-3-(3,5-dichloro-2-hydroxy-phenyl)-urea
1-(3,5-Dichloro-2-hydroxy-phenyl)-3-(4-phenoxy-phenyl)-urea
1-(3,5-Dichloro-2-hydroxy-phenyl)-3-phenyl-urea
3-[3-(3-Chloro-2-hydroxy-phenyl)-ureido]-propionic acid ethyl ester
1-(3-Chloro-2-hydroxy-phenyl)-3-pentyl-urea
1-Benzyl-3-(3-chloro-2-hydroxy-phenyl)-urea
1-(3-Chloro-2-hydroxy-phenyl)-3-(2-methyl-benzyl)-urea
1-(3-Chloro-2-hydroxy-phenyl)-3-phenethyl-urea
1-(3-Chloro-2-hydroxy-phenyl)-3-(1,1,3,3-tetramethyl-butyl)-urea
1-tert-Butyl-3-(3-chloro-2-hydroxy-phenyl)-urea
1-(3-Chloro-2-hydroxy-phenyl)-3-cyclohexylmethyl-urea
1-(3-Chloro-2-hydroxy-phenyl)-3-(4-trifluoromethyl-benzyl)-urea
1-(3-Chloro-2-hydroxy-phenyl)-3-(3,5-dichloro-phenyl)-urea
1-(3-Chloro-2-hydroxy-phenyl)-3-(4-chloro-phenyl)-urea
1-(3-Chloro-2-hydroxy-phenyl)-3-(4-trifluoromethyl-phenyl)-urea
1-(3-Chloro-2-hydroxy-phenyl)-3-cyclohexyl-urea
1-(3-Chloro-2-hydroxy-phenyl)-3-(4-trifluoromethoxy-phenyl)-urea
1-(3-Chloro-2-hydroxy-phenyl)-3-(4-cyano-phenyl)-urea
1-Benzo[1,3]dioxol-5-yl-3-(3-chloro-2-hydroxy-phenyl)-urea
1-(3-Chloro-2-hydroxy-phenyl)-3-o-tolyl-urea
1-(3-Chloro-2-hydroxy-phenyl)-3-(3-methoxy-phenyl)-urea
1-(3-Chloro-2-hydroxy-phenyl)-3-(2,6-dimethyl-phenyl)-urea
1-(3-Chloro-2-hydroxy-phenyl)-3-(3,4,5-trimethoxy-phenyl)-urea
1-(3-Chloro-2-hydroxy-phenyl)-3-naphthalen-1-yl-urea
1-Adamantan-1-yl-3-(3-chloro-2-hydroxy-phenyl)-urea
1-(3-Chloro-2-hydroxy-phenyl)-3-(4-phenoxy-phenyl)-urea
1-(3-Chloro-2-hydroxy-phenyl)-3-phenyl-urea
3-[3-(3-Fluoro-2-hydroxy-phenyl)-ureido]-propionic acid ethyl ester
1-(3-Fluoro-2-hydroxy-phenyl)-3-pentyl-urea
1-Benzyl-3-(3-fluoro-2-hydroxy-phenyl)-urea
1-(3-Fluoro-2-hydroxy-phenyl)-3-(2-methyl-benzyl)-urea
1-(3-Fluoro-2-hydroxy-phenyl)-3-phenethyl-urea
1-(3-Fluoro-2-hydroxy-phenyl)-3-(1,1,3,3-tetramethyl-butyl)-urea
1-tert-Butyl-3-(3-fluoro-2-hydroxy-phenyl)-urea
1-(3-Fluoro-2-hydroxy-phenyl)-3-cyclohexylmethyl-urea
1-(3-Fluoro-2-hydroxy-phenyl)-3-(4-trifluoromethyl-benzyl)-urea

1-(3-Fluoro-2-hydroxy-phenyl)-3-(3,5-dichloro-phenyl)-urea
1-(3-Fluoro-2-hydroxy-phenyl)-3-(4-chloro-phenyl)-urea
1-(3-Fluoro-2-hydroxy-phenyl)-3-(4-trifluoromethyl-phenyl)-urea
1-(3-Fluoro-2-hydroxy-phenyl)-3-cyclohexyl-urea
1-(3-Fluoro-2-hydroxy-phenyl)-3-(4-trifluoromethoxy-phenyl)-urea
1-(3-Fluoro-2-hydroxy-phenyl)-3-(4-cyano-phenyl)-urea
1-Benzo[1,3]dioxol-5-yl-3-(3-fluoro-2-hydroxy-phenyl)-urea
1-(3-Fluoro-2-hydroxy-phenyl)-3-o-tolyl-urea
1-(3-Fluoro-2-hydroxy-phenyl)-3-(3-methoxy-phenyl)-urea
1-(3-Fluoro-2-hydroxy-phenyl)-3-(2,6-dimethyl-phenyl)-urea
1-(3-Fluoro-2-hydroxy-phenyl)-3-(3,4,5-trimethoxy-phenyl)-urea
1-(3-Fluoro-2-hydroxy-phenyl)-3-naphthalen-1-yl-urea
1-Adamantan-1-yl-3-(3-fluoro-2-hydroxy-phenyl)-urea
1-(3-Fluoro-2-hydroxy-phenyl)-3-(4-phenoxy-phenyl)-urea
1-(3-Fluoro-2-hydroxy-phenyl)-3-phenyl-urea
3-[3-(3,5-Dichloro-2-hydroxy-4-methyl-phenyl)-ureido]-propionic acid ethyl ester
1-(3,5-Dichloro-2-hydroxy-4-methyl-phenyl)-3-pentyl-urea
1-Benzyl-3-(3,5-dichloro-2-hydroxy-4-methyl-phenyl)-urea
1-(3,5-Dichloro-2-hydroxy-4-methyl-phenyl)-3-(2-methyl-benzyl)-urea
1-(3,5-Dichloro-2-hydroxy-4-methyl-phenyl)-3-phenethyl-urea
1-(3,5-Dichloro-2-hydroxy-4-methyl-phenyl)-3-(1,1,3,3-tetramethyl-butyl)-urea
1-tert-Butyl-3-(3,5-dichloro-2-hydroxy-4-methyl-phenyl)-urea
1-Cyclohexylmethyl-3-(3,5-dichloro-2-hydroxy-4-methyl-phenyl)-urea
1-(3,5-Dichloro-2-hydroxy-4-methyl-phenyl)-3-(4-trifluoromethyl-benzyl)-urea
1-(3,5-Dichloro-2-hydroxy-4-methyl-phenyl)-3-(3,5-dichloro-phenyl)-urea
1-(4-Chloro-phenyl)-3-(3,5-dichloro-2-hydroxy-4-methyl-phenyl)-urea
1-(3,5-Dichloro-2-hydroxy-4-methyl-phenyl)-3-(4-trifluoromethyl-phenyl)-urea
1-Cyclohexyl-3-(3,5-dichloro-2-hydroxy-4-methyl-phenyl)-urea
1-(3,5-Dichloro-2-hydroxy-4-methyl-phenyl)-3-(4-trifluoromethoxy-phenyl)-urea
1-(4-Cyano-phenyl)-3-(3,5-dichloro-2-hydroxy-4-methyl-phenyl)-urea
1-Benzo[1,3]dioxol-5-yl-3-(3,5-dichloro-2-hydroxy-4-methyl-phenyl)-urea
1-(3,5-Dichloro-2-hydroxy-4-methyl-phenyl)-3-o-tolyl-urea
1-(3,5-Dichloro-2-hydroxy-4-methyl-phenyl)-3-(3-methoxy-phenyl)-urea
1-(3,5-Dichloro-2-hydroxy-4-methyl-phenyl)-3-(2,6-dimethyl-phenyl)-urea
1-(3,5-Dichloro-2-hydroxy-4-methyl-phenyl)-3-(3,4,5-trimethoxy-phenyl)-urea
1-(3,5-Dichloro-2-hydroxy-4-methyl-phenyl)-3-naphthalen-1-yl-urea
1-Adamantan-1-yl-3-(3,5-dichloro-2-hydroxy-4-methyl-phenyl)-urea
1-(3,5-Dichloro-2-hydroxy-4-methyl-phenyl)-3-(4-phenoxy-phenyl)-urea
1-(3,5-Dichloro-2-hydroxy-4-methyl-phenyl)-3-phenyl-urea
3-[3-(5-Bromo-3-fluoro-2-hydroxy-phenyl)-ureido]-propionic acid ethyl ester
1-(5-Bromo-3-fluoro-2-hydroxy-phenyl)-3-pentyl-urea
1-Benzyl-3-(5-bromo-3-fluoro-2-hydroxy-phenyl)-urea
1-(5-Bromo-3-fluoro-2-hydroxy-phenyl)-3-(2-methyl-benzyl)-urea
1-(5-Bromo-3-fluoro-2-hydroxy-phenyl)-3-phenethyl-urea
1-(5-Bromo-3-fluoro-2-hydroxy-phenyl)-3-(1,1,3,3-tetramethyl-butyl)-urea
1-tert-Butyl-3-(5-bromo-3-fluoro-2-hydroxy-phenyl)-urea
1-(5-Bromo-3-fluoro-2-hydroxy-phenyl)-3-cyclohexylmethyl-urea
1-(5-Bromo-3-fluoro-2-hydroxy-phenyl)-3-(4-trifluoromethyl-benzyl)-urea
1-(5-Bromo-3-fluoro-2-hydroxy-phenyl)-3-(3,5-dichloro-phenyl)-urea
1-(5-Bromo-3-fluoro-2-hydroxy-phenyl)-3-(4-chloro-phenyl)-urea
1-(5-Bromo-3-fluoro-2-hydroxy-phenyl)-3-(4-trifluoromethyl-phenyl)-urea
1-(5-Bromo-3-fluoro-2-hydroxy-phenyl)-3-cyclohexyl-urea
1-(5-Bromo-3-fluoro-2-hydroxy-phenyl)-3-(4-trifluoromethoxy-phenyl)-urea
1-(5-Bromo-3-fluoro-2-hydroxy-phenyl)-3-(4-cyano-phenyl)-urea
1-Benzo[1,3]dioxol-5-yl-3-(5-bromo-3-fluoro-2-hydroxy-phenyl)-urea
1-(5-Bromo-3-fluoro-2-hydroxy-phenyl)-3-o-tolyl-urea
1-(5-Bromo-3-fluoro-2-hydroxy-phenyl)-3-(3-methoxy-phenyl)-urea
1-(5-Bromo-3-fluoro-2-hydroxy-phenyl)-3-(2,6-dimethyl-phenyl)-urea

1-(5-Bromo-3-fluoro-2-hydroxy-phenyl)-3-(3,4,5-trimethoxy-phenyl)-urea
1-(5-Bromo-3-fluoro-2-hydroxy-phenyl)-3-naphthalen-1-yl-urea
1-Adamantan-1-yl-3-(5-Bromo-3-fluoro-2-hydroxy-phenyl)-urea
1-(5-Bromo-3-fluoro-2-hydroxy-phenyl)-3-(4-phenoxy-phenyl)-urea
1-(5-Bromo-3-fluoro-2-hydroxy-phenyl)-3-phenyl-urea
3-[3-(3,5-Difluoro-2-hydroxy-phenyl)-ureido]-propionic acid ethyl ester
1-(3,5-Difluoro-2-hydroxy-phenyl)-3-pentyl-urea
1-Benzyl-3-(3,5-difluoro-2-hydroxy-phenyl)-urea
1-(3,5-Difluoro-2-hydroxy-phenyl)-3-(2-methyl-benzyl)-urea
1-(3,5-Difluoro-2-hydroxy-phenyl)-3-phenethyl-urea
1-(3,5-Difluoro-2-hydroxy-phenyl)-3-(1,1,3,3-tetramethyl-butyl)-urea
1-tert-Butyl-3-(3,5-difluoro-2-hydroxy-phenyl)-urea
1-(3,5-Difluoro-2-hydroxy-phenyl)-3-cyclohexylmethyl-urea
1-(3,5-Difluoro-2-hydroxy-phenyl)-3-(4-trifluoromethyl-benzyl)-urea
1-(3,5-Difluoro-2-hydroxy-phenyl)-3-(3,5-dichloro-phenyl)-urea
1-(3,5-Difluoro-2-hydroxy-phenyl)-3-(4-chloro-phenyl)-urea
1-(3,5-Difluoro-2-hydroxy-phenyl)-3-(4-trifluoromethyl-phenyl)-urea
1-(3,5-Difluoro-2-hydroxy-phenyl)-3-cyclohexyl-urea
1-(3,5-Difluoro-2-hydroxy-phenyl)-3-(4-trifluoromethoxy-phenyl)-urea
1-(3,5-Difluoro-2-hydroxy-phenyl)-3-(4-cyano-phenyl)-urea
1-Benzo[1,3]dioxol-5-yl-3-(3,5-difluoro-2-hydroxy-phenyl)-urea
1-(3,5-Difluoro-2-hydroxy-phenyl)-3-o-tolyl-urea
1-(3,5-Difluoro-2-hydroxy-phenyl)-3-(3-methoxy-phenyl)-urea
1-(3,5-Difluoro-2-hydroxy-phenyl)-3-(2,6-dimethyl-phenyl)-urea
1-(3,5-Difluoro-2-hydroxy-phenyl)-3-(3,4,5-trimethoxy-phenyl)-urea
1-(3,5-Difluoro-2-hydroxy-phenyl)-3-naphthalen-1-yl-urea
1-Adamantan-1-yl-3-(3,5-difluoro-2-hydroxy-phenyl)-urea
1-(3,5-Difluoro-2-hydroxy-phenyl)-3-(4-phenoxy-phenyl)-urea
1-(3,5-Difluoro-2-hydroxy-phenyl)-3-phenyl-urea
3-{3-[5-Chloro-2-hydroxy-3-(1-hydroxy-ethyl)-phenyl]-ureido}-propionic acid ethyl ester
1-[5-Chloro-2-hydroxy-3-(1-hydroxy-ethyl)-phenyl]-3-pentyl-urea
1-[5-Chloro-2-hydroxy-3-(-hydroxy-ethyl)-phenyl]-3-pentyl-urea
1-[5-Chloro-2-hydroxy-3-(1-hydroxy-ethyl)-phenyl]-3-(2-methyl-benzyl)-urea
1-[5-Chloro-2-hydroxy-3-(1-hydroxy-ethyl)-phenyl]-3-phenethyl-urea
1-[5-Chloro-2-hydroxy-3-(1-hydroxy-ethyl)-phenyl]-3-(1,1,3,3-tetramethyl-butyl)-urea
1-tert-Butyl-3-[5-chloro-2-hydroxy-3-(1-hydroxy-ethyl)-phenyl]-urea
1-[5-Chloro-2-hydroxy-3-(1-hydroxy-ethyl)-phenyl]-3-cyclohexylmethyl-urea
1-[5-Chloro-2-hydroxy-3-(1-hydroxy-ethyl)-phenyl]-3-(4-trifluoromethyl-benzyl)-urea
1-[5-Chloro-2-hydroxy-3-(-hydroxy-ethyl)-phenyl]-3-(4-chloro-phenyl)-urea
1-[5-Chloro-2-hydroxy-3-(1-hydroxy-ethyl)-phenyl]-3-(4-chloro-phenyl)-urea
1-[5-Chloro-2-hydroxy-3-(1-hydroxy-ethyl)-phenyl]-3-(4-trifluoromethyl-phenyl)-urea
1-[5-Chloro-2-hydroxy-3-(1-hydroxy-ethyl)-phenyl]-3-cyclohexyl-urea
1-[5-Chloro-2-hydroxy-3-(1-hydroxy-ethyl)-phenyl]-3-(4-trifluoromethoxy-phenyl)-urea
1-[5-Chloro-2-hydroxy-3-(1-hydroxy-ethyl)-phenyl]-3-(4-cyano-phenyl)-urea
1-Benzo[1,3]dioxol-5-yl-3-[5-chloro-2-hydroxy-3-(1-hydroxy-ethyl)-phenyl]-urea
1-[5-Chloro-2-hydroxy-3-(1-hydroxy-ethyl)-phenyl]-3-o-tolyl-urea
1-[5-Chloro-2-hydroxy-3-(1-hydroxy-ethyl)-phenyl]-3-(3-methoxy-phenyl)-urea
1-[5-Chloro-2-hydroxy-3-(1-hydroxy-ethyl)-phenyl]-3-(2,6-dimethyl-phenyl)-urea
1-[5-Chloro-2-hydroxy-3-(1-hydroxy-ethyl)-phenyl]-3-(3,4,5-trimethoxy-phenyl)-urea
1-[5-Chloro-2-hydroxy-3-(1-hydroxy-ethyl)-phenyl]-3-naphthalen-1-yl-urea
1-Adamantan-1-yl-3-[5-chloro-2-hydroxy-3-(1-hydroxy-ethyl)-phenyl]-urea
1-[5-Chloro-2-hydroxy-3-(1-hydroxy-ethyl)-phenyl]-3-(4-phenoxy-phenyl)-urea
1-[5-Chloro-2-hydroxy-3-(1-hydroxy-ethyl)-phenyl]-3-phenyl-urea
1-(5-Chloro-2-hydroxy-phenyl)-3-pentyl-thiourea
1-Benzyl-3-(5-chloro-2-hydroxy-phenyl)-thiourea
1-(5-Chloro-2-hydroxy-phenyl)-3-(2-methyl-benzyl)-thiourea
1-(5-Chloro-2-hydroxy-phenyl)-3-phenethyl-thiourea
1-(5-Chloro-2-hydroxy-phenyl)-3-(1,1,3,3-tetramethyl-butyl)-thiourea

1-tert-Butyl-3-(5-chloro-2-hydroxy-phenyl)-thiourea
1-(5-Chloro-2-hydroxy-phenyl)-3-isopropyl-thiourea
1-(5-Chloro-2-hydroxy-phenyl)-3-cyclohexylmethyl-thiourea
1-(5-Chloro-2-hydroxy-phenyl)-3-(4-trifluoromethyl-benzyl)-thiourea
1-(5-Chloro-2-hydroxy-phenyl)-3-(3,5-dichloro-phenyl)-thiourea
1-(5-Chloro-2-hydroxy-phenyl)-3-(4-chloro-phenyl)-thiourea
1-(5-Chloro-2-hydroxy-phenyl)-3-(4-trifluoromethyl-phenyl)-thiourea
1-(5-Chloro-2-hydroxy-phenyl)-3-cyclohexyl-thiourea
1-(S-Chloro-2-hydroxy-phenyl)-3-(2-trifluoromethyl-phenyl)-thiourea
1-(5-Chloro-2-hydroxy-phenyl)-3-phenyl-thiourea
1-(3,5-Dichloro-2-hydroxy-phenyl)-3-pentyl-thiourea
1-Benzyl-3-(3,5-dichloro-2-hydroxy-phenyl)-thiourea
1-(3,5-Dichloro-2-hydroxy-phenyl)-3-(2-methyl-benzyl)-thiourea
1-(3,5-Dichloro-2-hydroxy-phenyl)-3-phenethyl-thiourea
1-(3,5-Dichloro-2-hydroxy-phenyl)-3-(1,1,3,3-tetramethyl-butyl)-thiourea
1-tert-Butyl-3-(3,5-dichloro-2-hydroxy-phenyl)-thiourea
1-(5-Chloro-2-hydroxy-phenyl)-3-isopropyl-thiourea
1-(3,5-Dichloro-2-hydroxy-phenyl)-3-cyclohexylmethyl-thiourea
1-(3,5-Dichloro-2-hydroxy-phenyl)-3-(4-trifluoromethyl-benzyl)-thiourea
1-(3,5-Dichloro-2-hydroxy-phenyl)-3-(3,5-dichloro-phenyl)-thiourea
1-(3,5-Dichloro-2-hydroxy-phenyl)-3-(4-chloro-phenyl)-thiourea
1-(3,5-Dichloro-2-hydroxy-phenyl)-3-(4-trifluoromethyl-phenyl)-thiourea
1-(3,5-Dichloro-2-hydroxy-phenyl)-3-cyclohexyl-thiourea
1-(3,5-Dichloro-2-hydroxy-phenyl)-3-(2-trifluoromethyl-phenyl)-thiourea
1-(3,5-Dichloro-2-hydroxy-phenyl)-3-phenyl-thiourea
1-(3-Chloro-2-hydroxy-phenyl)-3-pentyl-thiourea
1- -Benzyl-3-(3-chloro-2-hydroxy-phenyl)-thiourea
1-(3-Chloro-2-hydroxy-phenyl)-3-(2-methyl-benzyl)-thiourea
1-(3-Chloro-2-hydroxy-phenyl)-3-phenethyl-thiourea
1-(3-Chloro-2-hydroxy-phenyl)-3-(1,1,3,3-tetramethyl-butyl)-thiourea
1-tert-Butyl-3-(3-Chloro-2-hydroxy-phenyl)-thiourea
1-(3-Chloro-2-hydroxy-phenyl)-3-isopropyl-thiourea
1-(3-Chloro-2-hydroxy-phenyl)-3-cyclohexylmethyl-thiourea
1-(3-Chloro-2-hydroxy-phenyl)-3 -(4-trifluoromethyl-benzyl)-thiourea
1-(3-Chloro-2-hydroxy-phenyl)-3-(3,5-dichloro-phenyl)-thiourea
1-(3-Chloro-2-hydroxy-phenyl)-3-(4-chloro-phenyl)-thiourea
1-(3-Chloro-2-hydroxy-phenyl)-3-(4-trifluoromethyl-phenyl)-thiourea
1-(3-Chloro-2-hydroxy-phenyl)-3-cyclohexyl-thiourea
1-(3-Chloro-2-hydroxy-phenyl)-3-(2-trifluoromethyl-phenyl)-thiourea
1-(3-Chloro-2-hydroxy-phenyl)-3-phenyl-thiourea
1-(3-Fluoro-2-hydroxy-phenyl)-3-pentyl-thiourea
1-Benzyl-3-(3-fluoro-2-hydroxy-phenyl)-thiourea
1-(3-Fluoro-2-hydroxy-phenyl)-3-(2-methyl-benzyl)-thiourea
1-(3-Fluoro-2-hydroxy-phenyl)-3-phenethyl-thiourea
1-(3-Fluoro-2-hydroxy-phenyl)-3-(1,1,3,3-tetramethyl-butyl)-thiourea
1-tert-Butyl-3-(3-fluoro-2-hydroxy-phenyl)-thiourea
1-(3-Fluoro-2-hydroxy-phenyl)-3-isopropyl-thiourea
1-(3-Fluoro-2-hydroxy-phenyl)-3-cyclohexylmethyl-thiourea
1-(3-Fluoro-2-hydroxy-phenyl)-3-(4-trifluoromethyl-benzyl)-thiourea
1-(3-Fluoro-2-hydroxy-phenyl)-3 -(3,5 -dichloro-phenyl)-thiourea
1-(3-Fluoro-2-hydroxy-phenyl)-3-(4-chloro-phenyl)-thiourea
1-(3-Fluoro-2-hydroxy-phenyl)-3-(4-trifluoromethyl-phenyl)-thiourea
1-(3-Fluoro-2-hydroxy-phenyl)-3-cyclohexyl-thioruea
1-(3-Fluoro-2-hydroxy-phenyl)-3-(2-trifluoromethyl-phenyl)-thiourea
1-(3-Fluoro-2-hydroxy-phenyl)-3-phenyl-thiourea
1-(3,5-Dichloro-2-hydroxy-4-methyl-phenyl)-3-pentyl-thiourea
1-Benzyl-3-(3,5-dichloro-2-hydroxy-4-methyl-phenyl)-thiourea
1-(3,5-Dichloro-2-hydroxy-4-methyl-phenyl)-3-(2-methyl-benzyl)-thiourea

1-(3,5-Dichloro-2-hydroxy-4-methyl-phenyl)-3-phenethyl-thiourea
1-(3,5-Dichloro-2-hydroxy-4-methyl-phenyl)-3-(1,1,3,3-tetramethyl-butyl)-thiourea
1-tert-Butyl-3-(3,5-dichloro-2-hydroxy-4-methyl-phenyl)-thiourea
1-(5-Chloro-2-hydroxy-4-methyl-phenyl)-3-isopropyl-thiourea
1-(3,5-Dichloro-2-hydroxy-4-methyl-phenyl)-3-cyclohexylmethyl-thiourea
1-(3,5-Dichloro-2-hydroxy-4-methyl-phenyl)-3-(4-trifluoromethyl-benzyl)-thiourea
1-(3,5-Dichloro-2-hydroxy-4-methyl-phenyl)-3-(3,5-dichloro-phenyl)-thiourea
1-(3,5-Dichloro-2-hydroxy-4-methyl-phenyl)-3-(4-chloro-phenyl)-thiourea
1-(3,5-Dichloro-2-hydroxy-4-methyl-phenyl)-3-(4-trifluoromethyl-phenyl)-thiourea
1-(3,5-Dichloro-2-hydroxy-4-methyl-phenyl)-3-cyclohexyl-thiourea
1-(3,5-Dichloro-2-hydroxy-4-methyl-phenyl)-3-(2-trifluoromethyl-phenyl)-thiourea
1-(3,5-Dichloro-2-hydroxy-4-methyl-phenyl)-3-phenyl-thiourea
1-(5-Bromo-3-fluoro-2-hydroxy-phenyl)-3-pentyl-thiourea
1-Benzyl-3-(5-bromo-3-fluoro-2-hydroxy-phenyl)-thiourea
1-(5-Bromo-3-fluoro-2-hydroxy-phenyl)-3-(2-methyl-benzyl)-thiourea
1-(5-Bromo-3-fluoro-2-hydroxy-phenyl)-3-phenethyl-thiourea
1-(5-Bromo-3-fluoro-2-hydroxy-phenyl)-3-(1,1,3,3-tetramethyl-butyl)-thiourea
1-tert-Butyl-3-(5-bromo-3-fluoro-2-hydroxy-phenyl)-thiourea
1-(5-Bromo-3-fluoro-2-hydroxy-phenyl)-3-isopropyl-thiourea
1-(5-Bromo-3-fluoro-2-hydroxy-phenyl)-3-cyclohexylmethyl-thiourea
1-(5-Bromo-3-fluoro-2-hydroxy-phenyl)-3-(4-trifluoromethyl-benzyl)-thiourea
1-(5-Bromo-3-fluoro-2-hydroxy-phenyl)-3-(3,5-dichloro-phenyl)-thiourea
1-(5-Bromo-3-fluoro-2-hydroxy-phenyl)-3-(4-chloro-phenyl)-thiourea
1-(5-Bromo-3-fluoro-2-hydroxy-phenyl)-3-(4-trifluoromethyl-phenyl)-thiourea
1-(5-Bromo-3-fluoro-2-hydroxy-phenyl)-3-cyclohexyl-thiourea
1-(5-Bromo-3-fluoro-2-hydroxy-phenyl)-3-(2-trifluoromethyl-phenyl)-thiourea
1-(5-Bromo-3-fluoro-2-hydroxy-phenyl)-3-phenyl-thiourea
1-(3,4-Difluoro-2-hydroxy-phenyl)-3-pentyl-thiourea
1-Benzyl-3-(3,4-difluoro-2-hydroxy-phenyl)-thiourea
1-(3,4-Difluoro-2-hydroxy-phenyl)-3-(2-methyl-benzyl)-thiourea
1-(3,4-Difluoro-2-hydroxy-phenyl)-3-phenethyl-thiourea
1-(3,4-Difluoro-2-hydroxy-phenyl)-3-(1,1,3,3-tetramethyl-butyl)-thiourea
1-tert-Butyl-3-(3,4-Difluoro-2-hydroxy-phenyl)-thiourea
1-(3,4-Difluoro-2-hydroxy-phenyl)-3-isopropyl-thiourea
1-(3,4-Difluoro-2-hydroxy-phenyl)-3-cyclohexylmethyl-thiourea
1-(3,4-Difluoro-2-hydroxy-phenyl)-3-(4-trifluoromethyl-benzyl)-thiourea
1-(3,4-Difluoro-2-hydroxy-phenyl)-3-(3,5-dichloro-phenyl)-thiourea
1-(3,4-Difluoro-2-hydroxy-phenyl)-3-(4-chloro-phenyl)-thiourea
1-(3,4-Difluoro-2-hydroxy-phenyl)-3-(4-trifluoromethyl-phenyl)-thiourea
1-(3,4-Difluoro-2-hydroxy-phenyl)-3-cyclohexyl-thiourea
1-(3,4-Difluoro-2-hydroxy-phenyl)-3-(2-trifluoromethyl-phenyl)-thiourea
1-(3,4-Difluoro-2-hydroxy-phenyl)-3-phenyl-thiourea
1-[5-Chloro-2-hydroxy-3-(1-hydroxy-ethyl)-phenyl]-3-pentyl-thiourea
1-Benzyl-3-[5-chloro-2-hydroxy-3-(1-hydroxy-ethyl)-phenyl]-thiourea
1-[5-Chloro-2-hydroxy-3-(1-hydroxy-ethyl)-phenyl]-3-(2-methyl-benzyl)-thiourea
1-[5-Chloro-2-hydroxy-3-(1-hydroxy-ethyl)-phenyl]-3-phenethyl-thiourea
1-[5-Chloro-2-hydroxy-3-(1-hydroxy-ethyl)-phenyl]-3-(1,1,3,3-tetramethyl-butyl)-thiourea
1-tert-Butyl-3-[5-chloro-2-hydroxy-3-(1-hydroxy-ethyl)-phenyl]-thiourea
1-[5-Chloro-2-hydroxy-3-(1-hydroxy-ethyl)-phenyl]-3-isopropyl-thiourea
1-[5-Chloro-2-hydroxy-3-(-hydroxy-ethyl)-phenyl]-3-cyclohexylmethyl-thiourea
1-[5-Chloro-2-hydroxy-3-(-hydroxy-ethyl)-phenyl]-3-(4-trifluoromethyl-benzyl)-thiourea
1-[5-Chloro-2-hydroxy-3-(1-hydroxy-ethyl)-phenyl]-3-(3,5-dichloro-phenyl)-thiourea
1-[5-Chloro-2-hydroxy-3-(1-hydroxy-ethyl)-phenyl]-3-(4-chloro-phenyl)-thiourea
1-[5-Chloro-2-hydroxy-3-(1-hydroxy-ethyl)-phenyl]-3-(4-trifluoromethyl-phenyl)-thiourea
1-[5-Chloro-2-hydroxy-3-(1-hydroxy-ethyl)-phenyl]-3-cyclohexyl-thiourea
1-[5-Chloro-2-hydroxy-3-(1-hydroxy-ethyl)-phenyl]-3-(2-trifluoromethyl-phenyl)-thiourea
1-[5-Chloro-2-hydroxy-3-(1-hydroxy-ethyl)-phenyl]-3-phenyl-thiourea
4-Chloro-2-(2-phenylsulfanyl-benzylamino)-phenol

4-Chloro-2-(2-p-tolylsulfanyl-benzylamino)-phenol
4-Chloro-2-[2-(4-chloro-phenylsulfanyl)-benzylamino]-phenol
4-Chloro-2-[2-(4-nitro-phenylsulfanyl)-benzylamino]-phenol
4-Chloro-2-[2-(4-methoxy-phenylsulfanyl)-benzylamino]-phenol
4-Chloro-2-[2-(2-chloro-phenylsulfanyl)-benzylamino]-phenol
4-Chloro-2-[2-(3-chloro-phenylsulfanyl)-benzylamino]-phenol
4-Chloro-2-[2-(3,4-dichloro-phenylsulfanyl)-benzylamino]-phenol
N-(4-{2-[(5-Chloro-2-hydroxy-phenylamino)-methyl]-phenylsulfanyl}-phenyl)-acetamide
4-Chloro-2-[2-(quinolin-7-ylsulfanyl)-benzylamino]-phenol
4-Chloro-2-[2-(4-chloro-phenylsulfanyl)-5-nitro-benzylamino]-phenol
4-Chloro-2-(5-nitro-2-p-tolylsulfanyl-benzylamino)-phenol
2-[5-Amino-2-(4-chloro-phenylsulfanyl)-benzylamino]-4-chloro-phenol
4-Chloro-2-[2-(4-chloro-benzenesulfonyl)-benzylamino]-phenol
2,4-Dichloro-6-(2-phenylsulfanyl-benzylamino)-phenol
2,4-Dichloro-6-(2-p-tolylsulfanyl-benzylamino)-phenol
2,4-Dichloro-6-[2-(4-chloro-phenylsulfanyl)-benzylamino]-phenol
2,4-Dichloro-6-[2-(4-nitro-phenylsulfanyl)-benzylamino]-phenol
2,4-Dichloro-6-[2-(4-methoxy-phenylsulfanyl)-benzylamino]-phenol
2,4-Dichloro-6-[2-(2-chloro-phenylsulfanyl)-benzylamino]-phenol
2,4-Dichloro-6-[2-(3-chloro-phenylsulfanyl)-benzylamino]-phenol
2,4-Dichloro-6-[2-(3,4-dichloro-phenylsulfanyl)-benzylamino]-phenol
N-(4-{2-[(3,5-Dichloro-2-hydroxy-phenylamino)-methyl]-phenylsulfanyl}-phenyl)-acetamide
2,4-Dichloro-6-[2-(quinolin-7-ylsulfanyl)-benzylamino]-phenol
2,4-Dichloro-6-[2-(4-chloro-phenylsulfanyl)-5-nitro-benzylamino]-phenol
2,4-Dichloro-6-(5-nitro-2-p-tolylsulfanyl-benzylamino)-phenol
2-[5-Amino-2-(4-chloro-phenylsulfanyl)-benzylamino]-4,6-dichloro-phenol
2,4-Dichloro-6-[2-(4-chloro-benzenesulfonyl)-benzylamino]-phenol
2-Chloro-6-(2-phenylsulfanyl-benzylamino)-phenol
2-Chloro-6-(2-p-tolylsulfanyl-benzylamino)-phenol
2-Chloro-6-[2-(4-chloro-phenylsulfanyl)-benzylamino]-phenol
2-Chloro-6-[2-(4-nitro-phenylsulfanyl)-benzylamino]-phenol
2-Chloro-6-[2-(4-methoxy-phenylsulfanyl)-benzylamino]-phenol
2-Chloro-6-[2-(2-chloro-phenylsulfanyl)-benzylamino]-phenol
2-Chloro-6-[2-(3-chloro-phenylsulfanyl)-benzylamino]-phenol
2-Chloro-6-[2-(3,4-dichloro-phenylsulfanyl)-benzylamino]-phenol
N-(4-{2-[(3-Chloro-2-hydroxy-phenylamino)-methyl]-phenylsulfanyl}-phenyl)-acetamide
2-Chloro-6-[2-(quinolin-7-ylsulfanyl)-benzylamino]-phenol
2-Chloro-6-[2-(4-chloro-phenylsulfanyl)-5-nitro-benzylamino]-phenol
2-Chloro-6-(5-nitro-2-p-tolylsulfanyl-benzylamino)-phenol
2-[5-Amino-2-(4-chloro-phenylsulfanyl)-benzylamino]-6-chloro-phenol
2-Chloro-6-[2-(4-chloro-benzenesulfonyl)-benzylamino]-phenol
2-Fluoro-6-(2-phenylsulfanyl-benzylamino)-phenol
2-Fluoro-6-(2-p-tolylsulfanyl-benzylamino)-phenol
2-Fluoro-6-[2-(4-chloro-phenylsulfanyl)-benzylamino]-phenol
2-Fluoro-6-[2-(4-nitro-phenylsulfanyl)-benzylamino]-phenol
2-Fluoro-6-[2-(4-methoxy-phenylsulfanyl)-benzylamino]-phenol
2-Fluoro-6-[2-(2-chloro-phenylsulfanyl)-benzylamino]-phenol
2-Fluoro-6-[2-(3-chloro-phenylsulfanyl)-benzylamino]-phenol
2-Fluoro-6-[2-(3,4-dichloro-phenylsulfanyl)-benzylamino]-phenol
N-(4-{2-[(3-Fluoro-2-hydroxy-phenylamino)-methyl]-phenylsulfanyl}-phenyl)-acetamide
2-Fluoro-6-[2-(quinolin-7-ylsulfanyl)-benzylamino]-phenol
2-Fluoro-6-[2-(4-chloro-phenylsulfanyl)-5-nitro-benzylamino]-phenol
2-Fluoro-6-(5-nitro-2-p-tolylsulfanyl-benzylamino)-phenol
2-[5-Amino-2-(4-chloro-phenylsulfanyl)-benzylamino]-6-fluoro-phenol
2-Fluoro-6-[2-(4-chloro-benzenesulfonyl)-benzylamino]-phenol
2,4-Dichloro-3-methyl-6-(2-phenylsulfanyl-benzylamino)-phenol
2,4-Dichloro-3-methyl-6-(2-p-tolylsulfanyl-benzylamino)-phenol
2,4-Dichloro-3-methyl-6-[2-(4-chloro-phenylsulfanyl)-benzylamino]-phenol

2,4-Dichloro-3-methyl-6-[2-(4-nitro-phenylsulfanyl)-benzylamino]-phenol
2,4-Dichloro-3-methyl-6-[2-(4-methoxy-phenylsulfanyl)-benzylamino]-phenol
2,4-Dichloro-3-methyl-6-[2-(2-chloro-phenylsulfanyl)-benzylamino]-phenol
2,4-Dichloro-3-methyl-6-[2-(3-chloro-phenylsulfanyl)-benzylamino]-phenol
2,4-Dichloro-3-methyl-6-[2-(3,4-dichloro-phenylsulfanyl)-benzylamino]-phenol
N-(4-{2-[(3,5-Dichloro-2-hydroxy-4-methyl-phenylamino)-methyl]-phenylsulfanyl}-phenyl)-acetamide
2,4-Dichloro-3-methyl-6-[2-(quinolin-7-ylsulfanyl)-benzylamino]-phenol
2,4-Dichloro-3-methyl-6-[2-(4-chloro-phenylsulfanyl)-5-nitro-benzylamino]-phenol
2,4-Dichloro-3-methyl-6-(5-nitro-2-p-tolylsulfanyl-benzylamino)-phenol
2-[5-Amino-2-(4-chloro-phenylsulfanyl)-benzylamino]-4,6-dichloro-5-methyl-phenol
2,4-Dichloro-3-methyl-6-[2-(4-chloro-benzenesulfonyl)-benzylamino]-phenol
4-Bromo-2-fluoro-6-(2-phenylsulfanyl-benzylamino)-phenol
4-Bromo-2-fluoro-6-(2-p-tolylsulfanyl-benzylamino)-phenol
4-Bromo-2-[2-(4-chloro-phenylsulfanyl)-benzylamino]-6-fluoro-phenol
4-Bromo-2-fluoro-6-[2-(4-nitro-phenylsulfanyl)-benzylamino]-phenol
4-Bromo-2-fluoro-6-[2-(4-methoxy-phenylsulfanyl)-benzylamino]-phenol
4-Bromo-2-[2-(2-chloro-phenylsulfanyl)-benzylamino]-6-fluoro-phenol
4-Bromo-2-[2-(3-chloro-phenylsulfanyl)-benzylamino]-6-fluoro-phenol
4-Bromo-2-[2-(3,4-dichloro-phenylsulfanyl)-benzylamino]-6-fluoro-phenol
N-(4-{2-[(5-Bromo-3-fluoro-2-hydroxy-phenylamino)-methyl]-phenylsulfanyl}-phenyl)-acetamide
4-Bromo-2-fluoro-6-[2-(quinolin-7-ylsulfanyl)-benzylamino]-phenol
4-Bromo-2-[2-(4-chloro-phenylsulfanyl)-5-nitro-benzylamino]-6-fluoro-phenol
4-Bromo-2-fluoro-6-(5-nitro-2-p-tolylsulfanyl-benzylamino)-phenol
2-[5-Amino-2-(4-chloro-phenylsulfanyl)-benzylamino]-4-bromo-6-fluoro-phenol
4-Bromo-2-[2-(4-chloro-benzenesulfonyl)-benzylamino]-6-fluoro-phenol
2,3-Difluoro-6-(2-phenylsulfanyl-benzylamino)-phenol
2,3-Difluoro-6-(2-p-tolylsulfanyl-benzylamino)-phenol
6-[2-(4-Chloro-phenylsulfanyl)-benzylamino]-2,3-difluoro-phenol
2,3-Difluoro-6-[2-(4-nitro-phenylsulfanyl)-benzylamino]-phenol
2,3 -Difluoro-6- [2-(4-methoxy-phenylsulfanyl)-benzylamino] -phenol
6-[2-(2-Chloro-phenylsulfanyl)-benzylamino]-2,3-difluoro-phenol
6-[2-(3-Chloro-phenylsulfanyl)-benzylamino]-2,3-difluoro-phenol
6-[2-(3,4-Dichloro-phenylsulfanyl)-benzylamino]-2,3-difluoro-phenol
N-(4-{2-[(3,4-Difluoro-2-hydroxy-phenylamino)-methyl]-phenylsulfanyl}-phenyl)-acetamide
2,3-Difluoro-6-[2-(quinolin-7-ylsulfanyl)-benzylamino]-phenol
6-[2-(4-Chloro-phenylsulfanyl)-5-nitro-benzylamino]-2,3-difluoro-phenol
2,3-Difluoro-6-(5-nitro-2-p-tolylsulfanyl-benzylamino)-phenol
6-[5-Amino-2-(4-chloro-phenylsulfanyl)-benzylamino]-2,3-difluoro-phenol
6-[2-(4-Chloro-benzenesulfonyl)-benzylamino]-2,3-difluoro-phenol
4-Chloro-2-(1-hydroxy-ethyl)-6-(2-phenylsulfanyl-benzylamino)-phenol
4-Chloro-2-(1-hydroxy-ethyl)-6-(2-p-tolylsulfanyl-benzylamino)-phenol
4-Chloro-2-[2-(4-chloro-phenylsulfanyl)-benzylamino]-6-(1-hydroxy-ethyl)-phenol
4-Chloro-2-(1-hydroxy-ethyl)-6-[2-(4-nitro-phenylsulfanyl)-benzylamino]-phenol
4-Chloro-2-(1-hydroxy-ethyl)-6-[2-(4-methoxy-phenylsulfanyl)-benzylamino]-phenol
4-Chloro-2-[2-(2-chloro-phenylsulfanyl)-benzylamino]-6-(1-hydroxy-ethyl)-phenol
4-Chloro-2-[2-(3-chloro-phenylsulfanyl)-benzylamino]-6-(1-hydroxy-ethyl)-phenol
4-Chloro-2-[2-(3,4-dichloro-phenylsulfanyl)-benzylamino]-6-(1-hydroxy-ethyl)-phenol
N-[4-(2-{[5-Chloro-2-hydroxy-3-(1-hydroxy-ethyl)-phenylamino]-methyl}-phenylsulfanyl)-phenyl]-acetamide
4-Chloro-2-(1-hydroxy-ethyl)-6-[2-(quinolin-7-ylsulfanyl)-benzylamino]-phenol
4-Chloro-2-[2-(4-chloro-phenylsulfanyl)-5-nitro-benzylamino]-6-(1-hydroxy-ethyl)-phenol
4-Chloro-2-(1-hydroxy-ethyl)-6-(5-nitro-2-p-tolylsulfanyl-benzylamino)-phenol
2-[5-Amino-2-(4-chloro-phenylsulfanyl)-benzylamino]-4-chloro-6-(1-hydroxy-ethyl)-phenol
4-Chloro-2-[2-(4-chloro-benzenesulfonyl)-benzylamino]-6-(1-hydroxy-ethyl)-phenol
2-Hydroxymethyl-6-(2-phenylsulfanyl-benzylamino)-phenol
2-Hydroxymethyl-6-(2-p-tolylsulfanyl-benzylamino)-phenol
2-[2-(4-Chloro-phenylsulfanyl)-benzylamino]-6-hydroxymethyl-phenol
2-Hydroxymethyl-6-[2-(4-nitro-phenylsulfanyl)-benzylamino]-phenol
2-Hydroxymethyl-6-[2-(4-methoxy-phenylsulfanyl)-benzylamino]-phenol

2-[2-(2-Chloro-phenylsulfanyl)-benzylamino]-6-hydroxymethyl-phenol
2-[2-(3-Chloro-phenylsulfanyl)-benzylamino]-6-hydroxymethyl-phenol
2-[2-(3,4-Dichloro-phenylsulfanyl)-benzylamino]-6-hydroxymethyl-phenol
N-(4-{2-[(2-Hydroxy-3-hydroxymethyl-phenylamino)-methyl]-phenylsulfanyl}-phenyl)-acetamide
2-Hydroxymethyl-6-[2-(quinolin-7-ylsulfanyl)-benzylamino]-phenol
2-[2-(4-Chloro-phenylsulfanyl)-5-nitro-benzylamino]-6-hydroxymethyl-phenol
2-Hydroxymethyl-6-(5-nitro-2-p-tolylsulfanyl-benzylamino)-phenol
2-[5-Amino-2-(4-chloro-phenylsulfanyl)-benzylamino]-6-hydroxymethyl-phenol
2-[2-(4-Chloro-benzenesulfonyl)-benzylamino]-6-hydroxymethyl-phenol
2,4-Dichloro-3-ethyl-6-(2-phenylsulfanyl-benzylamino)-phenol
2,4-Dichloro-3-ethyl-6-(2-p-tolylsulfanyl-benzylamino)-phenol
2,4-Dichloro-3-ethyl-6-[2-(4-chloro-phenylsulfanyl)-benzylamino]-phenol
2,4-Dichloro-3-ethyl-6-[2-(4-nitro-phenylsulfanyl)-benzylamino]-phenol
2,4-Dichloro-3-ethyl-6-[2-(4-methoxy-phenylsulfanyl)-benzylamino]-phenol
2,4-Dichloro-3-ethyl-6-[2-(2-chloro-phenylsulfanyl)-benzylamino]-phenol
2,4-Dichloro-3-ethyl-6-[2-(3-chloro-phenylsulfanyl)-benzylamino]-phenol
2,4-Dichloro-3-ethyl-6-[2-(3,4-dichloro-phenylsulfanyl)-benzylamino]-phenol
N-(4-{2-[(3,5-Dichloro-4-ethyl-2-hydroxy-phenylamino)-methyl]-phenylsulfanyl}-phenyl)-acetamide
2,4-Dichloro-3-ethyl-6-[2-(quinolin-7-ylsulfanyl)-benzylamino]-phenol
2,4-Dichloro-3-ethyl-6-[2-(4-chloro-phenylsulfanyl)-5-nitro-benzylamino]-phenol
2,4-Dichloro-3-ethyl-6-(5-nitro-2-p-tolylsulfanyl-benzylamino)-phenol
2-[5-Amino-2-(4-chloro-phenylsulfanyl)-benzylamino]-4,6-dichloro-5-ethyl-phenol
2,4-Dichloro-3-ethyl-6-[2-(4-chloro-benzenesulfonyl)-benzylamino]-phenol
2-Hydroxy-3-(2-phenylsulfanyl-benzylamino)-benzoic acid
2-Hydroxy-3-(2-p-tolylsulfanyl-benzylamino)-benzoic acid
3-[2-(4-Chloro-phenylsulfanyl)-benzylamino]-2-hydroxy-benzoic acid
2-Hydroxy-3-[2-(4-nitro-phenylsulfanyl)-benzylamino]-benzoic acid
2-Hydroxy-3-[2-(4-methoxy-phenylsulfanyl)-benzylamino]-benzoic acid
3-[2-(2-Chloro-phenylsulfanyl)-benzylamino]-2-hydroxy-benzoic acid
3-[2-(3-Chloro-phenylsulfanyl)-benzylamino]-2-hydroxy-benzoic acid
3-[2-(3,4-Dichloro-phenylsulfanyl)-benzylamino]-2-hydroxy-benzoic acid
3-[2-(4-Acetylamino-phenylsulfanyl)-benzylamino]-2-hydroxy-benzoic acid
2-Hydroxy-3-[2-(quinolin-7-ylsulfanyl)-benzylamino]-benzoic acid
3-[2-(4-Chloro-phenylsulfanyl)-5-nitro-benzylamino]-2-hydroxy-benzoic acid
2-Hydroxy-3-(5-nitro-2-p-tolylsulfanyl-benzylamino)-benzoic acid
2-[5-Amino-2-(4-chloro-phenylsulfanyl)-benzylamino]-6-hydroxymethyl-phenol
3-[2-(4-Chloro-benzenesulfonyl)-benzylamino]-2-hydroxy-benzoic acid
2-Fluoro-4-nitro-6-(2-phenylsulfanyl-benzylamino)-phenol
2-Fluoro-4-nitro-6-(2-p-tolylsulfanyl-benzylamino)-phenol
2-[2-(4-Chloro-phenylsulfanyl)-benzylamino]-6-fluoro-4-nitro-phenol
2-Fluoro-4-nitro-6-[2-(4-nitro-phenylsulfanyl)-benzylamino]-phenol
2-Fluoro-6-[2-(4-methoxy-phenylsulfanyl)-benzylamino]-4-nitro-phenol
2-[2-(2-Chloro-phenylsulfanyl)-benzylamino]-6-fluoro-4-nitro-phenol
2-[2-(3-Chloro-phenylsulfanyl)-benzylamino]-6-fluoro-4-nitro-phenol
2-[2-(3,4-Dichloro-phenylsulfanyl)-benzylamino]-6-fluoro-4-nitro-phenol
N-(4-{2-[(3-Fluoro-2-hydroxy-5-nitro-phenylamino)-methyl]-phenylsulfanyl}-phenyl)-acetamide
2-Fluoro-4-nitro-6-[2-(quinolin-7-ylsulfanyl)-benzylamino]-phenol
2-[2-(4-Chloro-phenylsulfanyl)-5-nitro-benzylamino]-6-fluoro-4-nitro-phenol
2-Fluoro-4-nitro-6-(5-nitro-2-p-tolylsulfanyl-benzylamino)-phenol
2-[5-Amino-2-(4-chloro-phenylsulfanyl)-benzylamino]-6-fluoro-4-nitro-phenol
2-[2-(4-Chloro-benzenesulfonyl)-benzylamino]-6-fluoro-4-nitro-phenol
2,4-Dichloro-6-(3-phenoxy-benzylamino)-phenol
2,4-Dichloro-6-[3-(4-chloro-phenoxy)-benzylamino]-phenol
2-[3-(4-tert-Butyl-phenoxy)-benzylamino]-4,6-dichloro-phenol
2-(3-Benzyloxy-benzylamino)-4,6-dichloro-phenol
2-(2-Benzyloxy-benzylamino)-4,6-dichloro-phenol
2,4-Dichloro-6-[(naphthalen-1-ylmethyl)-amino]-phenol
2,4-Dichloro-6-(4-methylsulfanyl-benzylamino)-phenol

2,4-Dichloro-6-(2-ethylsulfanyl-benzylamino)-phenol
2,4-Dichloro-6-(2-morpholin-4-yl-benzylamino)-phenol
2,4-Dichloro-6-{[2-(4-chloro-phenylsulfanyl)-thiophen-3-ylmethyl]-amino}-phenol
2,4-Dichloro-6-[(5-phenyl-2H-imidazol-4-ylmethyl)-amino]-phenol
2-[(5-Bromo-thiophen-2-ylmethyl)-amino]-4,6-dichloro-phenol
2,4-Dichloro-6-[3-(4-methoxy-phenoxy)-benzylamino]-phenol
2,4-Dichloro-6-(3-methyl-benzylamino)-phenol
2,4-Dichloro-6-(3-trifluoromethyl-benzylamino)-phenol
2,4-Dichloro-6-(2-chloro-6-fluoro-benzylamino)-phenol
2,4-Dichloro-3-methyl-6-(3-phenoxy-benzylamino)-phenol
2,4-Dichloro-3-methyl-6-[3-(4-chloro-phenoxy)-benzylamino]-phenol
2-[3-(4-tert-Butyl-phenoxy)-benzylamino]-4,6-dichloro-3-methyl-phenol
2-(3-Benzyloxy-benzylamino)-4,6-dichloro-3-methyl-phenol
2-(2-Benzyloxy-benzylamino)-4,6-dichloro-3-methyl-phenol
2,4-Dichloro-3-methyl-6-[(naphthalen-1-ylmethyl)-amino]-phenol
2,4-Dichloro-3-methyl-6-(4-methylsulfanyl-benzylamino)-phenol
2,4-Dichloro-3-methyl-6-(2-ethylsulfanyl-benzylamino)-phenol
2,4-Dichloro-3-methyl-6-(2-morpholin-4-yl-benzylamino)-phenol
2,4-Dichloro-3-methyl-6-{[2-(4-chtoro-phenylsulfanyl)-thiophen-3-ylmethyl]-amino}-phenol
2,4-Dichloro-3-methyl-6-[(5-phenyl-2H-imidazol-4-ylmethyl)-amino]-phenol
2-[(5-Bromo-thiophen-2-ylmethyl)-amino]-4,6-dichloro-3-methyl-phenol
2,4-Dichloro-6-[3-(4-methoxy-phenoxy)-benzylamino]-3-methyl-phenol
2,4-Dichloro-6-(3-methyl-benzylamino)-3-methyl-phenol
2,4-Dichloro-3-methyl-6-(3-trifluoromethyl-benzylamino)-phenol
2,4-Dichloro-3-methyl-6-(2-chloro-6-fluoro-benzylamino)-phenol
2-Chloro-6-(3-phenoxy-benzylamino)-phenol
2-Chloro-6-[3-(4-chloro-phenoxy)-benzylamino]-phenol
2-[3-(4-tert-Butyl-phenoxy)-benzylamino]-6-chloro-phenol
2-(3-Benzyloxy-benzylamino)-6-chloro-phenol
2-(2-Benzyloxy-benzylamino)-6-chloro-phenol
2-Chloro-6-[(naphthalen-1-ylmethyl)-amino]-phenol
2-Chloro-6-(4-methylsulfanyl-benzylamino)-phenol
2-Chloro-6-(2-ethylsulfanyl-benzylamino)-phenol
2-Chloro-6-(2-morpholin-4-yl-benzylamino)-phenol
2-Chloro-6-{[2-(4-chloro-phenylsulfanyl)-thiophen-3-ylmethyl]-amino}-phenol
2-Chloro-6-[(5-phenyl-2H-imidazol-4-ylmethyl)-amino]-phenol
2-[(5-Bromo-thiophen-2-ylmethyl)-amino]-6-chloro-phenol
2-Chloro-6-[3-(4-methoxy-phenoxy)-benzylamino]-phenol
2-Chloro-6-(3-methyl-benzylamino)-phenol
2-Chloro-6-(3-trifluoromethyl-benzylamino)-phenol
2-Chloro-6-(2-chloro-6-fluoro-benzylamino)-phenol
2-Fluoro-6-(3-phenoxy-benzylamino)-phenol
2-Fluoro-6-[3-(4-chloro-phenoxy)-benzylamino]-phenol
2-[3-(4-tert-Butyl-phenoxy)-benzylamino]-6-fluoro-phenol
2-(3-Benzyloxy-benzylamino)-6-fluoro-phenol
2-(2-Benzyloxy-benzylamino)-6-fluoro-phenol
2-Fluoro-6-[(naphthalen-1-ylmethyl)-amino]-phenol
2-Fluoro-6-(4-methylsulfanyl-benzylamino)-phenol
2-Fluoro-6-(2-ethylsulfanyl-benzylamino)-phenol
2-Fluoro-6-(2-morpholin-4-yl-benzylamino)-phenol
2-Fluoro-6-{[2-(4-chloro-phenylsulfanyl)-thiophen-3-ylmethyl]-amino}-phenol
2-Fluoro-6-[(5-phenyl-2H-imidazol-4-ylmethyl)-amino]-phenol
2-[(5-Bromo-thiophen-2-ylmethyl)-amino]-6-fluoro-phenol
2-Fluoro-6-[3-(4-methoxy-phenoxy)-benzylamino]-phenol
2-Fluoro-6-(3-methyl-benzylamino)-phenol
2-Fluoro-6-(3-trifluoromethyl-benzylamino)-phenol
2-Fluoro-6-(2-chloro-6-fluoro-benzylamino)-phenol
2,3-Difluoro-6-(3-phenoxy-benzylamino)-phenol

2,3-Difluoro-6-[3-(4-chloro-phenoxy)-benzylamino]-phenol
2-[3-(4-tert-Butyl-phenoxy)-benzylamino]-5,6-difluoro-phenol
2-(3-Benzyloxy-benzylamino)-5,6-difluoro-phenol
2-(2-Benzyloxy-benzylamino)-5,6-difluoro-phenol
2,3-Difluoro-6-[(naphthalen-1-ylmethyl)-amino]-phenol
2,3-Difluoro-6-(4-methylsulfanyl-benzylamino)-phenol
2,3-Difluoro-6-(2-ethylsulfanyl-benzylamino)-phenol
2,3-Difluoro-6-(2-morpholin-4-yl-benzylamino)-phenol
2,3-Difluoro-6-{[2-(4-chloro-phenylsulfanyl)-thiophen-3-ylmethyl]-amino}-phenol
2,3-Difluoro-6-[(5-phenyl-2H-imidazol-4-ylmethyl)-amino]-phenol
2-[(5-Bromo-thiophen-2-ylmethyl)-amino]-5,6-difluoro-phenol
2,3-Difluoro-6-[3-(4-methoxy-phenoxy)-benzylamino]-phenol
2,3-Difluoro-6-(3-methyl-benzylamino)-phenol
2,3-Difluoro-6-(3-trifluoromethyl-benzylamino)-phenol
2,3-Difluoro-6-(2-chloro-6-fluoro-benzylamino)-phenol
N-(3,5-Dichloro-2-hydroxy-4-methyl-phenyl)-C-phenyl-methanesulfonamide
Butane-1-sulfonic acid (3,5-dichloro-2-hydroxy-4-methyl-phenyl)-amide
Octane-1-sulfonic acid (3,5-dichloro-2-hydroxy-4-methyl-phenyl)-amide
Propane-2-sulfonic acid (3,5-dichloro-2-hydroxy-4-methyl-phenyl)-amide
N-(3,5-Dichloro-2-hydroxy-phenyl)-C-phenyl-methanesulfonamide
Butane-1-sulfonic acid (3,5-dichloro-2-hydroxy-phenyl)-amide
Octane-1-sulfonic acid (3,5-dichloro-2-hydroxy-phenyl)-amide
Propane-2-sulfonic acid (3,5-dichloro-2-hydroxy-phenyl)-amide
N-(3-Chloro-2-hydroxy-phenyl)-C-phenyl-methanesulfonamide
Butane-1-sulfonic acid (3-chloro-2-hydroxy-phenyl)-amide
Octane-1-sulfonic acid (3-chloro-2-hydroxy-phenyl)-amide
Propane-2-sulfonic acid (3-chloro-2-hydroxy-phenyl)-amide
N-(3-Fluoro-2-hydroxy-phenyl)-C-phenyl-methanesulfonamide
Butane-1-sulfonic acid (3-fluoro-2-hydroxy-phenyl)-amide
Octane-1-sulfonic acid (3-fluoro-2-hydroxy-phenyl)-amide
Propane-2-sulfonic acid (3-fluoro-2-hydroxy-phenyl)-amide
N-(3,4-Difluoro-2-hydroxy-phenyl)-C-phenyl-methanesulfonamide
Butane-1-sulfonic acid (3,4-difluoro-2-hydroxy-phenyl)-amide
Octane-1-sulfonic acid (3,4-difluoro-2-hydroxy-phenyl)-amide
Propane-2-sulfonic acid (3,4-difluoro-2-hydroxy-phenyl)-amide
(3,5-Dichloro-2-hydroxy-4-methyl-phenyl)-carbamic acid hexyl ester
(3,5-Dichloro-2-hydroxy-phenyl)-carbamic acid hexyl ester
(3-Chloro-2-hydroxy-phenyl)-carbamic acid hexyl ester
(3-Fluoro-2-hydroxy-phenyl)-carbamic acid hexyl ester
(5-Bromo-3-fluoro-2-hydroxy-phenyl)-carbamic acid hexyl ester
(3,4-Difluoro-2-hydroxy-phenyl)-carbamic acid hexyl ester
2-[3-(3,5-Dichloro-2-hydroxy-4-methyl-phenyl)-ureido]-4-methyl-pentanoic acid ethyl ester
2-[3-(3,5-Dichloro-2-hydroxy-phenyl)-ureido]-4-methyl-pentanoic acid ethyl ester
2-[3-(3-Chloro-2-hydroxy-phenyl)-ureido]-4-methyl-pentanoic acid ethyl ester
2-[3-(3-Fluoro-2-hydroxy-phenyl)-ureido]-4-methyl-pentanoic acid ethyl ester
2-[3-(3,4-Difluoro-2-hydroxy-4-phenyl)-ureido]-4-methyl-pentanoic acid ethyl ester
2-[3-(5-Bromo-3-fluoro-2-hydroxy-4-methyl-phenyl)-ureido]-4-methyl-pentanoic acid ethyl ester
2-[3-(3,5-Dichloro-2-hydroxy-4-methyl-phenyl)-ureido]-3-phenyl-propionic acid ethyl ester
2-[3-(3,5-Dichloro-2-hydroxy-phenyl)-ureido]-3-phenyl-propionic acid ethyl ester
2-[3-(3-Chloro-2-hydroxy-phenyl)-ureido]-3-phenyl-propionic acid ethyl ester
2-[3-(3-Fluoro-2-hydroxy-phenyl)-ureido]-3-phenyl-propionic acid ethyl ester
2-[3-(3,4-Difluoro-2-hydroxy-phenyl)-ureido]-3-phenyl-propionic acid ethyl ester
2-[3-(5-Bromo-3-fluoro-2-hydroxy-phenyl)-ureido]-3-phenyl-propionic acid ethyl ester
3,5,5-Trimethyl-hexanoic acid (3,5-dichloro-2-hydroxy-4-methyl-phenyl)-amide
3,5,5-Trimethyl-hexanoic acid (3,5-dichloro-2-hydroxy-phenyl)-amide
3,5,5-Trimethyl-hexanoic acid (3-chloro-2-hydroxy-phenyl)-amide
3,5,5-Trimethyl-hexanoic acid (3-fluoro-2-hydroxy-phenyl)-amide
3,5,5-Trimethyl-hexanoic acid (3,4-difluoro-2-hydroxy-phenyl)-amide

3,5,5-Trimethyl-hexanoic acid (5-bromo-3-fluoro-2-hydroxy-phenyl)-amide
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-{3-Chloro-5-[2-(4-chloro-phenylsulfanyl)-benzylamino]-4-hydroxy-phenyl}-3-cyclohexyl-urea
1-{3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
1-tert-Butyl-3-[3-chloro-2-hydroxy-5-(3-phenethyl-ureido)-phenyl]-urea
[3-Chloro-2-hydroxy-5-(3-phenethyl-ureido)-phenyl]-carbamic acid isobutyl ester
[3-Chloro-2-hydroxy-5-(3-phenethyl-ureido)-phenyl]-carbamic acid sec-butyl ester
Cyclopentanecarboxylic acid [3-chloro-2-hydroxy-5-(3-phenethyl-ureido)-phenyl]-amide
Cyclohexanecarboxylic acid [3-chloro-2-hydroxy-5-(3-phenethyl-ureido)-phenyl]-amide
1-tert-Butyl-3-{3-chloro-2-hydroxy-5-[3-(1,1,3,3-tetramethyl-butyl)-ureido]-phenyl}-urea
{3-Chloro-2-hydroxy-5-[3-(1,1,3,3-tetramethyl-butyl)-ureido]-phenyl}-carbamic acid isobutyl ester
{3-Chloro-2-hydroxy-5-[3-(1,1,3,3-tetramethyl-butyl)-ureido]-phenyl}-carbamic acid sec-butyl ester
Cyclopropanecarboxylic acid {3-chloro-2-hydroxy-5-[3-(1,1,3,3-tetramethyl-butyl)-ureido]-phenyl}-amide
Cyclobutanecarboxylic acid {3-chloro-2-hydroxy-5-[3-(1,1,3,3-tetramethyl-butyl)-ureido]-phenyl}-amide
Cyclopentanecarboxylic acid {3-chloro-2-hydroxy-5-[3-(1,1,3,3-tetramethyl-butyl)-ureido]-phenyl}-amide
Cyclohexanecarboxylic acid {3-chloro-2-hydroxy-5-[3-(1,1,3,3-tetramethyl-butyl)-ureido]-phenyl}-amide
N-[3-(3-tert-Butyl-ureido)-5-chloro-4-hydroxy-phenyl]-3-phenyl-propionamide
[3-Chloro-2-hydroxy-5-(3-phenyl-propionylamino)-phenyl]-carbamic acid isobutyl ester
[3-Chloro-2-hydroxy-5-(3-phenyl-propionylamino)-phenyl]-carbamic acid sec-butyl ester
Cyclopropanecarboxylic acid [3-chloro-2-hydroxy-5-(3-phenyl-propionylamino)-phenyl]-amide
Cyclobutanecarboxylic acid [3-chloro-2-hydroxy-5-(3-phenyl-propionylamino)-phenyl]-amide
Cyclopentanecarboxylic acid [3-chloro-2-hydroxy-5-(3-phenyl-propionylamino)-phenyl]-amide
Cyclohexanecarboxylic acid [3-chloro-2-hydroxy-5-(3-phenyl-propionylamino)-phenyl]-amide
1-Cyclopentyl-3-(3,5-dichloro-2-hydroxy-4-methyl-phenyl)-thiourea
2-[2-(4-Chloro-phenylsulfanyl)-benzylamino]-phenol
1-Benzyl-3-{3-chloro-5-[2-(4-chloro-phenylsulfanyl)-benzylamino]-4-hydroxy-phenyl}-urea
1-{3-Chloro-5-[2-(4-chloro-phenylsulfanyl)-benzylamino]-4-hydroxy-phenyl}-3-phenethyl-urea
1-{3-Chloro-5-[2-(4-chloro-phenylsulfanyl)-benzylamino]-4-hydroxy-phenyl}-3-(4-chloro-phenyl)-urea
Ethanesulfonic acid [3-chloro-5-(3-cyclohexyl-ureido)-2-hydroxy-phenyl]-amide
N-[3-Chloro-5-(3-cyclohexyl-ureido)-2-hydroxy-phenyl]-3,3-dimethyl-butyramide
1-(5-Benzothiazol-2-yl-3-chloro-2-hydroxy-phenyl)-3-tert-butyl-urea
1-(5-Benzothiazol-2-yl-3-chloro-2-hydroxy-phenyl)-3-benzyl-urea
1-(5-Benzothiazol-2-yl-3-chloro-2-hydroxy-phenyl)-3-phenethyl-urea
1-(5-Benzothiazol-2-yl-3-chloro-2-hydroxy-phenyl)-3-isopropyl-thiourea
1-(5-Benzothiazol-2-yl-3-chloro-2-hydroxy-phenyl)-3-tert-butyl-thiourea
3,5,5-Trimethyl-hexanoic acid (5-benzothiazol-2-yl-3-chloro-2-hydroxy-phenyl)-amide
N-(5-Benzothiazol-2-yl-3-chloro-2-hydroxy-phenyl)-3-phenyl-propionamide
1-(2-Hydroxy-4-methyl-phenyl)-3-pentyl-urea
Biphenyl-4-carboxylic acid (3,5-dichloro-2-hydroxy-4-methyl-phenyl)-amide
Biphenyl-4-carboxylic acid (3,5-dichloro-2-hydroxy-phenyl)-amide
2,4-Dichloro-6-[(furan-2-ylmethyl)-amino]-3-methyl-phenol
2,4-Dichloro-6-[(furan-2-ylmethyl)-amino]-phenol
2,3-Difluoro-6-[(furan-2-ylmethyl)-amino]-phenol
2,4-Dichloro-3-methyl-6-(2-trifluoromethyl-benzylamino)-phenol
2,4-Dichloro-6-(2-trifluoromethyl-benzylamino)-phenol
2,3-Difluoro-6-(2-trifluoromethyl-benzylamino)-phenol
1-{3-Chloro-5-[2-(4-chloro-phenylsulfanyl)-benzylamino]-4-hydroxy-phenyl}-3-(2,6-dichloro-pyridin-4-yl)-urea
1-(5-Benzothiazol-2-yl-3-chloro-2-hydroxy-phenyl)-3-cyclopentyl-thiourea
1-{3-Chloro-5-[2-(4-chloro-phenylsulfanyl)-benzylamino]-4-hydroxy-phenyl}-3-morpholin-4-yl-urea
6-Benzylamino-2,4-dichloro-3-methyl-phenol
1-[2-(1H-Benzoimidazol-2-yl)-ethyl]-3-(3,5-dichloro-2-hydroxy-4-methyl-phenyl)-urea
1-(5-Benzothiazol-2-yl-3-chloro-2-hydroxy-phenyl)-3-cyclopentyl-thiourea
1-[5-Chloro-2-hydroxy-3-(2-phenylsulfanyl-benzylamino)-phenyl]-ethanone
1-[5-Chloro-2-hydroxy-3-(2-p-tolylsulfanyl-benzylamino)-phenyl]-ethanone
1-{5-Chloro-3-[2-(4-chloro-phenylsulfanyl)-benzylamino]-2-hydroxy-phenyl}-ethanone
1-{5-Chloro-2-hydroxy-3-[2-(4-nitro-phenylsulfanyl)-benzylamino]-phenyl}-ethanone

1-{5-Chloro-2-hydroxy-3-[2-(4-methoxy-phenylsulfanyl)-benzylamino]-phenyl}-ethanone
1-{5-Chloro-3-[2-(2-chloro-phenylsulfanyl)-benzylamino]-2-hydroxy-phenyl}-ethanone
1-{5-Chloro-3-[2-(3-chloro-phenylsulfanyl)-benzylamino]-2-hydroxy-phenyl}-ethanone
1-{5-Chloro-3-[2-(3,4-dichloro-phenylsulfanyl)-benzylamino]-2-hydroxy-phenyl}-ethanone
N-(4-{2-[(3-Acetyl-5-chloro-2-hydroxy-phenylamino)-methyl]-phenylsulfanyl}-phenyl)-acetamide
1-{5-Chloro-2-hydroxy-3-[2-(quinolin-7-ylsulfanyl)-benzylamino]-phenyl}-ethanone
1-{5-Chloro-3-[2-(4-chloro-phenylsulfanyl)-5-nitro-benzylamino]-2-hydroxy-phenyl}-ethanone
1-[5-Chloro-2-hydroxy-3-(5-nitro-2-p-tolylsulfanyl-benzylamino)-phenyl]-ethanone
1-{3-[5-Amino-2-(4-chloro-phenylsulfanyl)-benzylamino]-5-chloro-2-hydroxy-phenyl}-ethanone
1-{5-Chloro-3-[2-(4-chloro-benzenesulfonyl)-benzylamino]-2-hydroxy-phenyl}-ethanone
4-[2-(4-Chloro-phenylsulfanyl)-benzylamino]-benzene-1,3-diol
1,6-Di-(3,5-Dichloro-2-hydroxy-4-methyl-phenyl)-3-hexyl-urea
3-[3-(3-Chloro-4-hydroxy-phenyl)-ureido]-propionic acid ethyl ester
1-(3-Chloro-4-hydroxy-phenyl)-3-pentyl-urea
1-Benzyl-3-(3-chloro-4-hydroxy-phenyl)-urea
1-(3-Chloro-4-hydroxy-phenyl)-3-(2-methyl-benzyl)-urea
1-(3-Chloro-4-hydroxy-phenyl)-3-phenethyl-urea
1-(3-Chloro-4-hydroxy-phenyl)-3-(1,1,3,3-tetramethyl-butyl)-urea
1-tert-Butyl-3-(3-chloro-4-hydroxy-phenyl)-urea
1-(3-Chloro-4-hydroxy-phenyl)-3-cyclohexylmethyl-urea
1-(3-Chloro-4-hydroxy-phenyl)-3-(4-trifluoromethyl-benzyl)-urea
1-(3-Chloro-3-hydroxy-phenyl)-3-(3,5-dichloro-phenyl)-urea
1-(3-Chloro-4-hydroxy-phenyl)-3-(4-chloro-phenyl)-urea
1-(3-Chloro-4-hydroxy-phenyl)-3-(4-trifluoromethyl-phenyl)-urea
1-(3-Chloro-4-hydroxy-phenyl)-3-cyclohexyl-urea
1-(3-Chloro-4-hydroxy-phenyl)-3-(4-trifluoromethoxy-phenyl)-urea
1-(3-Chloro-4-hydroxy-phenyl)-3-(4-cyano-phenyl)-urea
1-Benzo[1,3]dioxol-5-yl-3-(3-chloro-4-hydroxy-phenyl)-urea
1-(3-Chloro-4-hydroxy-phenyl)-3-o-tolyl-urea
1-(3-Chloro-4-hydroxy-phenyl)-3-(3-methoxy-phenyl)-urea
1-(3-Chloro-4-hydroxy-phenyl)-3-(2,6-dimethyl-phenyl)-urea
1-(3-Chloro-4-hydroxy-phenyl)-3-(3,4,5-trimethoxy-phenyl)-urea
1-(3-Chloro-4-hydroxy-phenyl)-3-naphthalen-1-yl-urea
1-Adamantan-1-yl-3-(3-chloro-4-hydroxy-phenyl)-urea
1-(3-Chloro-4-hydroxy-phenyl)-3-(4-phenoxy-phenyl)-urea
1-(3-Chloro-4-hydroxy-phenyl)-3-phenyl-urea
3-[3-(3,5-Dichloro-4-hydroxy-phenyl)-ureido]-propionic acid ethyl ester
1-(3,5-Dichloro-4-hydroxy-phenyl)-3-pentyl-urea
1-Benzyl-3-(3,5-dichloro-4-hydroxy-phenyl)-urea
1-(3,5-Dichloro-4-hydroxy-phenyl)-3-(2-methyl-benzyl)-urea
1-(3,5-Dichloro-4-hydroxy-phenyl)-3-phenethyl-urea
1-(3,5-Dichloro-4-hydroxy-phenyl)-3-(1,1,3,3-tetramethyl-butyl)-urea
1-tert-Butyl-3-(3,5-dichloro-4-hydroxy-phenyl)-urea
1-(3,5-Dichloro-4-hydroxy-phenyl)-3-cyclohexylmethyl-urea
1-(3,5-Dichloro-4-hydroxy-phenyl)-3-(4-trifluoromethyl-benzyl)-urea
1-(3,5-Dichloro-4-hydroxy-phenyl)-3-(3,5-dichloro-phenyl)-urea
1-(3,5-Dichloro-4-hydroxy-phenyl)-3-(4-chloro-phenyl)-urea
1-(3,5-Dichloro-4-hydroxy-phenyl)-3-(4-trifluoromethyl-phenyl)-urea
1-(3,5-Dichloro-4-hydroxy-phenyl)-3-cyclohexyl-urea
1-(3,5-Dichloro-4-hydroxy-phenyl)-3-(4-trifluoromethoxy-phenyl)-urea
1-(3,5-Dichloro-4-hydroxy-phenyl)-3-(4-cyano-phenyl)-urea
1-Benzo[1,3]dioxol-5-yl-3-(3,5-dichloro-4-hydroxy-phenyl)-urea
1-(3,5-Dichloro-4-hydroxy-phenyl)-3-o-tolyl-urea
1-(3,5-Dichloro-4-hydroxy-phenyl)-3-(3-methoxy-phenyl)-urea
1-(3,5-Dichloro-4-hydroxy-phenyl)-3-(2,6-dimethyl-phenyl)-urea
1-(3,5-Dichloro-4-hydroxy-phenyl)-3-(3,4,5-trimethoxy-phenyl)-urea
1-(3,5-Dichloro-4-hydroxy-phenyl)-3-naphthalen-1-yl-urea
1-Adamantan-1-yl-3-(3,5-dichloro-4-hydroxy-phenyl)-urea

1-(3,5-Dichloro-4-hydroxy-phenyl)-3-(4-phenoxy-phenyl)-urea
1-(3,5-Dichloro-4-hydroxy-phenyl)-3-phenyl-urea
3-[3-(4-Hydroxy-3-nitro-phenyl)-ureido]-propionic acid ethyl ester
1-(4-Hydroxy-3-nitro-phenyl)-3-pentyl-urea
1-Benzyl-3-(4-hydroxy-3-nitro-phenyl)-urea
1-(4-Hydroxy-3-nitro-phenyl)-3-(2-methyl-benzyl)-urea
1-(4-Hydroxy-3-nitro-phenyl)-3-phenethyl-urea
1-(4-Hydroxy-3-nitro-phenyl)-3-(1,1,3,3-tetramethyl-butyl)-urea
1-tert-Butyl-3-(4-hydroxy-3-nitro-phenyl)-urea
1-Cyclohexylmethyl-3-(4-hydroxy-3-nitro-phenyl)-urea
1-(4-Hydroxy-3-nitro-phenyl)-3-(4-trifluoromethyl-benzyl)-urea
1-(3,5-Dichloro-phenyl)-3-(4-hydroxy-3-nitro-phenyl)-urea
1-(4-Chloro-phenyl)-3-(4-hydroxy-3-nitro-phenyl)-urea
1-(4-Hydroxy-3-nitro-phenyl)-3-(4-trifluoromethyl-phenyl)-urea
1-Cyclohexyl-3-(4-hydroxy-3-nitro-phenyl)-urea
1-(4-Hydroxy-3-nitro-phenyl)-3-(4-trifluoromethoxy-phenyl)-urea
1-(4-Cyano-phenyl)-3-(4-hydroxy-3-nitro-phenyl)-urea
1-Benzo[1,3]dioxol-5-yl-3-(4-hydroxy-3-nitro-phenyl)-urea
1-(4-Hydroxy-3-nitro-phenyl)-3-o-tolyl-urea
1-(4-Hydroxy-3-nitro-phenyl)-3-(3-methoxy-phenyl)-urea
1-(2,6-Dimethyl-phenyl)-3-(4-hydroxy-3-nitro-phenyl)-urea
1-(4-Hydroxy-3-nitro-phenyl)-3-(3,4,5-trimethoxy-phenyl)-urea
1-(4-Hydroxy-3-nitro-phenyl)-3-naphthalen-1-yl-urea
1-Adamantan-1-yl-3-(4-hydroxy-3-nitro-phenyl)-urea
1-(4-Hydroxy-3-nitro-phenyl)-3-(4-phenoxy-phenyl)-urea
1-(4-Hydroxy-3-nitro-phenyl)-3-phenyl-urea
3-[3-(3-Fluoro-4-hydroxy-phenyl)-ureido]-propionic acid ethyl ester
1-(3-Fluoro-4-hydroxy-phenyl)-3-pentyl-urea
1-Benzyl-3-(3-fluoro-4-hydroxy-phenyl)-urea
1-(3-Fluoro-4-hydroxy-phenyl)-3-(2-methyl-benzyl)-urea
1-(3-Fluoro-4-hydroxy-phenyl)-3-phenethyl-urea
1-(3-Fluoro-4-hydroxy-phenyl)-3-(1,1,3,3-tetramethyl-butyl)-urea
1-tert-Butyl-3-(3-fluoro-4-hydroxy-phenyl)-urea
1-(3-Fluoro-4-hydroxy-phenyl)-3-cyclohexylmethyl-urea
1-(3-Fluoro-4-hydroxy-phenyl)-3-(4-trifluoromethyl-benzyl)-urea
1-(3-Fluoro-4-hydroxy-phenyl)-3-(3,5-dichloro-phenyl)-urea
1-(3-Fluoro-4-hydroxy-phenyl)-3-(4-chloro-phenyl)-urea
1-(3-Fluoro-2-hydroxy-phenyl)-3-(4-trifluoromethyl-phenyl)-urea
1-(3-Fluoro-4-hydroxy-phenyl)-3-cyclohexyl-urea
1-(3-Fluoro-4-hydroxy-phenyl)-3-(4-trifluoromethoxy-phenyl)-urea
1-(3-Fluoro-4-hydroxy-phenyl)-3-(4-cyano-phenyl)-urea
1-Benzo[1,3]dioxol-5-yl-3-(3-fluoro-4-hydroxy-phenyl)-urea
1-(3-Fluoro-4-hydroxy-phenyl)-3-o-tolyl-urea
1-(3-Fluoro-4-hydroxy-phenyl)-3-(3-methoxy-phenyl)-urea
1-(3-Fluoro-4-hydroxy-phenyl)-3-(2,6-dimethyl-phenyl)-urea
1-(3-Fluoro-4-hydroxy-phenyl)-3-(3,4,5-trimethoxy-phenyl)-urea
1-(3-Fluoro-4-hydroxy-phenyl)-3-naphthalen-1-yl-urea
1-Adamantan-1-yl-3-(3-fluoro-4-hydroxy-phenyl)-urea
1-(3-Fluoro-4-hydroxy-phenyl)-3-(4-phenoxy-phenyl)-urea
1-(3-Fluoro-4-hydroxy-phenyl)-3-phenyl-urea
3-[3-(2,4-Dihydroxy-phenyl)-ureido]-propionic acid ethyl ester
1-(2,4-Dihydroxy-phenyl)-3-pentyl-urea
1-Benzyl-3-(2,4-dihydroxy-phenyl)-urea
1-(2,4-Dihydroxy-phenyl)-3-(2-methyl-benzyl)-urea
1-(2,4-Dihydroxy-phenyl)-3-phenethyl-urea
1-(2,4-Dihydroxy-phenyl)-3-(1,1,3,3-tetramethyl-butyl)-urea
1-tert-Butyl-3-(2,4-dihydroxy-phenyl)-urea
1-Cyclohexylmethyl-3-(2,4-dihydroxy-phenyl)-urea

1-(2,4-Dihydroxy-phenyl)-3-(4-trifluoromethyl-benzyl)-urea
1-(3,5-Dichloro-phenyl)-3-(2,4-dihydroxy-phenyl)-urea
1-(4-Chloro-phenyl)-3-(2,4-dihydroxy-phenyl)-urea
1-(2,4-Dihydroxy-phenyl)-3-(4-trifluoromethyl-phenyl)-urea
1-Cyclohexyl-3-(2,4-dihydroxy-phenyl)-urea
1-(2,4-Dihydroxy-phenyl)-3-(4-trifluoromethoxy-phenyl)-urea
1-(4-Cyano-phenyl)-3-(2,4-dihydroxy-phenyl)-urea
1-Benzo[1,3]dioxol-5-yl-3-(2,4-dihydroxy-phenyl)-urea
1-(2,4-Dihydroxy-phenyl)-3-o-tolyl-urea
1-(2,4-Dihydroxy-phenyl)-3-(3-methoxy-phenyl)-urea
1-(2,4-Dihydroxy-phenyl)-3-(2,6-dimethyl-phenyl)-urea
1-(2,4-Dihydroxy-phenyl)-3-(3,4,5-trimethoxy-phenyl)-urea
1-(2,4-Dihydroxy-phenyl)-3-naphthalen-1-yl-urea
1-Adamantan-1-yl-3-(2,4-dihydroxy-phenyl)-urea
1-(2,4-Dihydroxy-phenyl)-3-(4-phenoxy-phenyl)-urea
1-(2,4-Dihydroxy-phenyl)-3-phenyl-urea
3-[3-(3,5-Dibromo-4-hydroxy-phenyl)-ureido]-propionic acid ethyl ester
1-(3,5-Dibromo-4-hydroxy-phenyl)-3-pentyl-urea
1-Benzyl-3-(3,5-Dibromo-4-hydroxy-phenyl)-urea
1-(5-Bromo-3-fluoro-2-hydroxy-phenyl)-3-(2-methyl-benzyl)-urea
1-(3,5-Dibromo-4-hydroxy-phenyl)-3-phenethyl-urea
1-(3,5-Dibromo-4-hydroxy-phenyl)-3-(1,1,3,3-tetramethyl-butyl)-urea
1-tert-Butyl-3-(3,5-dibromo-4-hydroxy-phenyl)-urea
1-(3,5-Dibromo-4-hydroxy-phenyl)-3-cyclohexylmethyl-urea
1-(3,5-Dibromo-4-hydroxy-phenyl)-3-(4-trifluoromethyl-benzyl)-urea
1-(3,5-Dibromo-4-hydroxy-phenyl)-3-(3,5-dichloro-phenyl)-urea
1-(3,5-Dibromo-4-hydroxy-phenyl)-3-(4-chloro-phenyl)-urea
1-(3,5-Dibromo-4-hydroxy-phenyl)-3-(4-trifluoromethyl-phenyl)-urea
1-(3,5-Dibromo-4-hydroxy-phenyl)-3-cyclohexyl-urea
1-(3,5-Dibromo-4-hydroxy-phenyl)-3-(4-trifluoromethoxy-phenyl)-urea
1-(3,5-Dibromo-4-hydroxy-phenyl)-3-(4-cyano-phenyl)-urea
1-Benzo[1,3]dioxol-5-yl-3-(3,5-dibromo-4-hydroxy-phenyl)-urea
1-(3,5-Dibromo-4-hydroxy-phenyl)-3-o-tolyl-urea
1-(3,5-Dibromo-4-hydroxy-phenyl)-3-(3-methoxy-phenyl)-urea
1-(3,5-Dibromo-4-hydroxy-phenyl)-3-(2,6-dimethyl-phenyl)-urea
1-(3,5-Dibromo-4-hydroxy-phenyl)-3-(3,4,5-trimethoxy-phenyl)-urea
1-(3,5-Dibromo-4-hydroxy-phenyl)-3-naphthalen-1-yl-urea
1-Adamantan-1-yl-3-(3,5-dibromo-4-hydroxy-phenyl)-urea
1-(3,5-Dibromo-4-hydroxy-phenyl)-3-(4-phenoxy-phenyl)-urea
1-(3,5-Dibromo-4-hydroxy-phenyl)-3-phenyl-urea
3-[3-(3,5-Difluoro-4-hydroxy-phenyl)-ureido]-propionic acid ethyl ester
1-(3,5-Difluoro-4-hydroxy-phenyl)-3-pentyl-urea
1-Benzyl-3-(3,5-difluoro-4-hydroxy-phenyl)-urea
1-(3,5-Difluoro-4-hydroxy-phenyl)-3-(2-methyl-benzyl)-urea
1-(3,5-Difluoro-4-hydroxy-phenyl)-3-phenethyl-urea
1-(3,5-Difluoro-4-hydroxy-phenyl)-3-(1,1,3,3-tetramethyl-butyl)-urea
1-tert-Butyl-3-(3,5-difluoro-4-hydroxy-phenyl)-urea
1-(3,5-Difluoro-4-hydroxy-phenyl)-3-cyclohexylmethyl-urea
1-(3,5-Difluoro-4-hydroxy-phenyl)-3-(4-trifluoromethyl-benzyl)-urea
1-(3,5-Difluoro-4-hydroxy-phenyl)-3-(3,5-dichloro-phenyl)-urea
1-(3,5-Difluoro-4-hydroxy-phenyl)-3-(4-chloro-phenyl)-urea
1-(3,5-Difluoro-4-hydroxy-phenyl)-3-(4-trifluoromethyl-phenyl)-urea
1-(3,5-Difluoro-4-hydroxy-phenyl)-3-cyclohexyl-urea
1-(3,5-Difluoro-4-hydroxy-phenyl)-3-(4-trifluoromethoxy-phenyl)-urea
1-(3,5-Difluoro-4-hydroxy-phenyl)-3-(4-cyano-phenyl)-urea
1-Benzo[1,3]dioxol-5-yl-3-(3,5-difluoro-4-hydroxy-phenyl)-urea
1-(3,5-Difluoro-4-hydroxy-phenyl)-3-o-tolyl-urea
1-(3,5-Difluoro-4-hydroxy-phenyl)-3-(3-methoxy-phenyl)-urea

1-(3,5-Difluoro-4-hydroxy-phenyl)-3-(2,6-dimethyl-phenyl)-urea
1-(3,5-Difluoro-4-hydroxy-phenyl)-3-(3,4,5-trimethoxy-phenyl)-urea
1-(3,5-Difluoro-4-hydroxy-phenyl)-3-naphthalen-1-yl-urea
1-Adamantan-1-yl-3-(3,5-difluoro-4-hydroxy-phenyl)-urea
1-(3,5-Difluoro-4-hydroxy-phenyl)-3-(4-phenoxy-phenyl)-urea
1-(3,5-Difluoro-4-hydroxy-phenyl)-3-phenyl-urea
3-{3-[3-Chloro-4-hydroxy-5-(1-hydroxy-ethyl)-phenyl]-ureido}-propionic acid ethyl ester
1-[3-Chloro-4-hydroxy-5-(1-hydroxy-ethyl)-phenyl]-3-pentyl-urea
1-[3-Chloro-4-hydroxy-5-(1-hydroxy-ethyl)-phenyl]-3-pentyl-urea
1-[3-Chloro-4-hydroxy-5-(1-hydroxy-ethyl)-phenyl]-3-(2-methyl-benzyl)-urea
1-[3-Chloro-4-hydroxy-5-(1-hydroxy-ethyl)-phenyl]-3-phenethyl-urea
1-[3-Chloro-4-hydroxy-5-(1-hydroxy-ethyl)-phenyl]-3-(1,1,3,3-tetramethyl-butyl)-urea
1-tert-Butyl-3-[3-chloro-4-hydroxy-5-(1-hydroxy-ethyl)-phenyl]-urea
1-[3-Chloro-4-hydroxy-5-(1-hydroxy-ethyl)-phenyl]-3-cyclohexylmethyl-urea
1-[3-Chloro-4-hydroxy-5-(1-hydroxy-ethyl)-phenyl]-3-(4-trifluoromethyl-benzyl)-urea
1-[3-Chloro-4-hydroxy-5-(1-hydroxy-ethyl)-phenyl]-3-(4-chloro-phenyl)-urea
1-[3-Chloro-4-hydroxy-5-(1-hydroxy-ethyl)-phenyl]-3-(4-chloro-phenyl)-urea
1-[3-Chloro-4-hydroxy-5-(1-hydroxy-ethyl)-phenyl]-3-(4-trifluoromethyl-phenyl)-urea
1-[3-Chloro-4-hydroxy-5-(1-hydroxy-ethyl)-phenyl]-3-cyclohexyl-urea
1-[3-Chloro-4-hydroxy-5-(1-hydroxy-ethyl)-phenyl]-3-(4-trifluoromethoxy-phenyl)-urea
1-[3-Chloro-4-hydroxy-5-(1-hydroxy-ethyl)-phenyl]-3-(4-cyano-phenyl)-urea
1-Benzo[1,3]dioxol-5-yl-3-[3-chloro-4-hydroxy-5-(1-hydroxy-ethyl)-phenyl]-urea
1-[3-Chloro-4-hydroxy-5-(1-hydroxy-ethyl)-phenyl]-3-o-tolyl-urea
1-[3-Chloro-4-hydroxy-5-(1-hydroxy-ethyl)-phenyl]-3-(3-methoxy-phenyl)-urea
1 - [3 -Chloro-4-hydroxy-5-(1-hydroxy-ethyl)-phenyl]-3-(2,6-dimethyl-phenyl)-urea
1-[3-Chloro-4-hydroxy-5-(1-hydroxy-ethyl)-phenyl]-3-(3,4,5-trimethoxy-phenyl)-urea
1-[3-Chloro-4-hydroxy-5-(1-hydroxy-ethyl)-phenyl]-3-naphthalen-1-yl-urea
1-Adamantan-1-yl-3-[3-chloro-4-hydroxy-5-(1-hydroxy-ethyl)-phenyl]-urea
1-[3-Chloro-4-hydroxy-5-(1-hydroxy-ethyl)-phenyl]-3-(4-phenoxy-phenyl)-urea
1-[3-Chloro-4-hydroxy-5-(1-hydroxy-ethyl)-phenyl]-3-phenyl-urea
1-(3-Chloro-4-hydroxy-phenyl)-3-pentyl-thiourea
1-Benzyl-3-(3-chloro-4-hydroxy-phenyl)-thiourea
1-(3-Chloro-4-hydroxy-phenyl)-3-(2-methyl-benzyl)-thiourea
1-(3-Chloro-4-hydroxy-phenyl)-3-phenethyl-thiourea
1-(3-Chloro-4-hydroxy-phenyl)-3-(1,1,3,3-tetramethyl-butyl)-thiourea
1-tert-Butyl-3-(3-chloro-4-hydroxy-phenyl)-thiourea
1-(5-Chloro-2-hydroxy-phenyl)-3-isopropyl-thiourea
1-(3-Chloro-4-hydroxy-phenyl)-3-cyclohexylmethyl-thiourea
1-(3-Chloro-4-hydroxy-phenyl)-3-(4-trifluoromethyl-benzyl)-thiourea
1-(3-Chloro-4-hydroxy-phenyl)-3-(3,5-dichloro-phenyl)-thiourea
1-(3-Chloro-4-hydroxy-phenyl)-3-(4-chloro-phenyl)-thiourea
1-(3-Chloro-4-hydroxy-phenyl)-3-(4-trifluoromethyl-phenyl)-thiourea
1-(3-Chloro-4-hydroxy-phenyl)-3-cyclohexyl-thiourea
1-(3-Chloro-4-hydroxy-phenyl)-3-(2-trifluoromethyl-phenyl)-thiourea
1-(3-Chloro-4-hydroxy-phenyl)-3-phenyl-thiourea
1-(3,5-Dichloro-4-hydroxy-phenyl)-3-pentyl-thiourea
1-Benzyl-3-(3,5-dichloro-4-hydroxy-phenyl)-thiourea
1-(3,5-Dichloro-4-hydroxy-phenyl)-3-(2-methyl-benzyl)-thiourea
1-(3,5-Dichloro-4-hydroxy-phenyl)-3-phenethyl-thiourea
1-(3,5-Dichloro-4-hydroxy-phenyl)-3-(1,1,3,3-tetramethyl-butyl)-thiourea
1-tert-Butyl-3-(3,5-dichloro-4-hydroxy-phenyl)-thiourea
1-(5-Chloro-4-hydroxy-phenyl)-3-isopropyl-thiourea
1-(3,5-Dichloro-4-hydroxy-phenyl)-3-cyclohexylmethyl-thiourea
1-(3,5-Dichloro-4-hydroxy-phenyl)-3-(4-trifluoromethyl-benzyl)-thiourea
1-(3,5-Dichloro-4-hydroxy-phenyl)-3-(3,5-dichloro-phenyl)-thiourea
1-(3,5-Dichloro-4-hydroxy-phenyl)-3-(4-chloro-phenyl)-thiourea
1-(3,5-Dichloro-4-hydroxy-phenyl)-3-(4-trifluoromethyl-phenyl)-thiourea
1-(3,5-Dichloro-4-hydroxy-phenyl)-3-cyclohexyl-thiourea

1-(3,5-Dichloro-4-hydroxy-phenyl)-3-(2-trifluoromethyl-phenyl)-thiourea
1-(3,5-Dichloro-4-hydroxy-phenyl)-3-phenyl-thiourea
1-(4-Hydroxy-3-nitro-phenyl)-3-pentyl-thiourea
1-Benzyl-3-(4-hydroxy-3-nitro-phenyl)-thiourea
1-(4-Hydroxy-3-nitro-phenyl)-3-(2-methyl-benzyl)-thiourea
1-(4-Hydroxy-3-nitro-phenyl)-3-phenethyl-thiourea
1-(4-Hydroxy-3-nitro-phenyl)-3-(1,1,3,3-tetramethyl-butyl)-thiourea
1-tert-Butyl-3-(4-hydroxy-3-nitro-phenyl)-thiourea
1-(4-Hydroxy-3-nitro-phenyl)-3-isopropyl-thiourea
1-Cyclohexylmethyl-3-(4-hydroxy-3-nitro-phenyl)-thiourea
1-(4-Hydroxy-3-nitro-phenyl)-3-(4-trifluoromethyl-benzyl)-thiourea
1-(3,5-Dichloro-phenyl)-3-(4-hydroxy-3-nitro-phenyl)-thiourea
1-(4-Chloro-phenyl)-3-(4-hydroxy-3-nitro-phenyl)-thiourea
1-(4-Hydroxy-3-nitro-phenyl)-3-(4-trifluoromethyl-phenyl)-thiourea
1-Cyclohexyl-3-(4-hydroxy-3-nitro-phenyl)-thiourea
1-(4-Hydroxy-3-nitro-phenyl)-3-(2-trifluoromethyl-phenyl)-thiourea
1-(4-Hydroxy-3-nitro-phenyl)-3-phenyl-thiourea
1-(3-Fluoro-4-hydroxy-phenyl)-3-pentyl-thiourea
1-Benzyl-3-(3-fluoro-4-hydroxy-phenyl)-thiourea
1-(3-Fluoro-4-hydroxy-phenyl)-3-(2-methyl-benzyl)-thiourea
1-(3-Fluoro-4-hydroxy-phenyl)-3-phenethyl-thiourea
1-(3-Fluoro-4-hydroxy-phenyl)-3-(1,1,3,3-tetramethyl-butyl)-thiourea
1-tert-Butyl-3-(3-fluoro-4-hydroxy-phenyl)-thiourea
1-(3-Fluoro-4-hydroxy-phenyl)-3-isopropyl-thiourea
1-(3-Fluoro-4-hydroxy-phenyl)-3-cyclohexylmethyl-thiourea
1-(3-Fluoro-4-hydroxy-phenyl)-3-(4-trifluoromethyl-benzyl)-thiourea
1-(3-Fluoro-4-hydroxy-phenyl)-3-(3,5-dichloro-phenyl)-thiourea
1-(3-Fluoro-4-hydroxy-phenyl)-3-(4-chloro-phenyl)-thiourea
1-(3-Fluoro-4-hydroxy-phenyl)-3-(4-trifluoromethyl-phenyl)-thiourea
1-(3-Fluoro-4-hydroxy-phenyl)-3-cyclohexyl-thiourea
1-(3-Fluoro-4-hydroxy-phenyl)-3-(2-trifluoromethyl-phenyl)-thiourea
1-(3-Fluoro-4-hydroxy-phenyl)-3-phenyl-thiourea
1-(2,4-Dihydroxy-phenyl)-3-pentyl-thiourea
1-Benzyl-3-(2,4-dihydroxy-phenyl)-thiourea
1-(2,4-Dihydroxy-phenyl)-3-(2-methyl-benzyl)-thiourea
1-(2,4-Dihydroxy-phenyl)-3-phenethyl-thiourea
1-(2,4-Dihydroxy-phenyl)-3-(1,1,3,3-tetramethyl-butyl)-thiourea
1-tert-Butyl-3-(2,4-dihydroxy-phenyl)-thiourea
1-(2,4-Dihydroxy-phenyl)-3-isopropyl-thiourea
1-Cyclohexylmethyl-3-(2,4-dihydroxy-phenyl)-thiourea
1-(2,4-Dihydroxy-phenyl)-3-(4-trifluoromethyl-benzyl)-thiourea
1-(3,5-Dichloro-phenyl)-3-(2,4-dihydroxy-phenyl)-thiourea
1-(4-Chloro-phenyl)-3-(2,4-dihydroxy-phenyl)-thiourea
1-(2,4-Dihydroxy-phenyl)-3-(4-trifluoromethyl-phenyl)-thiourea
1-Cyclohexyl-3-(2,4-dihydroxy-phenyl)-thiourea
1-(2,4-Dihydroxy-phenyl)-3-(2-trifluoromethyl-phenyl)-thiourea
1-(2,4-Dihydroxy-phenyl)-3-phenyl-thiourea
1-(3,5-Dibromo-4-hydroxy-phenyl)-3-pentyl-thiourea
1-Benzyl-3-(3,5-dibromo-4-hydroxy-phenyl)-thiourea
1-(3,5-Dibromo-4-hydroxy-phenyl)-3-(2-methyl-benzyl)-thiourea
1-(3,5-Dibromo-4-hydroxy-phenyl)-3-phenethyl-thiourea
1-(3,5-Dibromo-4-hydroxy-phenyl)-3-(1,1,3,3-tetramethyl-butyl)-thiourea
1-tert-Butyl-3-(3,5-dibromo-4-hydroxy-phenyl)-thiourea
1-(3,5-Dibromo-4-hydroxy-phenyl)-3-isopropyl-thiourea
1-(3,5-Dibromo-4-hydroxy-phenyl)-3-cyclohexylmethyl-thiourea
1-(3,5-Dibromo-4-hydroxy-phenyl)-3-(4-trifluoromethyl-benzyl)-thiourea
1-(3,5-Dibromo-4-hydroxy-phenyl)-3-(3,5-dichloro-phenyl)-thiourea
1-(3,5-Dibromo-4-hydroxy-phenyl)-3-(4-chloro-phenyl)-thiourea

1-(3,5-Dibromo-4-hydroxy-phenyl)-3-(4-trifluoromethyl-phenyl)-thiourea
1-(3,5-Dibromo-4-hydroxy-phenyl)-3-cyclohexyl-thiourea
1-(3,5-Dibromo-4-hydroxy-phenyl)-3-(2-trifluoromethyl-phenyl)-thiourea
1-(3,5-Dibromo-4-hydroxy-phenyl)-3-phenyl-thiourea
1-(3,5-Difluoro-4-hydroxy-phenyl)-3-pentyl-thiourea
1-Benzyl-3-(3,5-difluoro-4-hydroxy-phenyl)-thiourea
1-(3,5-Difluoro-4-hydroxy-phenyl)-3-(2-methyl-benzyl)-thiourea
1-(3,5-Difluoro-4-hydroxy-phenyl)-3-phenethyl-thiourea
1-(3,5-Difluoro-4-hydroxy-phenyl)-3-(1,1,3,3-tetramethyl-butyl)-thiourea
1-tert-Butyl-3-(3,5-Difluoro-4-hydroxy-phenyl)-thiourea
1-(3,5-Difluoro-4-hydroxy-phenyl)-3-isopropyl-thiourea
1-(3,5-Difluoro-4-hydroxy-phenyl)-3-cyclohexylmethyl-thiourea
1-(3,5-Difluoro-4-hydroxy-phenyl)-3-(4-trifluoromethyl-benzyl)-thiourea
1-(3,5-Difluoro-4-hydroxy-phenyl)-3-(3,5-dichloro-phenyl)-thiourea
1-(3,5-Difluoro-4-hydroxy-phenyl)-3-(4-chloro-phenyl)-thiourea
1-(3,5-Difluoro-4-hydroxy-phenyl)-3-(4-trifluoromethyl-phenyl)-thiourea
1-(3,5-Difluoro-4-hydroxy-phenyl)-3-cyclohexyl-thiourea
1-(3,5-Difluoro-4-hydroxy-phenyl)-3-(2-trifluoromethyl-phenyl)-thiourea
1-(3,5-Difluoro-4-hydroxy-phenyl)-3-phenyl-thiourea
1-[3-Chloro-4-hydroxy-5-(1-hydroxy-ethyl)-phenyl]-3-pentyl-thiourea
1-Benzyl-3-[3-chloro-4-hydroxy-5-(1-hydroxy-ethyl)-phenyl]-thiourea
1-[3-Chloro-4-hydroxy-5-(1-hydroxy-ethyl)-phenyl]-3-(2-methyl-benzyl)-thiourea
1-[3-Chloro-4-hydroxy-5-(1-hydroxy-ethyl)-phenyl]-3-phenethyl-thiourea
1-[3-Chloro-4-hydroxy-5-(1-hydroxy-ethyl)-phenyl]-3-(1,1,3,3-tetramethyl-butyl)-thiourea
1-tert-Butyl-3-[3-chloro-4-hydroxy-5-(1-hydroxy-ethyl)-phenyl]-thiourea
1-[3-Chloro-4-hydroxy-5-(1-hydroxy-ethyl)-phenyl]-3-isopropyl-thiourea
1-[3-Chloro-4-hydroxy-5-(1-hydroxy-ethyl)-phenyl]-3-cyclohexylmethyl-thiourea
1-[3-Chloro-4-hydroxy-5-(1-hydroxy-ethyl)-phenyl]-3-(4-trifluoromethyl-benzyl)-thiourea
1-[3-Chloro-4-hydroxy-5-(1-hydroxy-ethyl)-phenyl]-3-(3,5-dichloro-phenyl)-thiourea
1-[3-Chloro-4-hydroxy-5-(1-hydroxy-ethyl)-phenyl]-3-(4-chloro-phenyl)-thiourea
1-[3-Chloro-4-hydroxy-5-(1-hydroxy-ethyl)-phenyl]-3-(4-trifluoromethyl-phenyl)-thiourea
1-[3-Chloro-4-hydroxy-5-(1-hydroxy-ethyl)-phenyl]-3-cyclohexyl-thiourea
1-[3-Chloro-4-hydroxy-5-(1-hydroxy-ethyl)-phenyl]-3-(2-trifluoromethyl-phenyl)-thiourea
1-[3-Chloro-4-hydroxy-5-(1-hydroxy-ethyl)-phenyl]-3-phenyl-thiourea
2-Chloro-4-(2-phenylsulfanyl-benzylamino)-phenol
2-Chloro-4-(2-p-tolylsulfanyl-benzylamino)-phenol
2-Chloro-4-[2-(4-chloro-phenylsulfanyl)-benzylamino]-phenol
2-Chloro-4-[2-(4-nitro-phenylsulfanyl)-benzylamino]-phenol
2-Chloro-4-[2-(4-methoxy-phenylsulfanyl)-benzylamino]-phenol
2-Chloro-4-[2-(2-chloro-phenylsulfanyl)-benzylamino]-phenol
2-Chloro-4-[2-(3-chloro-phenylsulfanyl)-benzylamino]-phenol
2-Chloro-4-[2-(3,4-dichloro-phenylsulfanyl)-benzylamino]-phenol
N-(4-{2-[(3-Chloro-4-hydroxy-phenylamino)-methyl]-phenylsulfanyl}-phenyl)-acetamide
2-Chloro-4-[2-(quinolin-7-ylsulfanyl)-benzylamino]-phenol
2-Chloro-4-[2-(4-chloro-phenylsulfanyl)-5-nitro-benzylamino]-phenol
2-Chloro-4-(5-nitro-2-p-tolylsulfanyl-benzylamino)-phenol
4-[5-Amino-2-(4-chloro-phenylsulfanyl)-benzylamino]-2-chloro-phenol
2-Chloro-4-[2-(4-chloro-benzenesulfonyl)-benzylamino]-phenol
2,6-Dichloro-4-(2-phenylsulfanyl-benzylamino)-phenol
2,6-Dichloro-4-(2-p-tolylsulfanyl-benzylamino)-phenol
2,6-Dichloro-4-[2-(4-chloro-phenylsulfanyl)-benzylamino]-phenol
2,6-Dichloro-4-[2-(4-nitro-phenylsulfanyl)-benzylamino]-phenol
2,6-Dichloro-4-[2-(4-methoxy-phenylsulfanyl)-benzylamino]-phenol
2,6-Dichloro-4-[2-(2-chloro-phenylsulfanyl)-benzylamino]-phenol
2,6-Dichloro-4-[2-(3-chloro-phenylsulfanyl)-benzylamino]-phenol
2,6-Dichloro-4-[2-(3,4-dichloro-phenylsulfanyl)-benzylamino]-phenol
N-(4-{2-[(3,5-Dichloro-4-hydroxy-phenylamino)-methyl]-phenylsulfanyl}-phenyl)-acetamide
2,6-Dichloro-4-[2-(quinolin-7-ylsulfanyl)-benzylamino]-phenol

2,6-Dichloro-4-[2-(4-chloro-phenylsulfanyl)-5-nitro-benzylamino]-phenol
2,6-Dichloro-4-(5-nitro-2-p-tolylsulfanyl-benzylamino)-phenol
4-[5-Amino-2-(4-chloro-phenylsulfanyl)-benzylamino]-2,6-dichloro-phenol
2,6-Dichloro-4-[2-(4-chloro-benzenesulfonyl)-benzylamino]-phenol
2-Nitro-4-(2-phenylsulfanyl-benzylamino)-phenol
2-Nitro-4-(2-p-tolylsulfanyl-benzylamino)-phenol
4-[2-(4-Chloro-phenylsulfanyl)-benzylamino]-2-nitro-phenol
2-Nitro-4-[2-(4-nitro-phenylsulfanyl)-benzylamino]-phenol
4-[2-(4-Methoxy-phenylsulfanyl)-benzylamino]-2-nitro-phenol
4-[2-(2-Chloro-phenylsulfanyl)-benzylamino]-2-nitro-phenol
4-[2-(3-Chloro-phenylsulfanyl)-benzylamino]-2-nitro-phenol
4-[2-(3,4-Dichloro-phenylsulfanyl)-benzylamino]-2-nitro-phenol
N-(4-{2-[(4-Hydroxy-3-nitro-phenylamino)-methyl]-phenylsulfanyl}-phenyl)-acetamide
2-Nitro-4-[2-(quinolin-7-ylsulfanyl)-benzylamino]-phenol
4-[2-(4-Chloro-phenylsulfanyl)-5-nitro-benzylamino]-2-nitro-phenol
2-Nitro-4-(5-nitro-2-p-tolylsulfanyl-benzylamino)-phenol
N-{4-(4-Chloro-phenylsulfanyl)-3-[(4-hydroxy-3-nitro-phenylamino)-methyl]-phenyl}-acetamide
4-[2-(4-Chloro-benzenesulfonyl)-benzylamino]-2-nitro-phenol
2-Fluoro-4-(2-phenylsulfanyl-benzylamino)-phenol
2-Fluoro-4-(2-p-tolylsulfanyl-benzylamino)-phenol
2-Fluoro-4-[2-(4-chloro-phenylsulfanyl)-benzylamino]-phenol
2-Fluoro-4-[2-(4-nitro-phenylsulfanyl)-benzylamino]-phenol
2-Fluoro-4-[2-(4-methoxy-phenylsulfanyl)-benzylamino]-phenol
2-Fluoro-4-[2-(2-chloro-phenylsulfanyl)-benzylamino]-phenol
2-Fluoro-4-[2-(3-chloro-phenylsulfanyl)-benzylamino]-phenol
2-Fluoro-4-[2-(3,4-dichloro-phenylsulfanyl)-benzylamino]-phenol
N-(4-{2-[(3-Fluoro-4-hydroxy-phenylamino)-methyl]-phenylsulfanyl}-phenyl)-acetamide
2-Fluoro-4-[2-(quinolin-7-ylsulfanyl)-benzylamino]-phenol
2-Fluoro-4-[2-(4-chloro-phenylsulfanyl)-5-nitro-benzylamino]-phenol
2-Fluoro-4-(5-nitro-2-p-tolylsulfanyl-benzylamino)-phenol
4-[5-Amino-2-(4-chloro-phenylsulfanyl)-benzylamino]-2-fluoro-phenol
2-Fluoro-4-[2-(4-chloro-benzenesulfonyl)-benzylamino]-phenol
4-(2-Phenylsulfanyl-benzylamino)-benzene-1,3-diol
4-(2-p-Tolylsulfanyl-benzylamino)-benzene-1,3-diol
4-[2-(4-Chloro-phenylsulfanyl)-benzylamino]-benzene-1,3-diol
4-[2-(4-Nitro-phenylsulfanyl)-benzylamino]-benzene-1,3-diol
4-[2-(4-Methoxy-phenylsulfanyl)-benzylamino]-benzene-1,3-diol
4-[2-(2-Chloro-phenylsulfanyl)-benzylamino]-benzene-1,3-diol
4-[2-(3-Chloro-phenylsulfanyl)-benzylamino]-benzene-1,3-diol
4-[2-(3,4-Dichloro-phenylsulfanyl)-benzylamino]-benzene-1,3-diol
N-(4-{2-[(2,4-Dihydroxy-phenylamino)-methyl]-phenylsulfanyl}-phenyl)-acetamide
4-[2-(Quinolin-7-ylsulfanyl)-benzylamino]-benzene-1,3-diol
4-[2-(4-Chloro-phenylsulfanyl)-5-nitro-benzylamino]-benzene-1,3-diol
4-(5-Nitro-2-p-tolylsulfanyl-benzylamino)-benzene-1,3-diol
4-[5-Amino-2-(4-chloro-phenylsulfanyl)-benzylamino]-benzene-1,3-diol
4-[2-(4-Chloro-benzenesulfonyl)-benzylamino]-benzene-1,3-diol
2,6-Dibromo-4-(2-phenylsulfanyl-benzylamino)-phenol
2,6-Dibromo-4-(2-p-tolylsulfanyl-benzylamino)-phenol
2,6-Dibromo-4-[2-(4-chloro-phenylsulfanyl)-benzylamino]-phenol
2,6-Dibromo-4-[2-(4-nitro-phenylsulfanyl)-benzylamino]-phenol
2,6-Dibromo-4-[2-(4-methoxy-phenylsulfanyl)-benzylamino]-phenol
2,6-Dibromo-4-[2-(2-chloro-phenylsulfanyl)-benzylamino]-phenol
2,6-Dibromo-4-[2-(3-chloro-phenylsulfanyl)-benzylamino]-phenol
2,6-Dibromo-4-[2-(3,4-dichloro-phenylsulfanyl)-benzylamino]-phenol
N-(4-{2-[(3,5-Dibromo-4-hydroxy-phenylamino)-methyl]-phenylsulfanyl}-phenyl)-acetamide
2,6-Dibromo-4-[2-(quinolin-7-ylsulfanyl)-benzylamino]-phenol
2,6-Dibromo-4-[2-(4-chloro-phenylsulfanyl)-5-nitro-benzylamino]-phenol
2,6-Dibromo-4-(5-nitro-2-p-tolylsulfanyl-benzylamino)-phenol

4-[5-Amino-2-(4-chloro-phenylsulfanyl)-benzylamino]-2,6-dibromo-phenol
2,6-Dibromo-4-[2-(4-chloro-benzenesulfonyl)-benzylamino]-phenol
2,6-Difluoro-4-(2-phenylsulfanyl-benzylamino)-phenol
2,6-Difluoro-4-(2-p-tolylsulfanyl-benzylamino)-phenol
4-[2-(4-Chloro-phenylsulfanyl)-benzylamino]-2,6-difluoro-phenol
2,6-Difluoro-4-[2-(4-nitro-phenylsulfanyl)-benzylamino]-phenol
2,6-Difluoro-4-[2-(4-methoxy-phenylsulfanyl)-benzylamino]-phenol
4-[2-(2-Chloro-phenylsulfanyl)-benzylamino]-2,6-difluoro-phenol
4-[2-(3-Chloro-phenylsulfanyl)-benzylamino]-2,6-difluoro-phenol
4-[2-(3,4-Dichloro-phenylsulfanyl)-benzylamino]-2,6-difluoro-phenol
N-(4-{2-[(3,5-Difluoro-4-hydroxy-phenylamino)-methyl]-phenylsulfanyl}-phenyl)-acetamide
2,6-Difluoro-4-[2-(quinolin-7-ylsulfanyl)-benzylamino]-phenol
4-[2-(4-Chloro-phenylsulfanyl)-5-nitro-benzylamino]-2,6-difluoro-phenol
2,6-Difluoro-4-(5-nitro-2-p-tolylsulfanyl-benzylamino)-phenol
4-[5-Amino-2-(4-chloro-phenylsulfanyl)-benzylamino]-2,6-difluoro-phenol
4-[2-(4-Chloro-benzenesulfonyl)-benzylamino]-2,6-difluoro-phenol
2-Chloro-6-(1-hydroxy-ethyl)-4-(2-phenylsulfanyl-benzylamino)-phenol
2-Chloro-6-(1-hydroxy-ethyl)-4-(2-p-tolylsulfanyl-benzylamino)-phenol
2-Chloro-4-[2-(4-chloro-phenylsulfanyl)-benzylamino]-6-(1-hydroxy-ethyl)-phenol
2-Chloro-6-(1-hydroxy-ethyl)-4-[2-(4-nitro-phenylsulfanyl)-benzylamino]-phenol
2-Chloro-6-(1-hydroxy-ethyl)-4-[2-(4-methoxy-phenylsulfanyl)-benzylamino]-phenol
2-Chloro-4-[2-(2-chloro-phenylsulfanyl)-benzylamino]-6-(1-hydroxy-ethyl)-phenol
2-Chloro-4-[2-(3-chloro-phenylsulfanyl)-benzylamino]-6-(1-hydroxy-ethyl)-phenol
2-Chloro-4-[2-(3,4-dichloro-phenylsulfanyl)-benzylamino]-6-(1-hydroxy-ethyl)-phenol
N-[4-(2-{[3-Chloro-4-hydroxy-5-(1-hydroxy-ethyl)-phenylamino]-methyl}-phenylsulfanyl)-phenyl]-acetamide
2-Chloro-6-(1-hydroxy-ethyl)-4-[2-(quinolin-7-ylsulfanyl)-benzylamino]-phenol
2-Chloro-4-[2-(4-chloro-phenylsulfanyl)-5-nitro-benzylamino]-6-(1-hydroxy-ethyl)-phenol
2-Chloro-4-(1-hydroxy-ethyl)-6-(5-nitro-2-p-tolylsulfanyl-benzylamino)-phenol
4-[5-Amino-2-(4-chloro-phenylsulfanyl)-benzylamino]-2-chloro-6-(1-hydroxy-ethyl)-phenol
2-Chloro-4-[2-(4-chloro-benzenesulfonyl)-benzylamino]-6-(1-hydroxy-ethyl)-phenol
2-Hydroxymethyl-4-(2-phenylsulfanyl-benzylamino)-phenol
2-Hydroxymethyl-4-(2-p-tolylsulfanyl-benzylamino)-phenol
4-[2-(4-Chloro-phenylsulfanyl)-benzylamino]-2-hydroxymethyl-phenol
2-Hydroxymethyl-4-[2-(4-nitro-phenylsulfanyl)-benzylamino]-phenol
2-Hydroxymethyl-4-[2-(4-methoxy-phenylsulfanyl)-benzylamino]-phenol
4-[2-(2-Chloro-phenylsulfanyl)-benzylamino]-2-hydroxymethyl-phenol
4-[2-(3-Chloro-phenylsulfanyl)-benzylamino]-2-hydroxymethyl-phenol
4-[2-(3,4-Dichloro-phenylsulfanyl)-benzylamino]-2-hydroxymethyl-phenol
N-(4-{2-[(4-Hydroxy-3-hydroxymethyl-phenylamino)-methyl]-phenylsulfanyl}-phenyl)-acetamide
2-Hydroxymethyl-4-[2-(quinolin-7-ylsulfanyl)-benzylamino]-phenol
4-[2-(4-Chloro-phenylsulfanyl)-5-nitro-benzylamino]-2-hydroxymethyl-phenol
2-Hydroxymethyl-4-(5-nitro-2-p-tolylsulfanyl-benzylamino)-phenol
4-[5-Amino-2-(4-chloro-phenylsulfanyl)-benzylamino]-2-hydroxymethyl-phenol
4-[2-(4-Chloro-benzenesulfonyl)-benzylamino]-2-hydroxymethyl-phenol
1-[3-Chloro-2-hydroxy-5-(2-phenylsulfanyl-benzylamino)-phenyl]-ethanone
1-[3-Chloro-2-hydroxy-5-(2-p-tolylsulfanyl-benzylamino)-phenyl]-ethanone
1-{3-Chloro-5-[2-(4-chloro-phenylsulfanyl)-benzylamino]-2-hydroxy-phenyl}-ethanone
1-{3-Chloro-2-hydroxy-5-[2-(4-nitro-phenylsulfanyl)-benzylamino]-phenyl}-ethanone
1-{3-Chloro-2-hydroxy-5-[2-(4-methoxy-phenylsulfanyl)-benzylamino]-phenyl}-ethanone
1-{3-Chloro-5-[2-(2-chloro-phenylsulfanyl)-benzylamino]-2-hydroxy-phenyl}-ethanone
1-{3-Chloro-5-[2-(3-chloro-phenylsulfanyl)-benzylamino]-2-hydroxy-phenyl}-ethanone
1-{3-Chloro-5-[2-(3,4-dichloro-phenylsulfanyl)-benzylamino]-2-hydroxy-phenyl}-ethanone
N-(4-{2-[(3-Acetyl-5-chloro-4-hydroxy-phenylamino)-methyl]-phenylsulfanyl}-phenyl)-acetamide
1-{3-Chloro-2-hydroxy-5-[2-(quinolin-7-ylsulfanyl)-benzylamino]-phenyl}-ethanone
1-{3-Chloro-5-[2-(4-chloro-phenylsulfanyl)-5-nitro-benzylamino]-2-hydroxy-phenyl}-ethanone
1-[3-Chloro-2-hydroxy-5-(5-nitro-2-p-tolylsulfanyl-benzylamino)-phenyl]-ethanone
1-{5-[5-Amino-2-(4-chloro-phenylsulfanyl)-benzylamino]-3-chloro-2-hydroxy-phenyl}-ethanone
1-{3-Chloro-5-[2-(4-chloro-benzenesulfonyl)-benzylamino]-2-hydroxy-phenyl}-ethanone

2-Hydroxy-5-(2-phenylsulfanyl-benzylamino)-benzoic acid
2-Hydroxy-5-(2-p-tolylsulfanyl-benzylamino)-benzoic acid
5-[2-(4-Chloro-phenylsulfanyl)-benzylamino]-2-hydroxy-benzoic acid
2-Hydroxy-5-[2-(4-nitro-phenylsulfanyl)-benzylamino]-benzoic acid
2-Hydroxy-5-[2-(4-methoxy-phenylsulfanyl)-benzylamino]-benzoic acid
5-[2-(2-Chloro-phenylsulfanyl)-benzylamino]-2-hydroxy-benzoic acid
5-[2-(3-Chloro-phenylsulfanyl)-benzylamino]-2-hydroxy-benzoic acid
5-[2-(3,4-Dichloro-phenylsulfanyl)-benzylamino]-2-hydroxy-benzoic acid
5-[2-(4-Acetylamino-phenylsulfanyl)-benzylamino]-2-hydroxy-benzoic acid
2-Hydroxy-5-[2-(quinolin-7-ylsulfanyl)-benzylamino]-benzoic acid
5-[2-(4-Chloro-phenylsulfanyl)-5-nitro-benzylamino]-2-hydroxy-benzoic acid
2-Hydroxy-5-(5-nitro-2-p-tolylsulfanyl-benzylamino)-benzoic acid
5-[5-Amino-2-(4-chloro-phenylsulfanyl)-benzylamino]-2-hydroxymethyl-phenol
5-[2-(4-Chloro-benzenesulfonyl)-benzylamino]-2-hydroxy-benzoic acid
2-Fluoro-6-nitro-4-(2-phenylsulfanyl-benzylamino)-phenol
2-Fluoro-6-nitro-4-(2-p-tolylsulfanyl-benzylamino)-phenol
4-[2-(4-Chloro-phenylsulfanyl)-benzylamino]-2-fluoro-6-nitro-phenol
2-Fluoro-6-nitro-4-[2-(4-nitro-phenylsulfanyl)-benzylamino]-phenol
2-Fluoro-4-[2-(4-methoxy-phenylsulfanyl)-benzylamino]-6-nitro-phenol
4-[2-(2-Chloro-phenylsulfanyl)-benzylamino]-2-fluoro-6-nitro-phenol
4-[2-(3-Chloro-phenylsulfanyl)-benzylamino]-2-fluoro-6-nitro-phenol
4-[2-(3,4-Dichloro-phenylsulfanyl)-benzylamino]-2-fluoro-6-nitro-phenol
N-(4-{2-[(3-Fluoro-4-hydroxy-5-nitro-phenylamino)-methyl]-phenylsulfanyl}-phenyl)-acetamide
2-Fluoro-6-nitro-4-[2-(quinolin-7-ylsulfanyl)-benzylamino]-phenol
4-[2-(4-Chloro-phenylsulfanyl)-5-nitro-benzylamino]-2-fluoro-6-nitro-phenol
2-Fluoro-6-nitro-4-(5-nitro-2-p-tolylsulfanyl-benzylamino)-phenol
4-[5-Amino-2-(4-chloro-phenylsulfanyl)-benzylamino]-2-fluoro-6-nitro-phenol
4-[2-(4-Chloro-benzenesulfonyl)-benzylamino]-2-fluoro-6-nitro-phenol
2,6-Dichloro-4-(3-phenoxy-benzylamino)-phenol
2,6-Dichloro-4-[3-(4-chloro-phenoxy)-benzylamino]-phenol
4-[3-(4-tert-Butyl-phenoxy)-benzylamino]-2,6-dichloro-phenol
4-(3-Benzyloxy-benzylamino)-2,6-dichloro-phenol
4-(2-Benzyloxy-benzylamino)-2,6-dichloro-phenol
2,6-Dichloro-4-[(naphthalen-1-ylmethyl)-amino]-phenol
2,6-Dichloro-4-(4-methylsulfanyl-benzylamino)-phenol
2,6-Dichloro-4-(2-ethylsulfanyl-benzylamino)-phenol
2,6-Dichloro-4-(2-morpholin-4-yl-benzylamino)-phenol
2,6-Dichloro-4-{[2-(4-chloro-phenylsulfanyl)-thiophen-3-ylmethyl]-amino}-phenol
2,6-Dichloro-4-[(5-phenyl-2H-imidazol-4-ylmethyl)-amino]-phenol
4-[(5-Bromo-thiophen-2-ylmethyl)-amino]-2,6-dichloro-phenol
2,6-Dichloro-4-[3-(4-methoxy-phenoxy)-benzylamino]-phenol
2,6-Dichloro-4-(3-methyl-benzylamino)-phenol
2,6-Dichloro-4-(3-trifluoromethyl-benzylamino)-phenol
2,6-Dichloro-6-(2-chloro-6-fluoro-benzylamino)-phenol
4-(3-Phenoxy-benzylamino)-benzene-1,3-diol
4-[3-(4-Chloro-phenoxy)-benzylamino]-benzene-1,3-diol
4-[3-(4-tert-Butyl-phenoxy)-benzylamino]-benzene-1,3-diol
4-(3-Benzyloxy-benzylamino)-benzene-1,3-diol
4-(2-Benzyloxy-benzylamino)-benzene-1,3-diol
4-[(Naphthalen-1-ylmethyl)-amino]-benzene-1,3-diol
4-(4-Methylsulfanyl-benzylamino)-benzene-1,3-diol
4-(2-Ethylsulfanyl-benzylamino)-benzene-1,3-diol
4-(2-Morpholin-4-yl-benzylamino)-benzene-1,3-diol
4-{[2-(4-Chloro-phenylsulfanyl)-thiophen-3-ylmethyl]-amino}-benzene-1,3-diol
4-[(5-Phenyl-2H-imidazol-4-ylmethyl)-amino]-benzene-1,3-diol
2-[(5-Bromo-thiophen-2-ylmethyl)-amino]-4,6-dichloro-3-methyl-phenol
4-[3-(4-Methoxy-phenoxy)-benzylamino]-benzene-1,3-diol
4-(3-Methyl-benzylamino)-benzene-1,3-diol

4-(3-Trifluoromethyl-benzylamino)-benzene-1,3-diol
4-(2-Chloro-6-fluoro-benzylamino)-benzene-1,3-diol
2-Chloro-4-(3-phenoxy-benzylamino)-phenol
2-Chloro-4-[3-(4-chloro-phenoxy)-benzylamino]-phenol
4-[3-(4-tert-Butyl-phenoxy)-benzylamino]-2-chloro-phenol
4-(3-Benzyloxy-benzylamino)-2-chloro-phenol
4-(2-Benzyloxy-benzylamino)-2-chloro-phenol
2-Chloro-4-[(naphthalen-1-ylmethyl)-amino]-phenol
2-Chloro-4-(4-methylsulfanyl-benzylamino)-phenol
2-Chloro-4-(2-ethylsulfanyl-benzylamino)-phenol
2-Chloro-4-(2-morpholin-4-yl-benzylamino)-phenol
2-Chloro-4-{[2-(4-chloro-phenylsulfanyl)-thiophen-3-ylmethyl]-amino}-phenol
2-Chloro-4-[(5-phenyl-2H-imidazol-4-ylmethyl)-amino]-phenol
4-[(5-Bromo-thiophen-2-ylmethyl)-amino]-2-chloro-phenol
2-Chloro-4-[3-(4-methoxy-phenoxy)-benzylamino]-phenol
2-Chloro-4-(3-methyl-benzylamino)-phenol
2-Chloro-4-(3-trifluoromethyl-benzylamino)-phenol
2-Chloro-4-(2-chloro-6-fluoro-benzylamino)-phenol
2-Fluoro-4-(3-phenoxy-benzylamino)-phenol
2-Fluoro-4-[3-(4-chloro-phenoxy)-benzylamino]-phenol
4-[3-(4-tert-Butyl-phenoxy)-benzylamino]-2-fluoro-phenol
4-(3-Benzyloxy-benzylamino)-2-fluoro-phenol
4-(2-Benzyloxy-benzylamino)-2-fluoro-phenol
2-Fluoro-4-[(naphthalen-1-ylmethyl)-amino]-phenol
2-Fluoro-4-(4-methylsulfanyl-benzylamino)-phenol
2-Fluoro-4-(2-ethylsulfanyl-benzylamino)-phenol
2-Fluoro-4-(2-morpholin-4-yl-benzylamino)-phenol
2-Fluoro-4-{[2-(4-chloro-phenylsulfanyl)-thiophen-3-ylmethyl]-amino}-phenol
2-Fluoro-4-[(5-phenyl-2H-imidazol-4-ylmethyl)-amino]-phenol
4-[(5-Bromo-thiophen-2-ylmethyl)-amino]-2-fluoro-phenol
2-Fluoro-4-[3-(4-methoxy-phenoxy)-benzylamino]-phenol
2-Fluoro-4-(3-methyl-benzylamino)-phenol
2-Fluoro-4-(3-trifluoromethyl-benzylamino)-phenol
2-Fluoro-4-(2-chloro-6-fluoro-benzylamino)-phenol
2,6-Difluoro-4-(3-phenoxy-benzylamino)-phenol
2,6-Difluoro-4-[3-(4-chloro-phenoxy)-benzylamino]-phenol
4-[3-(4-tert-Butyl-phenoxy)-benzylamino]-2,6-difluoro-phenol
4-(3-Benzyloxy-benzylamino)-2,6-difluoro-phenol
4-(2-Benzyloxy-benzylamino)-2,6-difluoro-phenol
2,6-Difluoro-4-[(naphthalen-1-ylmethyl)-amino]-phenol
2,6-Difluoro-4-(4-methylsulfanyl-benzylamino)-phenol
2,6-Difluoro-4-(2-ethylsulfanyl-benzylamino)-phenol
2,6-Difluoro-4-(2-morpholin-4-yl-benzylamino)-phenol
2,6-Difluoro-4-{[2-(4-chloro-phenylsulfanyl)-thiophen-3-ylmethyl]-amino}-phenol
2,6-Difluoro-4-[(5-phenyl-2H-imidazol-4-ylmethyl)-amino]-phenol
4-[(5-Bromo-thiophen-2-ylmethyl)-amino]-2,6-difluoro-phenol
2,6-Difluoro-4-[3-(4-methoxy-phenoxy)-benzylamino]-phenol
2,6-Difluoro-4-(3-methyl-benzylamino)-phenol
2,6-Difluoro-4-(3-trifluoromethyl-benzylamino)-phenol
2,6-Difluoro-4-(2-chloro-6-fluoro-benzylamino)-phenol
N-(2,4-Dihydroxy-phenyl)-C-phenyl-methanesulfonamide
Butane-1-sulfonic acid (2,4-dihydroxy-phenyl)-amide
Octane-1-sulfonic acid (2,4-dihydroxy-phenyl)-amide
Propane-2-sulfonic acid (2,4-dihydroxy-phenyl)-amide
N-(3,5-Dichloro-4-hydroxy-phenyl)-C-phenyl-methanesulfonamide
Butane-1-sulfonic acid (3,5-dichloro-4-hydroxy-phenyl)-amide
Octane-1-sulfonic acid (3,5-dichloro-4-hydroxy-phenyl)-amide
Propane-2-sulfonic acid (3,5-dichloro-4-hydroxy-phenyl)-amide

N-(3-Chloro-4-hydroxy-phenyl)-C-phenyl-methanesulfonamide
Butane-1-sulfonic acid (3-chloro-4-hydroxy-phenyl)-amide
Octane-1-sulfonic acid (3-chloro-4-hydroxy-phenyl)-amide
Propane-2-sulfonic acid (3-chloro-4-hydroxy-phenyl)-amide
N-(3-Fluoro-4-hydroxy-phenyl)-C-phenyl-methanesulfonamide
Butane-1-sulfonic acid (3-fluoro-4-hydroxy-phenyl)-amide
Octane-1-sulfonic acid (3-fluoro-4-hydroxy-phenyl)-amide
Propane-2-sulfonic acid (3-fluoro-4-hydroxy-phenyl)-amide
N-(3,5-Difluoro-4-hydroxy-phenyl)-C-phenyl-methanesulfonamide
Butane-1-sulfonic acid (3,5-difluoro-4-hydroxy-phenyl)-amide
Octane-1-sulfonic acid (3,5-difluoro-4-hydroxy-phenyl)-amide
Propane-2-sulfonic acid (3,5-difluoro-4-hydroxy-phenyl)-amide
(2,4-Dihydroxy-phenyl)-carbamic acid hexyl ester
(3,5-Dichloro-4-hydroxy-phenyl)-carbamic acid hexyl ester
(3-Chloro-4-hydroxy-phenyl)-carbamic acid hexyl ester
(3-Fluoro-4-hydroxy-phenyl)-carbamic acid hexyl ester
(3,5-Dibromo-4-hydroxy-phenyl)-carbamic acid hexyl ester
(3,5-Difluoro-4-hydroxy-phenyl)-carbamic acid hexyl ester
-[3-(2,4-Dihydroxy-phenyl)-ureido]-4-methyl-pentanoic acid ethyl ester
2-[3-(3,5-Dichloro-4-hydroxy-phenyl)-ureido]-4-methyl-pentanoic acid ethyl ester
2-[3-(3-Chloro-4-hydroxy-phenyl)-ureido]-4-methyl-pentanoic acid ethyl ester
2-[3-(3-Fluoro-4-hydroxy-phenyl)-ureido]-4-methyl-pentanoic acid ethyl ester
2-[3-(3,5-Difluoro-4-hydroxy-4-phenyl)-ureido]-4-methyl-pentanoic acid ethyl ester
2-[3-(3,5-Dibromo-4-hydroxy-4-methyl-phenyl)-ureido]-4-methyl-pentanoic acid ethyl ester
2-[3-(2,4-Dihydroxy-phenyl)-ureido]-3-phenyl-propionic acid ethyl ester
2-[3-(3,5-Dichloro-4-hydroxy-phenyl)-ureido]-3-phenyl-propionic acid ethyl ester
2-[3-(3-Chloro-4-hydroxy-phenyl)-ureido]-3-phenyl-propionic acid ethyl ester
2-[3-(3-Fluoro-4-hydroxy-phenyl)-ureido]-3-phenyl-propionic acid ethyl ester
2-[3-(3,5-Difluoro-4-hydroxy-phenyl)-ureido]-3-phenyl-propionic acid ethyl ester
2-[3-(3,5-Dibromo-3-fluoro-4-hydroxy-phenyl)-ureido]-3-phenyl-propionic acid ethyl ester
3,5,5-Trimethyl-hexanoic acid (2,4-dihydroxy-phenyl)-amide
3,5,5-Trimethyl-hexanoic acid (3,5-dichloro-4-hydroxy-phenyl)-amide
3,5,5-Trimethyl-hexanoic acid (3-chloro-4-hydroxy-phenyl)-amide
3,5,5-Trimethyl-hexanoic acid (3-fluoro-4-hydroxy-phenyl)-amide
3,5,5-Trimethyl-hexanoic acid (3,5-difluoro-4-hydroxy-phenyl)-amide
3,5,5-Trimethyl-hexanoic acid (3,5-dibromo-4-hydroxy-phenyl)-amide
1-(3-Benzothiazol-2-yl-5-chloro-4-hydroxy-phenyl)-3-tert-butyl-urea
1-(3-Benzothiazol-2-yl-5-chloro-4-hydroxy-phenyl)-3-benzyl-urea
1-(3-Benzothiazol-2-yl-5-chloro-4-hydroxy-phenyl)-3-phenethyl-urea
1-(3-Benzothiazol-2-yl-5-chloro-4-hydroxy-phenyl)-3-isopropyl-thiourea
1-(3-Benzothiazol-2-yl-5-chloro-4-hydroxy-phenyl)-3-tert-butyl-thiourea
3,5,5-Trimethyl-hexanoic acid (3-benzothiazol-2-yl-5-chloro-4-hydroxy-phenyl)-amide
N-(3-Benzothiazol-2-yl-5-chloro-4-hydroxy-phenyl)-3-phenyl-propionamide
1-(4-Hydroxy-2-methyl-phenyl)-3-pentyl-urea
Biphenyl-4-carboxylic acid (2,4-dihydroxy-phenyl)-amide
Biphenyl-4-carboxylic acid (3,5-dichloro-4-hydroxy-phenyl)-amide
4-[(Furan-2-ylmethyl)-amino]-benzene-1,3-diol
2,6-Dichloro-4-[(furan-2-ylmethyl)-amino]-phenol
2,6-Difluoro-4-[(furan-2-ylmethyl)-amino]-phenol
4-(2-Trifluoromethyl-benzylamino)-benzene-1,3-diol
2,6-Difluoro-4-(2-trifluoromethyl-benzylamino)-phenol
N-(3,5-Dichloro-4-hydroxy-phenyl)-3-phenyl-propionamide
N-(3,5-Dichloro-4-hydroxy-phenyl)-2-(2-nitro-phenyl)-acetamide
2-Benzo[1,3]dioxol-5-yl-N-(3,5-dichloro-4-hydroxy-phenyl)-acetamide
3-Methyl-but-2-enoic acid (3,5-dichloro-4-hydroxy-phenyl)-amide
Naphthalene-2-carboxylic acid (3,5-dichloro-4-hydroxy-phenyl)-amide
N-(3,5-Dichloro-4-hydroxy-phenyl)-benzamide
Furan-2-carboxylic acid (3,5-dichloro-4-hydroxy-phenyl)-amide

2,6-Dichloro-4-(2-trifluoromethyl-benzylamino)-phenol

**9.** A pharmaceutical composition comprising a compound according to any of claims 1 to 8 and a pharmaceutically acceptable carrier, diluent or excipient.

**10.** Use of a compound according to any of the preceding claims for the manufacture of a medicament.

**11.** Use of a compound for the manufacture of a medicament for the treatment of a disease, whereby the disease involves an abnormal cell proliferation, an undesired cell proliferation, an abnormal mitosis and/or an undesired mitosis.
whereby the compound is a compound according to any of the preceding claims.

**12.** The use according to claim 11, wherein the disease is selected from the group comprising neurodegenerative diseases, stroke, inflammatory diseases, immune based disorders, infectious diseases, heart diseases, cardiovascular diseases and cell proliferative diseases.

**13.** Use of a compound according to any of claims 1 to 8 as an inhibitor to a rotamase.

**14.** A method for the treatment of subject in need thereof comprising the administration of a compound according to any one of claims 1 to 8 or of a pharmaceutical composition according to claim 9.

**15.** A compound according to any of claims 1 to 8 for use in method of treatment of a subject.

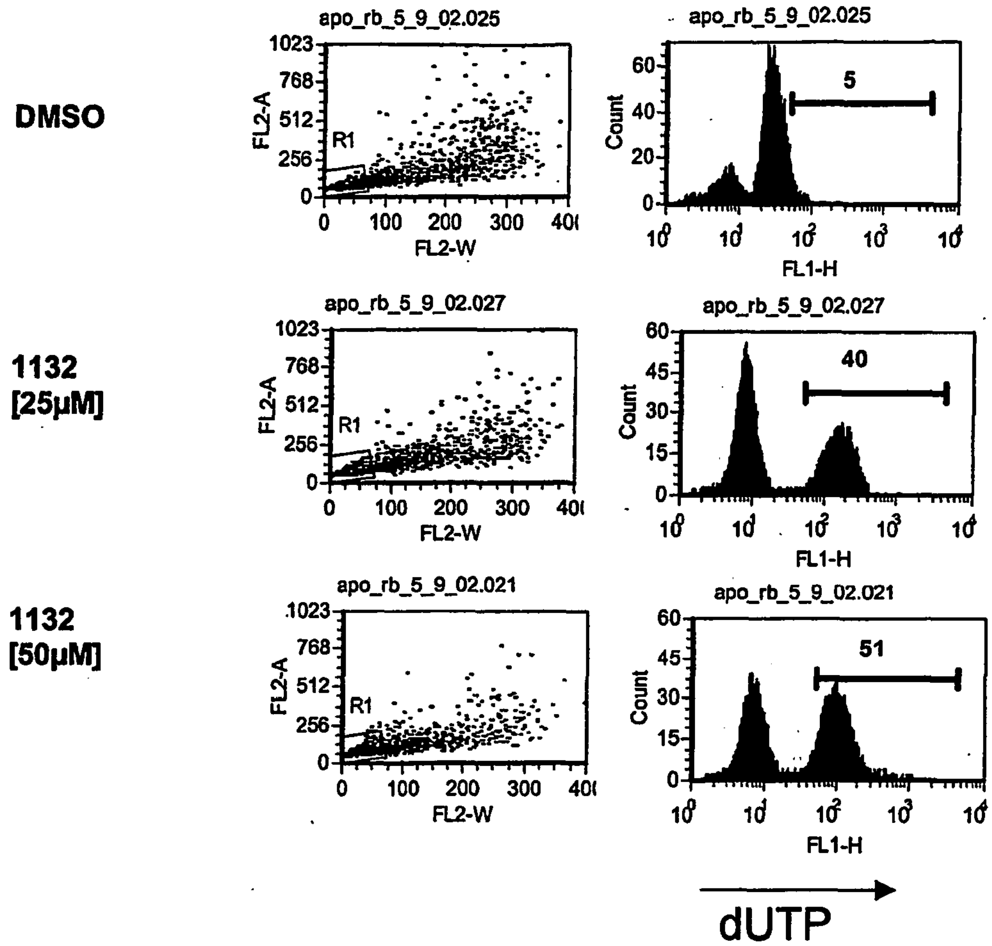
DMSO
1132
[25µM]
1132
[50µM]
apo_rb_5_9_02.025
apo_rb_5_9_02.027
apo_rb_5_9_02.021
FL2-A
FL2-W
R1
Count
FL1-H
5
40
51
dUTP

Fig. 1

# Apoptotic Activity on Tumor Cells
# FACS Analysis (TUNEL Assay)

Apoptotic HL60 cells appear as a population with higher fluorescence.

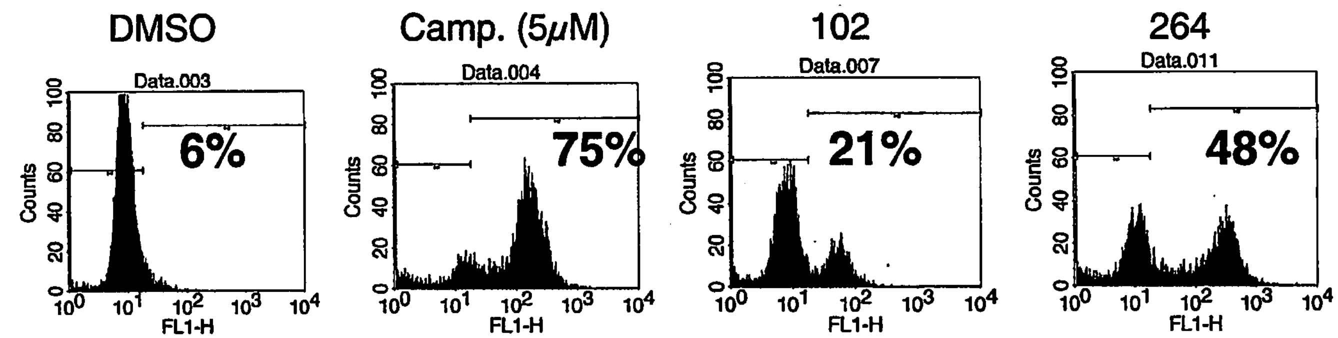


**Percentage of apoptotic cells**

Fig. 2

# Apoptotic Activity on Tumor Cells

Fluorescence microscope DAPI staining

HeLa

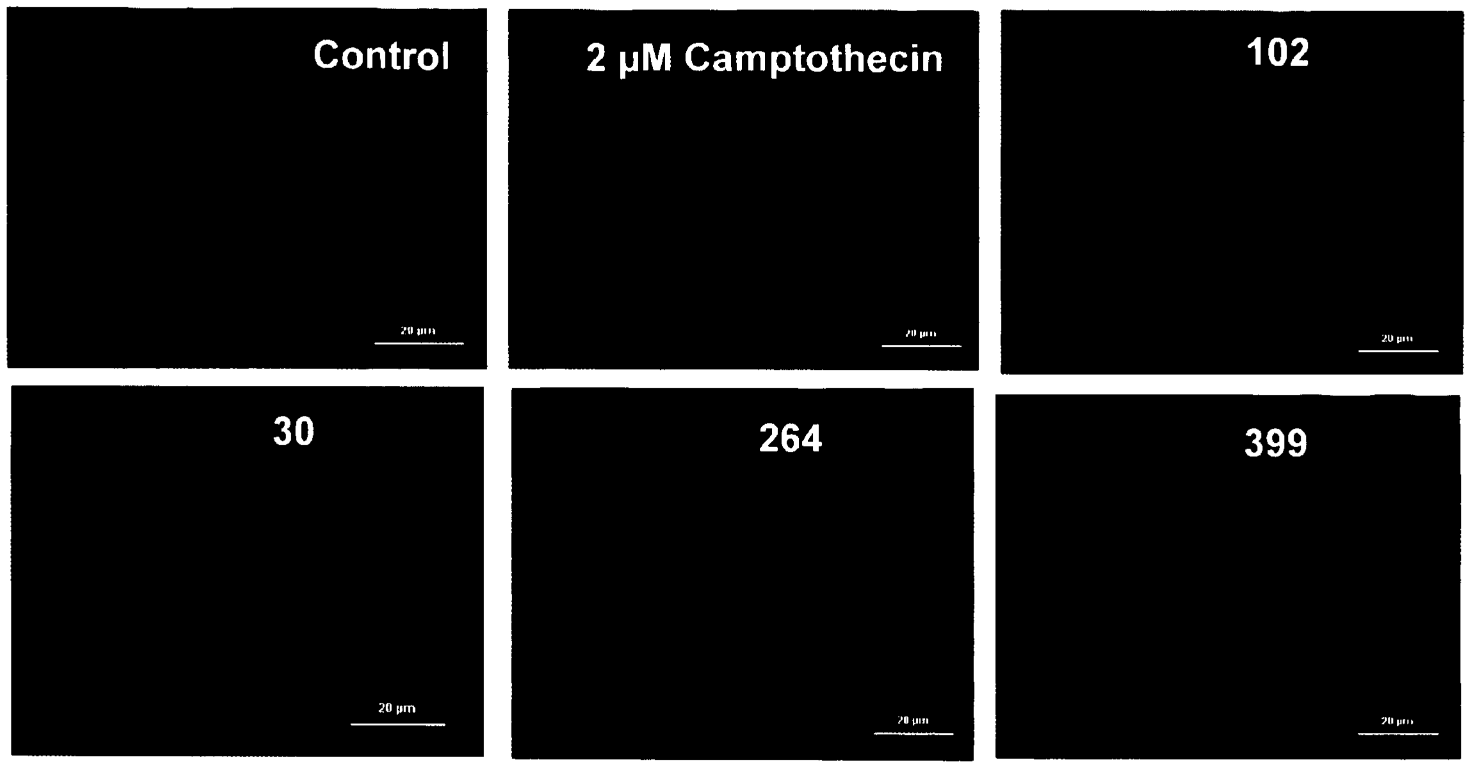


Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0610743 A
- WO 9119501 A
- US 4880635 A, Janoff

### Non-patent literature cited in the description


- **Lu et al.** *Nature,* 1996, vol. 380, 544-547
- **Campbell et al.** *Genomics,* 1997, vol. 44, 157-162
- **Rahfeld et al.** *FEBS Letts,* 1994, vol. 352, 180-184
- **Maleszka et al.** *Proc Natl Acad Sci USA,* 1996, vol. 93, 447-451
- **Hanes et al.** *Yeast,* 1989, vol. 5, 55-72
- **Hani et al.** *FEBS Letts,* 1995, vol. 365, 198-202
- *J. Med. Chem,* 1977, vol. 20, 901-906
- **Walsh.** Enzymatic Reaction Mechanisms. Freeman & Co, 1979
- **Irwin Segel.** Enzyme Kinetics: Behavior and analysis of rapid equilibrium and steady-state enzyme systems. Wiley-Interscience Publication, John Wiley & Sons, 1975
- **Irwin Segel.** Enzyme Kinetics: Behavior and analysis of rapid equilibrium and steady-state enzyme systems. John Wiley & Sons, 1975
- Remington's Pharmaceutical Sciences. Mack Publishing Co, 1990
- **Fischer, G. ; Bang, H. ; Mech, C.** Determination of enzymatic catalysis fort he cis-trans-isomerization of peptide binding in proline-containing peptides. [German]. *Biomed. Biochem. Acta,* 1984, vol. 43, 1101-1111
- **Hennig et al.** Selective Inactivation of Parvulin-like peptidyl-prolyl cis/trans isomerases by Juglon. *Biochemistry,* 1998, vol. 37 (17), 5953-5960
- **Gothel, S. F. ; Marahiel, M. A.** TI Peptidyl-prolyl cis-trans isomerases, a superfamily of ubiquitous folding catalysts [Review]. *Cell. Molec. Life Sci.,* 1999, vol. 55, 423-436
- **Lu, K. P. ; Hanes, S. D. ; Hunter, T.** A human peptidyl-prolyl isomerase essential for regulation of mitosis. *Nature 1996,* 1996, vol. 380, 544-547
- **Yaffe, M. B. ; Schutkowski, M. ; Shen, M. H. ; Zhou, X. Z. ; Stukenberg, P. T. ; Rahfeld, J. U. ; Xu, J. ; Kuang, J. ; Kirschner, M. W. ; Fischer, G.** SEQUENCE-SPECIFIC AND PHOSPHORYLATION-DEPENDENT PROLINE ISOMERIZATION - A POTENTIAL MITOTIC REGULATORY MECHANISM. *Science,* 1997, vol. 278, 1957-1960
- **Shen, M. ; Stukenberg, P. T. ; Kirschner, M. W. ; Lu, K. P.** The essential mitotic peptidyl-prolyl isomerase Pinl binds and regulates mitosis-specific phosphoproteins. *Genes Developm.,* 1998, vol. 12, 706-720
- **Rahfeld JU. ; Schierhorn A. ; Mann K. ; Fischer G.** A novel peptidyl-prolyl cis/trans isomerase from Escherichia coli. *FEBS Letters,* 1994, vol. 343, 65-69
- **Rahfeld JU. ; Rucknagel KP. ; Schelbert B. ; Ludwig B. ; Hacker J. ; Mann K. ; Fischer G.** Confirmation of the existence of a third family among peptidyl-prolyl cis/trans isomerases. Amino acid sequence and recombinant production of parvulin. *FEBS Letters.,* 1994, vol. 352, 180-184
- **Fischer, G.** Peptidyl-prolyl cis/trans isomerases and their effectors. *Angew. Chem., Int. Ed. Engl.,* 1994, vol. 33, 1415-1436
- **Galat, A. ; Metcalfe, S. M.** Peptidylproline cis/trans isomerases. *Prog. Biophys. Molec. Biol.,* 1995, vol. 63, 67-118
- **Schmidt B. ; Tradler T. ; Rahfeld JU. ; Ludwig B. ; Jain B. ; Mann K. ; Rucknagel KP. ; Janowski B. ; Schierhorn A. ; Kullertz G.** A cyclophilin-like peptidyl-prolyl cis/trans isomerase from Legionella pneumophila--characterization, molecular cloning and overexpression. *Mol. Microbiol.,* 1996, vol. 21, 1147-1160
- **Schomburg, B. ; Salzmann M.** GBF: Enzyme Handbook. Springer Verlag, 1991
- Enzymes 3: Peptides, Proteinases and Their Inhibitors. **Bergmeyer, H. U. ; Bergmeyer, J. ; Graß1, M.** Methods of Enzymatic Analysis. VCH, Weinheim, 1988, vol. V, 55-371
- *Nature,* 28 May 1998, vol. 393 (6683), 396
- *Nature,* 1998, vol. 391, 43-50

- **Darzynkiewicz Z. ; Juan G. ; Li X. ; Gorczyca W. ; Murakami T. ; Traganos F.** Cytometry in cell necrobiology: analysis of apoptosis and accidental cell death (necrosis). *Cytometry.,* 1997, vol. 27, 1-20